# EUROPEAN PATENT APPLICATION

(11) **EP 4 799 699 A2**
(43) Date of publication of application: **02.09.2026**
(21) Application number: 26192481.5
(22) Date of filing: 09.12.2022
(51) Int. Cl.: A61P 35/00

(54) **INHIBITORS OF MENIN-MLL INTERACTION**

(30) Priority: 09.12.2021 US 202163287716 P; 03.02.2022 US 202263306399 P; 11.08.2022 US 202263397322 P
(62) Document number(s) of the earlier application(s) in accordance with Art. 76 EPC: 22905177.6 / 4 444 300
(71) Applicant: Bala Therapeutics, Inc., San Diego, California 92130 (US)
(72) Inventor: ABAGYAN, Ruben, La Lolla, 92037 (US); PARCHINSKY, Vladislav Zenonovich, 141407 Moscow (RU); SAVCHUK, Nikolay, San Diego, 92130 (US); IVACHTCHENKO, Alexandre Vasilievich, Florida, 33009 (US); KHVAT, Alexander, San Diego, 92131 (US)
(74) Representative: Zellentin & Partner mbB Patentanwälte

(57) **Abstract**

The present invention is directed to the compounds of Formula (I) - inhibitors of the interaction of menin and MLL. The inhibitors described herein can be useful in the treatment of diseases or disorders associated with menin-MLL interaction, such as cancer. In particular, the invention is concerned with compounds and pharmaceutical compositions inhibiting/blocking menin-MLL interaction, methods of treating diseases or disorders associated with menin-MLL interaction, and methods of synthesizing these compounds.

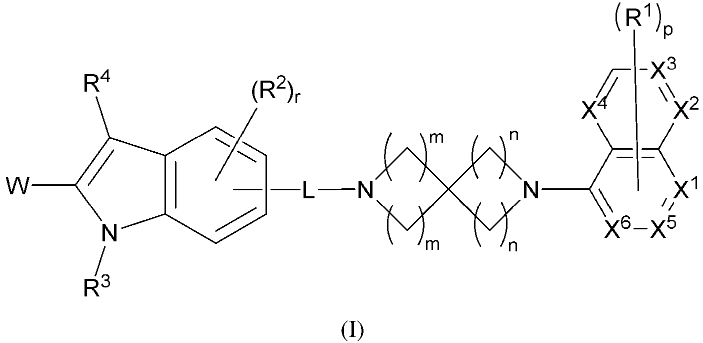

## Description

### CROSS-REFERENCE TO RELATED APPLICATIONS

This application claims priority to, and the benefit of, U.S. Provisional Patent 5 Application Serial No. 63/287,716 filed on December 9, 2021 and entitled "INHIBITORS OF MENIN-MLL INTERACTION," U.S. Provisional Patent Application Serial No. 63/306,399 filed on February 3, 2022 and entitled "INHIBITORS OF MENIN-MLL INTERACTION," and U.S. Provisional Patent Application Serial No. 63/397,322 filed on August 11, 2022 and entitled "INHIBITORS OF MENIN-MLL INTERACTION," the disclosures of which are 10 incorporated herein by reference in their entireties for all purposes.

### FIELD OF INVENTION

The present invention is directed to inhibitors of the interaction of menin and MLL. The inhibitors described herein can be useful in the treatment of diseases or disorders associated with menin-MLL interaction, such as cancer. In particular, the invention is concerned with compounds and pharmaceutical compositions inhibiting/blocking menin-MLL interaction, methods of treating diseases or disorders associated with menin-MLL interaction, and methods of synthesizing these compounds.

### BACKGROUND

Translocations of the MLL (mixed lineage leukemia) gene frequently occur in aggressive human acute myeloid and lymphoid leukemias in both children and adults. Fusion of MLL with 1 of more than 60 different genes results in chimeric MLL fusion proteins that enhance proliferation and block hematopoietic differentiation, ultimately leading to acute leukemia. Patients with leukemias harboring MLL translocations have very unfavorable prognoses and respond poorly to currently available treatments. The relapse risk is very high using conventional chemotherapy and stem cell transplantation, leading to an overall 5-year survival rate of only approximately 35% of patients.

Menin is an essential co-factor of oncogenic *MLL* fusion proteins and the menin-*MLL* interaction is critical for development of acute leukemia *in vivo*. Targeting the menin-*MLL* interaction with small molecules represents an attractive strategy to develop new anticancer agents. Recent developments, including determination of menin crystal structure and development of potent small molecule and peptidomimetic inhibitors, demonstrate feasibility of targeting the menin-*MLL* interaction. On the other hand, biochemical and structural studies revealed that MLL binds to menin in a complex bivalent mode engaging two *MLL* motifs, and therefore inhibition of this protein-protein interaction represents a challenge.

Chromosomal rearrangements of the *MLL* gene located at chromosome band 11q23 are found in patients with *de novo* acute myeloid (AML) and acute lymphoblastic (ALL) leukemias, and in therapy related leukemias or myelodysplastic syndrome (MDS). As a consequence of chromosomal translocations, the *MLL* gene is fused with one of over 60 different protein partners, such as the most frequent AF4, AF9, ENL, AF6, ELL, and AF10. Disruption of *MLL* by gene fusions upregulates expression of *HOXA9* and *MEIS1* genes that are critical to leukemogenesis. The role of *HOXA* genes in leukemic transformation has been verified in both, *in vitro* and *in vivo* models, demonstrating that MLL fusion protein mediated upregulation of *HOXA9* and *MEIS1* genes results in enhanced proliferation and blockage of hematopoietic differentiation, ultimately leading to acute leukemia. Patients with leukemias harboring *MLL* translocations have very unfavorable prognosis (20% event free survival at 3 years) and respond poorly to available treatments, demonstrating a clear need for new therapies.

The oncogenic function of MLL fusion proteins is critically dependent on their direct interaction with menin. Menin is a 67 kDa protein encoded by the *MEN1* (Multiple Endocrine Neoplasia I) gene localized on chromosome 11q13. Menin is a ubiquitously expressed protein, predominantly localized in the nucleus. Menin directly binds to the *N-*terminus of MLL that is retained in all MLL fusion proteins and plays an important role in recruitment of MLL and MLL fusions to target genes, including *HOXA9*. Loss of menin binding by MLL fusion proteins abolishes their oncogenic properties *in vitro* and *in vivo*. Mutations within the *N*-terminus of MLL-ENL oncoprotein, resulting in protein unable to associate with menin, abolish its potential to upregulate *Hox* gene expression and induce leukemia in mice. Expression of a dominant-negative inhibitor composed of the amino terminal MLL sequence inhibits growth of the MLL-AF9 transformed bone marrow cells and blocks leukemogenic transformation.

Inhibiting the interaction of menin with the histone methyltransferase MLL1 (KMT2A) has recently emerged as a novel therapeutic strategy. Beneficial therapeutic effects have been postulated in leukemia, prostate, breast, liver and in synovial sarcoma models. In those indications, MLL1 recruitment by menin was described to critically regulate the expression of disease associated genes.

Blocking the menin-MLL interaction might represent a viable approach to reverse the oncogenic activity of MLL fusion proteins in leukemia and may lead to novel therapeutics.

### SUMMARY

A first aspect of the invention relates to compounds of Formula (I): 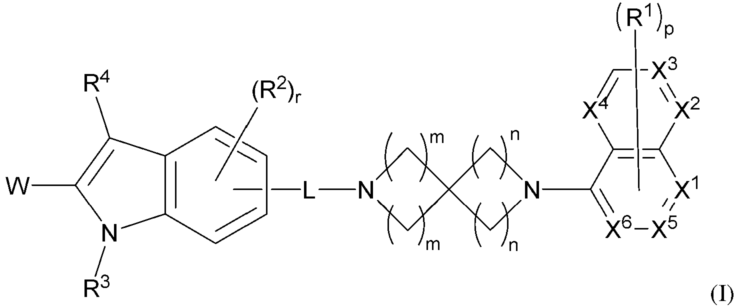
or a pharmaceutically acceptable salt, prodrug, stereoisomer, solvate, or tautomer thereof,
wherein
   X¹, X², X³, X⁴, X⁵, and X⁶ are each independently selected from CH or N;
   and at least one of X¹, X², X³, X⁴, X⁵, and X⁶ is N;
   W is -CN;
   or W and ring B together with the atoms to which they are attached and any intervening atoms, form a 5-10 membered heterocycle;
   each R¹ is independently selected from halogen, C₁-C₆ alkyl, C₁-C₆ alkoxy, C₃-C₁₀ cycloalkyl, C₂-C₆ alkenyl, C₂-C₆ alkynyl, heterocycle, aryl, heteroaryl, OH, NH₂, NHCH₃, and N(CH₃)₂ wherein the alkyl, alkoxy, alkenyl, alkynyl, heterocycle, cycloalkyl, aryl, or heteroaryl is optionally substituted with one or more substituents independently selected from halogen, OH, NR⁹R¹⁰, C₁-C₆ alkyl, C₁-C₆ alkoxy, C₃-C₁₀ cycloalkyl, C₂-C₆ alkenyl, C₂-C₆ alkynyl, heterocycle, aryl, heteroaryl;
   each R² is independently selected from halogen, OH, CN, C₁-C₆ alkyl, C₁-C₆ alkoxy, NR⁹R¹⁰, C₃-C₁₀ cycloalkyl, aryl, heterocyclyl, heteroaryl;
   L is selected from (CR⁵₂)_{q}, (CR⁵₂)_{q}O, (CR⁵₂)_{q}S(O)ₛ and (CR⁵₂)_{q}C(O);
   R³ is selected from hydrogen, deuterium, C₁-C₆ alkyl, C₃-C₁₀ cycloalkyl, heterocyclyl, aryl, heteroaryl and L⁴;
   L⁴ is 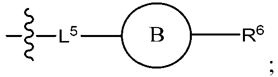 wherein,
      L⁵ is selected from (CR⁵₂)_{q}, (CR⁵₂)_{q}O, (CR⁵₂)_{q}S(O)ₛ and (CR⁵₂)_{q}C(O);
      Ring B is selected from C₃-C₁₄ cycloalkyl, 3- to 10-membered heterocycle, aryl, and heteroaryl, wherein cycloalkyl, heterocycle, aryl, or heteroaryl is optionally substituted with one or more substituents independently selected from halogen, CN, NO₂, oxo, OH, NR⁹R¹⁰, C₁-C₆ alkyl, C₁-C₆ alkoxy, cycloalkyl, heterocycle, aryl, and heteroaryl;
      R⁴ is selected from H, C₁-C₆ alkyl, C₂-C₆ alkenyl, C₂-C₆ alkynyl, C₃-C₁₀ cycloalkyl, C₁-C₆ alkyl-C₁-C₆ alkoxy, C(O)R⁷, heterocyclyl, aryl, or heteroaryl, wherein the alkyl, alkenyl, alkynyl, cycloalkyl, heterocyclyl, aryl, or heteroaryl is optionally substituted with one or more substituents independently selected from halogen, OH, NH₂, C₁-C₆ alkyl, and C₁-C₆ alkoxy;
      each R⁵ is independently selected from H, halogen, CN, OH, C₁-C₆ alkyl, C₂-C₆ alkenyl, C₂-C₆ alkynyl, C₃-C₁₀ cycloalkyl;
      R⁶ is selected from H, C₁-C₆ alkyl, -C(O)R⁷, -NHC(O)R⁷, S(O)ₛR¹¹, -NHS(O)ₛR¹¹, -NHS(O)₂NR⁹R¹⁰, 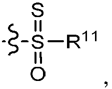 C₃-C₁₀ cycloalkyl, heterocyclyl, aryl, heteroaryl, C₁-C₆ alkanediyl C₃-C₁₀ cycloalkyl, C₁-C₆ alkanediyl heterocyclyl, C₁-C₆ alkanediyl aryl, and C₁-C₆ alkanediyl heteroaryl, wherein alkyl, cycloalkyl, aryl, or heteroaryl is optionally substituted with one or more substituents independently selected from halogen, CN, NO₂, oxo, OH, NH₂, C₁-C₆ alkyl, C₁-C₆ alkoxy, cycloalkyl, heterocycle, aryl, and heteroaryl;
      R⁷ is selected from R⁸, OR⁸, NR⁹R¹⁰,
      R⁸ is selected from H, C₁-C₆ alkyl, C₂-C₆ alkenyl, C₂-C₆ alkynyl, C₃-C₁₀ cycloalkyl, wherein alkyl, alkenyl, alkynyl or cycloalkyl is optionally substituted with one or more substituents independently selected from halogen, OH, CN, NO₂, NR⁹R¹⁰;
      each R⁹ and R¹⁰ is independently H, C₁-C₆ alkyl, C₂-C₆ alkenyl, C₂-C₆ alkynyl, C₃-C₁₀ cycloalkyl, aryl, heteroaryl, or heterocycle, wherein the alkyl, alkenyl, alkynyl, cycloalkyl, aryl, heteroaryl, or heterocycle is optionally substituted with one or more substituents independently selected from halogen, OH, NH₂, C₁-C₆ alkyl, and C₁-C₆ alkoxy;
      or R⁹ and R¹⁰ together with the atoms to which they are attached and any intervening atoms, form a 3-14 membered heterocycle;
      R¹¹ is selected from C₁-C₆ alkyl, C₃-C₁₀ cycloalkyl, C₁-C₆ alkoxy, NR⁹R¹⁰;
      m and n are each an integer independently selected from 1, 2, and 3;
      p is an integer selected from 0, 1, and 2;
      r is an integer selected from 0, 1, 2 and 3;
      q is an integer selected from 0, 1, 2, 3 and 4;
      s is an integer selected from 0, 1, and 2;
         wherein,
      cycloalkyl is a mono or polycyclic saturated carbon rings containing 3-18 carbon atoms;
      aryl is a cyclic, aromatic hydrocarbon groups that have 1 to 3 aromatic rings;
      heterocyclyl is a saturated or partially unsaturated 3-10 membered monocyclic, 7-12 membered bicyclic (fused, bridged, or spiro rings), or 11-14 membered tricyclic ring system (fused, bridged, or spiro rings) having one or more heteroatoms selected from O, N, S, P, Se, or B;
      heteroaryl is a monovalent monocyclic or a polycyclic aromatic radical of 5 to 24 ring atoms, containing one or more ring heteroatoms selected from N, O, S, P, or B, the remaining ring atoms being C.

Another aspect of the invention is directed to pharmaceutical compositions comprising a compound of Formula (I), or a pharmaceutically acceptable salt, hydrate, solvate, prodrug, stereoisomer, or tautomer thereof and a pharmaceutically acceptable carrier. The pharmaceutical acceptable carrier may further include an excipient, diluent, or surfactant.

Another aspect of the invention relates to a method of treating a disease or disorder associated with interaction of menin and MLL. The method comprises administering to a patient in need of a treatment for diseases or disorders associated with interaction of menin and MLL an effective amount of a compound of Formula (I), or a pharmaceutically acceptable salt, hydrate, solvate, prodrug, stereoisomer, tautomer, or pharmaceutical composition thereof.

Another aspect of the invention is directed to a method of inhibiting of interaction of menin and MLL. The method involves administering to a patient in need thereof an effective amount of a compound of Formula (I), or a pharmaceutically acceptable salt, hydrate, solvate, prodrug, stereoisomer, tautomer, or pharmaceutical composition thereof.

Another aspect of the present invention relates to compounds of Formula (I), or pharmaceutically acceptable salts, hydrates, solvates, prodrugs, stereoisomers, tautomers, or pharmaceutical compositions thereof, for use in the manufacture of a medicament for inhibiting interaction of menin and MLL.

Another aspect of the present invention relates to the use of compounds of Formula (I), or pharmaceutically acceptable salts, hydrates, solvates, prodrugs, stereoisomers, tautomers, or pharmaceutical compositions thereof, in the treatment of a disease associated with inhibiting of interaction of menin and MLL.

Another aspect of the present invention relates to compounds of Formula (I), or pharmaceutically acceptable salts, hydrates, solvates, prodrugs, stereoisomers, tautomers, or pharmaceutical compositions thereof, for use in the manufacture of a medicament for treating or preventing a disease or disorder disclosed herein.

Another aspect of the invention is directed to a method of treating or preventing a disease or disorder disclosed herein in a subject in need thereof. The method involves administering to a patient in need of the treatment an effective amount of a compound of Formula (I), or a pharmaceutically acceptable salt, hydrate, solvate, prodrug, stereoisomer, tautomer, or pharmaceutical composition thereof.

Another aspect of the present invention relates to the use of compounds of Formula (I), or pharmaceutically acceptable salts, hydrates, solvates, prodrugs, stereoisomers, tautomers, or pharmaceutical compositions thereof, in the treatment of a disease or disorder disclosed herein.

The present invention further provides methods of treating a disease or disorder associated with interaction of menin and MLL, comprising administering to a patient suffering from at least one of said diseases or disorders a compound of Formula (I), or a pharmaceutically acceptable salt, hydrate, solvate, prodrug, stereoisomer, tautomer, or pharmaceutical composition thereof.

The present invention provides inhibitors of interaction of menin and MLL that are therapeutic agents in the treatment of diseases and disorders.

The present invention further provides compounds and compositions with an improved efficacy and safety profile relative to known inhibitors of menin and MLL interaction. The present disclosure also provides agents with novel mechanisms of action toward interaction of menin and MLL in the treatment of various types of diseases.

The present invention further provides methods of treating a disease or disorder associated with interaction of menin and MLL, comprising administering to a patient suffering from at least one of said diseases or disorders a compound of Formula (I), or a pharmaceutically acceptable salt, hydrate, solvate, prodrug, stereoisomer, tautomer, or pharmaceutical composition thereof.

The present invention provides inhibitors of interaction of menin and MLL that are therapeutic agents in the treatment of diseases and disorders.

The present invention further provides methods of treating a disease, disorder, or condition selected from cancer, acute myeloid (AML) and acute lymphoblastic (ALL) leukemias, or myelodysplastic syndrome (MDS), comprising administering to a patient suffering from at least one of said diseases or disorders a compound of Formula (I), or a pharmaceutically acceptable salt, hydrate, solvate, prodrug, stereoisomer, tautomer, or pharmaceutical composition thereof.

In some aspects, the present disclosure provides a compound obtainable by, or obtained by, a method for preparing compounds described herein (*e.g.*, a method comprising one or more steps described in General Procedure A or B).

In some aspects, the present disclosure provides an intermediate as described herein, being suitable for use in a method for preparing a compound as described herein (*e.g.*, the intermediate is selected from the intermediates described in Preparative part - **P1**-**P175**).

In some aspects, the present disclosure provides a method of preparing compounds of the present disclosure.

In some aspects, the present disclosure provides a method of preparing compounds of the present disclosure, comprising one or more steps described herein.

Unless otherwise defined, all technical and scientific terms used herein have the same meaning as commonly understood by one of ordinary skill in the art to which this disclosure belongs. In the specification, the singular forms also include the plural unless the context clearly dictates otherwise. Although methods and materials similar or equivalent to those described herein can be used in the practice or testing of the present disclosure, suitable methods and materials are described below. All publications, patent applications, patents and other references mentioned herein are incorporated by reference. The references cited herein are not admitted to be prior art to the claimed invention. In the case of conflict, the present specification, including definitions, will control. In addition, the materials, methods and examples are illustrative only and are not intended to be limiting. In the case of conflict between the chemical structures and names of the compounds disclosed herein, the chemical structures will control.

Other features and advantages of the disclosure will be apparent from the following detailed description and claims

### DETAILED DESCRIPTION

The present disclosure provides methods of treating, preventing, or ameliorating a disease or disorder in which associated with the inhibition of the interaction of menin and MLL1 by administering to a subject in need thereof a therapeutically effective amount of a compound as disclosed herein.

The details of the disclosure are set forth in the accompanying description below. Although methods and materials similar or equivalent to those described herein can be used in the practice or testing of the present disclosure, illustrative methods and materials are now described. Other features, objects, and advantages of the disclosure will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this disclosure belongs. All patents and publications cited in this specification are incorporated herein by reference in their entireties.

### Definitions

The articles "a" and "an" are used in this disclosure to refer to one or more than one (*i.e.*, to at least one) of the grammatical object of the article. By way of example, "an element" means one element or more than one element.

The term "and/or" is used in this disclosure to mean either "and" or "or" unless indicated otherwise.

The term "optionally substituted" is understood to mean that a given chemical moiety (*e.g.*, an alkyl group) can (but is not required to) be bonded other substituents (*e.g.*, heteroatoms). For instance, an alkyl group that is optionally substituted can be a fully saturated alkyl chain (*i.e.*, a pure hydrocarbon). Alternatively, the same optionally substituted alkyl group can have one or more substituents different from hydrogen. For instance, it can, at any point along the chain be bounded to a halogen atom, a hydroxyl group, or any other substituent described herein. Thus, the term "optionally substituted" means that a given chemical moiety has the potential to contain other functional groups but does not necessarily have any further functional groups. Suitable substituents used in the optional substitution of the described groups include, without limitation, halogen, oxo, - OH, -CN, -COOH, -CH₂CN, -O-(C₁-C₆) alkyl, (C₁-C₆) alkyl, (C₁-C₆) alkoxy, (C₁-C₆) haloalkyl, (C₁-C₆) haloalkoxy, -O-(C₂-C₆) alkenyl, -O-(C₂-C₆) alkynyl, (C₂-C₆) alkenyl, (C₂-C₆) alkynyl, -OH, -OP(O)(OH)₂, -OC(O)(C₁-C₆) alkyl, -C(O)(C₁-C₆) alkyl, - OC(O)O(C₁-C₆) alkyl, -NH₂, -NH((C₁-C₆) alkyl), -N((C₁-C₆) alkyl)₂, -NHC(O)(C₁-C₆) alkyl, -C(O)NH(C₁-C₆) alkyl, -S(O)₂(C₁-C₆) alkyl, -S(O)NH(C₁-C₆)alkyl, and - S(O)N((C₁-C₆)alkyl)₂. The substituents can themselves be optionally substituted. "Optionally substituted" as used herein also refers to substituted or unsubstituted whose meaning is described below.

As used herein, the term "substituted" means that the specified group or moiety bears one or more suitable substituents wherein the substituents may connect to the specified group or moiety at one or more positions. For example, an aryl substituted with a cycloalkyl may indicate that the cycloalkyl connects to one atom of the aryl with a bond or by fusing with the aryl and sharing two or more common atoms.

As used herein, the term "unsubstituted" means that the specified group bears no substituents.

Unless otherwise specifically defined, the term "aryl" refers to cyclic, aromatic hydrocarbon groups that have 1 to 3 aromatic rings, including monocyclic or bicyclic groups such as phenyl, biphenyl or naphthyl. Where containing two aromatic rings (bicyclic, etc.), the aromatic rings of the aryl group may be joined at a single point (*e.g.*, biphenyl), or fused (*e.g.*, naphthyl). The aryl group may be optionally substituted by one or more substituents, *e.g.*, 1 to 5 substituents, at any point of attachment. Exemplary substituents include, but are not limited to, -H, -halogen, -O-(C₁-C₆)alkyl, (C₁-C₆)alkyl, - O-(C₂-C₆)alkenyl, -O-(C₂-C₆) alkynyl, (C₂-C₆)alkenyl, (C₂-C₆)alkynyl, -OH, - OP(O)(OH)₂, -OC(O)(C₁-C₆)alkyl, -C(O)(C₁-C₆) alkyl, -OC(O)O(C₁-C₆)alkyl, -NH₂, - NH((C₁-C₆)alkyl), -N((C₁-C₆)alkyl)₂, -S(O)₂-(C₁-C₆) alkyl, -S(O)NH(C₁-C₆)alkyl, and - S(O)N((C₁-C₆)alkyl)₂. The substituents can themselves be optionally substituted. Furthermore, when containing two fused rings the aryl groups herein defined may have one or more saturated or partially unsaturated ring fused with a fully unsaturated aromatic ring. Exemplary ring systems of these aryl groups include, but are not limited to, phenyl, biphenyl, naphthyl, anthracenyl, phenalenyl, phenanthrenyl, indanyl, indenyl, tetrahydronaphthalenyl, tetrahydrobenzoannulenyl, and the like.

Unless otherwise specifically defined, "heteroaryl" means a monovalent monocyclic or a polycyclic aromatic radical of 5 to 24 ring atoms, containing one or more ring heteroatoms selected from N, O, S, P, or B, the remaining ring atoms being C. A polycyclic aromatic radical includes two or more fused rings and may further include two or more spiro-fused rings, e.g., bicyclic, tricyclic, tetracyclic, and the like. Unless otherwise specifically defined, "fused" means two rings sharing two ring atoms. Unless otherwise specifically defined, "spiro-fused" means two rings sharing one ring atom. Heteroaryl as herein defined also means a bicyclic heteroaromatic group wherein the heteroatom is selected from N, O, S, P, or B. Heteroaryl as herein defined also means a tricyclic heteroaromatic group containing one or more ring heteroatoms selected from N, O, S, P, or B. Heteroaryl as herein defined also means a tetracyclic heteroaromatic group containing one or more ring heteroatoms selected from N, O, S, P, or B. The aromatic radical is optionally substituted independently with one or more substituents described herein. Examples include, but are not limited to, furyl, thienyl, pyrrolyl, pyridyl, pyrazolyl, pyrimidinyl, imidazolyl, isoxazolyl, oxazolyl, oxadiazolyl, pyrazinyl, indolyl, thiophen-2-yl, quinolyl, benzopyranyl, isothiazolyl, thiazolyl, thiadiazole, indazole, benzimidazolyl, thieno[3,2-b]thiophene, triazolyl, triazinyl, imidazo[1,2-b]pyrazolyl, furo[2,3-c]pyridinyl, imidazo[1,2-a]pyridinyl, indazolyl, pyrrolo[2,3-c]pyridinyl, pyrrolo[3,2-c]pyridinyl, pyrazolo[3,4-c]pyridinyl, thieno[3,2-c]pyridinyl, thieno[2,3-c]pyridinyl, thieno[2,3-b]pyridinyl, benzothiazolyl, indolyl, indolinyl, indolinonyl, dihydrobenzothiophenyl, dihydrobenzofuranyl, benzofuran, chromanyl, thiochromanyl, tetrahydroquinolinyl, dihydrobenzothiazine, quinolinyl, isoquinolinyl, 1,6-naphthyridinyl, benzo[de]isoquinolinyl, pyrido[4,3-b][1,6]naphthyridinyl, thieno[2,3-b]pyrazinyl, quinazolinyl, tetrazolo[1,5-a]pyridinyl, [1,2,4]triazolo[4,3-a]pyridinyl, isoindolyl, pyrrolo[2,3-b]pyridinyl, pyrrolo[3,4-b]pyridinyl, pyrrolo[3,2-b]pyridinyl, imidazo[5,4-b]pyridinyl, pyrrolo[1,2-a]pyrimidinyl, tetrahydro pyrrolo[1,2-a]pyrimidinyl, 3,4-dihydro-2H-1-pyrrolo[2,1-b]pyrimidine, dibenzo[b,d] thiophene, pyridin-2-one, furo[3,2-c]pyridinyl, furo[2,3-c]pyridinyl, 1H-pyrido[3,4-b][1,4] thiazinyl, benzooxazolyl, benzoisoxazolyl, furo[2,3-b]pyridinyl, benzothiophenyl, 1,5-naphthyridinyl, furo[3,2-b]pyridine, [1,2,4]triazolo[1,5-a]pyridinyl, benzo [1,2,3]triazolyl, imidazo[1,2-a]pyrimidinyl, [1,2,4]triazolo[4,3-b]pyridazinyl, benzo[c][1,2,5]thiadiazolyl, benzo[c][1,2,5]oxadiazole, 1,3-dihydro-2H-benzo[d]imidazol-2-one, 3,4-dihydro-2H-pyrazolo [1,5-b][1,2]oxazinyl, 4,5,6,7-tetrahydropyrazolo[1,5-a]pyridinyl, thiazolo[5,4-d]thiazolyl, imidazo[2,1-b][1,3,4]thiadiazolyl, thieno[2,3-b]pyrrolyl, 3H-indolyl, and derivatives thereof. Furthermore, when containing two or more fused rings, the heteroaryl groups defined herein may have one or more saturated or partially unsaturated ring fused with one or more fully unsaturated aromatic ring. In heteroaryl ring systems containing more than two fused rings, a saturated or partially unsaturated ring may further be fused with a saturated or partially unsaturated ring described herein. Furthermore, when containing three or more fused rings, the heteroaryl groups defined herein may have one or more saturated or partially unsaturated ring spiro-fused. Any saturated or partially unsaturated ring described herein is optionally substituted with one or more oxo. Exemplary ring systems of these heteroaryl groups include, for example, indolinyl, indolinonyl, dihydrobenzothiophenyl, dihydrobenzofuran, chromanyl, thiochromanyl, tetrahydroquinolinyl, dihydrobenzothiazine, 3,4-dihydro-1H--isoquinolinyl, 2,3-dihydrobenzofuranyl, benzofuranonyl, indolinyl, oxindolyl, indolyl, 1,6-dihydro-7H-pyrazolo[3,4-c]pyridin-7-onyl, 7,8-dihydro-6H-pyrido[3,2-b]pyrrolizinyl, 8H-pyrido[3,2-b]pyrrolizinyl, 1,5,6,7-tetrahydrocyclopenta[b]pyrazolo[4,3-e]pyridinyl, 7,8-dihydro-6H-pyrido[3,2-b]pyrrolizine, pyrazolo[1,5-a]pyrimidin-7(4H)-only, 3,4-dihydropyrazino[1,2-a]indol-1(2H)-onyl, benzo[*c*][1,2]oxaborol-1(*3H*)-olyl, 6,6a,7,8-tetrahydro-9*H-*pyrido[2,3-*b*]puyrrolo[1,2-*d*][1,4]oxazin-9-onyl, or 6a',7'-dihydro-6'H,9'H-spiro[cyclopropane-1,8'-pyrido[2,3-*b*]pyrrolo[1,2-*d*][1,4]oxazin]-9'-onyl.

Halogen or "halo" refers to fluorine, chlorine, bromine, or iodine.

Alkyl refers to a straight or branched chain saturated hydrocarbon containing 1-12 carbon atoms. Examples of a (C₁-C₆) alkyl group include, but are not limited to, methyl, ethyl, propyl, butyl, pentyl, hexyl, *iso*-propyl, *iso*-butyl, *sec*-butyl, *tert*-butyl, *iso*-pentyl, *neo*-pentyl, and *iso*-hexyl.

"Alkoxy" refers to a straight or branched chain saturated hydrocarbon containing 1-12 carbon atoms containing a terminal "O" in the chain, *i.e.,* -O(alkyl). Examples of alkoxy groups include without limitation, methoxy, ethoxy, propoxy, butoxy, t-butoxy, or pentoxy groups.

"Alkenyl" refers to a straight or branched chain unsaturated hydrocarbon containing 2-12 carbon atoms. The "alkenyl" group contains at least one double bond in the chain. The double bond of an alkenyl group can be unconjugated or conjugated to another unsaturated group. Examples of alkenyl groups include ethenyl, propenyl, *n-*butenyl, *iso*-butenyl, pentenyl, or hexenyl. An alkenyl group can be unsubstituted or substituted. Alkenyl, as herein defined, may be straight or branched.

"Alkynyl" refers to a straight or branched chain unsaturated hydrocarbon containing 2-12 carbon atoms. The "alkynyl" group contains at least one triple bond in the chain. Examples of alkenyl groups include ethynyl, propargyl, *n-*butynyl, *iso*-butynyl, pentynyl, or hexynyl. An alkynyl group can be unsubstituted or substituted.

The term "alkylene" or "alkylenyl" refers to a divalent alkyl radical. Any of the above mentioned monovalent alkyl groups may be an alkylene by abstraction of a second hydrogen atom from the alkyl. As herein defined, alkylene may also be a C₁-C₆ alkylene. An alkylene may further be a C₁-C₄ alkylene. Typical alkylene groups include, but are not limited to, -CH₂-, -CH(CH₃)-, -C(CH₃)₂-, -CH₂CH₂-, -CH₂CH(CH₃)-, -CH₂C(CH₃)₂-, - CH₂CH₂CH₂-, -CH₂CH₂CH₂CH₂-, and the like.

"Cycloalkyl" means mono or polycyclic saturated or partially unsaturated carbon rings containing 3-18 carbon atoms. Polycyclic cycloalkyl may be fused bicyclic cycloalkyl, bridged bicyclic cycloalkyl, or spiro-fused bicyclic cycloalkyl. A polycyclic cycloalkyl comprises at least one non-aromatic ring. Examples of cycloalkyl groups include, without limitations, cyclopropyl, cyclobutyl, cyclopentyl, cyclohexyl, cycloheptanyl, cyclooctanyl, norbornyl, norborenyl, 1,2,3,4-tetrahydronaphthyl, 2,3-dihydro-1H-indenyl, spiro[3.5]nonyl, spiro [5.5]undecyl, bicyclo[1.1.1]pentanyl, bicyclo[2.2.2]octanyl, or bicyclo[2.2.2]octenyl.

"Heterocyclyl", "heterocycle" or "heterocycloalkyl" mono or polycyclic rings containing 3-24 atoms which include carbon and one or more heteroatoms selected from N, O, S, P, or B and wherein the rings are not aromatic. The heterocycloalkyl ring structure may be substituted by one or more substituents. A polycyclic heterocycloalkyl comprises at least one non-aromatic ring. Polycyclic heterocycles may be bridged, fused, or spiro-fused. The substituents can themselves be optionally substituted. Examples of heterocyclyl rings include, but are not limited to, oxetanyl, azetidinyl, tetrahydrofuranyl, tetrahydropyranyl, pyrrolidinyl, oxazolinyl, oxazolidinyl, thiazolinyl, thiazolidinyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, oxazolidinonyl, homotropanyl, 2-oxa-5-azabicyclo[2.2.2]octane, and 2,6-diazaspiro[3.3]heptanyl.

The term "aromatic" means a planar ring having 4*n* + 2 electrons in a conjugated system. As used herein, "conjugated system" means a system of connected p-orbitals with delocalized electrons, and the system may include lone electron pairs.

The term "haloalkyl" as used herein refers to an alkyl group, as defined herein, which is substituted one or more halogen. Examples of haloalkyl groups include, but are not limited to, trifluoromethyl, difluoromethyl, pentafluoroethyl, trichloromethyl, etc.

The term "haloalkoxy" as used herein refers to an alkoxy group, as defined herein, which is substituted with one or more halogen. Examples of haloalkyl groups include, but are not limited to, trifluoromethoxy, difluoromethoxy, pentafluoroethoxy, trichloromethoxy, etc.

The term "cyano" as used herein means a substituent having a carbon atom joined to a nitrogen atom by a triple bond, *i.e.*, C≡N.

"Spirocycloalkyl" or "spirocyclyl" means carbogenic bicyclic ring systems with both rings connected through a single atom. The ring can be different in size and nature, or identical in size and nature. Examples include spiropentane, spriohexane, spiroheptane, spirooctane, spirononane, or spirodecane. One or both of the rings in a spirocycle can be fused to another ring carbocyclic, heterocyclic, aromatic, or heteroaromatic ring. One or more of the carbon atoms in the spirocycle can be substituted with a heteroatom (*e.g.*, O, N, S, or P). A (C₃-C₁₂) spirocycloalkyl is a spirocycle containing between 3 and 12 carbon atoms. One or more of the carbon atoms can be substituted with a heteroatom.

The term "spiroheterocycloalkyl", "spiroheterocycle", or "spiroheterocyclyl" is understood to mean a spirocycle wherein at least one of the rings is a heterocycle (*e.g.*, at least one of the rings is furanyl, morpholinyl, or piperidinyl).

The term "solvate" refers to a complex of variable stoichiometry formed by a solute and solvent. Such solvents for the purpose of the disclosure may not interfere with the biological activity of the solute. Examples of suitable solvents include, but are not limited to, water, MeOH, EtOH, and AcOH. Solvates wherein water is the solvent molecule are typically referred to as hydrates. Hydrates include compositions containing stoichiometric amounts of water, as well as compositions containing variable amounts of water.

As used herein, the term "alkyl-aryl" (and variations thereof, e.g. C₁-C₆ alkyl-aryl) refers to a chemical moiety comprising an alkyl group covalently attached to an aryl group, wherein the linkage to the rest of the molecule is on the first group recited, i.e., the alkyl group. Similarly, alkyl-alkoxy refers to a chemical moiety comprising an alkyl group covalently attached to an alkoxy group wherein the linkage to the rest of the molecule is on the alkyl group. This nomenclature may also be used for, e.g. alkenyl-aryl, alkenyl-heteroaryl, alkynyl-aryl, alkynyl-heteroaryl. As non-limiting examples, C₁ alkyl-phenyl refers to 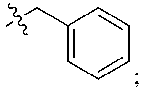 C₂ alkenyl-furanyl refers to 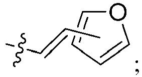 C₁ alkyl-C₂ alkoxy refers to 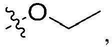 and so on.

The term "isomer" refers to compounds that have the same composition and molecular weight but differ in physical and/or chemical properties. The structural difference may be in constitution (geometric isomers) or in the ability to rotate the plane of polarized light (stereoisomers). With regard to stereoisomers, the compounds of Formula (I) may have one or more asymmetric carbon atom and may occur as racemates, racemic mixtures and as individual enantiomers or diastereomers.

The present disclosure also contemplates isotopically-labelled compounds of Formula I (*e.g.*, those labeled with ²H and ¹⁴C). Deuterated (*i.e.*, ²H or D) and carbon-14 (*i.e.*, ¹⁴C) isotopes are particularly preferred for their ease of preparation and detectability. Further, substitution with heavier isotopes such as deuterium may afford certain therapeutic advantages resulting from greater metabolic stability (*e.g.*, increased *in vivo* half-life or reduced dosage requirements) and hence may be preferred in some circumstances. Isotopically labelled compounds of Formula I can generally be prepared by following procedures analogous to those disclosed in the Schemes and/or in the Examples herein below, by substituting an appropriate isotopically labelled reagent for a non-isotopically labelled reagent.

The disclosure also includes pharmaceutical compositions comprising a therapeutically effective amount of a disclosed compound and a pharmaceutically acceptable carrier. Representative "pharmaceutically acceptable salts" include, *e.g.*, watersoluble and water-insoluble salts, such as the acetate, amsonate (4,4-diaminostilbene-2,2-disulfonate), benzenesulfonate, benzoate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, magnesium, malate, maleate, mandelate, mesylate, methylbromide, methylnitrate, methylsulfate, mucate, napsylate, nitrate, *N-*methylglucamine ammonium salt, 3-hydroxy-2-naphthoate, oleate, oxalate, palmitate, pamoate, pantothenate, phosphate/diphosphate, picrate, polygalacturonate, propionate, p-toluenesulfonate, salicylate, stearate, subacetate, succinate, sulfate, sulfosalicylate, tannate, tartrate, teoclate, tosylate, triethiodide, and valerate salts.

A "patient" or "subject" is a mammal, *e.g.*, a human, mouse, rat, guinea pig, dog, cat, horse, cow, pig, or non-human primate, such as a monkey, chimpanzee, baboon, or rhesus.

An "effective amount" when used in connection with a compound is an amount effective for treating or preventing a disease in a subject as described herein.

The term "carrier", as used in this disclosure, encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body of a subject.

The term "treating" with regard to a subject, refers to improving at least one symptom of the subject's disorder. Treating includes curing, improving, or at least partially ameliorating the disorder.

The term "disorder" is used in this disclosure to mean, and is used interchangeably with, the terms disease, condition, or illness, unless otherwise indicated.

The term "administer", "administering", or "administration" as used in this disclosure refers to either directly administering a disclosed compound or pharmaceutically acceptable salt of the disclosed compound or a composition to a subject, or administering a prodrug derivative or analog of the compound or pharmaceutically acceptable salt of the compound or composition to the subject, which can form an equivalent amount of active compound within the subject's body.

The term "prodrug," as used in this disclosure, means a compound which is convertible in vivo by metabolic means (*e.g.*, by hydrolysis) to a disclosed compound

The term "salt' refers to pharmaceutically acceptable salts

The term "pharmaceutically acceptable salt" also refers to a salt of the compositions of the present disclosure having an acidic functional group, such as a carboxylic acid functional group, and a base.

"Menin/MLL interaction inhibitor" as used herein refer to compounds of Formula I and/or compositions comprising a compound of Formula I which inhibits the interaction of menin and MLL.

The amount of compound of composition described herein needed for achieving a therapeutic effect may be determined empirically in accordance with conventional procedures for the particular purpose. Generally, for administering therapeutic agents (*e.g.* compounds or compositions of Formula I (and/or additional agents) described herein) for therapeutic purposes, the therapeutic agents are given at a pharmacologically effective dose. A "pharmacologically effective amount," "pharmacologically effective dose," "therapeutically effective amount," or "effective amount" refers to an amount sufficient to produce the desired physiological effect or amount capable of achieving the desired result, particularly for treating the disorder or disease. An effective amount as used herein would include an amount sufficient to, for example, delay the development of a symptom of the disorder or disease, alter the course of a symptom of the disorder or disease (*e.g.*, slow the progression of a symptom of the disease), reduce or eliminate one or more symptoms or manifestations of the disorder or disease, and reverse a symptom of a disorder or disease. For example, administration of therapeutic agents to a subject suffering from cancer provides a therapeutic benefit not only when the underlying condition is eradicated or ameliorated, but also when the subject reports a decrease in the severity or duration of the symptoms associated with the disease, *e.g.*, a decrease in tumor burden, a decrease in circulating tumor cells, an increase in progression free survival. Therapeutic benefit also includes halting or slowing the progression of the underlying disease or disorder, regardless of whether improvement is realized.

### Compounds of the Present Disclosure

In one aspect, the present disclosure provides compounds of Formula (I) and salts, stereoisomers, solvates, prodrugs, isotopic derivatives, and tautomers thereof: 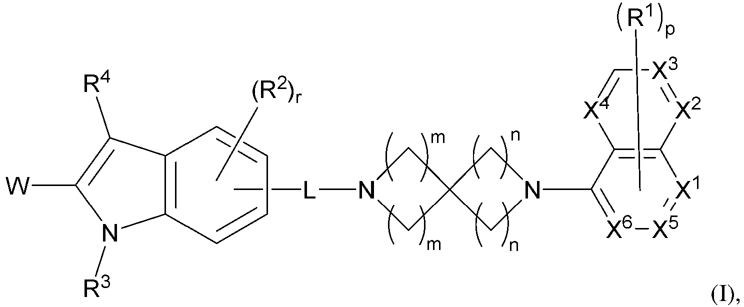 wherein R¹, R², R³, R⁴, X¹, X^{1'}, X², X³, X⁴, X⁵, X⁶, Y, L, m, n, p, and r are as described herein.

It is understood that, for a compound of Formula (I), R¹, R², R³, R⁴, R⁵, R⁶, R⁷, R⁸, R⁹, R¹⁰, R¹¹, L, L⁴, L⁵, B, W, X¹, X², X³, X⁴, X⁵, X⁶, m, n, p, r, q, and s can each be, where applicable, selected from the groups described herein, and any group described herein for any of R¹, R², R³, R⁴, R⁵, R⁶, R⁷, R⁸, R⁹, R¹⁰, R¹¹, L, L⁴, L⁵, B, W, X¹, X², X³, X⁴, X⁵, X⁶, m, n, p, r, q, and s can be combined, where applicable, with any group described herein for one or more of the remainder of R¹, R², R³, R⁴, R⁵, R⁶, R7, R8, R9, R10, R11, L, L⁴, L⁵, B, W, X¹, X², X³, X⁴, X⁵, X⁶, m, n, p, r, q, and s.

In some embodiments,
X¹, X², X³, X⁴, X⁵, and X⁶ are each independently selected from CH or N;
and at least one of X¹, X², X³, X⁴, X⁵, and X⁶ is N;
W is -CN;
or W and ring B together with the atoms to which they are attached and any intervening atoms, form a 5-10 membered heterocycle;
each R¹ is independently selected from halogen, C₁-C₆ alkyl, C₁-C₆ alkoxy, C₃-C₁₀ cycloalkyl, C₂-C₆ alkenyl, C₂-C₆ alkynyl, heterocycle, aryl, heteroaryl, C₁-C₆ alkyl-aryl, C₁-C₆ alkyl-heteroaryl, C₂-C₆ alkenyl-aryl, C₂-C₆ alkenyl-heteroaryl, C₂-C₆ alkynyl-aryl, C₂-C₆ alkynyl-heteroaryl, OH, NH₂, NHCH₃, and N(CH₃)₂ wherein the alkyl, alkoxy, alkenyl, alkynyl, heterocycle, cycloalkyl, aryl, or heteroaryl is optionally substituted with one or more substituents independently selected from halogen, OH, NR⁹R¹⁰, C₁-C₆ alkyl, C₁-C₆ alkoxy, C₃-C₁₀ cycloalkyl, C₂-C₆ alkenyl, C₂-C₆ alkynyl, heterocycle, aryl, heteroaryl;
each R² is independently selected from halogen, OH, CN, C₁-C₆ alkyl, C₁-C₆ alkoxy, NR⁹R¹⁰, C₃-C₁₀ cycloalkyl, aryl, heterocyclyl, heteroaryl;
L is selected from (CR⁵₂)_{q}, (CR⁵₂)_{q}O, (CR⁵₂)_{q}S(O)ₛ and (CR⁵₂)_{q}C(O);
R³ is selected from hydrogen, deuterium, C₁-C₆ alkyl, C₃-C₁₀ cycloalkyl, heterocyclyl, aryl, heteroaryl and L⁴;
L⁴ is 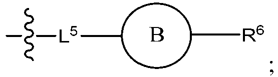 wherein,
   L⁵ is selected from (CR⁵₂)_{q}, (CR⁵₂)_{q}O, (CR⁵₂)_{q}S(O)ₛ and (CR⁵₂)_{q}C(O);
   Ring B is selected from C₃-C₁₄ cycloalkyl, 3- to 10-membered heterocycle, aryl, and heteroaryl, wherein cycloalkyl, heterocycle, aryl, or heteroaryl is optionally substituted with one or more substituents independently selected from halogen, CN, NO₂, oxo, OH, NR⁹R¹⁰, C₁-C₆ alkyl, C₁-C₆ alkoxy, cycloalkyl, heterocycle, aryl, and heteroaryl;
   R⁴ is selected from H, C₁-C₆ alkyl, C₂-C₆ alkenyl, C₂-C₆ alkynyl, C₃-C₁₀ cycloalkyl, C₁-C₆ alkyl-C₁-C₆ alkoxy, C(O)R⁷, heterocyclyl, aryl, or heteroaryl, wherein the alkyl, alkenyl, alkynyl, cycloalkyl, heterocyclyl, aryl, or heteroaryl is optionally substituted with one or more substituents independently selected from halogen, OH, NH₂, C₁-C₆ alkyl, and C₁-C₆ alkoxy;
   each R⁵ is independently selected from H, halogen, CN, OH, C₁-C₆ alkyl, C₂-C₆ alkenyl, C₂-C₆ alkynyl, C₃-C₁₀ cycloalkyl;
   R⁶ is selected from H, C₁-C₆ alkyl, -C(O)R⁷, -NHC(O)R⁷, S(O)ₛR¹¹, -NHS(O)ₛR¹¹, -NHS(O)₂NR⁹R¹⁰, 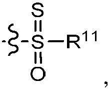 C₃-C₁₀ cycloalkyl, heterocyclyl, aryl, heteroaryl, C₁-C₆ alkanediyl C₃-C₁₀ cycloalkyl, C₁-C₆ alkanediyl heterocyclyl, C₁-C₆ alkanediyl aryl, and C₁-C₆ alkanediyl heteroaryl, wherein alkyl, cycloalkyl, aryl, or heteroaryl is optionally substituted with one or more substituents independently selected from halogen, CN, NO₂, oxo, OH, NH₂, C₁-C₆ alkyl, C₁-C₆ alkoxy, cycloalkyl, heterocycle, aryl, and heteroaryl;
   R⁷ is selected from R⁸, OR⁸, NR⁹R¹⁰,
   R⁸ is selected from H, C₁-C₆ alkyl, C₂-C₆ alkenyl, C₂-C₆ alkynyl, C₃-C₁₀ cycloalkyl, wherein alkyl, alkenyl, alkynyl or cycloalkyl is optionally substituted with one or more substituents independently selected from halogen, OH, CN, NO₂, NR⁹R¹⁰;
   each R⁹ and R¹⁰ is independently H, C₁-C₆ alkyl, C₂-C₆ alkenyl, C₂-C₆ alkynyl, C₃-C₁₀ cycloalkyl, aryl, heteroaryl, or heterocycle, wherein the alkyl, alkenyl, alkynyl, cycloalkyl, aryl, heteroaryl, or heterocycle is optionally substituted with one or more substituents independently selected from halogen, OH, NH₂, C₁-C₆ alkyl, and C₁-C₆ alkoxy;
   or R⁹ and R¹⁰ together with the atoms to which they are attached and any intervening atoms, form a 3-14 membered heterocycle;
   R¹¹ is selected from C₁-C₆ alkyl, C₃-C₁₀ cycloalkyl, C₁-C₆ alkoxy, NR⁹R¹⁰;
   m and n are each an integer independently selected from 1, 2, and 3;
   p is an integer selected from 0, 1, and 2;
   r is an integer selected from 0, 1, 2 and 3;
   q is an integer selected from 0, 1, 2, 3 and 4;
   s is an integer selected from 0, 1, and 2.

In some embodiments, each from X¹, X², X³, X⁴, X⁵, and X⁶ is independently selected from CH or N; and at least one of X¹, X², X³, X⁴, X⁵, and X⁶ is N.

In some embodiments, each from X¹, X², X³, X⁴, X⁵, and X⁶ is independently selected from CH or N; and at least two of X¹, X², X³, X⁴, X⁵, and X⁶ are N.

In some embodiments, each from X¹, X², X³, X⁴, X⁵, and X⁶ is independently selected from CH or N; and at least three of X¹, X², X³, X⁴, X⁵, and X⁶ are N.

In some embodiments, each from X¹, X², X³, X⁴, X⁵, and X⁶ is independently selected from CH or N; and at least four from X¹, X², X³, X⁴, X⁵, and X⁶ are N.

In some embodiments, X¹ is CH or N.

In some embodiments, X¹ is CH, wherein H is optionally replaced with R¹.

In some embodiments, X¹ is CH.

In some embodiments, X¹ is CR¹.

In some embodiments, X¹ is N.

In some embodiments, X² is CH or N.

In some embodiments, X² is CH, wherein H is optionally replaced with R¹.

In some embodiments, X² is CH.

In some embodiments, X² is CR¹.

In some embodiments, X² is N.

In some embodiments, X³ is CH or N.

In some embodiments, X³ is CH, wherein H is optionally replaced with R¹.

In some embodiments, X³ is CH.

In some embodiments, X³ is CR¹.

In some embodiments, X³ is N.

In some embodiments, X⁴ is CH or N.

In some embodiments, X⁴ is CH, wherein H is optionally replaced with R¹.

In some embodiments, X⁴ is CH.

In some embodiments, X⁴ is CR¹.

In some embodiments, X⁴ is N.

In some embodiments, X⁵ is CH or N.

In some embodiments, X⁵ is CH, wherein H is optionally replaced with R¹.

In some embodiments, X⁵ is CR¹.

In some embodiments, X⁵ is N.

In some embodiments, X⁶ is CH or N.

In some embodiments, X⁶ is CH, wherein H is optionally replaced with R¹.

In some embodiments, X⁶ is CH.

In some embodiments, X⁶ is CR¹.

In some embodiments, X⁶ is N.

In some embodiments, each from X¹ and X⁶ is N.

In some embodiments, each from X¹ and X⁶ is N and each from X², X³, X⁴ and X⁵ is CH, wherein each H is optionally replaced with R¹.

In some embodiments, each from X¹ and X⁶ is N and each from X², X³, X⁴ and X⁵ is CH.

In some embodiments, each from X¹ and X⁶ is N; each from X², X⁴ and X⁵ is CH; and X³ is CR¹.

In some embodiments, each from X¹ and X⁶ is N; each from X², X⁴ and X⁵ is CH, and X³ is CF.

In some embodiments, each from X¹ and X⁶ is N; each from X², X³ and X⁵ is CH, and X⁴ is CR¹.

In some embodiments, each from X¹ and X⁶ is N; each from X², X³ and X⁵ is CH, and X⁴ is CF.

In some embodiments, each from X⁵ and X⁶ is N.

In some embodiments, each from X⁵ and X⁶ is N and each from X¹, X², X³ and X⁴ is CH, wherein each H is optionally replaced by R¹.

In some embodiments, each from X⁵ and X⁶ is N and each of X¹, X², X³ and X⁴ is CH.

In some embodiments, each from X¹ and X⁵ is N.

In some embodiments, each from X¹ and X⁵ is N and each from X², X³, X⁴ and X⁶ is CH, wherein each H is optionally replaced by R¹.

In some embodiments, each from X¹ and X⁵ is N and each of X², X³, X⁴ and X⁶ is CH.

In some embodiments, each from X¹, X⁴ and X⁶ is N.

In some embodiments, each from X¹, X⁴ and X⁶ is N and each from X², X³ and X⁵ is CH, wherein each H is optionally replaced by R¹.

In some embodiments, each from X¹, X⁴ and X⁶ is N and each of X², X³ and X⁵ is CH.

In some embodiments, each from X¹, X³ and X⁶ is N.

In some embodiments, each from X¹, X³ and X⁶ is N and each from X², X⁴ and X⁵ is CH, wherein each H is optionally replaced by R¹.

In some embodiments, each from X¹, X³ and X⁶ is N and each of X², X⁴ and X⁵ is CH.

In some embodiments, each from X¹, X² and X⁶ is N.

In some embodiments, each from X¹, X² and X⁶ is N and each from X³, X⁴ and X⁵ is CH, wherein each H is optionally replaced by R¹.

In some embodiments, each from X¹, X² and X⁶ is N and each of X³, X⁴ and X⁵ is CH.

In some embodiments, each from X¹, X², X⁴ and X⁶ is N.

In some embodiments, each from X¹, X², X⁴ and X⁶ is N and each from X³ and X⁵ is CH, wherein each H is optionally replaced by R¹.

In some embodiments, each from X¹, X², X⁴ and X⁶ is N and each of X³ and X⁵ is CH.

In some embodiments, each R¹ is independently selected from halogen, C₁-C₆ alkyl, C₁-C₆ alkoxy, C₃-C₁₀ cycloalkyl, C₂-C₆ alkenyl, C₂-C₆ alkynyl, heterocycle, aryl, heteroaryl, C₁-C₆ alkyl-aryl, C₁-C₆ alkyl-heteroaryl, C₂-C₆ alkenyl-aryl, C₂-C₆ alkenyl-heteroaryl, C₂-C₆ alkynyl-aryl, C₂-C₆ alkynyl-heteroaryl, and NR⁹R¹⁰ wherein the alkyl, alkoxy, alkenyl, alkynyl, heterocycle, cycloalkyl, aryl, or heteroaryl is optionally substituted with one or more substituents independently selected from halogen, OH, NR⁹R¹⁰, C₁-C₆ alkyl, C₁-C₆ alkoxy, C₃-C₁₀ cycloalkyl, C₂-C₆ alkenyl, C₂-C₆ alkynyl, heterocycle, aryl, heteroaryl.

In some embodiments, R¹ is halogen.

In some embodiments, R¹ is F. In some embodiments, R¹ is Cl. In some embodiments, R¹ is Br. In some embodiments, R¹ is I.

In some embodiments, R¹ is F.

In some embodiments, each R¹ is independently selected from C₃-C₁₀ cycloalkyl, heterocycle, aryl, heteroaryl, C₁-C₆ alkyl-aryl, C₁-C₆ alkyl-heteroaryl, C₂-C₆ alkenyl-aryl, C₂-C₆ alkenyl-heteroaryl, C₂-C₆ alkynyl-aryl, and C₂-C₆ alkynyl-heteroaryl. In some embodiments, each R¹ is independently selected from C₃-C₁₀ cycloalkyl, heterocycle, aryl, and heteroaryl.

In some embodiments, each R¹ is independently selected from C₁-C₆ alkyl and C₁-C₆ alkoxy. In some embodiments, each R¹ is independently selected from C₃-C₁₀ cycloalkyl and aryl. In some embodiments, each R¹ is independently selected from heterocycle and heteroaryl.

In some embodiments, R¹ is C₁-C₆ alkyl.

In some embodiments, R¹ is methyl. In some embodiments, R¹ is ethyl. In some embodiments, R¹ is propyl. In some embodiments, R¹ is *n-*propyl. In some embodiments, R¹ is *iso*-propyl. In some embodiments, R¹ is butyl. In some embodiments, R¹ is *n-*butyl. In some embodiments, R¹ is *iso*-butyl. In some embodiments, R¹ is *sec*-butyl. In some embodiments, R¹ is *tert*-butyl. In some embodiments, R¹ is pentyl. In some embodiments, R¹ is hexyl.

In some embodiments, R¹ is C₁-C₆ alkoxy.

In some embodiments, R¹ is methoxy. In some embodiments, R¹ is ethoxy. In some embodiments, R¹ is propoxy. In some embodiments, R¹ is butoxy. In some embodiments, R¹ is pentoxy. In some embodiments, one R¹ is hexoxy.

In some embodiments, R¹ is

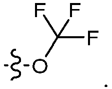

In some embodiments, p is 1 and R¹ is

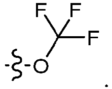

In some embodiments, R¹ is C₃-C₁₀ cycloalkyl.

In some embodiments, R¹ is a monocyclic C₃-C₁₀ cycloalkyl. In some embodiments, R¹ is a polycyclic C₃-C₁₀ cycloalkyl.

In some embodiments, R¹ is C₅-C₆ cycloalkyl.

In some embodiments, R¹ is cyclopropyl, cyclobutyl, cyclopentyl, cyclohexyl, cycloheptyl, cyclooctyl, cyclononyl, or cyclodecyl. In some embodiments, R¹ is cyclopropyl. In some embodiments, R¹ is cyclobutyl. In some embodiments, R¹ is cyclopentyl. In some embodiments, R¹ is cyclohexyl. In some embodiments, R¹ is cycloheptyl. In some embodiments, R¹ is cyclooctyl. In some embodiments, R¹ is cyclononyl. In some embodiments, R¹ is cyclodecyl.

In some embodiments, R¹ is a fused polycyclic C₃-C₁₀ cycloalkyl. In some embodiments, R¹ is a bridged polycyclic C₃-C₁₀ cycloalkyl. In some embodiments, R¹ is a C₃-C₁₀ spirocycloalkyl.

In some embodiments, R¹ is C₂-C₆ alkenyl.

In some embodiments, R¹ is C₂ alkenyl. In some embodiments, R¹ is C₃ alkenyl. In some embodiments, R¹ is C₄ alkenyl. In some embodiments, R¹is C₅ alkenyl. In some embodiments, R¹ is C₆ alkenyl.

In some embodiments, R¹ is C₂-C₆ alkynyl.

In some embodiments, R¹ is C₂ alkynyl. In some embodiments, R¹ is C₃ alkynyl. In some embodiments, R¹ is C₄ alkynyl. In some embodiments, R¹ is C₅ alkynyl. In some embodiments, R¹ is C₆ alkynyl.

In some embodiments, R¹ is heterocycle. In some embodiments, R¹ is 3-10 membered heterocycle. In some embodiments, R¹ is heterocycle comprising one, two, or three heteroatoms. In some embodiments, R¹ is 3-10 membered heterocycle comprising one, two, or three heteroatoms.

In some embodiments, R¹ is a monocyclic heterocycle. In some embodiments, R¹ is a polycyclic heterocycle.

In some embodiments, R¹ is 3-membered heterocycle. In some embodiments, R¹ is 4-membered heterocycle. In some embodiments, R¹ is 5-membered heterocycle. In some embodiments, R¹ is 6-membered heterocycle. In some embodiments, R¹ is 7-membered heterocycle. In some embodiments, R¹ is 8-membered heterocycle. In some embodiments, R¹ is 9-membered heterocycle. In some embodiments, R¹ is 10-membered heterocycle.

In some embodiments, R¹ is 5- to 6-membered heterocycle.

In some embodiments, R¹ is aryl.

In some embodiments, R¹ is C₆ aryl (e.g., phenyl).

In some embodiments, R¹ is heteroaryl. In some embodiments, R¹ is 5- to 6-membered heteroaryl.

In some embodiments, R¹ is C₁-C₆ alkyl-aryl.

In some embodiments, R¹ is methyl-aryl. In some embodiments, R¹ is ethyl-aryl. In some embodiments, R¹ is propyl-aryl. In some embodiments, R¹ is *n-*propyl-aryl. In some embodiments, R¹ is *iso*-propyl-aryl. In some embodiments, R¹ is butyl-aryl. In some embodiments, R¹ is *n-*butyl-aryl. In some embodiments, R¹ is isobutyl-aryl. In some embodiments, R¹ is *sec*-butyl-aryl. In some embodiments, R¹ is *tert*-butyl-aryl. In some embodiments, R¹ is pentyl-aryl. In some embodiments, R¹ is hexyl-aryl.

In some embodiments, R¹ is C₁-C₆ alkyl-heteroaryl.

In some embodiments, R¹ is methyl-heteroaryl. In some embodiments, R¹ is ethyl-heteroaryl. In some embodiments, R¹ is propyl-heteroaryl. In some embodiments, R¹ is *n-*propyl-heteroaryl. In some embodiments, R¹ is isopropyl-heteroaryl. In some embodiments, R¹ is butyl-heteroaryl. In some embodiments, R¹ is *n-*butyl-heteroaryl. In some embodiments, R¹ is *iso*-butyl-heteroaryl. In some embodiments, R¹ is *sec*-butyl-heteroaryl. In some embodiments, R¹ is *tert*-butyl-heteroaryl. In some embodiments, R¹ is pentyl-heteroaryl. In some embodiments, R¹ is hexyl-heteroaryl.

In some embodiments, R¹ is C₂-C₆ alkenyl-aryl.

In some embodiments, R¹ is C₂ alkenyl-aryl. In some embodiments, R¹ is C₃ alkenyl-aryl. In some embodiments, R¹ is C₄ alkenyl-aryl. In some embodiments, R¹is C₅ alkenyl-aryl. In some embodiments, R¹ is C₆ alkenyl-aryl.

In some embodiments, R¹ is C₂-C₆ alkenyl-heteroaryl.

In some embodiments, R¹ is C₂ alkenyl-heteroaryl. In some embodiments, R¹ is C₃ alkenyl-heteroaryl. In some embodiments, R¹ is C₄ alkenyl-heteroaryl. In some embodiments, R¹is C₅ alkenyl-heteroaryl. In some embodiments, R¹ is C₆ alkenyl-heteroaryl.

In some embodiments, R¹ is C₂-C₆ alkynyl-aryl.

In some embodiments, R¹ is C₂ alkynyl-aryl. In some embodiments, R¹ is C₃ alkynyl-aryl. In some embodiments, R¹ is C₄ alkynyl-aryl. In some embodiments, R¹ is C₅ alkynyl-aryl. In some embodiments, R¹ is C₆ alkynyl-aryl.

In some embodiments, R¹ is and C₂-C₆ alkynyl-heteroaryl.

In some embodiments, R¹ is C₂ alkynyl-heteroaryl. In some embodiments, R¹ is C₃ alkynyl-heteroaryl. In some embodiments, R¹ is C₄ alkynyl-heteroaryl. In some embodiments, R¹ is C₅ alkynyl-heteroaryl. In some embodiments, R¹ is C₆ alkynyl-heteroaryl.

In some embodiments, R¹ is NR⁹R¹⁰. In some embodiments, R¹ is NHR¹⁰. In some embodiments, R¹ is NHCH₃.

In some embodiments, p is 2, one R¹ is NR⁹R¹⁰, and the other R¹ is selected from C₁-C₆ alkyl, C₁-C₆ alkoxy, C₃-C₁₀ cycloalkyl, C₂-C₆ alkenyl, C₂-C₆ alkynyl, heterocycle, aryl, heteroaryl, C₁-C₆ alkyl-aryl, C₁-C₆ alkyl-heteroaryl, C₂-C₆ alkenyl-aryl, C₂-C₆ alkenyl-heteroaryl, C₂-C₆ alkynyl-aryl, C₂-C₆ alkynyl-heteroaryl, and NR⁹R¹⁰ wherein the alkyl, alkoxy, alkenyl, alkynyl, heterocycle, cycloalkyl, aryl, or heteroaryl is optionally substituted with one or more substituents independently selected from halogen, OH, C₁-C₆ alkyl, C₁-C₆ alkoxy, NR⁹R¹⁰, C₃-C₁₀ cycloalkyl, C₂-C₆ alkenyl, C₂-C₆ alkynyl, heterocycle, aryl, heteroaryl.

In some embodiments, p is 2, one R¹ is NHCH₃, and the other R¹ is C₁-C₆ alkyl optionally substituted with one or more halogen. In some embodiments, p is 2, one R¹ is NHCH₃, and the other R¹ is C₁-C₆ alkyl optionally substituted with one or more fluoro. In some embodiments, one R¹ is NHCH₃, and the other R¹ is CH₂CF₃.

In some embodiments, R¹ is C₁-C₆ alkyl substituted with one or more halogen. In some embodiments, R¹ is C₁-C₆ alkyl substituted with one or more F. In some embodiments, R¹ is C₁-C₆ alkyl substituted with one or more Cl. In some embodiments, R¹ is C₁-C₆ alkyl substituted with one or more Br. In some embodiments, R¹ is C₁-C₆ alkyl substituted with one or more I. In some embodiments, R¹ is C₁-C₆ alkyl substituted with one or more OH. In some embodiments, R¹ is C₁-C₆ alkyl substituted with one or more NH₂. In some embodiments, R¹ is C₁-C₆ alkyl substituted with one or more C₁-C₆ alkyl. In some embodiments, R¹ is C₁-C₆ alkyl substituted with one or more C₁-C₆ alkoxy. In some embodiments, R¹ is C₁-C₆ alkyl substituted with one or more NR⁹R¹⁰. In some embodiments, R¹ is C₁-C₆ alkyl substituted with one or more C₃-C₁₀ cycloalkyl. In some embodiments, R¹ is C₁-C₆ alkyl substituted with one or more C₂-C₆ alkenyl. In some embodiments, R¹ is C₁-C₆ alkyl substituted with one or more C₂-C₆ alkynyl. In some embodiments, R¹ is C₁-C₆ alkyl substituted with one or more heterocycle. In some embodiments, R¹ is C₁-C₆ alkyl substituted with one or more aryl. In some embodiments, R¹ is C₁-C₆ alkyl substituted with one or more heteroaryl.

In some embodiments, R¹ is C₁-C₆ alkyl substituted with one or more F substituted with one or more F. In some embodiments, R¹ is methyl substituted with one or more F. In some embodiments, R¹ is ethyl substituted with one or more F. In some embodiments, R¹ is propyl substituted with one or more F. In some embodiments, R¹ is *n-*propyl substituted with one or more F. In some embodiments, R¹ is *iso*-propyl substituted with one or more F. In some embodiments, R¹ is butyl substituted with one or more F. In some embodiments, R¹ is *n-*butyl substituted with one or more F. In some embodiments, R¹ is *iso*-butyl substituted with one or more F. In some embodiments, R¹ is *sec*-butyl substituted with one or more F. In some embodiments, R¹ is *tert*-butyl substituted with one or more F. In some embodiments, R¹ is pentyl substituted with one or more F. In some embodiments, R¹ is hexyl substituted with one or more F.

In some embodiments, R¹ is (CH₂)₀₋₅CF₃. In some embodiments, R¹ is CF₃. In some embodiments, R¹ is CH₂CF₃.

In some embodiments, R¹ is -CH₂CF₃. In some embodiments, R¹ is -CF₂CF₃.

In some embodiments, p is 1 and R¹ is (CH₂)₀₋₅CF₃. In some embodiments, p is 1 and R¹ is CF₃. In some embodiments, p is 1 and R¹ is CH₂CF₃.

In some embodiments, p is 2 and at least one and R¹ is (CH₂)₀₋₅CF₃. In some embodiments, p is 2 and at least one and R¹ is CF₃. In some embodiments, p is 2 and at least one and R¹ is CH₂CF₃.

In some embodiments, R¹ is

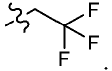

In some embodiments, R¹ is

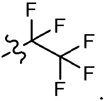

In some embodiments, R¹ is C₁-C₆ alkoxy substituted with one or more halogen. In some embodiments, R¹ is C₁-C₆ alkoxy substituted with one or more F. In some embodiments, R¹ is C₁-C₆ alkoxy substituted with one or more Cl. In some embodiments, R¹ is C₁-C₆ alkoxy substituted with one or more Br. In some embodiments, R¹ is C₁-C₆ alkoxy substituted with one or more I. In some embodiments, R¹ is C₁-C₆ alkoxy substituted with one or more OH. In some embodiments, R¹ is C₁-C₆ alkoxy substituted with one or more NH2. In some embodiments, R¹ is C₁-C₆ alkoxy substituted with one or more C₁-C₆ alkyl. In some embodiments, R¹ is C₁-C₆ alkoxy substituted with one or more C₁-C₆ alkoxy. In some embodiments, R¹ is C₁-C₆ alkoxy substituted with one or more N R¹2 R¹3. In some embodiments, R¹ is C₁-C₆ alkoxy substituted with one or more C₃-C₁₀ cycloalkyl. In some embodiments, R¹ is C₁-C₆ alkoxy substituted with one or more C₂-C₆ alkenyl. In some embodiments, R¹ is C₁-C₆ alkoxy substituted with one or more C₂-C₆ alkynyl. In some embodiments, R¹ is C₁-C₆ alkoxy substituted with one or more heterocycle. In some embodiments, R¹ is C₁-C₆ alkoxy substituted with one or more aryl. In some embodiments, R¹ is C₁-C₆ alkoxy substituted with one or more heteroaryl.

In some embodiments, R¹ is C₃-C₁₀ cycloalkyl substituted with one or more halogen. In some embodiments, R¹ is C₃-C₁₀ cycloalkyl substituted with one or more F. In some embodiments, R¹ is C₃-C₁₀ cycloalkyl substituted with one or more Cl. In some embodiments, R¹ is C₃-C₁₀ cycloalkyl substituted with one or more Br. In some embodiments, R¹ is C₃-C₁₀ cycloalkyl substituted with one or more I. In some embodiments, R¹ is C₃-C₁₀ cycloalkyl substituted with one or more OH. In some embodiments, R¹ is C₃-C₁₀ cycloalkyl substituted with one or more NH₂. In some embodiments, R¹ is C₃-C₁₀ cycloalkyl substituted with one or more C₁-C₆ alkyl. In some embodiments, R¹ is C₃-C₁₀ cycloalkyl substituted with one or more C₁-C₆ alkoxy. In some embodiments, R¹ is C₃-C₁₀ cycloalkyl substituted with one or more NR¹²R¹³. In some embodiments, R¹ is C₃-C₁₀ cycloalkyl substituted with one or more C₃-C₁₀ cycloalkyl. In some embodiments, R¹ is C₃-C₁₀ cycloalkyl substituted with one or more C₂-C₆ alkenyl. In some embodiments, R¹ is C₃-C₁₀ cycloalkyl substituted with one or more C₂-C₆ alkynyl. In some embodiments, R¹ is C₃-C₁₀ cycloalkyl substituted with one or more heterocycle. In some embodiments, R¹ is C₃-C₁₀ cycloalkyl substituted with one or more aryl. In some embodiments, R¹ is C₃-C₁₀ cycloalkyl substituted with one or more heteroaryl.

In some embodiments, R¹ is C₂-C₆ alkenyl substituted with one or more halogen. In some embodiments, R¹ is C₂-C₆ alkenyl substituted with one or more F. In some embodiments, R¹ is C₂-C₆ alkenyl substituted with one or more Cl. In some embodiments, R¹ is C₂-C₆ alkenyl substituted with one or more Br. In some embodiments, R¹ is C₂-C₆ alkenyl substituted with one or more I. In some embodiments, R¹ is C₂-C₆ alkenyl substituted with one or more OH. In some embodiments, R¹ is C₂-C₆ alkenyl substituted with one or more NH2. In some embodiments, R¹ is C₂-C₆ alkenyl substituted with one or more C₁-C₆ alkyl. In some embodiments, R¹ is C₂-C₆ alkenyl substituted with one or more C₁-C₆ alkoxy. In some embodiments, R¹ is C₂-C₆ alkenyl substituted with one or more NR¹²R¹³. In some embodiments, R¹ is C₂-C₆ alkenyl substituted with one or more C₃-C₁₀ cycloalkyl. In some embodiments, R¹ is C₂-C₆ alkenyl substituted with one or more C₂-C₆ alkenyl. In some embodiments, R¹ is C₂-C₆ alkenyl substituted with one or more C₂-C₆ alkynyl. In some embodiments, R¹ is C₂-C₆ alkenyl substituted with one or more heterocycle. In some embodiments, R¹ is C₂-C₆ alkenyl substituted with one or more aryl. In some embodiments, R¹ is C₂-C₆ alkenyl substituted with one or more heteroaryl.

In some embodiments, R¹ is C₂-C₆ alkynyl substituted with one or more halogen. In some embodiments, R¹ is C₂-C₆ alkynyl substituted with one or more heteroaryl.

In some embodiments, R¹ is heterocycle substituted with one or more halogen. In some embodiments, R¹ is heterocycle substituted with one or more heteroaryl.

In some embodiments, R¹ is aryl substituted with one or more halogen. In some embodiments, R¹ is aryl substituted with one or more heteroaryl.

In some embodiments, R¹ is heteroaryl substituted with one or more halogen. In some embodiments, R¹ is heteroaryl substituted with one or more heteroaryl.

In some embodiments, R¹ is C₁-C₆ alkyl-aryl substituted with one or more halogen. In some embodiments, R¹ is C₁-C₆ alkyl-aryl substituted with one or more heteroaryl.

In some embodiments, R¹ is

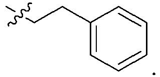

In some embodiments, p is 1 and R¹ is

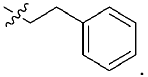

In some embodiments, R¹ is C₁-C₆ alkyl-heteroaryl substituted with one or more halogen. In some embodiments, R¹ is C₁-C₆ alkyl-heteroaryl substituted with one or more heteroaryl.

In some embodiments, R¹ is

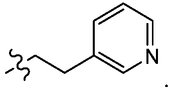

In some embodiments, p is 1 and R¹ is

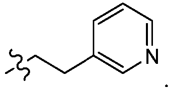

In some embodiments, R¹ is

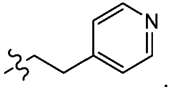

In some embodiments, p is 1 and R¹ is

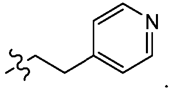

In some embodiments, R¹ is

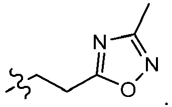

In some embodiments, p is 1 and R¹ is

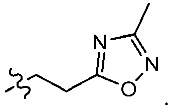

In some embodiments, R¹ is C₂-C₆ alkenyl-aryl substituted with one or more halogen. In some embodiments, R¹ is C₂-C₆ alkenyl-aryl substituted with one or more heteroaryl.

In some embodiments, R¹ is C₂-C₆ alkenyl-heteroaryl substituted with one or more halogen. In some embodiments, R¹ is C₂-C₆ alkenyl-heteroaryl substituted with one or more heteroaryl.

In some embodiments, R¹ is C₂-C₆ alkynyl-aryl substituted with one or more halogen. In some embodiments, R¹ is C₂-C₆ alkynyl-aryl substituted with one or more heteroaryl.

In some embodiments, R¹ is

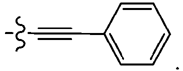

In some embodiments, p is 1 and R¹ is

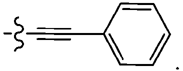

In some embodiments, R¹ is C₂-C₆ alkynyl-heteroaryl substituted with one or more halogen. In some embodiments, R¹ is C₂-C₆ alkynyl-heteroaryl substituted with one or more heteroaryl.

In some embodiments, R¹ is unsubstituted C₁-C₆ alkyl. In some embodiments, R¹ is unsubstituted C₁-C₆ alkoxy. In some embodiments, R¹ is unsubstituted C₃-C₁₀ cycloalkyl. In some embodiments, R¹ is unsubstituted C₂-C₆ alkenyl. In some embodiments, R¹ is unsubstituted C₂-C₆ alkynyl. In some embodiments, R¹ is unsubstituted heterocycle. In some embodiments, R¹ is unsubstituted aryl. In some embodiments, R¹ is unsubstituted heteroaryl. In some embodiments, R¹ is unsubstituted C₁-C₆ alkyl-aryl. In some embodiments, R¹ is unsubstituted C₁-C₆ alkyl-heteroaryl. In some embodiments, R¹ is unsubstituted C₂-C₆ alkenyl-aryl. In some embodiments, R¹ is unsubstituted C₂-C₆ alkenyl-heteroaryl. In some embodiments, R¹ is unsubstituted C₂-C₆ alkynyl-aryl. In some embodiments, R¹ is unsubstituted C₂-C₆ alkynyl-heteroaryl.

In some embodiments, R¹ is -OH.

In some embodiments, R¹ is -NH₂.

In some embodiments, R¹ is -NHCH₃.

In some embodiments, R¹ is -N(CH₃)₂

In some embodiments, L is selected from (CR⁵₂)_{q}, (CR⁵₂)_{q}O, (CR⁵₂)_{q}S(O)ₛ and (CR⁵₂)_{q}C(O).

In some embodiments, L is (CR⁵₂)_{q}.

In some embodiments, L is (CR⁵₂)_{q}O.

In some embodiments, L is (CR⁵₂)_{q}S(O)ₛ.

In some embodiments, s is an integer selected from 0, 1, and 2.

In some embodiments, s is 0.

In some embodiments, s is 1.

In some embodiments, s is 2.

In some embodiments, s is 0 and L is (CR⁵₂)_{q}S.

In some embodiments, s is 1 and L is (CR⁵₂)_{q}S(O)_{.}

In some embodiments, s is 2 and L is (CR⁵₂)_{q}S(O)₂.

In some embodiments, L is (CR⁵₂)_{q}C(O).

In some embodiments, L is (CR⁵₂)_{q} and the compound is of Formula I-1:

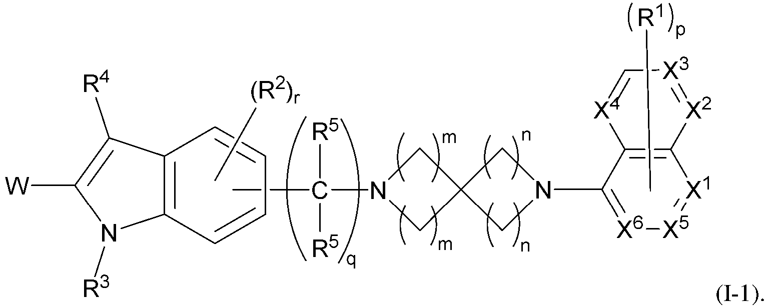

In some embodiments, L is (CR⁵₂)_{q}O and the compound is of Formula I-2:

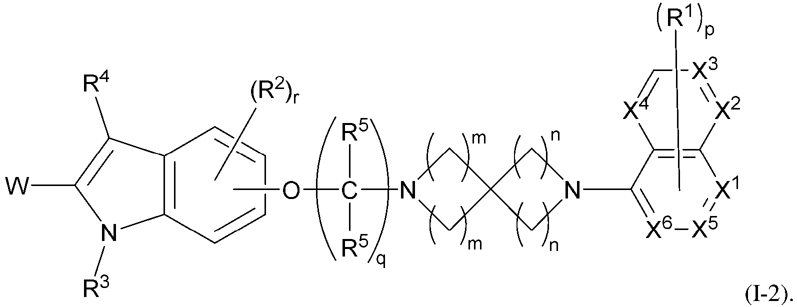

In some embodiments, L is (CR⁵₂)_{q}S(O)ₛ and the compound is of Formula I-3:

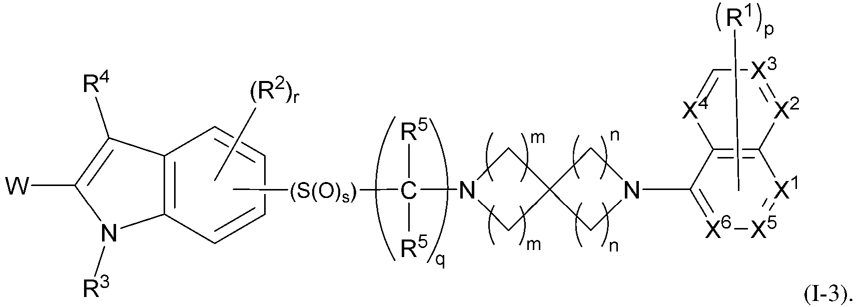

In some embodiments, L is (CR⁵₂)_{q}S(O)₂ and the compound is of Formula I-3':

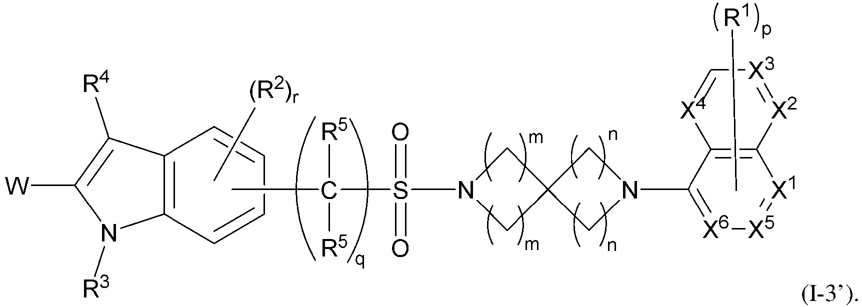

In some embodiments, L is (CR⁵₂)_{q}C(O) and the compound is of Formula I-4:

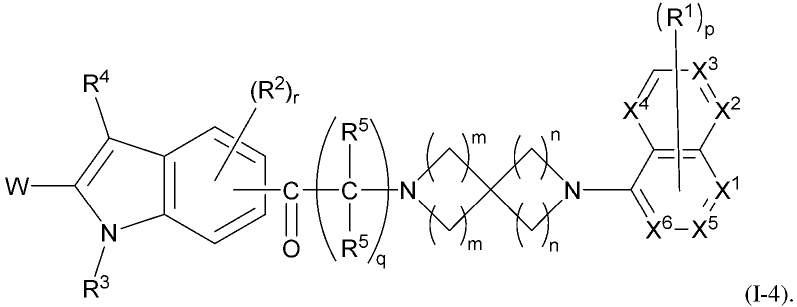

In some embodiments, L is (CR⁵₂)_{q}C(O) and the compound is of Formula I-4':

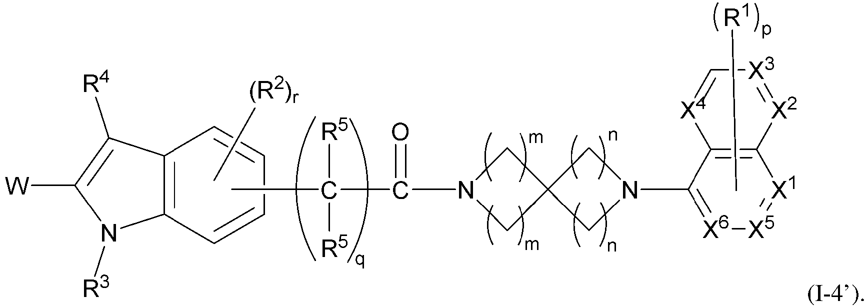

In some embodiments, q is an integer selected from 0, 1, 2, 3 and 4.

In some embodiments, q is 0.

In some embodiments, q is 1.

In some embodiments, q is 2.

In some embodiments, q is 3.

In some embodiments, q is 4.

In some embodiments, q is 0 and L is bond.

In some embodiments, q is 0 and L is S(O)₂.

In some embodiments, q is 0 and L is C(O).

In some embodiments, each R⁵ is independently selected from H, halogen, CN, OH, C₁-C₆ alkyl, C₂-C₆ alkenyl, C₂-C₆ alkynyl, C₃-C₁₀ cycloalkyl.

In some embodiments, R⁵ is H.

In some embodiments, R⁵ is C₁-C₆ alkyl.

In some embodiments, R⁵ is methyl.

In some embodiments, q is 1 and L is C(R⁵)₂.

In some embodiments, q is 1 and L is CH₂.

In some embodiments, q is 1 and L is C(R⁵)₂O.

In some embodiments, q is 1 and L is CH₂O.

In some embodiments, q is 1 and L is C(R⁵)₂S.

In some embodiments, q is 1 and L is CH₂S.

In some embodiments, q is 1 and L is C(R⁵)₂S(O).

In some embodiments, q is 1 and L is CH₂S(O).

In some embodiments, q is 1 and L is C(R⁵)₂S(O)₂.

In some embodiments, q is 1 and L is CH₂S(O)₂.

In some embodiments, q is 1 and L is C(R⁵)₂C(O).

In some embodiments, q is 1 and L is CH₂C(O).

In some embodiments, q is 1 and L is C(R⁵)₂C(R⁵)₂.

In some embodiments, q is 1 and L is CH₂CH₂.

In some embodiments, q is 1 and L is C(R⁵)₂C(R⁵)₂O.

In some embodiments, q is 1 and L is CH₂CH₂O.

In some embodiments, q is 1 and L is C(R⁵)₂C(R⁵)₂S.

In some embodiments, q is 1 and L is CH₂CH₂S.

In some embodiments, q is 1 and L is C(R⁵)₂C(R⁵)₂S(O).

In some embodiments, q is 1 and L is CH₂CH₂S(O).

In some embodiments, q is 1 and L is C(R⁵)₂C(R⁵)₂S(O)₂.

In some embodiments, q is 1 and L is CH₂CH₂S(O)₂.

In some embodiments, q is 1 and L is C(R⁵)₂C(R⁵)₂C(O).

In some embodiments, q is 1 and L is CH₂CH₂C(O).

In some embodiments, q is 1 and L is (C(R⁵)₂)₃.

In some embodiments, q is 1 and L is CH₂CH₂CH₂.

In some embodiments, q is 1 and L is (C(R⁵)₂)₃O.

In some embodiments, q is 1 and L is CH₂CH₂CH₂O.

In some embodiments, q is 1 and L is (C(R⁵)₂)₃S.

In some embodiments, q is 1 and L is CH₂CH₂CH₂S.

In some embodiments, q is 1 and L is (C(R⁵)₂)₃S(O).

In some embodiments, q is 1 and L is CH₂CH₂CH₂S(O).

In some embodiments, q is 1 and L is (C(R⁵)₂)₃S(O)₂.

In some embodiments, q is 1 and L is CH₂CH₂CH₂S(O)₂.

In some embodiments, q is 1 and L is (C(R⁵)₂)₃C(O).

In some embodiments, q is 1 and L is CH₂CH₂CH₂C(O).

In some embodiments, q is 1 and L is (C(R⁵)₂)₄.

In some embodiments, q is 1 and L is CH₂CH₂CH₂CH₂.

In some embodiments, q is 1 and L is (C(R⁵)₂)₄O.

In some embodiments, q is 1 and L is CH₂CH₂CH₂CH₂O.

In some embodiments, q is 1 and L is (C(R⁵)₂)₄S.

In some embodiments, q is 1 and L is CH₂CH₂CH₂CH₂S.

In some embodiments, q is 1 and L is (C(R⁵)₂)₄S(O).

In some embodiments, q is 1 and L is CH₂CH₂CH₂CH₂S(O).

In some embodiments, q is 1 and L is (C(R⁵)₂)₄S(O)₂.

In some embodiments, q is 1 and L is CH₂CH₂CH₂CH₂S(O)₂.

In some embodiments, q is 1 and L is (C(R⁵)₂)₄C(O).

In some embodiments, q is 1 and L is CH₂CH₂CH₂CH₂C(O).

In some embodiments, L is CH₂.

In some embodiments, L is CH₂CH₂.

In some embodiments, L is C(O).

In some embodiments, L is CH₂CH₂O.

In some embodiments, each R² is independently selected from halogen, OH, CN, C₁-C₆ alkyl, C₁-C₆ alkoxy, NR⁹R¹⁰, C₃-C₁₀ cycloalkyl, aryl, heterocyclyl, heteroaryl.

In some embodiments, R² halogen.

In some embodiments, R² is F.

In some embodiments, R² is Cl.

In some embodiments, R² is Br.

In some embodiments, R² is I.

In some embodiments, R² is OH.

In some embodiments, R² is CN.

In some embodiments, R² is C₁-C₆ alkyl.

In some embodiments, R² is methyl.

In some embodiments, R² is methyl. In some embodiments, R² is ethyl. In some embodiments, R² is propyl. In some embodiments, R² is *n-*propyl. In some embodiments, R² is *iso*-propyl. In some embodiments, R² is butyl. In some embodiments, R² is *n-*butyl. In some embodiments, R² is *iso*-butyl. In some embodiments, R² is *sec*-butyl. In some embodiments, R² is *tert*-butyl. In some embodiments, R² is pentyl. In some embodiments, R² is hexyl.

In some embodiments, R² is C₁-C₆ alkoxy.

In some embodiments, R² is methoxy.

In some embodiments, R² is ethoxy.

In some embodiments, R² is propoxy.

In some embodiments, R² is

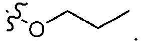

In some embodiments, R² is

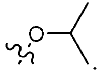

In some embodiments, R² is butoxy.

In some embodiments, R² is

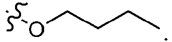

In some embodiments, R² is

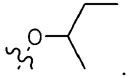

In some embodiments, R² is

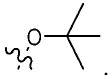

In some embodiments, R² is pentoxy.

In some embodiments, R² is hexoxy.

In some embodiments, R² is NR⁹R¹⁰.

In some embodiments, R² is NH₂.

In some embodiments, R² is NHCH₃.

In some embodiments, R² is N(CH₃)₂.

In some embodiments, R² is C₃-C₁₀ cycloalkyl.

In some embodiments, R² is cyclopropyl.

In some embodiments, R² is cyclobutyl.

In some embodiments, R² is cyclopentyl.

In some embodiments, R² is aryl.

In some embodiments, R² is phenyl.

In some embodiments, R³ is selected from hydrogen, deuterium, C₁-C₆ alkyl, C₃-C₁₀ cycloalkyl, and L⁴.

In some embodiments, R³ is H.

In some embodiments, R³ is C₁-C₆ alkyl.

In some embodiments, R³ is methyl.

In some embodiments, R³ is C₃-C₁₀ cycloalkyl.

In some embodiments, R³ is cyclopropyl.

In some embodiments, R³ is L⁴.

In some embodiments, L⁴ is

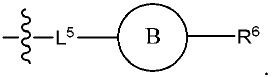

In some embodiments, L⁵ is selected from (CR⁵₂)_{q}, (CR⁵₂)_{q}O, (CR⁵₂)_{q}S(O)ₛ and (CR⁵₂)_{q}C(O).

In some embodiments, L⁵ is (CR⁵₂)_{q}.

In some embodiments, L⁵ is (CR⁵₂)_{q}O.

In some embodiments, L⁵ is (CR⁵₂)_{q}S(O)ₛ.

In some embodiments, s is an integer selected from 0, 1, and 2.

In some embodiments, s is 0.

In some embodiments, s is 1.

In some embodiments, s is 2.

In some embodiments, s is 0 and L is (CR⁵₂)_{q}S.

In some embodiments, s is 1 and L is (CR⁵₂)_{q}S(O)_{.}

In some embodiments, s is 2 and L is (CR⁵₂)_{q}S(O)₂.

In some embodiments, L⁵ is (CR⁵₂)_{q}C(O).

In some embodiments, L⁵ is (CR⁵₂)_{q} and the compound is of Formula I-11:

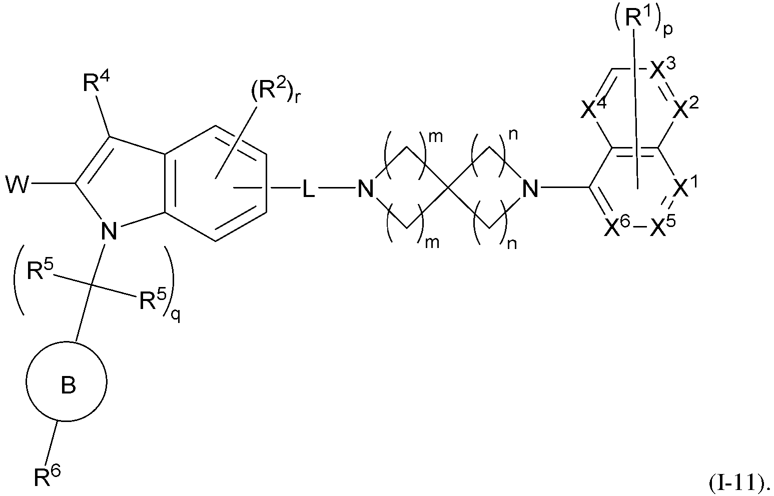

In some embodiments, L⁵ is (CR⁵₂)_{q}O and the compound is of Formula I-12:

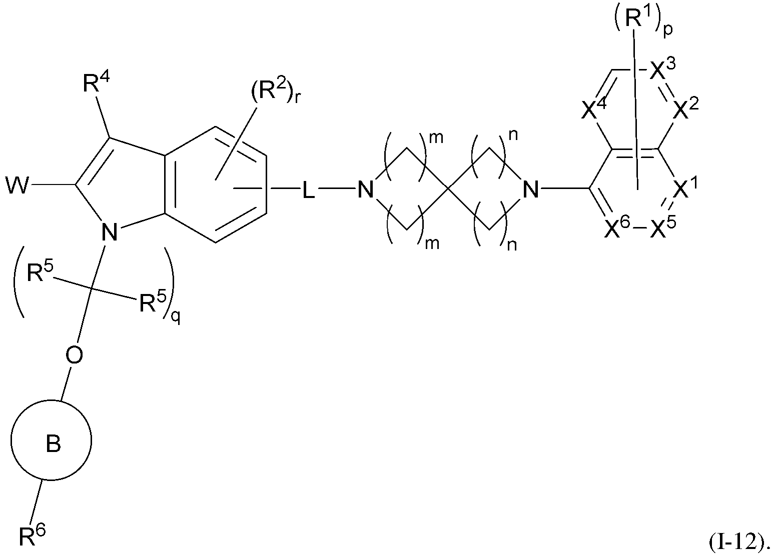

In some embodiments, L⁵ is (CR⁵₂)_{q}S(O)₂ and the compound is of Formula I-13:

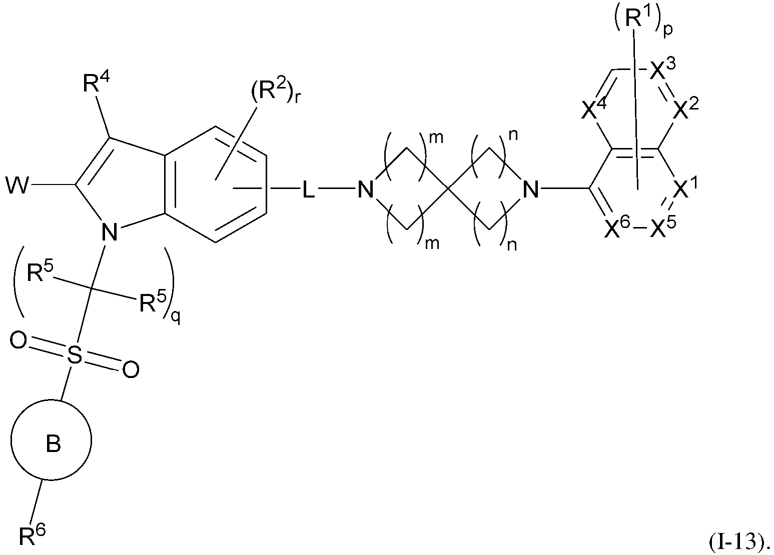

In some embodiments, L⁵ is (CR⁵₂)_{q}S(O)₂ and the compound is of Formula I-14:

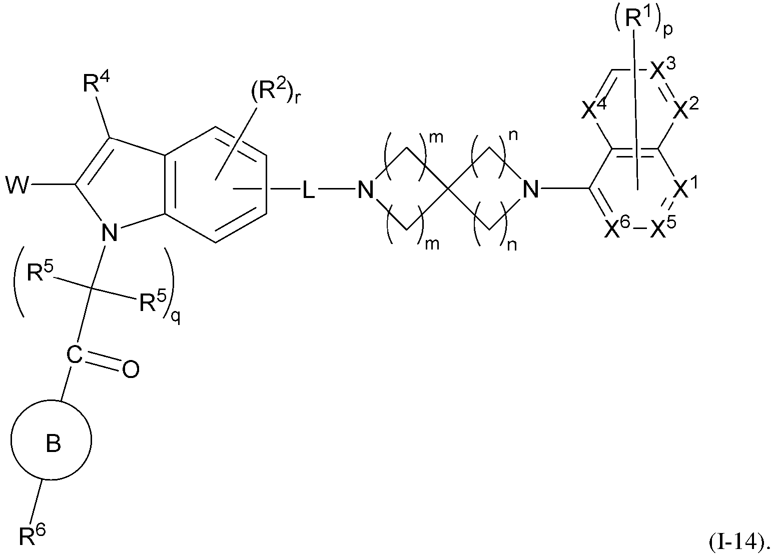

In some embodiments, q is an integer selected from 0, 1, 2, 3 and 4.

In some embodiments, q is 0.

In some embodiments, q is 1.

In some embodiments, q is 2.

In some embodiments, q is 3.

In some embodiments, q is 4.

In some embodiments, q is 0 and L⁵ is bond.

In some embodiments, q is 0 and L⁵ is S(O)₂.

In some embodiments, q is 0 and L⁵ is C(O).

In some embodiments, each R⁵ is independently selected from H, halogen, CN, OH, C₁-C₆ alkyl, C₂-C₆ alkenyl, C₂-C₆ alkynyl, C₃-C₁₀ cycloalkyl.

In some embodiments, R⁵ is H.

In some embodiments, R⁵ is C₁-C₆ alkyl.

In some embodiments, R⁵ is methyl.

In some embodiments, q is 1 and L⁵ is C(R⁵)₂.

In some embodiments, q is 1 and L⁵ is CH₂.

In some embodiments, q is 1 and L⁵ is C(R⁵)₂O.

In some embodiments, q is 1 and L⁵ is CH₂O.

In some embodiments, q is 1 and L⁵ is C(R⁵)₂S.

In some embodiments, q is 1 and L⁵ is CH₂S.

In some embodiments, q is 1 and L⁵ is C(R⁵)₂S(O).

In some embodiments, q is 1 and L⁵ is CH₂S(O).

In some embodiments, q is 1 and L⁵ is C(R⁵)₂S(O)₂.

In some embodiments, q is 1 and L⁵ is CH₂S(O)₂.

In some embodiments, q is 1 and L⁵ is C(R⁵)₂C(O).

In some embodiments, q is 1 and L⁵ is CH₂C(O).

In some embodiments, q is 1 and L⁵ is C(R⁵)₂C(R⁵)₂.

In some embodiments, q is 1 and L⁵ is CH₂CH₂.

In some embodiments, q is 1 and L⁵ is C(R⁵)₂C(R⁵)₂O.

In some embodiments, q is 1 and L⁵ is CH₂CH₂O.

In some embodiments, q is 1 and L⁵ is C(R⁵)₂C(R⁵)₂S.

In some embodiments, q is 1 and L⁵ is CH₂CH₂S.

In some embodiments, q is 1 and L⁵ is C(R⁵)₂C(R⁵)₂S(O).

In some embodiments, q is 1 and L⁵ is CH₂CH₂S(O).

In some embodiments, q is 1 and L⁵ is C(R⁵)₂C(R⁵)₂S(O)₂.

In some embodiments, q is 1 and L⁵ is CH₂CH₂S(O)₂.

In some embodiments, q is 1 and L⁵ is C(R⁵)₂C(R⁵)₂C(O).

In some embodiments, q is 1 and L⁵ is CH₂CH₂C(O).

In some embodiments, q is 1 and L⁵ is (C(R⁵)₂)₃.

In some embodiments, q is 1 and L⁵ is CH₂CH₂CH₂.

In some embodiments, q is 1 and L⁵ is (C(R⁵)₂)₃O.

In some embodiments, q is 1 and L⁵ is CH₂CH₂CH₂O.

In some embodiments, q is 1 and L⁵ is (C(R⁵)₂)₃S.

In some embodiments, q is 1 and L⁵ is CH₂CH₂CH₂S.

In some embodiments, q is 1 and L⁵ is (C(R⁵)₂)₃S(O).

In some embodiments, q is 1 and L⁵ is CH₂CH₂CH₂S(O).

In some embodiments, q is 1 and L⁵ is (C(R⁵)₂)₃S(O)₂.

In some embodiments, q is 1 and L⁵ is CH₂CH₂CH₂S(O)₂.

In some embodiments, q is 1 and L⁵ is (C(R⁵)₂)₃C(O).

In some embodiments, q is 1 and L⁵ is CH₂CH₂CH₂C(O).

In some embodiments, q is 1 and L⁵ is (C(R⁵)₂)₄.

In some embodiments, q is 1 and L⁵ is CH₂CH₂CH₂CH₂.

In some embodiments, q is 1 and L⁵ is (C(R⁵)₂)₄O.

In some embodiments, q is 1 and L⁵ is CH₂CH₂CH₂CH₂O.

In some embodiments, q is 1 and L⁵ is (C(R⁵)₂)₄S.

In some embodiments, q is 1 and L⁵ is CH₂CH₂CH₂CH₂S.

In some embodiments, q is 1 and L⁵ is (C(R⁵)₂)₄S(O).

In some embodiments, q is 1 and L⁵ is CH₂CH₂CH₂CH₂S(O).

In some embodiments, q is 1 and L⁵ is (C(R⁵)₂)₄S(O)₂.

In some embodiments, q is 1 and L⁵ is CH₂CH₂CH₂CH₂S(O)₂.

In some embodiments, q is 1 and L⁵ is (C(R⁵)₂)₄C(O).

In some embodiments, q is 1 and L⁵ is CH₂CH₂CH₂CH₂C(O).

In some embodiments, L⁵ is -CH₂-.

In some embodiments, L⁵ is

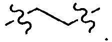

In some embodiments, L⁵ is

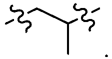

In some embodiments, L⁵ is 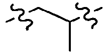 and the compound is of Formula I-21:

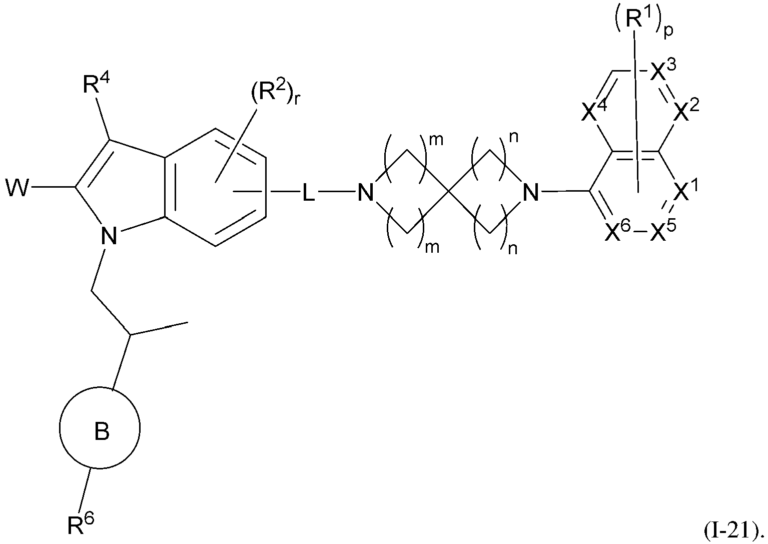

In some embodiments, L⁵ is

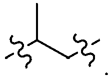

In some embodiments, L⁵ is 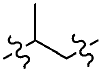 and the compound is of Formula I-22:

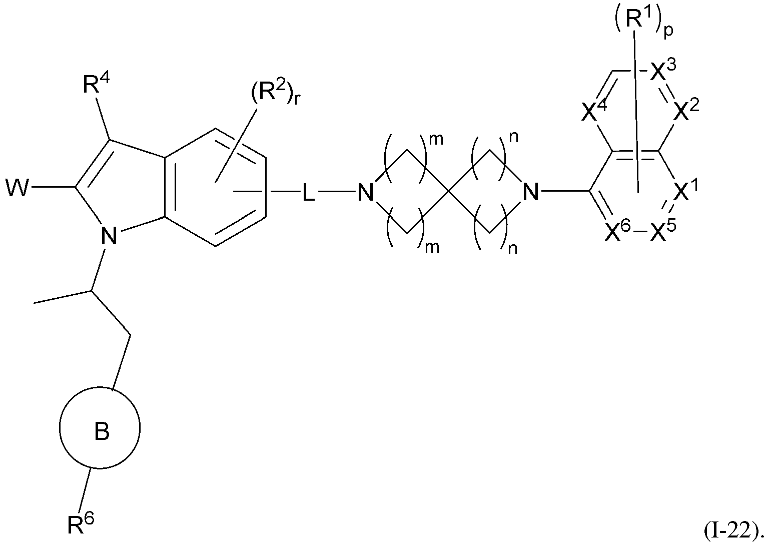

In some embodiments, L⁵ is

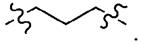

In some embodiments, L⁵ is

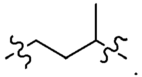

In some embodiments, L⁵ is 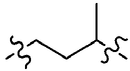 and the compound is of Formula I-23:

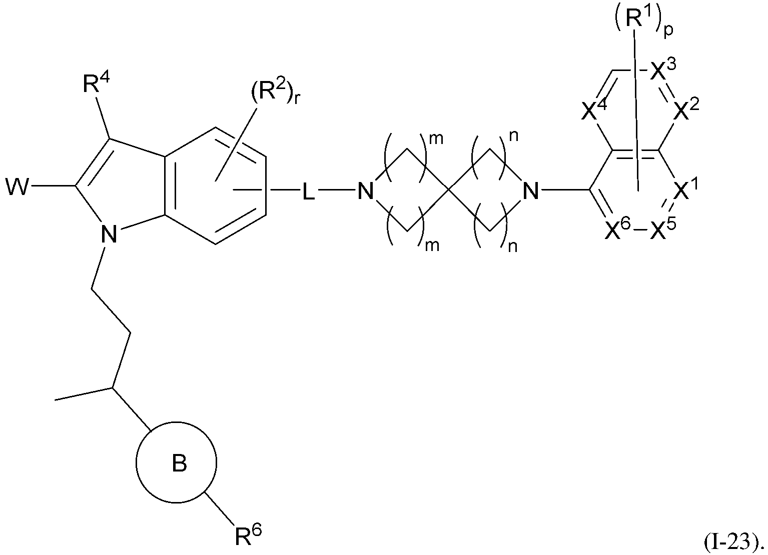

In some embodiments, L⁵ is

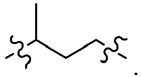

In some embodiments, L⁵ is 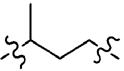 and the compound is of Formula I-24:

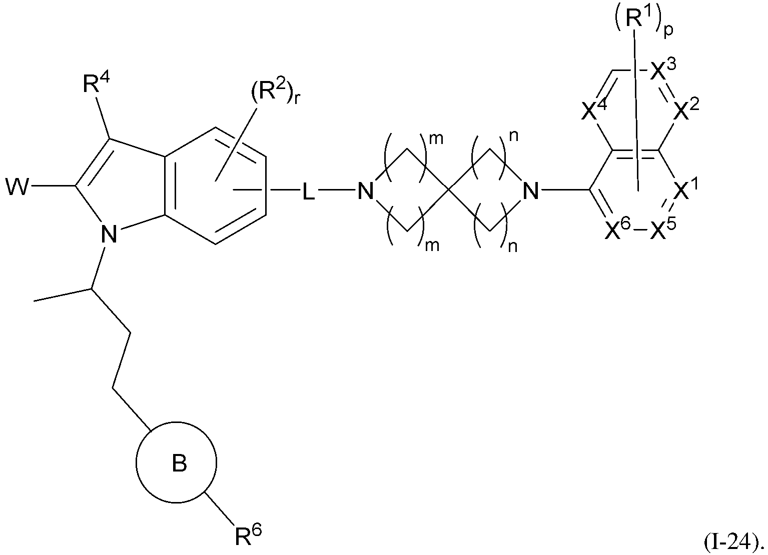

In some embodiments, L⁵ is

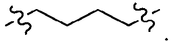

In some embodiments, Ring B is aryl, heteroaryl, 3- to 8-membered heterocycle, or C₃-C₁₄ cycloalkyl, wherein aryl, heteroaryl, heterocycle or cycloalkyl is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is aryl wherein aryl is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is phenyl wherein phenyl is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is benzenediyl wherein benzenediyl is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is benzenediyl-1,4 wherein benzenediyl-1,4 is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is benzenediyl-1,3 wherein benzenediyl-1,3 is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is benzenediyl-1,2 wherein benzenediyl-1,2 is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is phenyl.

In some embodiments, Ring B is benzenediyl.

In some embodiments, Ring B is benzenediyl-1,4.

In some embodiments, Ring B is benzenediyl-1,3.

In some embodiments, Ring B is benzenediyl-1,2.

In some embodiments, Ring B is heteroaryl, wherein heteroaryl is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is heteroaryl.

In some embodiments, Ring B is monocyclic 5-membered heteroaryl.

In some embodiments, Ring B is monocyclic 5-membered heteroaryl containing one N as the ring heteroatom, the remaining ring atoms being C.

In some embodiments, Ring B is monocyclic 5-membered heteroaryl containing two N as the ring heteroatoms, the remaining ring atoms being C.

In some embodiments, Ring B is monocyclic 5-membered heteroaryl containing one N and one O as the ring heteroatoms, the remaining ring atoms being C.

In some embodiments, Ring B is monocyclic 5-membered heteroaryl containing one O as the ring heteroatom, the remaining ring atoms being C.

In some embodiments, Ring B is monocyclic 5-membered heteroaryl containing one S as the ring heteroatom, the remaining ring atoms being C.

In some embodiments, Ring B is monocyclic 6-membered heteroaryl.

In some embodiments, Ring B is monocyclic 6-membered heteroaryl containing one N as the ring heteroatom, the remaining ring atoms being C.

In some embodiments, Ring B is monocyclic 6-membered heteroaryl containing two N as the ring heteroatoms, the remaining ring atoms being C.

In some embodiments, Ring B is monocyclic 6-membered heteroaryl containing three N as the ring heteroatoms, the remaining ring atoms being C.

In some embodiments, Ring B is bicyclic 9-membered heteroaryl.

In some embodiments, Ring B is bicyclic 9-membered heteroaryl containing one N as the ring heteroatoms, the remaining ring atoms being C.

In some embodiments, Ring B is bicyclic 9-membered heteroaryl containing two N as the ring heteroatoms, the remaining ring atoms being C.

In some embodiments, Ring B is bicyclic 9-membered heteroaryl containing three N as the ring heteroatoms, the remaining ring atoms being C.

In some embodiments, Ring B is bicyclic 9-membered heteroaryl containing one O as the ring heteroatom, the remaining ring atoms being C.

In some embodiments, Ring B is bicyclic 9-membered heteroaryl containing one S as the ring heteroatom, the remaining ring atoms being C.

In some embodiments, Ring B is bicyclic 10-membered heteroaryl.

In some embodiments, Ring B is bicyclic 10-membered heteroaryl containing one N as the ring heteroatom, the remaining ring atoms being C.

In some embodiments, Ring B is bicyclic 10-membered heteroaryl containing two N as the ring heteroatoms, the remaining ring atoms being C.

In some embodiments, Ring B is bicyclic 10-membered heteroaryl containing three N as the ring heteroatoms, the remaining ring atoms being C.

In some embodiments, Ring B is 3- to 8-membered heterocycle, wherein heterocycle is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is 3- to 8-membered heterocycle.

In some embodiments, Ring B is 3-membered heterocycle.

In some embodiments, Ring B is 3-membered heterocycle containing one N as the ring heteroatom, the remaining ring atoms being C.

In some embodiments, Ring B is 4-membered heterocycle.

In some embodiments, Ring B is 4-membered heterocycle containing one N as the ring heteroatom, the remaining ring atoms being C.

In some embodiments, Ring B is 5-membered heterocycle.

In some embodiments, Ring B is 5-membered heterocycle containing one N as the ring heteroatom, the remaining ring atoms being C.

In some embodiments, Ring B is

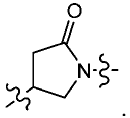

In some embodiments, Ring B is

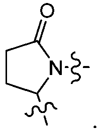

In some embodiments, Ring B is 5-membered heterocycle containing two N as the ring heteroatoms, the remaining ring atoms being C.

In some embodiments, Ring B is

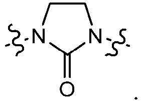

In some embodiments, Ring B is 5-membered heterocycle containing one O as the ring heteroatom, the remaining ring atoms being C.

In some embodiments, Ring B is 5-membered heterocycle containing one S as the ring heteroatom, the remaining ring atoms being C.

In some embodiments, Ring B is 6-membered heterocycle.

In some embodiments, Ring B is 6-membered heterocycle containing one N as the ring heteroatom, the remaining ring atoms being C.

In some embodiments, Ring B is

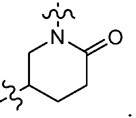

In some embodiments, Ring B is 6-membered heterocycle containing one N as the ring heteroatom, the remaining ring atoms being C substituted with aryl.

In some embodiments, Ring B is

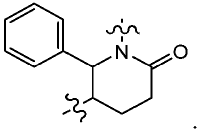

In some embodiments, Ring B is

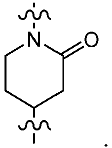

In some embodiments, Ring B is 6-membered heterocycle containing two N as the ring heteroatoms, the remaining ring atoms being C.

In some embodiments, Ring B is

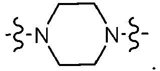

In some embodiments, Ring B is

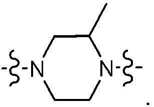

In some embodiments, Ring B is

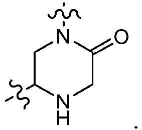

In some embodiments, Ring B is

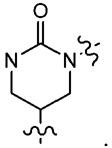

In some embodiments, Ring B is 6-membered heterocycle containing one N and one O as the ring heteroatoms, the remaining ring atoms being C.

In some embodiments, Ring B is

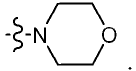

In some embodiments, Ring B is

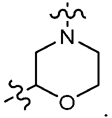

In some embodiments, Ring B is

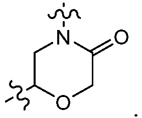

In some embodiments, Ring B is

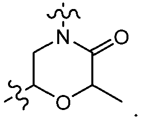

In some embodiments, Ring B is

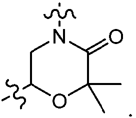

In some embodiments, Ring B is 6-membered heterocycle containing one N, one S and one O as the ring heteroatoms, the remaining ring atoms being C.

In some embodiments, Ring B is

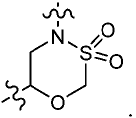

In some embodiments, Ring B is 7-membered heterocycle.

In some embodiments, Ring B is monocyclic 7-membered heterocycle.

In some embodiments, Ring B is monocyclic 7-membered heterocycle containing one N as the ring heteroatom, the remaining ring atoms being C.

In some embodiments, Ring B is

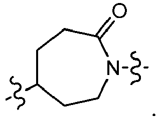

In some embodiments, Ring B is monocyclic 7-membered heterocycle containing two N as the ring heteroatoms, the remaining ring atoms being C.

In some embodiments, Ring B is

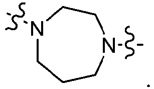

In some embodiments, Ring B is monocyclic 7-membered heterocycle containing one N and one O as the ring heteroatoms, the remaining ring atoms being C.

In some embodiments, Ring B is

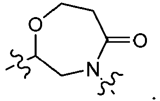

In some embodiments, Ring B is

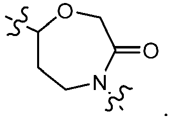

In some embodiments, Ring B is bicyclic 7-membered heterocycle.

In some embodiments, Ring B is bicyclic 7-membered heterocycle containing two N as the ring heteroatoms, the remaining ring atoms being C.

In some embodiments, Ring B is

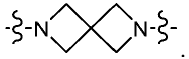

In some embodiments, Ring B is C₃-C₁₄ cycloalkyl, wherein cycloalkyl is optionally substituted with one or more substituents independently selected from halogen, CN, NO₂, oxo, OH, NR⁹R¹⁰, C₁-C₆ alkyl, C₁-C₆ alkoxy, aryl, heteroaryl, cycloalkyl, and heterocycle.

In some embodiments, Ring B is monocyclic cycloalkyl.

In some embodiments, Ring B is fused bicyclic cycloalkyl.

In some embodiments, Ring B is bridged bicyclic cycloalkyl.

In some embodiments, Ring B is spiro bicyclic cycloalkyl.

In some embodiments, Ring B is 3-membered cycloalkyl.

In some embodiments, Ring B is 4-membered cycloalkyl.

In some embodiments, Ring B is 5-membered cycloalkyl.

In some embodiments, Ring B is monocyclic 5-membered cycloalkyl.

In some embodiments, Ring B is bridged bicyclic 5-membered cycloalkyl.

In some embodiments, Ring B is

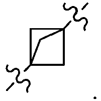

In some embodiments, Ring B is

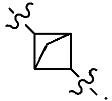

In some embodiments, Ring B is 6-membered cycloalkyl.

In some embodiments, Ring B is monocyclic 6-membered cycloalkyl.

In some embodiments, Ring B is

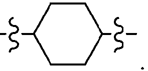

In some embodiments, Ring B is 7-membered cycloalkyl.

In some embodiments, Ring B is monocyclic 7-membered cycloalkyl.

In some embodiments, Ring B is

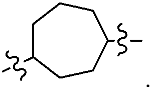

In some embodiments, R⁶ is selected from H, C₁-C₆ alkyl, -C(O)R⁷, -NHC(O)R⁷, S(O)ₛR¹¹, -NHS(O)ₛR¹¹, -NHS(O)₂NR⁹R¹⁰, 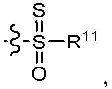 C₃-C₁₀ cycloalkyl, heterocyclyl, aryl, heteroaryl, C₁-C₆ alkanediyl C₃-C₁₀ cycloalkyl, C₁-C₆ alkanediyl heterocyclyl, C₁-C₆ alkanediyl aryl, and C₁-C₆ alkanediyl heteroaryl, wherein alkyl, cycloalkyl, aryl, or heteroaryl is optionally substituted with one or more substituents independently selected from halogen, CN, NO₂, oxo, OH, NR⁹R¹⁰, C₁-C₆ alkyl, C₁-C₆ alkoxy, cycloalkyl, heterocycle, aryl, and heteroaryl.

In some embodiments, R⁶ is H.

In some embodiments, R⁶ is C₁-C₆ alkyl, wherein alkyl is optionally substituted with one or more substituents independently selected from halogen, OH, CN, NR⁹R¹⁰, cycloalkyl, heterocyclyl, aryl, heteroaryl.

In some embodiments, R⁶ is C₁-C₆ alkyl.

In some embodiments, R⁶ is CH₃.

In some embodiments, R⁶ is C₁-C₆ alkanediyl aryl optionally substituted with one or more substituents independently selected from halogen, CN, NO₂, oxo, OH, NR⁹R¹⁰, C₁-C₆ alkyl, C₁-C₆ alkoxy, cycloalkyl, heterocycle, aryl, and heteroaryl.

In some embodiments, R⁶ is C₁-C₆ alkanediyl aryl optionally substituted with one C₁-C₆ alkoxy.

In some embodiments, R⁶ is

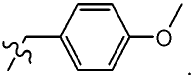

In some embodiments, R⁶ is C₂H₅.

In some embodiments, R⁶ is propyl.

In some embodiments, R⁶ is *n*-propyl.

In some embodiments, R⁶ is *i*-propyl.

In some embodiments, R⁶ is butyl.

In some embodiments, R⁶ is *n*-butyl.

In some embodiments, R⁶ is *i*-butyl.

In some embodiments, R⁶ is

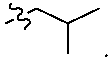

In some embodiments, R⁶ is *tert*-butyl.

In some embodiments, R⁶ is pentyl.

In some embodiments, R⁶ is hexyl.

In some embodiments, R⁶ is -C(O)R⁷.

In some embodiments, R⁷ is selected from R⁸, OR⁸, NR⁹R¹⁰.

In some embodiments, R⁷ is R⁸.

In some embodiments, R⁷ is OR⁸.

In some embodiments, R⁷ is NR⁹R¹⁰.

In some embodiments, R⁸ is selected from H, C₁-C₆ alkyl, C₂-C₆ alkenyl, C₂-C₆ alkynyl, C₃-C₁₀ cycloalkyl, wherein alkyl, alkenyl, alkynyl or cycloalkyl is optionally substituted with one or more substituents independently selected from halogen, OH, CN, NO₂, NR⁹R¹⁰.

In some embodiments, R⁸ is H.

In some embodiments, R⁸ is C₁-C₆ alkyl, wherein alkyl is optionally substituted with one or more substituents independently selected from halogen, OH, CN, NO₂, NR⁹R¹⁰.

In some embodiments, R⁸ is C-C₆ alkyl.

In some embodiments, R⁸ is -CH₃. In some embodiments, R⁸ is -C₂H₅. In some embodiments, R⁸ is -CH₂CH₂CH₃. In some embodiments, R⁸ is -CH(CH₃)₂. In some embodiments, R⁸ is -CH₂CH₂CH₂CH₃. In some embodiments, R⁸ is -CH₂CH(CH₃)₂. In some embodiments, R⁸ is pentyl. In some embodiments, R⁸ is hexyl.

In some embodiments, R⁸ is C₂-C₆ alkenyl, wherein alkenyl is optionally substituted with one or more substituents independently selected from halogen, OH, CN, NO₂, NR⁹R¹⁰.

In some embodiments, R⁸ is C₂-C₆ alkenyl.

In some embodiments, R⁸ is -CH=CH₂.

In some embodiments, R⁸ is -CH=CH-CH₃.

In some embodiments, R⁸ is -CH=CH-CH₂-NR⁹R¹⁰.

In some embodiments, R⁸ is

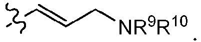

In some embodiments, R⁸ is

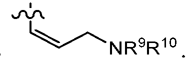

In some embodiments, R⁸ is C₂-C₆ alkynyl, wherein alkynyl is optionally substituted with one or more substituents independently selected from halogen, OH, CN, NO₂, NR⁹R¹⁰.

In some embodiments, R⁸ is C₃-C₁₀ cycloalkyl, wherein cycloalkyl is optionally substituted with one or more substituents independently selected from halogen, OH, CN, NO₂, NR⁹R¹⁰.

In some embodiments R⁶ is -NHC(O)R⁷.

In some embodiments, R⁷ is selected from R⁸, OR⁸, NR⁹R¹⁰

In some embodiments, R⁷ is NR⁹R¹⁰.

In some embodiments, R⁷ is NH₂.

In some embodiments, R⁷ is NHCH₃.

In some embodiments, R⁷ is R⁸.

In some embodiments, R⁸ is selected from H, C₁-C₆ alkyl, C₂-C₆ alkenyl, C₂-C₆ alkynyl, C₃-C₁₀ cycloalkyl, wherein alkyl, alkenyl, alkynyl or cycloalkyl is optionally substituted with one or more substituents independently selected from halogen, OH, CN, NO₂, NR⁹R¹⁰.

In some embodiments, R⁸ is H.

In some embodiments, R⁸ is C₁-C₆ alkyl, wherein alkyl is optionally substituted with one or more substituents independently selected from halogen, OH, CN, NO₂, NR⁹R¹⁰.

In some embodiments, R⁸ is C₁-C₆ alkyl.

In some embodiments, R⁸ methyl.

In some embodiments, R⁶ is S(O)ₛR¹¹.

In some embodiments, s is an integer selected from 0, 1, and 2.

In some embodiments, s is 0.

In some embodiments, s is 1.

In some embodiments, s is 2.

In some embodiments R⁶ is -SR¹¹.

In some embodiments, R⁶ is -S(O)R¹¹.

In some embodiments R⁶ is -S(O)₂R¹¹.

In some embodiments, R¹¹ is selected from C₁-C₆ alkyl, C₃-C₁₀ cycloalkyl, C₁-C₆ alkoxy, NR⁹R¹⁰.

In some embodiments, R¹¹ is C₁-C₆ alkyl.

In some embodiments, R¹¹ is methyl.

In some embodiments, R¹¹ is ethyl.

In some embodiments, R¹¹ is propyl.

In some embodiments, R¹¹ is *n*-propyl.

In some embodiments, R¹¹ is *i*-propyl.

In some embodiments, R¹¹ is butyl.

In some embodiments, R¹¹ is *n*-butyl.

In some embodiments, R¹¹ is *i*-butyl.

In some embodiments, R¹¹ is *tert*-butyl.

In some embodiments, R¹¹ is pentyl. In some embodiments, R¹¹ is hexyl.

In some embodiments, R¹¹ is C₃-C₁₀ cycloalkyl.

In some embodiments, R¹¹ is monocyclic C₃-C₁₀ cycloalkyl.

In some embodiments, R¹¹ is cyclopropyl.

In some embodiments, R¹¹ is cyclobutyl.

In some embodiments, R¹¹ is cyclopentyl.

In some embodiments, R¹¹ is cyclohexyl.

In some embodiments, R¹¹ is cycloheptyl.

In some embodiments, R¹¹ is bicyclic C₅-C₁₀ cycloalkyl.

In some embodiments, R¹¹ is bicyclic fused C₅-C₁₀ cycloalkyl.

In some embodiments, R¹¹ is bicyclic bridged C₅-C₁₀ cycloalkyl.

In some embodiments, R¹¹ is bicyclic spiro C₅-C₁₀ cycloalkyl.

In some embodiments, R¹¹ is C₁-C₆ alkoxy.

In some embodiments, R¹¹ is NR⁹R¹⁰.

In some embodiments, R⁶ is -S(O)₂-C₁-C₆-alkyl.

In some embodiments, R⁶ is -S(O)₂CH₃.

In some embodiments, R⁶ is -S(O)₂CH₂CH₃.

In some embodiments, R⁶ is -S(O)₂(CH₂)₂CH₃.

In some embodiments, R⁶ is -S(O)₂(CH₂)₃CH₃.

In some embodiments, R⁶ is -S(O)₂CH(CH₃)₂.

In some embodiments, R⁶ is

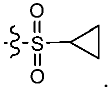

In some embodiments, R⁶ is -NHS(O)₂R¹¹.

In some embodiments, R⁶ is -NHS(O)₂CH₃.

In some embodiments, R⁶ is -NHS(O)₂CH₂CH₃.

In some embodiments, R⁶ is -NHS(O)₂NR⁹R¹⁰.

In some embodiments, R⁶ is -NHS(O)₂NH₂.

In some embodiments, R⁶ is

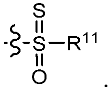

In some embodiments, R⁶ is

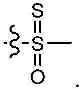

In some embodiments, L⁴ is selected from the **Table 1**.

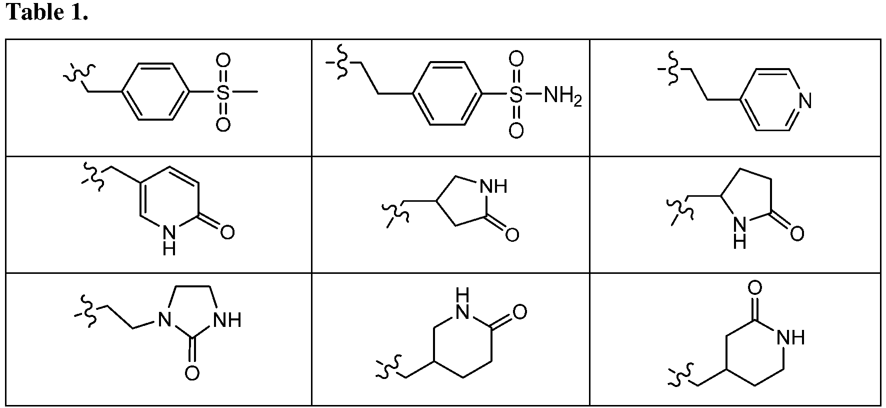

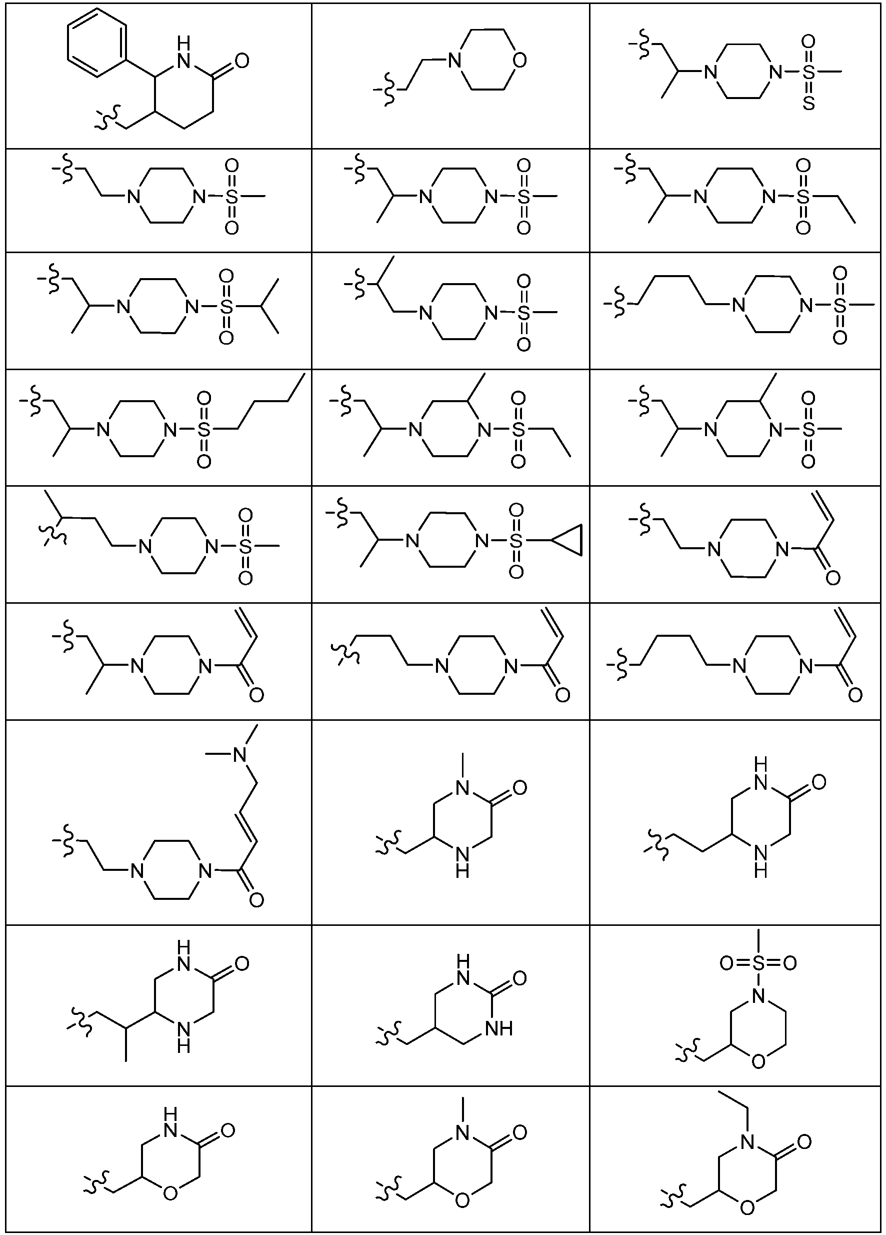

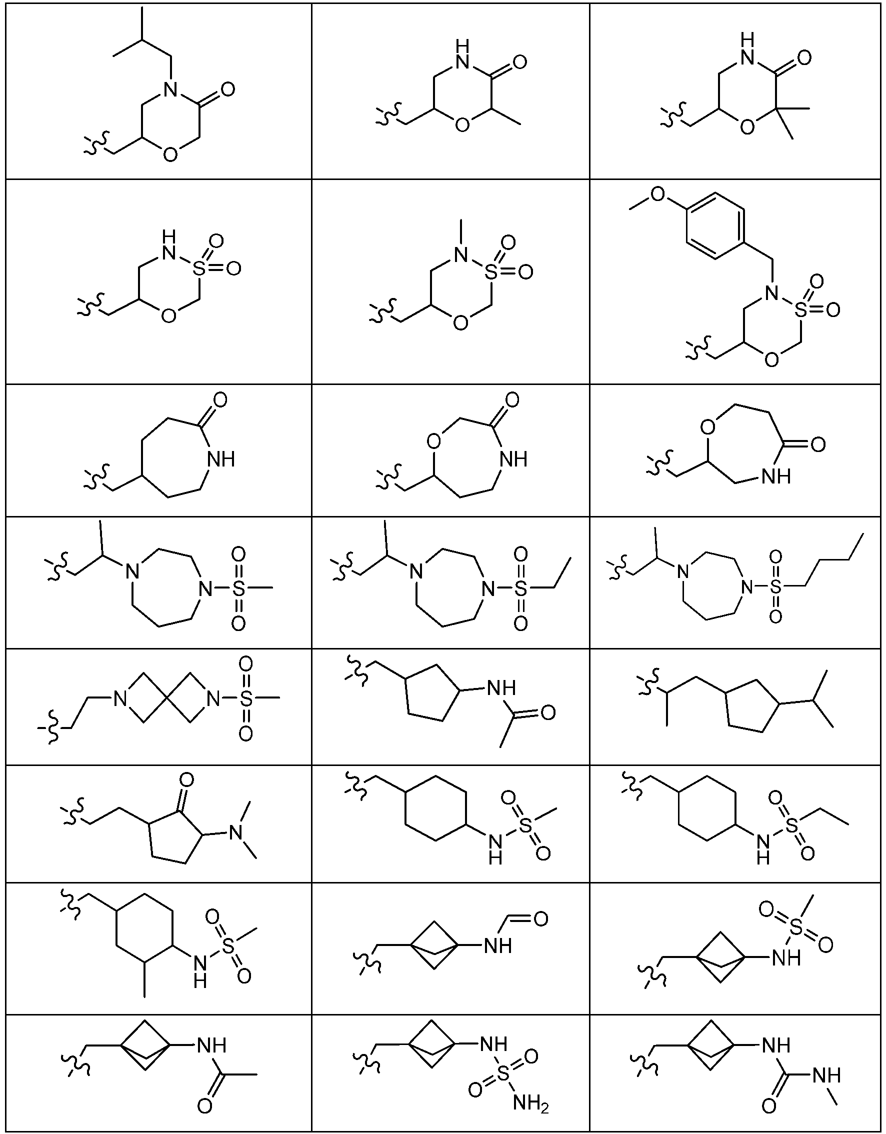

In some embodiments, R⁴ is selected from H, C₁-C₆ alkyl, C₂-C₆ alkenyl, C₂-C₆ alkynyl, C₃-C₁₀ cycloalkyl, C₁-C₆ alkyl-C₁-C₆ alkoxy, C(O)R⁷, aryl, heteroaryl, or heterocycle, wherein the alkyl, alkenyl, alkynyl, cycloalkyl, aryl, heteroaryl, or heterocycle is optionally substituted with one or more substituents independently selected from halogen, OH, NR⁹R¹⁰, C₁-C₆ alkyl, and C₁-C₆ alkoxy.

In some embodiments, R⁴ is H.

In some embodiments, R⁴ is C₁-C₆ alkyl.

In some embodiments, R⁴ is methyl.

In some embodiments, W is -CN.

In some embodiments, W, and ring B together with the atoms to which they are attached and any intervening atoms, form a 5-10 membered heterocycle.

In some embodiments, the compound is of Formula I-31:

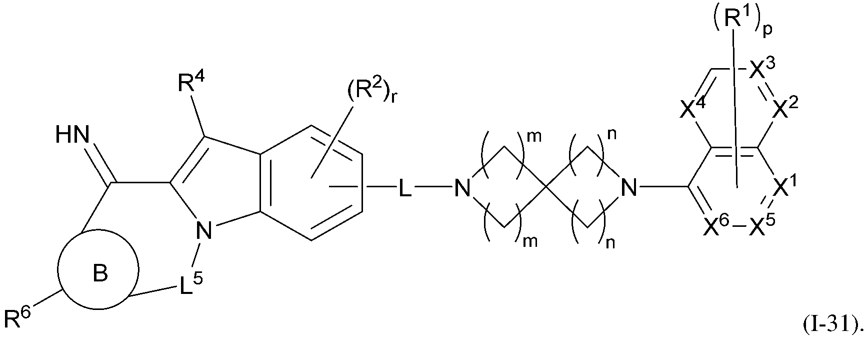

In some embodiments, the compound is of Formula I-32:

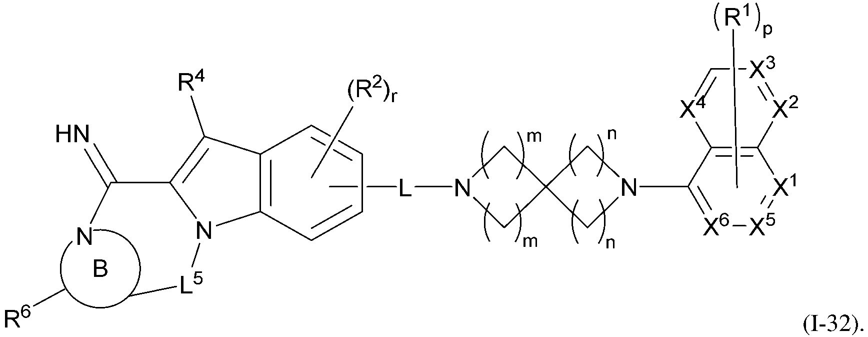

In some embodiments, the compound is of Formula I-33: 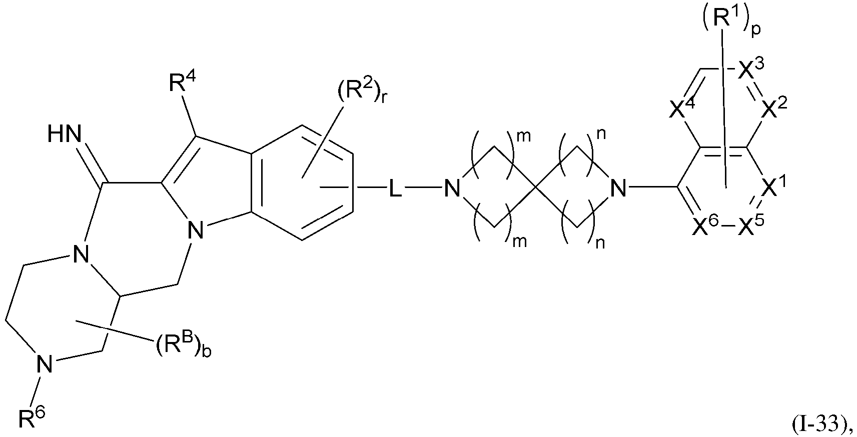 wherein R^{B} is selected from halogen, CN, NO₂, oxo, OH, NR⁹R¹⁰, C₁-C₆ alkyl, C₁-C₆ alkoxy, aryl, heteroaryl, cycloalkyl, and heterocycle; b is an integer selected from 0, 1, 2, 3, 4; and all other variables are as defined herein.

In some embodiments, m and n are each an integer independently selected from 1, 2, and 3.

In some embodiments, each m is 1.

In some embodiments, each m is 2.

In some embodiments, each m is 3.

In some embodiments, each n is 1.

In some embodiments, each n is 2.

In some embodiments, each n is 3.

In some embodiments, the value of m and n is selected from the **Table 2**

**Table 2**

| Option | m | n |
|---|---|---|
| 1 | 1 | 1 |
| 2 | 1 | 2 |
| 3 | 1 | 3 |
| 4 | 2 | 1 |
| 5 | 2 | 2 |
| 6 | 2 | 3 |
| 7 | 3 | 1 |
| 8 | 3 | 2 |
| 9 | 3 | 3 |

In some embodiments, p is an integer selected from 0, 1, and 2.

In some embodiments, p is 0.

In some embodiments, p is 1.

In some embodiments, p is 2.

In some embodiments, r is an integer selected from 0, 1, 2 and 3.

In some embodiments, r is 0.

In some embodiments, r is 1.

In some embodiments, r is 2.

In some embodiments, r is 3.

In some embodiments, the compound is of Formula (I-A): 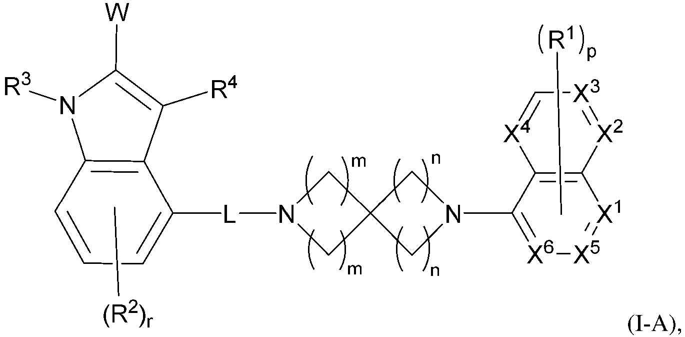 or a pharmaceutically acceptable salt, stereoisomer, solvate, prodrug, isotopic derivative, or tautomer thereof wherein all variables are as defined herein.

In some embodiments, the compound is of Formula (I-A'): 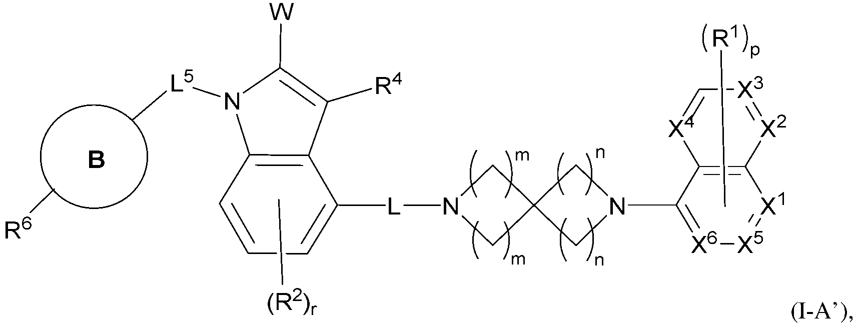 or a pharmaceutically acceptable salt, stereoisomer, solvate, prodrug, isotopic derivative, or tautomer thereof wherein all variables are as defined herein.

In some embodiments, the compound is of Formula (I-B): 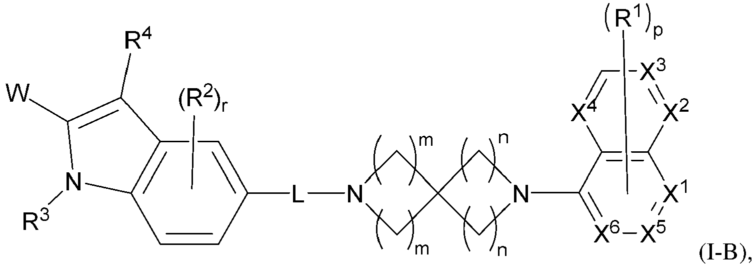 or a pharmaceutically acceptable salt, stereoisomer, solvate, prodrug, isotopic derivative, or tautomer thereof wherein all variables are as defined herein.

In some embodiments, the compound is of Formula (I-B'): 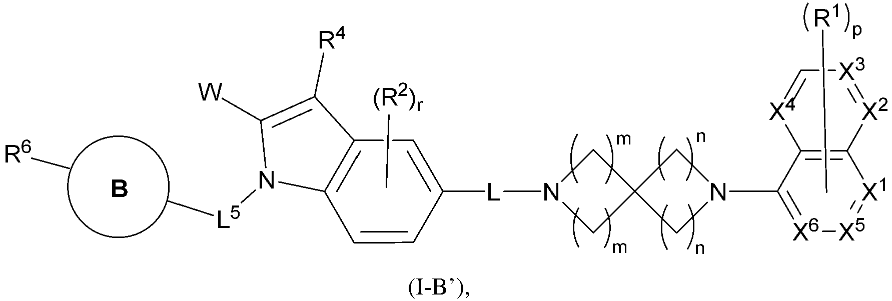 or a pharmaceutically acceptable salt, stereoisomer, solvate, prodrug, isotopic derivative, or tautomer thereof wherein all variables are as defined herein.

In some embodiments, the compound is of Formula (I-C): 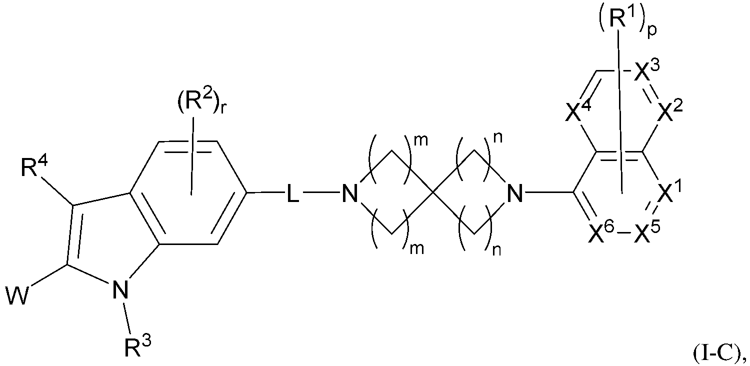 or a pharmaceutically acceptable salt, stereoisomer, solvate, prodrug, isotopic derivative, or tautomer thereof wherein all variables are as defined herein.

In some embodiments, the compound is of Formula (I-C'): 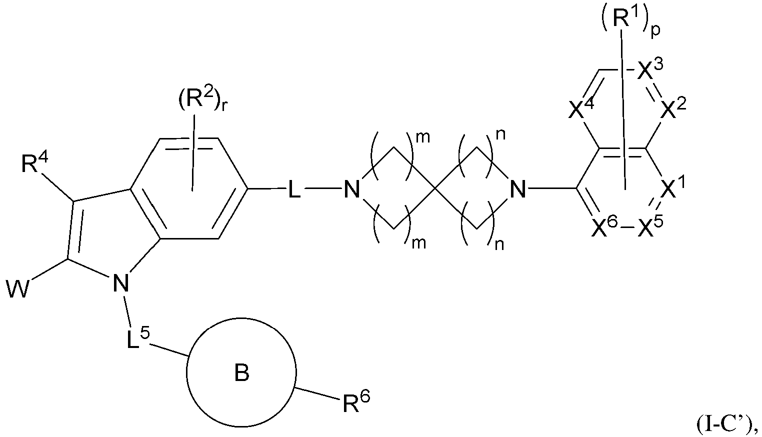 or a pharmaceutically acceptable salt, stereoisomer, solvate, prodrug, isotopic derivative, or tautomer thereof wherein all variables are as defined herein.

In some embodiments, the compound is of Formula (I-D): 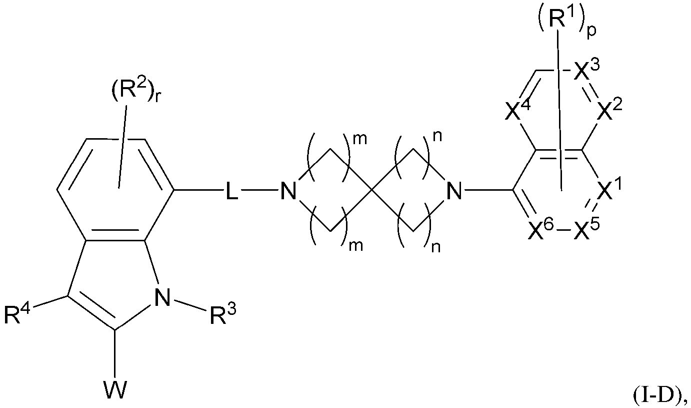 or a pharmaceutically acceptable salt, stereoisomer, solvate, prodrug, isotopic derivative, or tautomer thereof wherein all variables are as defined herein.

In some embodiments, the compound is of Formula (I-D'): 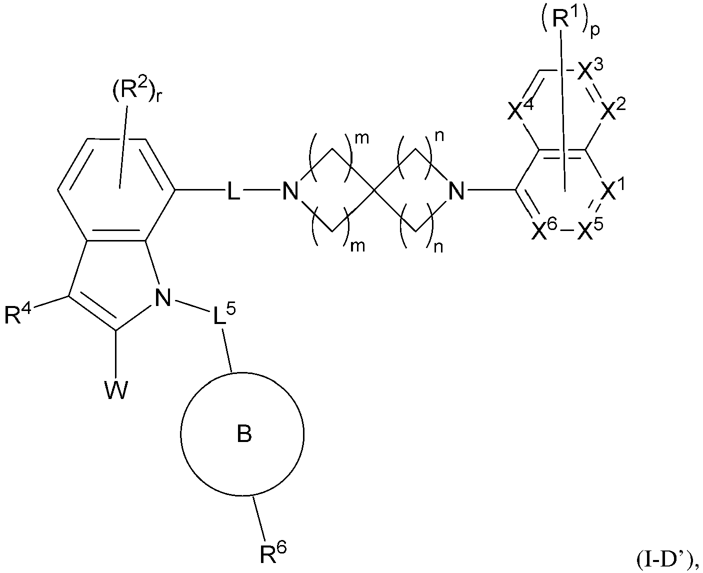 or a pharmaceutically acceptable salt, stereoisomer, solvate, prodrug, isotopic derivative, or tautomer thereof wherein all variables are as defined herein.

In some embodiments, the compound is of Formula (I-I): 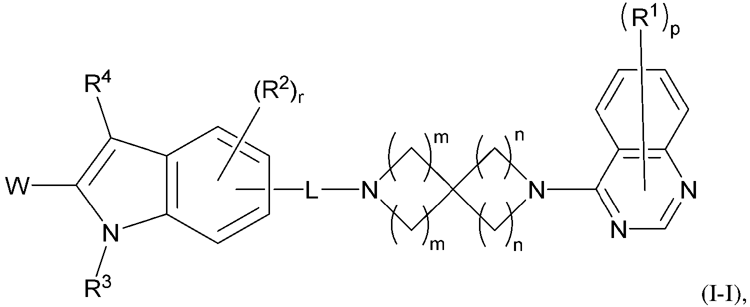 or a pharmaceutically acceptable salt, stereoisomer, solvate, prodrug, isotopic derivative, or tautomer thereof wherein all variables are as defined herein.

In some embodiments, the compound is of Formula (I-I'): 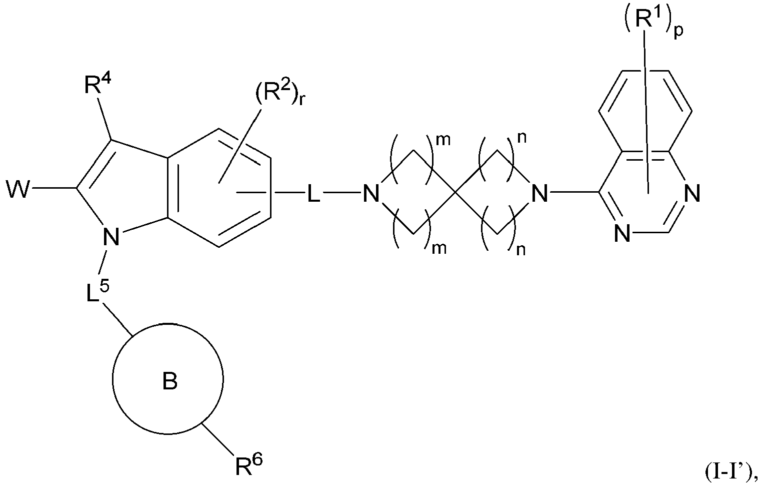 or a pharmaceutically acceptable salt, stereoisomer, solvate, prodrug, isotopic derivative, or tautomer thereof wherein all variables are as defined herein.

In some embodiments, the compound is of Formula (I-II): 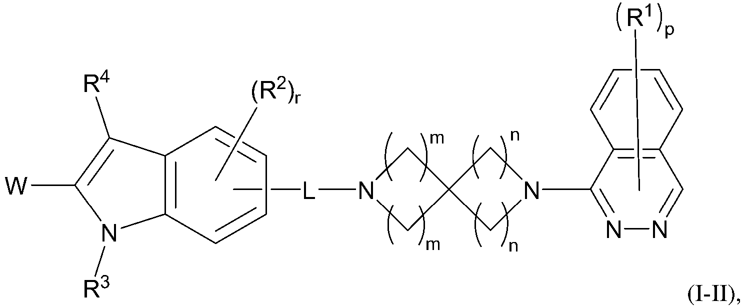 or a pharmaceutically acceptable salt, stereoisomer, solvate, prodrug, isotopic derivative, or tautomer thereof wherein all variables are as defined herein.

In some embodiments, the compound is of Formula (I-II'): 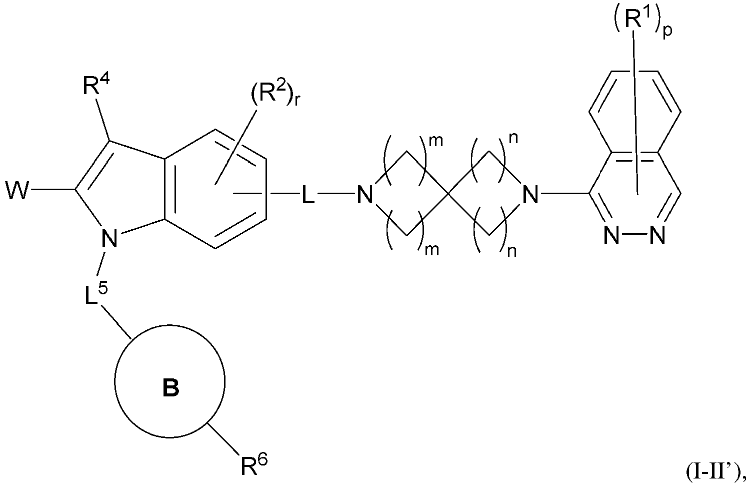 or a pharmaceutically acceptable salt, stereoisomer, solvate, prodrug, isotopic derivative, or tautomer thereof wherein all variables are as defined herein.

In some embodiments, the compound is of Formula (I-III): 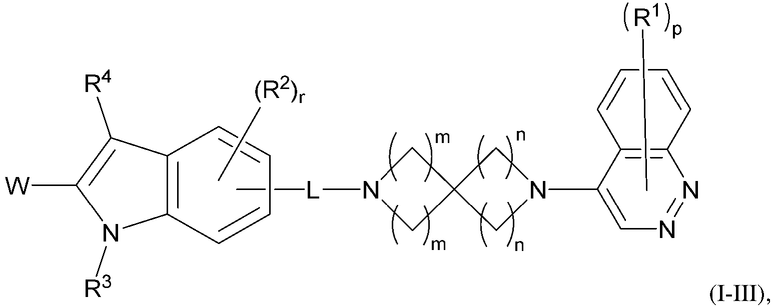 or a pharmaceutically acceptable salt, stereoisomer, solvate, prodrug, isotopic derivative, or tautomer thereof wherein all variables are as defined herein.

In some embodiments, the compound is of Formula (I-III'): 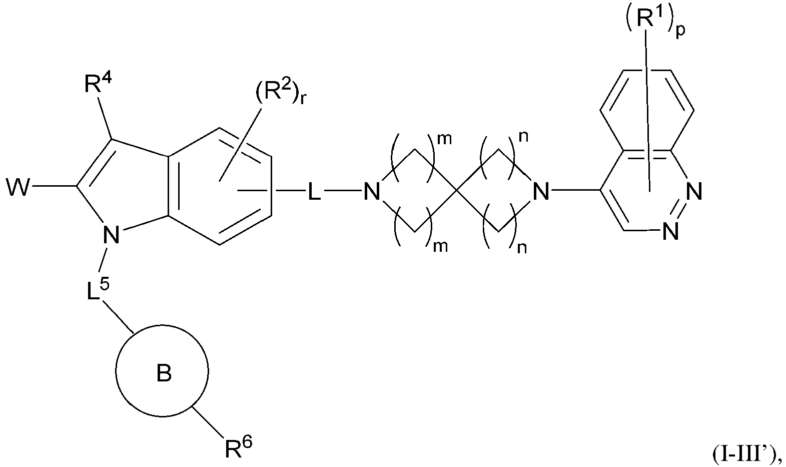 or a pharmaceutically acceptable salt, stereoisomer, solvate, prodrug, isotopic derivative, or tautomer thereof wherein all variables are as defined herein.

In some embodiments, the compound is of Formula (I-IV): 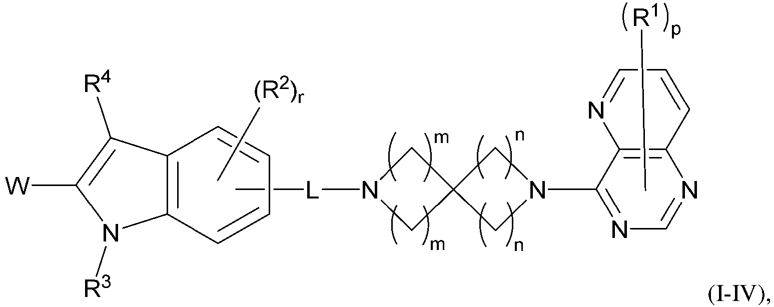 or a pharmaceutically acceptable salt, stereoisomer, solvate, prodrug, isotopic derivative, or tautomer thereof wherein all variables are as defined herein.

In some embodiments, the compound is of Formula (I-IV'): 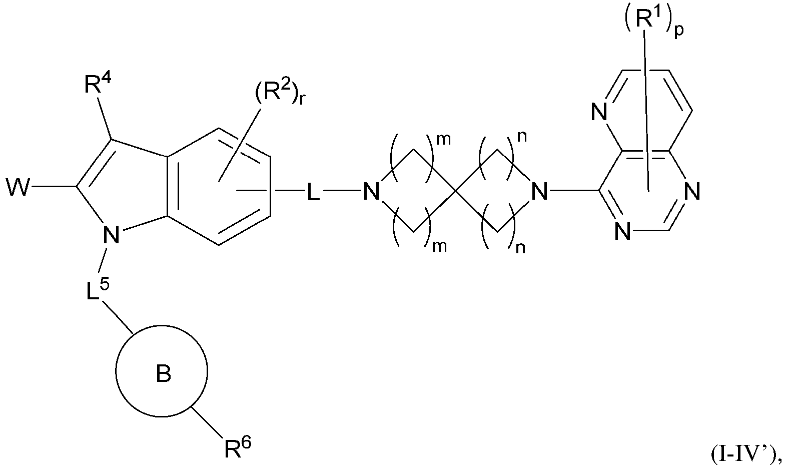 or a pharmaceutically acceptable salt, stereoisomer, solvate, prodrug, isotopic derivative, or tautomer thereof wherein all variables are as defined herein.

In some embodiments, the compound is of Formula (I-V): 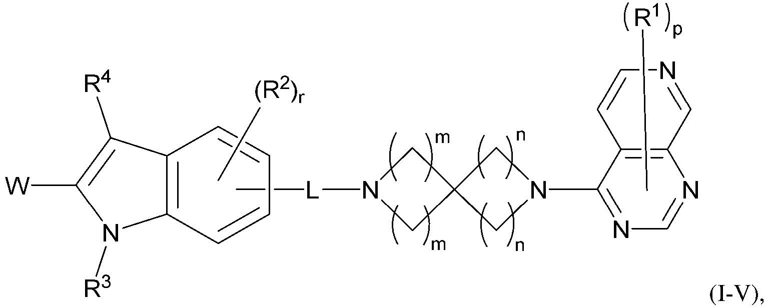 or a pharmaceutically acceptable salt, stereoisomer, solvate, prodrug, isotopic derivative, or tautomer thereof wherein all variables are as defined herein.

In some embodiments, the compound is of Formula (I-V'): 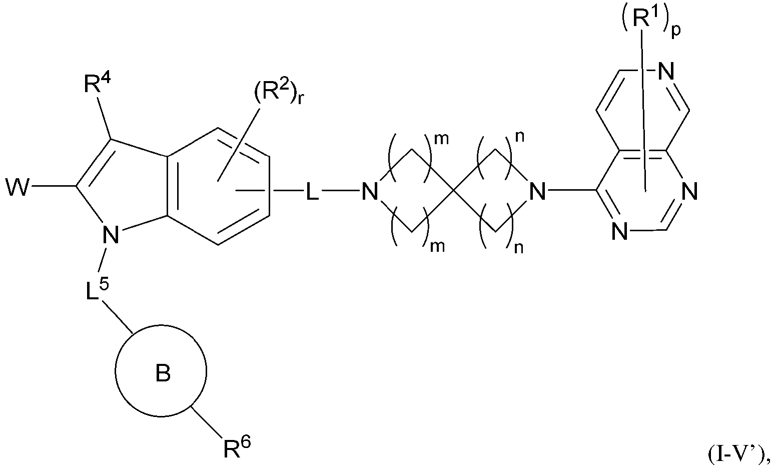 or a pharmaceutically acceptable salt, stereoisomer, solvate, prodrug, isotopic derivative, or tautomer thereof wherein all variables are as defined herein.

In some embodiments, the compound is of Formula (I-VI): 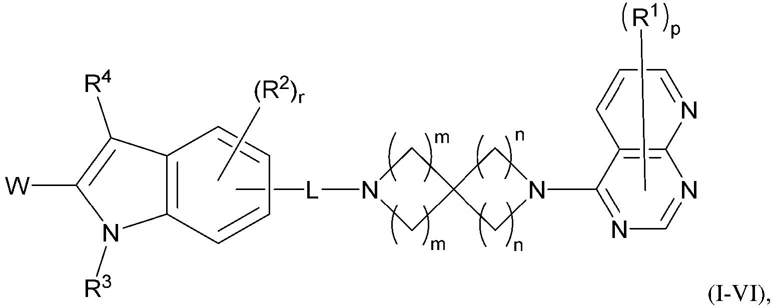 or a pharmaceutically acceptable salt, stereoisomer, solvate, prodrug, isotopic derivative, or tautomer thereof wherein all variables are as defined herein.

In some embodiments, the compound is of Formula (I-VI'): 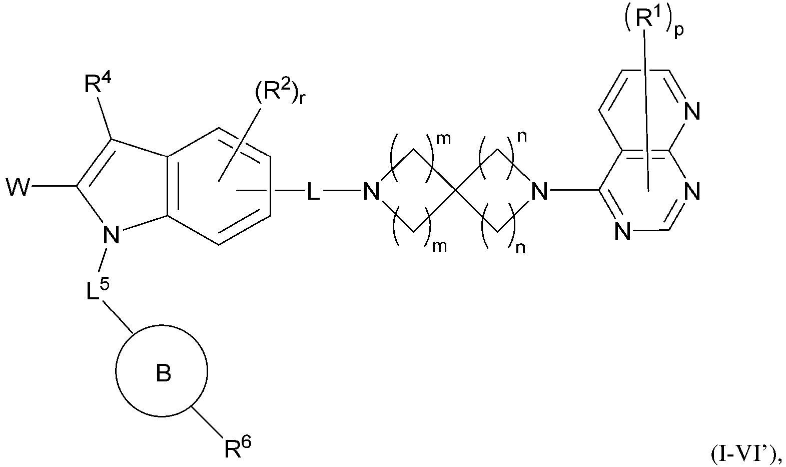 or a pharmaceutically acceptable salt, stereoisomer, solvate, prodrug, isotopic derivative, or tautomer thereof wherein all variables are as defined herein.

In some embodiments, the compound is of Formula (I-VII): 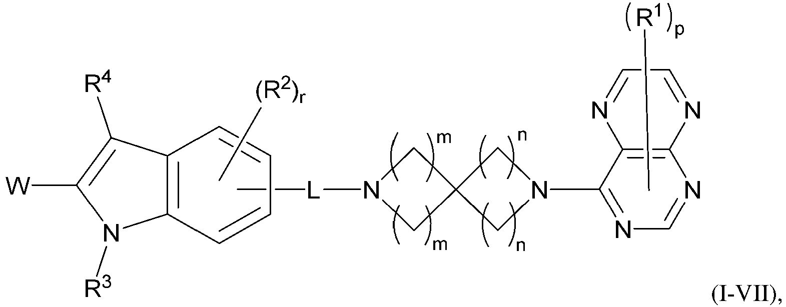 or a pharmaceutically acceptable salt, stereoisomer, solvate, prodrug, isotopic derivative, or tautomer thereof wherein all variables are as defined herein.

In some embodiments, the compound is of Formula (I-VII'): 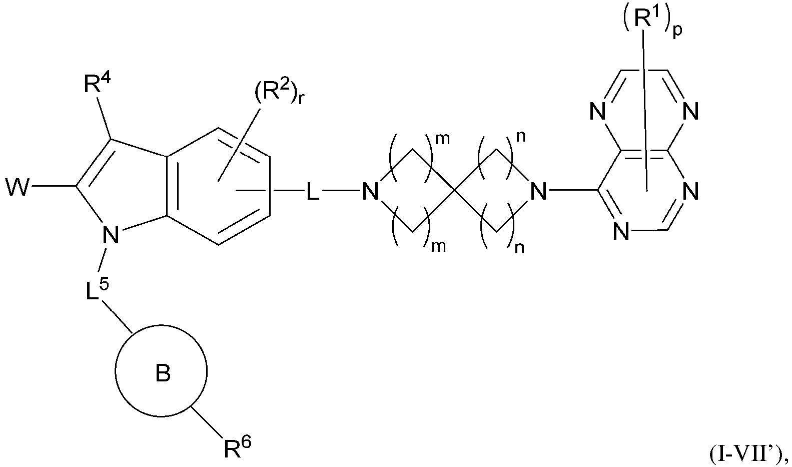 or a pharmaceutically acceptable salt, stereoisomer, solvate, prodrug, isotopic derivative, or tautomer thereof wherein all variables are as defined herein.

In some embodiments, the compound is of Formula (I-a): 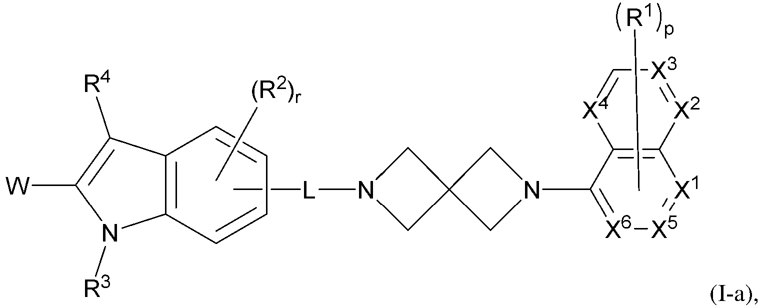 or a pharmaceutically acceptable salt, stereoisomer, solvate, prodrug, isotopic derivative, or tautomer thereof wherein all variables are as defined herein.

In some embodiments, the compound is of Formula (I-b): 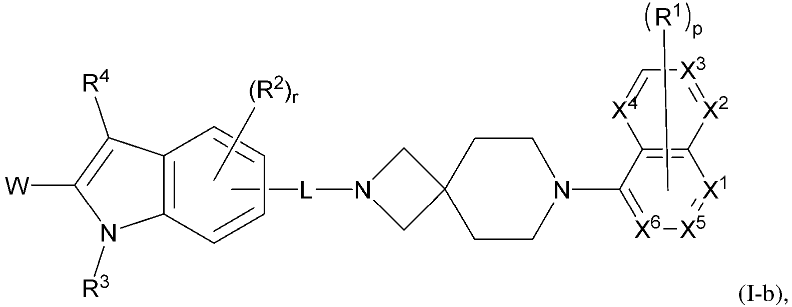 or a pharmaceutically acceptable salt, stereoisomer, solvate, prodrug, isotopic derivative, or tautomer thereof wherein all variables are as defined herein.

In some embodiments, the compound is of Formula (I-c): 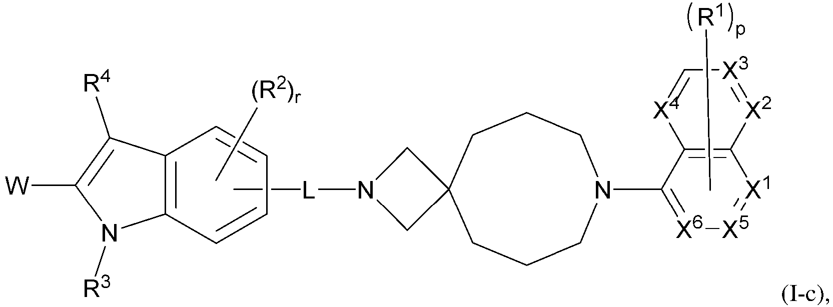 or a pharmaceutically acceptable salt, stereoisomer, solvate, prodrug, isotopic derivative, or tautomer thereof wherein all variables are as defined herein.

In some embodiments, the compound is of Formula (I-d): 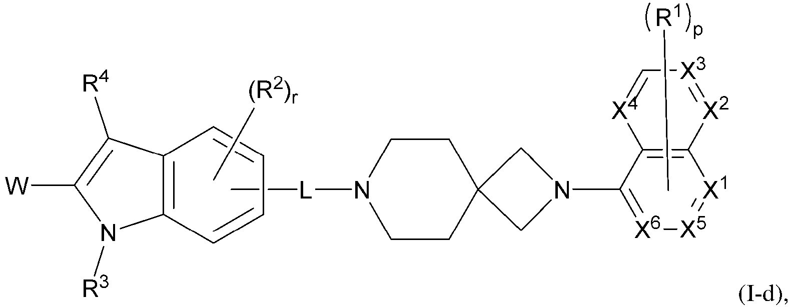 or a pharmaceutically acceptable salt, stereoisomer, solvate, prodrug, isotopic derivative, or tautomer thereof wherein all variables are as defined herein.

In some embodiments, the compound is of Formula (I-e): 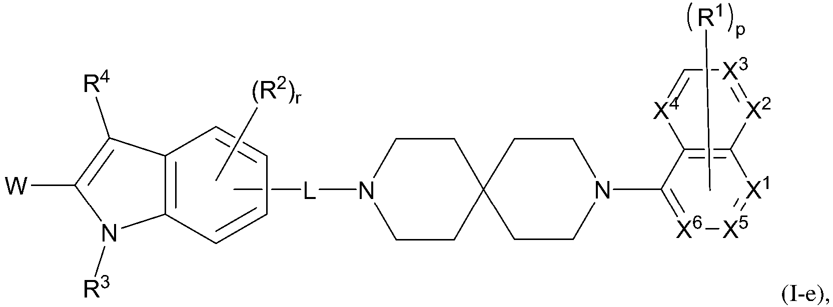 or a pharmaceutically acceptable salt, stereoisomer, solvate, prodrug, isotopic derivative, or tautomer thereof wherein all variables are as defined herein.

In some embodiments, the compound is of Formula (I-f): 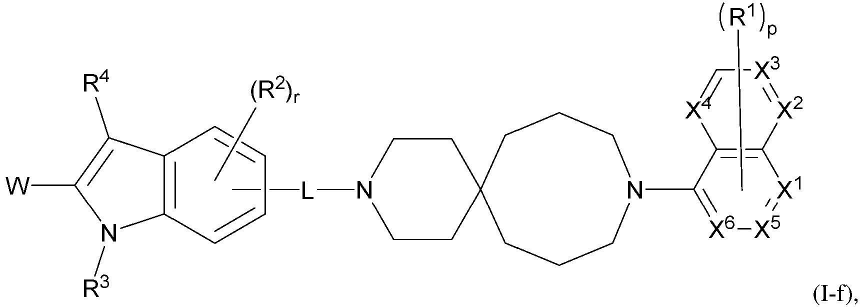 or a pharmaceutically acceptable salt, stereoisomer, solvate, prodrug, isotopic derivative, or tautomer thereof wherein all variables are as defined herein.

In some embodiments, the compound is of Formula (I-g): 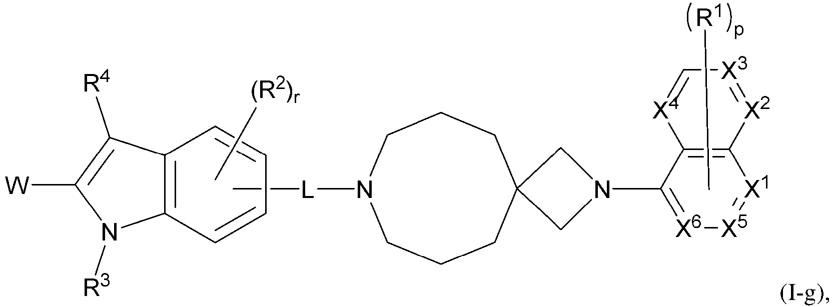 or a pharmaceutically acceptable salt, stereoisomer, solvate, prodrug, isotopic derivative, or tautomer thereof wherein all variables are as defined herein.

In some embodiments, the compound is of Formula (I-h): 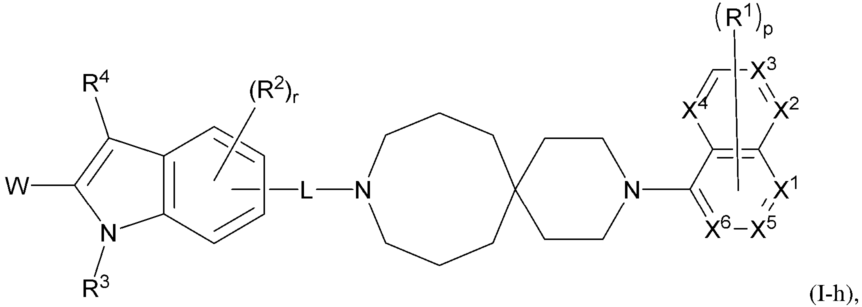 or a pharmaceutically acceptable salt, stereoisomer, solvate, prodrug, isotopic derivative, or tautomer thereof wherein all variables are as defined herein.

In some embodiments, the compound is of Formula (I-i): 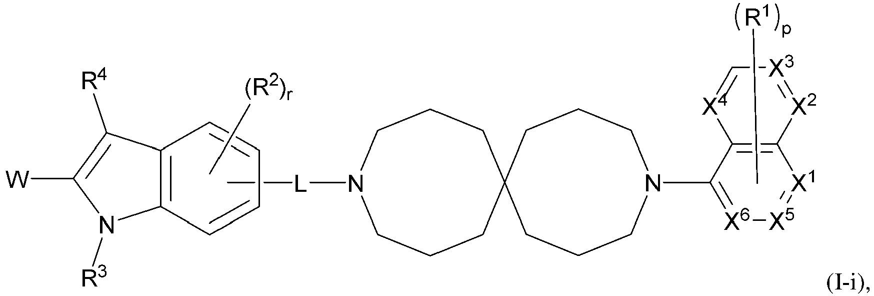 or a pharmaceutically acceptable salt, stereoisomer, solvate, prodrug, isotopic derivative, or tautomer thereof wherein all variables are as defined herein.

In some embodiments, the compound is selected from the **Table 3** or its pharmaceutically acceptable salt, stereoisomer, solvate, prodrug, isotopic derivative, or tautomer thereof wherein all variables are as defined herein.

**Table 3.**

| Compound # | Structure |
|---|---|
| I-A-I-a | 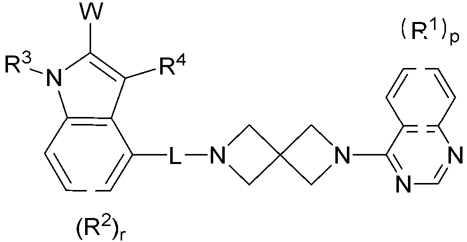 |
| I-A-I-b | 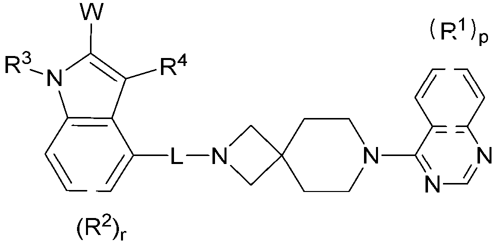 |
| I-A-I-c | 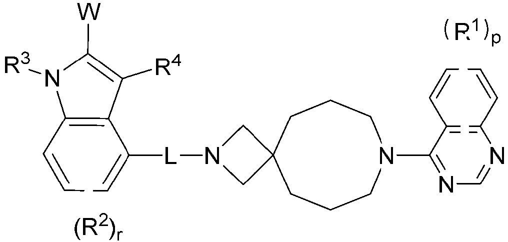 |
| I-A-1-d | 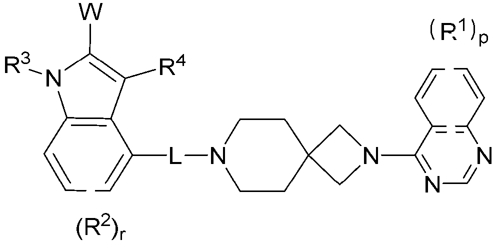 |
| I-A-I-e | 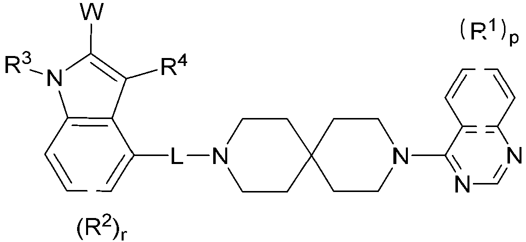 |
| I-A-1-f | 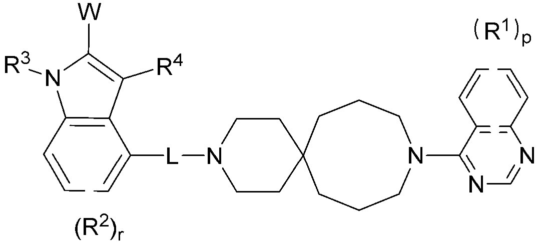 |
| I-A-I-g | 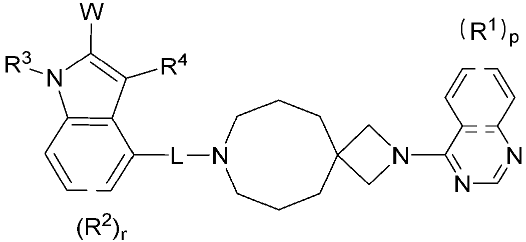 |
| I-A-I-h | 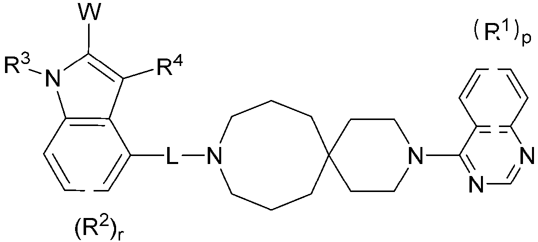 |
| I-A-I-i | 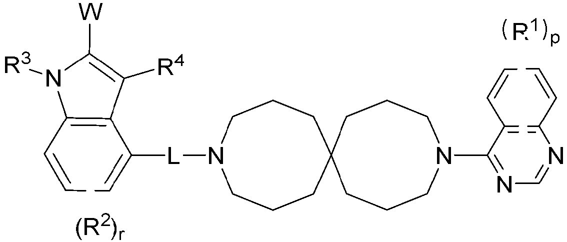 |
| I-A-II-a | 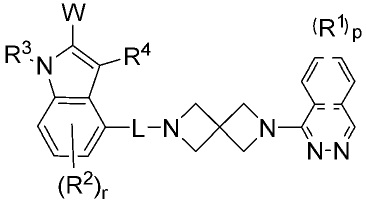 |
| I-A-II-b | 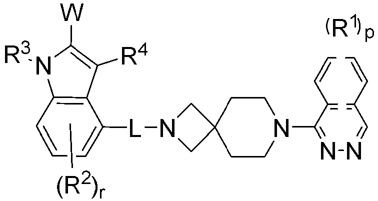 |
| I-A-II-c | 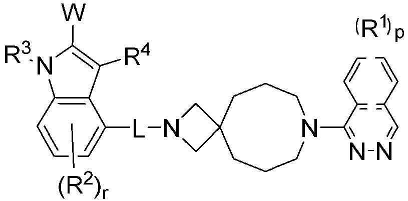 |
| I-A-II-d | 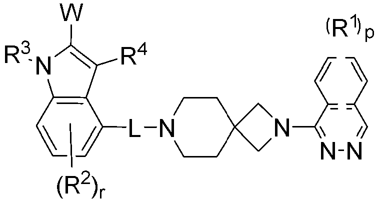 |
| I-A-II-e | 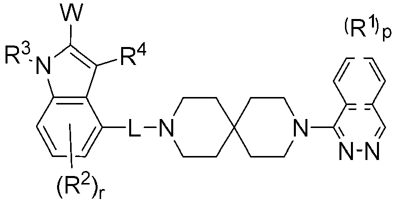 |
| I-A-II-f | 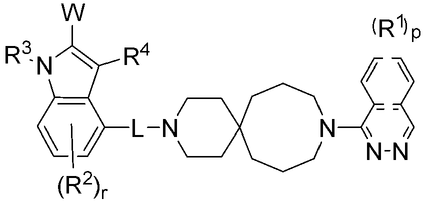 |
| I-A-II-g | 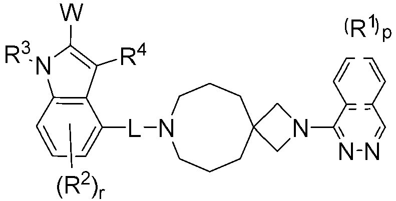 |
| I-A-II-h | 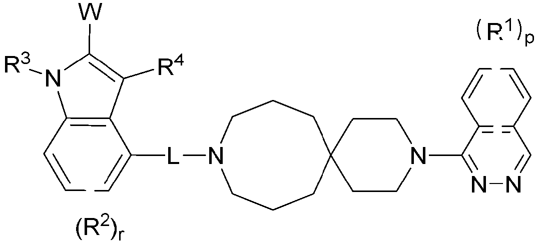 |
| I-A-II-i | 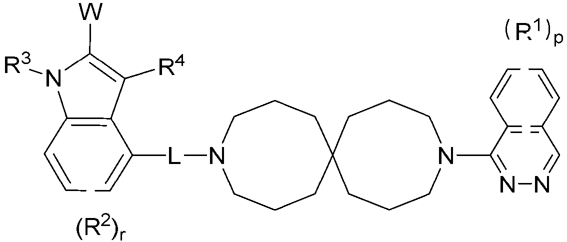 |
| I-A-III-a | 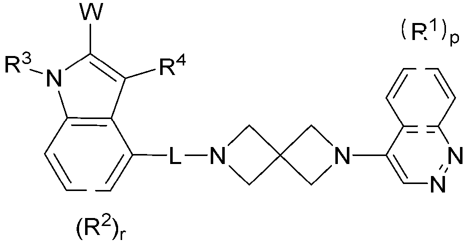 |
| I-A-III-b | 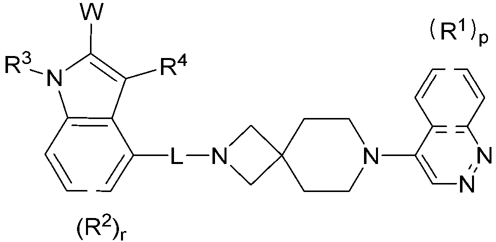 |
| I-A-III-c | 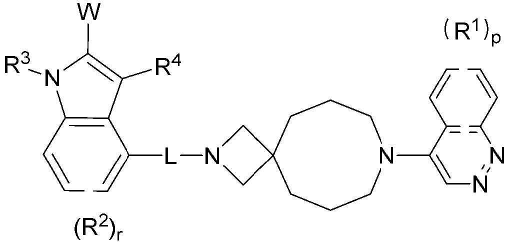 |
| I-A-III-d | 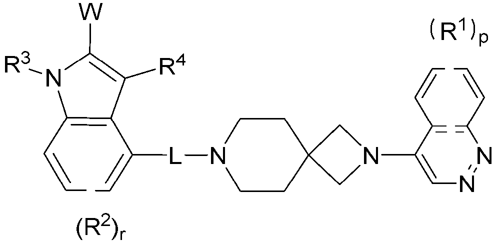 |
| I-A-III-e | 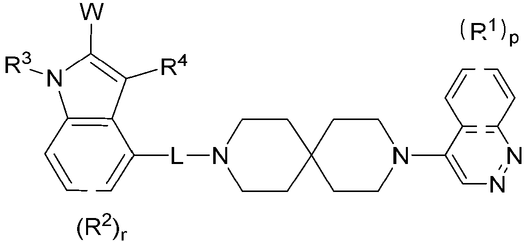 |
| I-A-III-f | 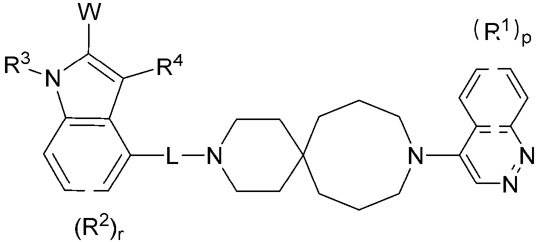 |
| I-A-III-g | 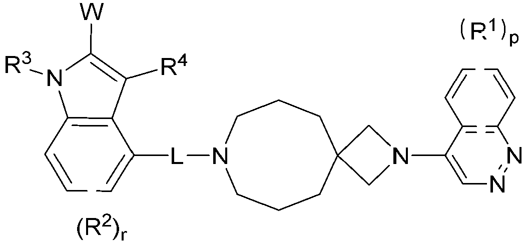 |
| I-A-III-h | 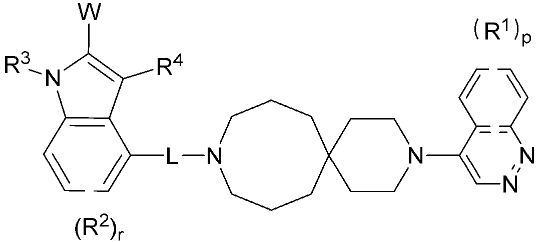 |
| I-A-III-i | 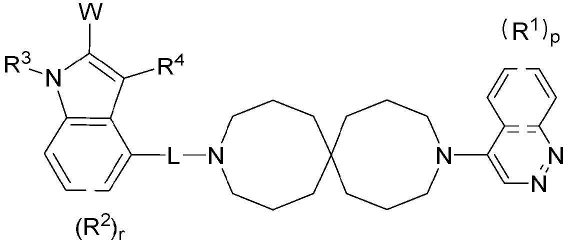 |
| I-A-IV-a | 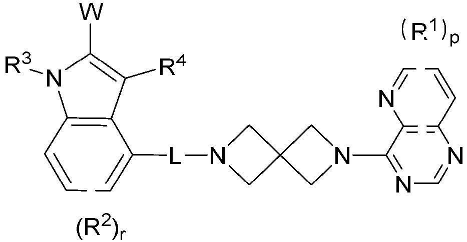 |
| I-A-IV-b | 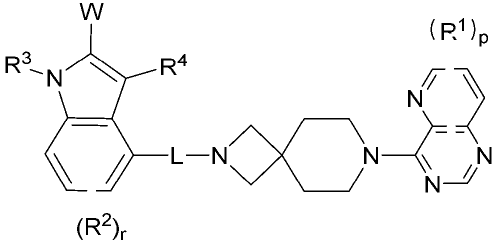 |
| I-A-IV-c | 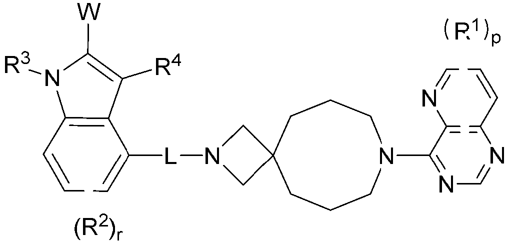 |
| I-A-IV-d | 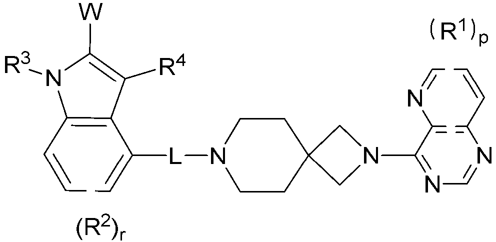 |
| I-A-1V-e | 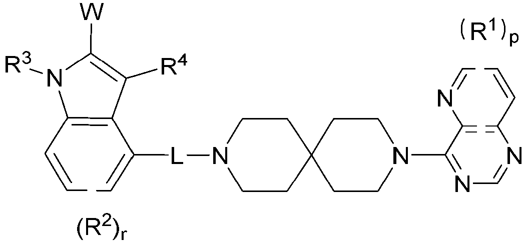 |
| I-A-IV-f | 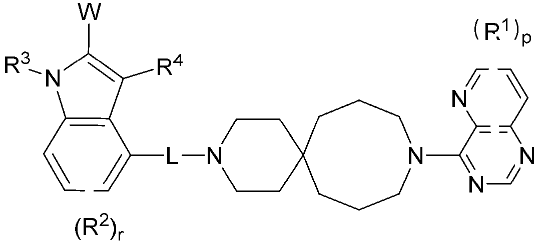 |
| I-A-IV-g | 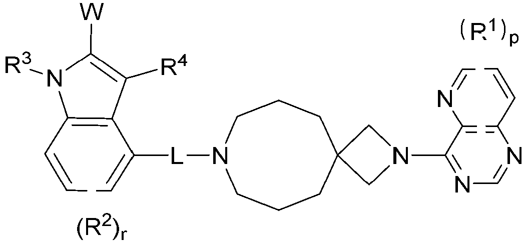 |
| I-A-IV-h | 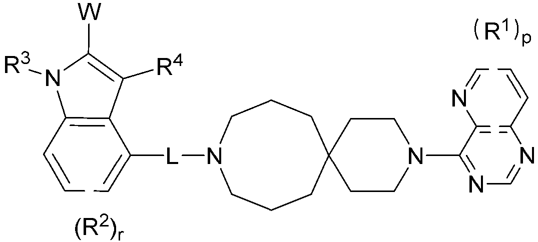 |
| I-A-IV-i | 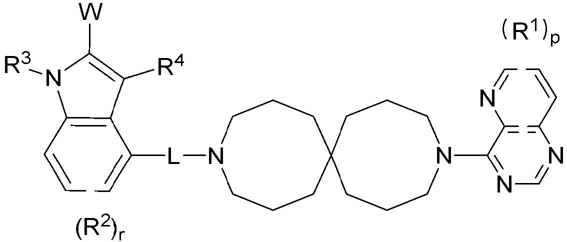 |
| I-A-V-a | 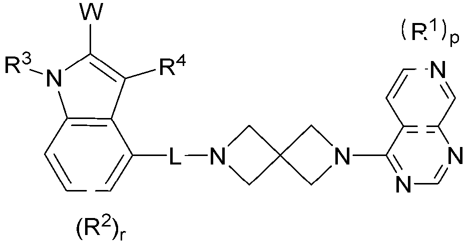 |
| I-A-V-b | 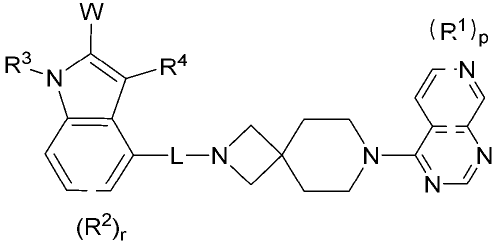 |
| I-A-V-c | 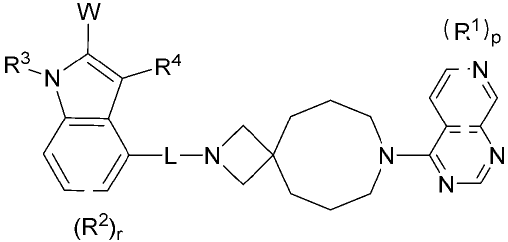 |
| I-A-V-d | 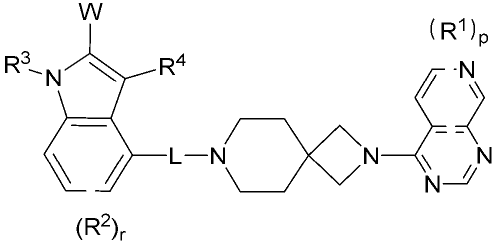 |
| I-A-V-e | 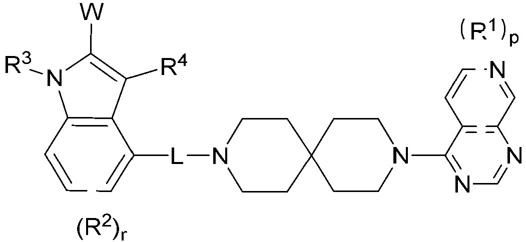 |
| I-A-V-f | 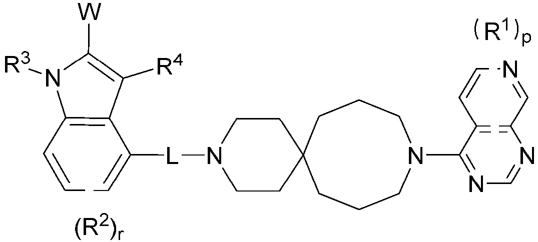 |
| I-A-V-g | 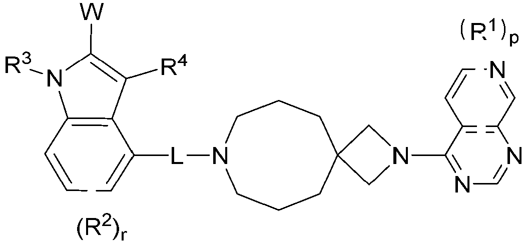 |
| I-A-V-h | 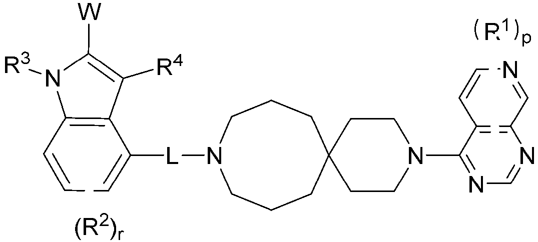 |
| I-A-V-i | 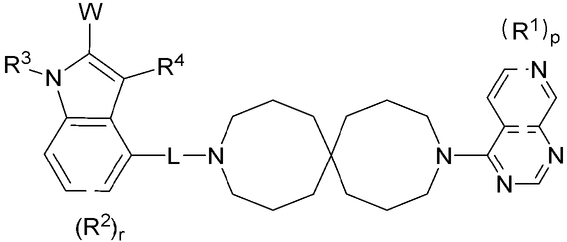 |
| I-A-VI-a | 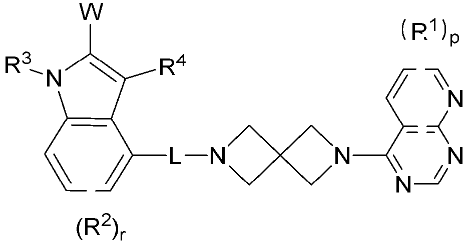 |
| I-A-VI-b | 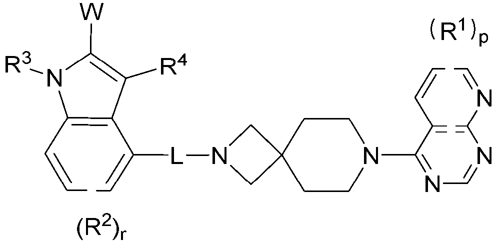 |
| I-A-VI-c | 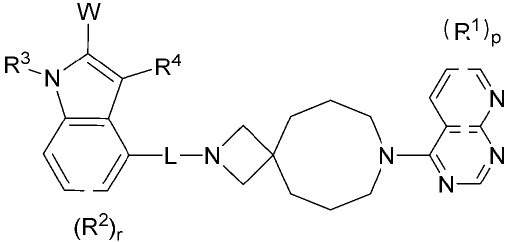 |
| I-A-VI-d | 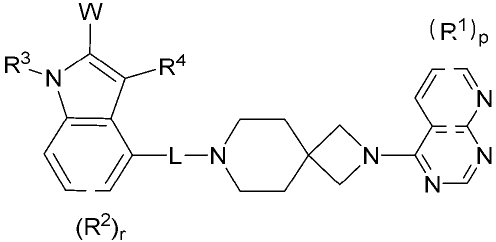 |
| I-A-VI-e | 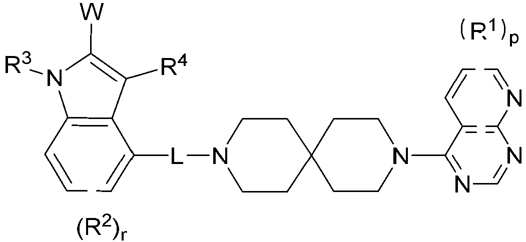 |
| I-A-VI-f | 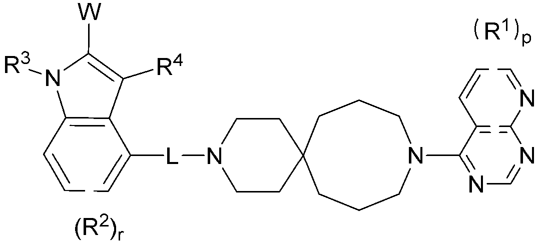 |
| I-A-VI-g | 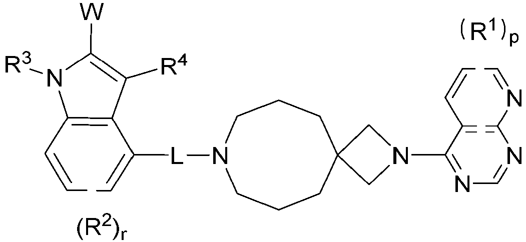 |
| I-A-VI-h | 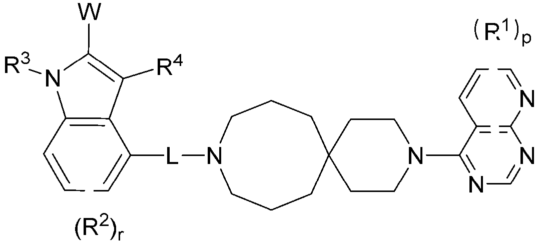 |
| I-A-VI-i | 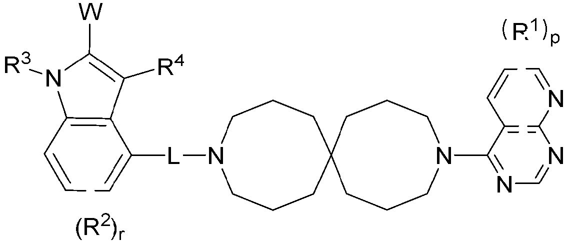 |
| I-A-VII-a | 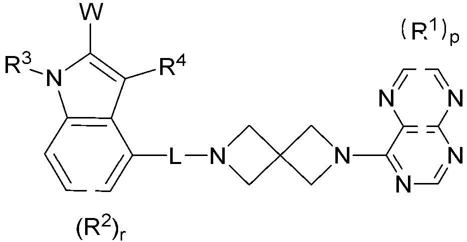 |
| I-A-VII-b | 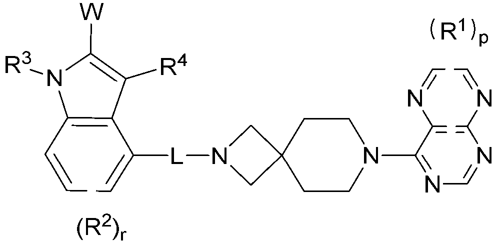 |
| I-A-VII-c | 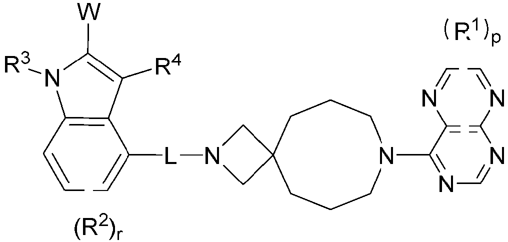 |
| I-A-VII-d | 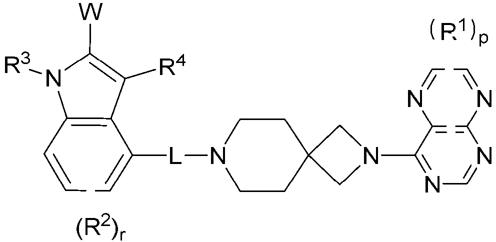 |
| I-A-VII-e | 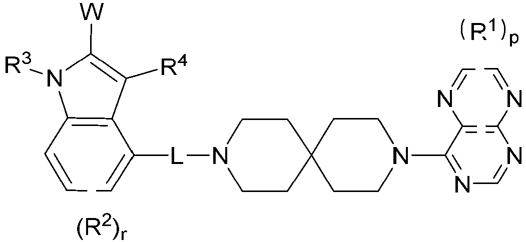 |
| I-A-VII-f | 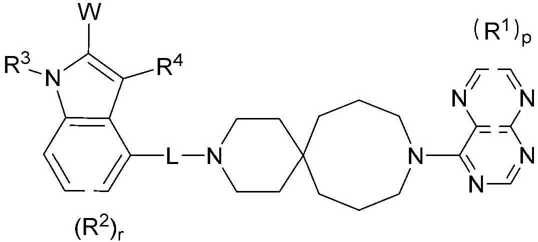 |
| I-A-VII-g | 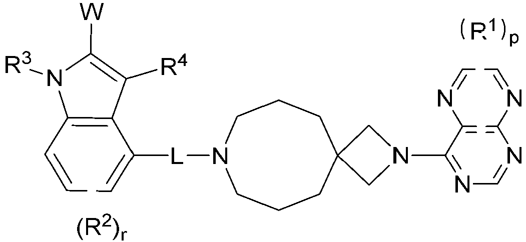 |
| I-A-VII-h | 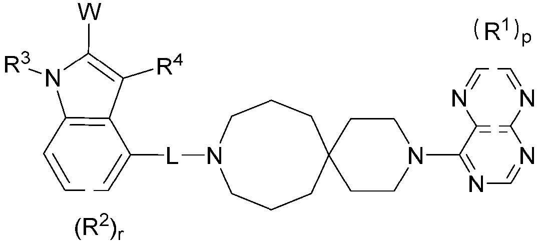 |
| I-A-VII-i | 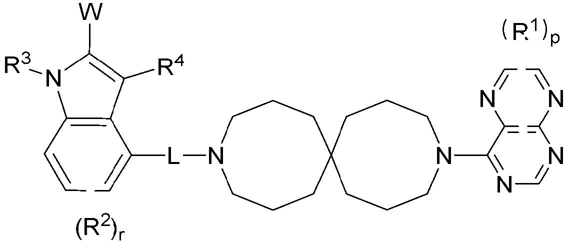 |
| I-B-I-a | 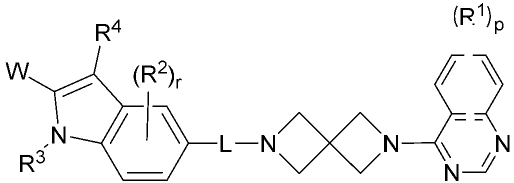 |
| I-B-I-b | 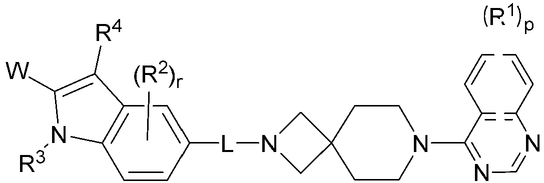 |
| I-B-I-c | 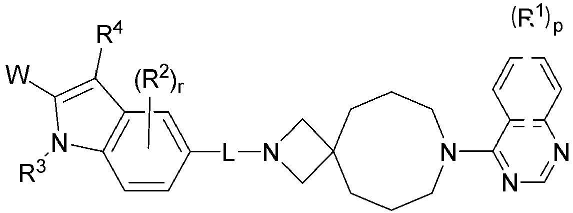 |
| I-B-I-d | 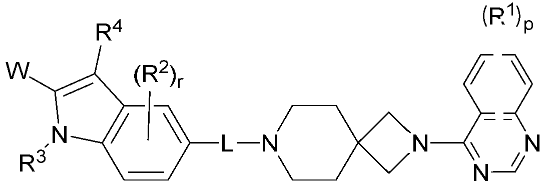 |
| I-B-I-e | 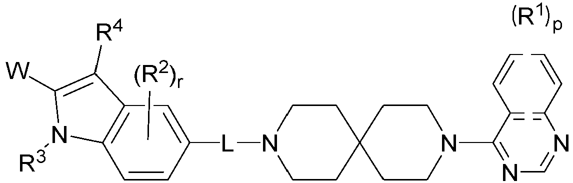 |
| I-B-I-f | 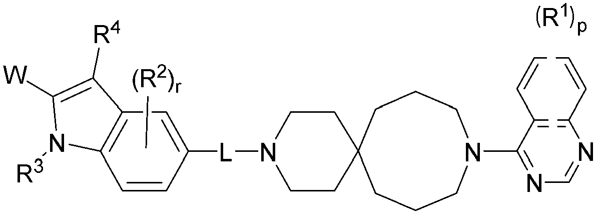 |
| I-B-I-g | 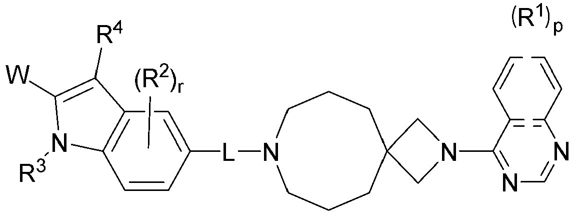 |
| I-B-I-h | 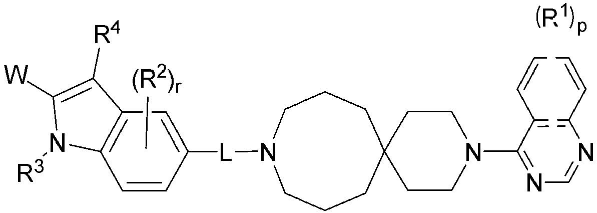 |
| I-B-I-i | V 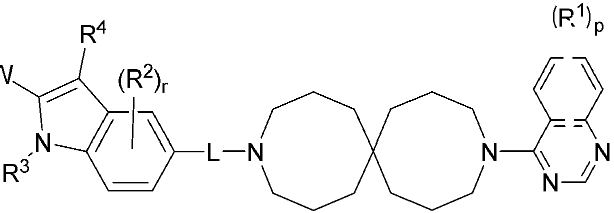 |
| I-B-II-a | 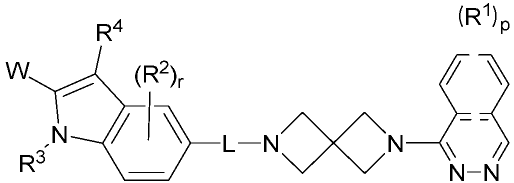 |
| I-B-II-b | 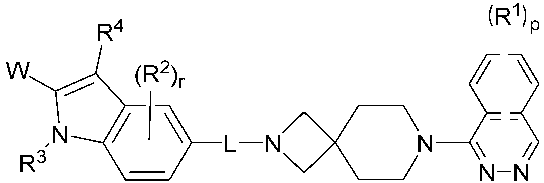 |
| I-B-II-c | 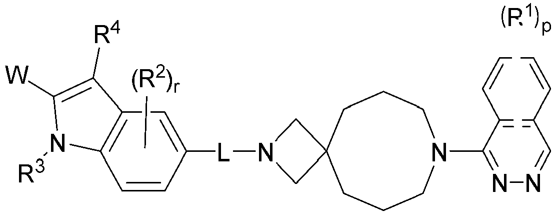 |
| I-B-II-d | 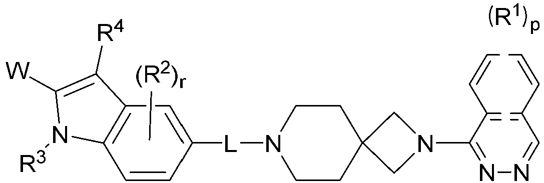 |
| I-B-II-e | 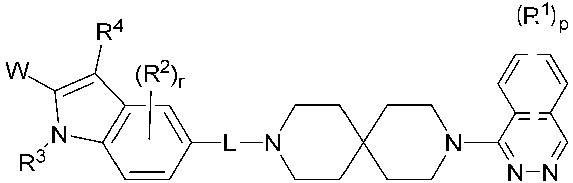 |
| I-B-II-f | 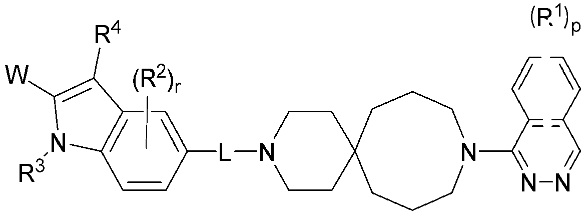 |
| I-B-II-g | 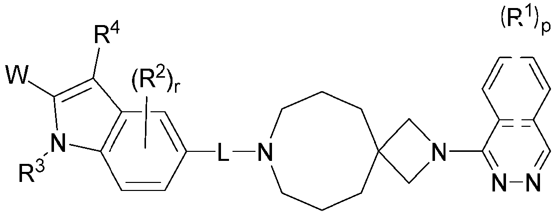 |
| I-B-II-h | 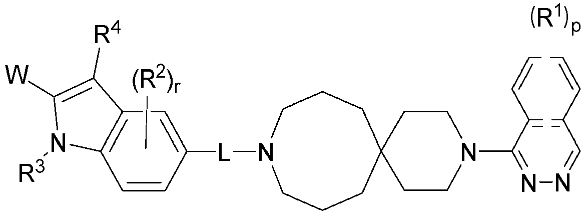 |
| I-B-II-i | 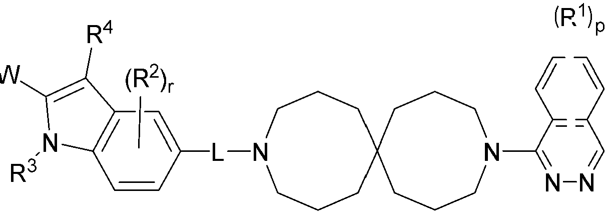 |
| I-B-III-a | 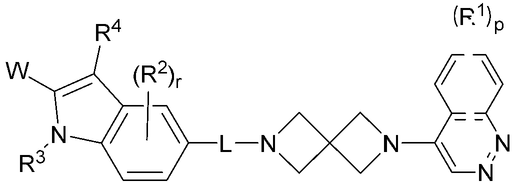 |
| I-B-III-b | 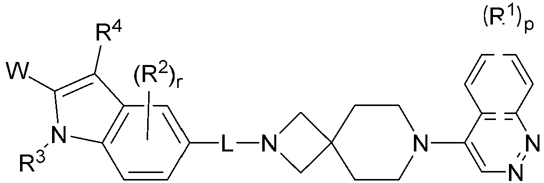 |
| I-B-III-c | 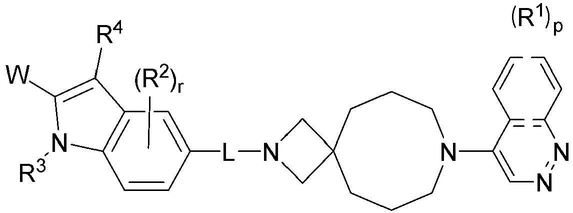 |
| I-B-III-d | 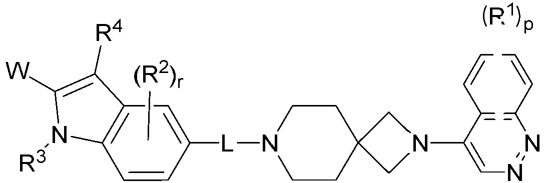 |
| I-B-III-e | 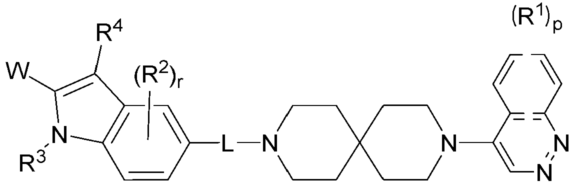 |
| I-B-III-f | 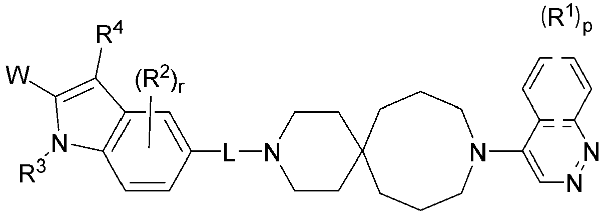 |
| I-B-III-g | 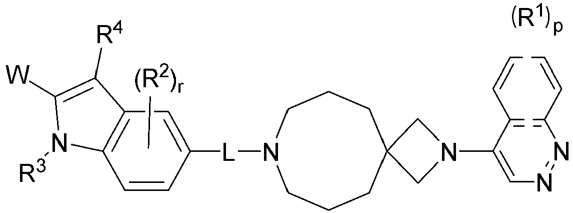 |
| I-B-III-h | 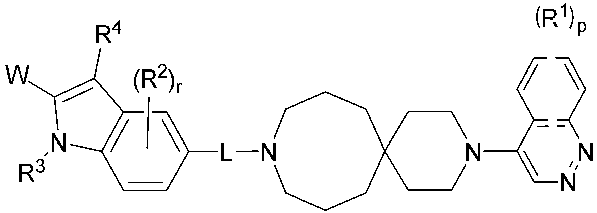 |
| I-B-III-i | 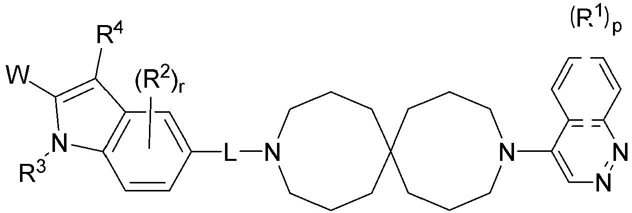 |
| I-B-IV-a | 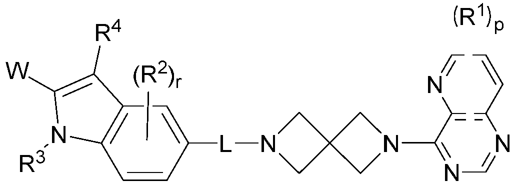 |
| I-B-IV-b | 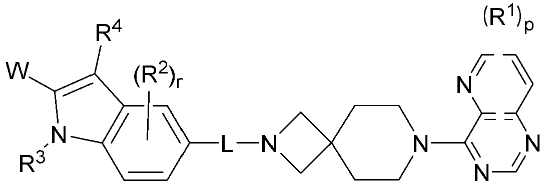 |
| I-B-IV-c | 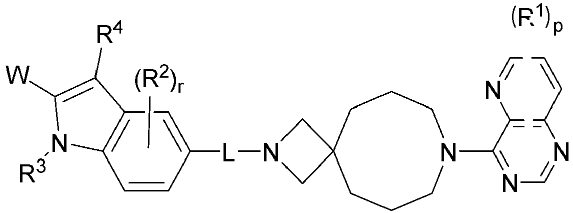 |
| I-B-IV-d | 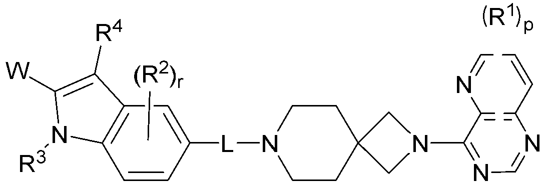 |
| I-B-IV-e | 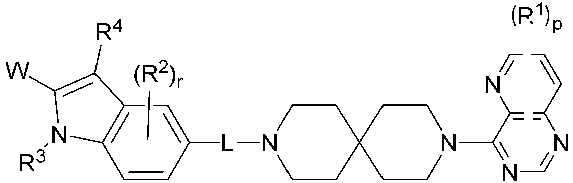 |
| I-B-IV-f | 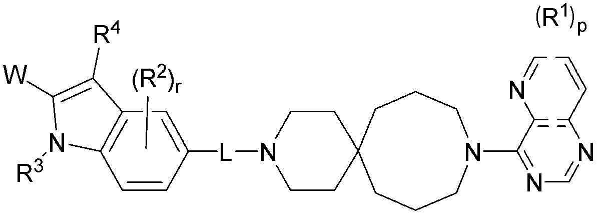 |
| I-B-IV-g | 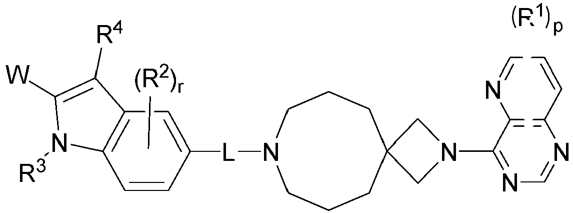 |
| I-B-IV-h | 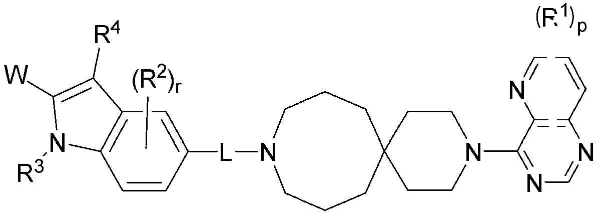 |
| I-B-IV-i | 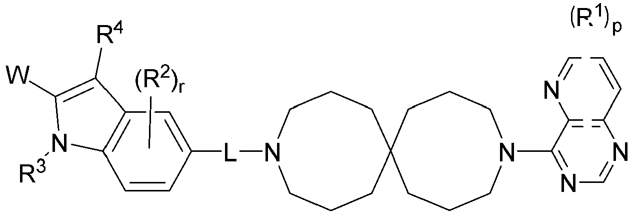 |
| I-B-V-a | 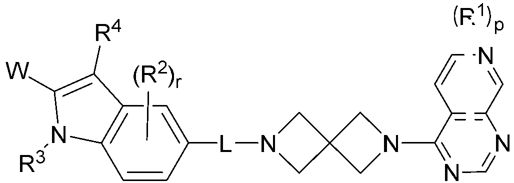 |
| I-B-V-b | 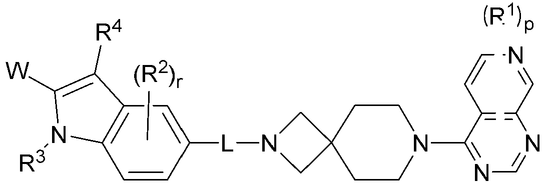 |
| I-B-V-c | 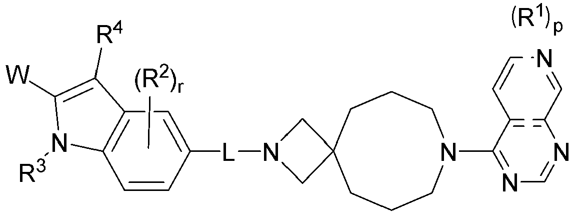 |
| I-B-V-d | 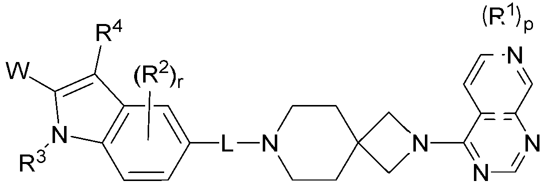 |
| I-B-V-e | 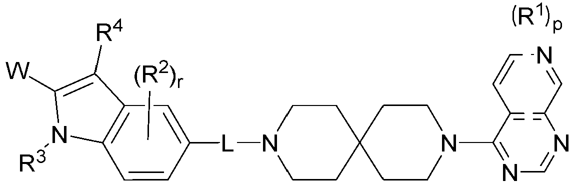 |
| I-B-V-f | 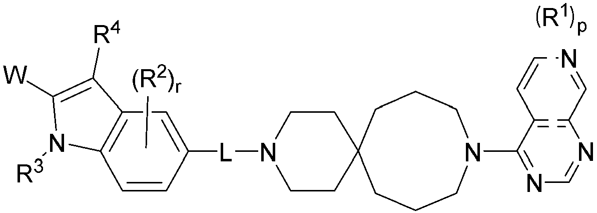 |
| I-B-V-g | 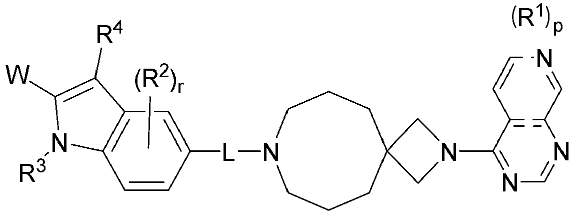 |
| I-B-V-h | 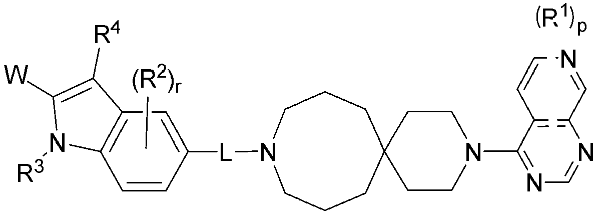 |
| I-B-V-i | V 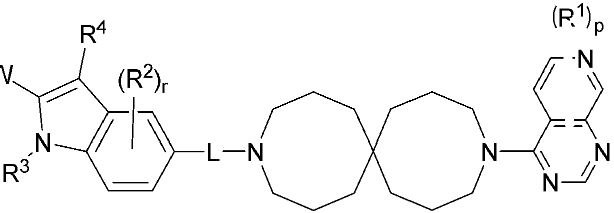 |
| I-B-VI-a | 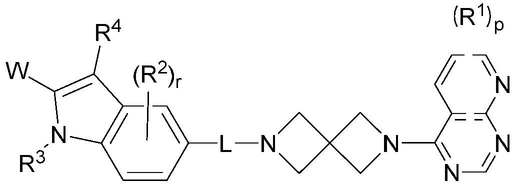 |
| I-B-VI-b | 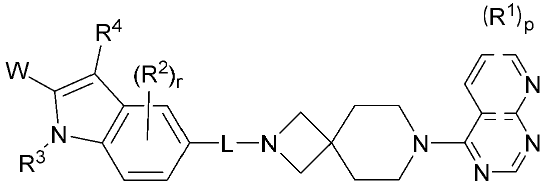 |
| I-B-VI-c | 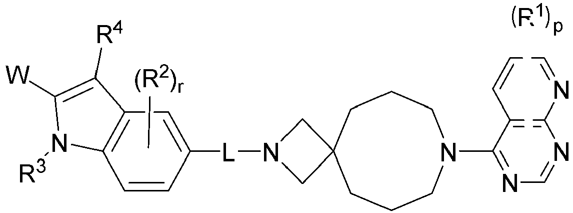 |
| I-B-VI-d | 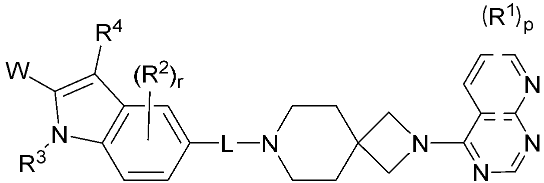 |
| I-B-VI-e | 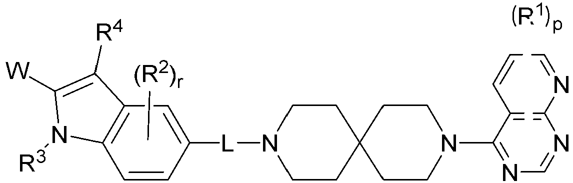 |
| I-B-VI-f | 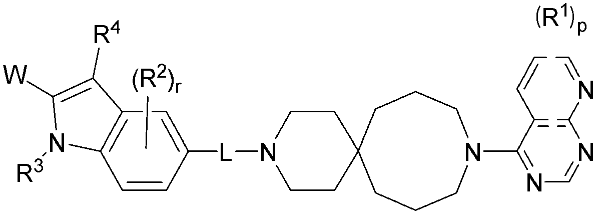 |
| I-B-VI-g | 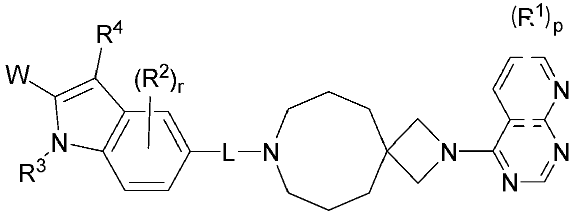 |
| I-B-VI-h | 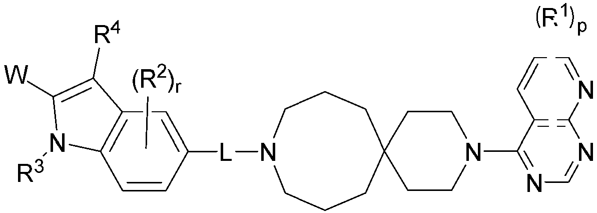 |
| I-B-VI-i | 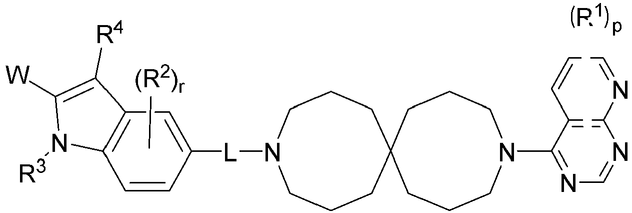 |
| I-B-VII-a | 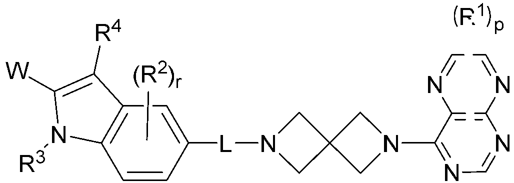 |
| I-B-VII-b | 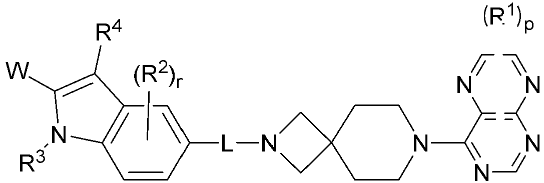 |
| I-B-VII-c | 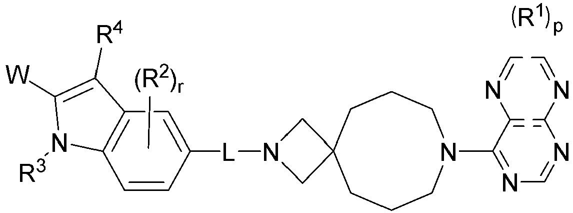 |
| I-B-VII-d | 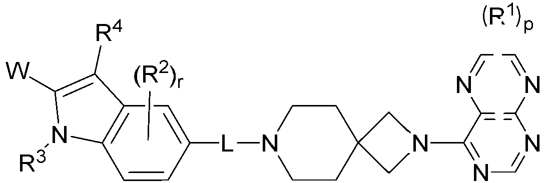 |
| I-B-VII-e | 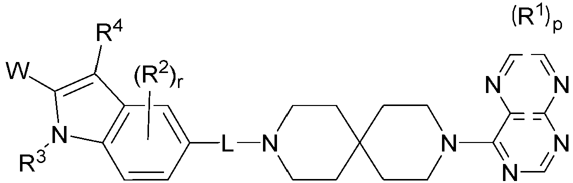 |
| I-B-VII-f | 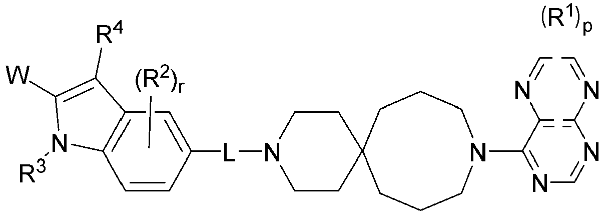 |
| I-B-VII-g | 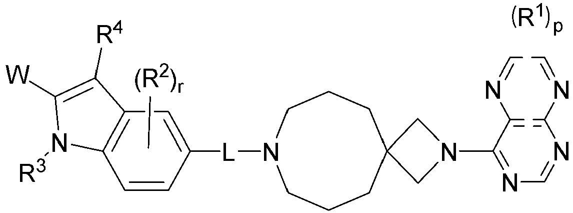 |
| I-B-VII-h | 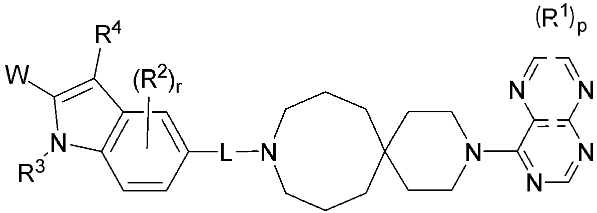 |
| I-B-VII-i | 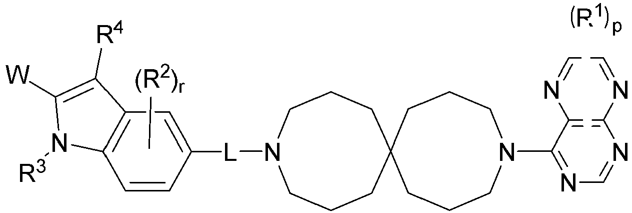 |
| I-C-I-a | 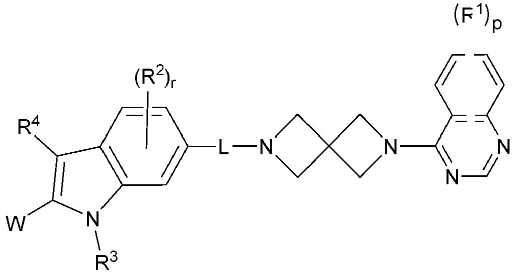 |
| I-C-I-b | 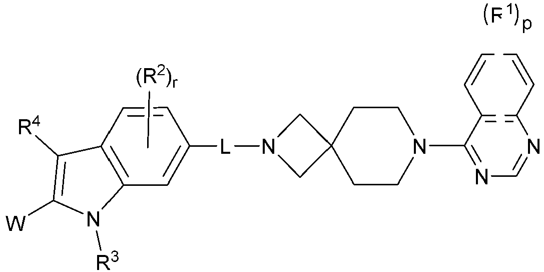 |
| I-C-I-c | 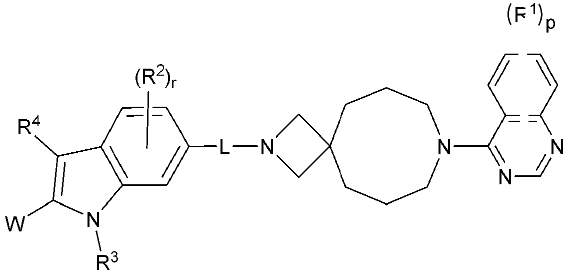 |
| I-C-I-d | 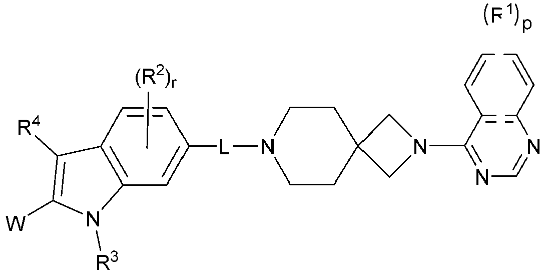 |
| I-C-I-e | 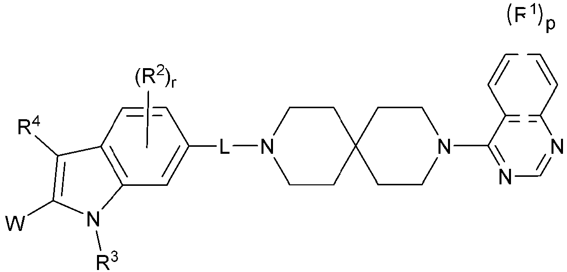 |
| I-C-I-f | 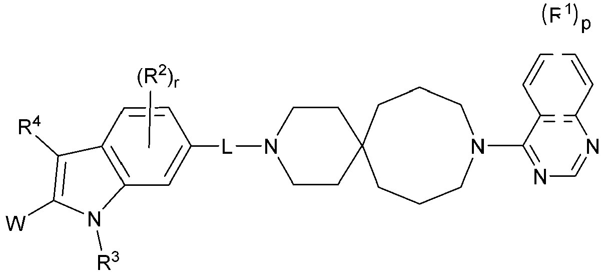 |
| I-C-I-g | 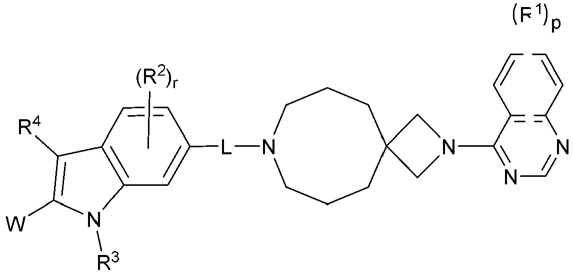 |
| I-C-I-h | 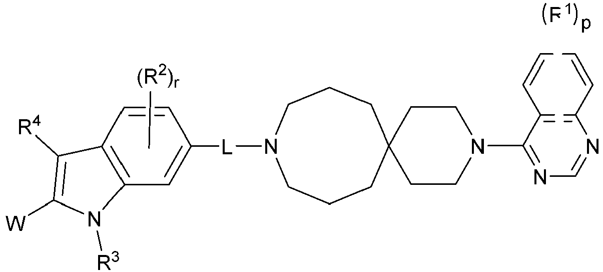 |
| I-C-I-i | 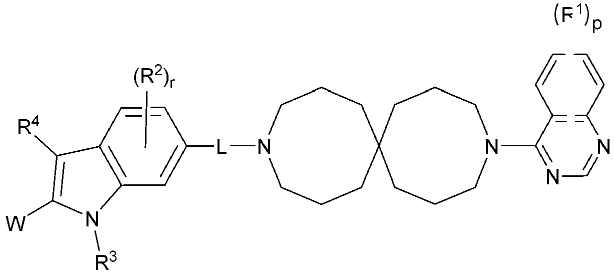 |
| I-C-II-a | 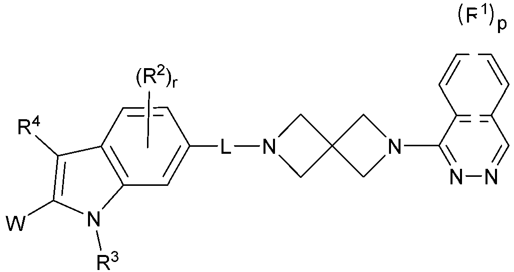 |
| I-C-II-b | 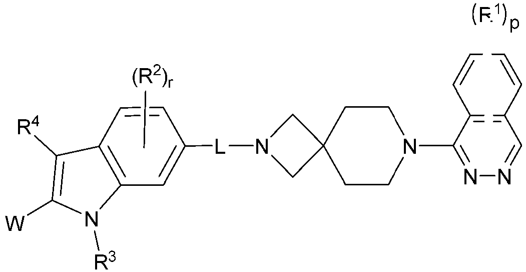 |
| I-C-II-c | 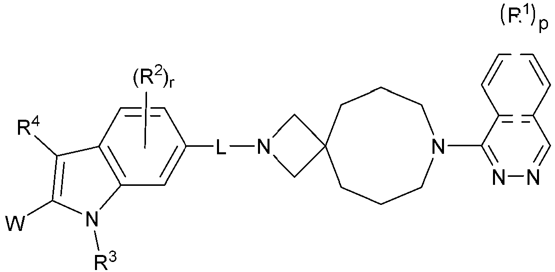 |
| I-C-II-d | 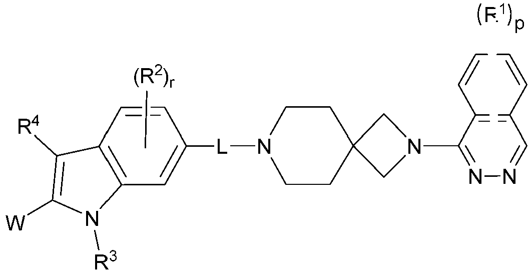 |
| I-C-II-e | 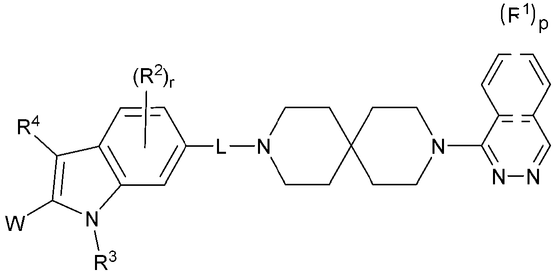 |
| I-C-II-f | 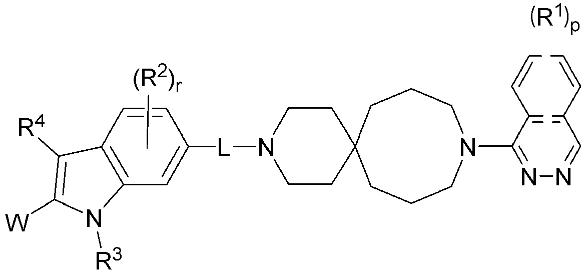 |
| I-C-II-g | 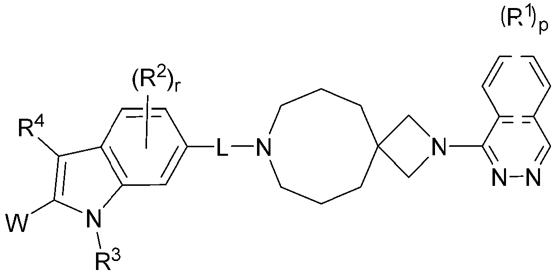 |
| I-C-II-h | 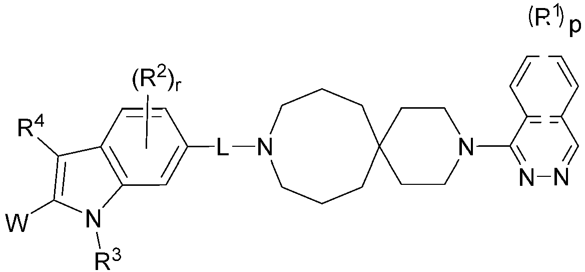 |
| I-C-II-i | 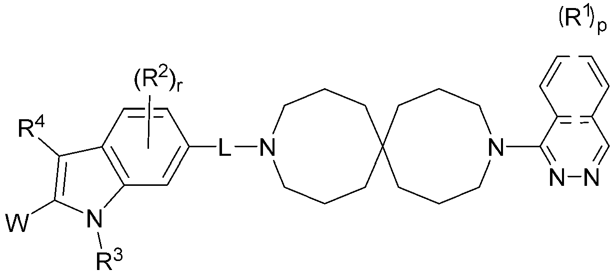 |
| I-C-III-a | 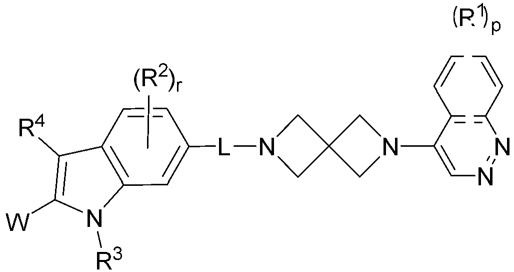 |
| I-C-III-b | 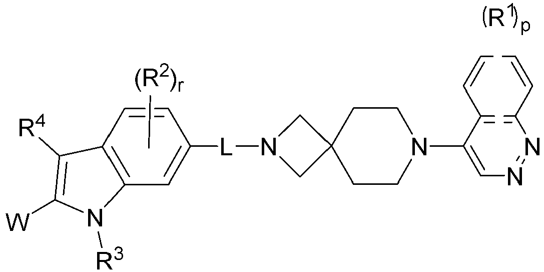 |
| I-C-III-c | 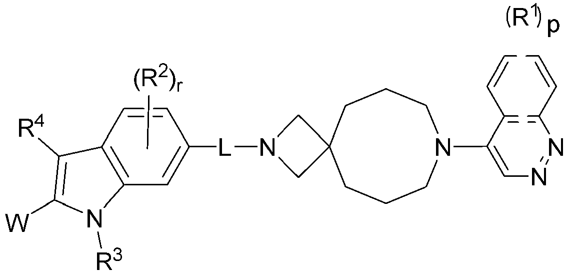 |
| I-C-III-d | 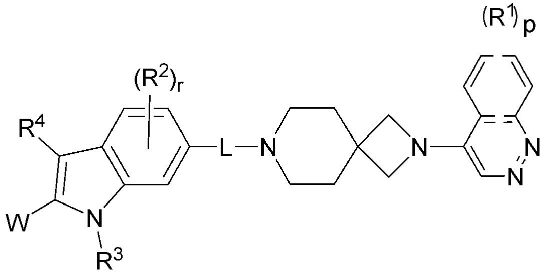 |
| I-C-III-e | 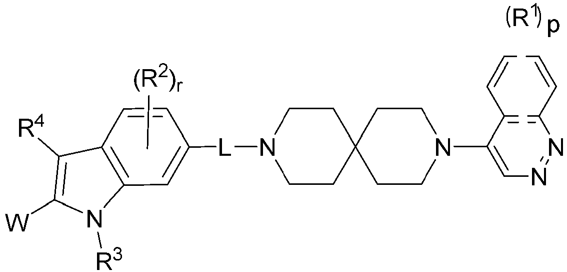 |
| I-C-III-f | 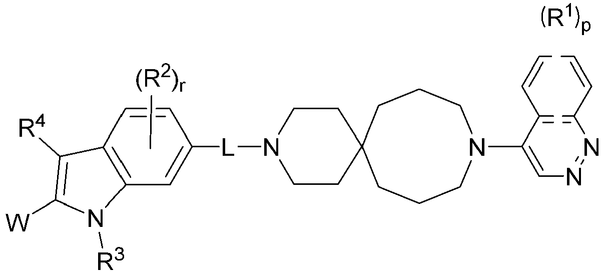 |
| I-C-III-g | 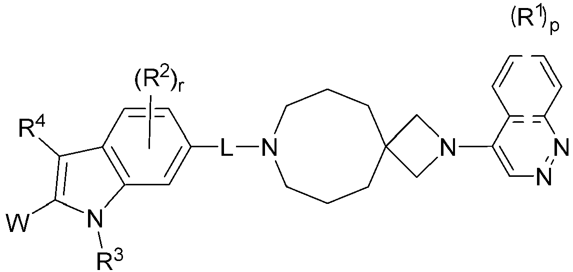 |
| I-C-III-h | 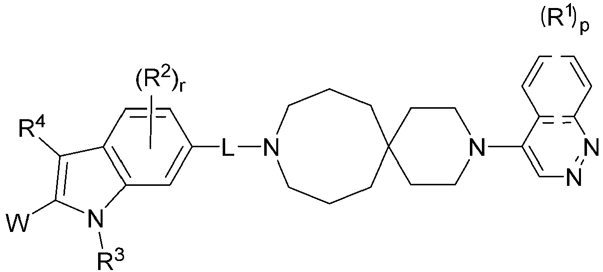 |
| I-C-III-i | 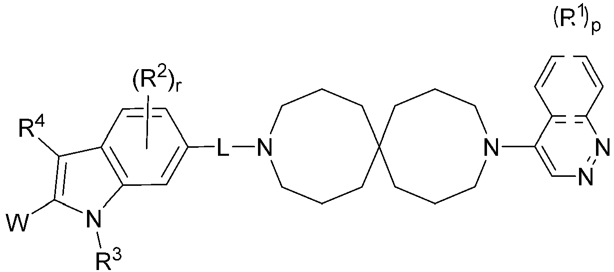 |
| I-C-IV-a | 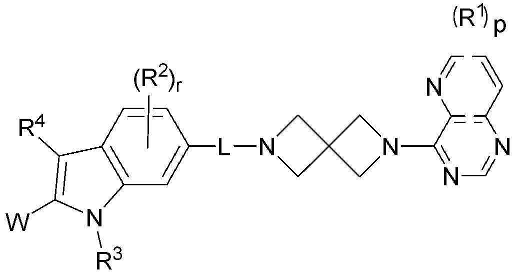 |
| I-C-IV-b | 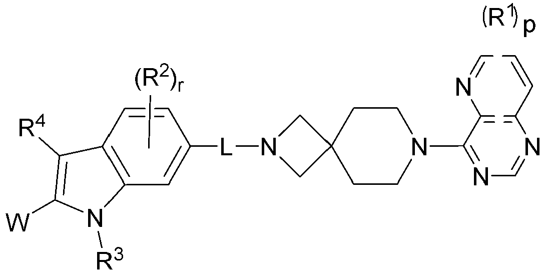 |
| I-C-IV-c | 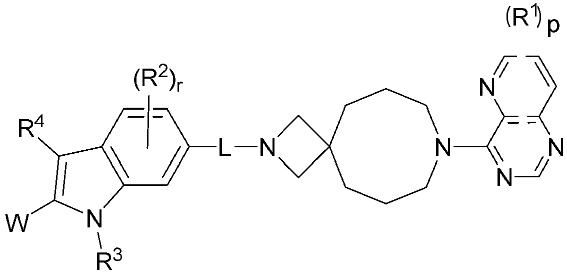 |
| I-C-IV-d | 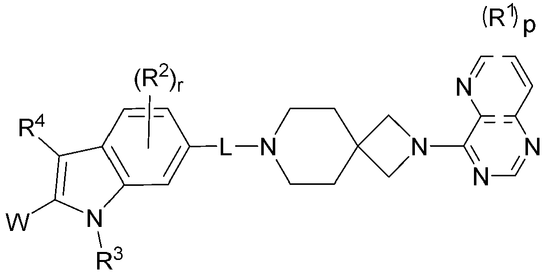 |
| I-C-IV-e | 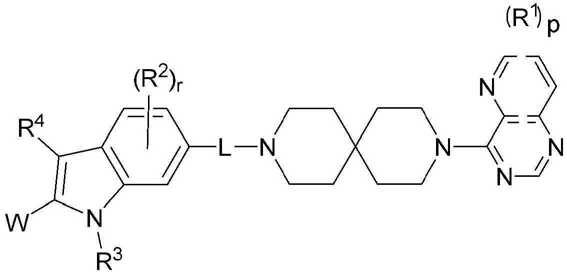 |
| I-C-IV-f | 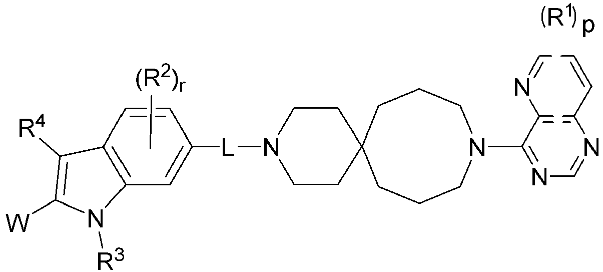 |
| I-C-IV-g | 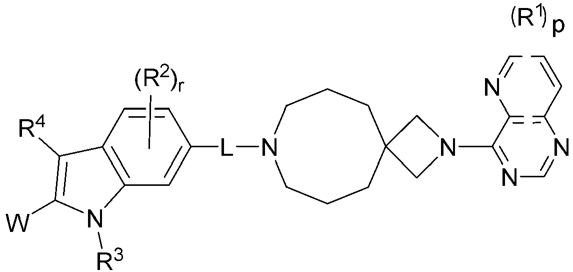 |
| I-C-IV-h | 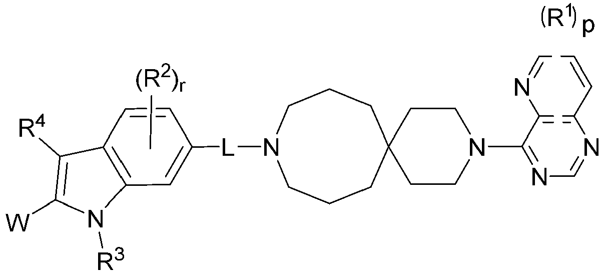 |
| I-C-IV-i | 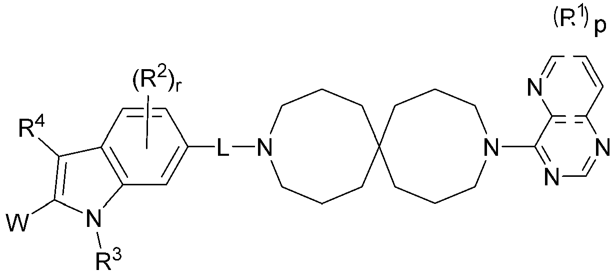 |
| I-C-V-a | 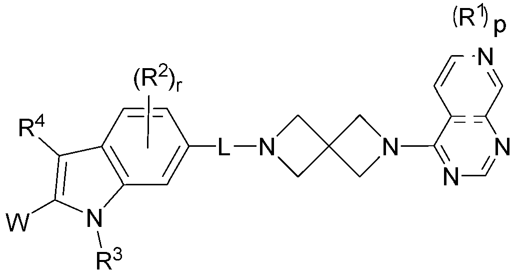 |
| I-C-V-b | 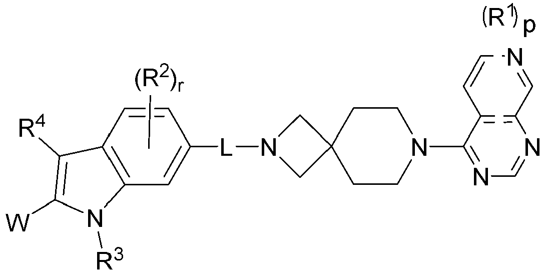 |
| I-C-V-c | 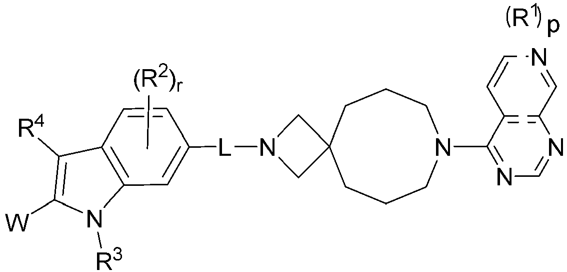 |
| I-C-V-d | 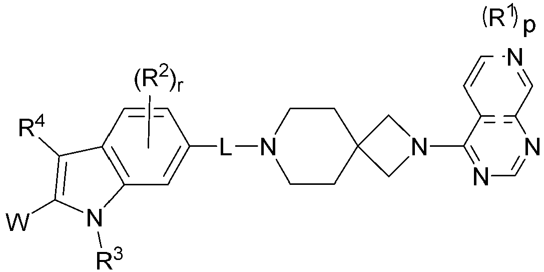 |
| I-C-V-e | 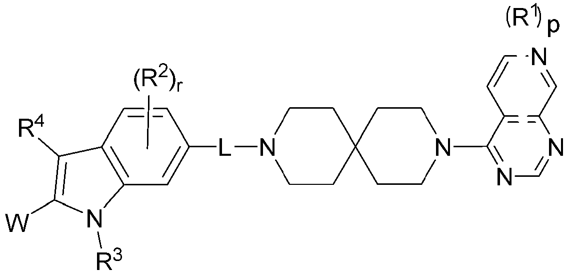 |
| I-C-V-f | 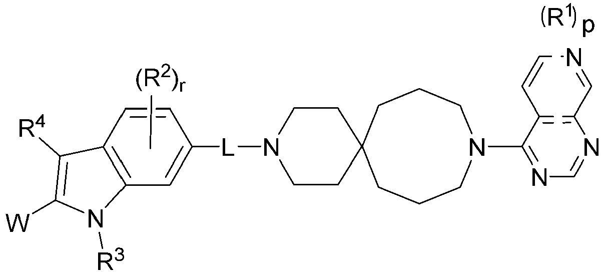 |
| I-C-V-g | 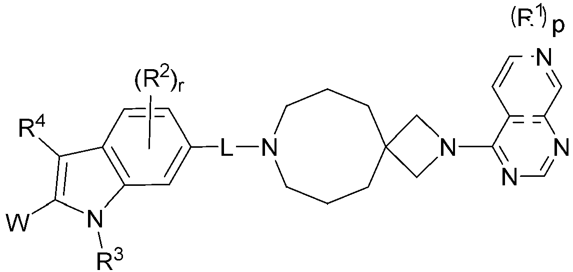 |
| I-C-V-h | 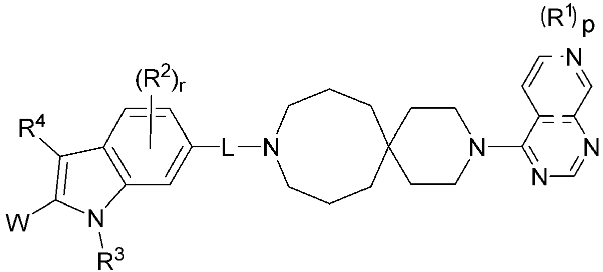 |
| I-C-V-i | 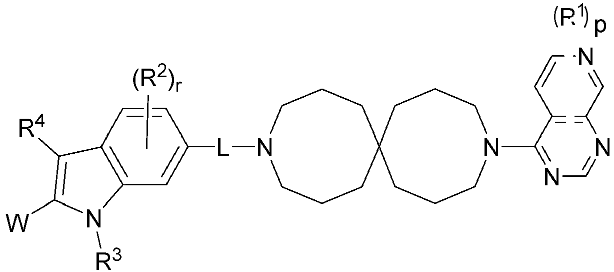 |
| I-C-VI-a | 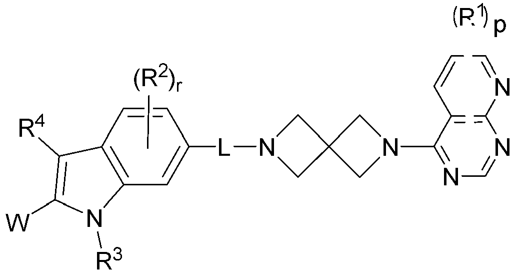 |
| I-C-VI-b | 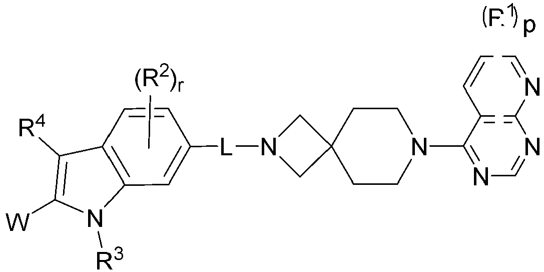 |
| I-C-VI-c | 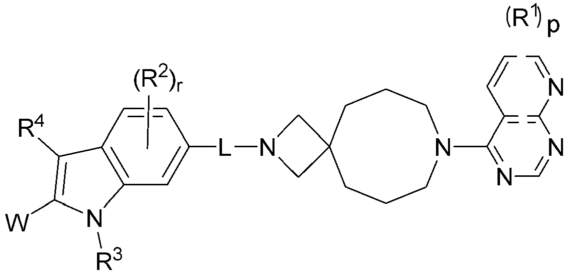 |
| I-C-VI-d | 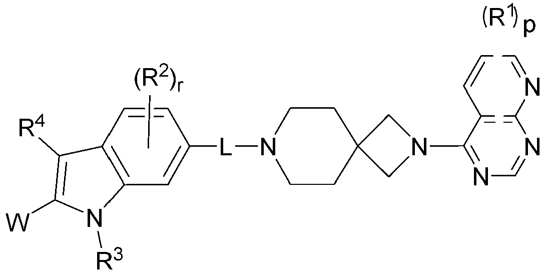 |
| I-C-VI-e | 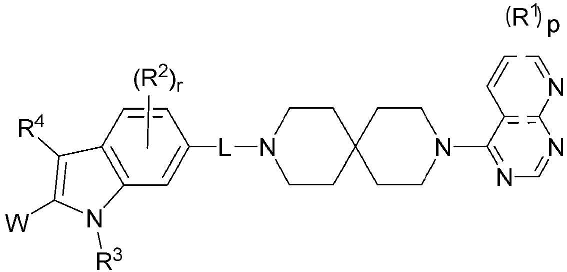 |
| I-C-VI-f | 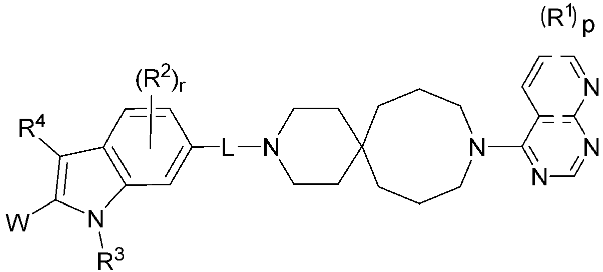 |
| I-C-VI-g | 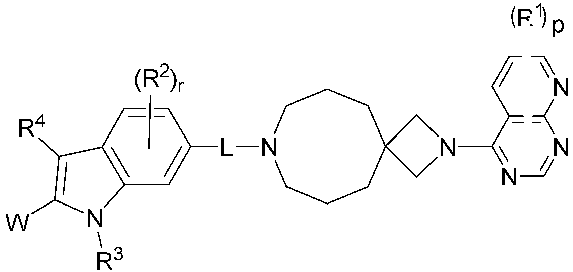 |
| I-C-VI-h | 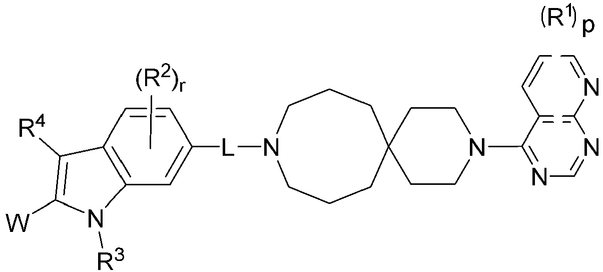 |
| I-C-VI-i | 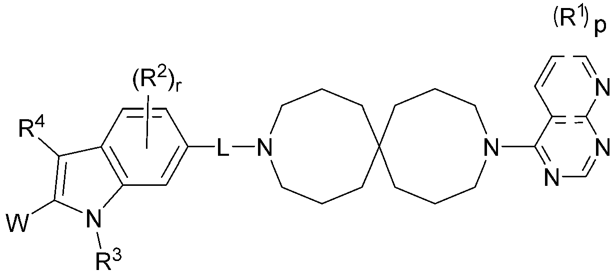 |
| I-C-VII-a | 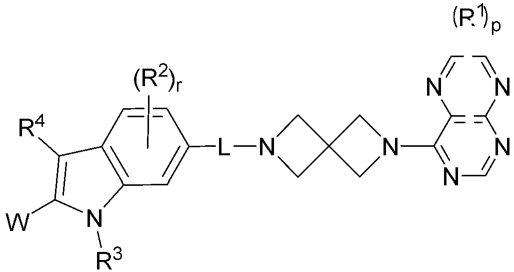 |
| I-C-VII-b | 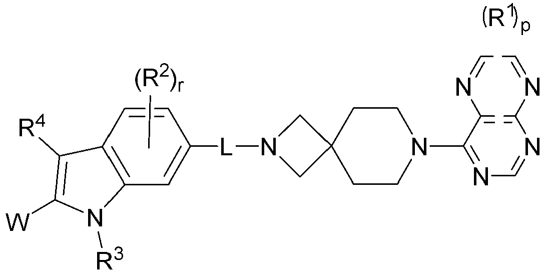 |
| I-C-VII-c | 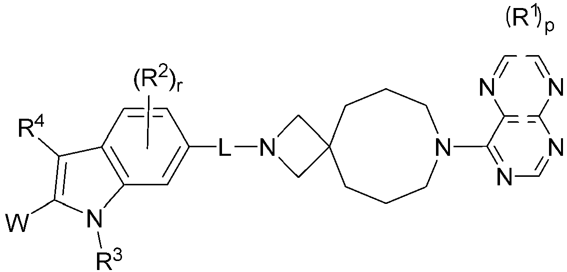 |
| I-C-VII-d | 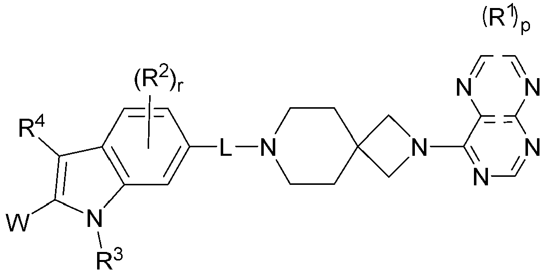 |
| I-C-VII-e | 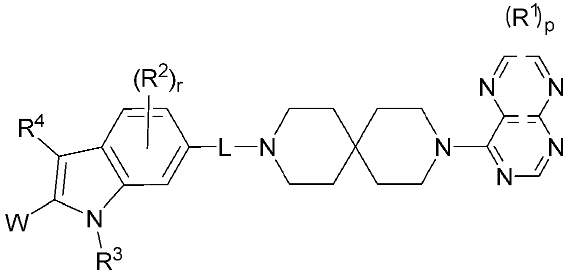 |
| I-C-VII-f | 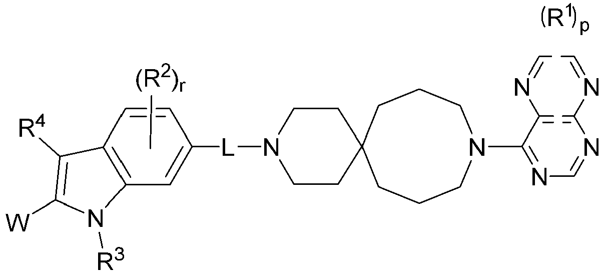 |
| I-C-VII-g | 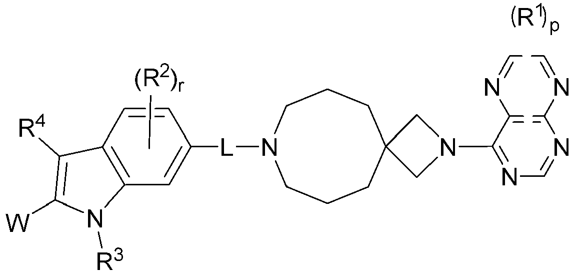 |
| I-C-VII-h | 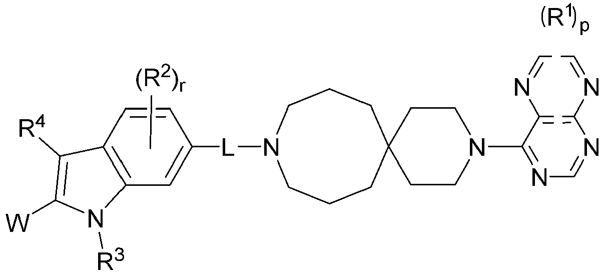 |
| I-C-VII-i | 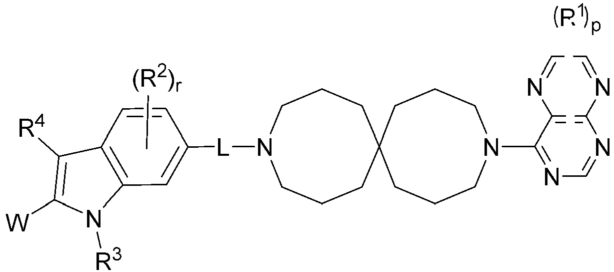 |
| I-D-I-a | 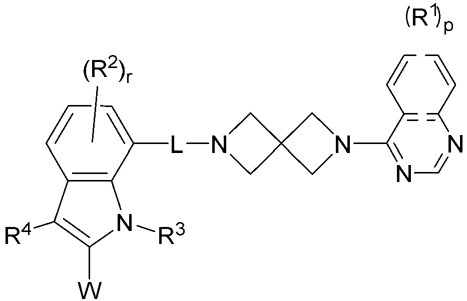 |
| I-D-I-b | 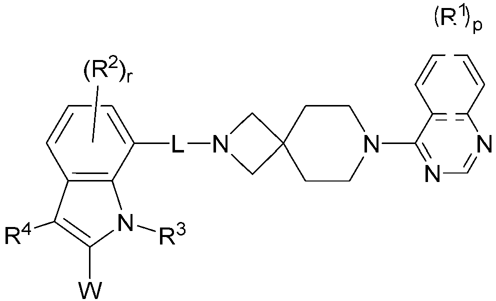 |
| I-D-I-c | 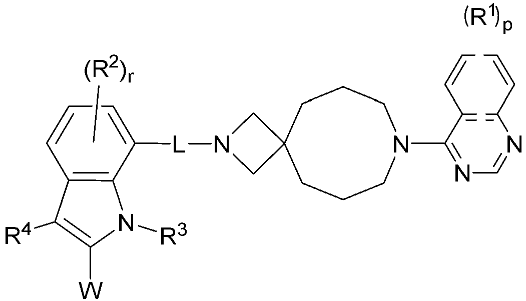 |
| I-D-I-d | 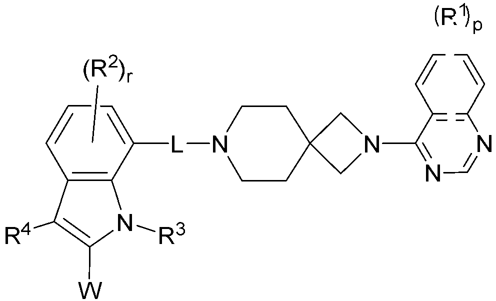 |
| I-D-I-e | 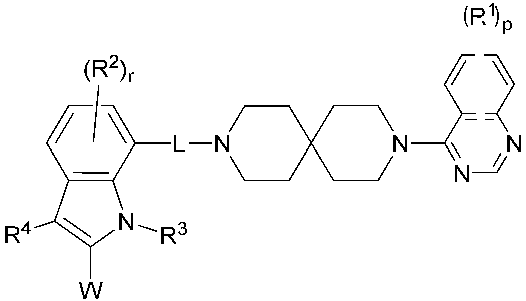 |
| I-D-I-f | 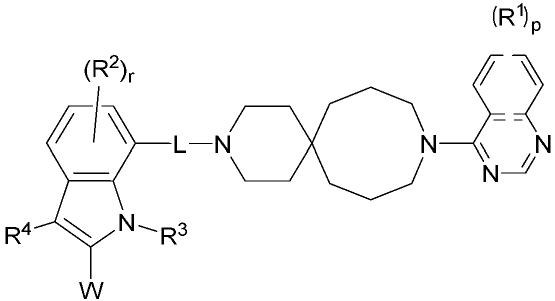 |
| I-D-I-g | 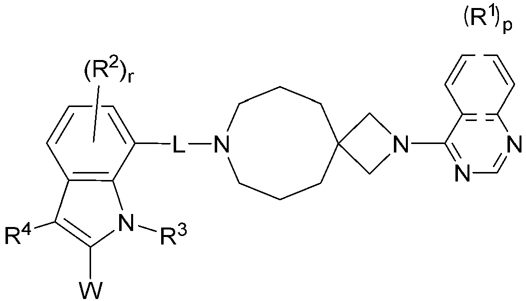 |
| I-D-I-h | 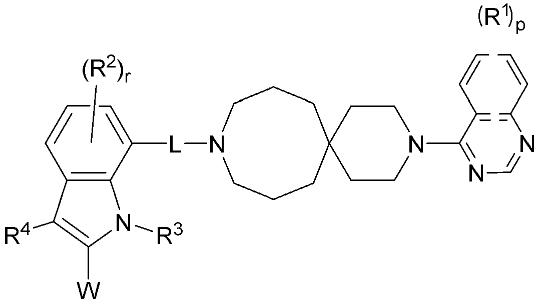 |
| I-D-I-i | 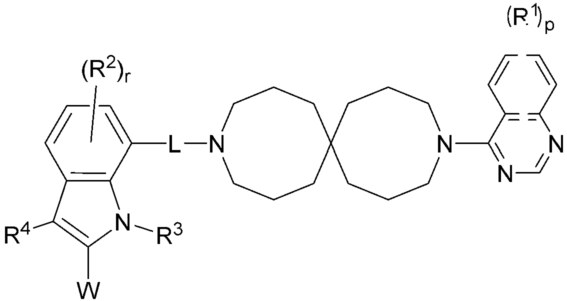 |
| I-D-II-a | 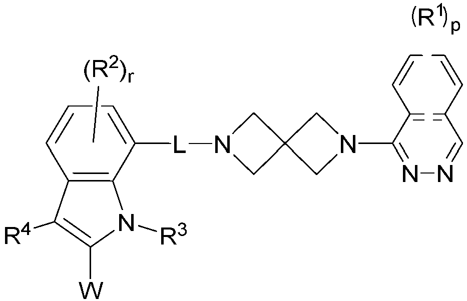 |
| I-D-II-b | 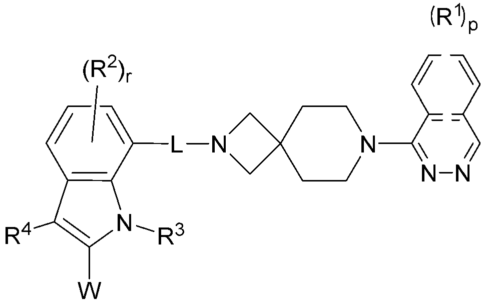 |
| I-D-II-c | 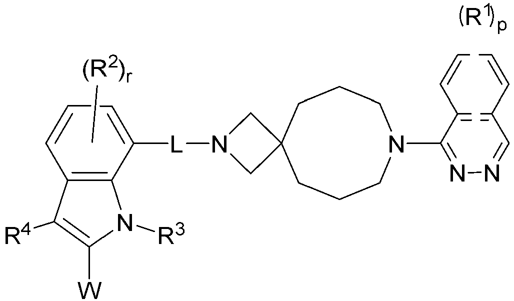 |
| I-D-II-d | 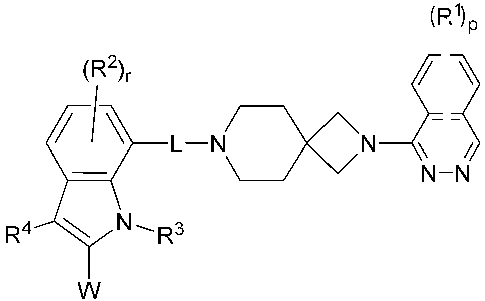 |
| I-D-II-e | 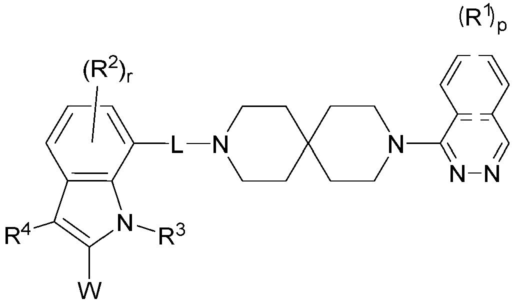 |
| I-D-II-f | 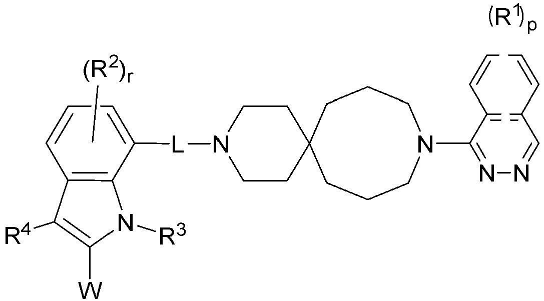 |
| I-D-II-g | 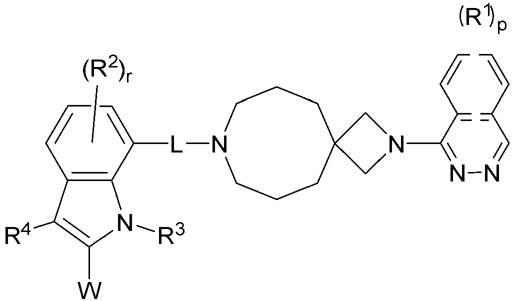 |
| I-D-II-h | 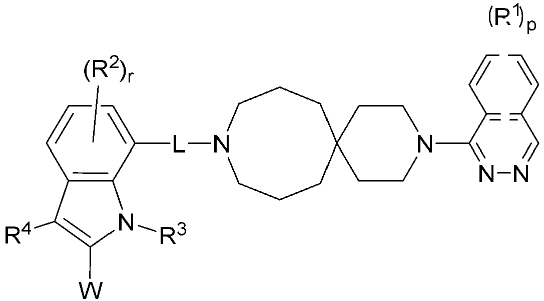 |
| I-D-II-i | 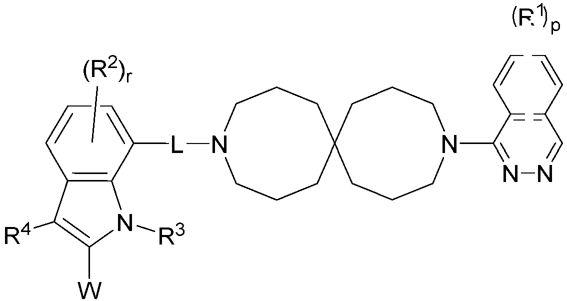 |
| I-D-III-a | 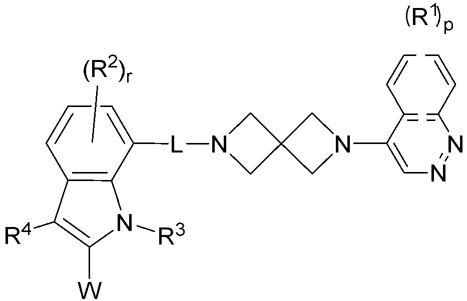 |
| I-D-III-b | 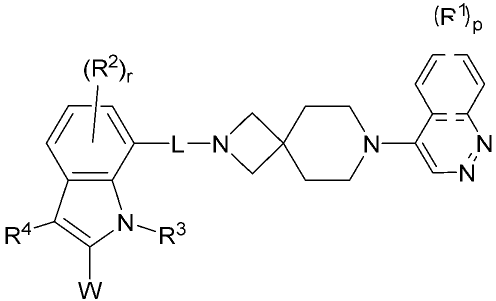 |
| I-D-III-c | 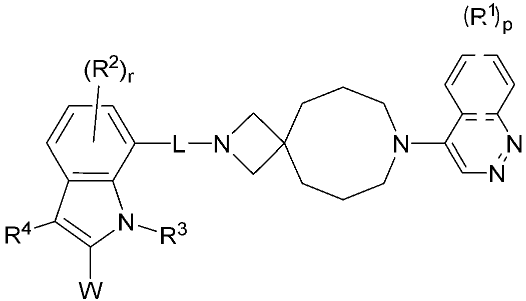 |
| I-D-III-d | 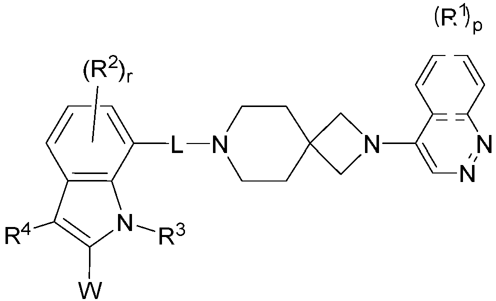 |
| I-D-III-e | 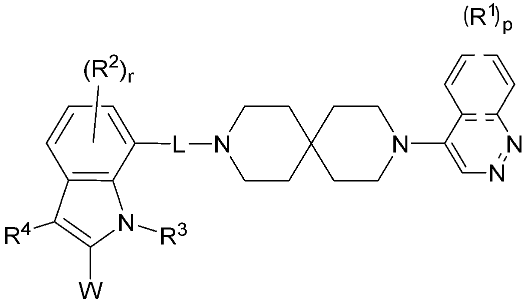 |
| I-D-III-f | 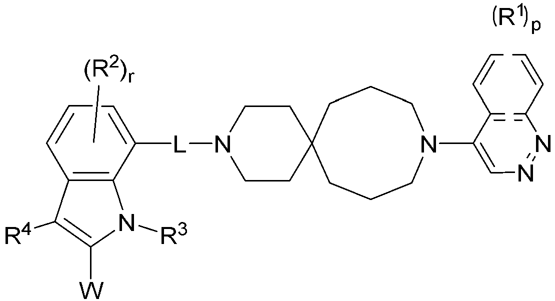 |
| I-D-III-g | 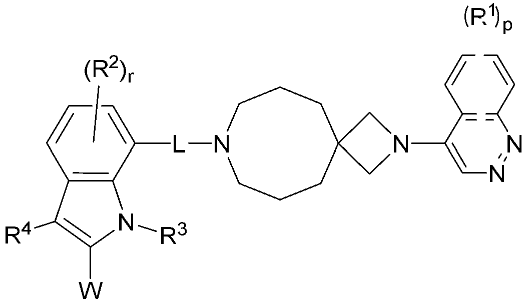 |
| I-D-III-h | 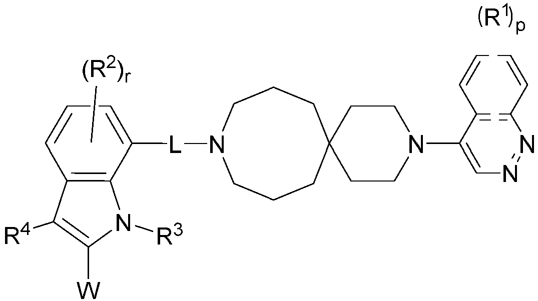 |
| I-D-III-i | 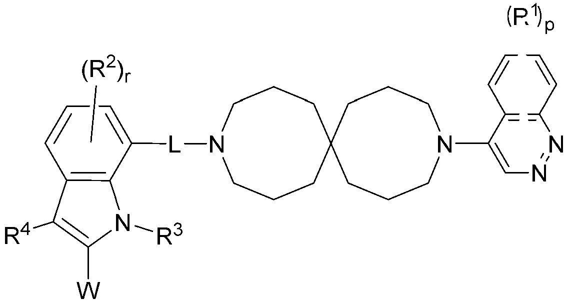 |
| I-D-IV-a | 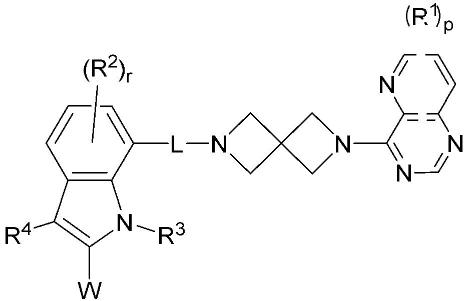 |
| I-D-IV-b | 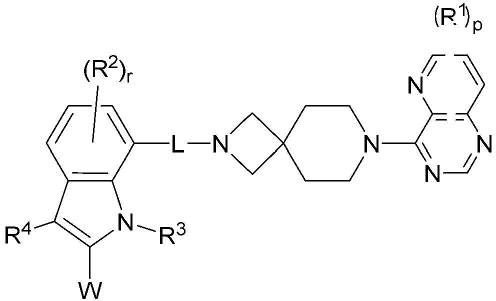 |
| I-D-IV-c | 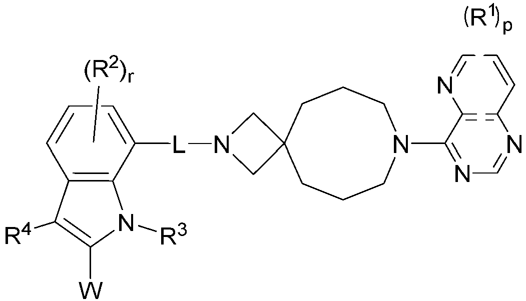 |
| I-D-IV-d | 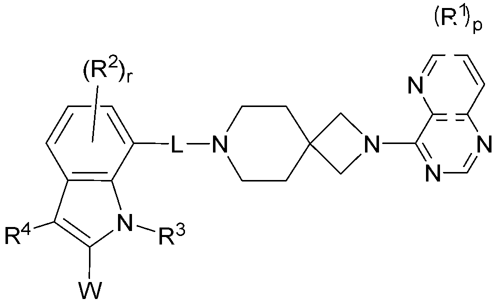 |
| I-D-IV-e | 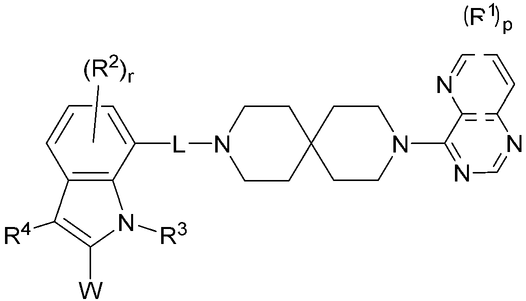 |
| I-D-IV-f | 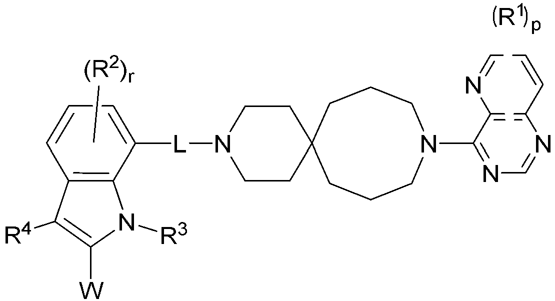 |
| I-D-IV-g | 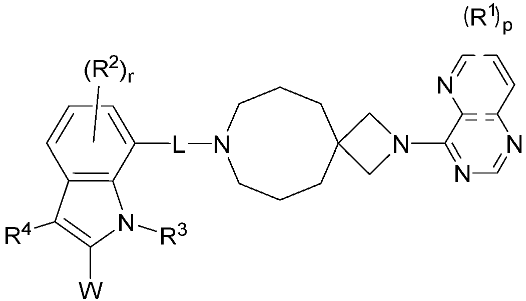 |
| I-D-IV-h | 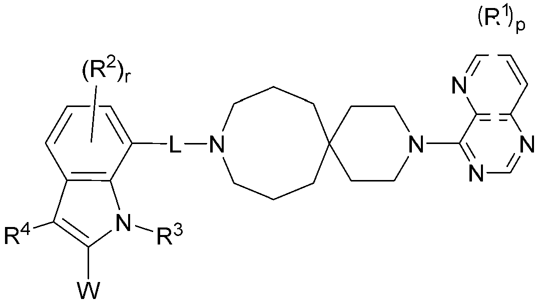 |
| I-D-IV-i | 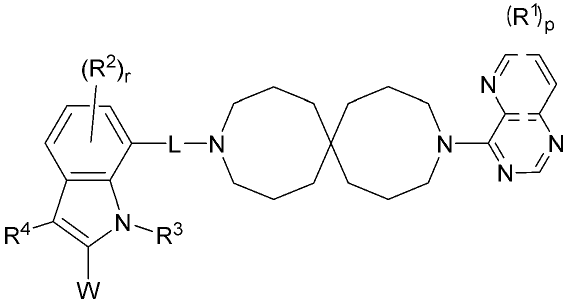 |
| I-D-V-a | 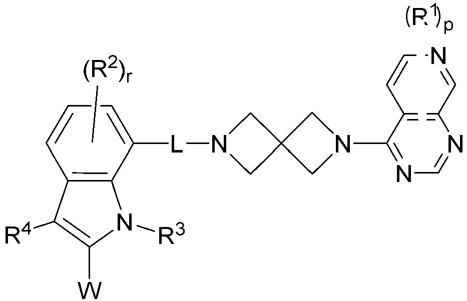 |
| I-D-V-b | 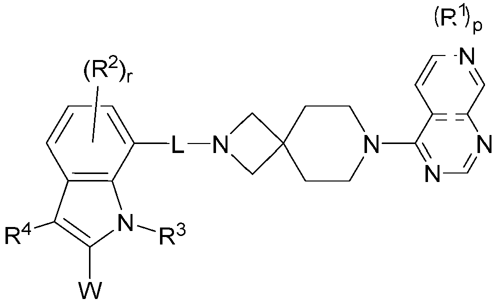 |
| I-D-V-c | 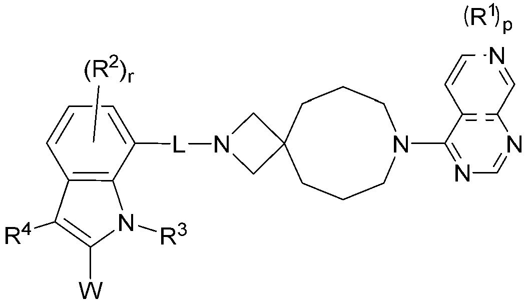 |
| I-D-V-d | 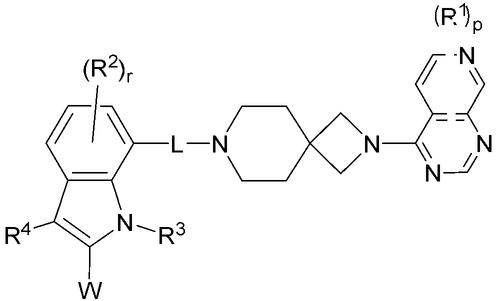 |
| I-D-V-e | 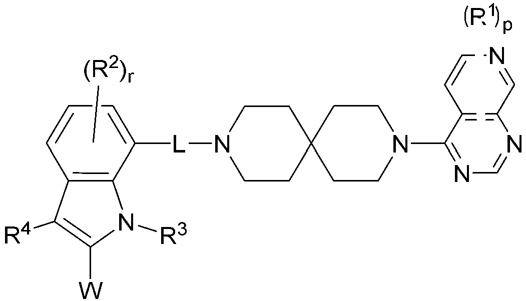 |
| I-D-V-f | 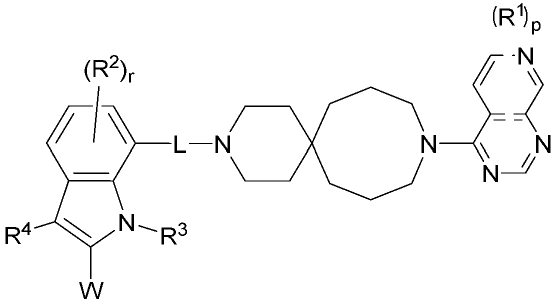 |
| I-D-V-g | 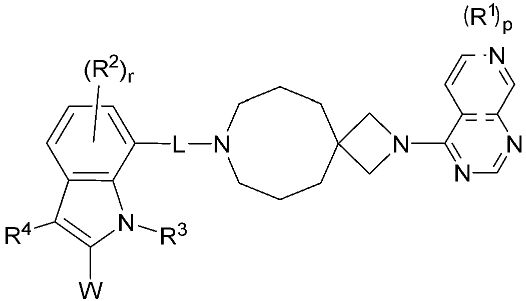 |
| I-D-V-h | 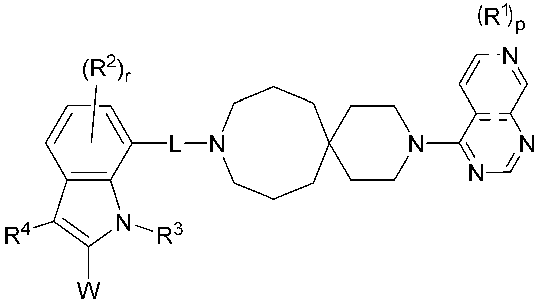 |
| I-D-V-i | 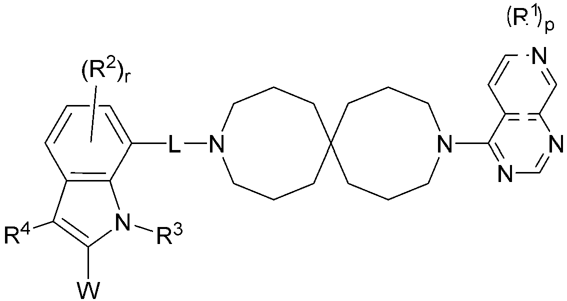 |
| I-D-VI-a | 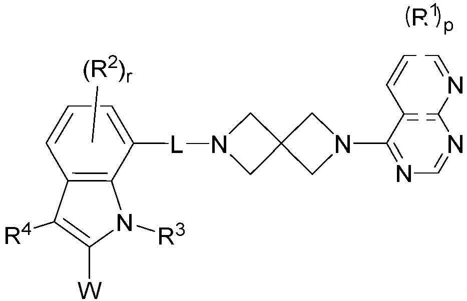 |
| I-D-VI-b | 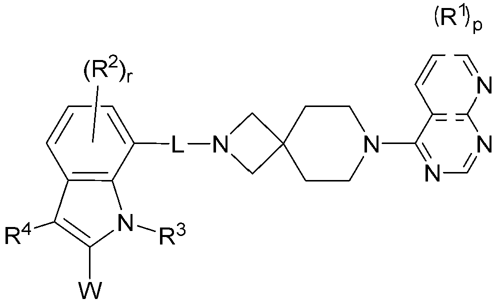 |
| I-D-VI-c | 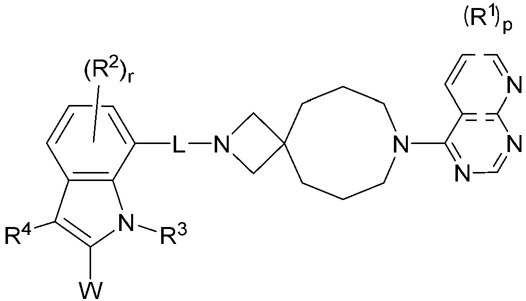 |
| I-D-VI-d | 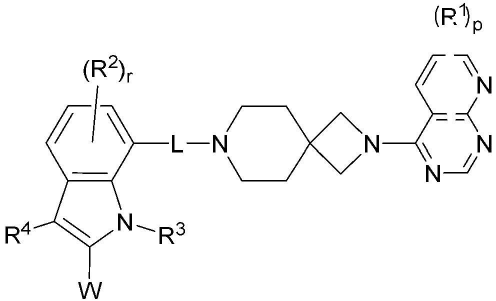 |
| I-D-VI-e | 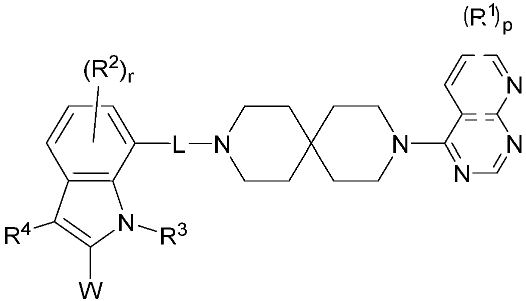 |
| I-D-VI-f | 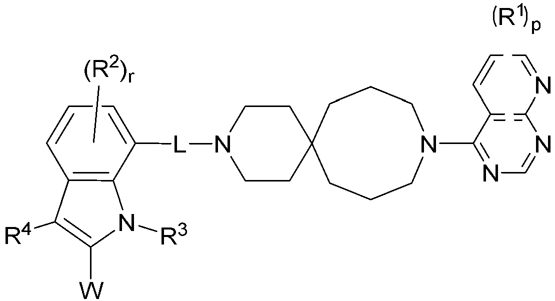 |
| I-D-VI-g | 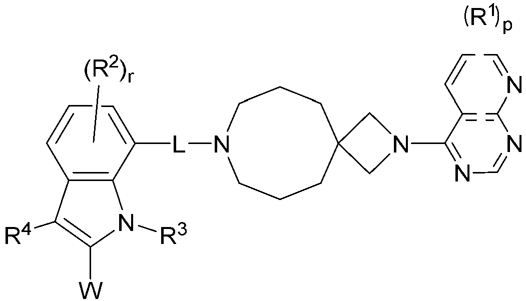 |
| I-D-VI-h | 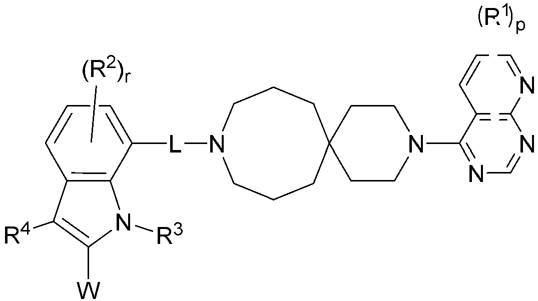 |
| I-D-VI-i | 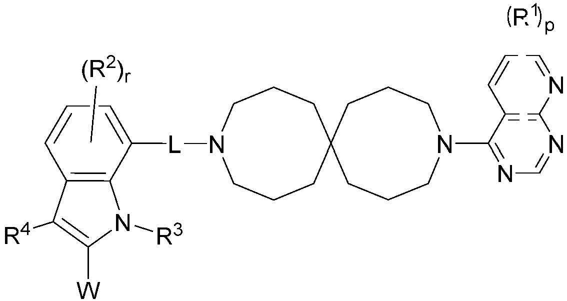 |
| I-D-VII-a | 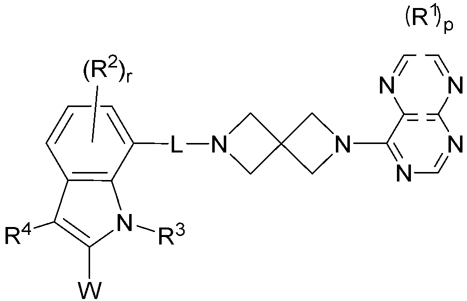 |
| I-D-VII-b | 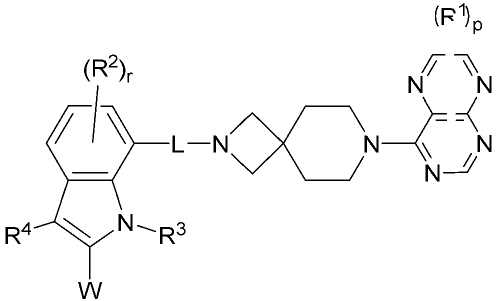 |
| I-D-VII-c | 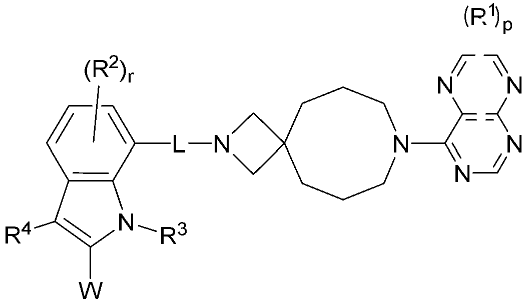 |
| I-D-VII-d | 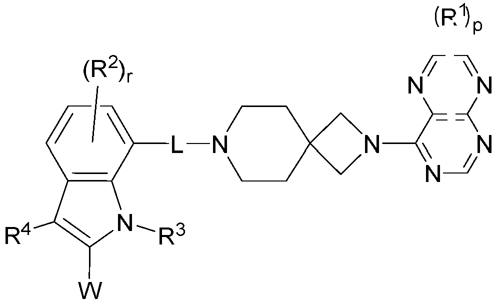 |
| I-D-VII-e | 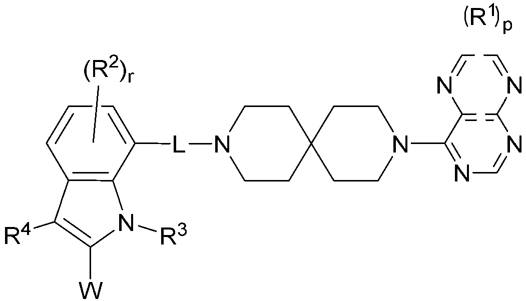 |
| I-D-VII-f | 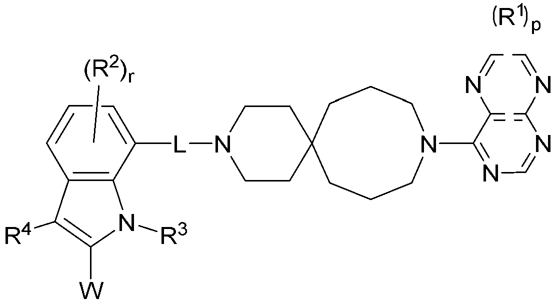 |
| I-D-VII-g | 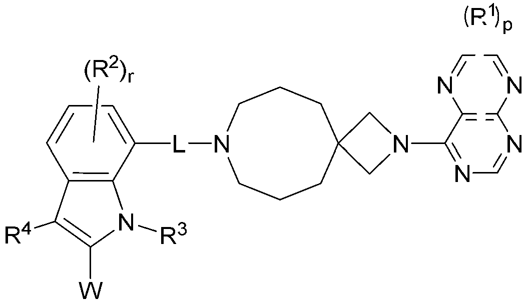 |
| I-D-VII-h | 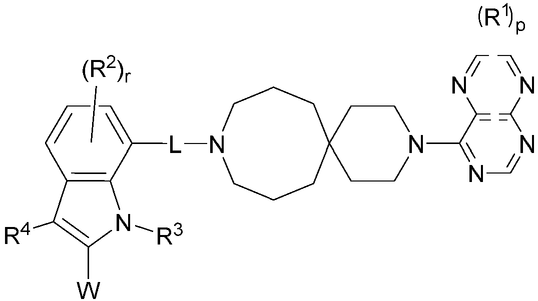 |
| I-D-VII-i | 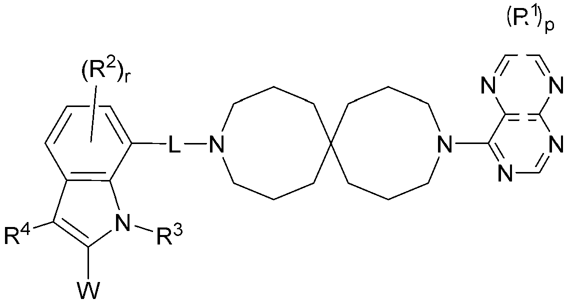 |

In some embodiments, the compound is selected from the Table 4 or its pharmaceutically acceptable salt, stereoisomer, solvate, prodrug, isotopic derivative, or tautomer thereof wherein all variables are as defined herein.

**Table 4.**

| Compound # | Structure |
|---|---|
| I-A-I-a-I | 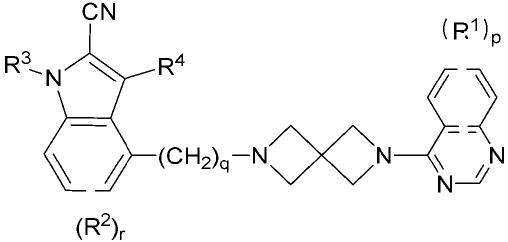 |
| I-A-I-b-I | 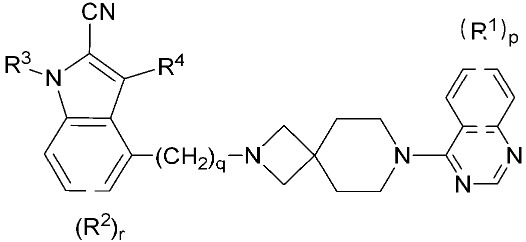 |
| I-A-I-c-I | 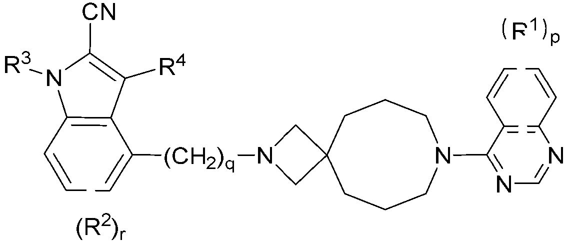 |
| I-A-I-d-I | 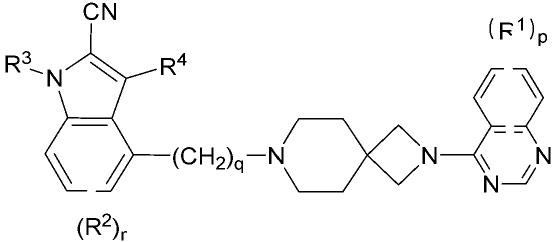 |
| I-A-I-e-I | 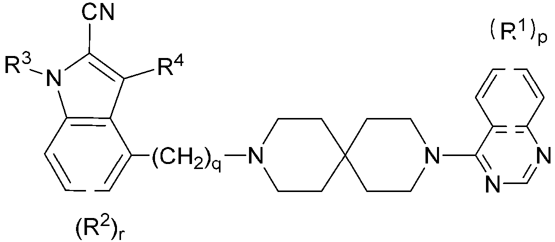 |
| I-A-I-f-I | 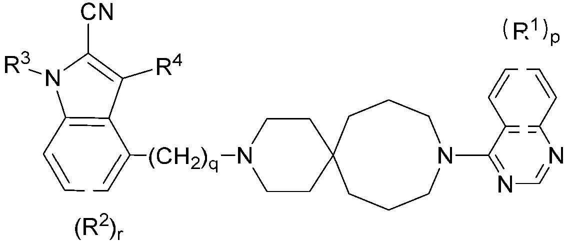 |
| I-A-I-g-I | 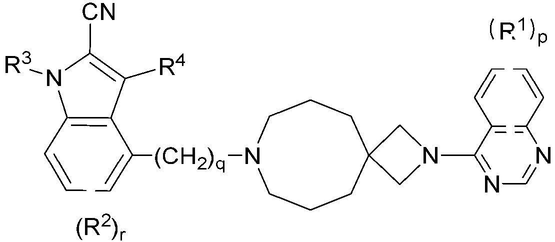 |
| I-A-I-h-I | 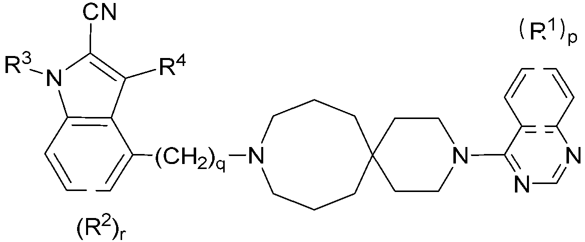 |
| I-A-I-i-I | 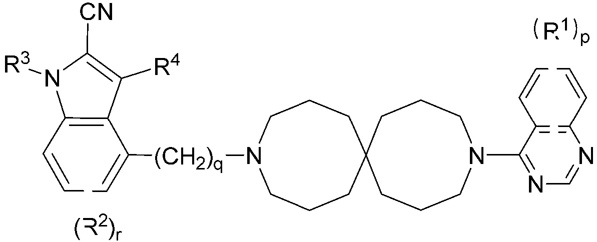 |
| I-A-II-a-I | 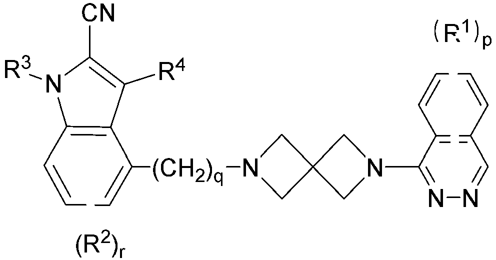 |
| I-A-II-b-I | 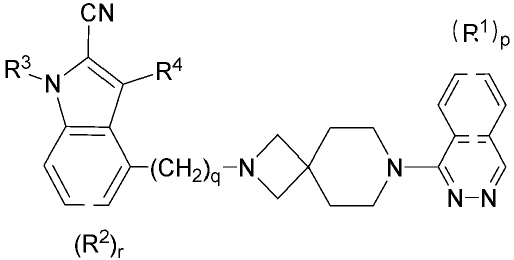 |
| I-A-II-c-I | 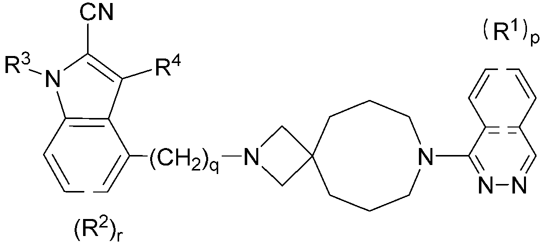 |
| I-A-II-d-I | 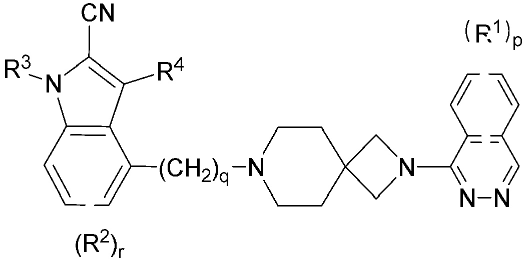 |
| I-A-II-e-I | 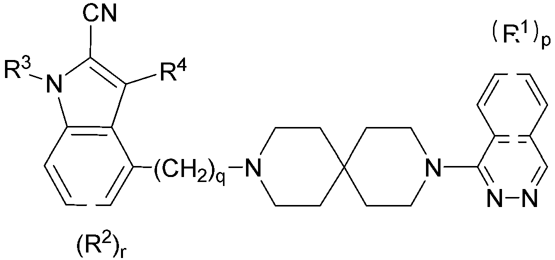 |
| I-A-II-f-I | 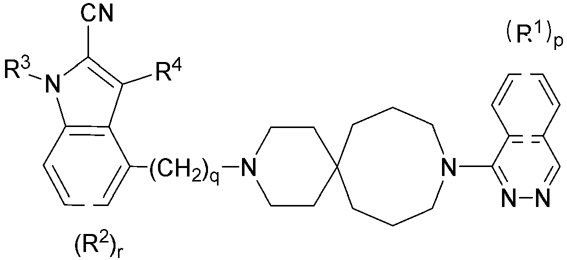 |
| I-A-II-g-I | 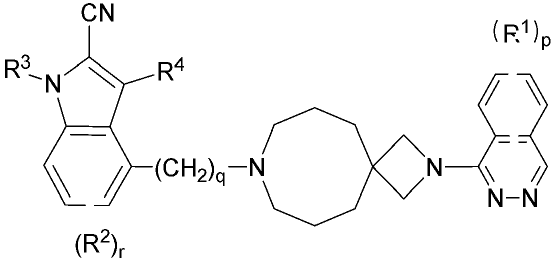 |
| I-A-II-h-I | 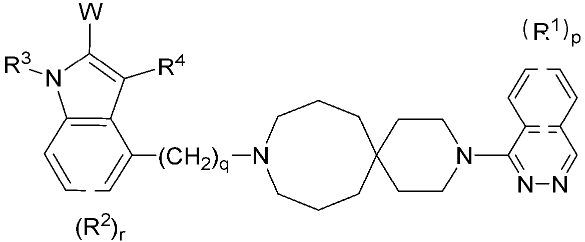 |
| I-A-II-i-I | 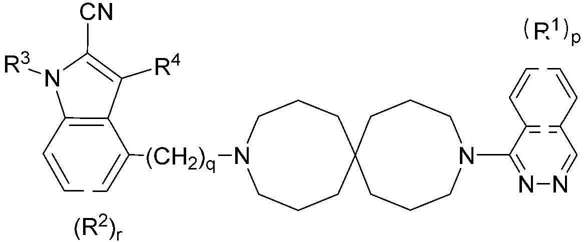 |
| I-A-III-a-I | 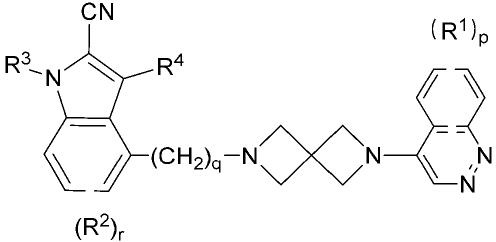 |
| I-A-III-b-I | 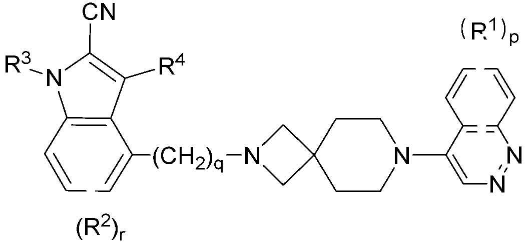 |
| I-A-III-c-I | 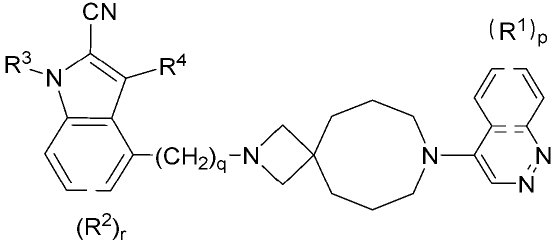 |
| I-A-III-d-I | 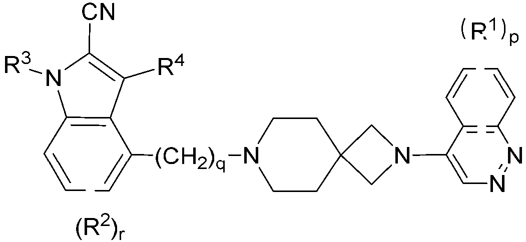 |
| I-A-III-e-I | 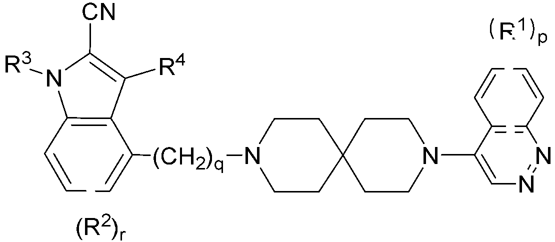 |
| I-A-III-f-I | 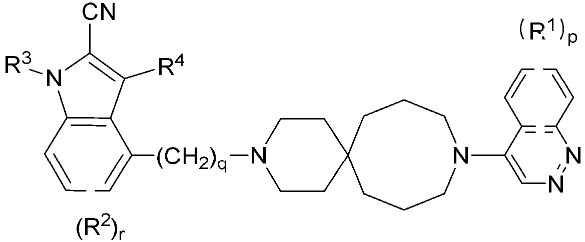 |
| I-A-III-g-I | 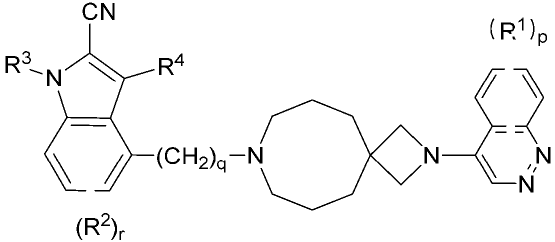 |
| I-A-III-h-I | 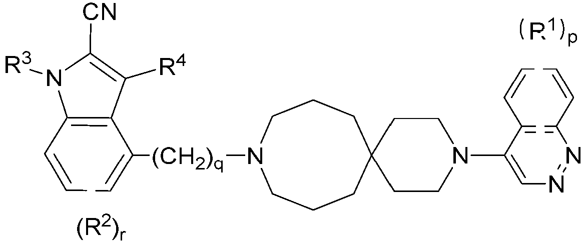 |
| I-A-III-i-I | 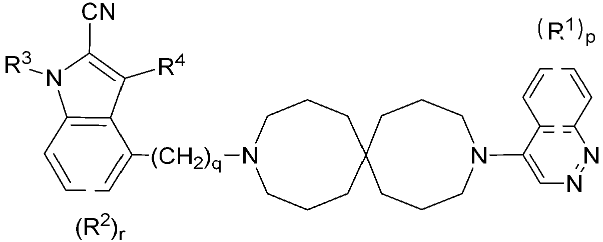 |
| I-A-IV-a-I | 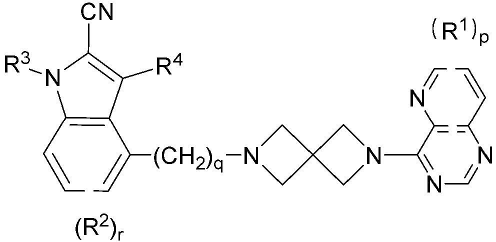 |
| I-A-IV-b-I | 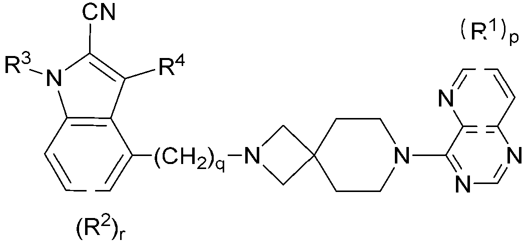 |
| I-A-IV-c-I | 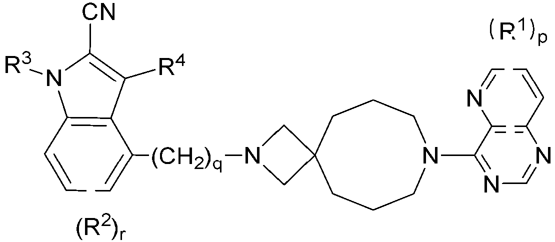 |
| I-A-IV-d-1 | 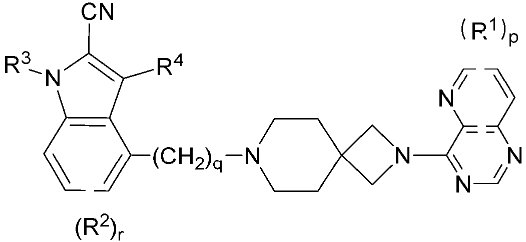 |
| I-A-IV-e-I | 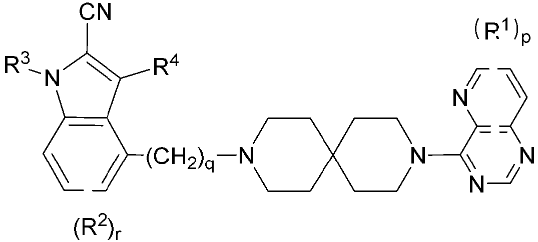 |
| I-A-IV-f-I | 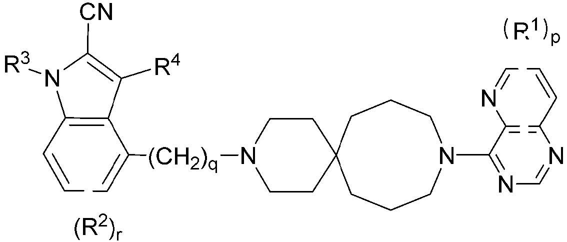 |
| I-A-IV-g-I | 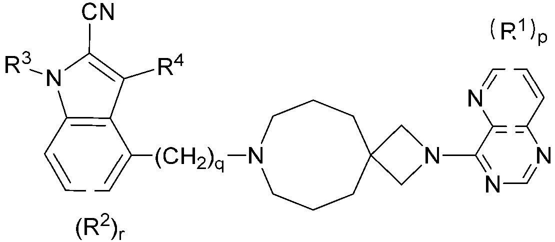 |
| I-A-IV-h-I | 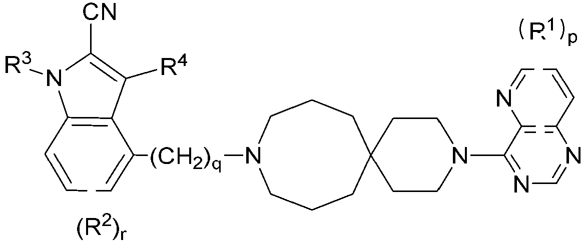 |
| I-A-IV-i-I | 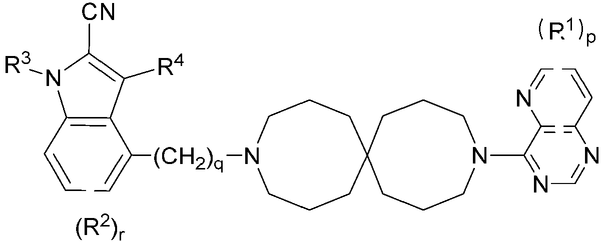 |
| I-A-V-a-I | 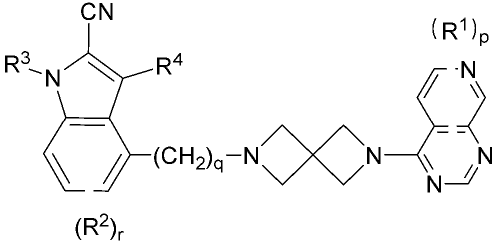 |
| I-A-V-b-I | 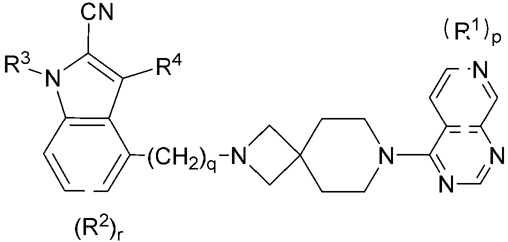 |
| I-A-V-c-I | 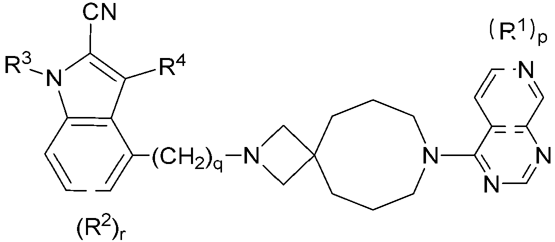 |
| I-A-V-d-I | 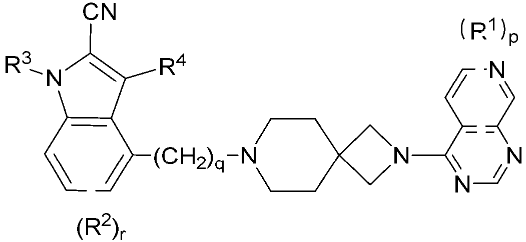 |
| I-A-V-e-I | 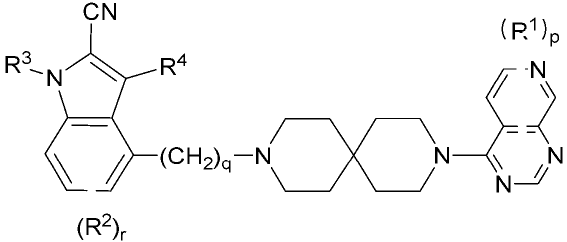 |
| I-A-V-f-I | 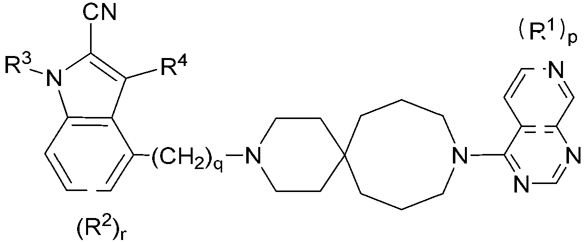 |
| I-A-V-g-I | 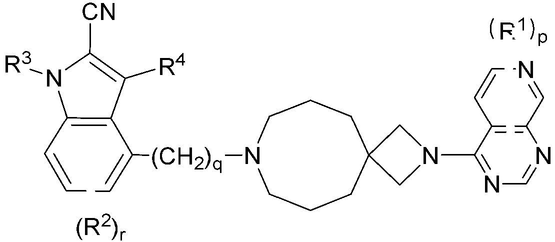 |
| I-A-V-h-I | 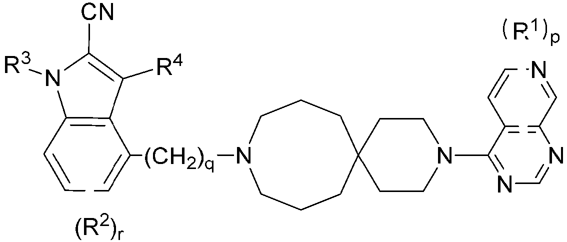 |
| I-A-V-i-I | 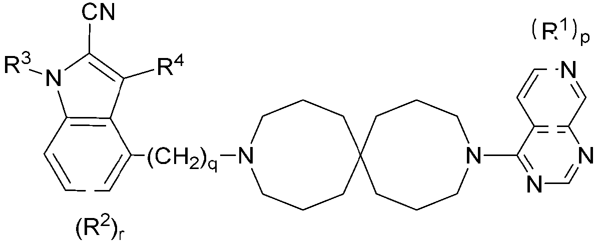 |
| I-A-VI-a-I | 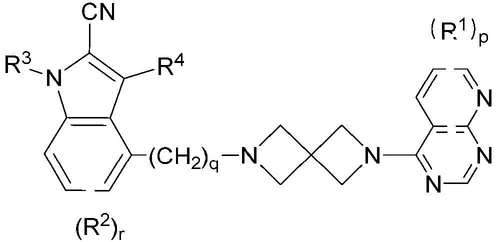 |
| I-A-VI-b-I | 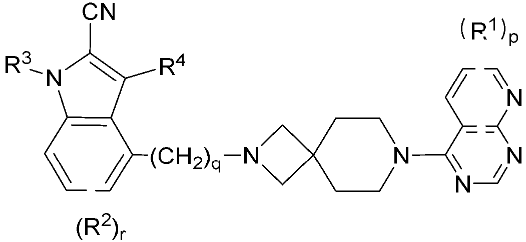 |
| I-A-VI-c-I | 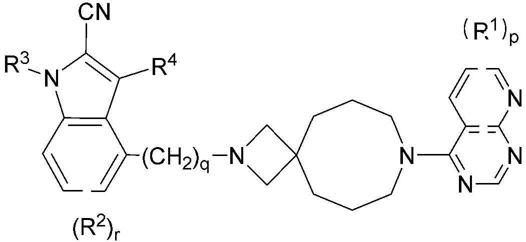 |
| I-A-VI-d-I | 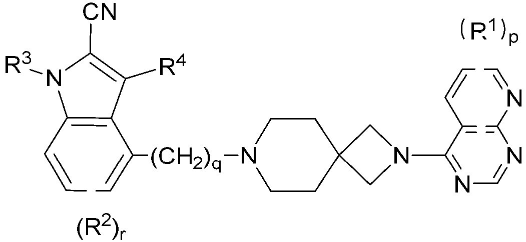 |
| I-A-VI-e-I | 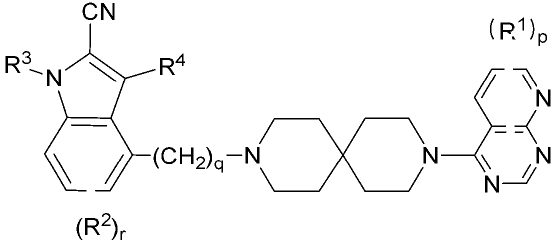 |
| I-A-VI-f-1 | 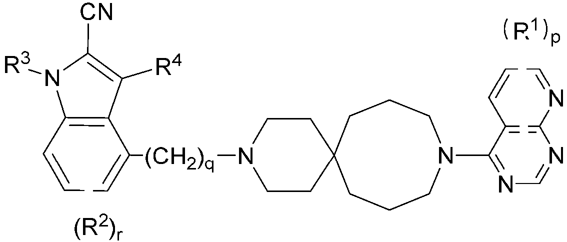 |
| I-A-VI-g-I | 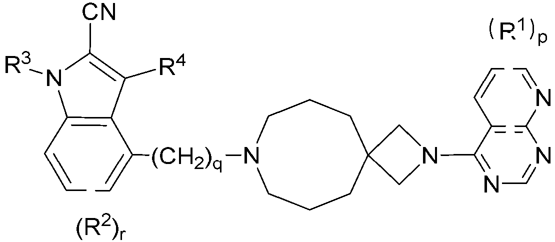 |
| I-A-VI-h-I | 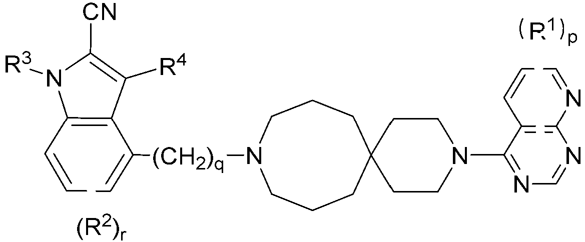 |
| I-A-VI-i-I | 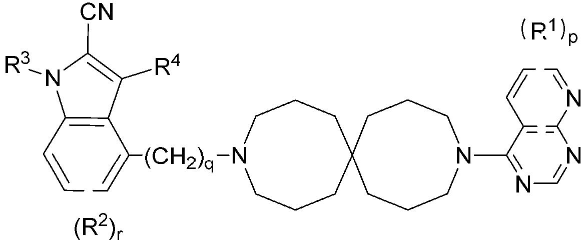 |
| I-A-VII-a-I | 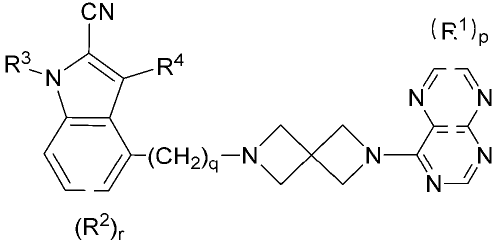 |
| I-A-VII-b-1 | 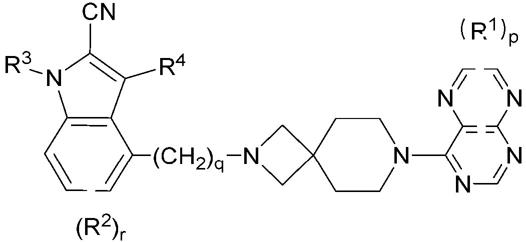 |
| I-A-VII-c-I | 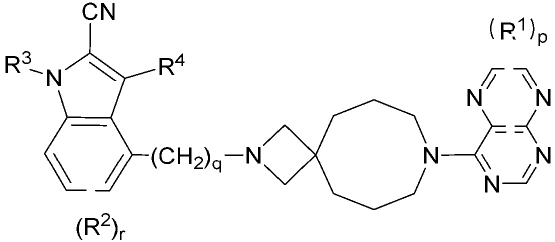 |
| I-A-VII-d-I | 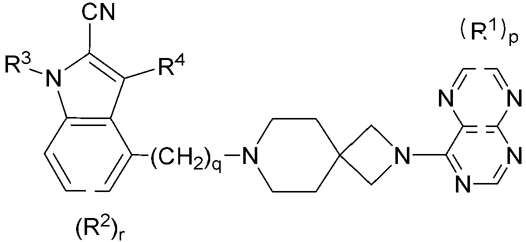 |
| I-A-VII-e-I | 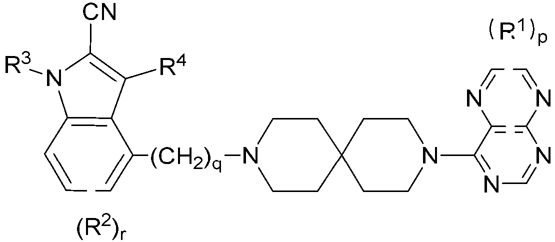 |
| I-A-VII-f-I | 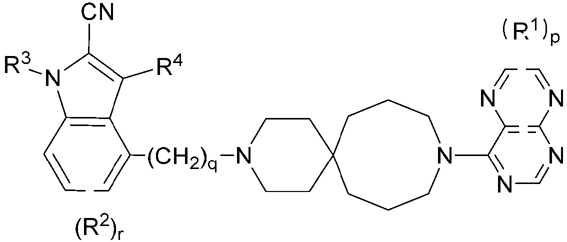 |
| I-A-VII-g-I | 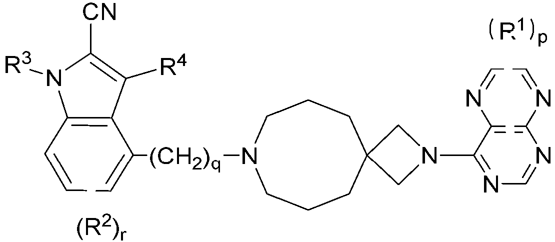 |
| I-A-VII-h-I | 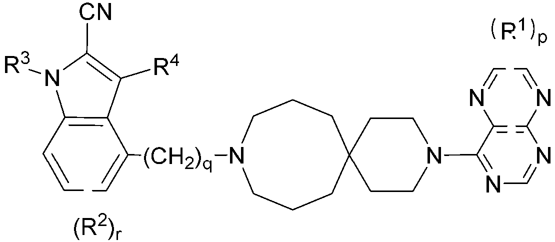 |
| I-A-VII-i-I | 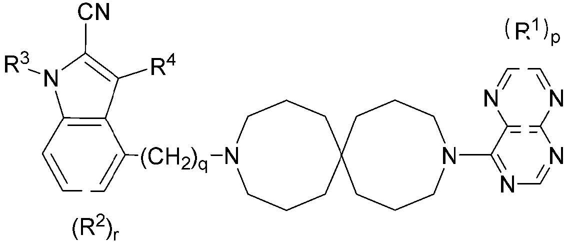 |
| I-B-I-a-I | 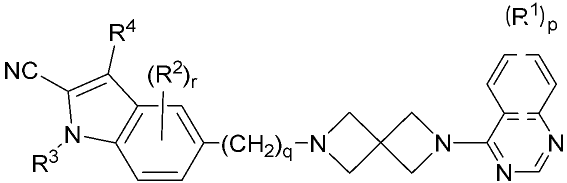 |
| I-B-I-b-I | 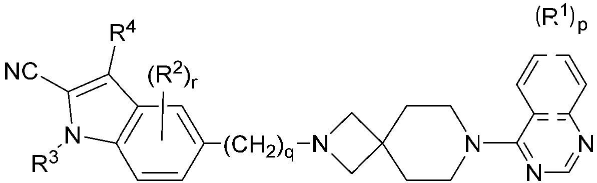 |
| I-B-I-c-I | 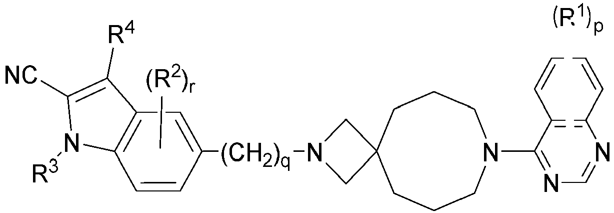 |
| I-B-I-d-I | 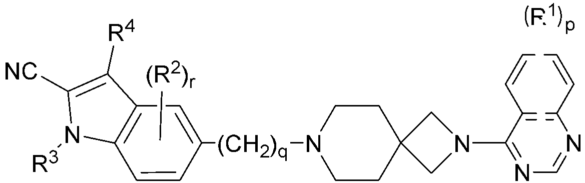 |
| I-B-I-e-I | 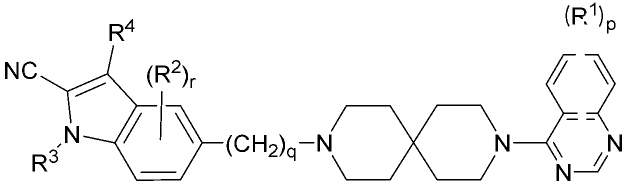 |
| I-B-I-f-I | 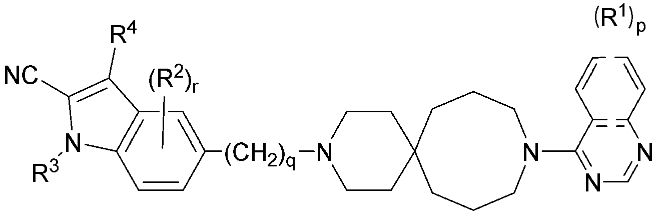 |
| I-B-I-g-I | 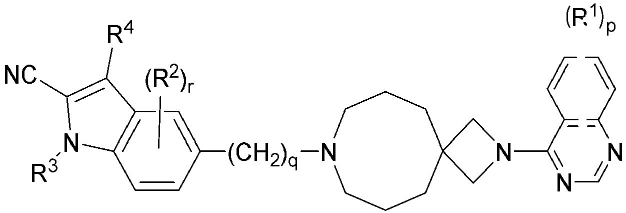 |
| I-B-I-h-I | 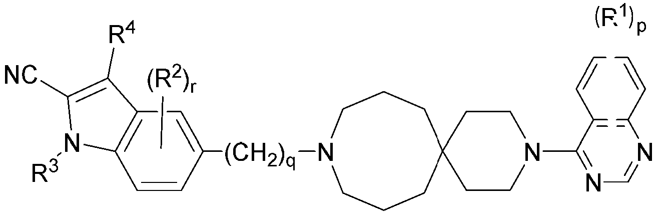 |
| I-B-I-i-I | 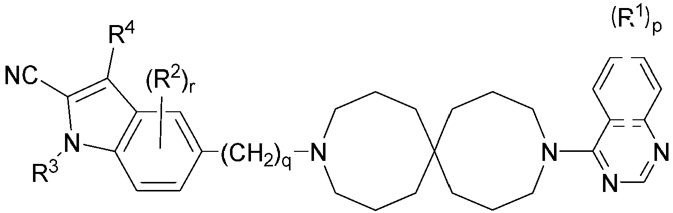 |
| I-B-II-a-I | 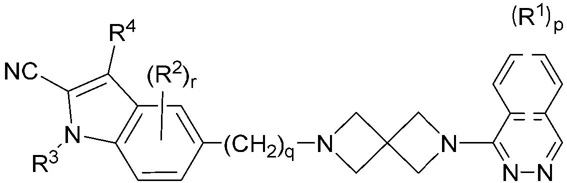 |
| I-B-II-b-I | 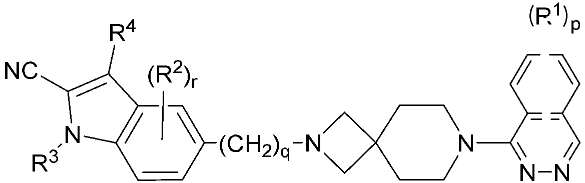 |
| I-B-II-c-I | 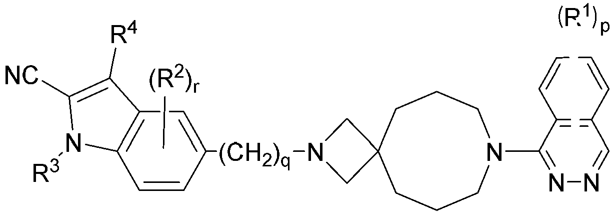 |
| I-B-II-d-I | 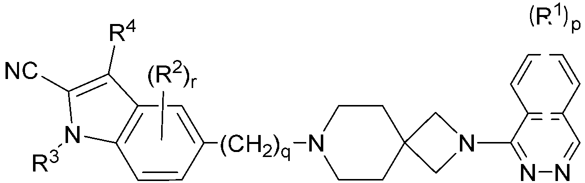 |
| I-B-II-e-I | 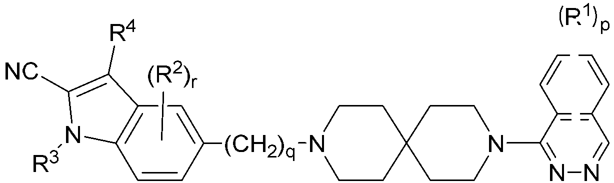 |
| I-B-II-f-I | 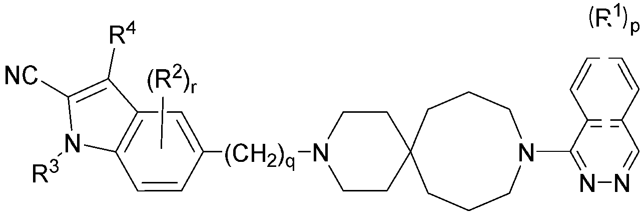 |
| I-B-II-g-I | 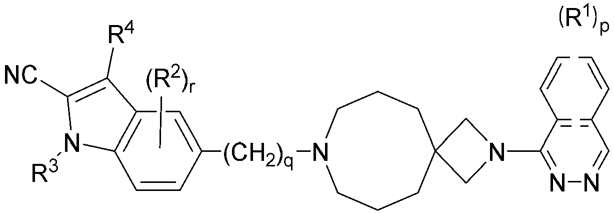 |
| I-B-II-h-I | 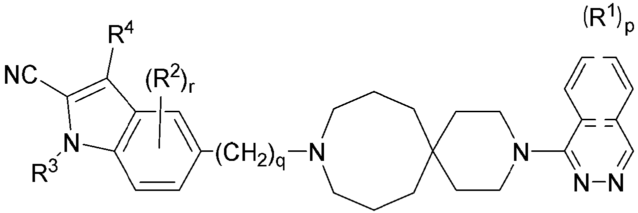 |
| I-B-II-i-I | 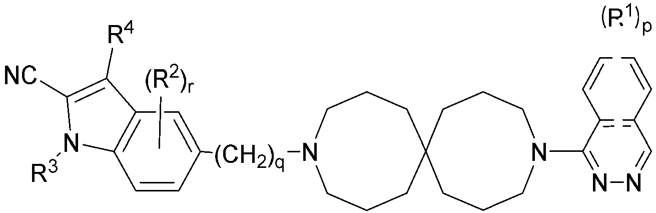 |
| I-B-III-a-I | 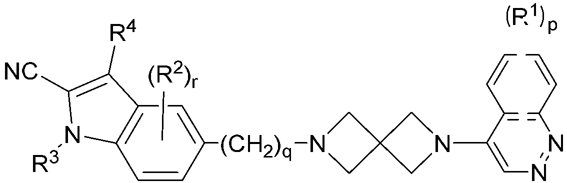 |
| I-B-III-b-I | 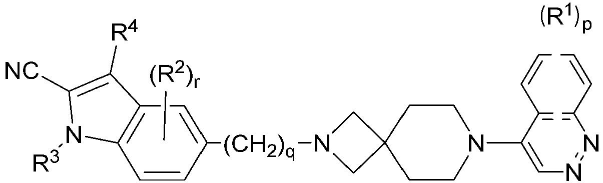 |
| I-B-III-c-I | 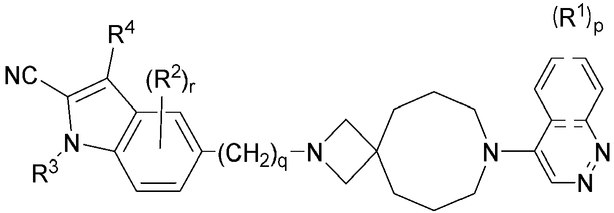 |
| I-B-III-d-I | 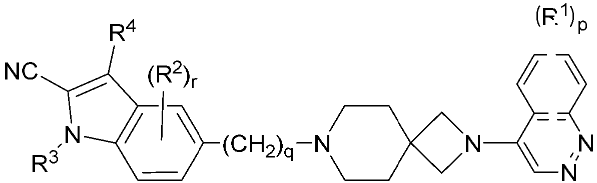 |
| I-B-III-e-I | 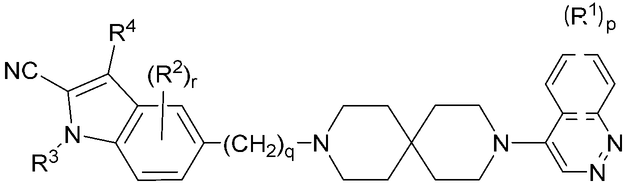 |
| I-B-III-f-I | 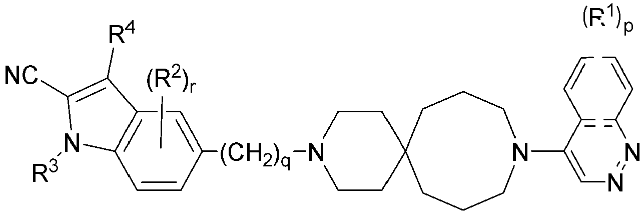 |
| I-B-III-g-I | 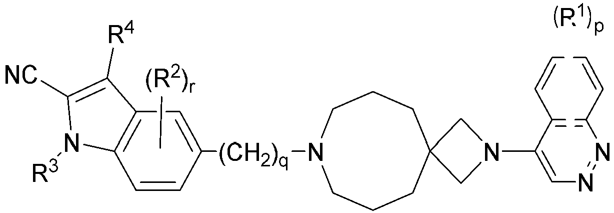 |
| I-B-III-h-I | 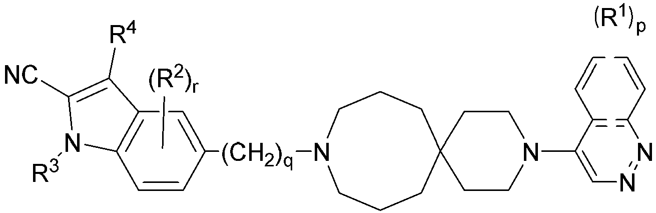 |
| I-B-III-i-I | 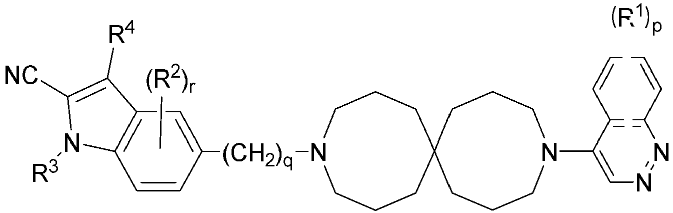 |
| I-B-IV-a-I | 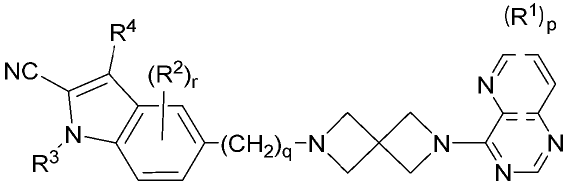 |
| I-B-IV-b-I | 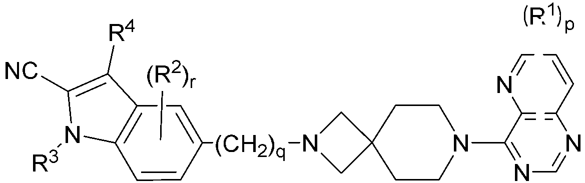 |
| I-B-IV-c-I | 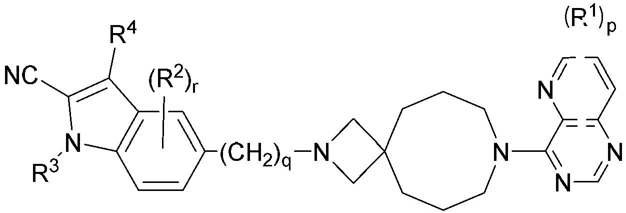 |
| I-B-IV-d-I | 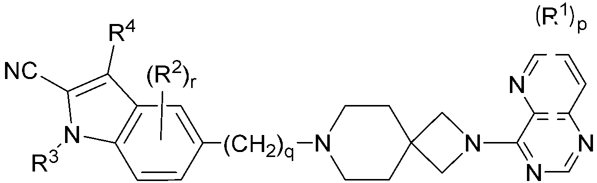 |
| I-B-IV-e-I | 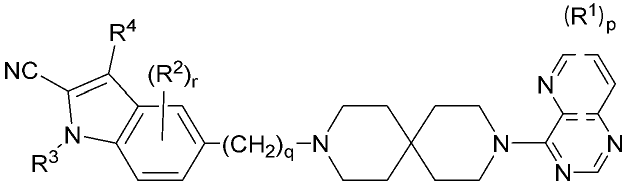 |
| I-B-IV-f-I | 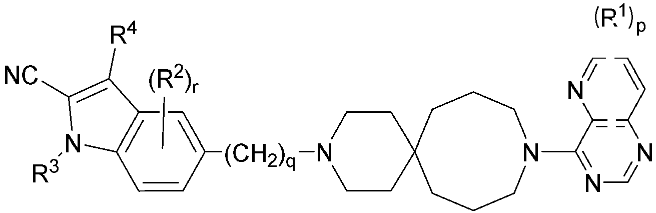 |
| I-B-IV-g-I | 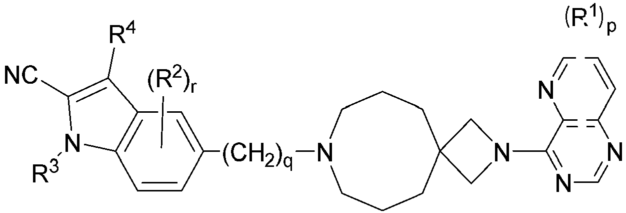 |
| I-B-IV-h-I | 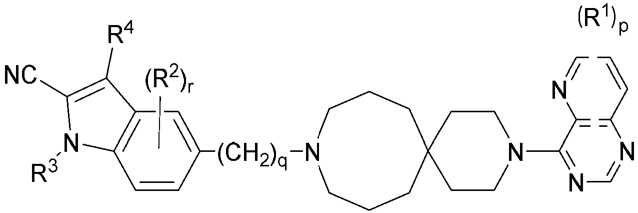 |
| I-B-IV-i-I | 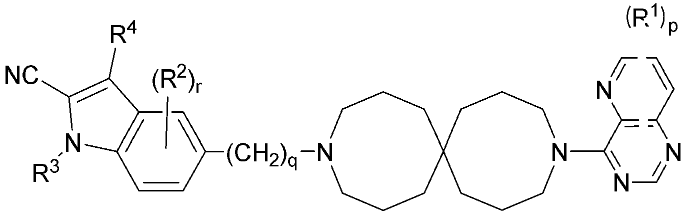 |
| I-B-V-a-I | 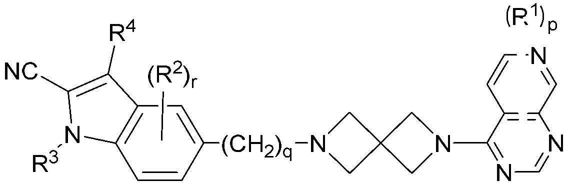 |
| I-B-V-b-I | 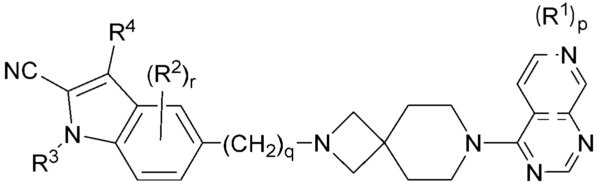 |
| I-B-V-c | 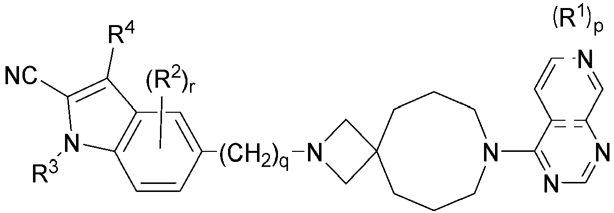 |
| I-B-V-d-I | 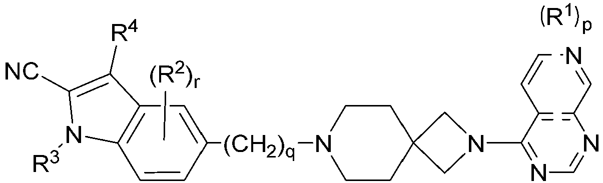 |
| I-B-V-e | 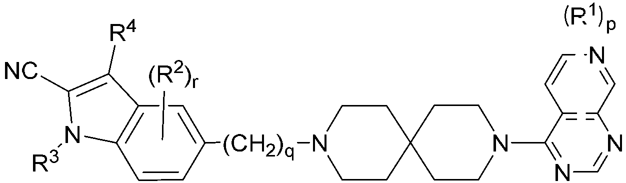 |
| I-B-V-f | 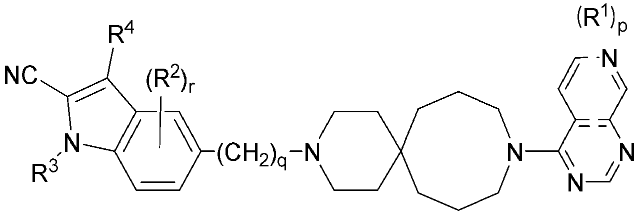 |
| I-B-V-g | 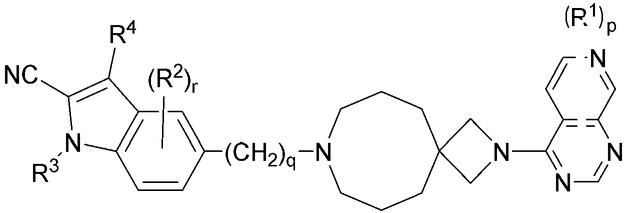 |
| I-B-V-h | 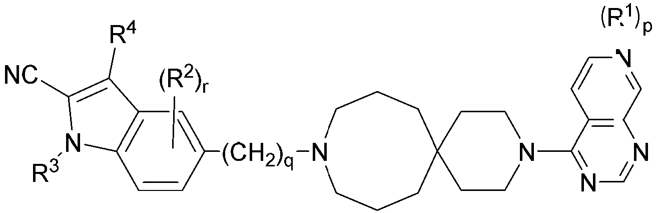 |
| I-B-V-i | 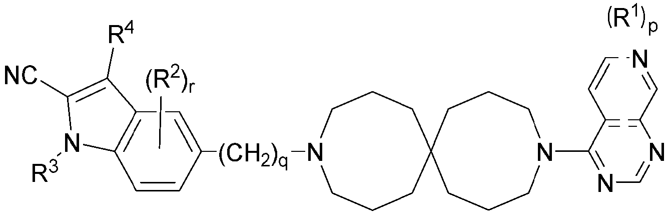 |
| I-B-VI-a | 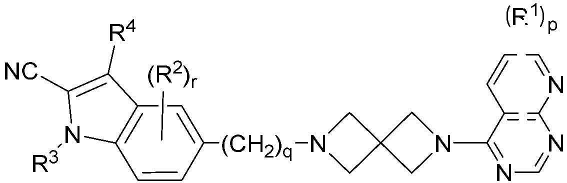 |
| I-B-VI-b | 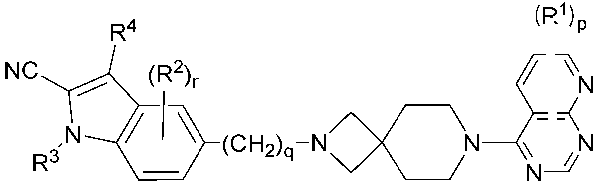 |
| I-B-VI-c | 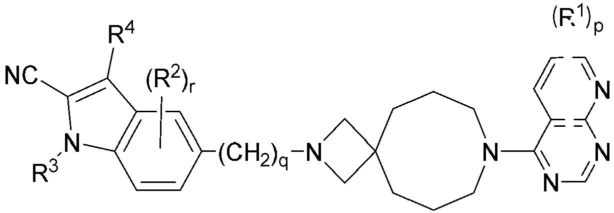 |
| I-B-VI-d-I | 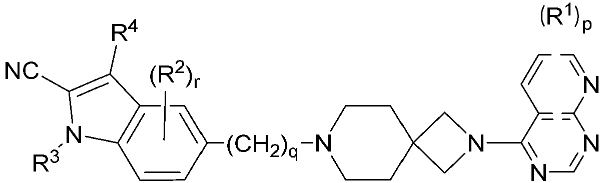 |
| I-B-VI-e | 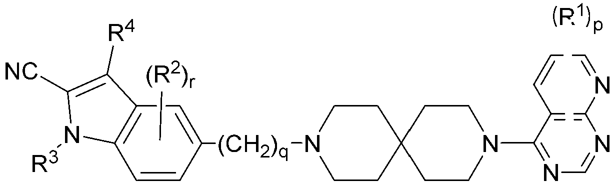 |
| I-B-VI-f | 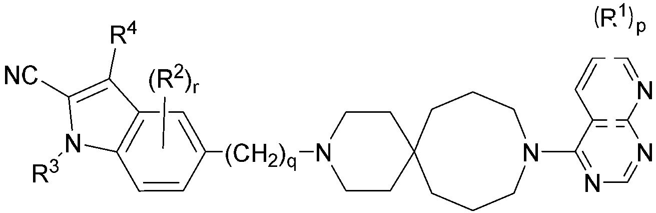 |
| I-B-VI-g | 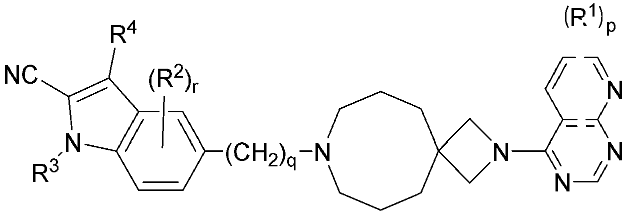 |
| I-B-VI-h | 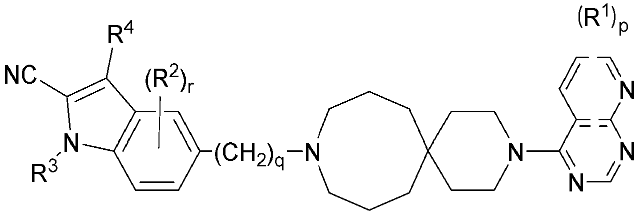 |
| I-B-VI-i | 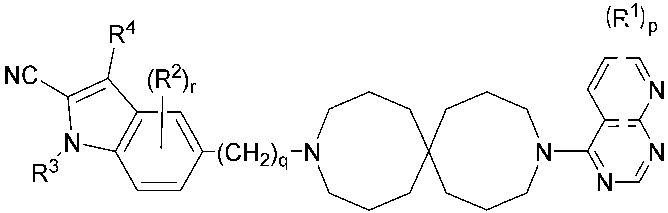 |
| I-B-VII-a | 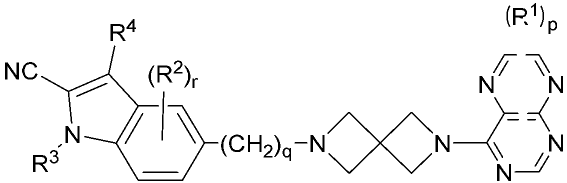 |
| I-B-VII-b | 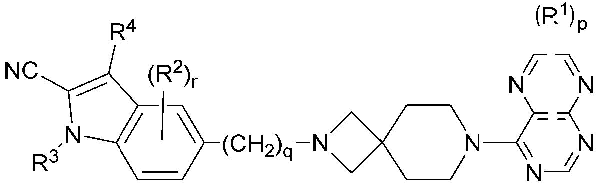 |
| I-B-VII-c | 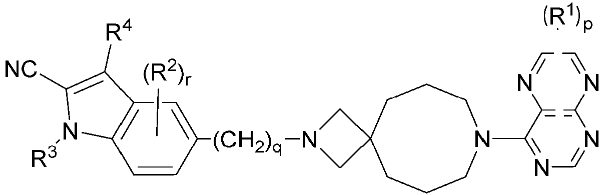 |
| I-B-VII-d-I | 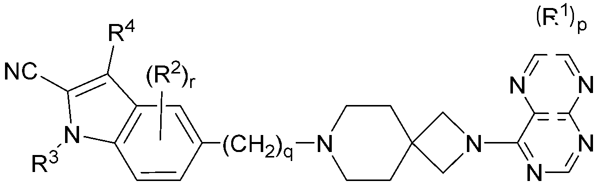 |
| I-B-VII-e | 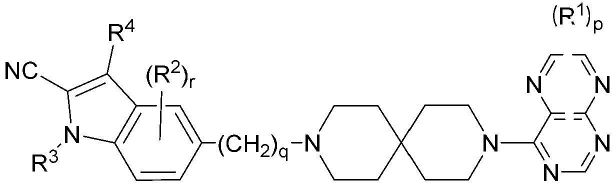 |
| I-B-VII-f | 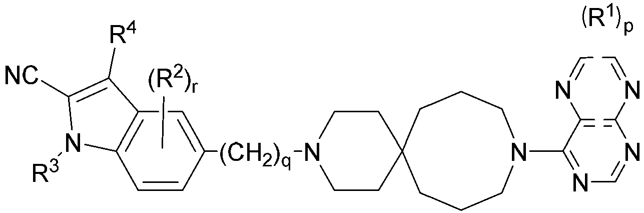 |
| I-B-VII-g | 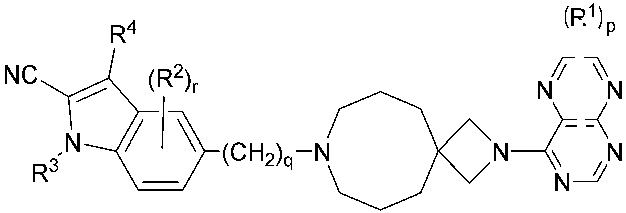 |
| I-B-VII-h | 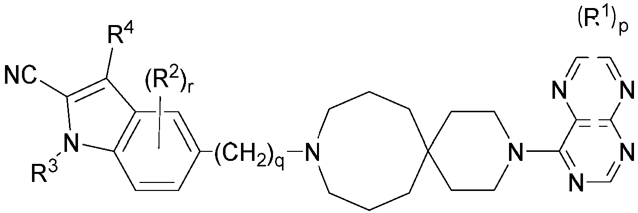 |
| I-B-VII-i | 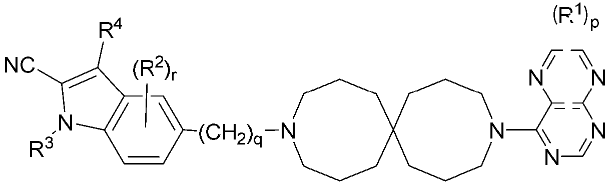 |
| I-C-1-a | 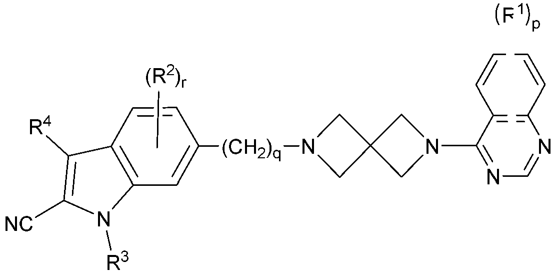 |
| I-C-I-b | 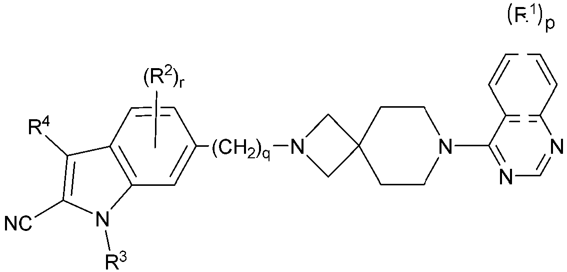 |
| I-C-I-c | 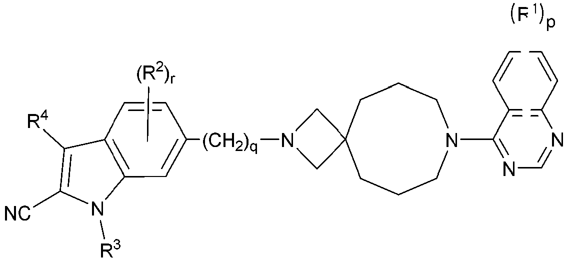 |
| I-C-I-d | 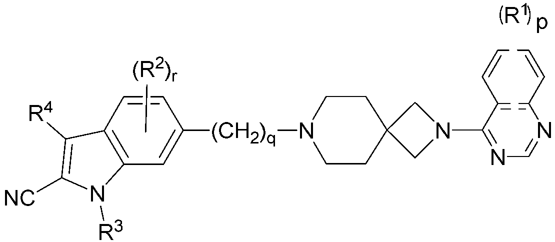 |
| I-C-I-e | 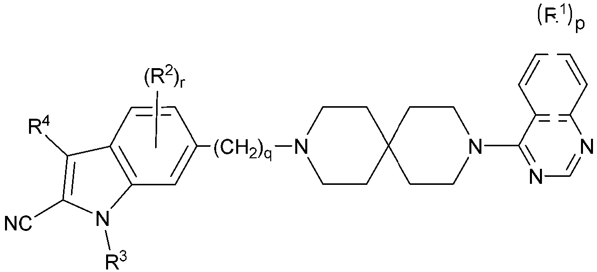 |
| I-C-I-f | 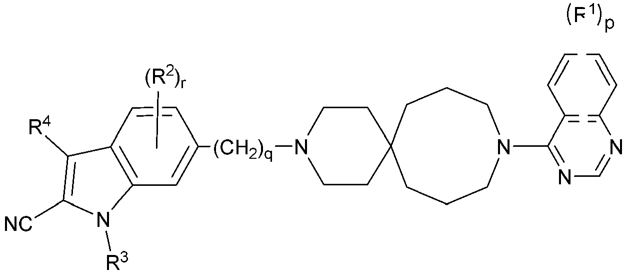 |
| I-C-I-g | 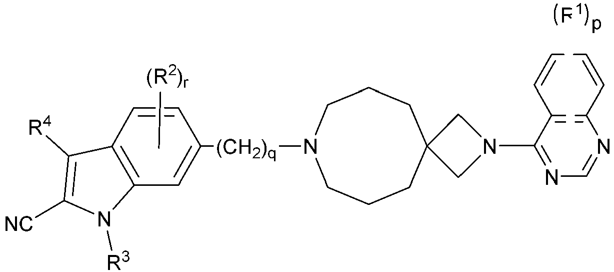 |
| I-C-I-h | 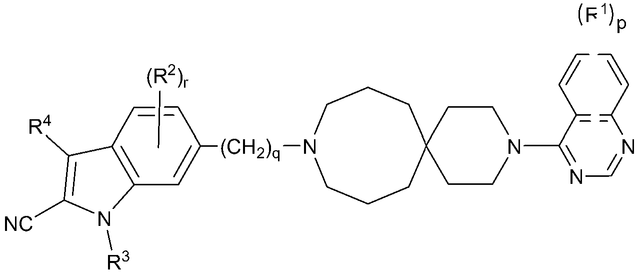 |
| I-C-I-i | 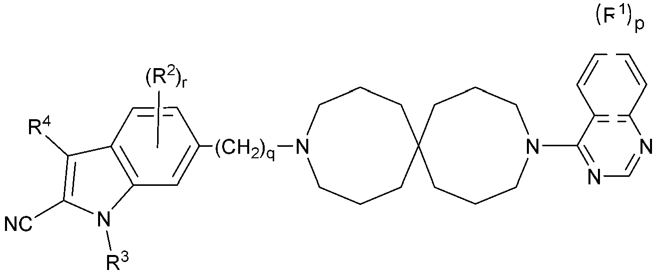 |
| I-C-II-a | 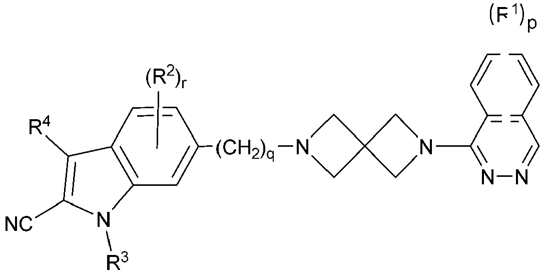 |
| I-C-II-b | 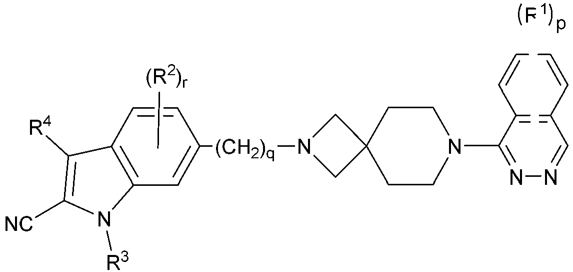 |
| I-C-II-c | 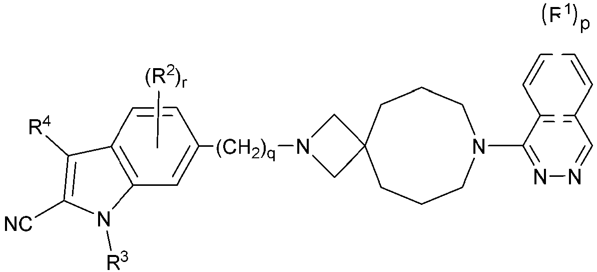 |
| I-C-II-d | 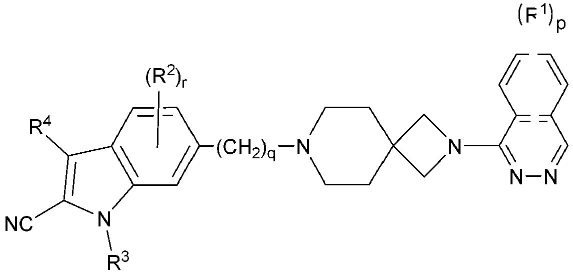 |
| I-C-II-e | 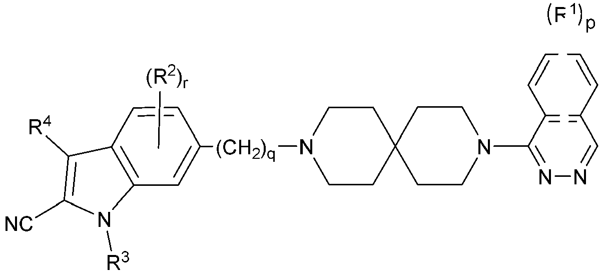 |
| I-C-II-f | 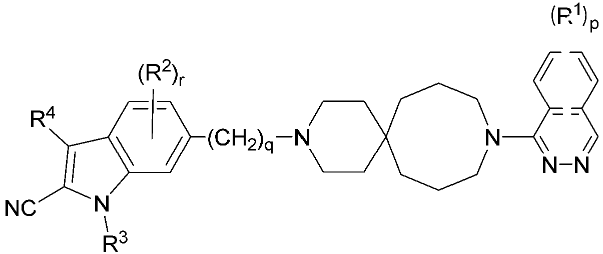 |
| I-C-II-g | 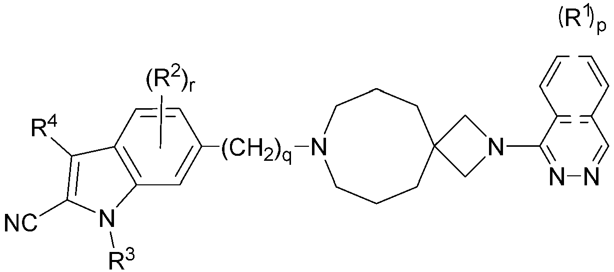 |
| I-C-II-h | 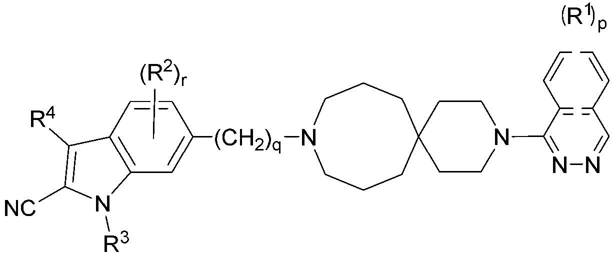 |
| I-C-II-i | 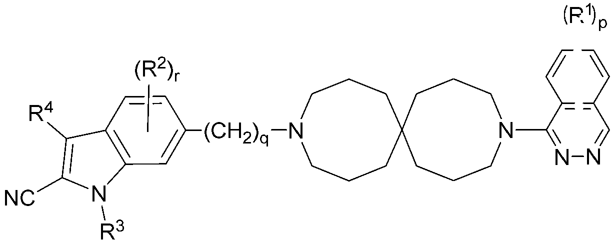 |
| I-C-III-a | 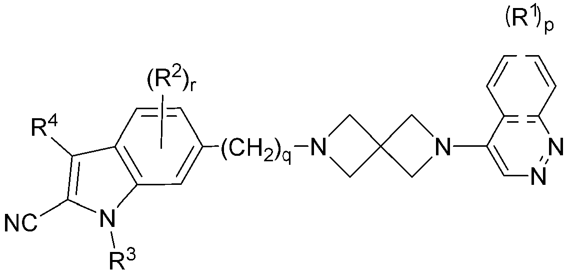 |
| I-C-III-b | 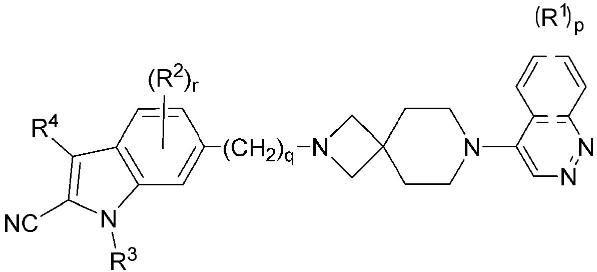 |
| I-C-III-c | 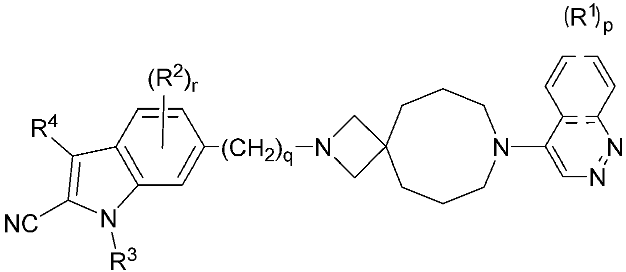 |
| I-C-III-d | 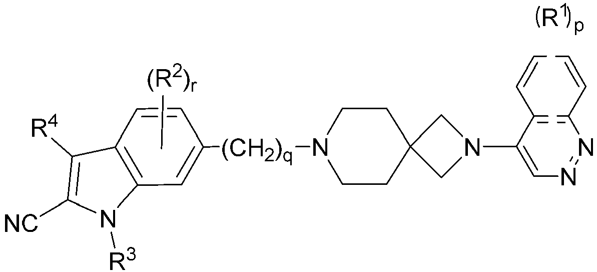 |
| I-C-III-e | 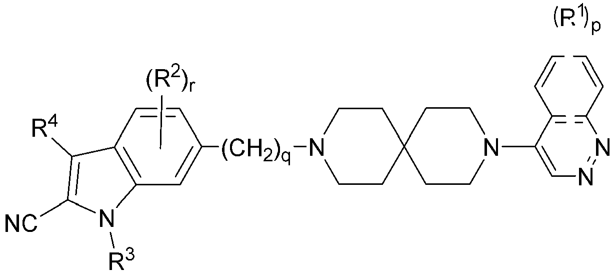 |
| I-C-III-f | 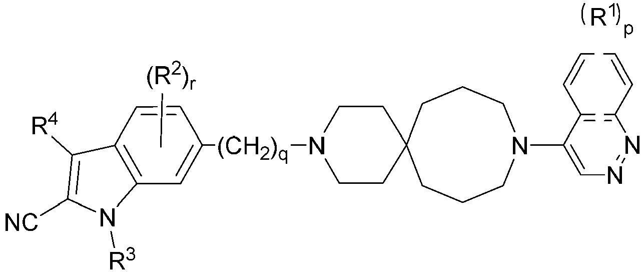 |
| I-C-III-g | 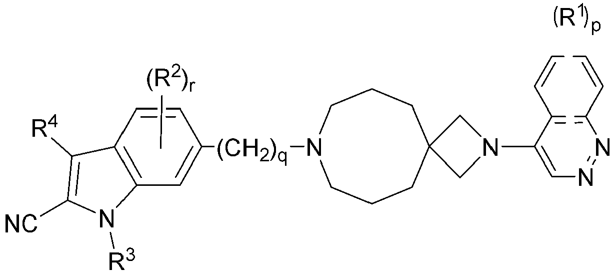 |
| I-C-III-h | 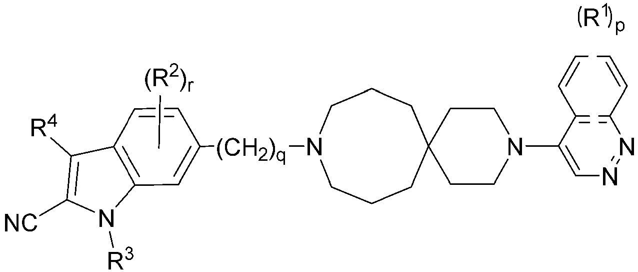 |
| I-C-III-i | 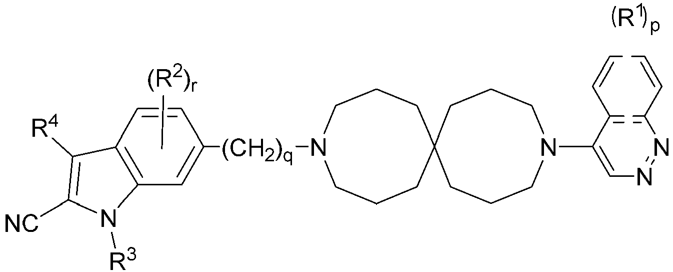 |
| I-C-IV-a | 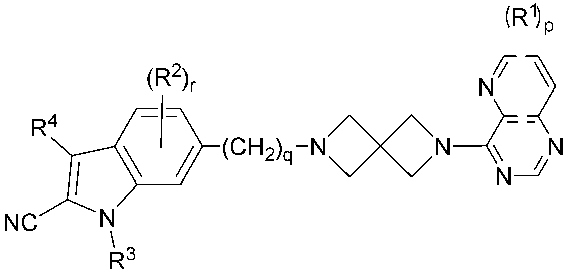 |
| I-C-IV-b | 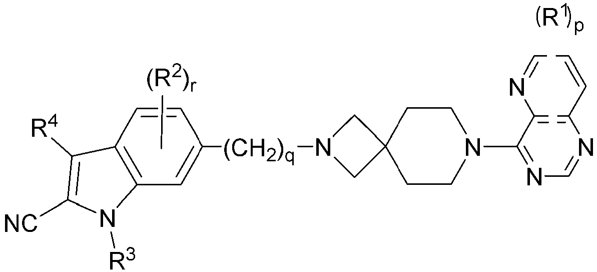 |
| I-C-IV-c | 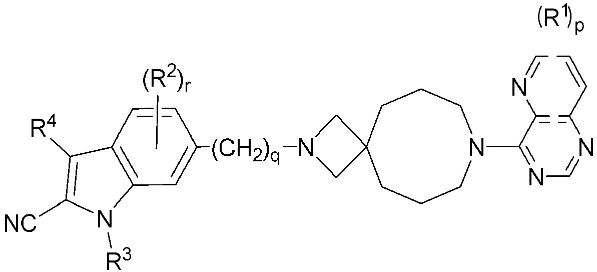 |
| I-C-IV-d | 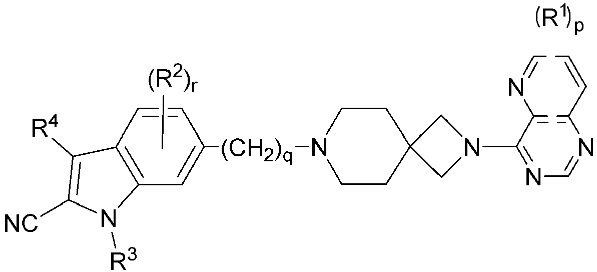 |
| I-C-IV-e | 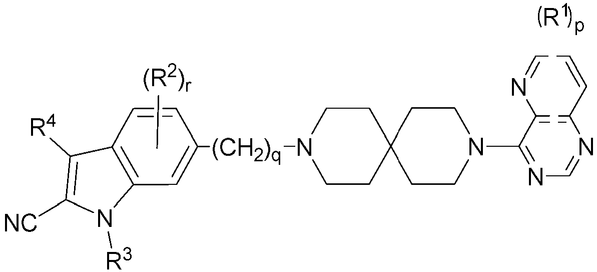 |
| I-C-IV-f | 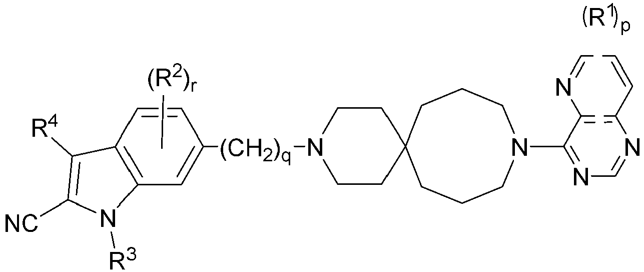 |
| I-C-IV-g | 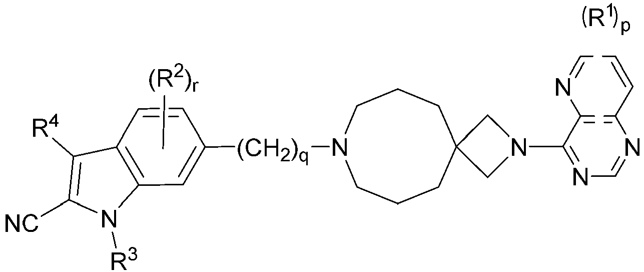 |
| I-C-IV-h | 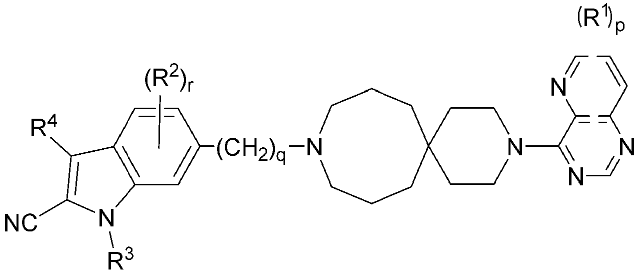 |
| I-C-IV-i | 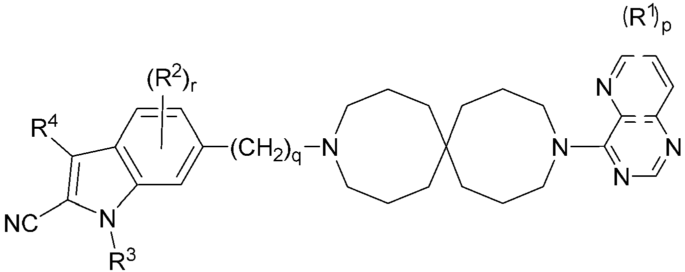 |
| I-C-V-a | 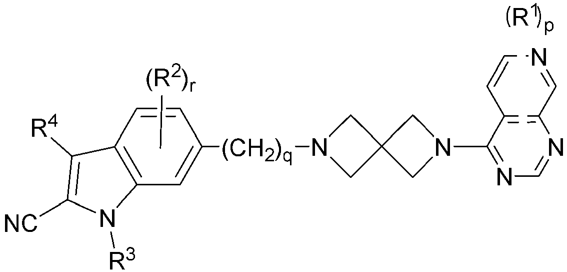 |
| I-C-V-b | 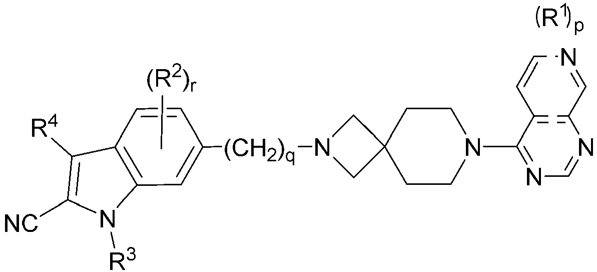 |
| I-C-V-c | 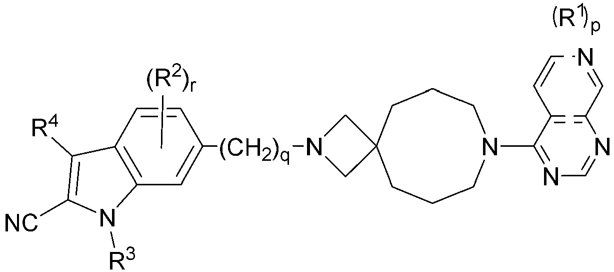 |
| I-C-V-d | 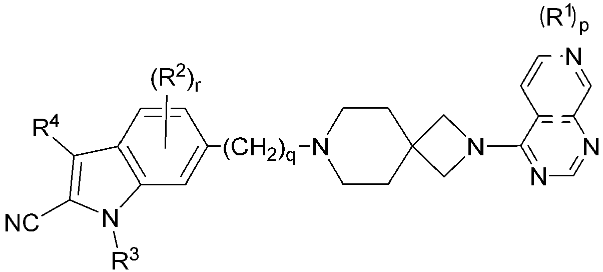 |
| I-C-V-e | 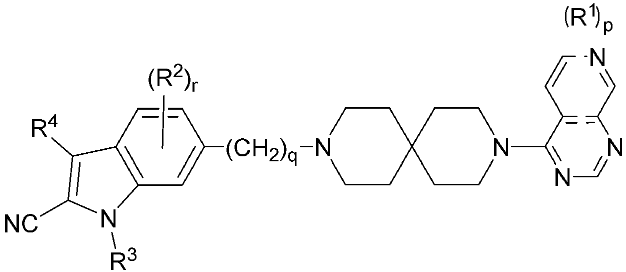 |
| I-C-V-f | 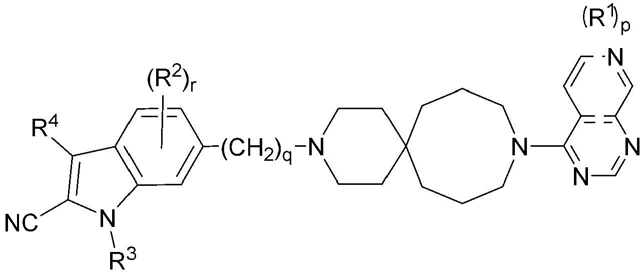 |
| I-C-V-g | 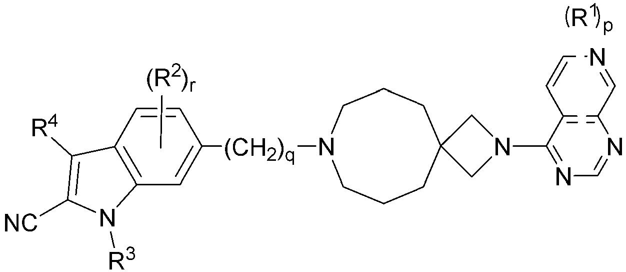 |
| I-C-V-h | 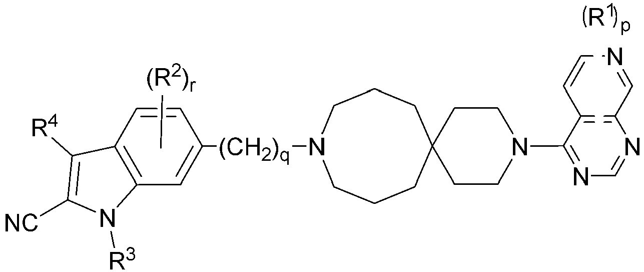 |
| I-C-V-i | 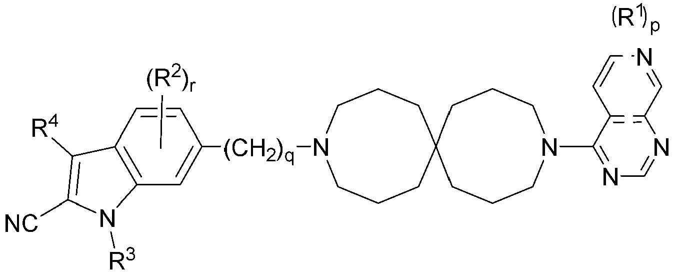 |
| I-C-VI-a | 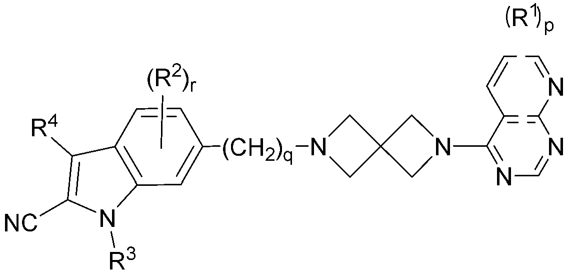 |
| I-C-VI-b | 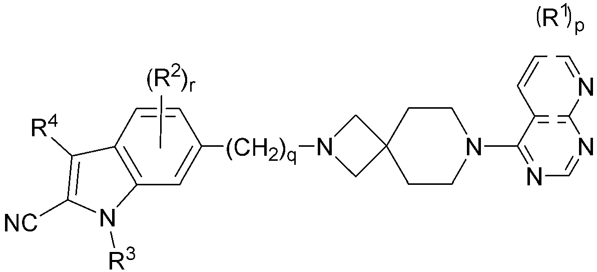 |
| I-C-VI-c | 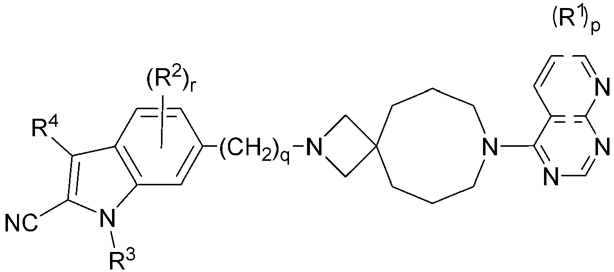 |
| I-C-VI-d | 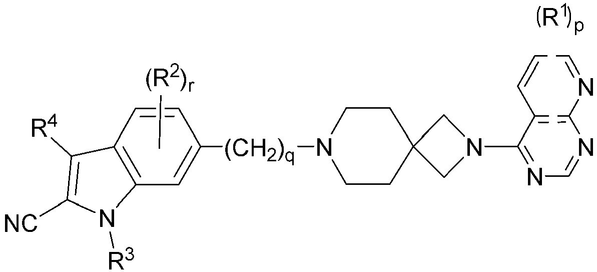 |
| I-C-VI-e | 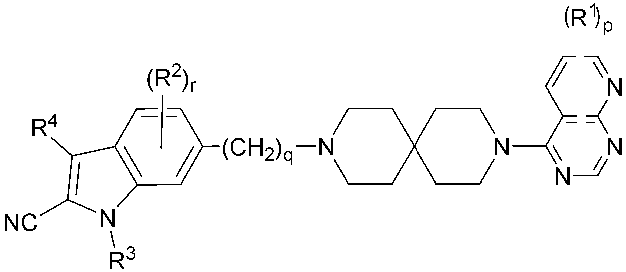 |
| I-C-VI-f | 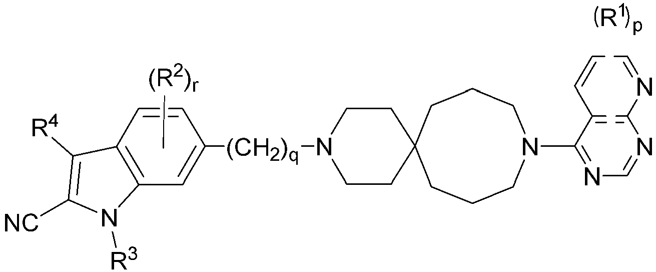 |
| I-C-VI-g | 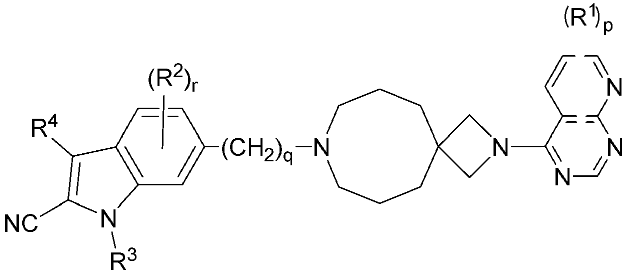 |
| I-C-VI-h | 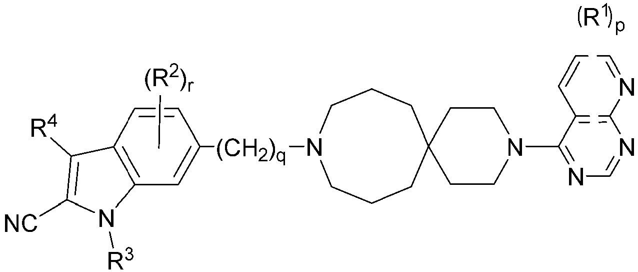 |
| I-C-VI-i | 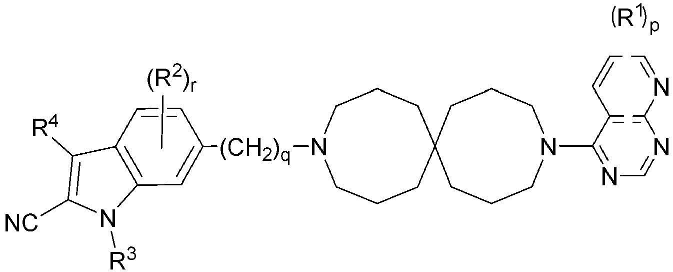 |
| I-C-VII-a | 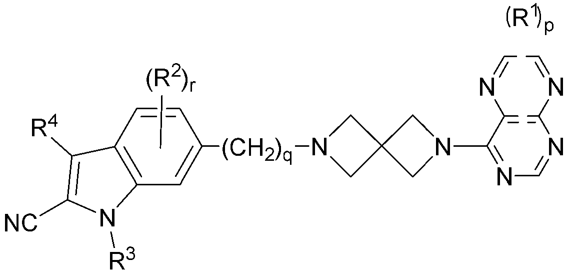 |
| I-C-VII-b | 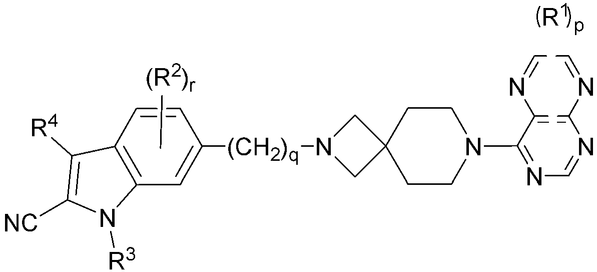 |
| I-C-VII-c | 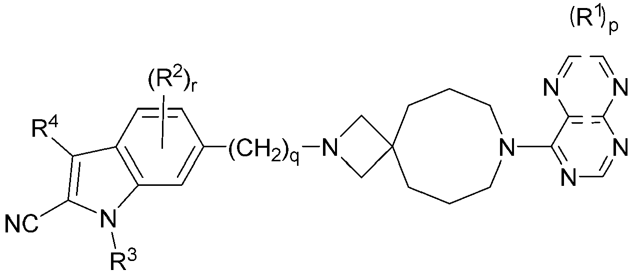 |
| I-C-VII-d | 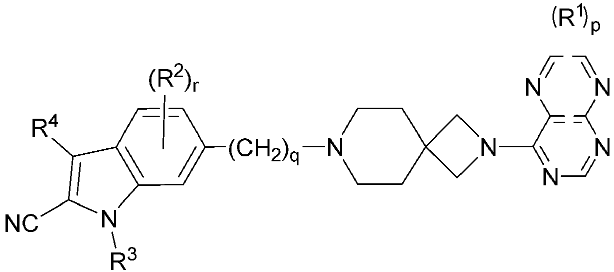 |
| I-C-VII-e | 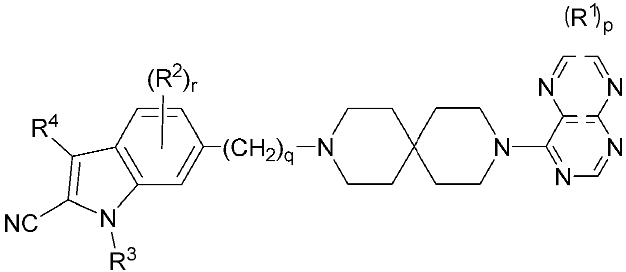 |
| I-C-VII-f | 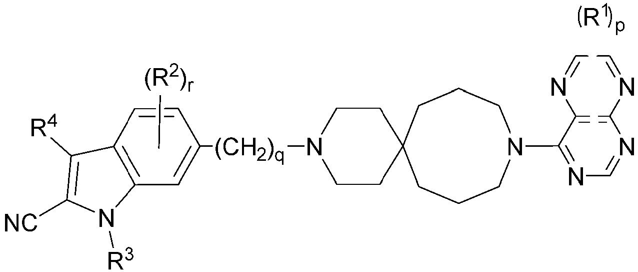 |
| I-C-VII-g | 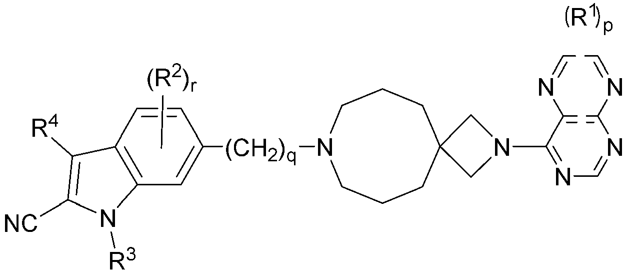 |
| I-C-VII-h | 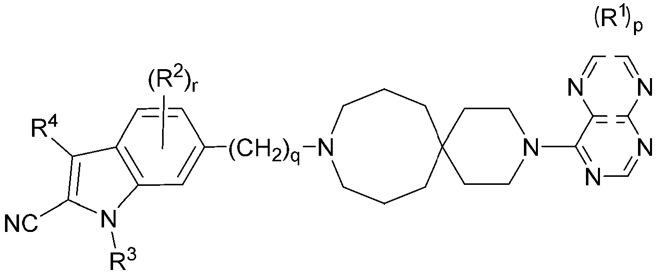 |
| I-C-VII-i | 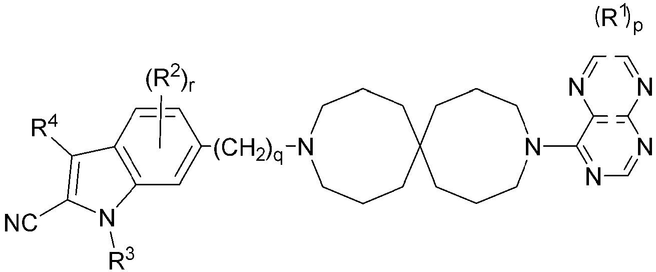 |
| I-D-I-a | 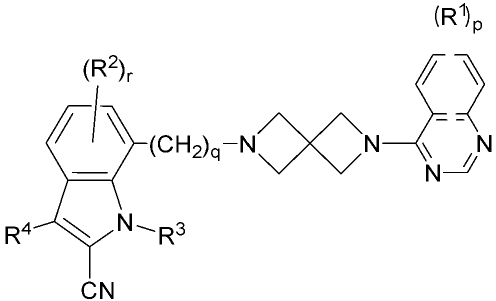 |
| I-D-1-b | 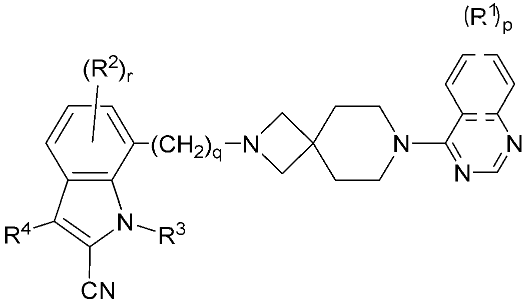 |
| I-D-I-c | 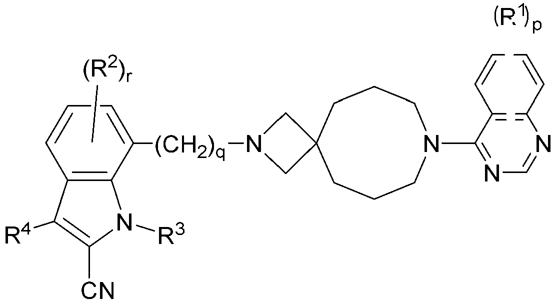 |
| I-D-1-d | 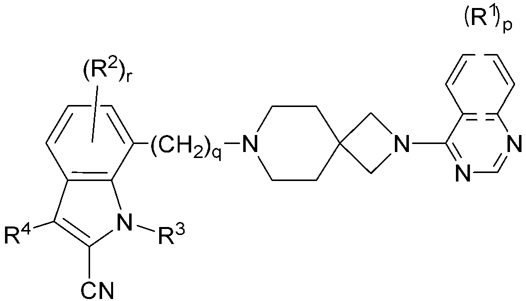 |
| I-D-I-e | 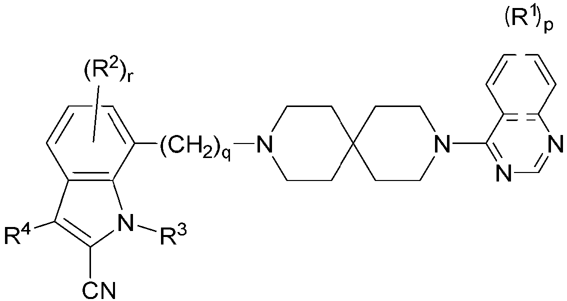 |
| I-D-1-f | 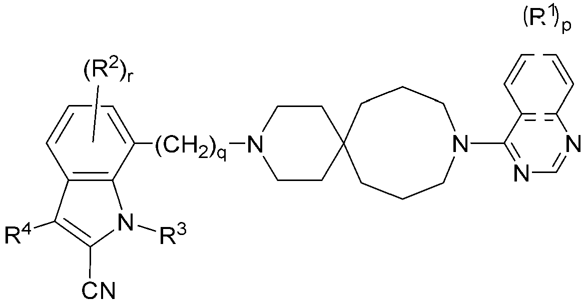 |
| I-D-I-g | 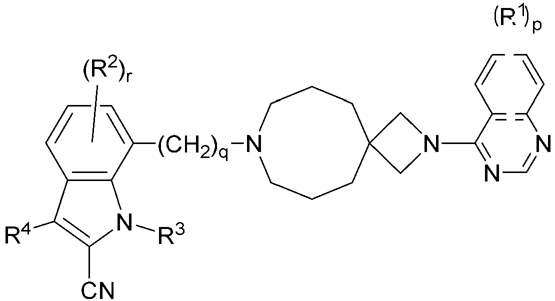 |
| I-D-1-h | 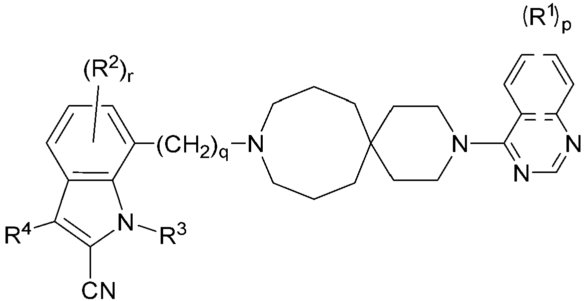 |
| I-D-I-i | 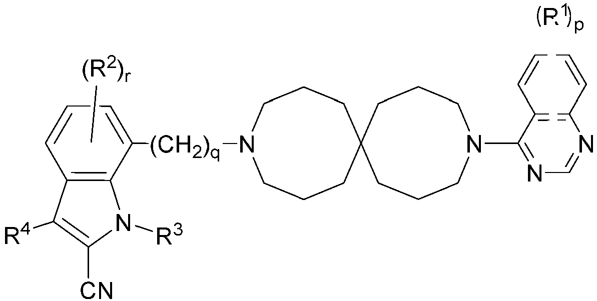 |
| I-D-II-a | 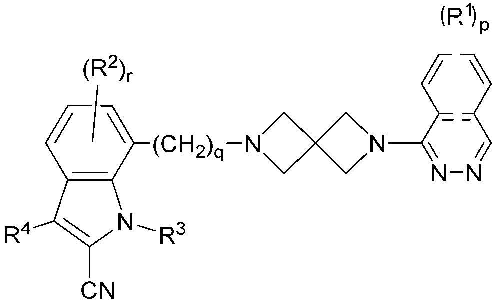 |
| I-D-II-b | 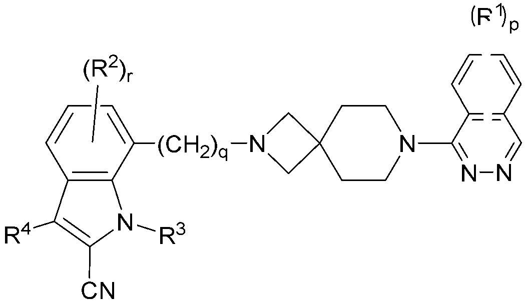 |
| I-D-II-c | 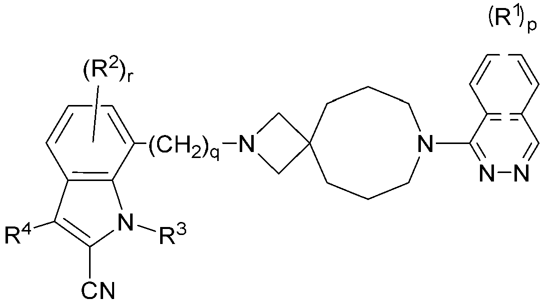 |
| I-D-II-d-I | 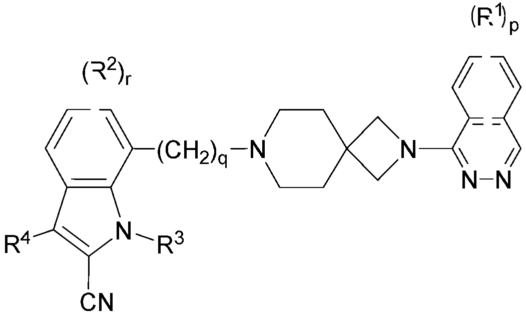 |
| I-D-II-e | 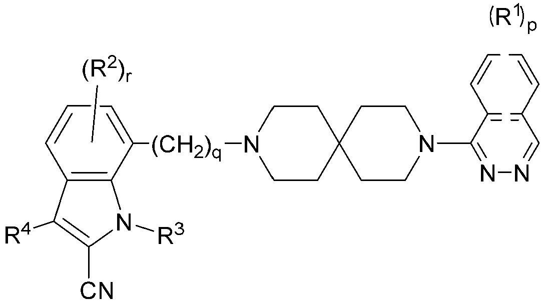 |
| I-D-II-f | 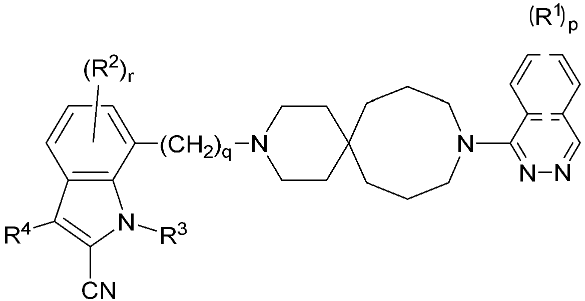 |
| I-D-II-g | 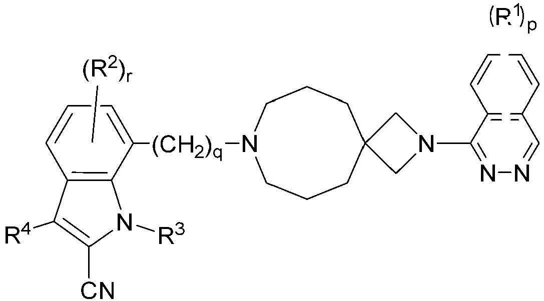 |
| I-D-II-h | 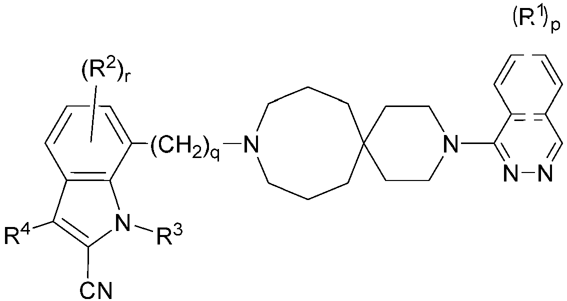 |
| I-D-II-i | 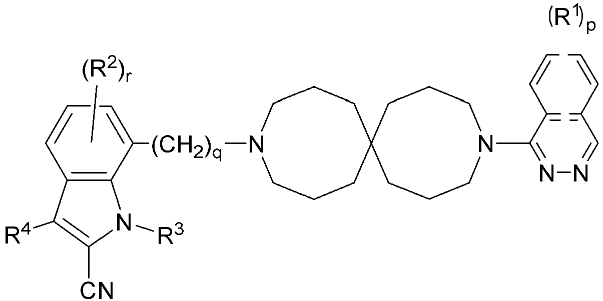 |
| I-D-III-a | 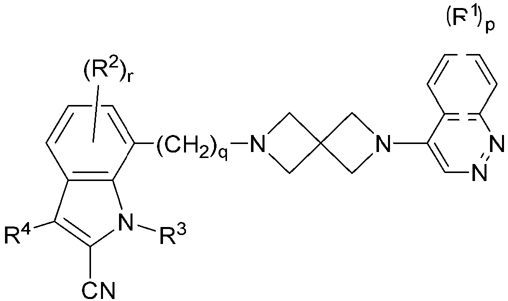 |
| I-D-III-b | 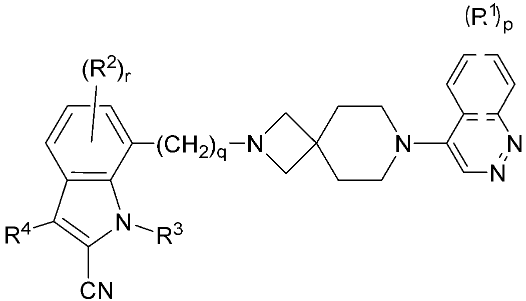 |
| I-D-III-c | 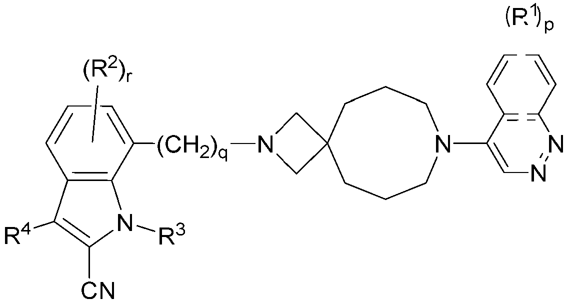 |
| I-D-III-d | 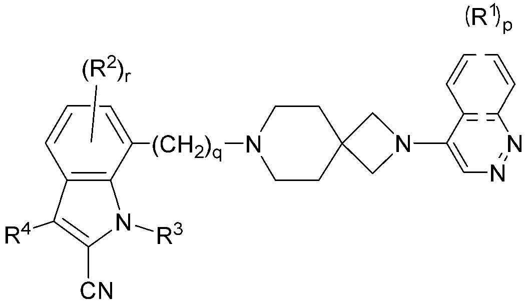 |
| I-D-III-e | 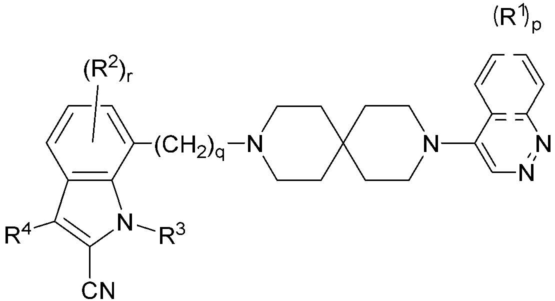 |
| I-D-III-f | 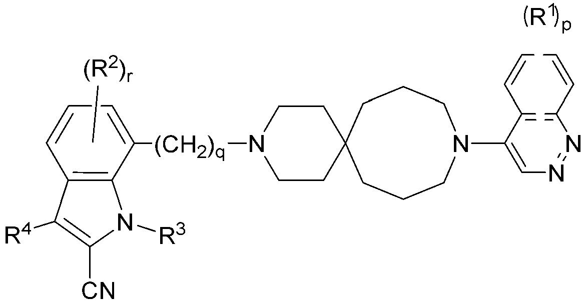 |
| I-D-111-g | 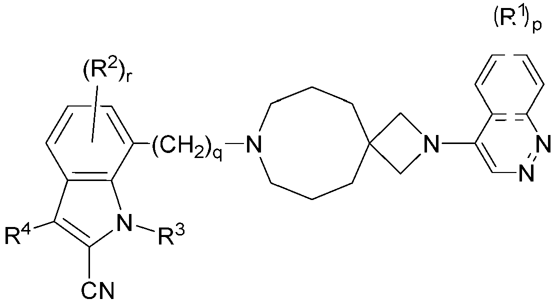 |
| I-D-III-h | 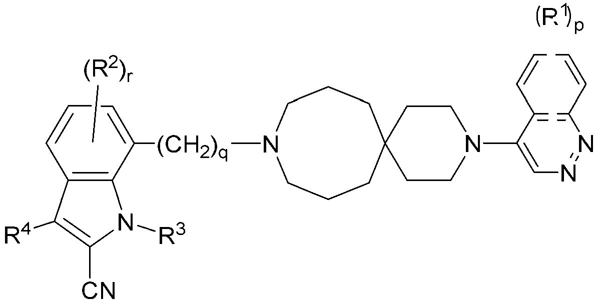 |
| I-D-III-i | 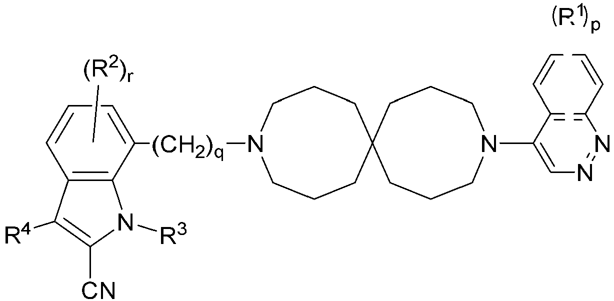 |
| I-D-IV-a | 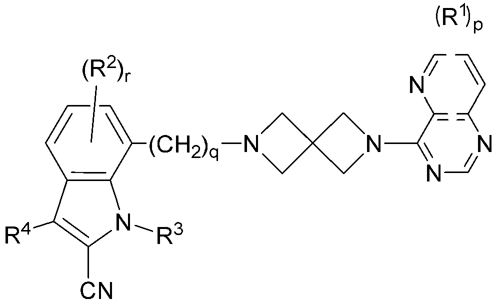 |
| I-D-IV-b | 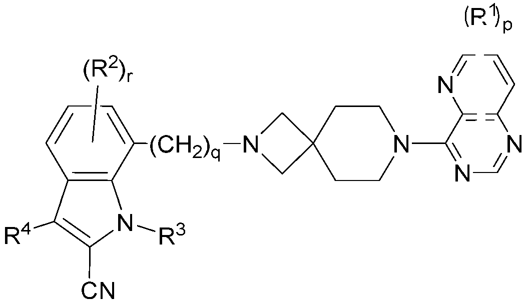 |
| I-D-IV-c | 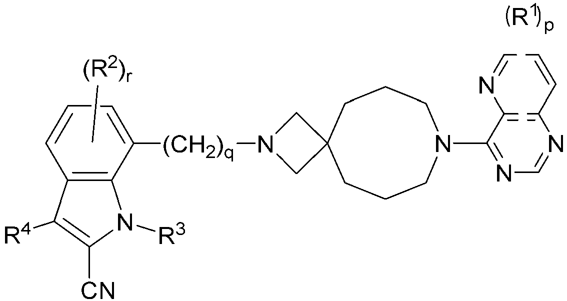 |
| I-D-IV-d | 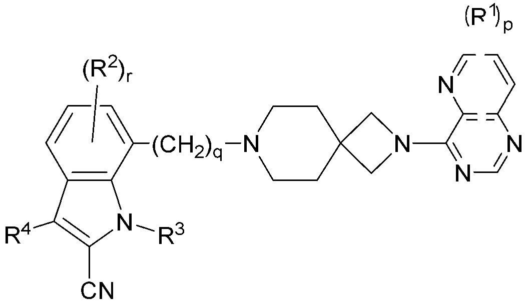 |
| I-D-IV-e | 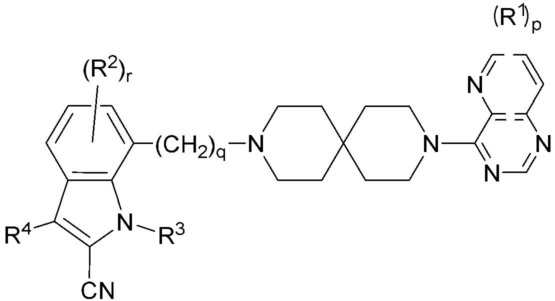 |
| I-D-1V-f | 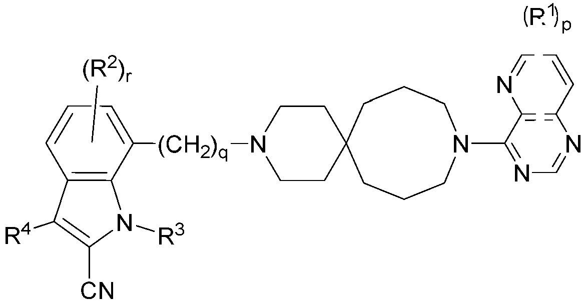 |
| I-D-1V-g | 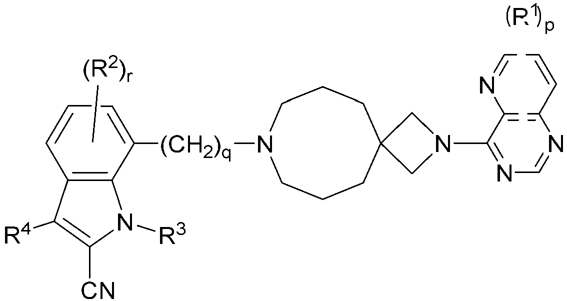 |
| I-D-IV-h | 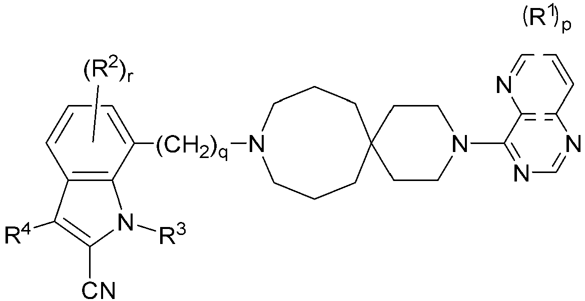 |
| I-D-IV-i | 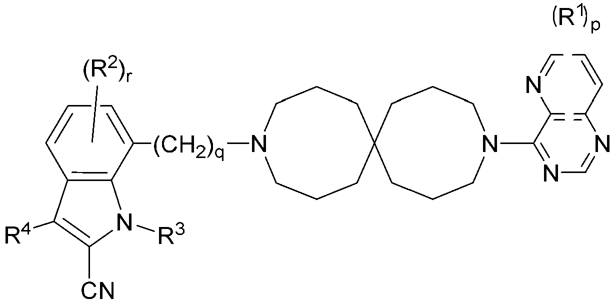 |
| I-D-V-a | 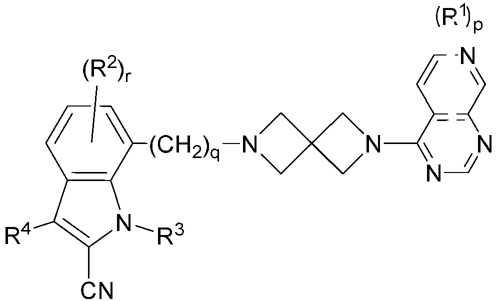 |
| Compound # | Structure |
| I-D-V-b | 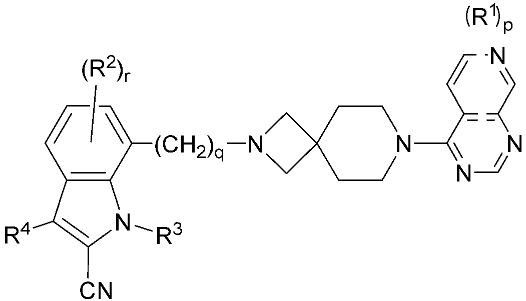 |
| I-D-V-c | 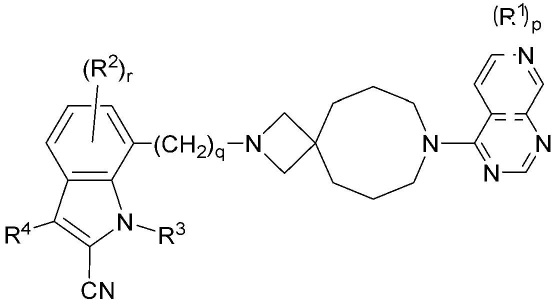 |
| I-D-V-d | 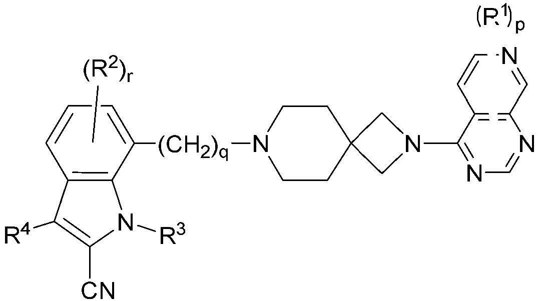 |
| I-D-V-e | 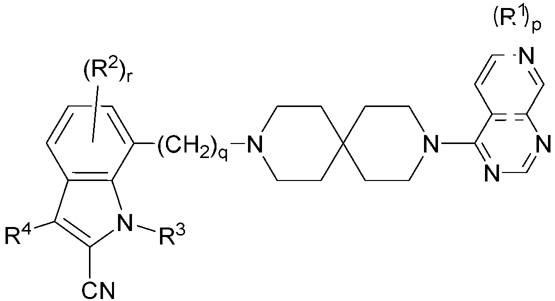 |
| I-D-V-f | 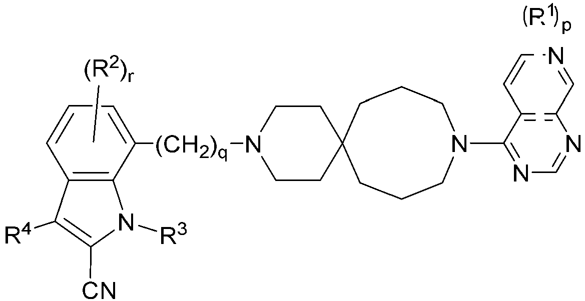 |
| I-D-V-g | 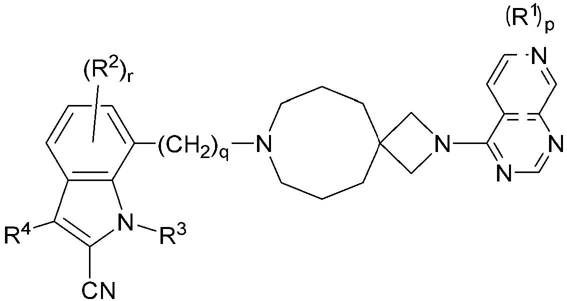 |
| I-D-V-h | 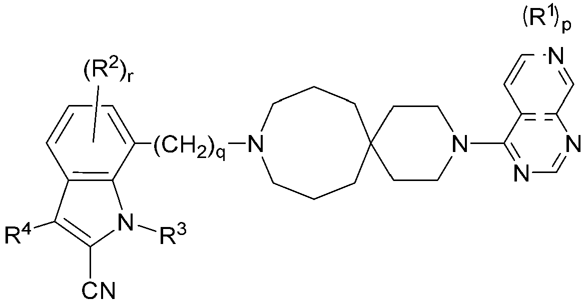 |
| I-D-V-i | 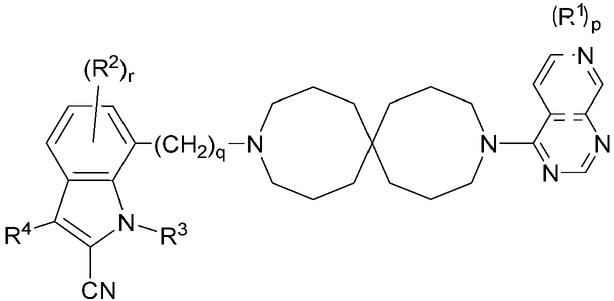 |
| I-D-VI-a | 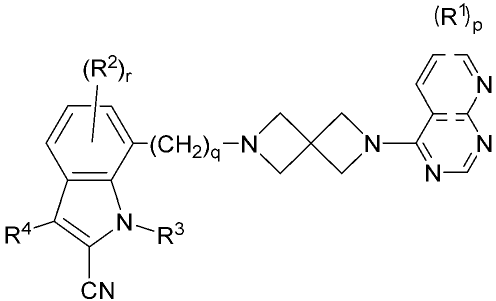 |
| I-D-VI-b | 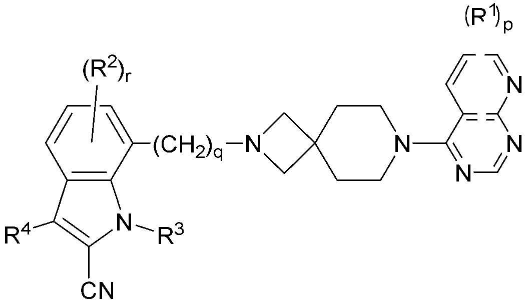 |
| I-D-VI-c | 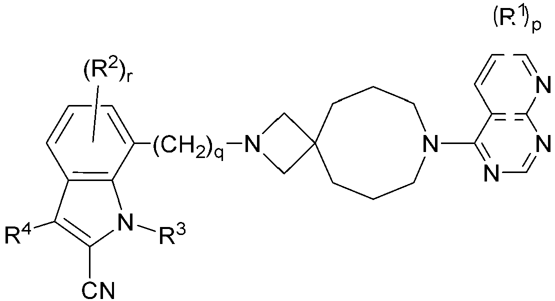 |
| I-D-VI-d | 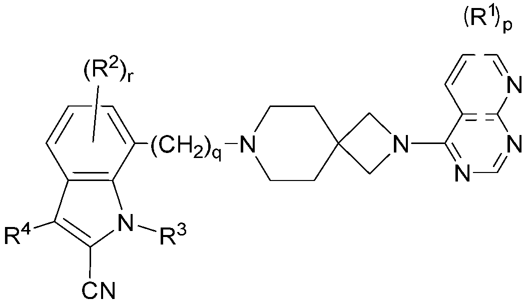 |
| I-D-VI-e | 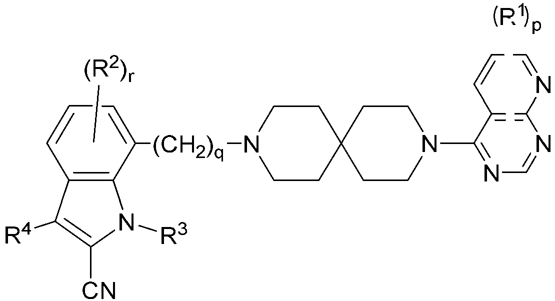 |
| I-D-VI-f | 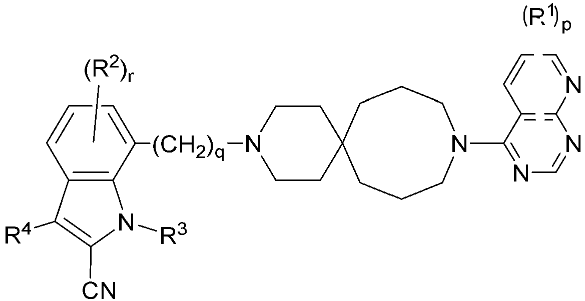 |
| I-D-VI-g | 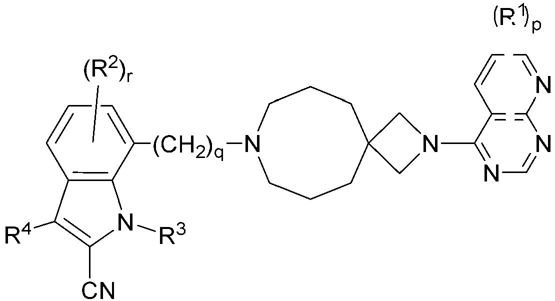 |
| I-D-VI-h | 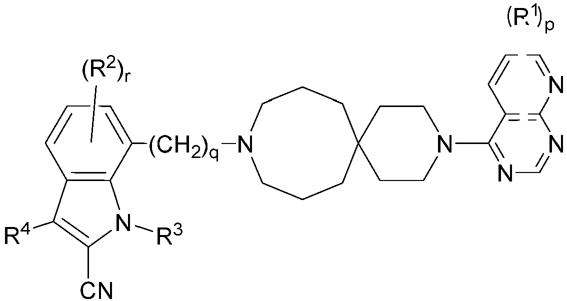 |
| I-D-VI-i | 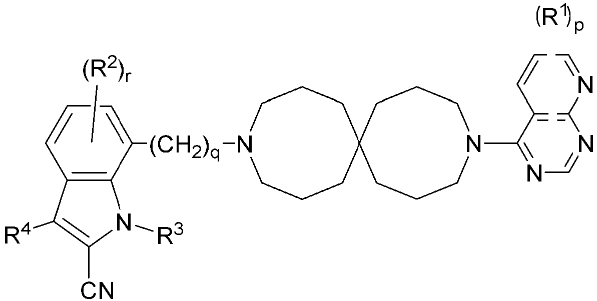 |
| I-D-VII-a | 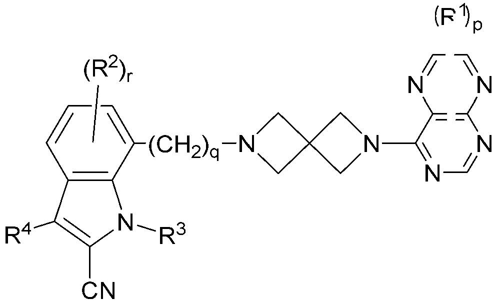 |
| I-D-VII-b | 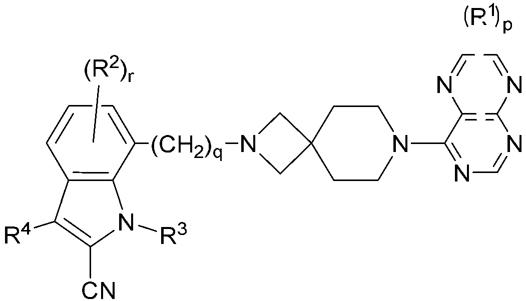 |
| I-D-VII-c | 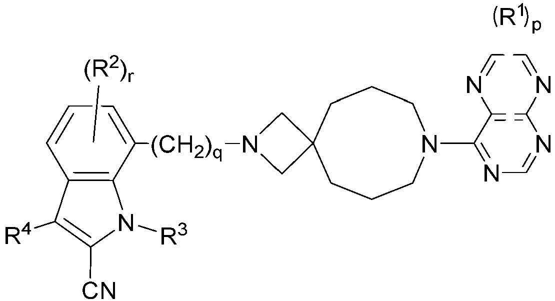 |
| I-D-VII-d | 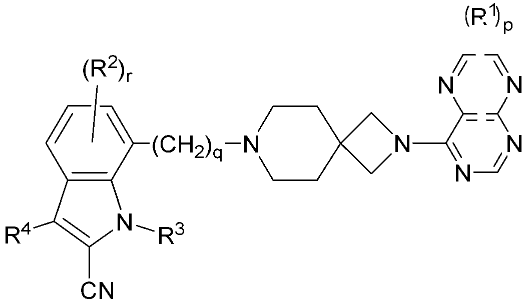 |
| I-D-VII-e | 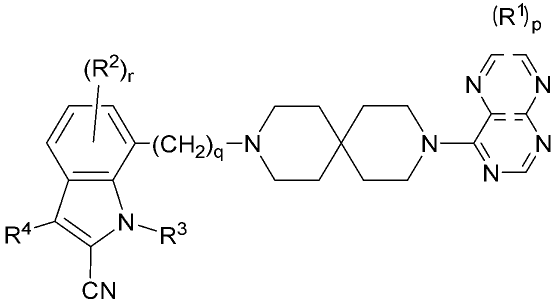 |
| I-D-VII-f | 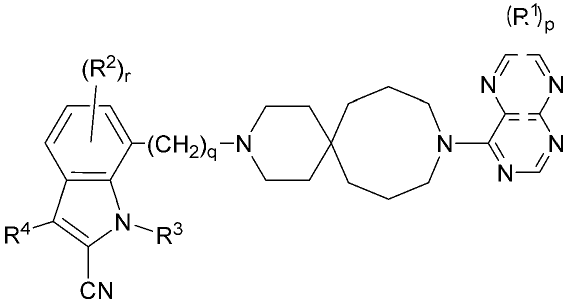 |
| I-D-VII-g | 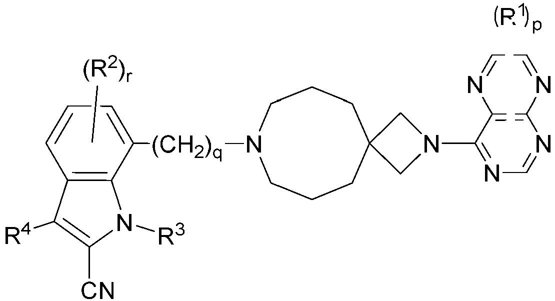 |
| I-D-VII-h | 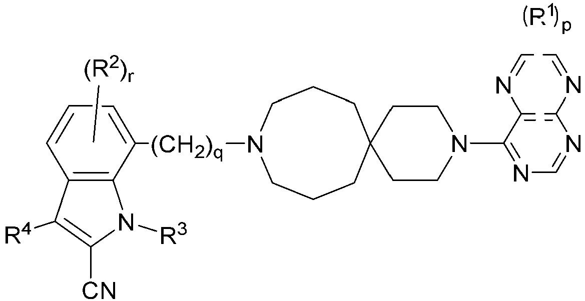 |
| I-D-VII-i | 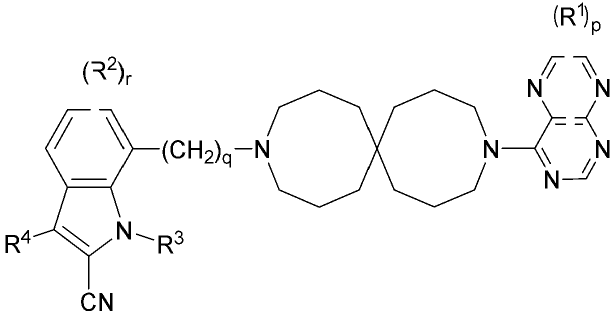 |

In some embodiments the compound is of Formula (I-A-1-d-1): 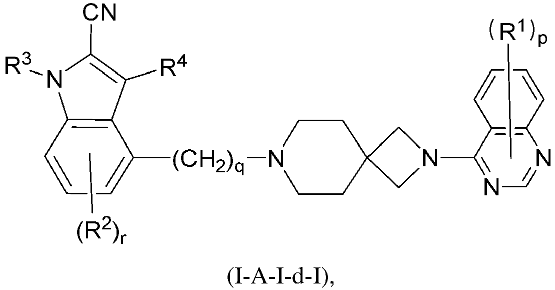 or a pharmaceutically acceptable salt, stereoisomer, solvate, prodrug, isotopic derivative, or tautomer thereof.

In some embodiments the compound is of Formula (I-A-1-d-1-1): 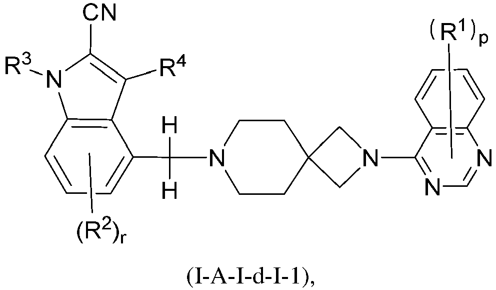 or a pharmaceutically acceptable salt, stereoisomer, solvate, prodrug, isotopic derivative, or tautomer thereof.

In some embodiments the compound is of Formula (I-A-I-d-I-1-A): 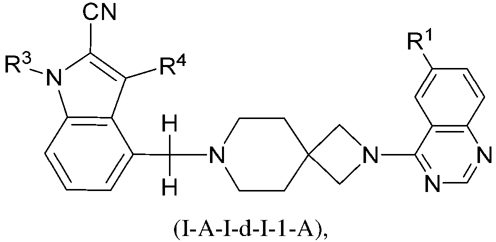 or a pharmaceutically acceptable salt, stereoisomer, solvate, prodrug, isotopic derivative, or tautomer thereof.

In some embodiments the compound is of Formula (I-A-I-d-I-1-A-H): 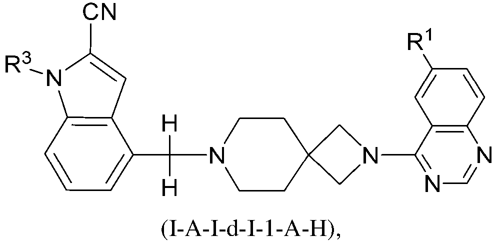 or a pharmaceutically acceptable salt, stereoisomer, solvate, prodrug, isotopic derivative, or tautomer thereof.

In some embodiments the compound is of Formula (I-A-I-d-I-1-A-H-1): 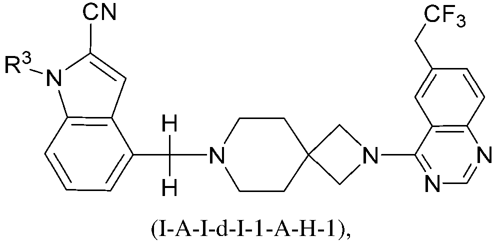 or a pharmaceutically acceptable salt, stereoisomer, solvate, prodrug, isotopic derivative, or tautomer thereof.

In some embodiments the compound is of Formula (I-B-I-d-I): 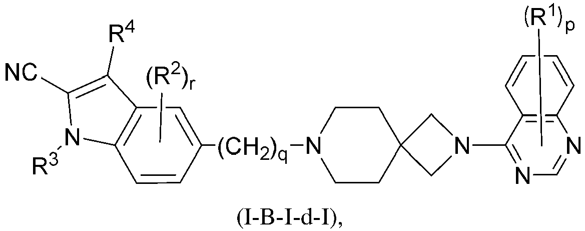 or a pharmaceutically acceptable salt, stereoisomer, solvate, prodrug, isotopic derivative, or tautomer thereof.

In some embodiments the compound is of Formula (I-B-I-d-I-1): 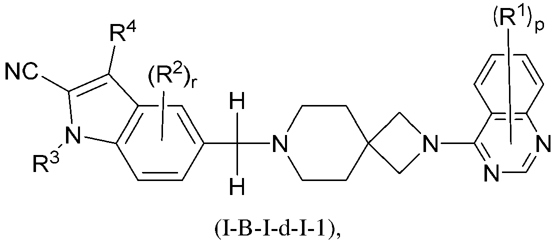 or a pharmaceutically acceptable salt, stereoisomer, solvate, prodrug, isotopic derivative, or tautomer thereof.

In some embodiments the compound is of Formula (I-B-I-d-I-1-A): 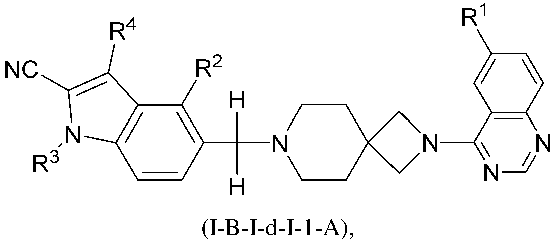 or a pharmaceutically acceptable salt, stereoisomer, solvate, prodrug, isotopic derivative, or tautomer thereof.

In some embodiments the compound is of Formula (I-B-I-d-I-1-A-H): 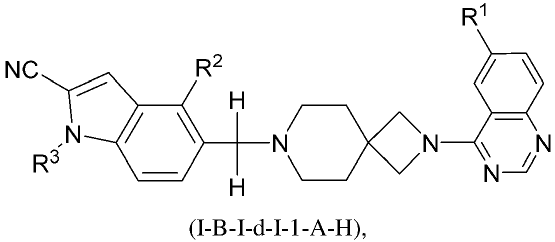 or a pharmaceutically acceptable salt, stereoisomer, solvate, prodrug, isotopic derivative, or tautomer thereof.

In some embodiments the compound is of Formula (I-B-I-d-I-1-A-H-1): 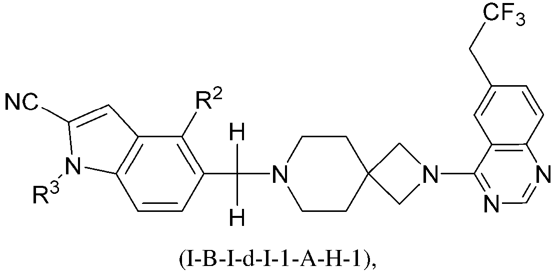 or a pharmaceutically acceptable salt, stereoisomer, solvate, prodrug, isotopic derivative, or tautomer thereof.

In some embodiments the compound is of Formula (I-B-I-d-I-1-A-H-1-h): 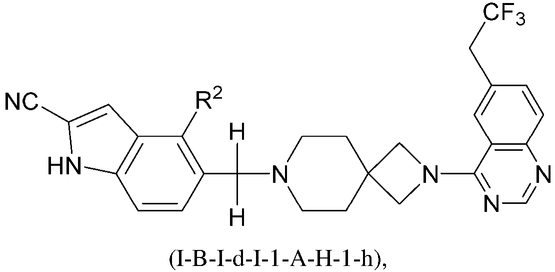 or a pharmaceutically acceptable salt, stereoisomer, solvate, prodrug, isotopic derivative, or tautomer thereof.

In some embodiments the compound is of Formula (I-B-I-d-I-1-A-H-1-B): 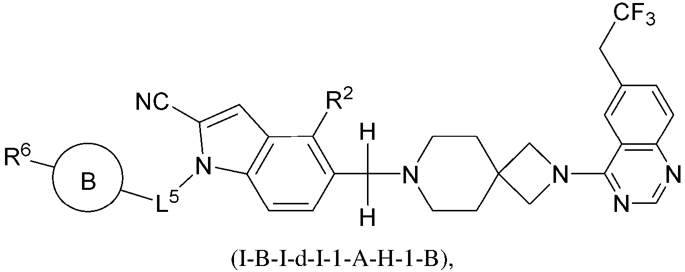 or a pharmaceutically acceptable salt, stereoisomer, solvate, prodrug, isotopic derivative, or tautomer thereof.

In some embodiments the compound is of Formula (I-B-I-d-I-1-A-H-1-B-G): 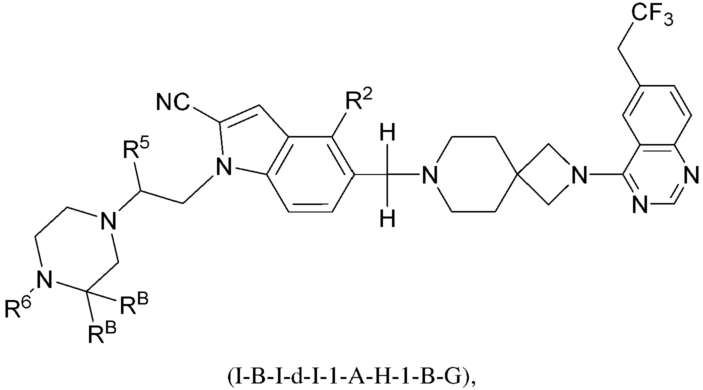 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo and all other variables are as defined herein.

In some embodiments the compound is of Formula (I-B-I-d-I-1-A-H-1-B-I): 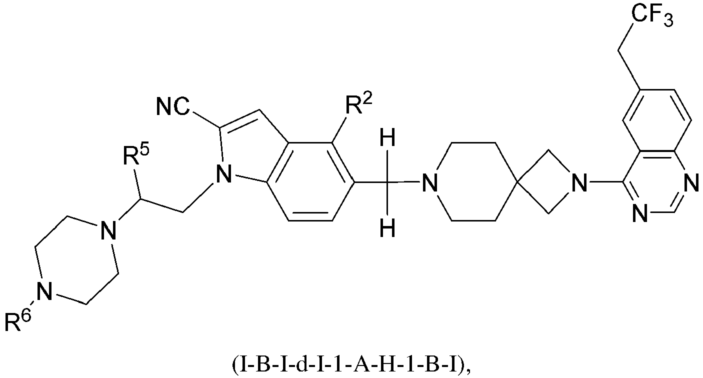 or a pharmaceutically acceptable salt, stereoisomer, solvate, prodrug, isotopic derivative, or tautomer thereof.

In some embodiments the compound is of Formula (I-B-I-d-I-1-A-H-1-B-I-a): 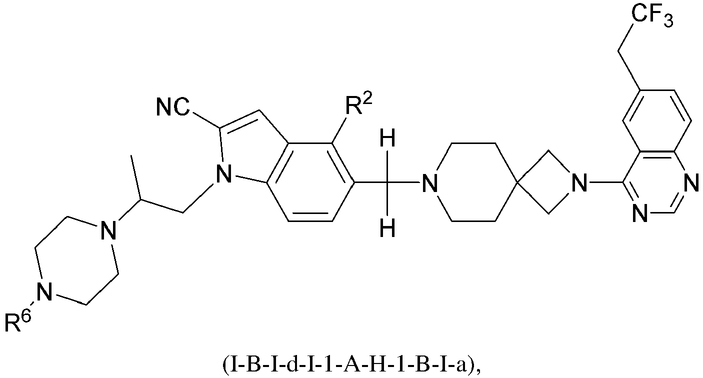 or a pharmaceutically acceptable salt, stereoisomer, solvate, prodrug, isotopic derivative, or tautomer thereof.

In some embodiments the compound is of Formula (I-B-I-d-I-1-A-H-1-B-I-a*): 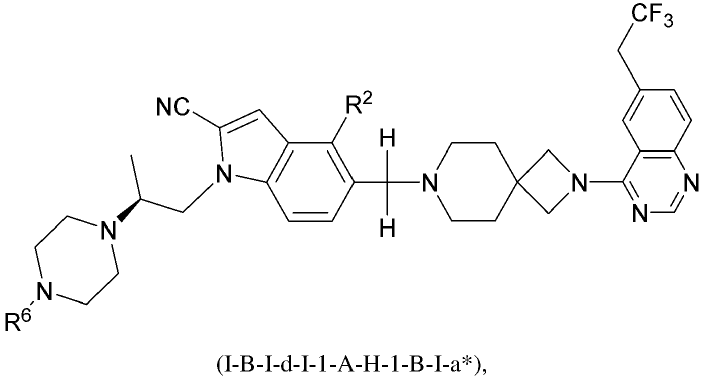 or a pharmaceutically acceptable salt, stereoisomer, solvate, prodrug, isotopic derivative, or tautomer thereof.

In some embodiments the compound is of Formula (I-B-I-d-I-1-A-H-1-B-I-a**): 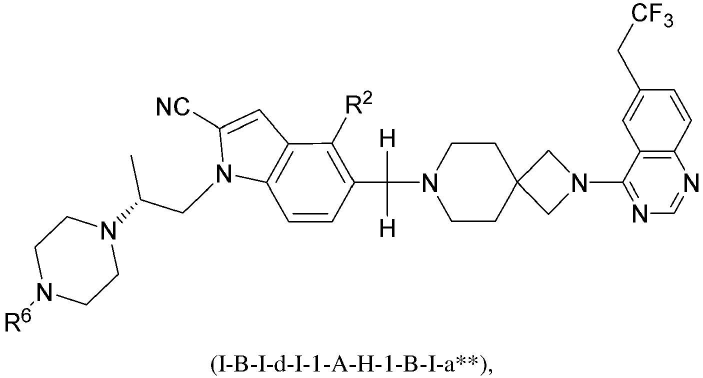 or a pharmaceutically acceptable salt, stereoisomer, solvate, prodrug, isotopic derivative, or tautomer thereof.

In some embodiments the compound is of Formula (I-B-I-d-I-1-A-H-1-B-II): 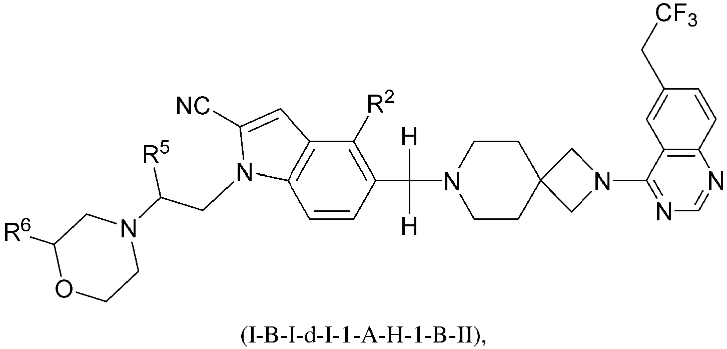 or a pharmaceutically acceptable salt, stereoisomer, solvate, prodrug, isotopic derivative, or tautomer thereof.

In some embodiments the compound is of Formula (I-B-I-d-I-1-A-H-1-B-II-a): 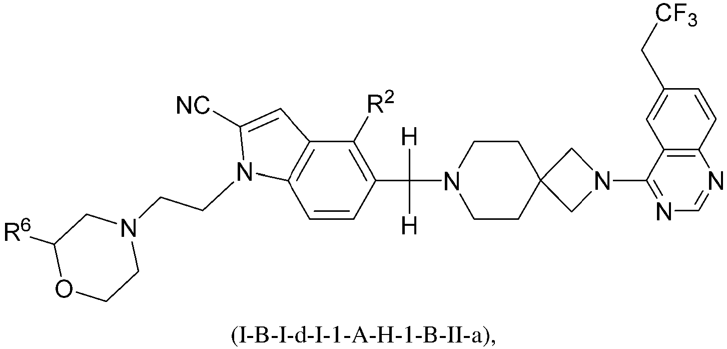 or a pharmaceutically acceptable salt, stereoisomer, solvate, prodrug, isotopic derivative, or tautomer thereof.

In some embodiments the compound is of Formula (I-B-I-d-I-1-A-H-1-B-III): 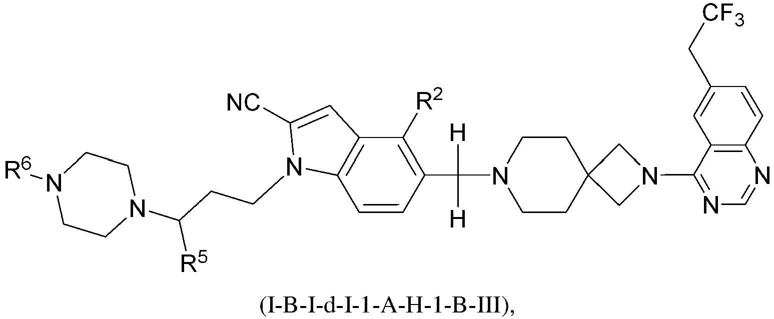 or a pharmaceutically acceptable salt, stereoisomer, solvate, prodrug, isotopic derivative, or tautomer thereof.

In some embodiments the compound is of Formula (I-B-I-d-I-1-A-H-1-B-III-a): 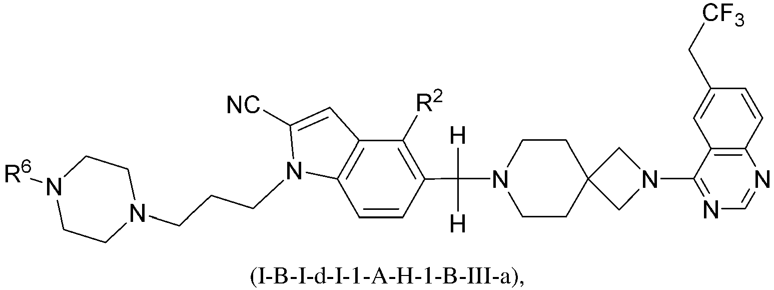 or a pharmaceutically acceptable salt, stereoisomer, solvate, prodrug, isotopic derivative, or tautomer thereof.

In some embodiments the compound is of Formula (I-B-I-d-I-1-A-H-1-B-IV): 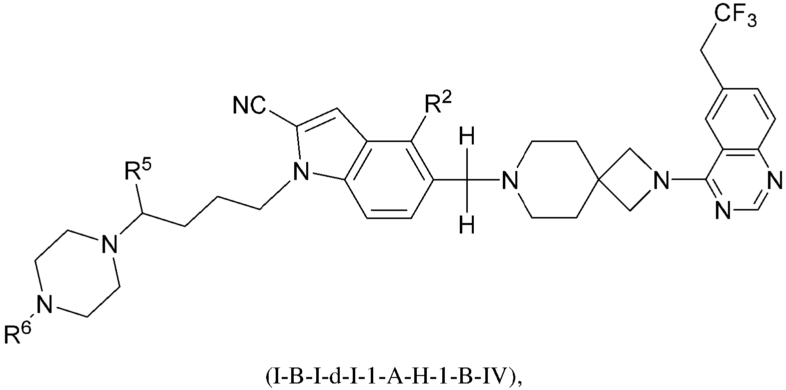 or a pharmaceutically acceptable salt, stereoisomer, solvate, prodrug, isotopic derivative, or tautomer thereof.

In some embodiments the compound is of Formula (I-B-I-d-I-1-A-H-1-B-IV-a): 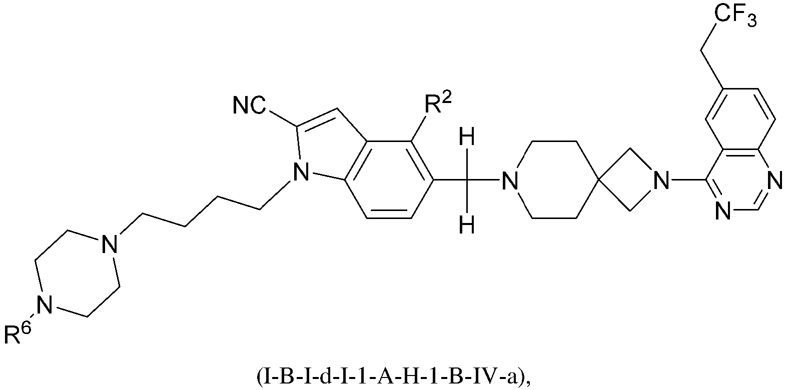 or a pharmaceutically acceptable salt, stereoisomer, solvate, prodrug, isotopic derivative, or tautomer thereof.

In some embodiments the compound is of Formula (I-B-I-d-I-1-A-H-1-B-V): 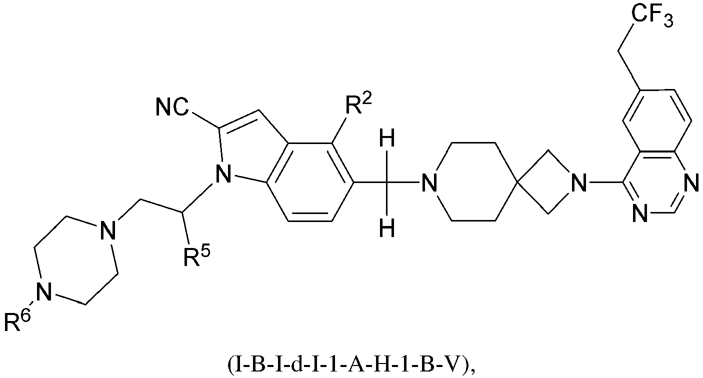 or a pharmaceutically acceptable salt, stereoisomer, solvate, prodrug, isotopic derivative, or tautomer thereof.

In some embodiments the compound is of Formula (I-B-I-d-I-1-A-H-1-B-V-a): 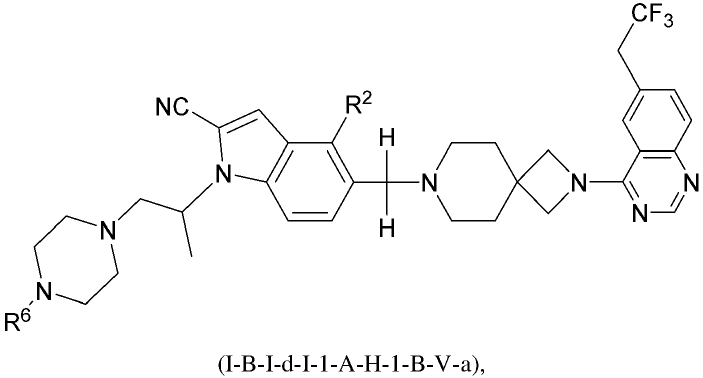 or a pharmaceutically acceptable salt, stereoisomer, solvate, prodrug, isotopic derivative, or tautomer thereof.

In some embodiments the compound is of Formula (I-B-I-d-I-1-A-H-1-B-V-a*): 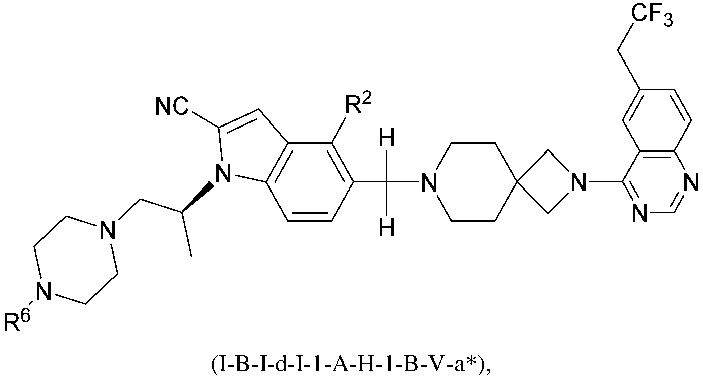 or a pharmaceutically acceptable salt, stereoisomer, solvate, prodrug, isotopic derivative, or tautomer thereof.

In some embodiments the compound is of Formula (I-B-I-d-I-1-A-H-1-B-V-a**): 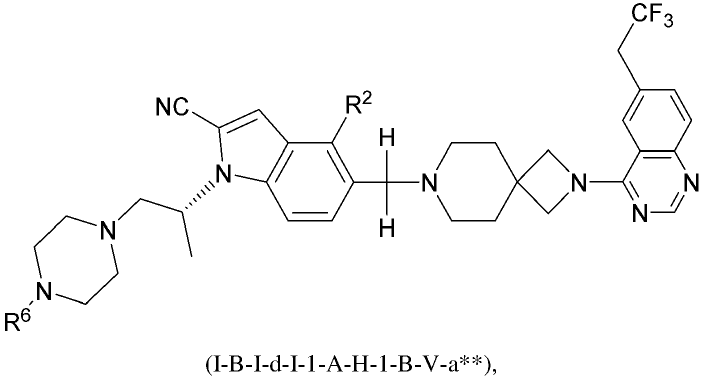 or a pharmaceutically acceptable salt, stereoisomer, solvate, prodrug, isotopic derivative, or tautomer thereof.

In some embodiments the compound is of Formula (I-B-I-d-I-1-A-H-1-B-VI): 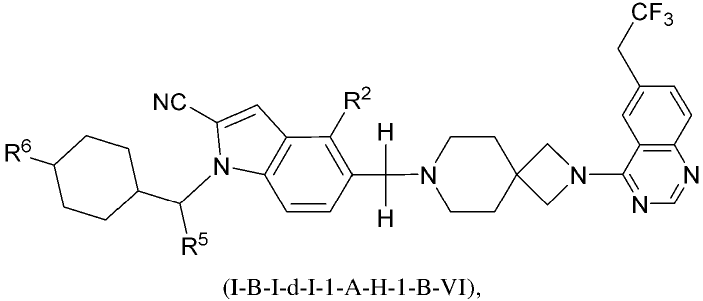 or a pharmaceutically acceptable salt, stereoisomer, solvate, prodrug, isotopic derivative, or tautomer thereof.

In some embodiments the compound is of Formula (I-B-I-d-I-1-A-H-1-B-VI-a): 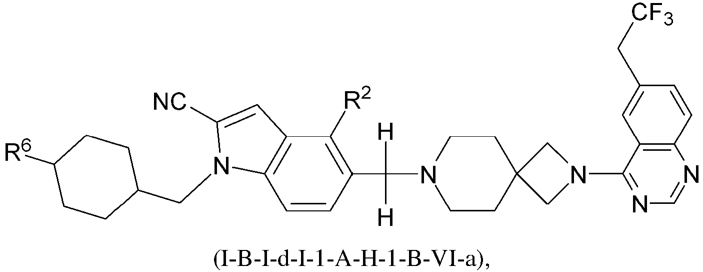 or a pharmaceutically acceptable salt, stereoisomer, solvate, prodrug, isotopic derivative, or tautomer thereof.

In some embodiments the compound is of Formula (I-B-I-d-I-1-A-H-1-B-VI-a*): 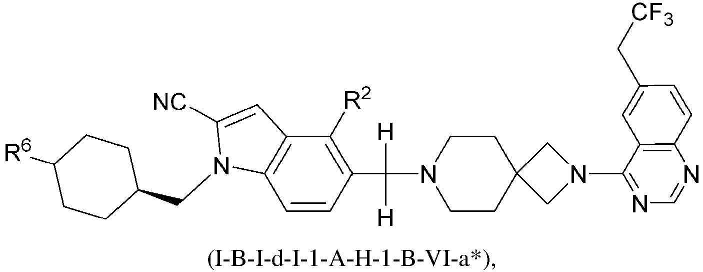 or a pharmaceutically acceptable salt, stereoisomer, solvate, prodrug, isotopic derivative, or tautomer thereof.

In some embodiments the compound is of Formula (I-B-I-d-I-1-A-H-1-B-VI-a**): 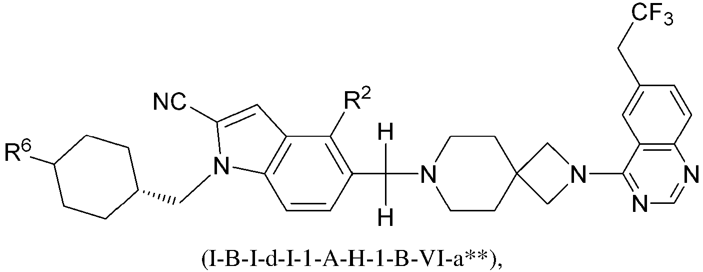 or a pharmaceutically acceptable salt, stereoisomer, solvate, prodrug, isotopic derivative, or tautomer thereof.

In some embodiments the compound is of Formula (I-B-I-d-I-1-A-H-1-B-VII): 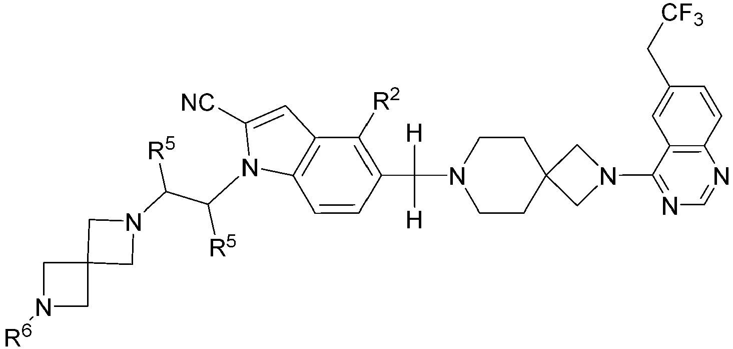 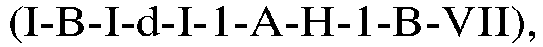 or a pharmaceutically acceptable salt, stereoisomer, solvate, prodrug, isotopic derivative, or tautomer thereof.

In some embodiments the compound is of Formula (I-B-I-d-I-1-A-H-1-B-VII-a): 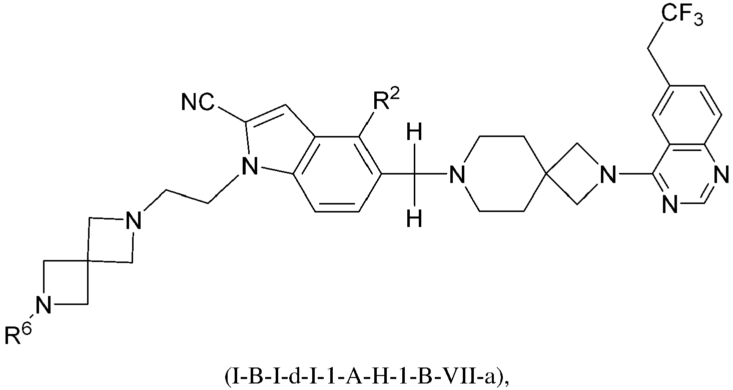 or a pharmaceutically acceptable salt, stereoisomer, solvate, prodrug, isotopic derivative, or tautomer thereof.

In some embodiments the compound is of Formula (I-B-I-d-I-1-A-H-1-B-VIII): 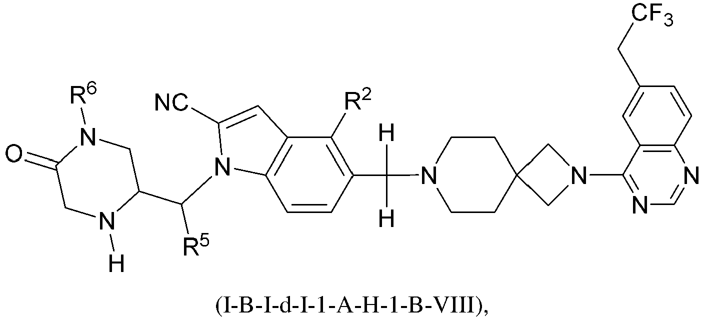 or a pharmaceutically acceptable salt, stereoisomer, solvate, prodrug, isotopic derivative, or tautomer thereof.

In some embodiments the compound is of Formula (I-B-I-d-I-1-A-H-1-B-VIII-a): 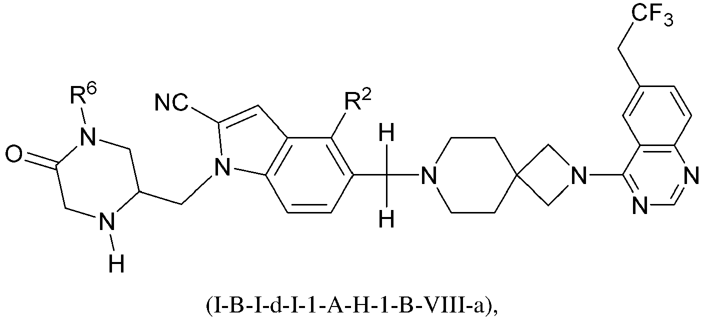 or a pharmaceutically acceptable salt, stereoisomer, solvate, prodrug, isotopic derivative, or tautomer thereof.

In some embodiments the compound is of Formula (I-B-I-d-I-1-A-H-1-B-VIII-a*): 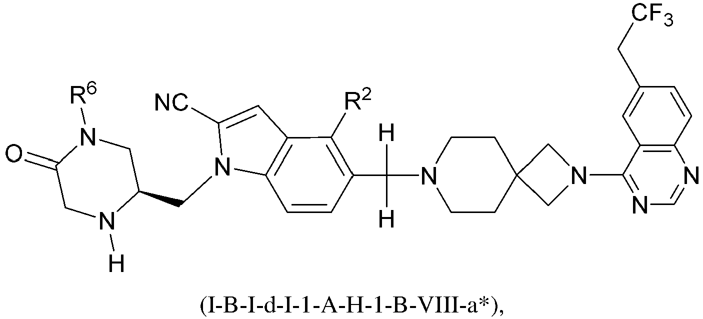 or a pharmaceutically acceptable salt, stereoisomer, solvate, prodrug, isotopic derivative, or tautomer thereof.

In some embodiments the compound is of Formula (I-B-I-d-I-1-A-H-1-B-VIII-a**): 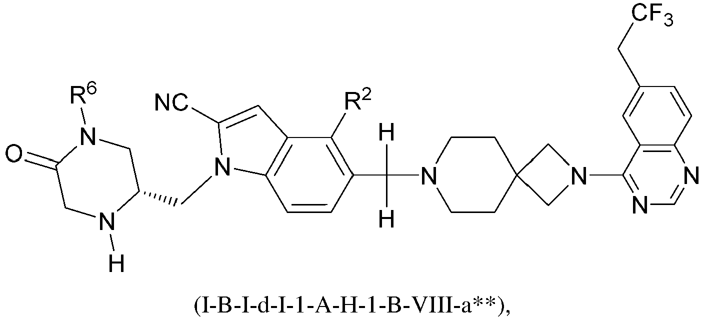 or a pharmaceutically acceptable salt, stereoisomer, solvate, prodrug, isotopic derivative, or tautomer thereof.

In some embodiments the compound is of Formula (I-B-I-d-I-1-A-H-1-B-IX): 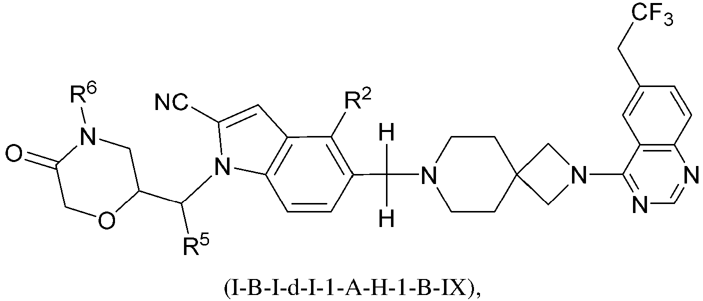 or a pharmaceutically acceptable salt, stereoisomer, solvate, prodrug, isotopic derivative, or tautomer thereof.

In some embodiments the compound is of Formula (I-B-I-d-I-1-A-H-1-B-IX-a): 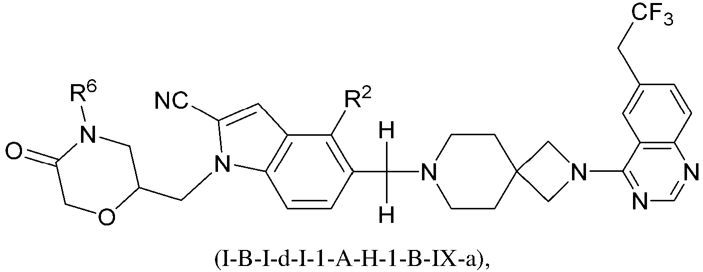 or a pharmaceutically acceptable salt, stereoisomer, solvate, prodrug, isotopic derivative, or tautomer thereof.

In some embodiments the compound is of Formula (I-B-I-d-I-1-A-H-1-B-IX-a*): 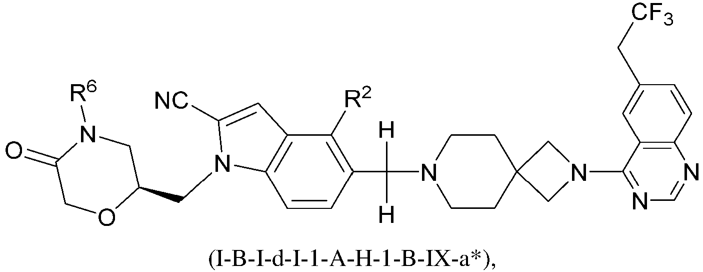 or a pharmaceutically acceptable salt, stereoisomer, solvate, prodrug, isotopic derivative, or tautomer thereof.

In some embodiments the compound is of Formula (I-B-I-d-I-1-A-H-1-B-IX-a**): 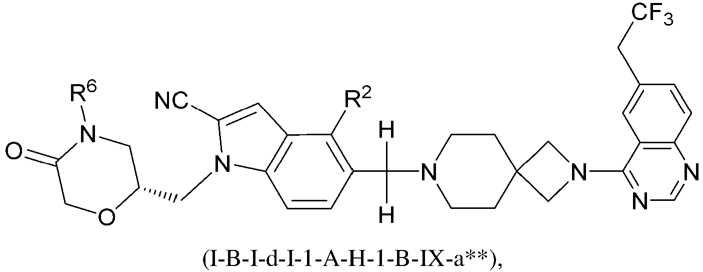 or a pharmaceutically acceptable salt, stereoisomer, solvate, prodrug, isotopic derivative, or tautomer thereof.

In some embodiments the compound is of Formula (I-B-I-d-I-1-A-H-1-B-X): 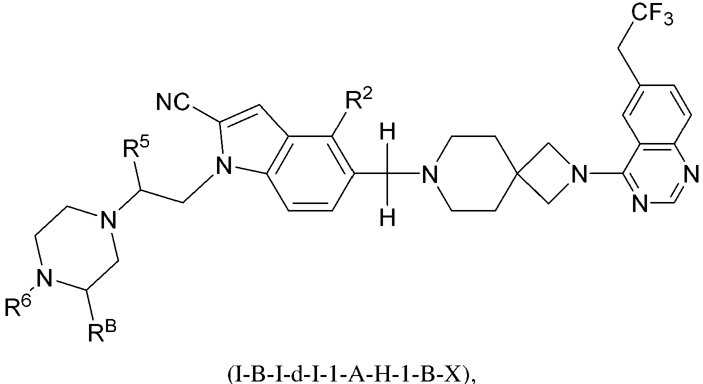 or a pharmaceutically acceptable salt, stereoisomer, solvate, prodrug, isotopic derivative, or tautomer thereof wherein R^{B} is C₁-C₆ alkyl, and all other variables as defined herein.

In some embodiments the compound is of Formula (I-B-I-d-I-1-A-H-1-B-X-a): 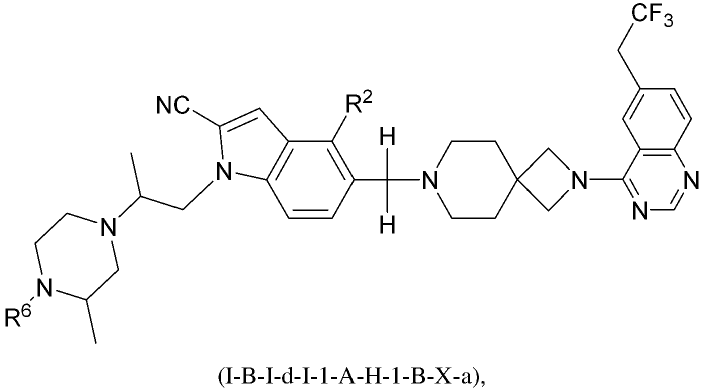 or a pharmaceutically acceptable salt, stereoisomer, solvate, prodrug, isotopic derivative, or tautomer thereof.

In some embodiments the compound is of Formula (I-B-I-d-I-1-A-H-1-B-X-a*): 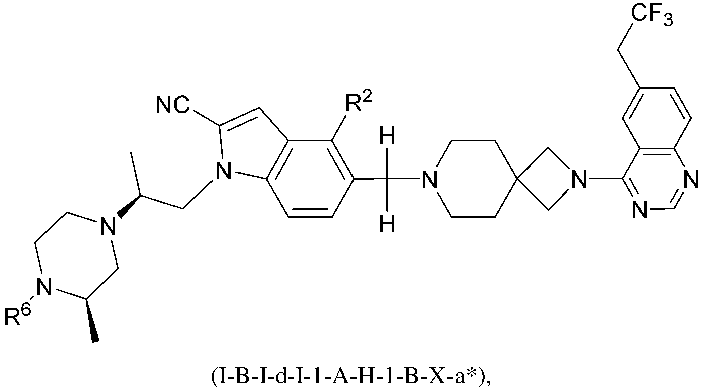 or a pharmaceutically acceptable salt, stereoisomer, solvate, prodrug, isotopic derivative, or tautomer thereof.

In some embodiments the compound is of Formula (I-B-I-d-I-1-A-H-1-B-X-a**): 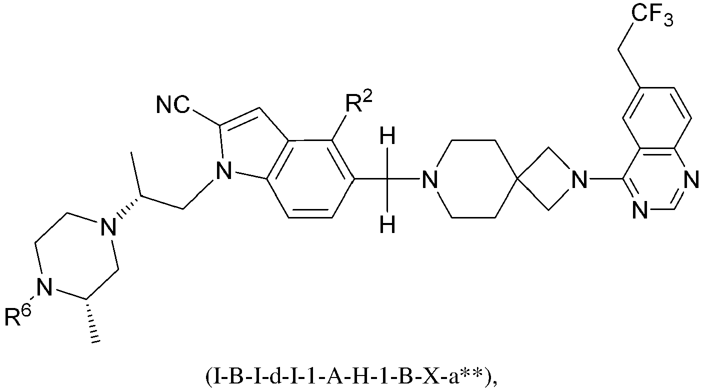 or a pharmaceutically acceptable salt, stereoisomer, solvate, prodrug, isotopic derivative, or tautomer thereof.

In some embodiments the compound is of Formula (I-B-I-d-I-1-A-H-1-B-X-a***): 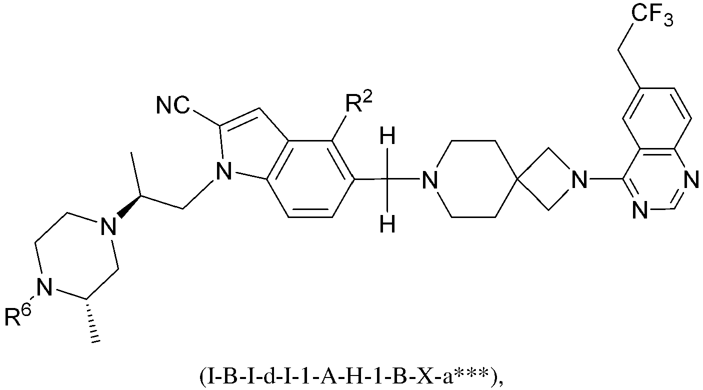 or a pharmaceutically acceptable salt, stereoisomer, solvate, prodrug, isotopic derivative, or tautomer thereof.

In some embodiments the compound is of Formula (I-B-I-d-I-1-A-H-1-B-X-a****): 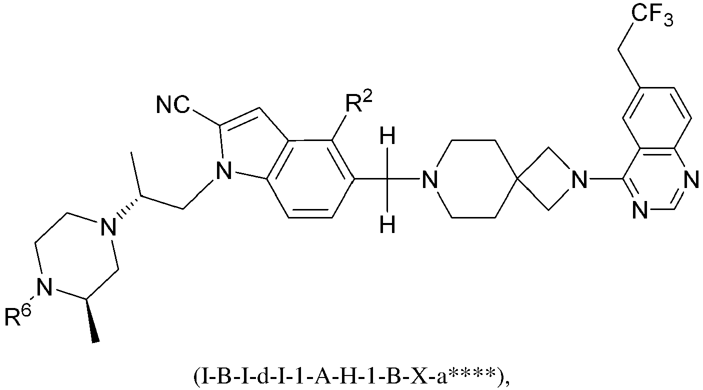 or a pharmaceutically acceptable salt, stereoisomer, solvate, prodrug, isotopic derivative, or tautomer thereof.

In some embodiments the compound is of Formula (I-B-I-d-I-1-A-H-1-B-XI): 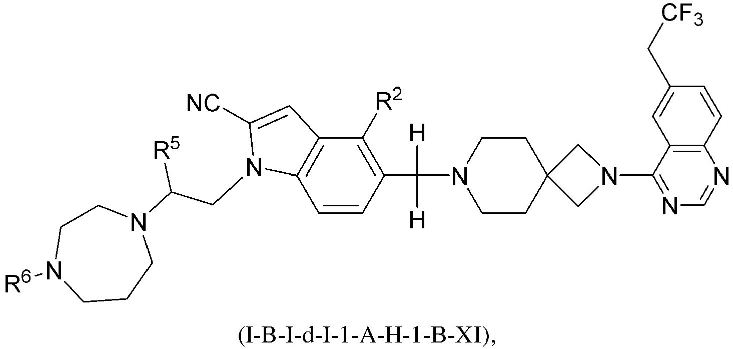 or a pharmaceutically acceptable salt, stereoisomer, solvate, prodrug, isotopic derivative, or tautomer thereof.

In some embodiments the compound is of Formula (I-B-I-d-I-1-A-H-1-B-XI-a): 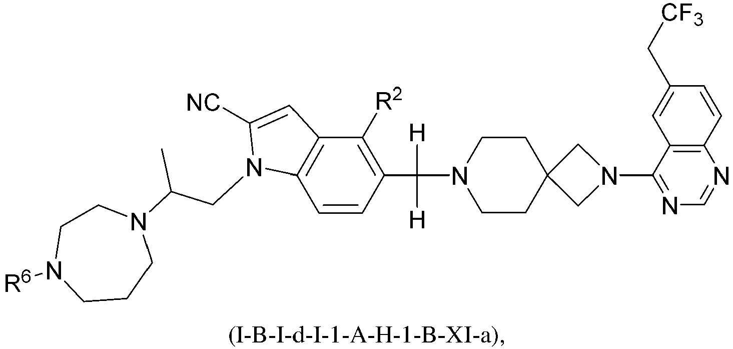 or a pharmaceutically acceptable salt, stereoisomer, solvate, prodrug, isotopic derivative, or tautomer thereof.

In some embodiments the compound is of Formula (I-B-I-d-I-1-A-H-1-B-XI-a*): 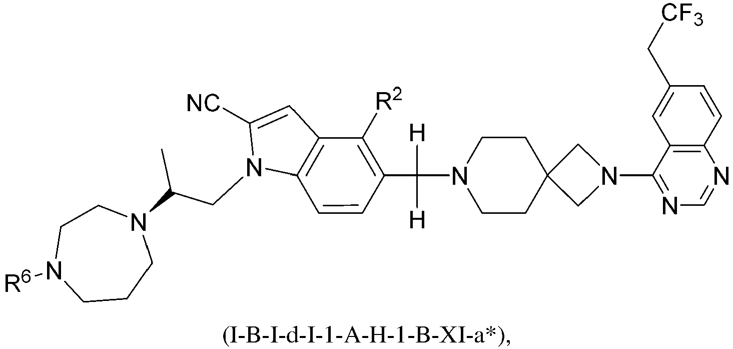 or a pharmaceutically acceptable salt, stereoisomer, solvate, prodrug, isotopic derivative, or tautomer thereof.

In some embodiments the compound is of Formula (I-B-I-d-I-1-A-H-1-B-XI-a**): 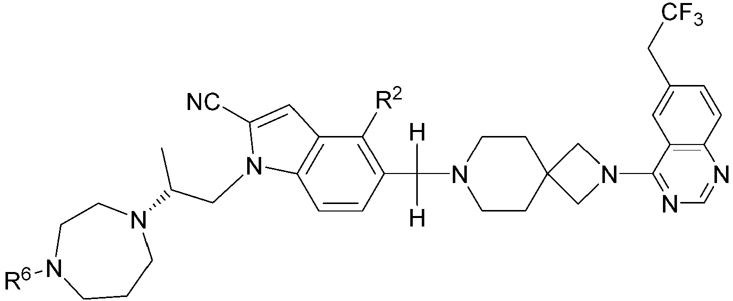 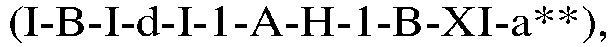 or a pharmaceutically acceptable salt, stereoisomer, solvate, prodrug, isotopic derivative, or tautomer thereof.

In some embodiments the compound is of Formula (I-B-I-d-I-1-A-H-1-B-IX): 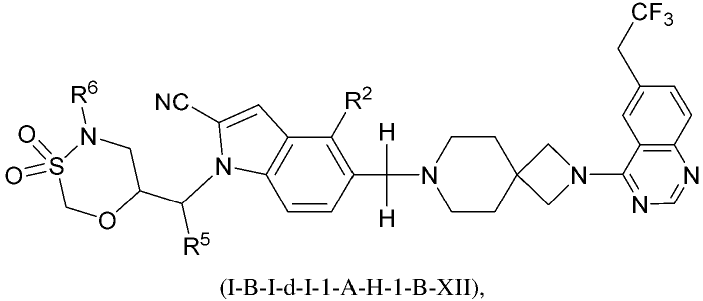 or a pharmaceutically acceptable salt, stereoisomer, solvate, prodrug, isotopic derivative, or tautomer thereof.

In some embodiments the compound is of Formula (I-B-I-d-I-1-A-H-1-B-XII-a): 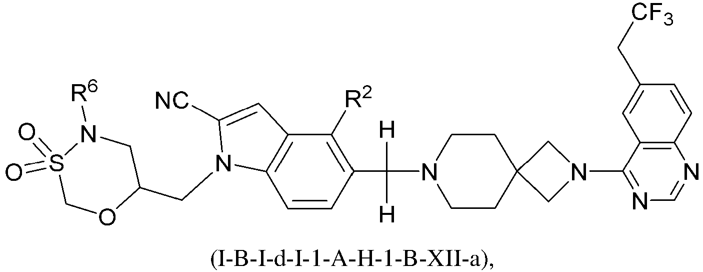 or a pharmaceutically acceptable salt, stereoisomer, solvate, prodrug, isotopic derivative, or tautomer thereof.

In some embodiments the compound is of Formula (I-B-I-d-I-1-A-H-1-B-IX-a*): 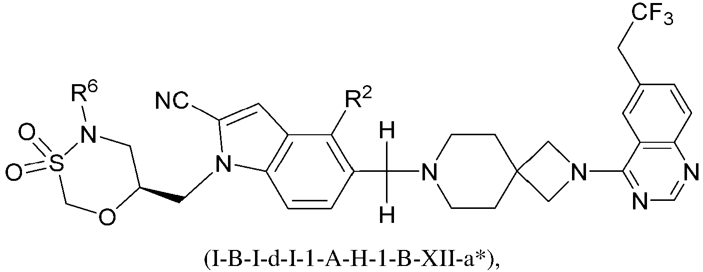 or a pharmaceutically acceptable salt, stereoisomer, solvate, prodrug, isotopic derivative, or tautomer thereof.

In some embodiments the compound is of Formula (I-B-I-d-I-1-A-H-1-B-XII-a**): 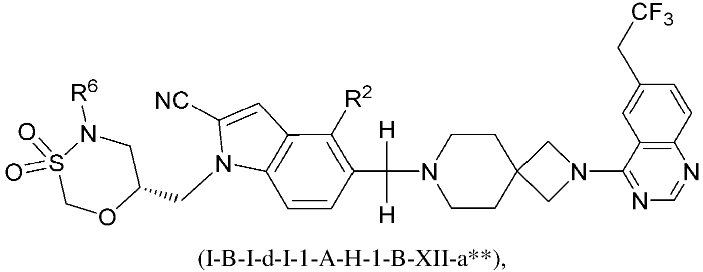 or a pharmaceutically acceptable salt, stereoisomer, solvate, prodrug, isotopic derivative, or tautomer thereof.

In some embodiments the compound is of Formula (I-B-I-d-I-1-A-H-1-B-XIII): 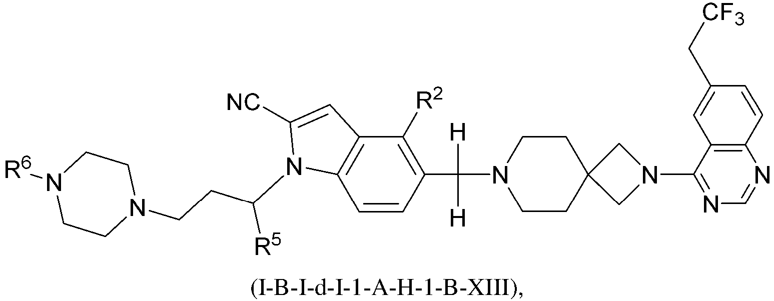 or a pharmaceutically acceptable salt, stereoisomer, solvate, prodrug, isotopic derivative, or tautomer thereof.

In some embodiments the compound is of Formula (I-B-I-d-I-1-A-H-1-B-XIII-a): 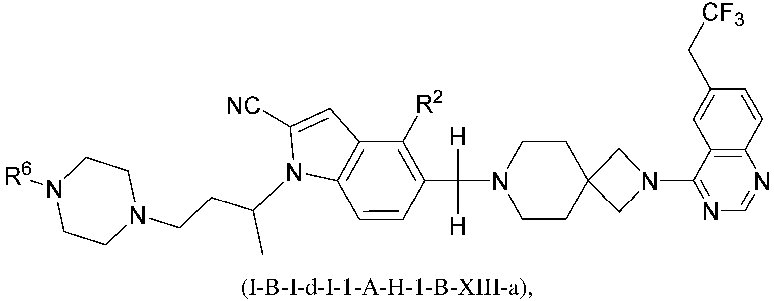 or a pharmaceutically acceptable salt, stereoisomer, solvate, prodrug, isotopic derivative, or tautomer thereof.

In some embodiments the compound is of Formula (I-B-I-d-I-1-A-H-1-B-XIV): 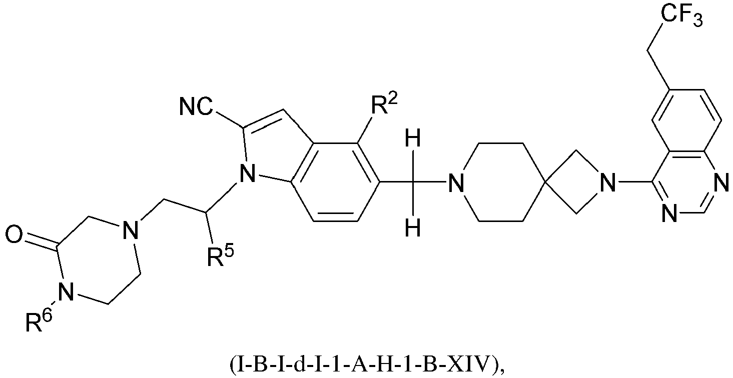 or a pharmaceutically acceptable salt, stereoisomer, solvate, prodrug, isotopic derivative, or tautomer thereof.

In some embodiments the compound is of Formula (I-B-I-d-I-1-A-H-1-B-XIV-a): 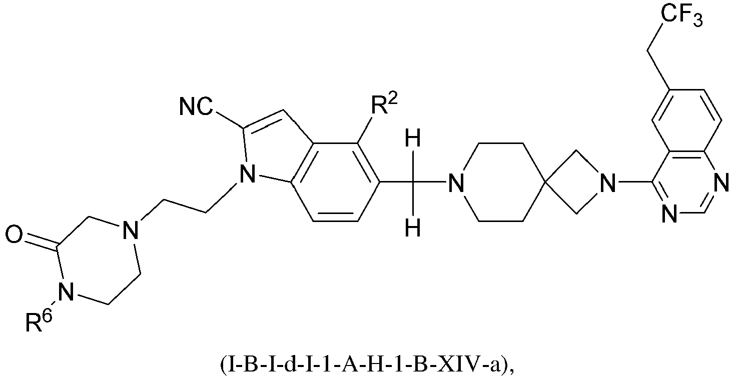 or a pharmaceutically acceptable salt, stereoisomer, solvate, prodrug, isotopic derivative, or tautomer thereof.

In some embodiments the compound is of Formula (I-B-I-d-I-1-A-H-1-B-XV): 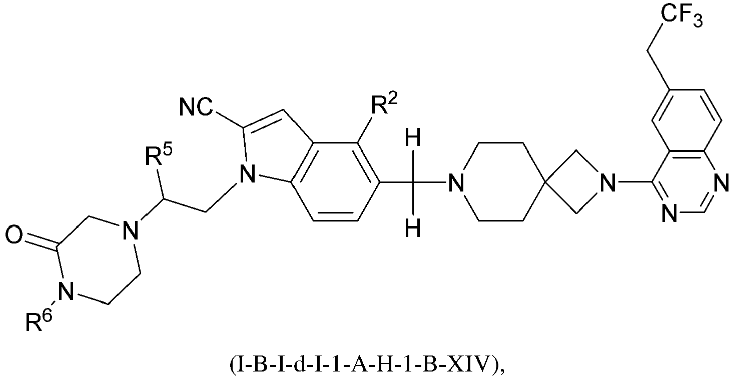 or a pharmaceutically acceptable salt, stereoisomer, solvate, prodrug, isotopic derivative, or tautomer thereof.

In some embodiments the compound is of Formula (I-B-I-d-I-1-A-H-1-B-XV-a): 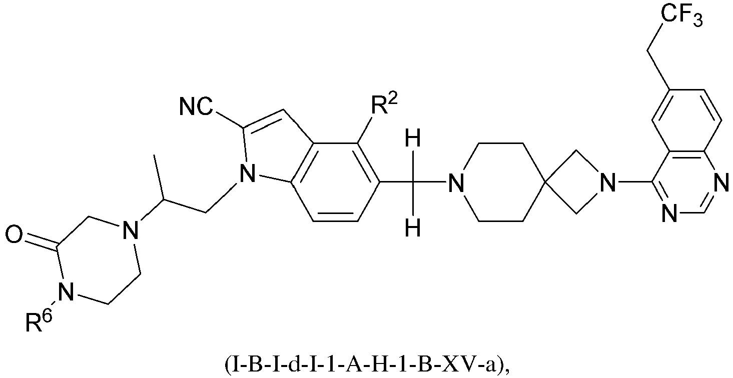 or a pharmaceutically acceptable salt, stereoisomer, solvate, prodrug, isotopic derivative, or tautomer thereof.

In some embodiments the compound is of Formula (I-B-I-d-I-1-A-H-1-B-XVI): 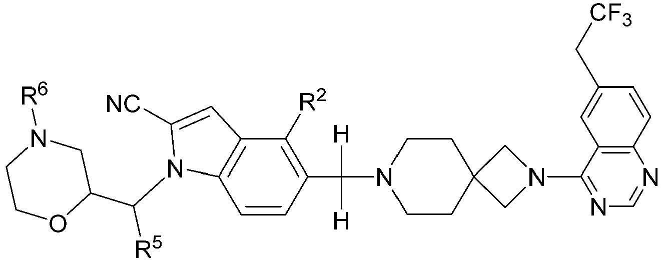 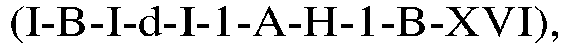 or a pharmaceutically acceptable salt, stereoisomer, solvate, prodrug, isotopic derivative, or tautomer thereof.

In some embodiments the compound is of Formula (I-B-I-d-I-1-A-H-1-B-XVI-a): 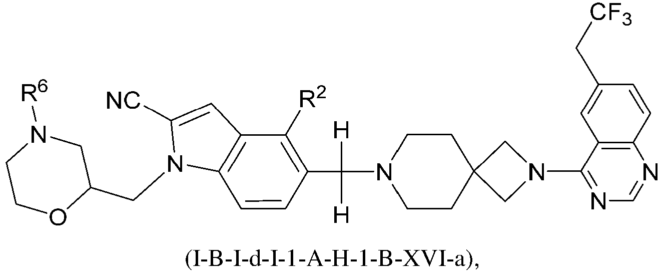 or a pharmaceutically acceptable salt, stereoisomer, solvate, prodrug, isotopic derivative, or tautomer thereof.

In some embodiments the compound is of Formula (I-B-I-d-I-1-A-H-1-B-XVI-a*): 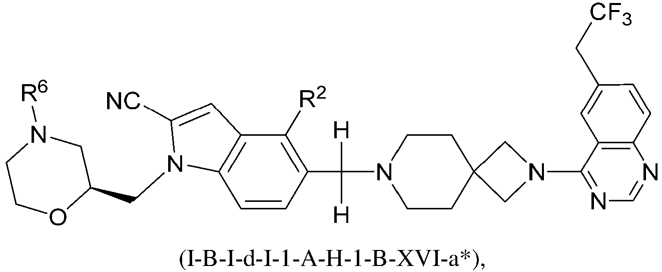 or a pharmaceutically acceptable salt, stereoisomer, solvate, prodrug, isotopic derivative, or tautomer thereof.

In some embodiments the compound is of Formula (I-B-I-d-I-1-A-H-1-B-XVI-a**): 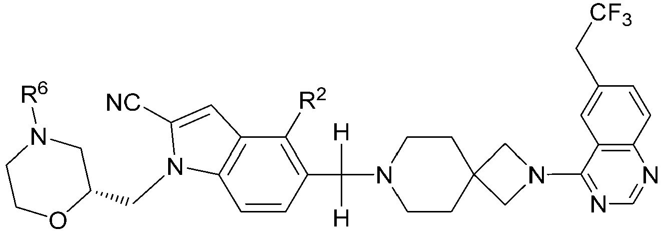 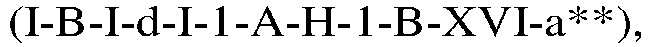 or a pharmaceutically acceptable salt, stereoisomer, solvate, prodrug, isotopic derivative, or tautomer thereof.

In some embodiments the compound is of Formula (I-B-I-d-I-1-A-H-1-B-XVII): 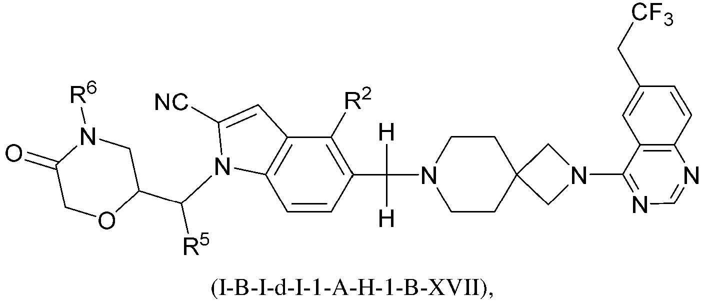 or a pharmaceutically acceptable salt, stereoisomer, solvate, prodrug, isotopic derivative, or tautomer thereof.

In some embodiments the compound is of Formula (I-B-I-d-I-1-A-H-1-B-XVII-a): 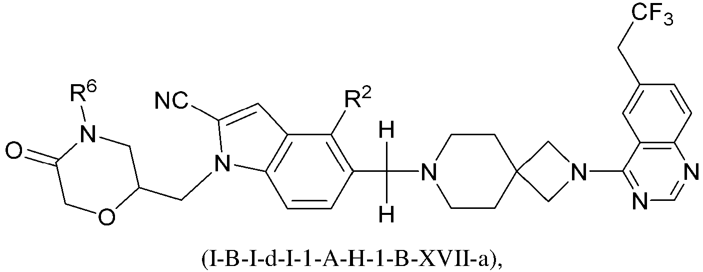 or a pharmaceutically acceptable salt, stereoisomer, solvate, prodrug, isotopic derivative, or tautomer thereof.

In some embodiments the compound is of Formula (I-B-I-d-I-1-A-H-1-B-XVII-a*): 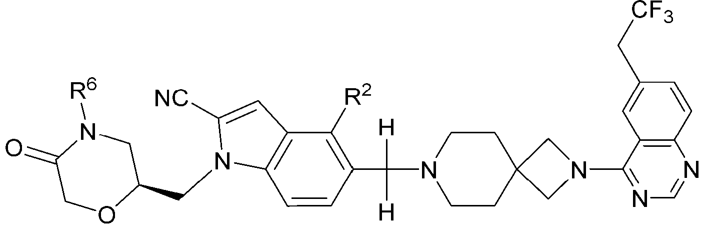 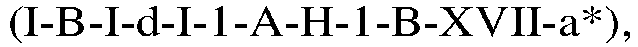 or a pharmaceutically acceptable salt, stereoisomer, solvate, prodrug, isotopic derivative, or tautomer thereof.

In some embodiments the compound is of Formula (I-B-I-d-I-1-A-H-1-B-XVII-a**): 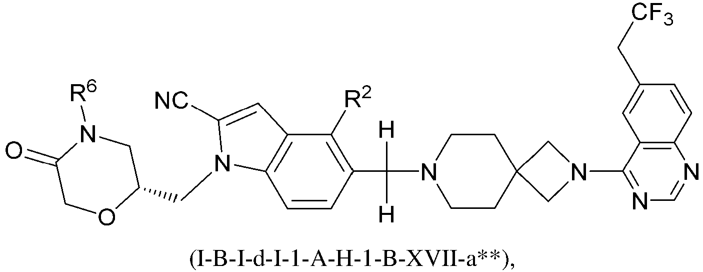 or a pharmaceutically acceptable salt, stereoisomer, solvate, prodrug, isotopic derivative, or tautomer thereof.

In some embodiments the compound is of Formula (I-B-I-d-I-1-A-H-1-B-GG): 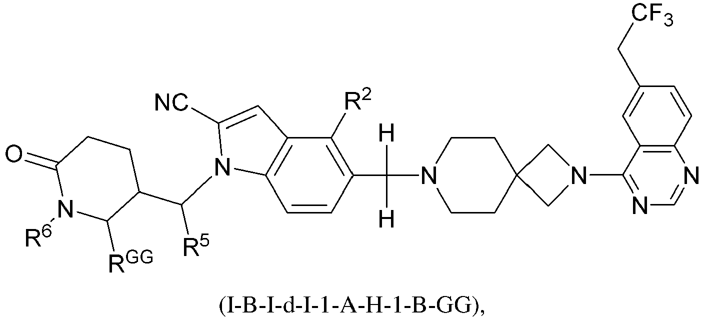 or a pharmaceutically acceptable salt, stereoisomer, solvate, prodrug, isotopic derivative, or tautomer thereof, wherein R^{GG} is selected from H, C₁-C₆ alkyl, aryl, heteroaryl and all other variables are as defined herein.

In some embodiments the compound is of Formula (I-B-I-d-I-1-A-H-1-B-XVIII): 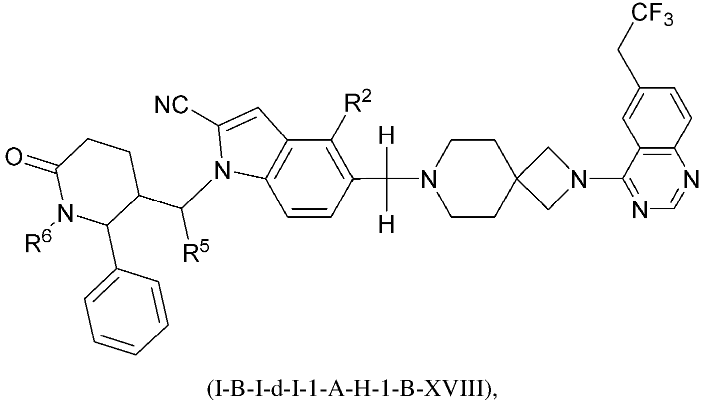 or a pharmaceutically acceptable salt, stereoisomer, solvate, prodrug, isotopic derivative, or tautomer thereof.

In some embodiments the compound is of Formula (I-B-I-d-I-1-A-H-1-B-XVIII-a): 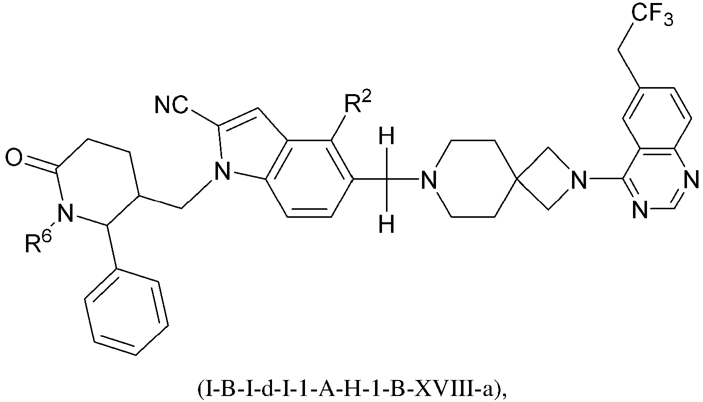 or a pharmaceutically acceptable salt, stereoisomer, solvate, prodrug, isotopic derivative, or tautomer thereof.

In some embodiments the compound is of Formula (I-B-I-d-I-1-A-H-1-B-XVIII-a*): 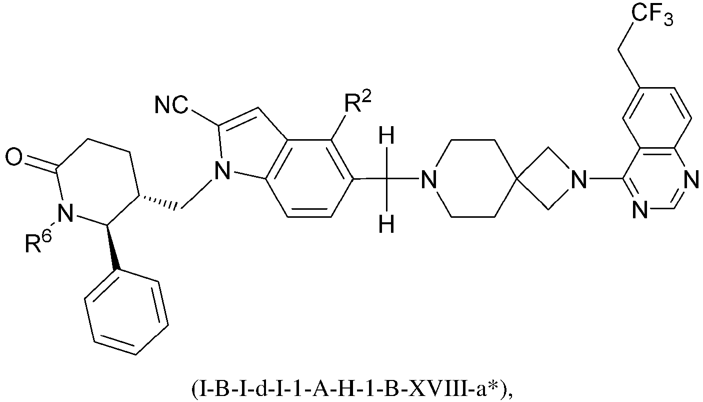 or a pharmaceutically acceptable salt, stereoisomer, solvate, prodrug, isotopic derivative, or tautomer thereof.

In some embodiments the compound is of Formula (I-B-I-d-I-1-A-H-1-B-XVIII-a**): 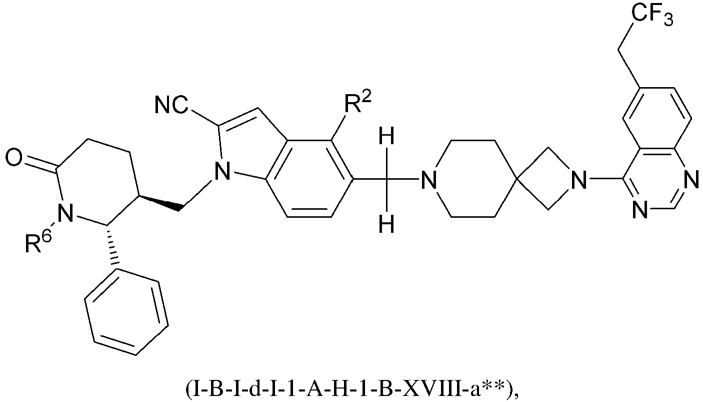 or a pharmaceutically acceptable salt, stereoisomer, solvate, prodrug, isotopic derivative, or tautomer thereof.

In some embodiments the compound is of Formula (I-B-I-d-I-1-A-H-1-B-XVIII-a***): 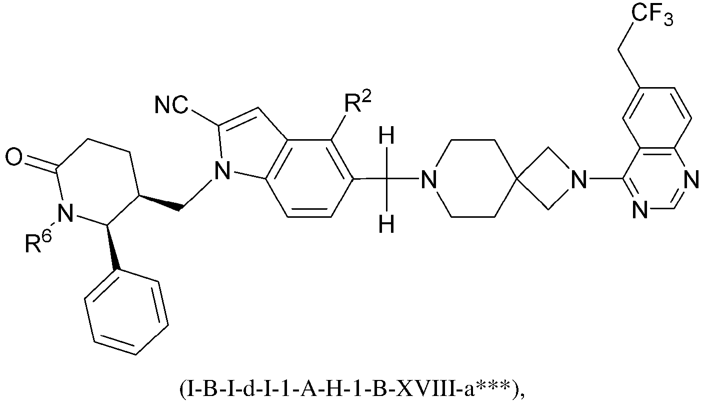 or a pharmaceutically acceptable salt, stereoisomer, solvate, prodrug, isotopic derivative, or tautomer thereof.

In some embodiments the compound is of Formula (I-B-I-d-I-1-A-H-1-B-XVIII-a****): 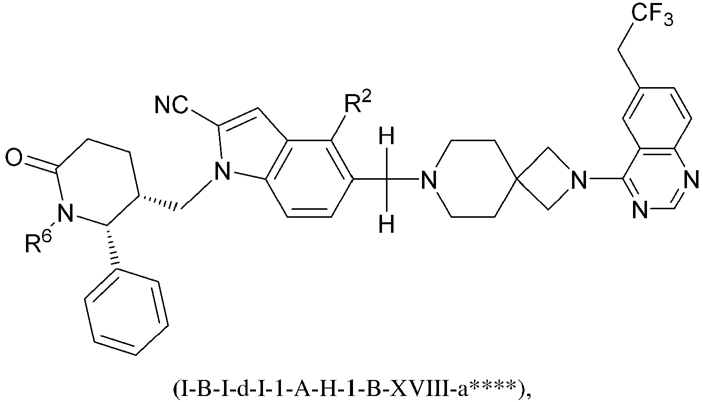 or a pharmaceutically acceptable salt, stereoisomer, solvate, prodrug, isotopic derivative, or tautomer thereof.

In some embodiments the compound is of Formula (I-B-I-d-I-1-A-H-1-B-XIX): 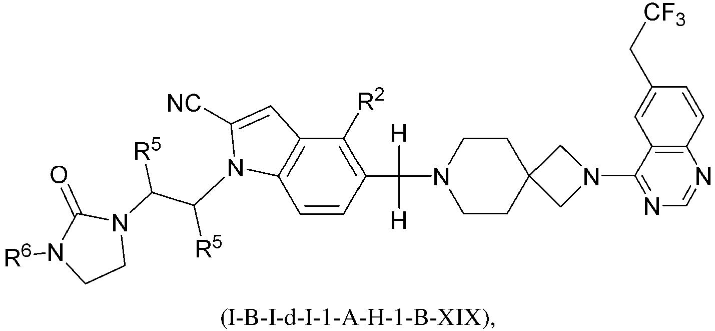 or a pharmaceutically acceptable salt, stereoisomer, solvate, prodrug, isotopic derivative, or tautomer thereof.

In some embodiments the compound is of Formula (I-B-I-d-I-1-A-H-1-B-XIX-a): 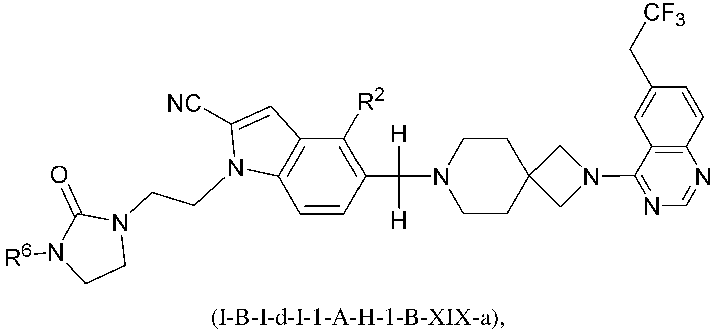 or a pharmaceutically acceptable salt, stereoisomer, solvate, prodrug, isotopic derivative, or tautomer thereof.

In some embodiments the compound is of Formula (I-B-I-d-I-1-A-H-1-B-XX): 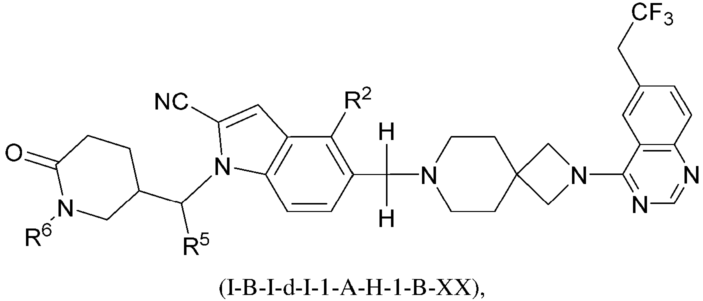 or a pharmaceutically acceptable salt, stereoisomer, solvate, prodrug, isotopic derivative, or tautomer thereof.

In some embodiments the compound is of Formula (I-B-I-d-I-1-A-H-1-B-XX-a): 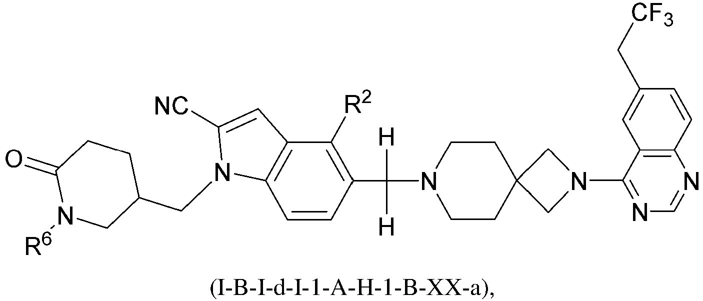 or a pharmaceutically acceptable salt, stereoisomer, solvate, prodrug, isotopic derivative, or tautomer thereof.

In some embodiments the compound is of Formula (I-B-I-d-I-1-A-H-1-B-XXI): 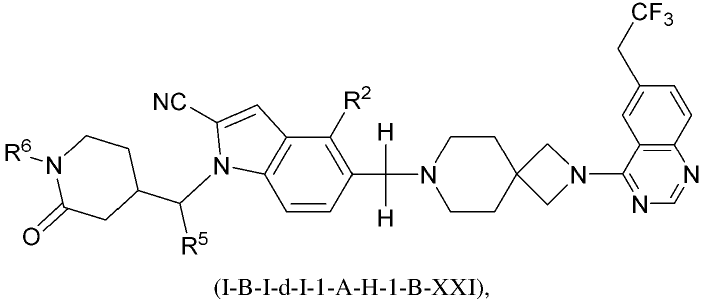 or a pharmaceutically acceptable salt, stereoisomer, solvate, prodrug, isotopic derivative, or tautomer thereof.

In some embodiments the compound is of Formula (I-B-I-d-I-1-A-H-1-B-XXI-a): 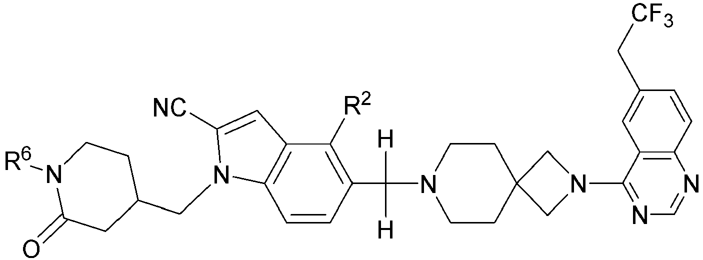 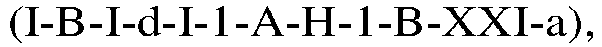 or a pharmaceutically acceptable salt, stereoisomer, solvate, prodrug, isotopic derivative, or tautomer thereof.

In some embodiments the compound is of Formula (I-B-I-d-I-1-A-H-1-B-XXII): 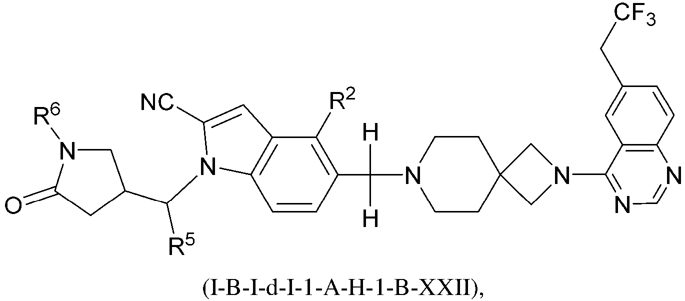 or a pharmaceutically acceptable salt, stereoisomer, solvate, prodrug, isotopic derivative, or tautomer thereof.

In some embodiments the compound is of Formula (I-B-I-d-I-1-A-H-1-B-XXII-a): 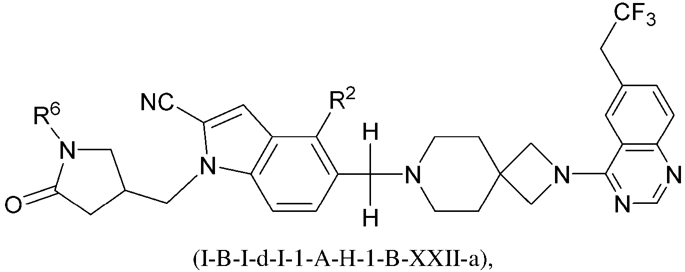 or a pharmaceutically acceptable salt, stereoisomer, solvate, prodrug, isotopic derivative, or tautomer thereof.

In some embodiments the compound is of Formula (I-B-I-d-I-1-A-H-1-B-XXIII): 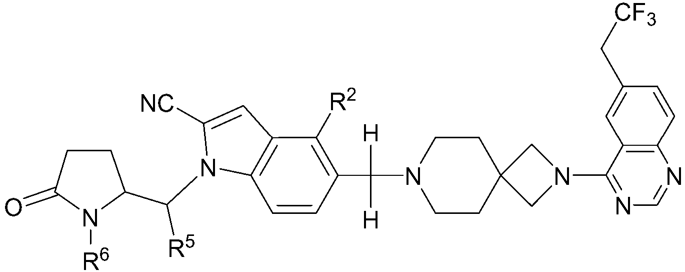 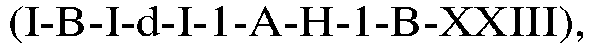 or a pharmaceutically acceptable salt, stereoisomer, solvate, prodrug, isotopic derivative, or tautomer thereof.

In some embodiments the compound is of Formula (I-B-I-d-I-1-A-H-1-B-XXIII-a): 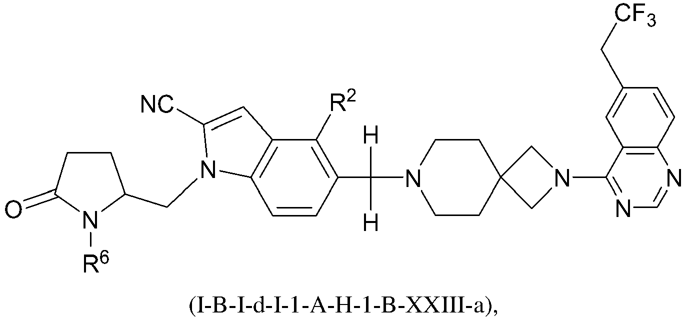 or a pharmaceutically acceptable salt, stereoisomer, solvate, prodrug, isotopic derivative, or tautomer thereof.

In some embodiments the compound is of Formula (I-B-I-d-I-1-A-H-1-B-XXIV): 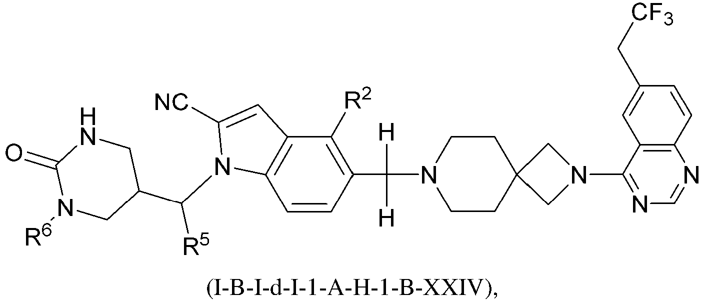 or a pharmaceutically acceptable salt, stereoisomer, solvate, prodrug, isotopic derivative, or tautomer thereof.

In some embodiments the compound is of Formula (I-B-I-d-I-1-A-H-1-B-XXIV-a): 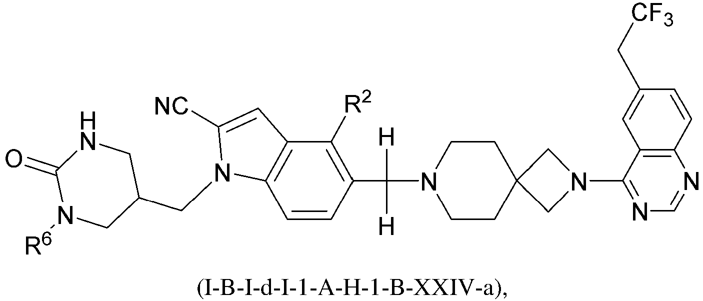 or a pharmaceutically acceptable salt, stereoisomer, solvate, prodrug, isotopic derivative, or tautomer thereof.

In some embodiments the compound is of Formula (I-B-I-d-I-1-A-H-1-B-XXV): 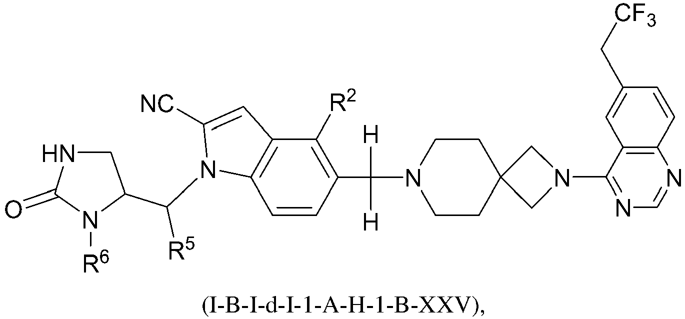 or a pharmaceutically acceptable salt, stereoisomer, solvate, prodrug, isotopic derivative, or tautomer thereof.

In some embodiments the compound is of Formula (I-B-I-d-I-1-A-H-1-B-XXV-a): 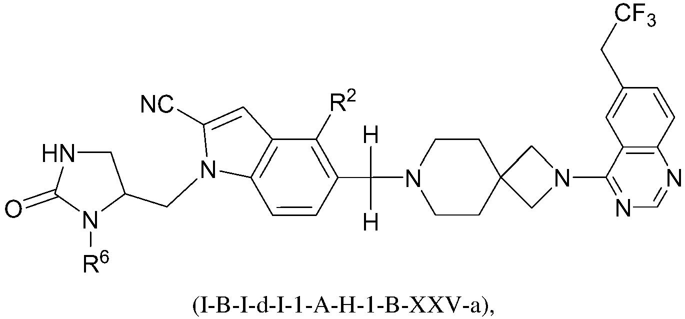 or a pharmaceutically acceptable salt, stereoisomer, solvate, prodrug, isotopic derivative, or tautomer thereof.

In some embodiments the compound is of Formula (I-B-I-d-I-1-A-H-1-B-GT): 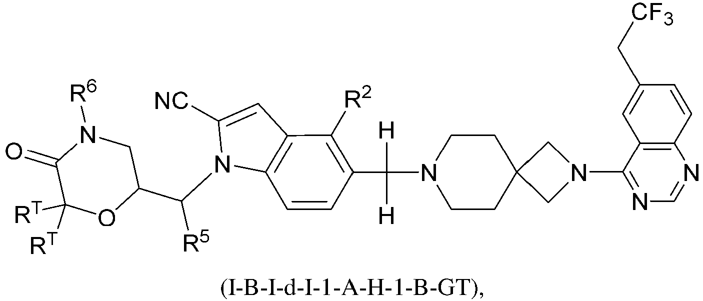 or a pharmaceutically acceptable salt, stereoisomer, solvate, prodrug, isotopic derivative, or tautomer thereof, wherein each R^{T} is independently selected from H, C₁-C₆ alkyl, C₃-C₁₀ cycloalkyl, aryl, heteroaryl.

In some embodiments the compound is of Formula (I-B-I-d-I-1-A-H-1-B-XXVI): 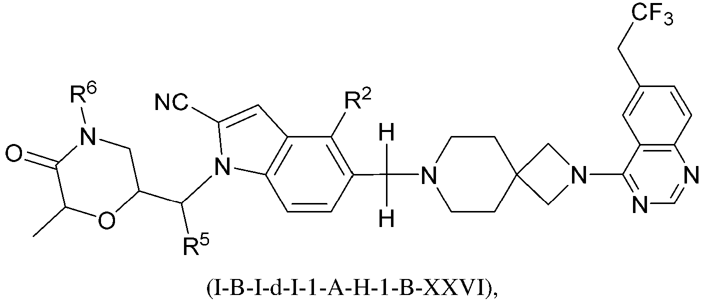 or a pharmaceutically acceptable salt, stereoisomer, solvate, prodrug, isotopic derivative, or tautomer thereof.

In some embodiments the compound is of Formula (I-B-I-d-I-1-A-H-1-B-XXVI-a): 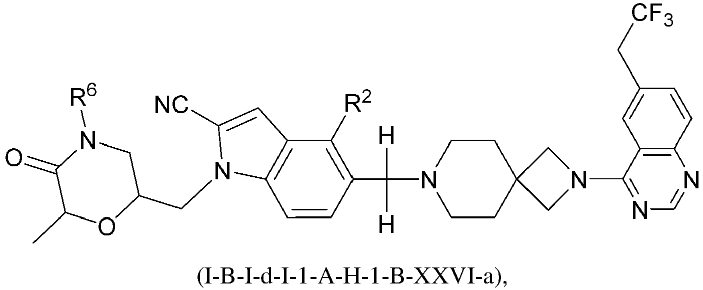 or a pharmaceutically acceptable salt, stereoisomer, solvate, prodrug, isotopic derivative, or tautomer thereof.

In some embodiments the compound is of Formula (I-B-I-d-I-1-A-H-1-B-XXVII): 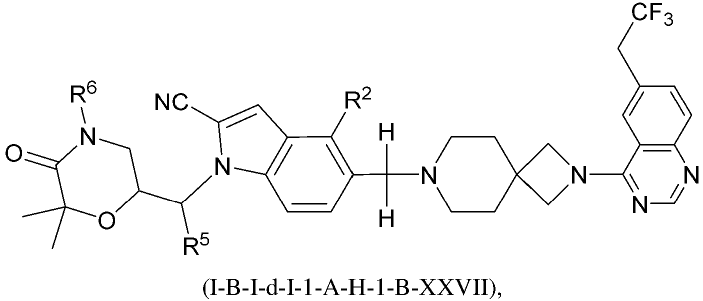 or a pharmaceutically acceptable salt, stereoisomer, solvate, prodrug, isotopic derivative, or tautomer thereof.

In some embodiments the compound is of Formula (I-B-I-d-I-1-A-H-1-B-XXVII-a): 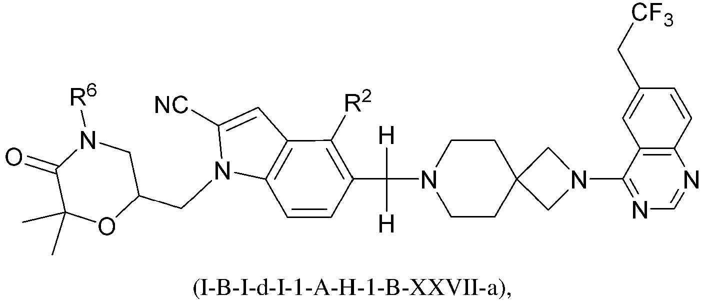 or a pharmaceutically acceptable salt, stereoisomer, solvate, prodrug, isotopic derivative, or tautomer thereof.

In some embodiments the compound is of Formula (I-B-I-d-I-1-A-H-1-B-GGG): 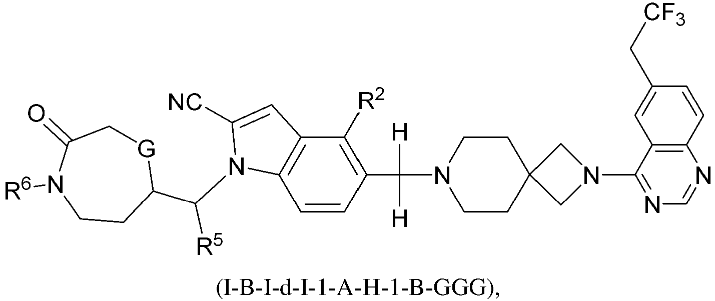 or a pharmaceutically acceptable salt, stereoisomer, solvate, prodrug, isotopic derivative, or tautomer thereof, wherein G is selected from CH₂, O, S, NH and all\other variables are as defined herein.

In some embodiments the compound is of Formula (I-B-I-d-I-1-A-H-1-B-XXVIII): 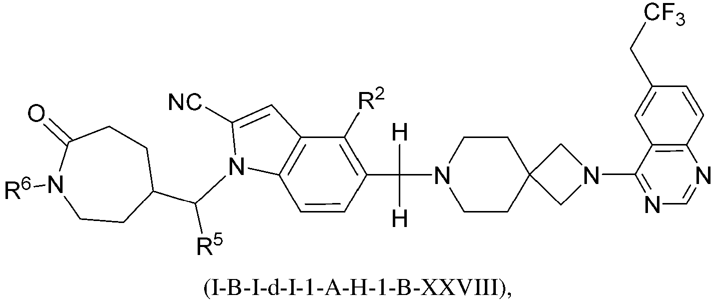 or a pharmaceutically acceptable salt, stereoisomer, solvate, prodrug, isotopic derivative, or tautomer thereof.

In some embodiments the compound is of Formula (I-B-I-d-I-1-A-H-1-B-XXVIII-a): 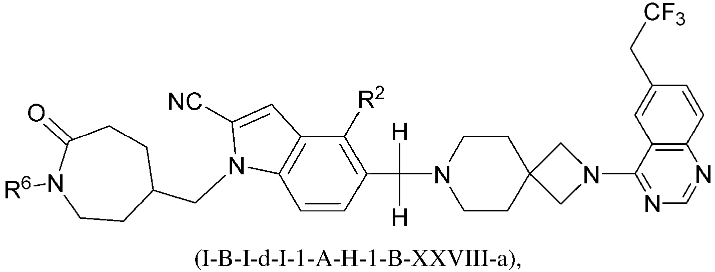 or a pharmaceutically acceptable salt, stereoisomer, solvate, prodrug, isotopic derivative, or tautomer thereof.

In some embodiments the compound is of Formula (I-B-I-d-I-1-A-H-1-B-XXIX): 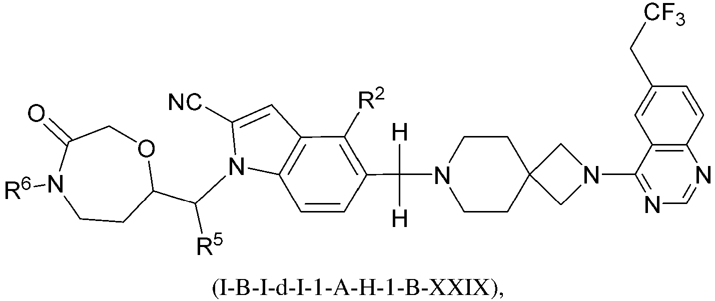 or a pharmaceutically acceptable salt, stereoisomer, solvate, prodrug, isotopic derivative, or tautomer thereof.

In some embodiments the compound is of Formula (I-B-I-d-I-1-A-H-1-B-XXIX-a): 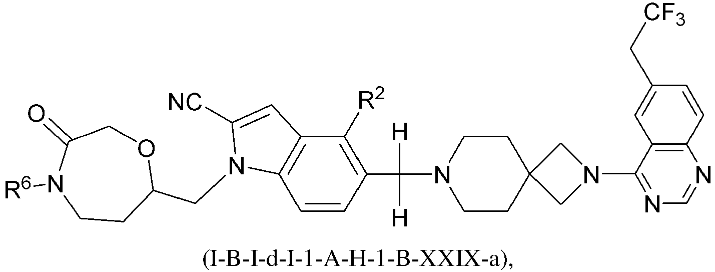 or a pharmaceutically acceptable salt, stereoisomer, solvate, prodrug, isotopic derivative, or tautomer thereof.

In some embodiments the compound is of Formula (I-B-I-d-I-1-A-H-1-B-XXX): 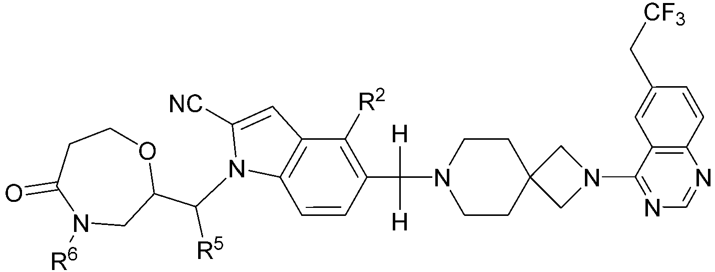 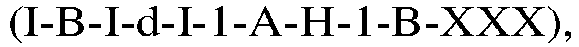 or a pharmaceutically acceptable salt, stereoisomer, solvate, prodrug, isotopic derivative, or tautomer thereof.

In some embodiments the compound is of Formula (I-B-I-d-I-1-A-H-1-B-XXX-a): 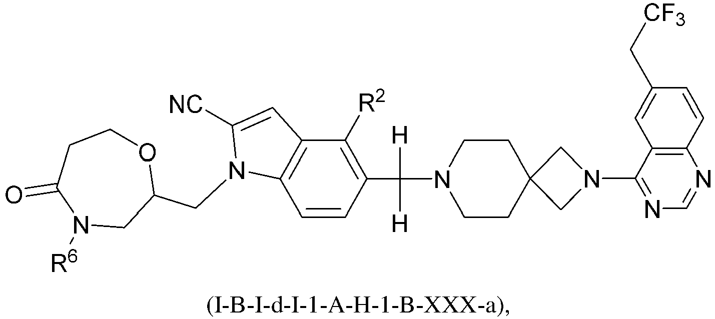 or a pharmaceutically acceptable salt, stereoisomer, solvate, prodrug, isotopic derivative, or tautomer thereof.

In some embodiments the compound is of Formula (I-B-I-d-I-1-A-H-1-B-XXXI): 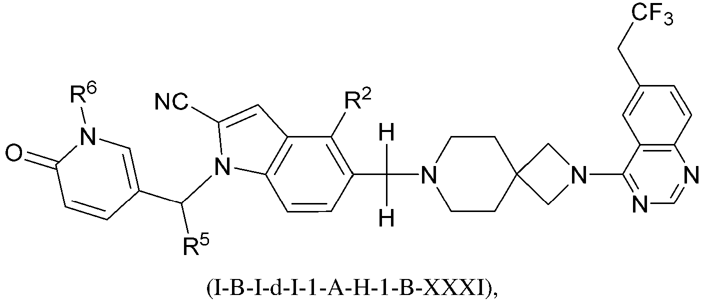 or a pharmaceutically acceptable salt, stereoisomer, solvate, prodrug, isotopic derivative, or tautomer thereof.

In some embodiments the compound is of Formula (I-B-I-d-I-1-A-H-1-B-XXXI-a): 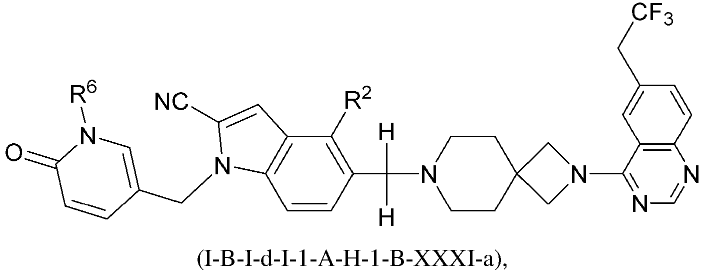 or a pharmaceutically acceptable salt, stereoisomer, solvate, prodrug, isotopic derivative, or tautomer thereof.

In some embodiments the compound is of Formula (I-B-I-d-I-1-A-H-1-B-XXXII): 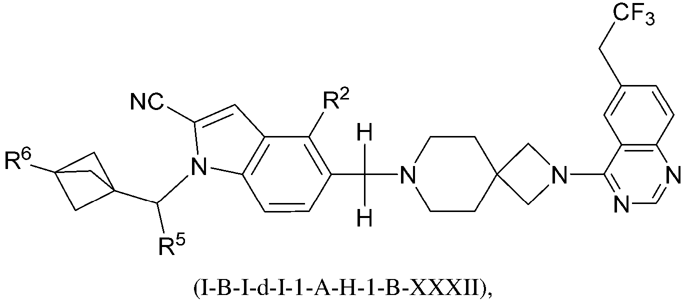 or a pharmaceutically acceptable salt, stereoisomer, solvate, prodrug, isotopic derivative, or tautomer thereof.

In some embodiments the compound is of Formula (I-B-I-d-I-1-A-H-1-B-XXXII-a): 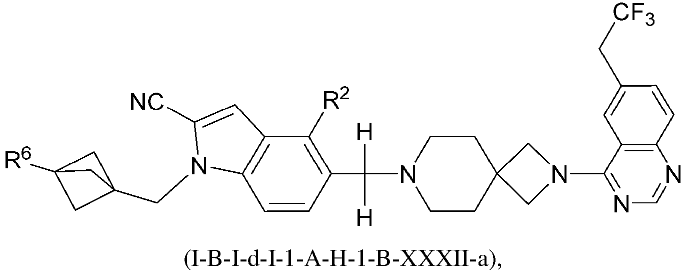 or a pharmaceutically acceptable salt, stereoisomer, solvate, prodrug, isotopic derivative, or tautomer thereof.

In some embodiments the compound is of Formula (I-B-I-d-I-1-A-H-1-B-XXXIII): 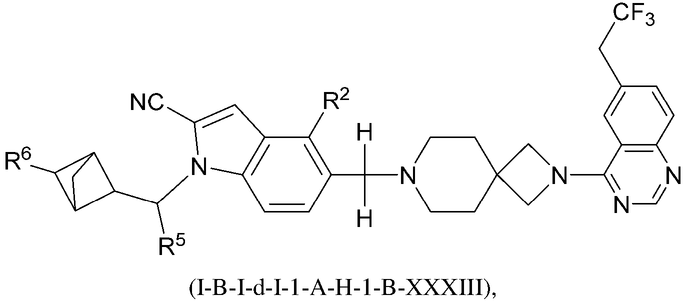 or a pharmaceutically acceptable salt, stereoisomer, solvate, prodrug, isotopic derivative, or tautomer thereof.

In some embodiments the compound is of Formula (I-B-I-d-I-1-A-H-1-B-XXXIII-a): 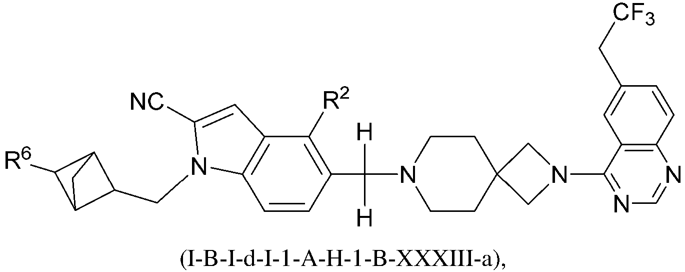 or a pharmaceutically acceptable salt, stereoisomer, solvate, prodrug, isotopic derivative, or tautomer thereof.

In some embodiments the compound is of Formula (I-B-I-d-I-1-A-H-2): 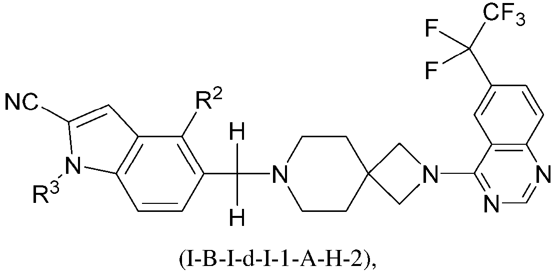 or a pharmaceutically acceptable salt, stereoisomer, solvate, prodrug, isotopic derivative, or tautomer thereof.

In some embodiments the compound is of Formula (I-B-I-d-I-1-A-H-2-h): 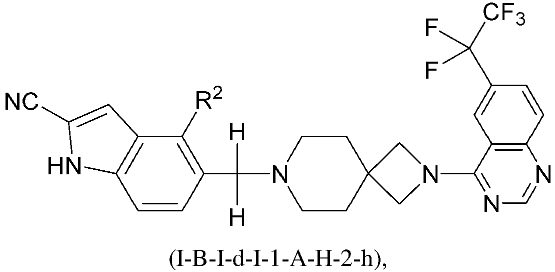 or a pharmaceutically acceptable salt, stereoisomer, solvate, prodrug, isotopic derivative, or tautomer thereof.

In some embodiments the compound is of Formula (I-B-I-d-I-1-A-H-2-B): 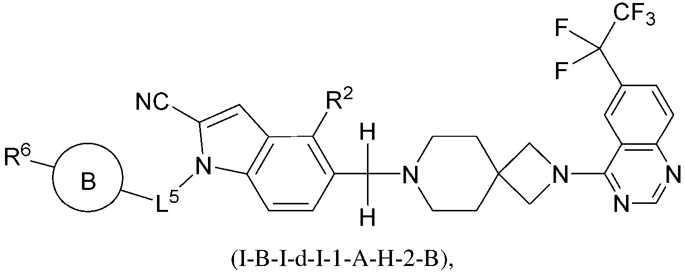 or a pharmaceutically acceptable salt, stereoisomer, solvate, prodrug, isotopic derivative, or tautomer thereof.

In some embodiments the compound is of Formula (I-B-I-d-I-1-A-H-2-B-G): 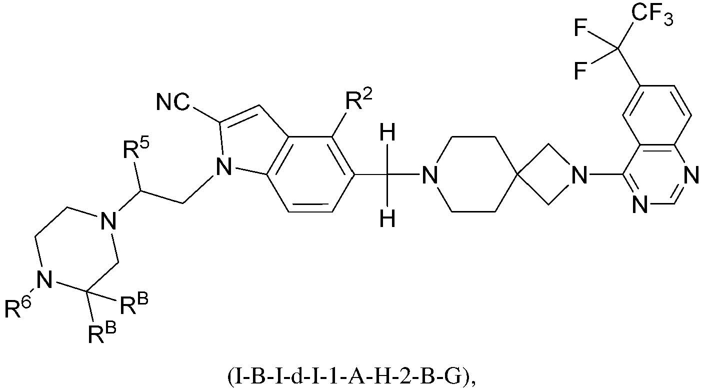 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-B-I-d-I-1-A-H-2-B-I): 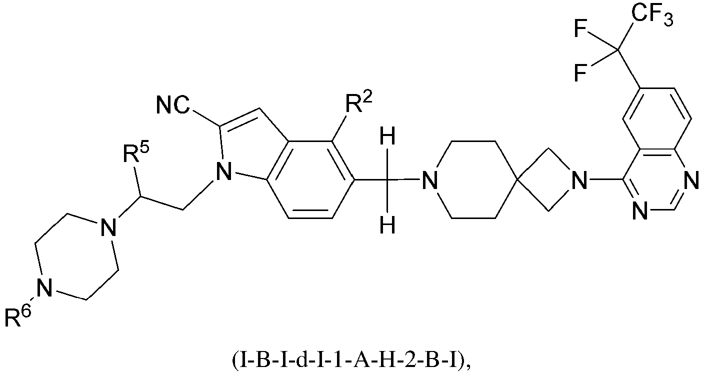 or a pharmaceutically acceptable salt, stereoisomer, solvate, prodrug, isotopic derivative, or tautomer thereof.

In some embodiments the compound is of Formula (I-B-I-d-I-1-A-H-2-B-I-a): 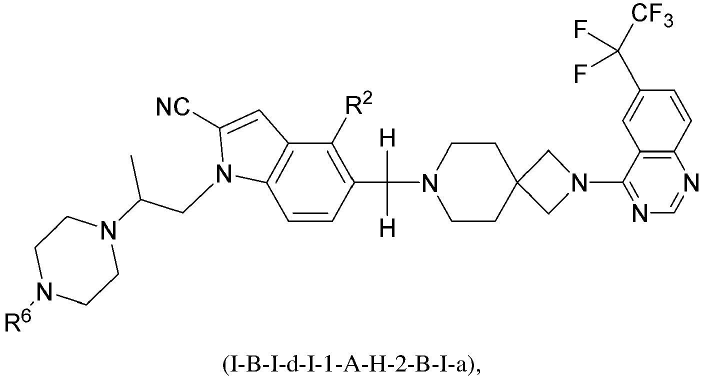 or a pharmaceutically acceptable salt, stereoisomer, solvate, prodrug, isotopic derivative, or tautomer thereof.

In some embodiments the compound is of Formula (I-B-I-d-I-1-A-H-2-B-I-a*): 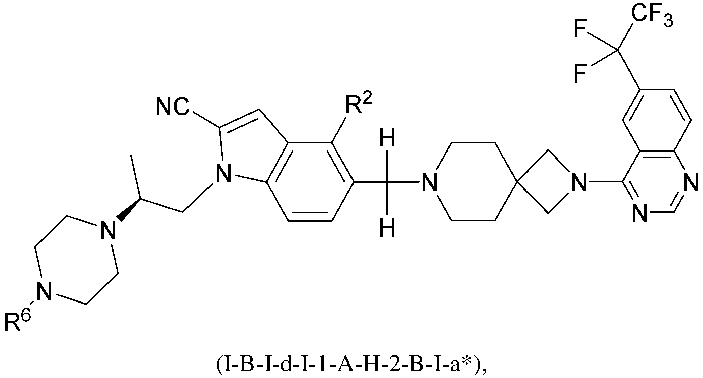 or a pharmaceutically acceptable salt, stereoisomer, solvate, prodrug, isotopic derivative, or tautomer thereof.

In some embodiments the compound is of Formula (I-B-I-d-I-1-A-H-2-B-I-a**): 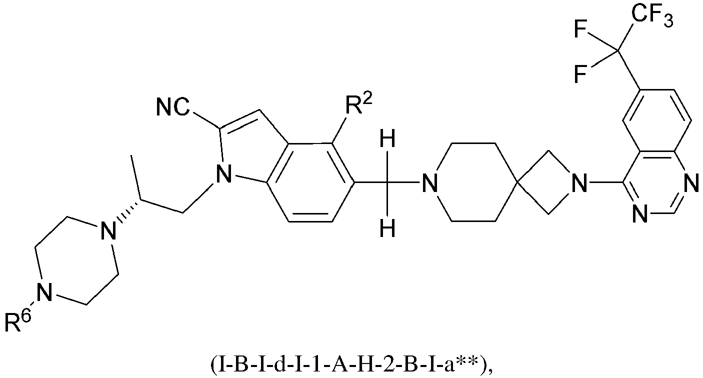 or a pharmaceutically acceptable salt, stereoisomer, solvate, prodrug, isotopic derivative, or tautomer thereof.

In some embodiments the compound is of Formula (I-B-I-d-I-1-A-H-3): 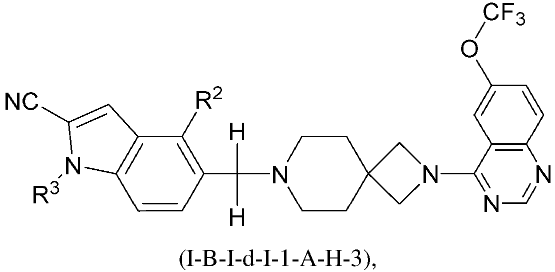 or a pharmaceutically acceptable salt, stereoisomer, solvate, prodrug, isotopic derivative, or tautomer thereof.

In some embodiments the compound is of Formula (I-B-I-d-I-1-A-H-3-h): 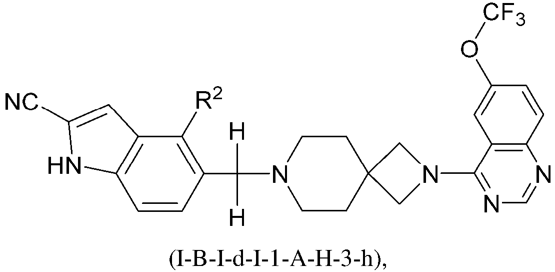 or a pharmaceutically acceptable salt, stereoisomer, solvate, prodrug, isotopic derivative, or tautomer thereof.

In some embodiments the compound is of Formula (I-B-I-d-I-1-A-H-3-B): 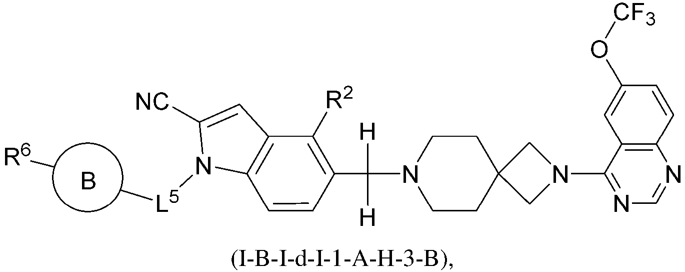 or a pharmaceutically acceptable salt, stereoisomer, solvate, prodrug, isotopic derivative, or tautomer thereof.

In some embodiments the compound is of Formula (I-B-I-d-I-1-A-H-3-B-I): 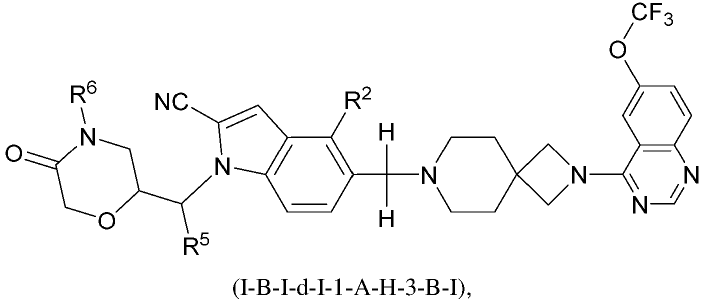 or a pharmaceutically acceptable salt, stereoisomer, solvate, prodrug, isotopic derivative, or tautomer thereof.

In some embodiments the compound is of Formula (I-B-I-d-I-1-A-H-3-B-I-a): 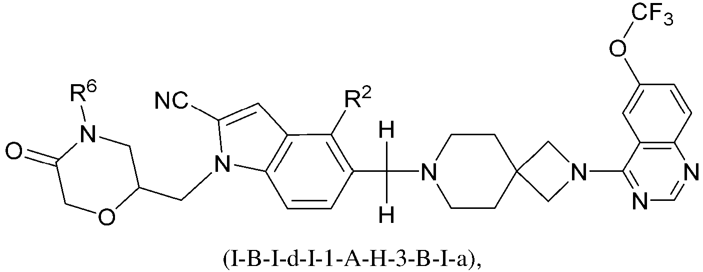 or a pharmaceutically acceptable salt, stereoisomer, solvate, prodrug, isotopic derivative, or tautomer thereof.

In some embodiments the compound is of Formula (I-B-I-d-I-1-A-H-3-B-I-a*): 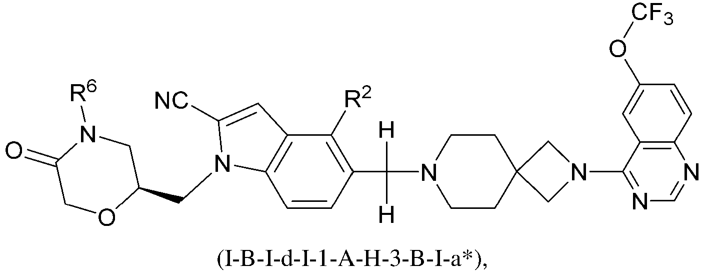 or a pharmaceutically acceptable salt, stereoisomer, solvate, prodrug, isotopic derivative, or tautomer thereof.

In some embodiments the compound is of Formula (I-B-I-d-I-1-A-H-3-B-I-a**): 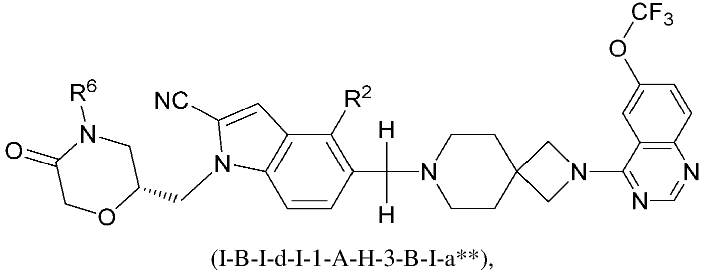 or a pharmaceutically acceptable salt, stereoisomer, solvate, prodrug, isotopic derivative, or tautomer thereof.

In some embodiments the compound is of Formula (I-B-I-d-I-1-B): 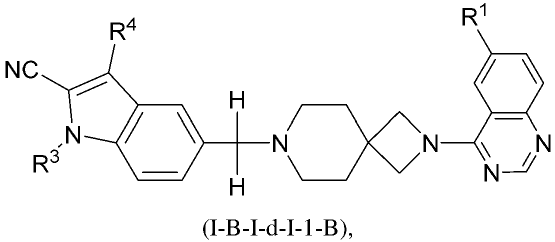 or a pharmaceutically acceptable salt, stereoisomer, solvate, prodrug, isotopic derivative, or tautomer thereof.

In some embodiments the compound is of Formula (I-B-I-d-I-1-B-H): 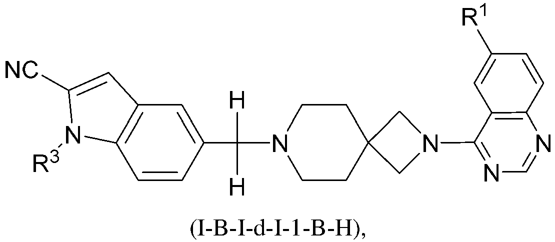 or a pharmaceutically acceptable salt, stereoisomer, solvate, prodrug, isotopic derivative, or tautomer thereof.

In some embodiments the compound is of Formula (I-B-I-d-I-1-B-H-1): 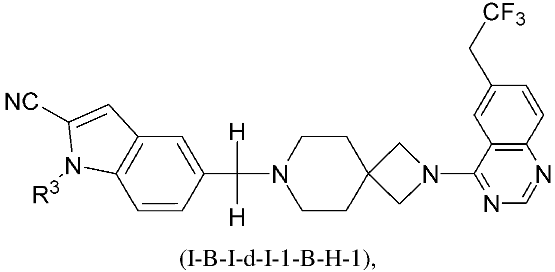 or a pharmaceutically acceptable salt, stereoisomer, solvate, prodrug, isotopic derivative, or tautomer thereof.

In some embodiments the compound is of Formula (I-B-I-d-I-1-B-H-1-B): 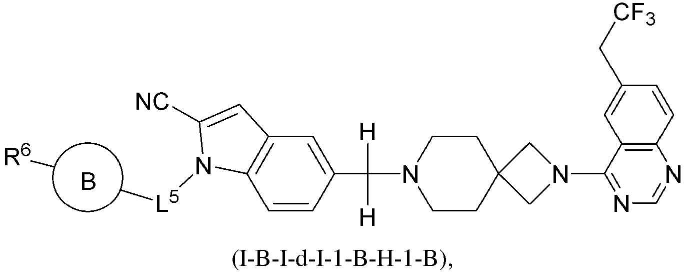 or a pharmaceutically acceptable salt, stereoisomer, solvate, prodrug, isotopic derivative, or tautomer thereof.

In some embodiments the compound is of Formula (I-B-I-d-I-1-B-H-1-B-I): 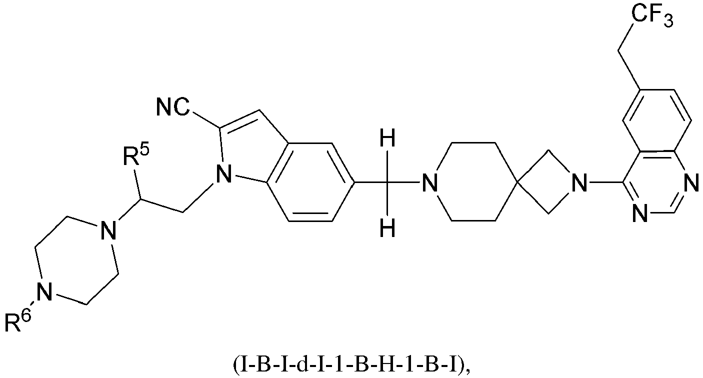 or a pharmaceutically acceptable salt, stereoisomer, solvate, prodrug, isotopic derivative, or tautomer thereof.

In some embodiments the compound is of Formula (I-B-I-d-I-1-B-H-1-B-I-a): 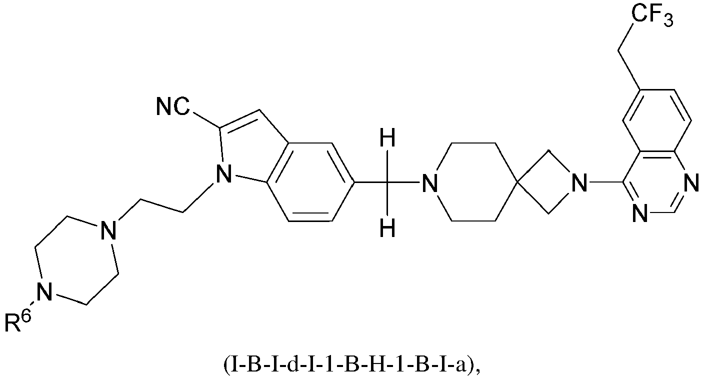 or a pharmaceutically acceptable salt, stereoisomer, solvate, prodrug, isotopic derivative, or tautomer thereof.

In some embodiments the compound is of Formula (I-B-I-d-I-1-C): 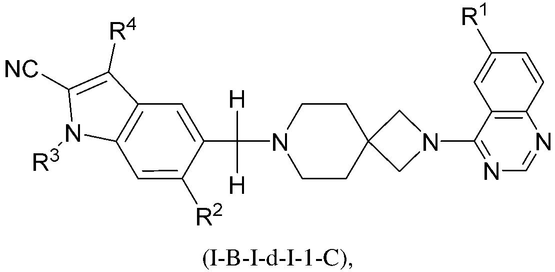 or a pharmaceutically acceptable salt, stereoisomer, solvate, prodrug, isotopic derivative, or tautomer thereof.

In some embodiments the compound is of Formula (I-B-I-d-I-1-C-H): 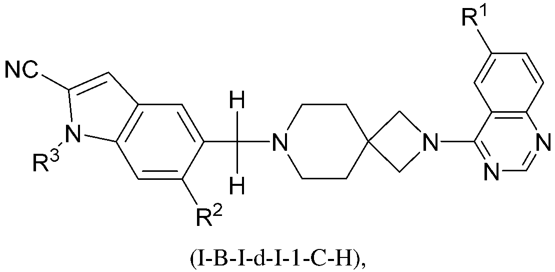 or a pharmaceutically acceptable salt, stereoisomer, solvate, prodrug, isotopic derivative, or tautomer thereof.

In some embodiments the compound is of Formula (I-B-I-d-I-1-C-H-1): 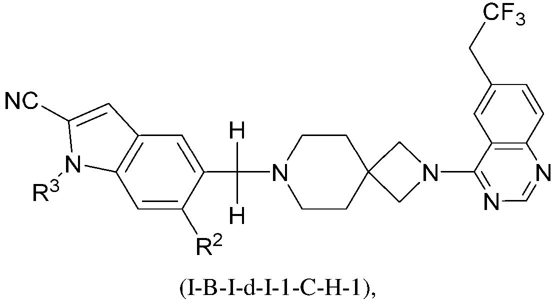 or a pharmaceutically acceptable salt, stereoisomer, solvate, prodrug, isotopic derivative, or tautomer thereof.

In some embodiments the compound is of Formula (I-B-I-d-I-1-C-H-1-h): 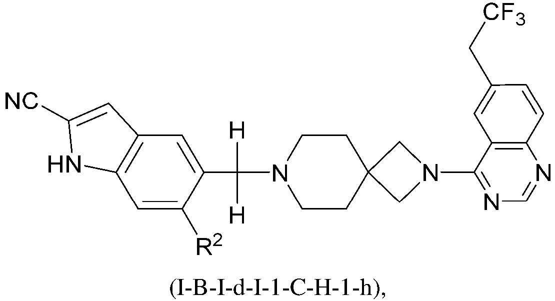 or a pharmaceutically acceptable salt, stereoisomer, solvate, prodrug, isotopic derivative, or tautomer thereof.

In some embodiments the compound is of Formula (I-B-I-d-I-1-C-H-1-B): 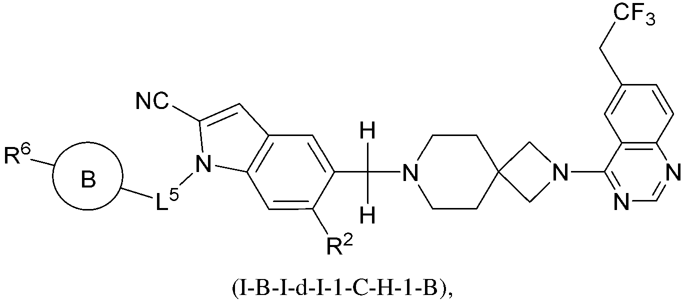 or a pharmaceutically acceptable salt, stereoisomer, solvate, prodrug, isotopic derivative, or tautomer thereof.

In some embodiments the compound is of Formula (I-B-I-d-I-1-C-H-1-B-I): 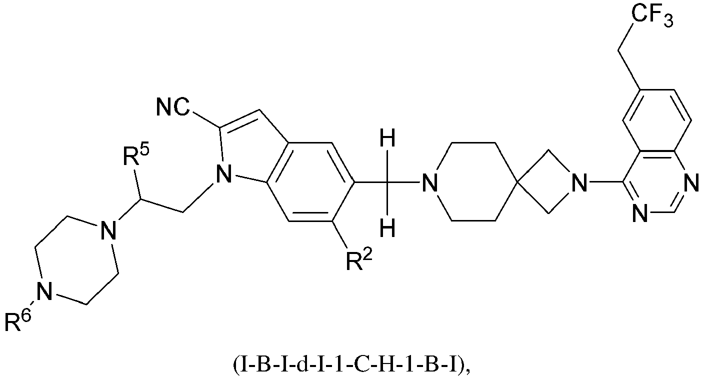 or a pharmaceutically acceptable salt, stereoisomer, solvate, prodrug, isotopic derivative, or tautomer thereof.

In some embodiments the compound is of Formula (I-B-I-d-I-1-C-H-1-B-I-a): 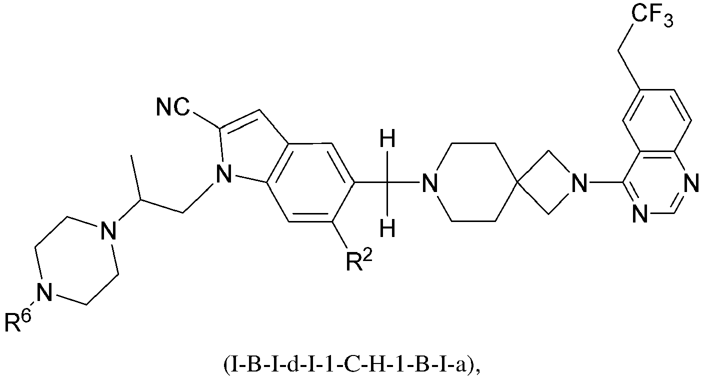 or a pharmaceutically acceptable salt, stereoisomer, solvate, prodrug, isotopic derivative, or tautomer thereof.

In some embodiments the compound is of Formula (I-B-I-d-I-1-C-H-1-B-I-a*): 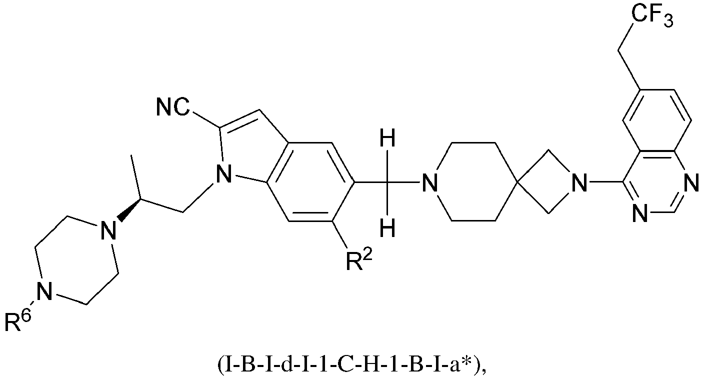 or a pharmaceutically acceptable salt, stereoisomer, solvate, prodrug, isotopic derivative, or tautomer thereof.

In some embodiments the compound is of Formula (I-B-I-d-I-1-C-H-1-B-I-a**): 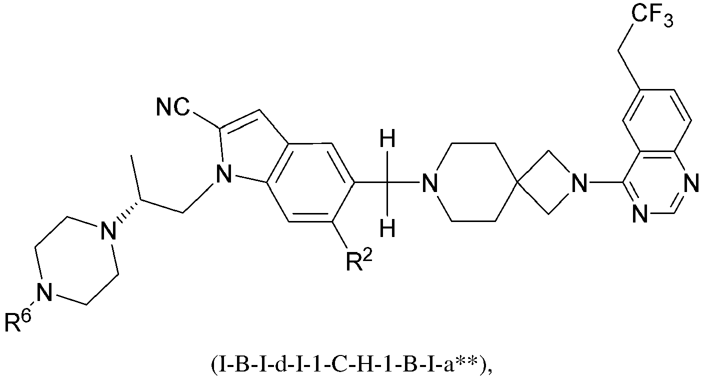 or a pharmaceutically acceptable salt, stereoisomer, solvate, prodrug, isotopic derivative, or tautomer thereof.

In some embodiments the compound is of Formula (I-B-I-d-I-1-D): 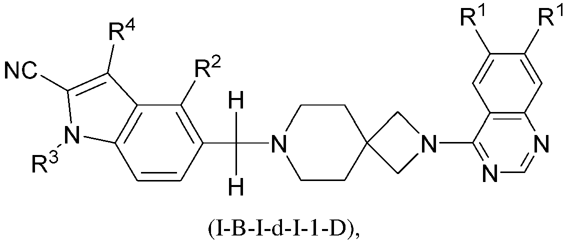 or a pharmaceutically acceptable salt, stereoisomer, solvate, prodrug, isotopic derivative, or tautomer thereof.

In some embodiments the compound is of Formula (I-B-I-d-I-1-D-H): 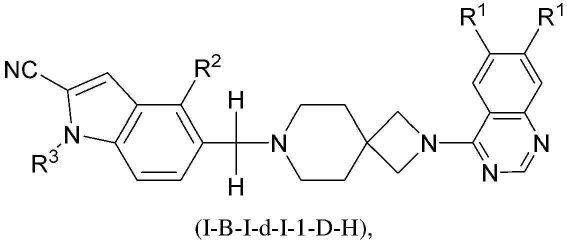 or a pharmaceutically acceptable salt, stereoisomer, solvate, prodrug, isotopic derivative, or tautomer thereof.

In some embodiments the compound is of Formula (I-B-I-d-I-1-D-H-1): 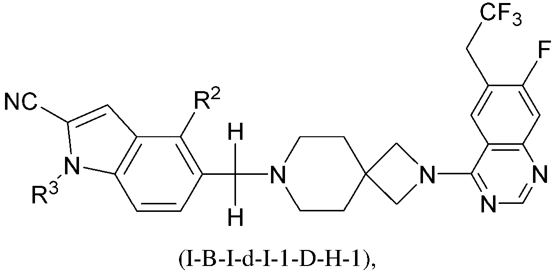 or a pharmaceutically acceptable salt, stereoisomer, solvate, prodrug, isotopic derivative, or tautomer thereof.

In some embodiments the compound is of Formula (I-B-I-d-I-1-D-H-1-h): 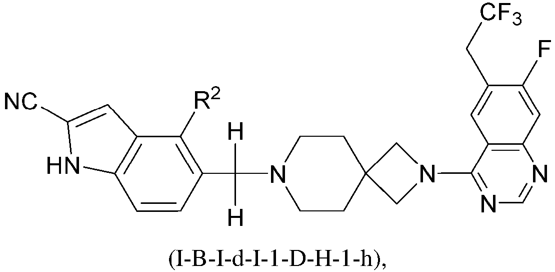 or a pharmaceutically acceptable salt, stereoisomer, solvate, prodrug, isotopic derivative, or tautomer thereof.

In some embodiments the compound is of Formula (I-B-I-d-I-1-D-H-1-B): 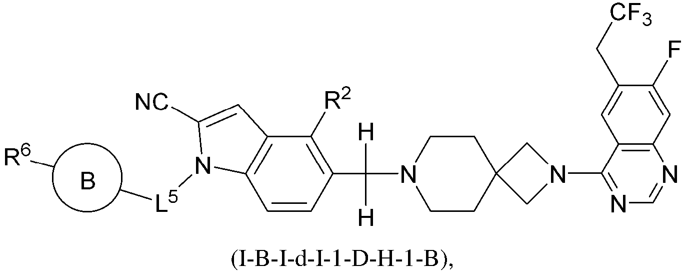 or a pharmaceutically acceptable salt, stereoisomer, solvate, prodrug, isotopic derivative, or tautomer thereof.

In some embodiments the compound is of Formula (I-B-I-d-I-1-D-H-1-B-G): 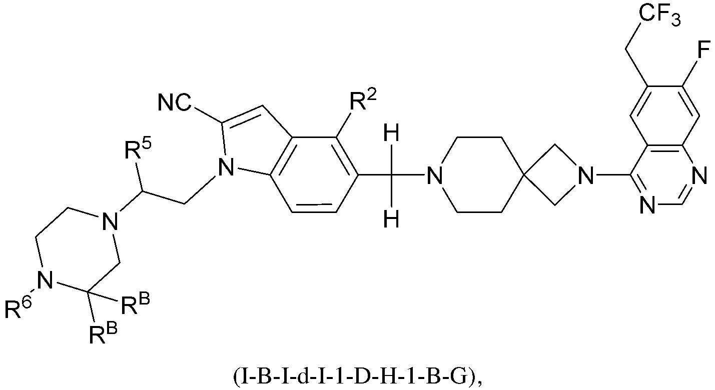 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-B-I-d-I-1-D-H-1-B-I): 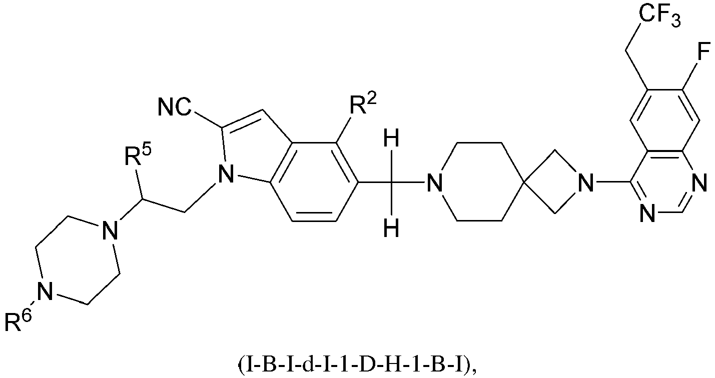 or a pharmaceutically acceptable salt, stereoisomer, solvate, prodrug, isotopic derivative, or tautomer thereof.

In some embodiments the compound is of Formula (I-B-I-d-I-1-D-H-1-B-I-a): 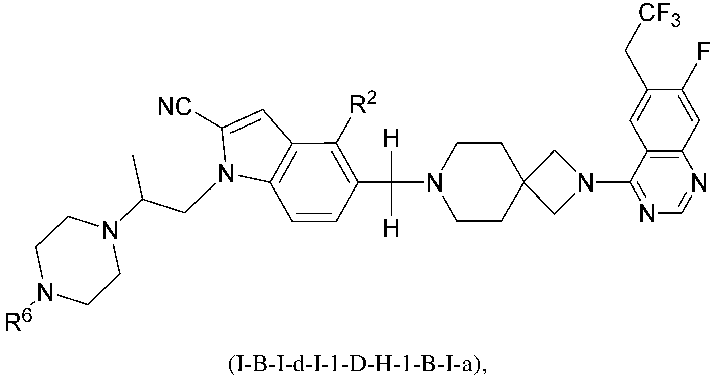 or a pharmaceutically acceptable salt, stereoisomer, solvate, prodrug, isotopic derivative, or tautomer thereof.

In some embodiments the compound is of Formula (I-B-I-d-I-1-D-H-1-B-I-a*): 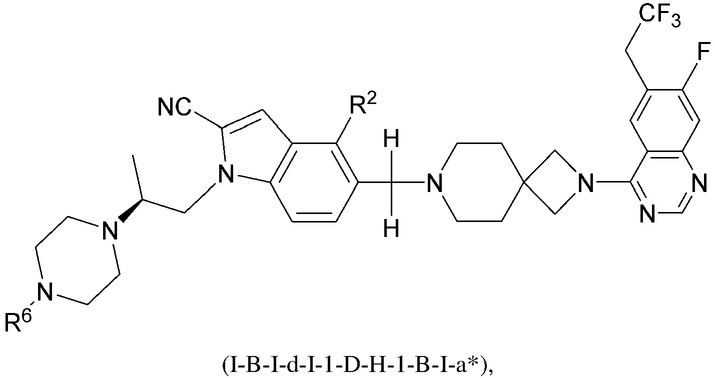 or a pharmaceutically acceptable salt, stereoisomer, solvate, prodrug, isotopic derivative, or tautomer thereof.

In some embodiments the compound is of Formula (I-B-I-d-I-1-D-H-1-B-I-a**): 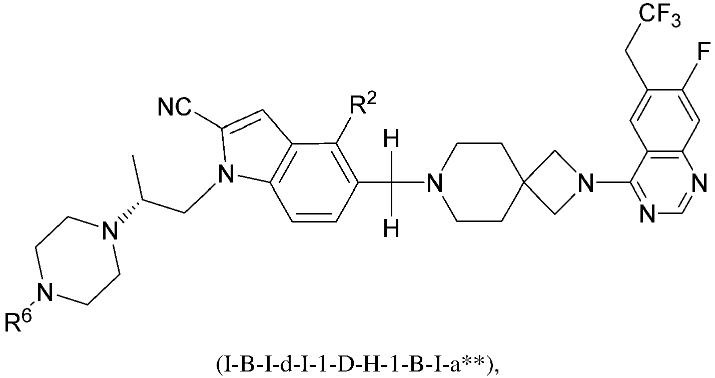 or a pharmaceutically acceptable salt, stereoisomer, solvate, prodrug, isotopic derivative, or tautomer thereof.

In some embodiments the compound is of Formula (I-B-I-d-I-1-E): 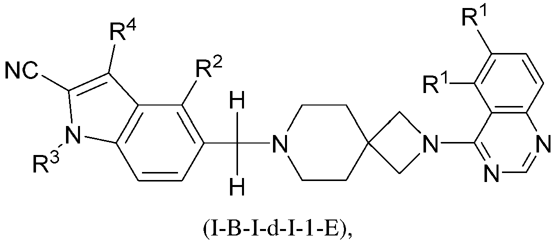 or a pharmaceutically acceptable salt, stereoisomer, solvate, prodrug, isotopic derivative, or tautomer thereof.

In some embodiments the compound is of Formula (I-B-I-d-I-1-E-H): 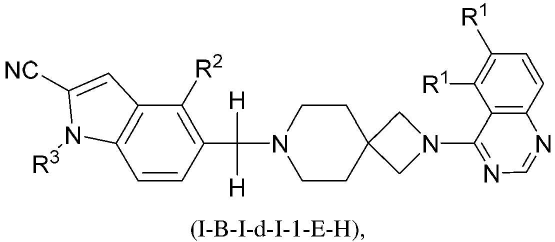 or a pharmaceutically acceptable salt, stereoisomer, solvate, prodrug, isotopic derivative, or tautomer thereof.

In some embodiments the compound is of Formula (I-B-I-d-I-1-E-H-1): 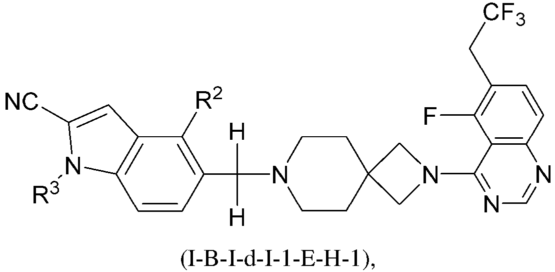 or a pharmaceutically acceptable salt, stereoisomer, solvate, prodrug, isotopic derivative, or tautomer thereof.

In some embodiments the compound is of Formula (I-B-I-d-I-1-E-H-1-h): 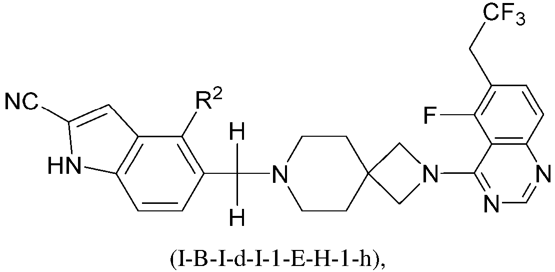 or a pharmaceutically acceptable salt, stereoisomer, solvate, prodrug, isotopic derivative, or tautomer thereof.

In some embodiments the compound is of Formula (I-B-I-d-I-1-E-H-1-B): 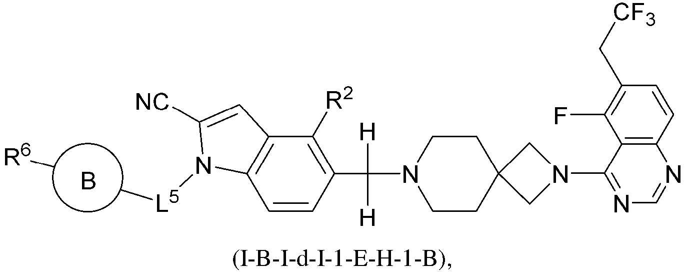 or a pharmaceutically acceptable salt, stereoisomer, solvate, prodrug, isotopic derivative, or tautomer thereof.

In some embodiments the compound is of Formula (I-B-I-d-I-1-E-H-1-B-G): 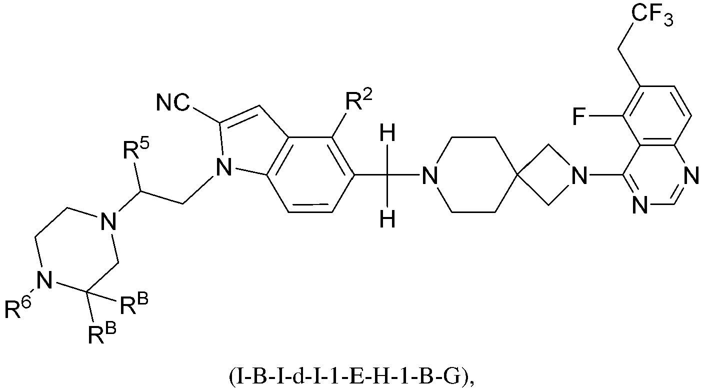 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-B-I-d-I-1-E-H-1-B-I): 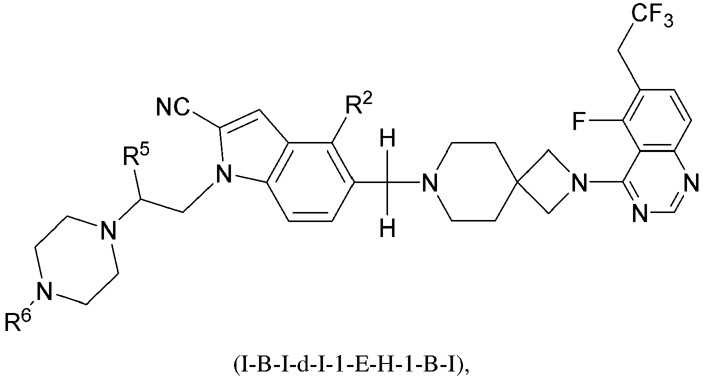 or a pharmaceutically acceptable salt, stereoisomer, solvate, prodrug, isotopic derivative, or tautomer thereof.

In some embodiments the compound is of Formula (I-B-I-d-I-1-E-H-1-B-I-a): 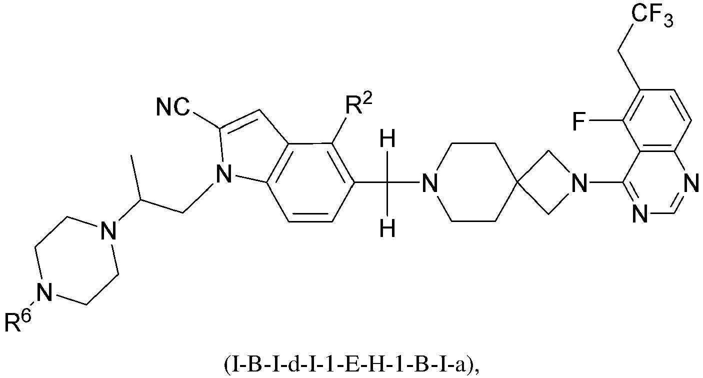 or a pharmaceutically acceptable salt, stereoisomer, solvate, prodrug, isotopic derivative, or tautomer thereof.

In some embodiments the compound is of Formula (I-B-I-d-I-1-E-H-1-B-I-a*): 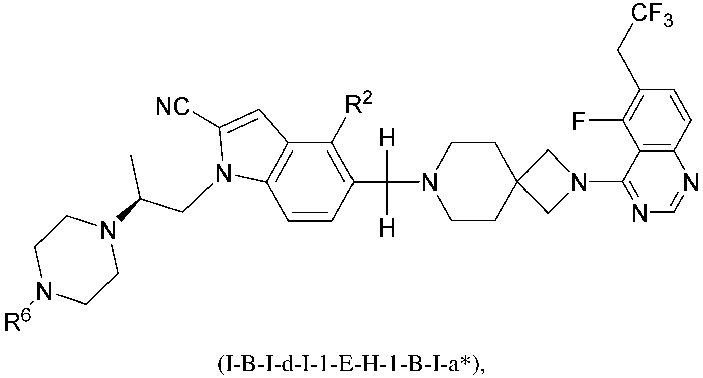 or a pharmaceutically acceptable salt, stereoisomer, solvate, prodrug, isotopic derivative, or tautomer thereof.

In some embodiments the compound is of Formula (I-B-I-d-I-1-E-H-1-B-I-a**): 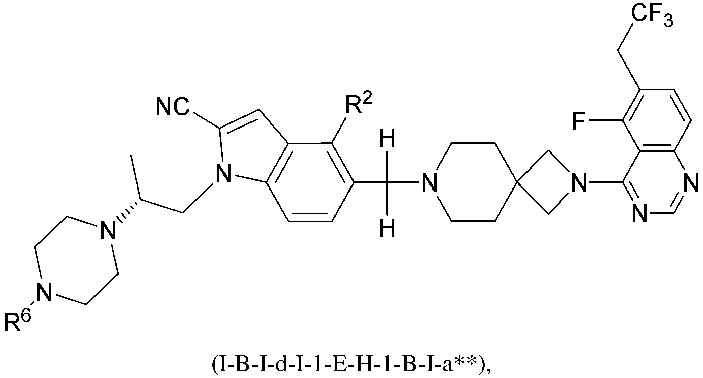 or a pharmaceutically acceptable salt, stereoisomer, solvate, prodrug, isotopic derivative, or tautomer thereof.

In some embodiments the compound is of Formula (I-B-I-d-I-1-E-H-1-B-II): 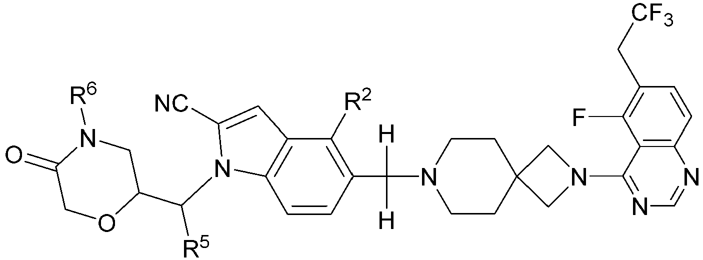 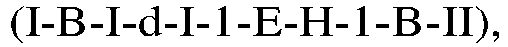 or a pharmaceutically acceptable salt, stereoisomer, solvate, prodrug, isotopic derivative, or tautomer thereof.

In some embodiments the compound is of Formula (I-B-I-d-I-1-E-H-1-B-II-a): 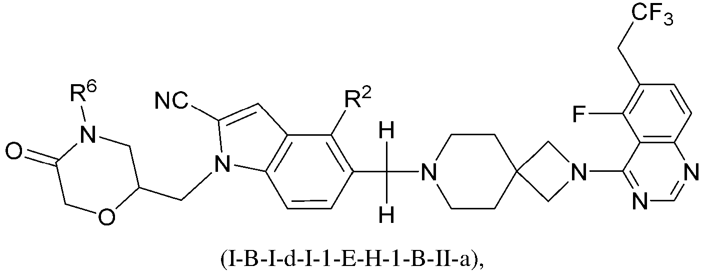 or a pharmaceutically acceptable salt, stereoisomer, solvate, prodrug, isotopic derivative, or tautomer thereof.

In some embodiments the compound is of Formula (I-B-I-d-I-1-E-H-1-B-II-a*): 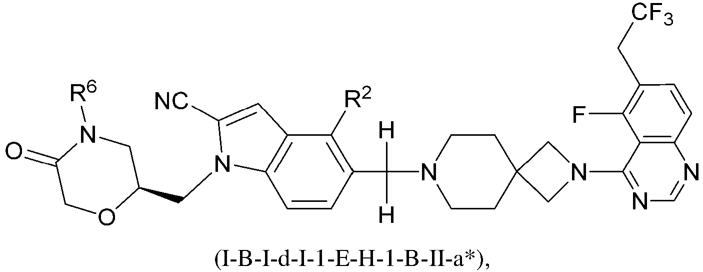 or a pharmaceutically acceptable salt, stereoisomer, solvate, prodrug, isotopic derivative, or tautomer thereof.

In some embodiments the compound is of Formula (I-B-I-d-I-1-E-H-1-B-II-a**): 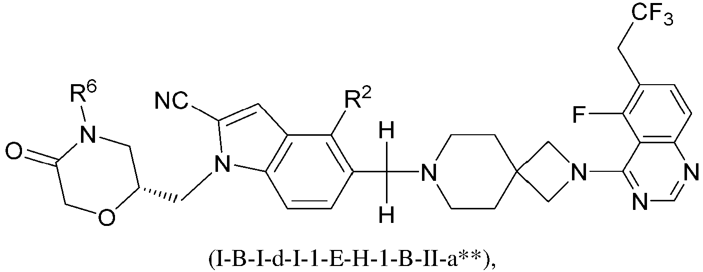 or a pharmaceutically acceptable salt, stereoisomer, solvate, prodrug, isotopic derivative, or tautomer thereof.

In some embodiments the compound is of Formula (I-B-I-d-I-2): 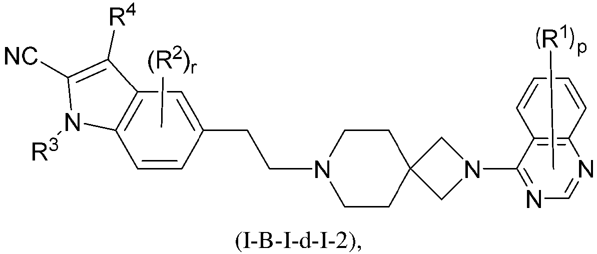 or a pharmaceutically acceptable salt, stereoisomer, solvate, prodrug, isotopic derivative, or tautomer thereof.

In some embodiments the compound is of Formula (I-B-I-d-I-2-A): 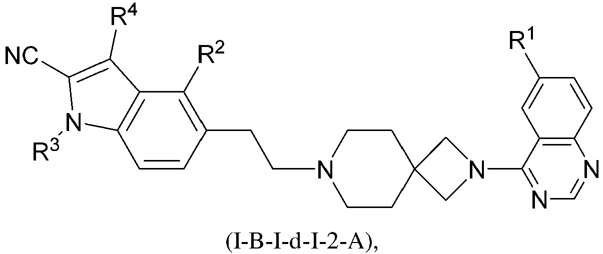 or a pharmaceutically acceptable salt, stereoisomer, solvate, prodrug, isotopic derivative, or tautomer thereof.

In some embodiments the compound is of Formula (I-B-I-d-I-2-A-H): 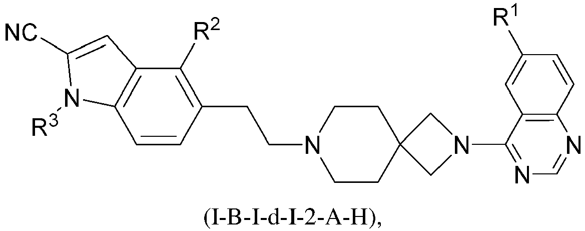 or a pharmaceutically acceptable salt, stereoisomer, solvate, prodrug, isotopic derivative, or tautomer thereof.

In some embodiments the compound is of Formula (I-B-I-d-I-2-A-H-1): 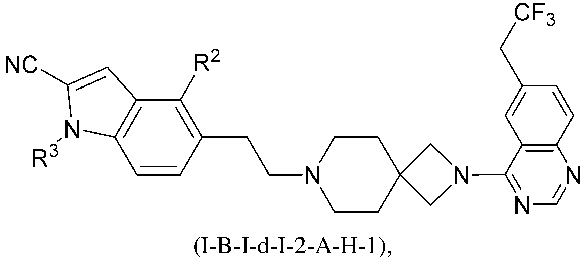 or a pharmaceutically acceptable salt, stereoisomer, solvate, prodrug, isotopic derivative, or tautomer thereof.

In some embodiments the compound is of Formula (I-B-I-d-I-2-A-H-1-h): 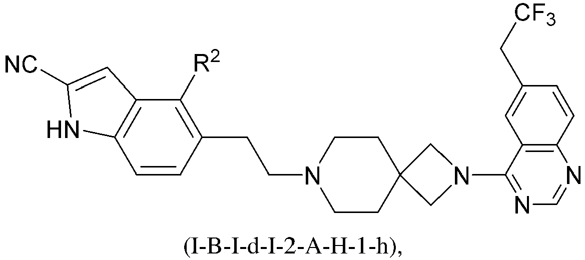 or a pharmaceutically acceptable salt, stereoisomer, solvate, prodrug, isotopic derivative, or tautomer thereof.

In some embodiments the compound is of Formula (I-B-I-d-I-2-A-H-1-B): 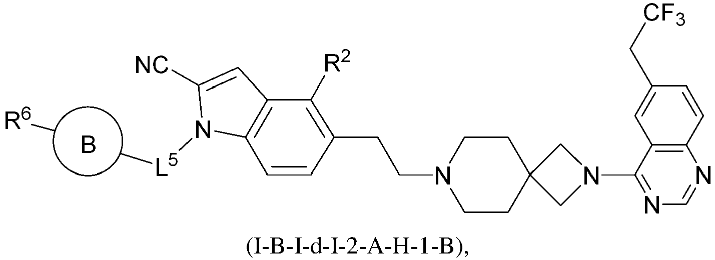 or a pharmaceutically acceptable salt, stereoisomer, solvate, prodrug, isotopic derivative, or tautomer thereof.

In some embodiments the compound is of Formula (I-B-I-d-I-2-A-H-1-B-I): 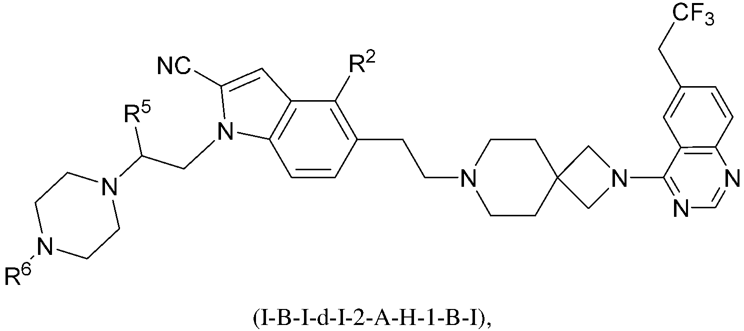 or a pharmaceutically acceptable salt, stereoisomer, solvate, prodrug, isotopic derivative, or tautomer thereof.

In some embodiments the compound is of Formula (I-B-I-d-I-2-A-H-1-B-I-a): 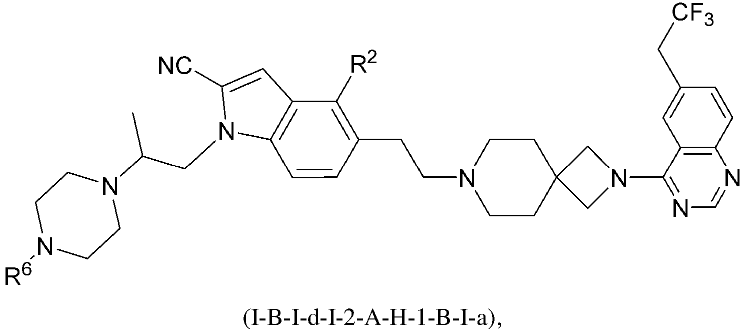 or a pharmaceutically acceptable salt, stereoisomer, solvate, prodrug, isotopic derivative, or tautomer thereof.

In some embodiments the compound is of Formula (I-B-I-d-I-2-A-H-1-B-I-a*): 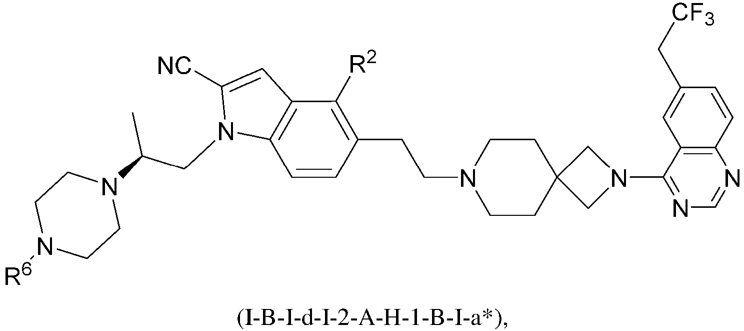 or a pharmaceutically acceptable salt, stereoisomer, solvate, prodrug, isotopic derivative, or tautomer thereof.

In some embodiments the compound is of Formula (I-B-I-d-I-2-A-H-1-B-I-a**): 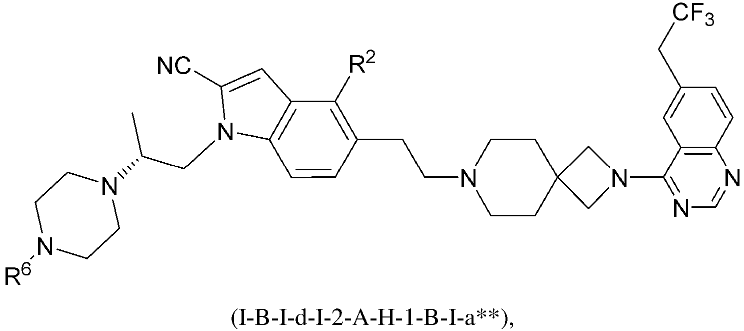 or a pharmaceutically acceptable salt, stereoisomer, solvate, prodrug, isotopic derivative, or tautomer thereof.

In some embodiments the compound is of Formula (I-B-II-d-I): 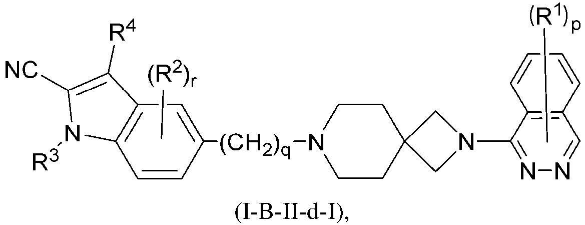 or a pharmaceutically acceptable salt, stereoisomer, solvate, prodrug, isotopic derivative, or tautomer thereof.

In some embodiments the compound is of Formula (I-B-II-d-I-1): 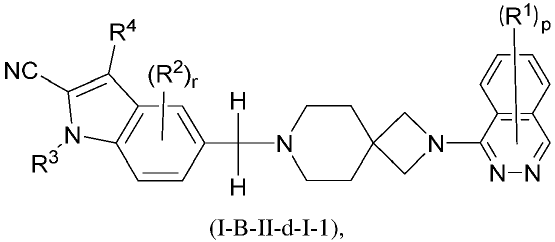 or a pharmaceutically acceptable salt, stereoisomer, solvate, prodrug, isotopic derivative, or tautomer thereof.

In some embodiments the compound is of Formula (I-B-II-d-I-1-A): 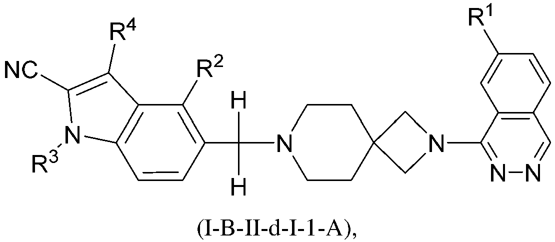 or a pharmaceutically acceptable salt, stereoisomer, solvate, prodrug, isotopic derivative, or tautomer thereof.

In some embodiments the compound is of Formula (I-B-II-d-I-1-A-H): 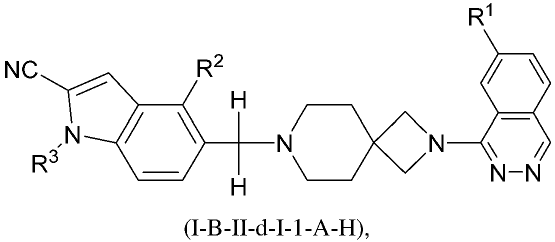 or a pharmaceutically acceptable salt, stereoisomer, solvate, prodrug, isotopic derivative, or tautomer thereof.

In some embodiments the compound is of Formula (I-B-II-d-I-1-A-H-1): 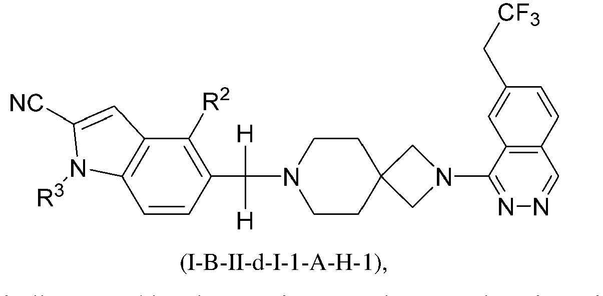 or a pharmaceutically acceptable salt, stereoisomer, solvate, prodrug, isotopic derivative, or tautomer thereof.

In some embodiments the compound is of Formula (I-B-II-d-I-1-A-H-1-B): 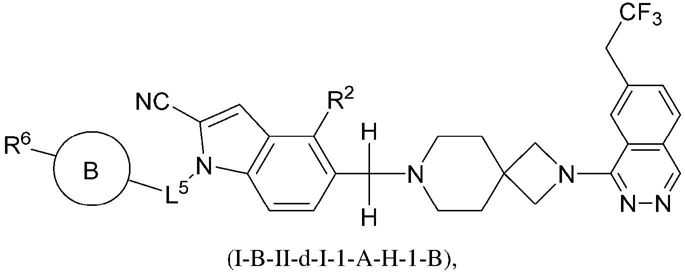 or a pharmaceutically acceptable salt, stereoisomer, solvate, prodrug, isotopic derivative, or tautomer thereof.

In some embodiments the compound is of Formula (I-B-II-d-I-1-A-H-1-B-I): 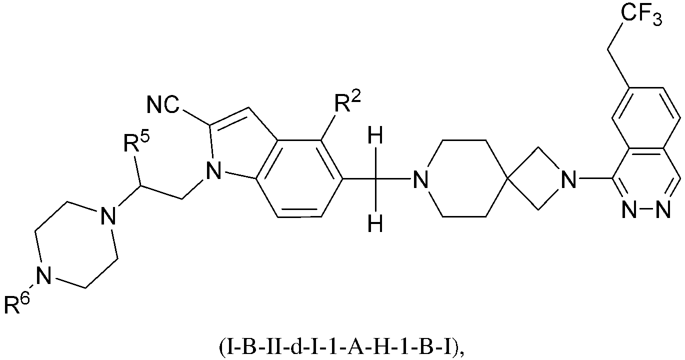 or a pharmaceutically acceptable salt, stereoisomer, solvate, prodrug, isotopic derivative, or tautomer thereof.

In some embodiments the compound is of Formula (I-B-II-d-I-1-A-H-1-B-I-a): 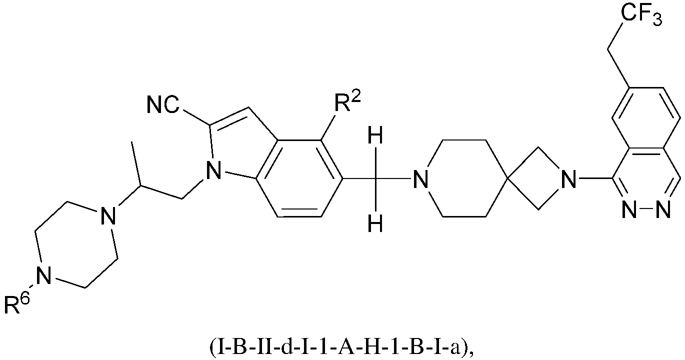 or a pharmaceutically acceptable salt, stereoisomer, solvate, prodrug, isotopic derivative, or tautomer thereof.

In some embodiments the compound is of Formula (I-B-II-d-I-1-A-H-1-B-I-a*): 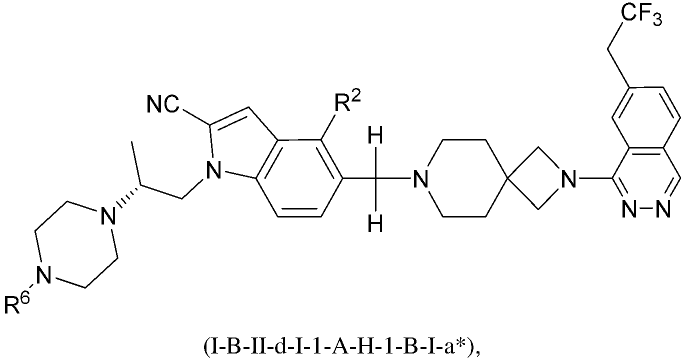 or a pharmaceutically acceptable salt, stereoisomer, solvate, prodrug, isotopic derivative, or tautomer thereof.

In some embodiments the compound is of Formula (I-B-II-d-I-1-A-H-1-B-I-a**): 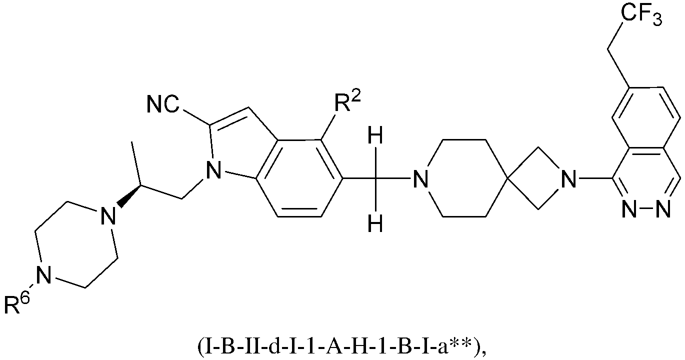 or a pharmaceutically acceptable salt, stereoisomer, solvate, prodrug, isotopic derivative, or tautomer thereof.

In some embodiments the compound is of Formula (I-B-III-d-I): 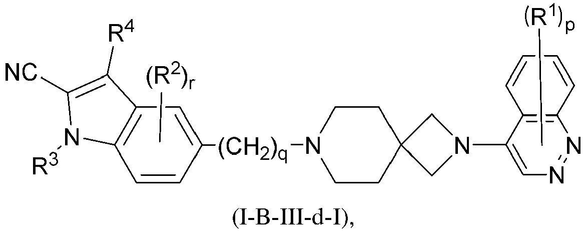 or a pharmaceutically acceptable salt, stereoisomer, solvate, prodrug, isotopic derivative, or tautomer thereof.

In some embodiments the compound is of Formula (I-B-III-d-I-1): 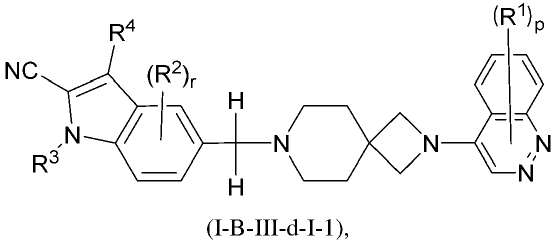 or a pharmaceutically acceptable salt, stereoisomer, solvate, prodrug, isotopic derivative, or tautomer thereof.

In some embodiments the compound is of Formula (I-B-III-d-I-1-A): 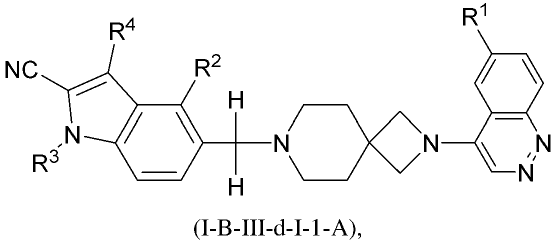 or a pharmaceutically acceptable salt, stereoisomer, solvate, prodrug, isotopic derivative, or tautomer thereof.

In some embodiments the compound is of Formula (I-B-III-d-I-1-A-H): 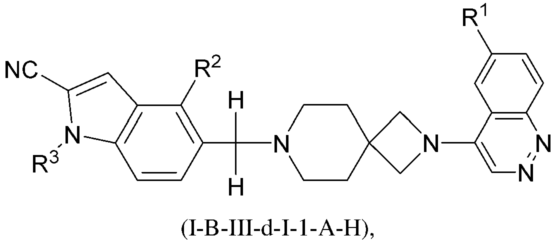 or a pharmaceutically acceptable salt, stereoisomer, solvate, prodrug, isotopic derivative, or tautomer thereof.

In some embodiments the compound is of Formula (I-B-III-d-I-1-A-H-1): 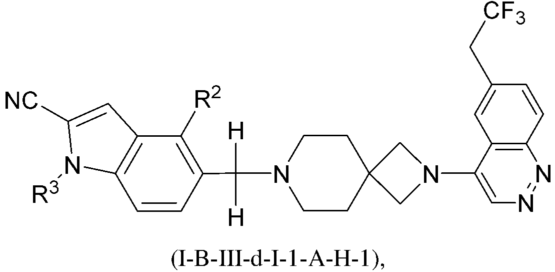 or a pharmaceutically acceptable salt, stereoisomer, solvate, prodrug, isotopic derivative, or tautomer thereof.

In some embodiments the compound is of Formula (I-B-III-d-I-1-A-H-1-B): 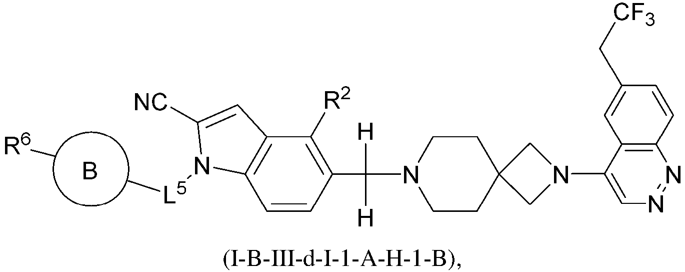 or a pharmaceutically acceptable salt, stereoisomer, solvate, prodrug, isotopic derivative, or tautomer thereof.

In some embodiments the compound is of Formula (I-B-III-d-I-1-A-H-1-B-I): 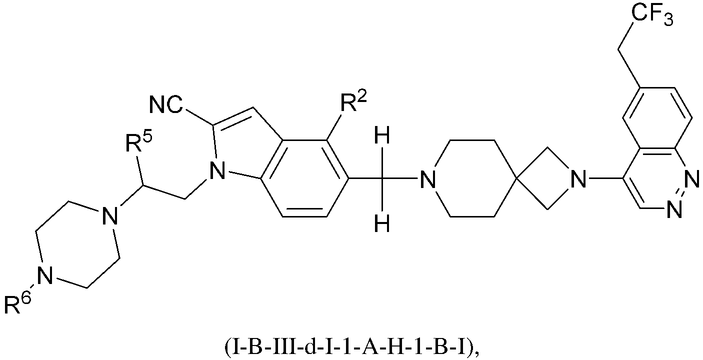 or a pharmaceutically acceptable salt, stereoisomer, solvate, prodrug, isotopic derivative, or tautomer thereof.

In some embodiments the compound is of Formula (I-B-III-d-I-1-A-H-1-B-I-a): 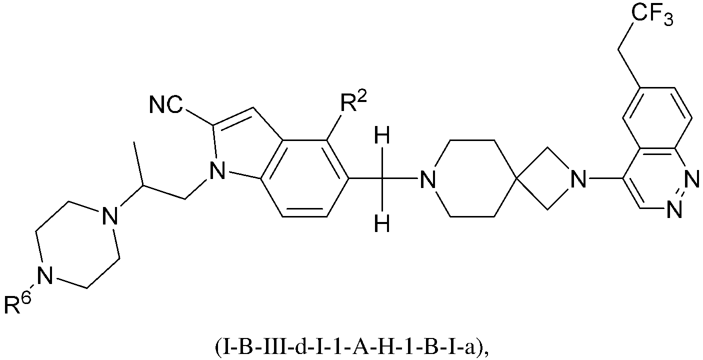 or a pharmaceutically acceptable salt, stereoisomer, solvate, prodrug, isotopic derivative, or tautomer thereof.

In some embodiments the compound is of Formula (I-B-III-d-I-1-A-H-1-B-I-a*): 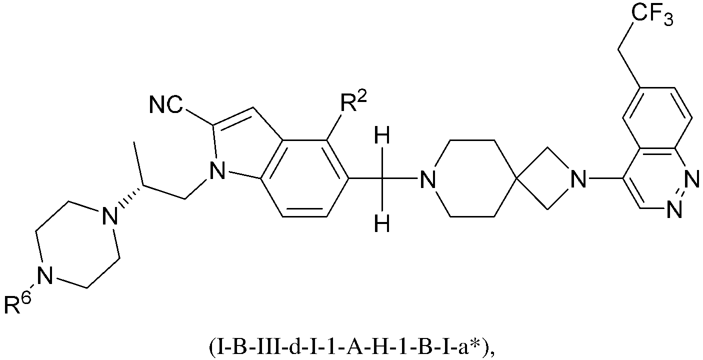 or a pharmaceutically acceptable salt, stereoisomer, solvate, prodrug, isotopic derivative, or tautomer thereof.

In some embodiments the compound is of Formula (I-B-III-d-I-1-A-H-1-B-I-a**): 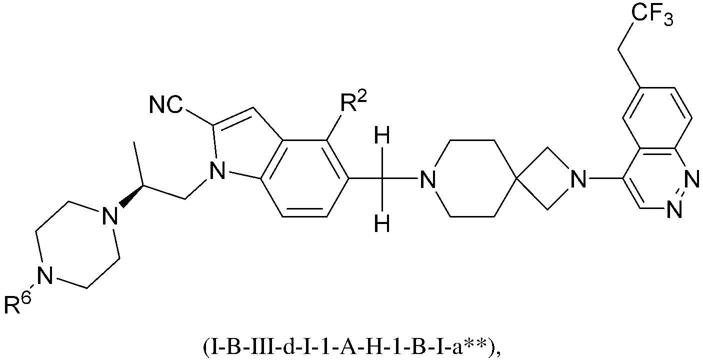 or a pharmaceutically acceptable salt, stereoisomer, solvate, prodrug, isotopic derivative, or tautomer thereof.

In some embodiments the compound is of Formula (I-B-IV-d-I): 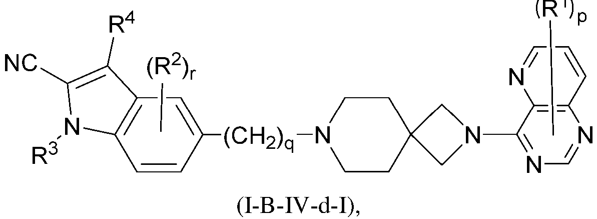 or a pharmaceutically acceptable salt, stereoisomer, solvate, prodrug, isotopic derivative, or tautomer thereof.

In some embodiments the compound is of Formula (I-B-IV-d-I-1): 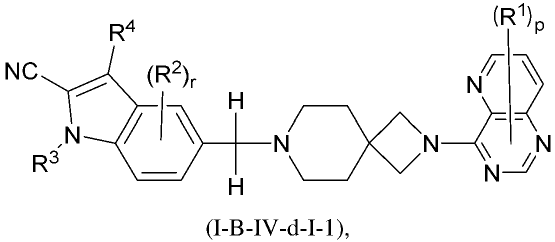 or a pharmaceutically acceptable salt, stereoisomer, solvate, prodrug, isotopic derivative, or tautomer thereof.

In some embodiments the compound is of Formula (I-B-IV-d-I-1-A): 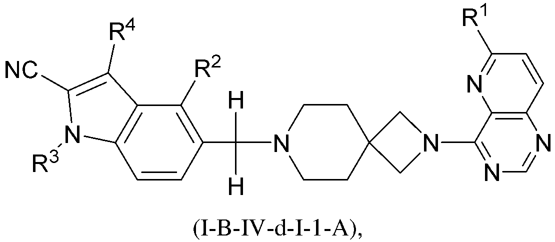 or a pharmaceutically acceptable salt, stereoisomer, solvate, prodrug, isotopic derivative, or tautomer thereof.

In some embodiments the compound is of Formula (I-B-IV-d-I-1-A-H): 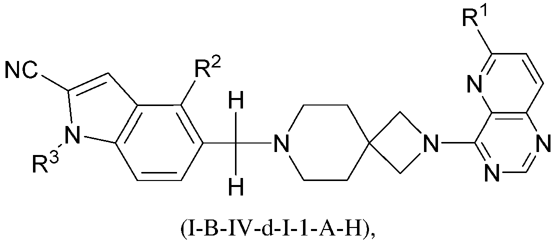 or a pharmaceutically acceptable salt, stereoisomer, solvate, prodrug, isotopic derivative, or tautomer thereof.

In some embodiments the compound is of Formula (I-B-IV-d-I-1-A-H-1): 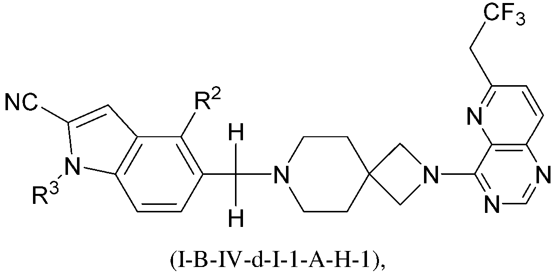 or a pharmaceutically acceptable salt, stereoisomer, solvate, prodrug, isotopic derivative, or tautomer thereof.

In some embodiments the compound is of Formula (I-B-IV-d-I-1-A-H-1-B): 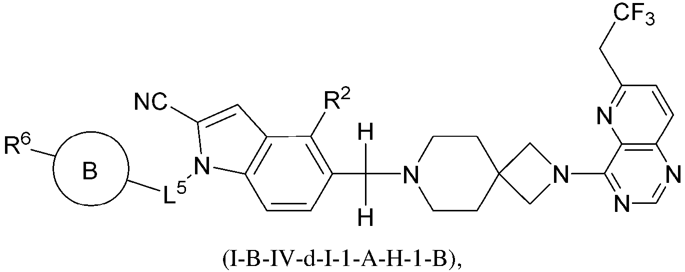 or a pharmaceutically acceptable salt, stereoisomer, solvate, prodrug, isotopic derivative, or tautomer thereof.

In some embodiments the compound is of Formula (I-B-IV-d-I-1-A-H-1-B-I): 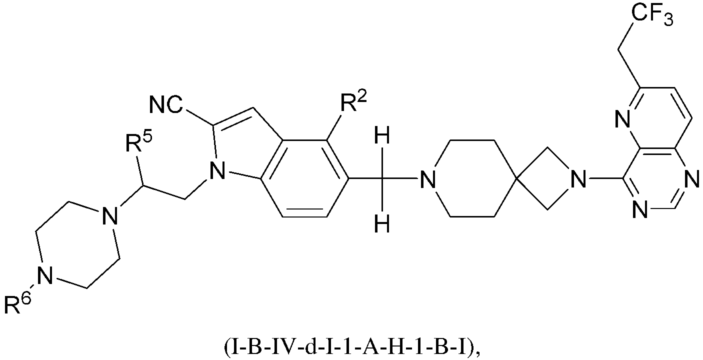 or a pharmaceutically acceptable salt, stereoisomer, solvate, prodrug, isotopic derivative, or tautomer thereof.

In some embodiments the compound is of Formula (I-B-IV-d-I-1-A-H-1-B-I-a): 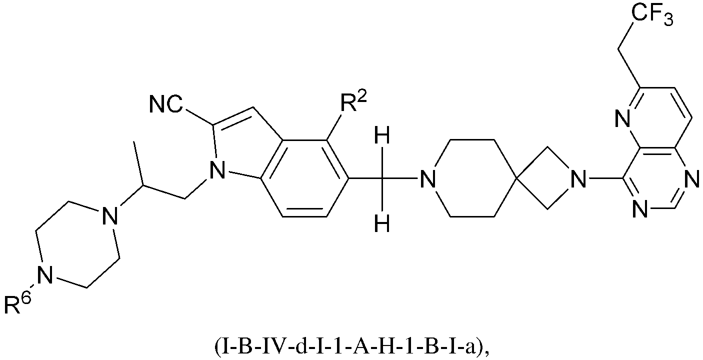 or a pharmaceutically acceptable salt, stereoisomer, solvate, prodrug, isotopic derivative, or tautomer thereof.

In some embodiments the compound is of Formula (I-B-IV-d-I-1-A-H-1-B-I-a*): 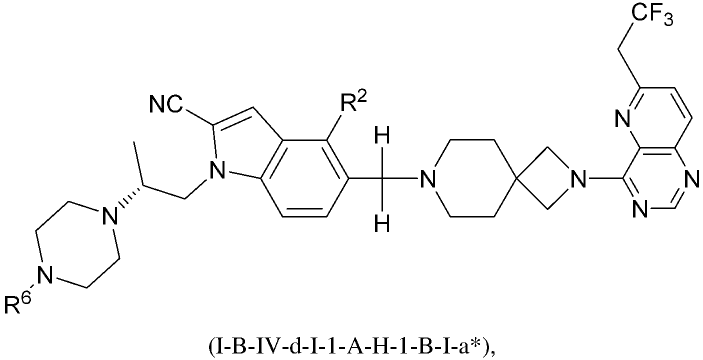 or a pharmaceutically acceptable salt, stereoisomer, solvate, prodrug, isotopic derivative, or tautomer thereof.

In some embodiments the compound is of Formula (I-B-IV-d-I-1-A-H-1-B-I-a**): 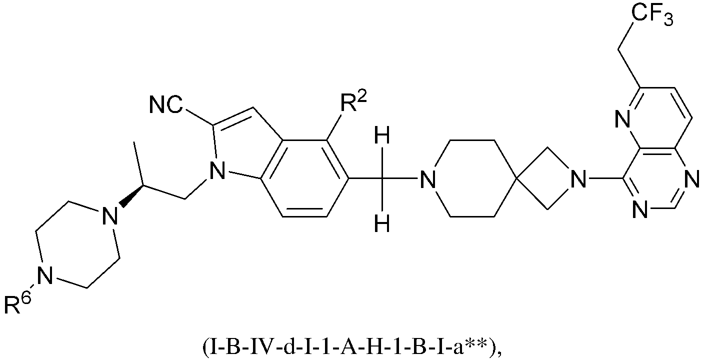 or a pharmaceutically acceptable salt, stereoisomer, solvate, prodrug, isotopic derivative, or tautomer thereof.

In some embodiments the compound is of Formula (I-B-V-d-I): 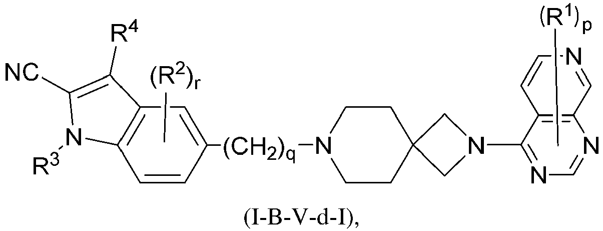 or a pharmaceutically acceptable salt, stereoisomer, solvate, prodrug, isotopic derivative, or tautomer thereof.

In some embodiments the compound is of Formula (I-B-V-d-I-1): 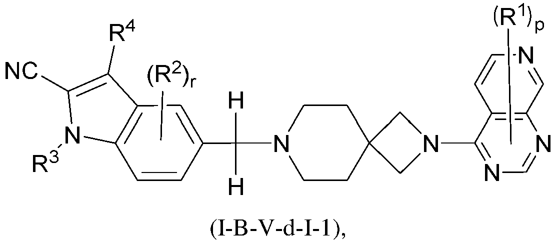 or a pharmaceutically acceptable salt, stereoisomer, solvate, prodrug, isotopic derivative, or tautomer thereof.

In some embodiments the compound is of Formula (I-B-V-d-I-1-A): 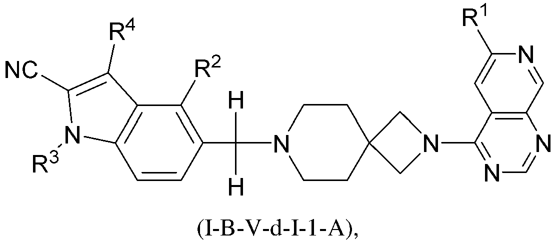 or a pharmaceutically acceptable salt, stereoisomer, solvate, prodrug, isotopic derivative, or tautomer thereof.

In some embodiments the compound is of Formula (I-B-V-d-I-1-A-H): 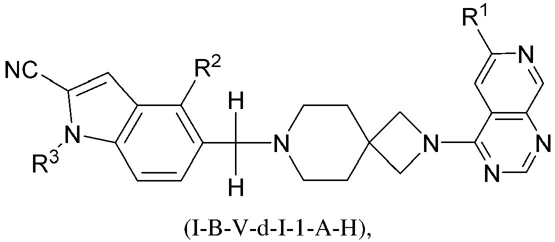 or a pharmaceutically acceptable salt, stereoisomer, solvate, prodrug, isotopic derivative, or tautomer thereof.

In some embodiments the compound is of Formula (I-B-V-d-I-1-A-H-1): 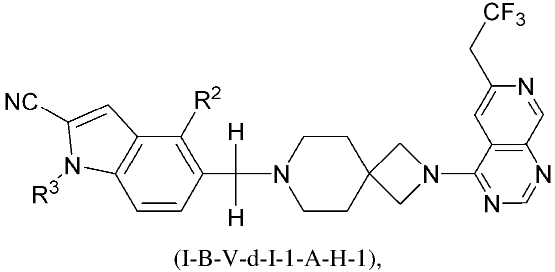 or a pharmaceutically acceptable salt, stereoisomer, solvate, prodrug, isotopic derivative, or tautomer thereof.

In some embodiments the compound is of Formula (I-B-V-d-I-1-A-H-1-B): 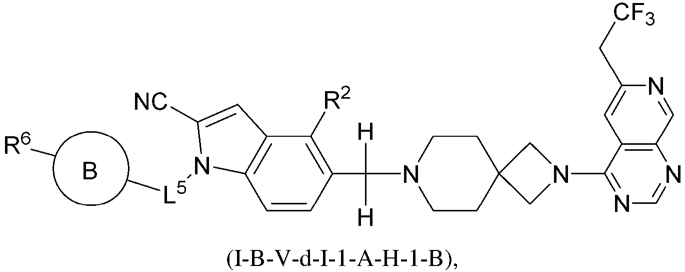 or a pharmaceutically acceptable salt, stereoisomer, solvate, prodrug, isotopic derivative, or tautomer thereof.

In some embodiments the compound is of Formula (I-B-V-d-I-1-A-H-1-B-I): 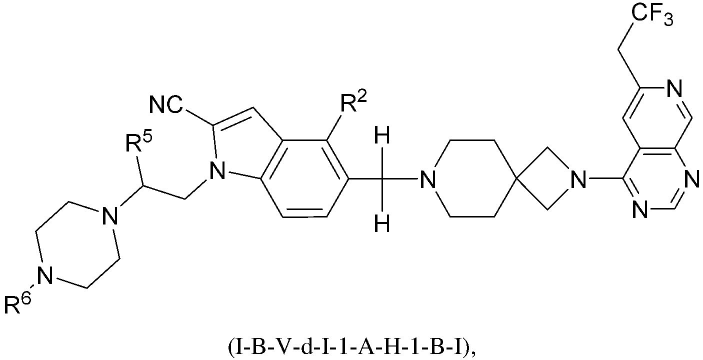 or a pharmaceutically acceptable salt, stereoisomer, solvate, prodrug, isotopic derivative, or tautomer thereof.

In some embodiments the compound is of Formula (I-B-V-d-I-1-A-H-1-B-I-a): 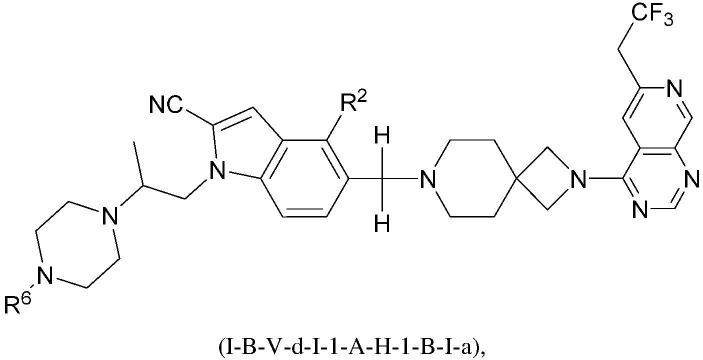 or a pharmaceutically acceptable salt, stereoisomer, solvate, prodrug, isotopic derivative, or tautomer thereof.

In some embodiments the compound is of Formula (I-B-V-d-I-1-A-H-1-B-I-a*): 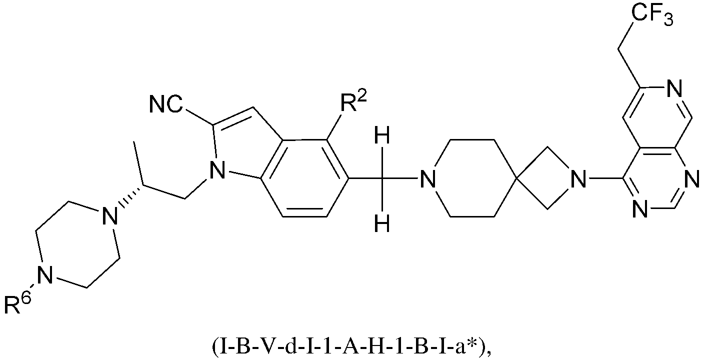 or a pharmaceutically acceptable salt, stereoisomer, solvate, prodrug, isotopic derivative, or tautomer thereof.

In some embodiments the compound is of Formula (I-B-V-d-I-1-A-H-1-B-I-a**): 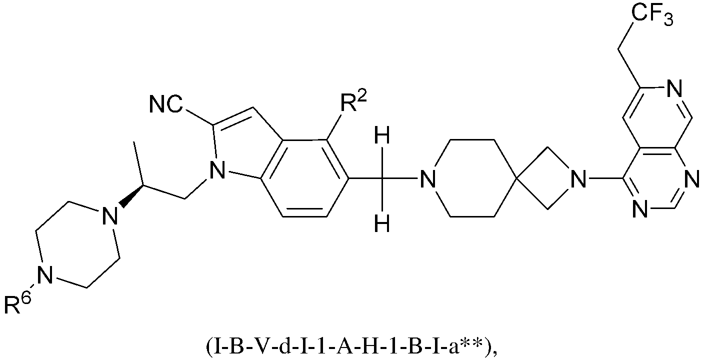 or a pharmaceutically acceptable salt, stereoisomer, solvate, prodrug, isotopic derivative, or tautomer thereof.

In some embodiments the compound is of Formula (I-B-VI-d-I): 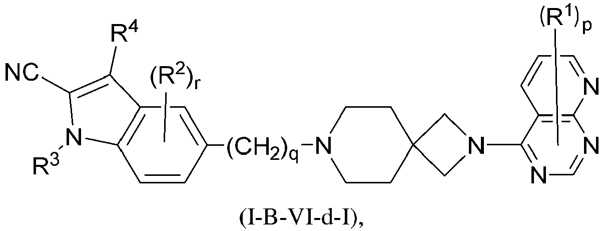 or a pharmaceutically acceptable salt, stereoisomer, solvate, prodrug, isotopic derivative, or tautomer thereof.

In some embodiments the compound is of Formula (I-B-VI-d-I-1): 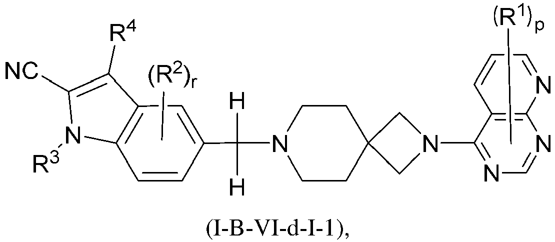 or a pharmaceutically acceptable salt, stereoisomer, solvate, prodrug, isotopic derivative, or tautomer thereof.

In some embodiments the compound is of Formula (I-B-VI-d-I-1-A): 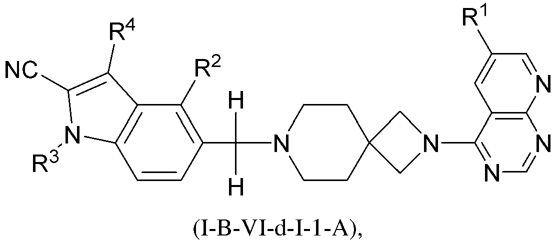 or a pharmaceutically acceptable salt, stereoisomer, solvate, prodrug, isotopic derivative, or tautomer thereof.

In some embodiments the compound is of Formula (I-B-VI-d-I-1-A-H): 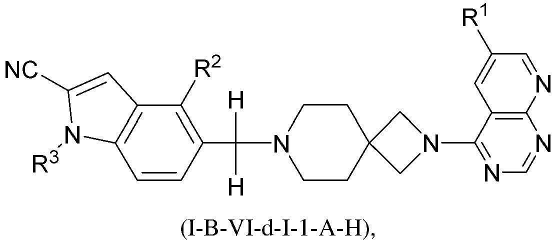 or a pharmaceutically acceptable salt, stereoisomer, solvate, prodrug, isotopic derivative, or tautomer thereof.

In some embodiments the compound is of Formula (I-B-VI-d-I-1-A-H-1): 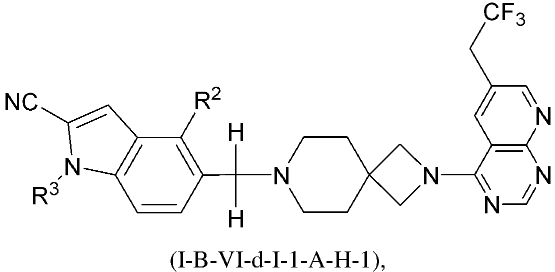 or a pharmaceutically acceptable salt, stereoisomer, solvate, prodrug, isotopic derivative, or tautomer thereof.

In some embodiments the compound is of Formula (I-B-VI-d-I-1-A-H-1-B): 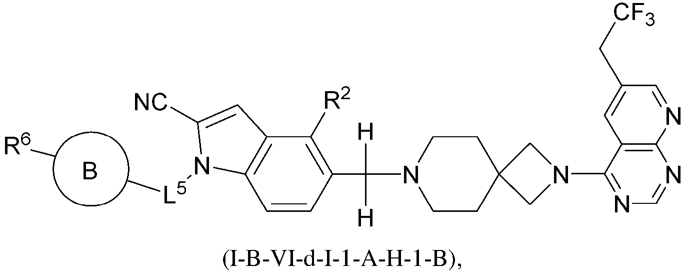 or a pharmaceutically acceptable salt, stereoisomer, solvate, prodrug, isotopic derivative, or tautomer thereof.

In some embodiments the compound is of Formula (I-B-VI-d-I-1-A-H-1-B-I): 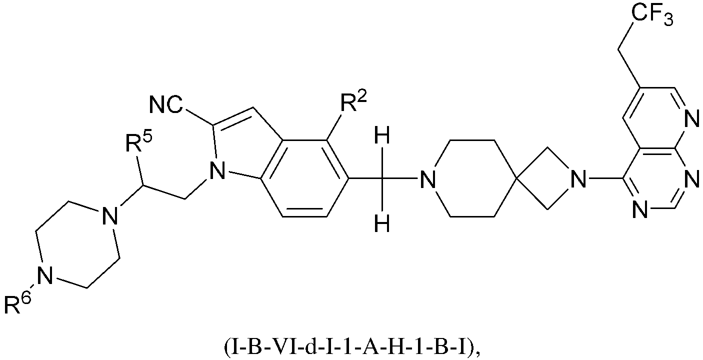 or a pharmaceutically acceptable salt, stereoisomer, solvate, prodrug, isotopic derivative, or tautomer thereof.

In some embodiments the compound is of Formula (I-B-VI-d-I-1-A-H-1-B-I-a): 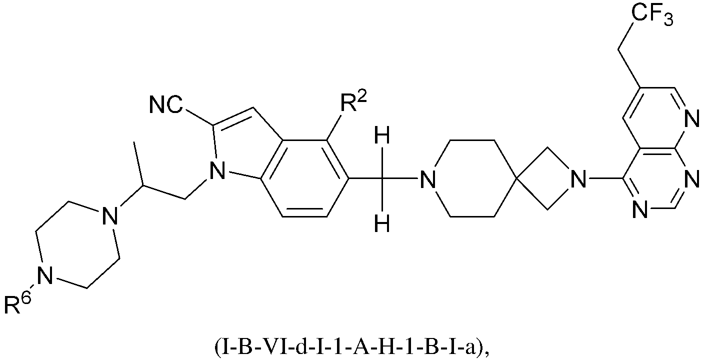 or a pharmaceutically acceptable salt, stereoisomer, solvate, prodrug, isotopic derivative, or tautomer thereof.

In some embodiments the compound is of Formula (I-B-VI-d-I-1-A-H-1-B-I-a*): 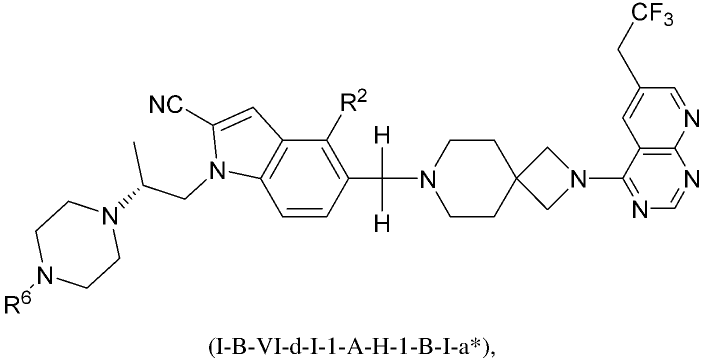 or a pharmaceutically acceptable salt, stereoisomer, solvate, prodrug, isotopic derivative, or tautomer thereof.

In some embodiments the compound is of Formula (I-B-VI-d-I-1-A-H-1-B-I-a**): 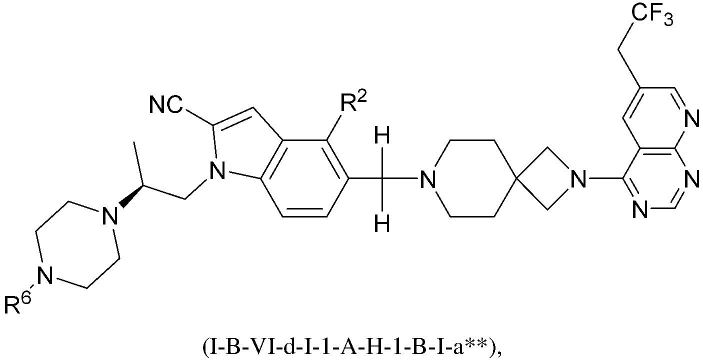 or a pharmaceutically acceptable salt, stereoisomer, solvate, prodrug, isotopic derivative, or tautomer thereof.

In some embodiments the compound is of Formula (I-B-VII-d-I): 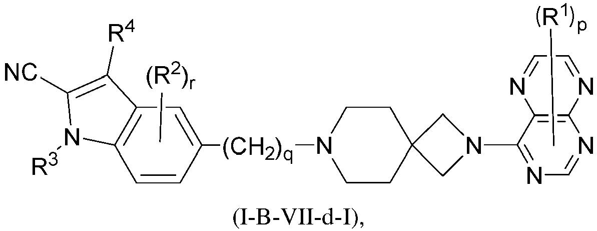 or a pharmaceutically acceptable salt, stereoisomer, solvate, prodrug, isotopic derivative, or tautomer thereof.

In some embodiments the compound is of Formula (I-B-VII-d-I-1): 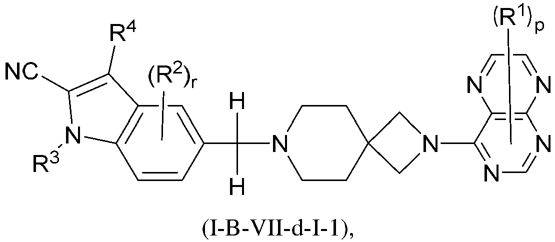 or a pharmaceutically acceptable salt, stereoisomer, solvate, prodrug, isotopic derivative, or tautomer thereof.

In some embodiments the compound is of Formula (I-B-VII-d-I-1-A): 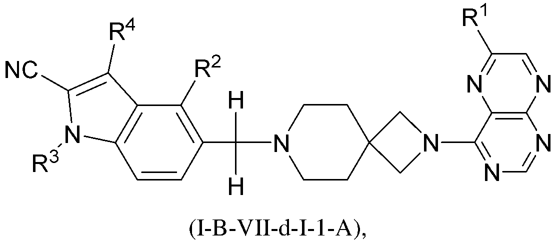 or a pharmaceutically acceptable salt, stereoisomer, solvate, prodrug, isotopic derivative, or tautomer thereof.

In some embodiments the compound is of Formula (I-B-VII-d-I-1-A-H): 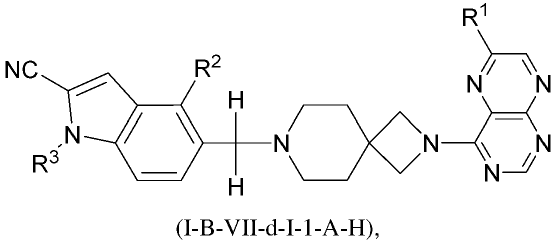 or a pharmaceutically acceptable salt, stereoisomer, solvate, prodrug, isotopic derivative, or tautomer thereof.

In some embodiments the compound is of Formula (I-B-VII-d-I-1-A-H-1): 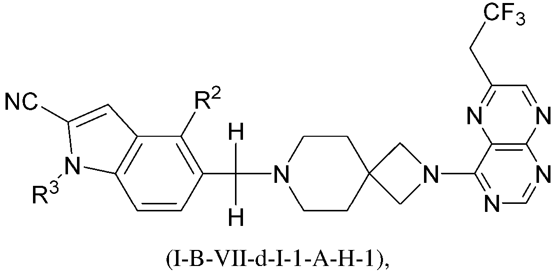 or a pharmaceutically acceptable salt, stereoisomer, solvate, prodrug, isotopic derivative, or tautomer thereof.

In some embodiments the compound is of Formula (I-B-VII-d-I-1-A-H-1-B): 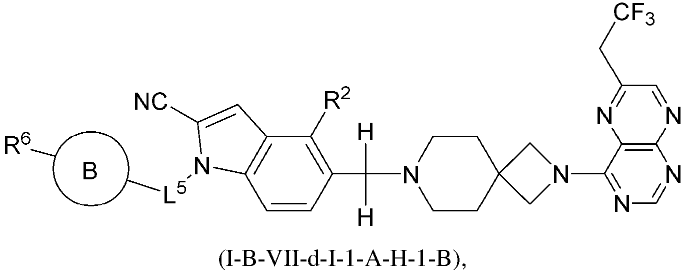 or a pharmaceutically acceptable salt, stereoisomer, solvate, prodrug, isotopic derivative, or tautomer thereof.

In some embodiments the compound is of Formula (I-B-VII-d-I-1-A-H-1-B-I): 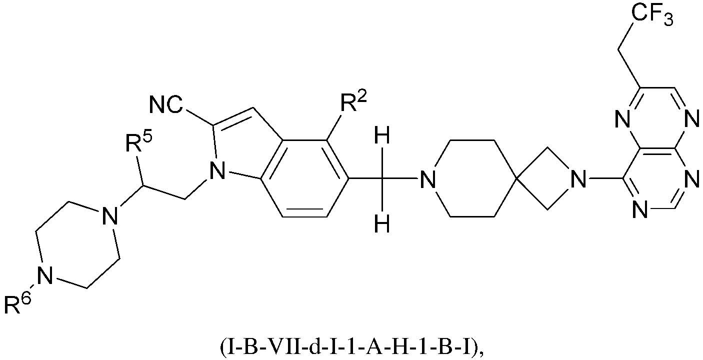 or a pharmaceutically acceptable salt, stereoisomer, solvate, prodrug, isotopic derivative, or tautomer thereof.

In some embodiments the compound is of Formula (I-B-VII-d-I-1-A-H-1-B-I-a): 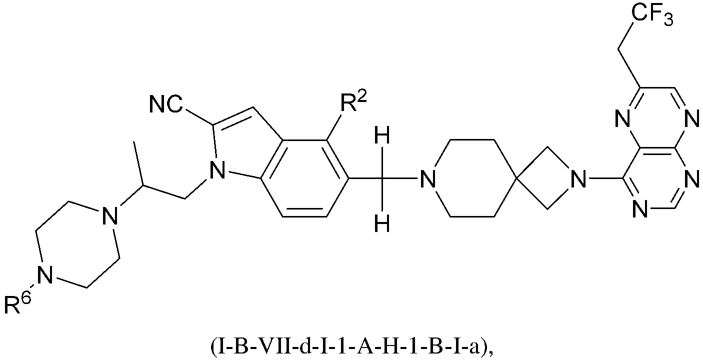 or a pharmaceutically acceptable salt, stereoisomer, solvate, prodrug, isotopic derivative, or tautomer thereof.

In some embodiments the compound is of Formula (I-B-VII-d-I-1-A-H-1-B-I-a*): 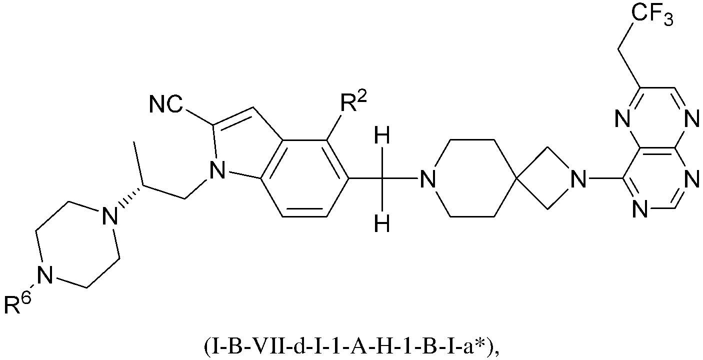 or a pharmaceutically acceptable salt, stereoisomer, solvate, prodrug, isotopic derivative, or tautomer thereof.

In some embodiments the compound is of Formula (I-B-VII-d-I-1-A-H-1-B-I-a**): 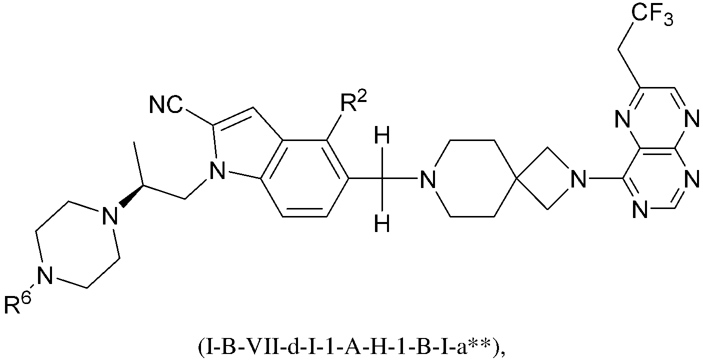 or a pharmaceutically acceptable salt, stereoisomer, solvate, prodrug, isotopic derivative, or tautomer thereof.

In some embodiments the compound is of Formula (I-C-I-d-I): 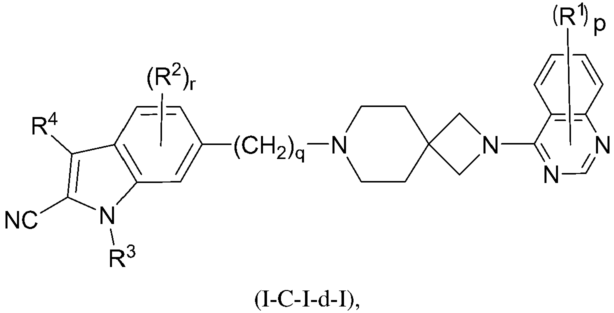 or a pharmaceutically acceptable salt, stereoisomer, solvate, prodrug, isotopic derivative, or tautomer thereof.

In some embodiments the compound is of Formula (I-C-1-d-1-1): 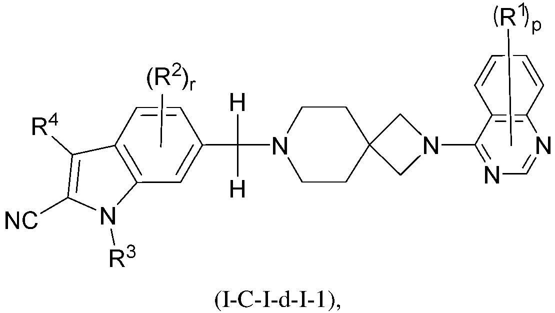 or a pharmaceutically acceptable salt, stereoisomer, solvate, prodrug, isotopic derivative, or tautomer thereof.

In some embodiments the compound is of Formula (I-C-I-d-I-1-A): 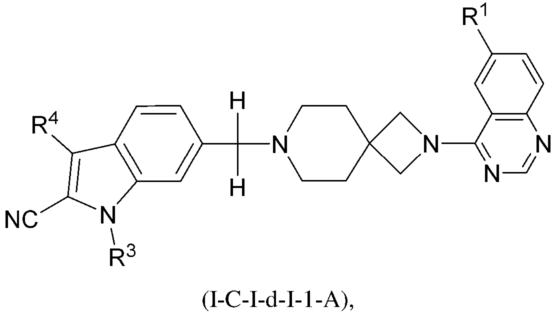 or a pharmaceutically acceptable salt, stereoisomer, solvate, prodrug, isotopic derivative, or tautomer thereof.

In some embodiments the compound is of Formula (I-C-I-d-I-1-A-H): 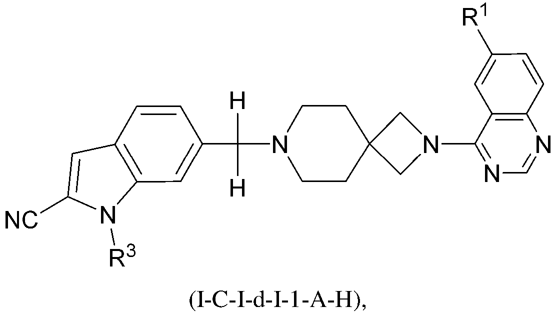 or a pharmaceutically acceptable salt, stereoisomer, solvate, prodrug, isotopic derivative, or tautomer thereof.

In some embodiments the compound is of Formula (I-C-I-d-I-1-A-H-1): 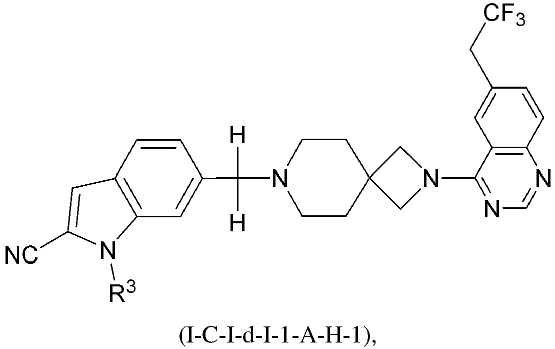 or a pharmaceutically acceptable salt, stereoisomer, solvate, prodrug, isotopic derivative, or tautomer thereof.

In some embodiments the compound is of Formula (I-C-I-d-I-1-B): 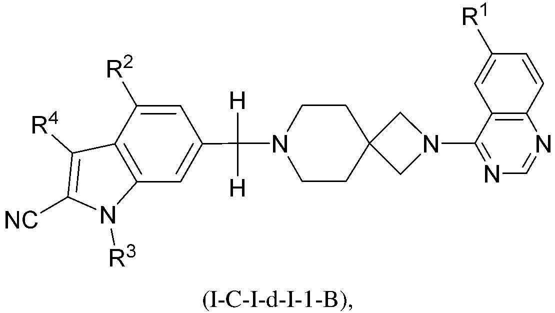 or a pharmaceutically acceptable salt, stereoisomer, solvate, prodrug, isotopic derivative, or tautomer thereof.

In some embodiments the compound is of Formula (I-C-I-d-I-1-B-H): 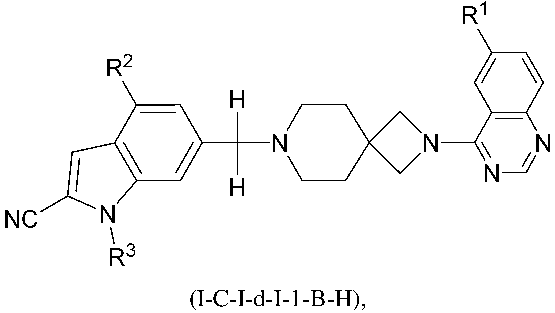 or a pharmaceutically acceptable salt, stereoisomer, solvate, prodrug, isotopic derivative, or tautomer thereof.

In some embodiments the compound is of Formula (I-C-I-d-I-1-B-H-1): 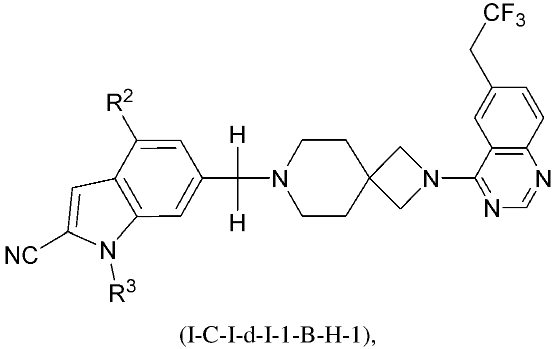 or a pharmaceutically acceptable salt, stereoisomer, solvate, prodrug, isotopic derivative, or tautomer thereof.

In some embodiments the compound is of Formula (I-C-I-d-I-1-B-H-1-B): 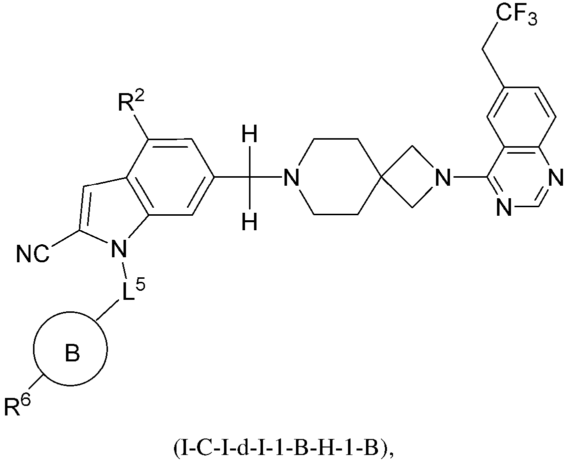 or a pharmaceutically acceptable salt, stereoisomer, solvate, prodrug, isotopic derivative, or tautomer thereof.

In some embodiments the compound is of Formula (I-C-I-d-I-1-B-H-1-B-I): 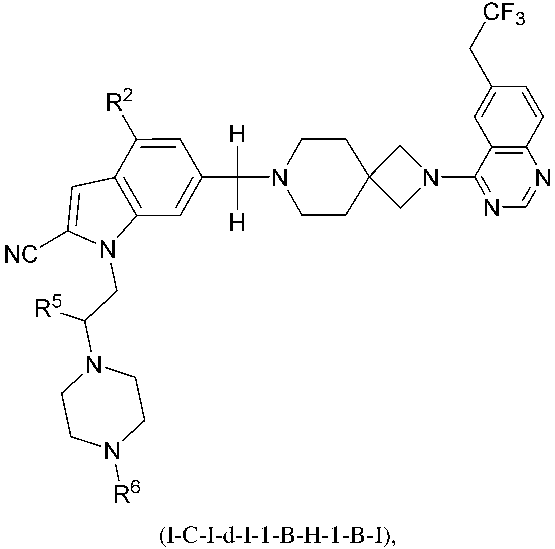 or a pharmaceutically acceptable salt, stereoisomer, solvate, prodrug, isotopic derivative, or tautomer thereof.

In some embodiments the compound is of Formula (I-C-I-d-I-1-B-H-1-B-I-a): 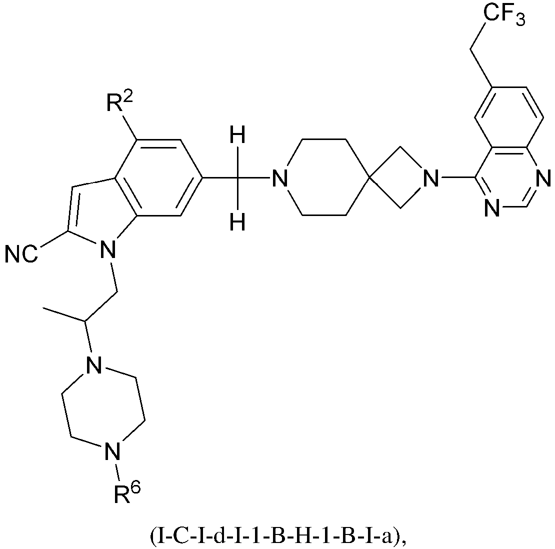 or a pharmaceutically acceptable salt, stereoisomer, solvate, prodrug, isotopic derivative, or tautomer thereof.

In some embodiments the compound is of Formula (I-C-I-d-I-1-B-H-1-B-I-a*): 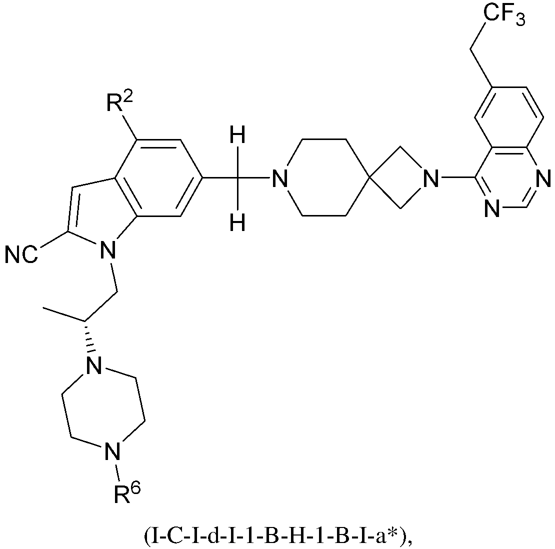 or a pharmaceutically acceptable salt, stereoisomer, solvate, prodrug, isotopic derivative, or tautomer thereof.

In some embodiments the compound is of Formula (I-C-I-d-I-1-B-H-1-B-I-a**): 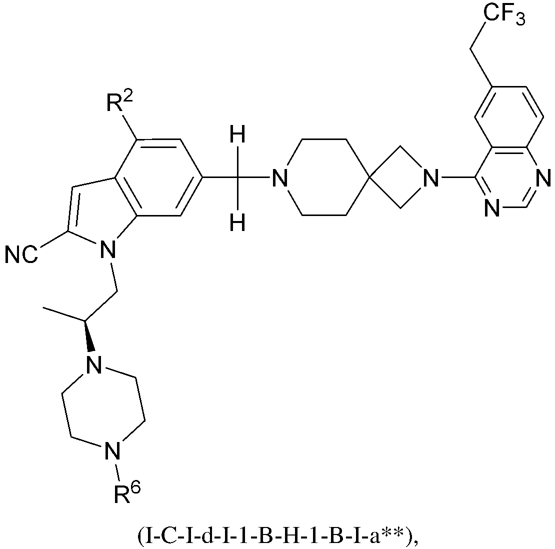 or a pharmaceutically acceptable salt, stereoisomer, solvate, prodrug, isotopic derivative, or tautomer thereof.

In some embodiments the compound is of Formula (II): 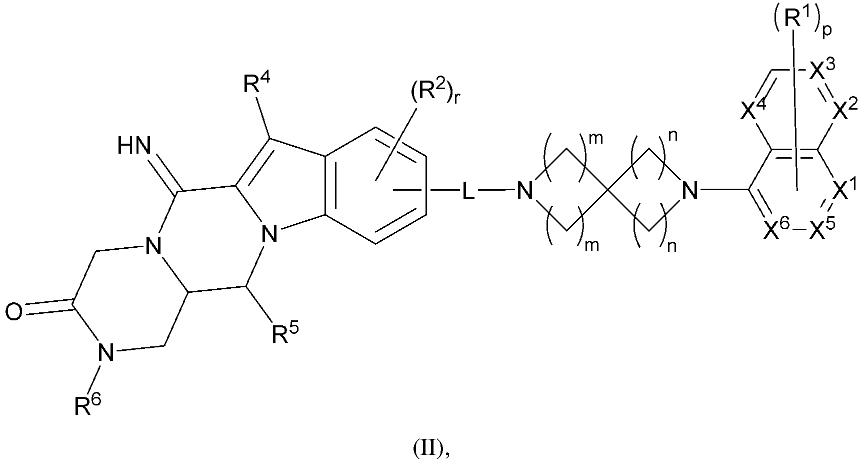 or a pharmaceutically acceptable salt, stereoisomer, solvate, prodrug, isotopic derivative, or tautomer thereof.

In some embodiments the compound is of Formula (I-B-I-d-I-II): 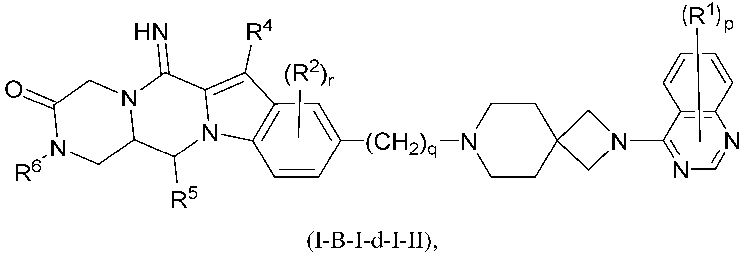 or a pharmaceutically acceptable salt, stereoisomer, solvate, prodrug, isotopic derivative, or tautomer thereof.

In some embodiments the compound is of Formula (I-B-I-d-I-II-1): 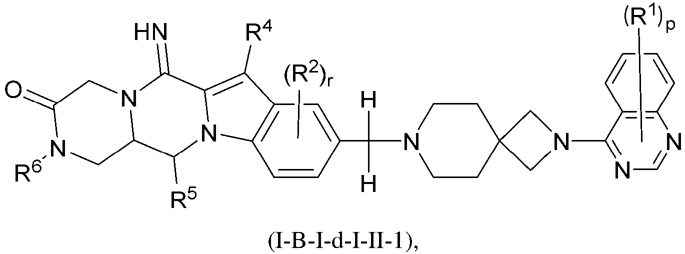 or a pharmaceutically acceptable salt, stereoisomer, solvate, prodrug, isotopic derivative, or tautomer thereof.

In some embodiments the compound is of Formula (I-B-I-d-I-II-1-A): 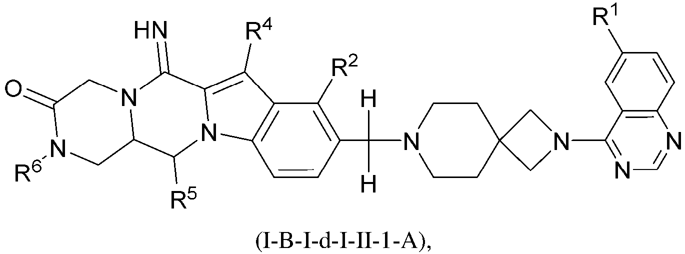 or a pharmaceutically acceptable salt, stereoisomer, solvate, prodrug, isotopic derivative, or tautomer thereof.

In some embodiments the compound is of Formula (I-B-I-d-I-II-1-A-H): 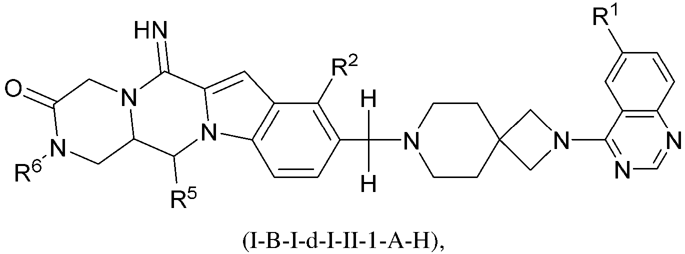 or a pharmaceutically acceptable salt, stereoisomer, solvate, prodrug, isotopic derivative, or tautomer thereof.

In some embodiments the compound is of Formula (I-B-I-d-I-II-1-A-H-1): 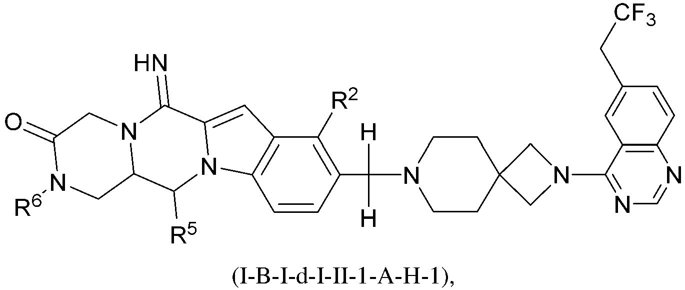 or a pharmaceutically acceptable salt, stereoisomer, solvate, prodrug, isotopic derivative, or tautomer thereof.

In some embodiments the compound is of Formula (I-a): 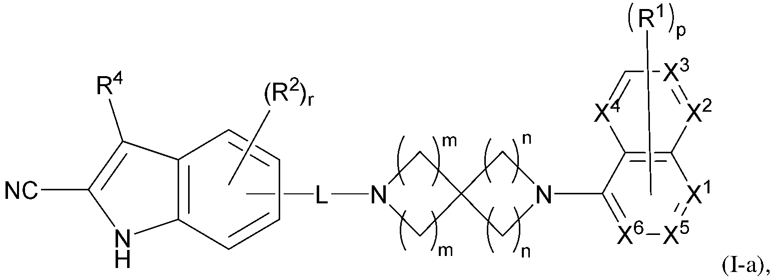 or a pharmaceutically acceptable salt, stereoisomer, solvate, prodrug, isotopic derivative, or tautomer thereof.

In some embodiments the compound is of Formula I': 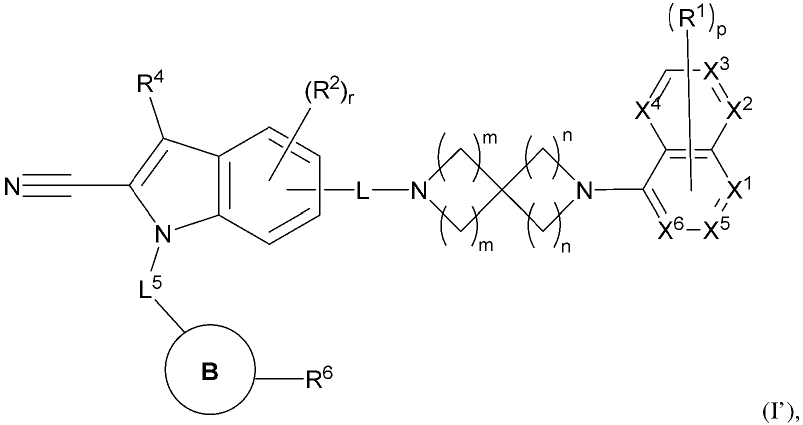 or a pharmaceutically acceptable salt, stereoisomer, solvate, prodrug, isotopic derivative, or tautomer thereof.

In some embodiments the compound is of Formula (I-b): 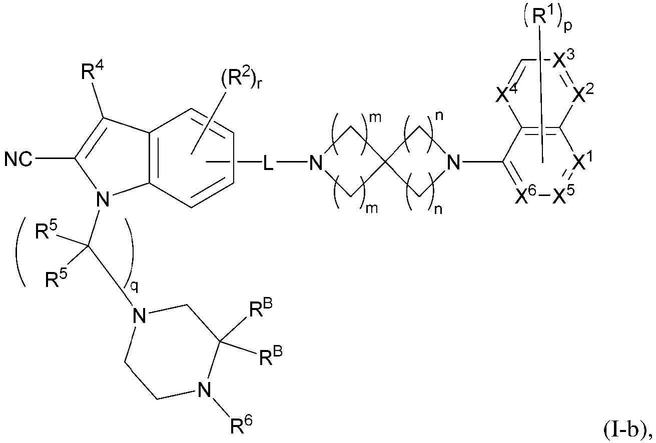 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-c): 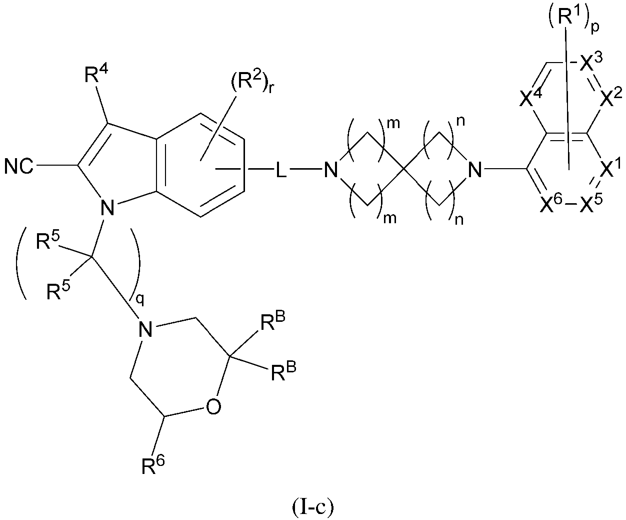 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-d): 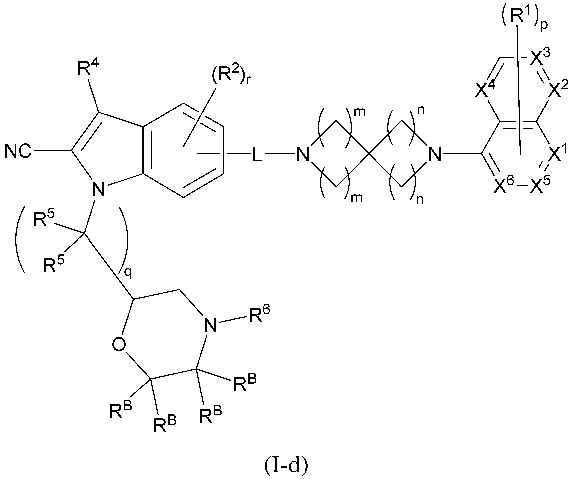 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-e): 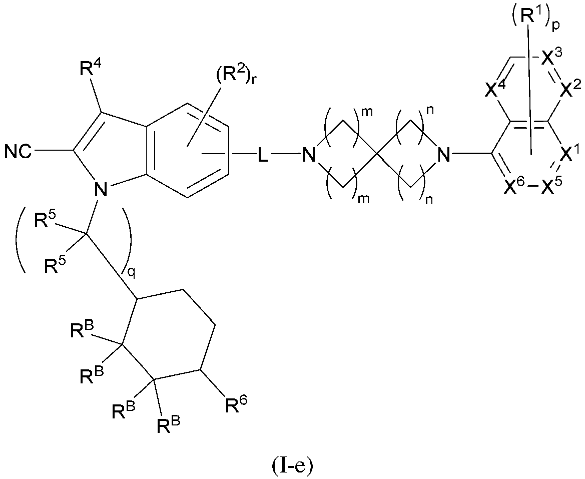 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-f): 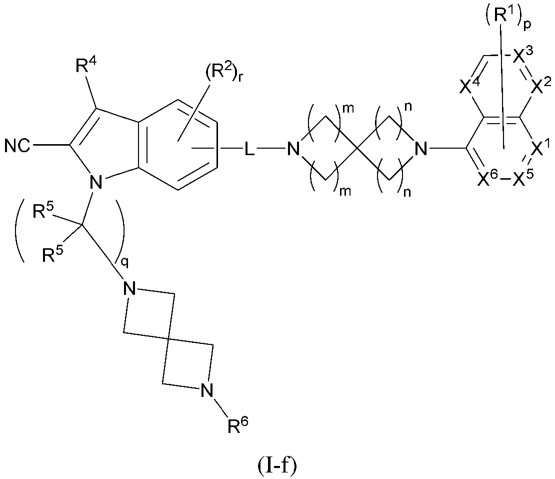 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-g): 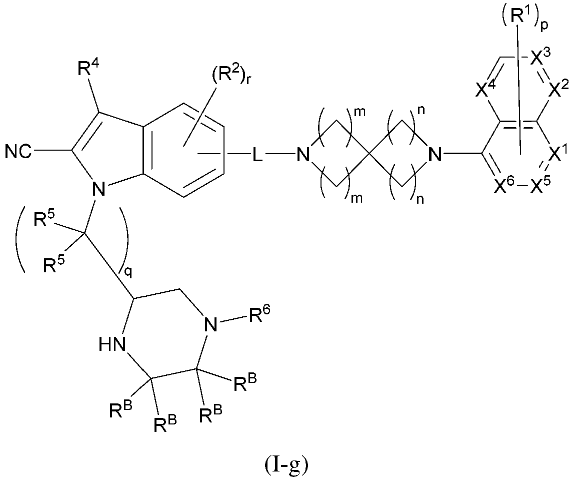 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-h): 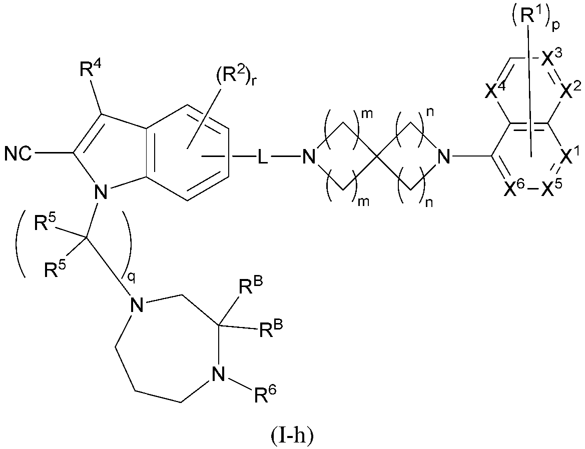 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo all other variables are as defined herein.

In some embodiments the compound is of Formula (I-i): 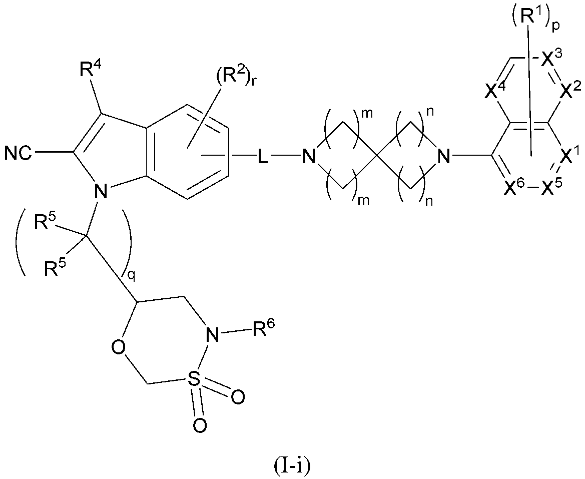 or a pharmaceutically acceptable salt, stereoisomer, solvate, prodrug, isotopic derivative, or tautomer thereof.

In some embodiments the compound is of Formula (I-j): 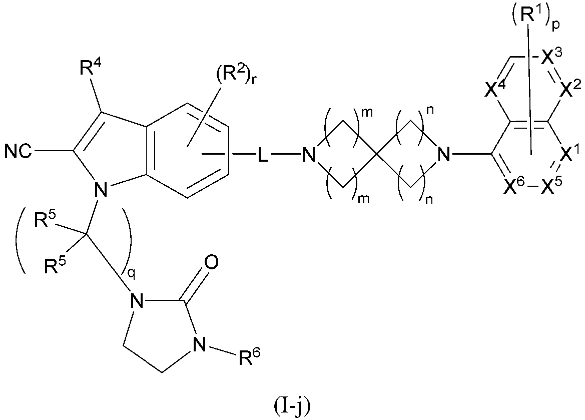 or a pharmaceutically acceptable salt, stereoisomer, solvate, prodrug, isotopic derivative, or tautomer thereof.

In some embodiments the compound is of Formula (I-k): 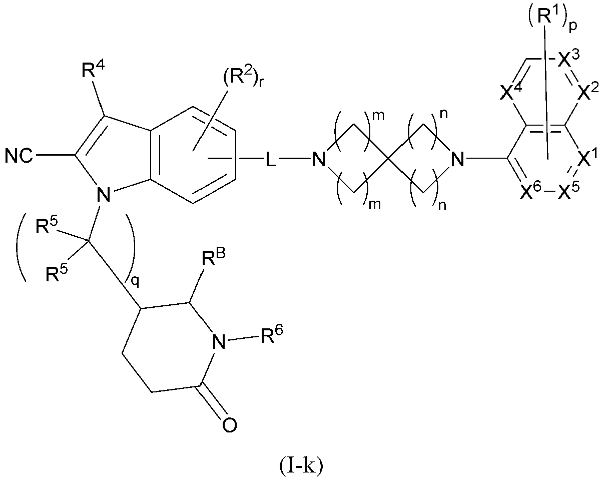 or a pharmaceutically acceptable salt, stereoisomer, solvate, prodrug, isotopic derivative, or tautomer thereof wherein R^{B} is selected from H, C₁-C₆ alkyl, C₃-C₁₀ cycloalkyl, aryl, heteroaryl, and all other variables are as defined herein.

In some embodiments the compound is of Formula (I-l): 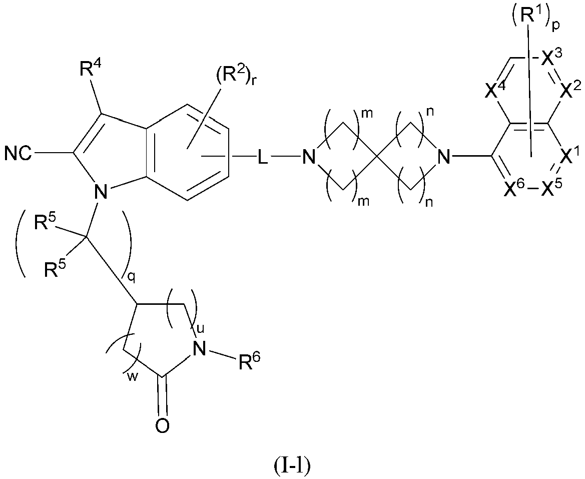 or a pharmaceutically acceptable salt, stereoisomer, solvate, prodrug, isotopic derivative, or tautomer thereof wherein index u is an integer selected from 1, 2 and 3, index w is an integer selected from 1, 2 and 3 and all other variables are as defined herein.

In some embodiments the compound is of Formula (I-m): 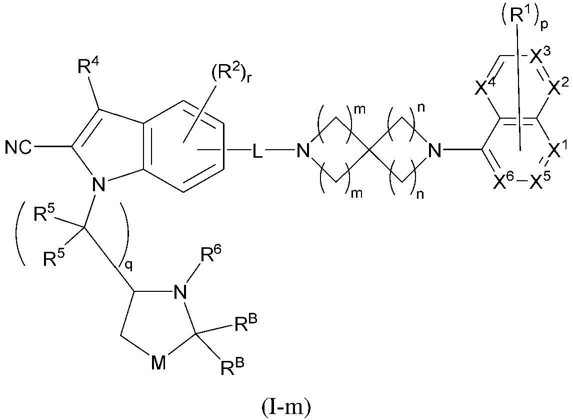 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo, M is selected from CH₂, O and NH, and all other variables are as defined herein.

In some embodiments the compound is of Formula (I-n): 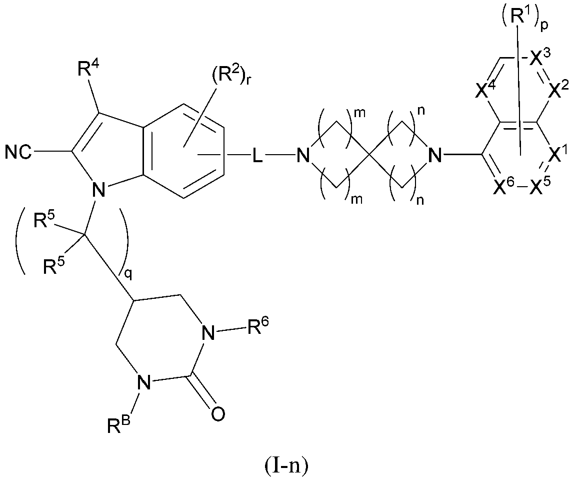 or a pharmaceutically acceptable salt, stereoisomer, solvate, prodrug, isotopic derivative, or tautomer thereof wherein R^{B} is selected from H, C₁-C₆ alkyl, aryl, and all other variables are as defined herein.

In some embodiments the compound is of Formula (I-o): 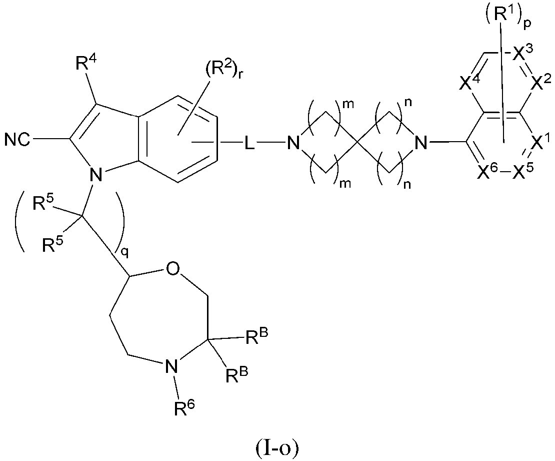 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo, and all other variables are as defined herein.

In some embodiments the compound is of Formula (I-p): 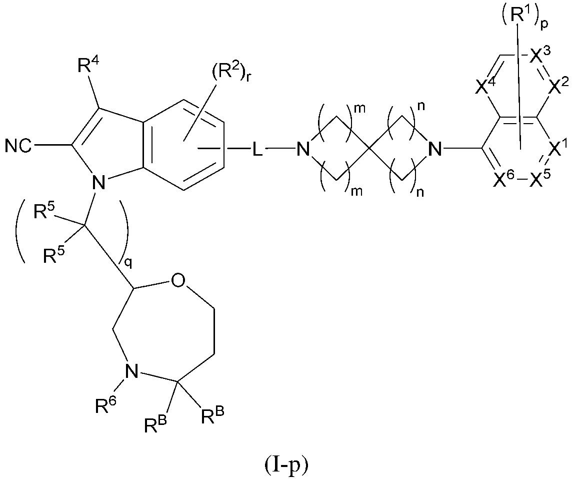 or a pharmaceutically acceptable salt, stereoisomer, solvate, prodrug, isotopic derivative, or tautomer thereof wherein each R^{B} is independently selected from H, C₁-C₆ alkyl, or two R^{B} form oxo, and all other variables are as defined herein.

In some embodiments the compound is of Formula (I-r): 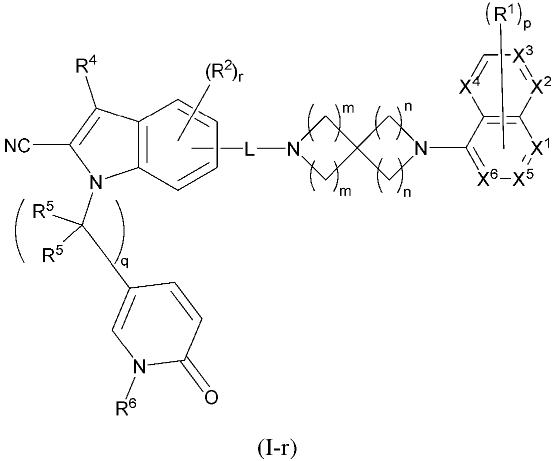 or a pharmaceutically acceptable salt, stereoisomer, solvate, prodrug, isotopic derivative, or tautomer thereof.

In some embodiments the compound is of Formula (I-s): 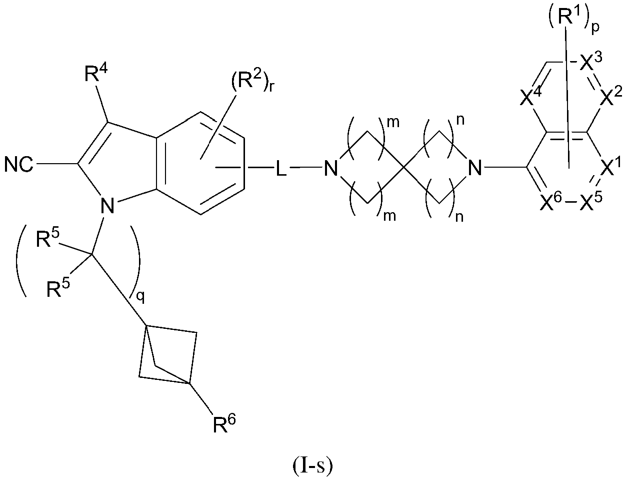 or a pharmaceutically acceptable salt, stereoisomer, solvate, prodrug, isotopic derivative, or tautomer thereof.

In some embodiments the compound is of Formula (I-t): 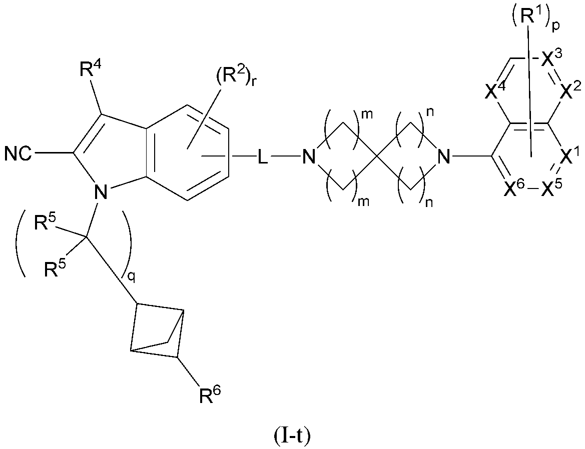 or a pharmaceutically acceptable salt, stereoisomer, solvate, prodrug, isotopic derivative, or tautomer thereof.

In some embodiments the compound is of Formula I'-a: 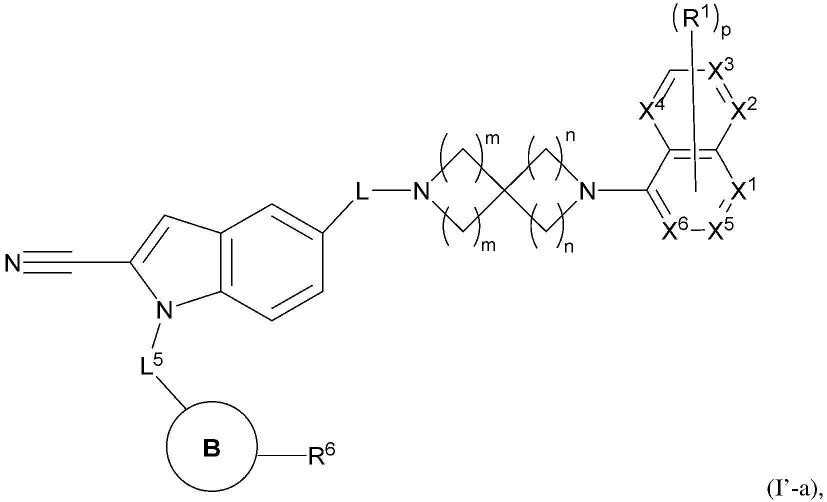 or a pharmaceutically acceptable salt, stereoisomer, solvate, prodrug, isotopic derivative, or tautomer thereof.

In some embodiments the compound is of Formula I'-b: 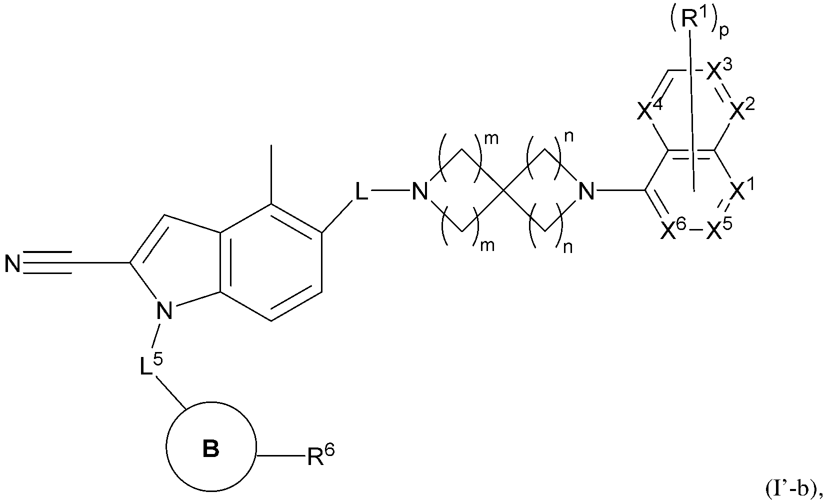 or a pharmaceutically acceptable salt, stereoisomer, solvate, prodrug, isotopic derivative, or tautomer thereof.

In some embodiments the compound is of Formula I'-c: 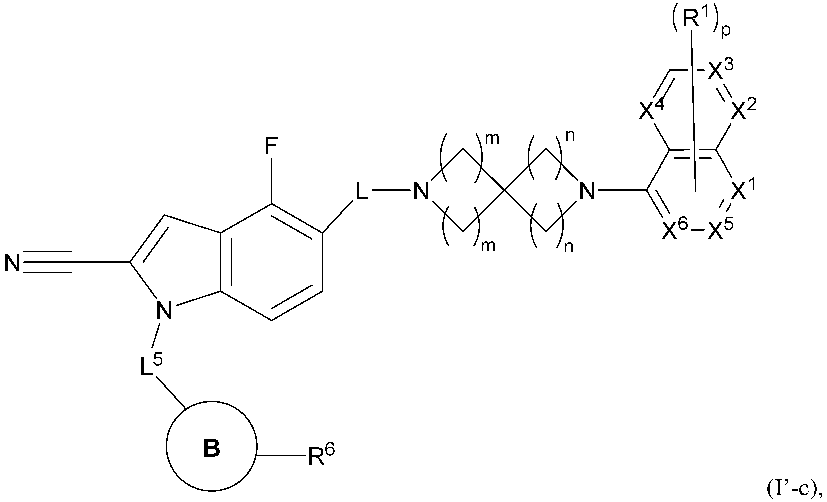 or a pharmaceutically acceptable salt, stereoisomer, solvate, prodrug, isotopic derivative, or tautomer thereof.

In some embodiments the compound is of Formula I'-d: 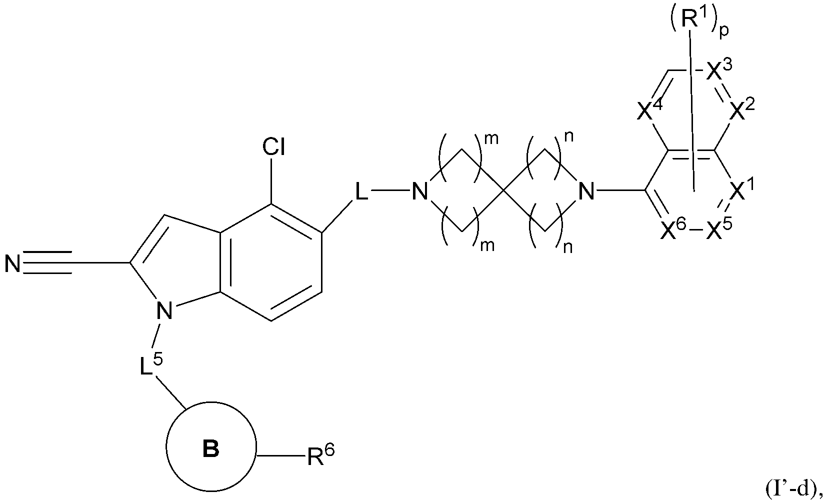 or a pharmaceutically acceptable salt, stereoisomer, solvate, prodrug, isotopic derivative, or tautomer thereof.

In some embodiments the compound is of Formula I'-e: 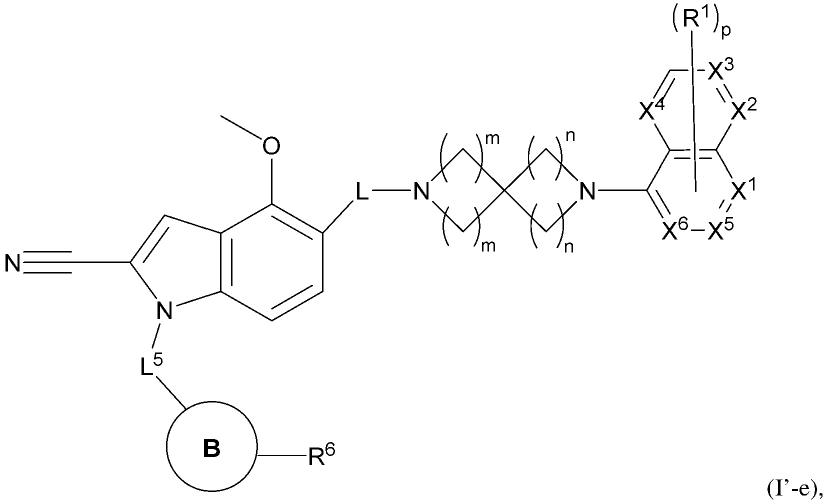 or a pharmaceutically acceptable salt, stereoisomer, solvate, prodrug, isotopic derivative, or tautomer thereof.

In some embodiments the compound is of Formula I'-f: 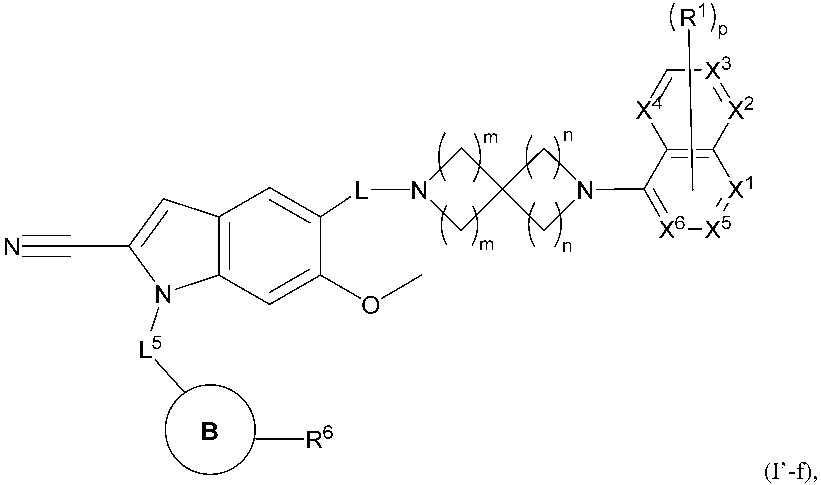 or a pharmaceutically acceptable salt, stereoisomer, solvate, prodrug, isotopic derivative, or tautomer thereof.

In some embodiments the compound is of Formula I'-g: 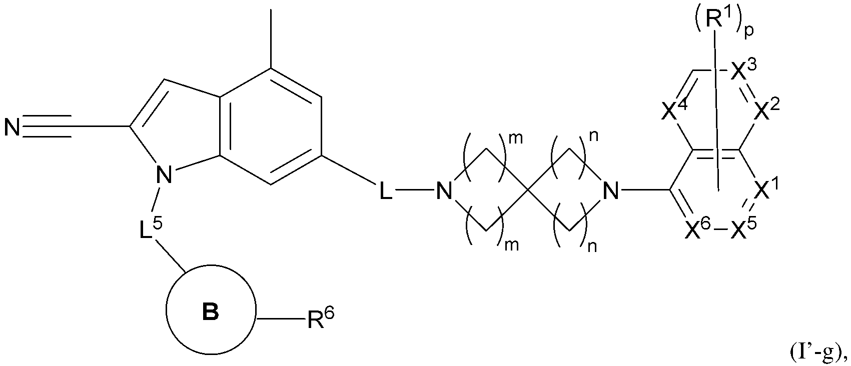 or a pharmaceutically acceptable salt, stereoisomer, solvate, prodrug, isotopic derivative, or tautomer thereof.

In some embodiments the compound is of Formula I'-h: 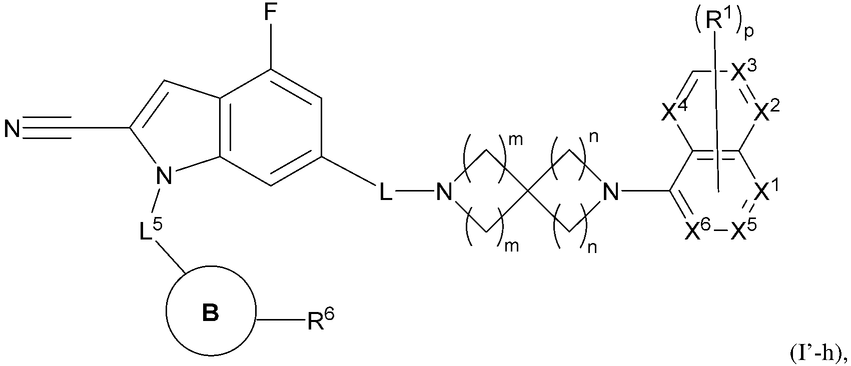 or a pharmaceutically acceptable salt, stereoisomer, solvate, prodrug, isotopic derivative, or tautomer thereof.

In some embodiments the compound is of Formula I'-i: 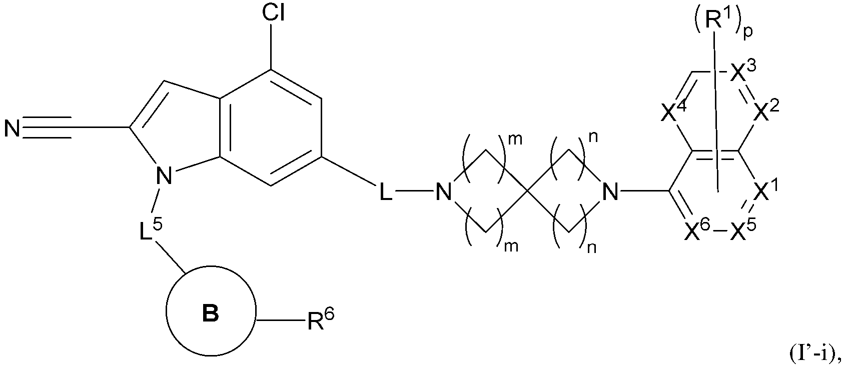 or a pharmaceutically acceptable salt, stereoisomer, solvate, prodrug, isotopic derivative, or tautomer thereof.

In some embodiments, the compound is selected from the compounds described in **Table 5** and pharmaceutically acceptable salts, stereoisomers, solvates, prodrugs, isotopic derivatives, or tautomers thereof.

In some embodiments, the compound is selected from the compounds described in Table 1 and prodrugs and pharmaceutically acceptable salts thereof.

In some embodiments, the compound is selected from the compounds described in Table 1 and pharmaceutically acceptable salts thereof.

In some embodiments, the compound is selected from the prodrugs of the compounds described in **Table 5** and pharmaceutically acceptable salts thereof.

In some embodiments, the compound is selected from the compounds described in **Table 5**.

**Table 5. Certain examples of the compound of Formula I**

| **#** | **IUPAC Name** |
|---|---|
| **1** | 4-methyl-1-[[(2S)-5-oxomorpholin-2-yl]methyl]-5-[[2-[6-(trifluoromethoxy)quinazolin-4-yl]-2,7-diazaspiro[3.5]nonan-7-yl]methyl]indole-2-carbonitrile |
| **2** | 4-methyl-1-[(3-oxo-1,4-oxazepan-7-yl)methyl]-5-[[2-[6-(2,2,2-trifluoroethyl)quinazolin-4-yl]-2,7-diazaspiro[3.5]nonan-7-yl]methyl]indole-2-carbonitrile |
| **3** | 4-methyl-1-[2-(2-oxoimidazolidin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **4** | 4-methyl-1-[(2-oxohexahydropyrimidin-5-yl)methyl]-5-[[2-[6-(2,2,2-trifluoroethyl)quinazolin-4-yl]-2,7-diazaspiro[3.5]nonan-7-yl]methyl]indole-2-carbonitrile |
| **5** | 4-methyl-1-[(7-oxoazepan-4-yl)methyl]-5-[[2-[6-(2,2,2-trifluoroethyl)quinazolin-4-yl]-2,7-diazaspiro[3.5]nonan-7-yl]methyl]indole-2-carbonitrile |
| **6** | 4-methyl-1-[(2-oxoimidazolidin-4-yl)methyl]-5-[[2-[6-(2,2,2-trifluoroethyl)quinazolin-4-yl]-2,7-diazaspiro[3.5]nonan-7-yl]methyl]indole-2-carbonitrile |
| **7** | 4-methyl-1-[(6-methyl-5-oxo-morpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **8** | 1-[(6,6-dimethyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **9** | 4-methyl-1-[(4-methylsulfonylmorpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **10** | 5-[[2-[5-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile |
| **11** | 5-[[2-[7-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile |
| **12** | 5-[[2-[5-fluoro-6-(2,2,2-trifluoroethyl)quinazolin-4-yl]-2,7-diazaspiro[3.5]nonan-7-yl]methyl]-4-methyl-1-[(5-oxomorpholin-2-yl)methyl]indole-2-carbonitrile |
| **13** | 4-methyl-1-[1-methyl-3-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **14** | 4-methyl-1-[(6-oxo-1H-pyridin-3-yl)methyl]-5-[[2-[6-(2,2,2-trifluoroethyl)quinazolin-4-yl]-2,7-diazaspiro[3.5]nonan-7-yl]methyl]indole-2-carbonitrile |
| **15** | 4-methyl-1-[(4-methyl-5-oxo-morpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **16** | 4-methyl-1-[(4-methyl-3,3-dioxo-1,3,4-oxathiazinan-6-yl)methyl]-5-[[2-[6-(2,2,2-trifluoroethyl)quinazolin-4-yl]-2,7-diazaspiro[3.5]nonan-7-yl]methyl]indole-2-carbonitrile |
| **17** | 1-[2-(4-butylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **18** | 1-[2-(4-cyclopropylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **19** | 2-imino-6,15-dimethyl-14-[[2-[6-(2,2,2-trifluoroethyl)quinazolin-4-yl]-2,7-diazaspiro[3.5]nonan-7-yl]methyl]-3,6,10-triazatetracyclo[8.7.0.03,8.011,16]heptadeca-1(17),11(16),12,14-tetraen-5-one |
| **20** | 6-methoxy-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **21** | 1-[(3,3-dioxo-1,3,4-oxathiazinan-6-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **22** | 4-methyl-1-[(2-oxo-4-piperidyl)methyl]-5-[[2-[6-(2,2,2-trifluoroethyl)quinazolin-4-yl]-2,7-diazaspiro[3.5]nonan-7-yl]methyl]indole-2-carbonitrile |
| **23** | 4-methyl-1-[(1R)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **24** | 4-methyl-1-[(1S)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **25** | 1-[(2R)-2-[(3S)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **26** | 1-[(2S)-2-[(3S)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **27** | 4-methyl-1-[(2R)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile |
| **28** | 4-methyl-1-[(2S)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile |
| **29** | 4-methyl-1-[2-(4-methylsulfonyl-1,4-diazepan-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **30** | 5-[[2-[7-fluoro-6-(2,2,2-trifluoroethyl)quinazolin-4-yl]-2,7-diazaspiro[3.5]nonan-7-yl]methyl]-4-methyl-1-[2-(4-methylsulfonylpiperazin-1-yl)propyl]indole-2-carbonitrile |
| **31** | 1-[(4-isobutyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **32** | 1-[2-(4-butyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **33** | 4-methyl-1-[2-(4-methylsulfonylpiperazin-1-yl)propyl]-5-[[3-[7-(2,2,2-trifluoroethyl)quinazolin-4-yl]-3,9-diazaspiro[5.5]undecan-9-yl]methyl]indole-3-carbonitrile |
| **34** | 6-((2-(6-(2,2,2-trifluoroethyl)quinazolin-4-yl)-2,7-diazaspiro[3.5]nonan-7-yl)methyl)-1H-indole-2-carbonitrile |
| **35** | 1-[(2S)-2-[(3R)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **36** | 1-[(2S)-2-[(3S)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **37** | 4-methyl-1-[(2R)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile |
| **38** | 4-methyl-1-[(2S)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile |
| **39** | 4-methyl-1-[2-(4-methylsulfonyl-1,4-diazepan-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **40** | *N-*(1R,4R)-[4-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]cyclohexyl]methanesulfonamide |
| **41** | *N-*(1R,4R)-[4-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]cyclohexyl]ethanesulfonamide |
| **42** | 4-methyl-1-[2-(4-methylsulfonylpiperazin-1-yl)propyl]-5-[[2-[6-(2,2,2-trifluoroethyl)pteridin-4-yl]-2,7-diazaspiro[3.5]nonan-7-yl]methyl]indole-2-carbonitrile |
| **43** | 4-methyl-1-[2-(2-methylsulfonyl-2,6-diazaspiro[3.3]heptan-6-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **44** | 4-methyl-1-[4-(4-methylsulfonylpiperazin-1-yl)butyl]-5-[[2-[6-(2,2,2-trifluoroethyl)quinazolin-4-yl]-2,7-diazaspiro[3.5]nonan-7-yl]methyl]indole-2-carbonitrile |
| **45** | 4-methyl-1-[2-(4-methylsulfonylpiperazin-1-yl)propyl]-5-[[2-[6-(2,2,2-trifluoroethyl)pyrido[3,4-d]pyrimidin-4-yl]-2,7-diazaspiro[3.5]nonan-7-yl]methyl]indole-2-carbonitrile |
| **46** | 4-methyl-1-[(4-methyl-5-oxo-piperazin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **47** | 4-methoxy-1-[1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **48** | 4-methyl-1-[(1R)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **49** | 4-methyl-1-[(1S)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **50** | 4-methoxy-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **51** | 4-methyl-1-[(5-oxopyrrolidin-3-yl)methyl]-5-[[2-[6-(2,2,2-trifluoroethyl)quinazolin-4-yl]-2,7-diazaspiro[3.5]nonan-7-yl]methyl]indole-2-carbonitrile |
| **52** | 4-methyl-1-[(5-oxopyrrolidin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **53** | 1-[2-(4-ethylsulfonyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **54** | 1-[[4-[(4-methoxyphenyl)methyl]-3,3-dioxo-1,3,4-oxathiazinan-6-yl]methyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **55** | 4-methyl-1-[2-(4-methylsulfonylpiperazin-1-yl)propyl]-5-[[7-[6-(2,2,2-trifluoroethyl)quinazolin-4-yl]-2,7-diazaspiro[3.5]nonan-2-yl]methyl]indole-3-carbonitrile |
| **56** | 5-[[2-[7-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile |
| **57** | 5-[[2-[5-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile |
| **58** | 4-methyl-5-[[2-[2-(methylamino)-6-(2,2,2-trifluoroethyl)quinazolin-4-yl]-2,7-diazaspiro[3.5]nonan-7-yl]methyl]-1-[[(2S)-5-oxomorpholin-2-yl]methyl]indole-2-carbonitrile |
| **59** | *N*-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]acetamide |
| **60** | 1-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]-3-methyl-urea |
| **61** | 4-[7-[[2-cyano-4-methyl-1-[[3-(sulfamoylamino)-1-bicyclo[1.1.1]pentanyl]methyl]indol-5-yl]methyl]-2,7-diazaspiro[3.5]nonan-2-yl]-6-(2,2,2-trifluoroe thyl)quinazoline |
| **62** | 4-methyl-5-[[2-[6-(2,2,2-trifluoroethyl)quinazolin-4-yl]-2,7-diazaspiro[3.5]nonan-7-yl]methyl]-1-[[3-(1,1,4-trioxo-1,2,5-thiadiazolidin-2-yl)-1-bicyclo[1.1.1]pentanyl]methyl]indole-2-carbonitrile |
| **63** | 4-methyl-1-[2-(4-prop-2-enoylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **64** | 1-[2-(4-isopropylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **65** | 4-methyl-1-[4-(4-prop-2-enoylpiperazin-1-yl)butyl]-5-[[2-[6-(2,2,2-trifluoroethyl)quinazolin-4-yl]-2,7-diazaspiro[3.5]nonan-7-yl]methyl]indole-2-carbonitrile |
| **66** | 4-methyl-1-[3-(4-prop-2-enoylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **67** | 4-methyl-1-[2-(4-methylsulfonylpiperazin-1-yl)propyl]-5-[[2-[6-(2,2,2-trifluoroethyl)pyrido[3,2-d]pyrimidin-4-yl]-2,7-diazaspiro[3.5]nonan-7-yl]methyl]indole-2-carbonitrile |
| **68** | 4-methyl-5-[[2-[6-(2,2,2-trifluoroethyl)quinazolin-4-yl]-2,7-diazaspiro[3.5]nonan-7-yl]methyl]-1H-indole-2-carbonitrile |
| **69** | 5-[[2-[5-fluoro-6-(2,2,2-trifluoroethyl)quinazolin-4-yl]-2,7-diazaspiro[3.5]nonan-7-yl]methyl]-4-methyl-1-[2-(4-methylsulfonylpiperazin-1-yl)propyl]indole-2-carbonitrile |
| **70** | 4-methyl-1-[1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **71** | 4-methyl-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **72** | 4-methyl-1-[(6-oxo-3-piperidyl)methyl]-5-[[2-[6-(2,2,2-trifluoroethyl)quinazolin-4-yl]-2,7-diazaspiro[3.5]nonan-7-yl]methyl]indole-2-carbonitrile |
| **73** | 1-[2-(4-butylsulfonyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **74** | 1-[2-(4-ethylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **75** | 4-methyl-1-[(2S)-2-(4-methylsulfonylpiperazin-1-yl)propyl]-5-[[2-[6-(1,1,2,2,2-pentafluoroethyl)quinazolin-4-yl]-2,7-diazaspiro[3.5]nonan-7-yl]methyl]indole-2-carbonitrile |
| **76** | 4-methyl-1-(2-(4-(methylsulfonothioyl)piperazin-1-yl)propyl)-5-((2-(6-(2,2,2-trifluoroethyl)quinazolin-4-yl)-2,7-diazaspiro[3.5]nonan-7-yl)methyl)-1H-indole-2-carbonitrile |
| **77** | 4-chloro-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **78** | 4-fluoro-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **79** | 4-methyl-1-[2-(4-methylsulfonylpiperazin-1-yl)propyl]-5-[[2-[7-(2,2,2-trifluoroethyl)phthalazin-1-yl]-2,7-diazaspiro[3.5]nonan-7-yl]methyl]indole-2-carbonitrile |
| **80** | 4-methyl-1-[2-(4-methylsulfonylpiperazin-1-yl)propyl]-5-[[2-[6-(2,2,2-trifluoroethyl)cinnolin-4-yl]-2,7-diazaspiro[3.5]nonan-7-yl]methyl]indole-2-carbonitrile |
| **81** | 4-methyl-1-[(2S)-2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **82** | 4-methyl-1-[(5-oxomorpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **83** | 4-methyl-1-[2-(4-methylsulfonylpiperazin-1-yl)propyl]-6-[[2-[6-(2,2,2-trifluoroethyl)quinazolin-4-yl]-2,7-diazaspiro[3.5]nonan-7-yl]methyl]indole-2-carbonitrile |
| **84** | 4-fluoro-1-[2-(4-methylsulfonylpiperazin-1-yl)propyl]-6-[[2-[6-(2,2,2-trifluoroethyl)quinazolin-4-yl]-2,7-diazaspiro[3.5]nonan-7-yl]methyl]indole-2-carbonitrile |
| **85** | 4-chloro-1-[2-(4-methylsulfonylpiperazin-1-yl)propyl]-6-[[2-[6-(2,2,2-trifluoroethyl)quinazolin-4-yl]-2,7-diazaspiro[3.5]nonan-7-yl]methyl]indole-2-carbonitrile |
| **86** | 1-(2-(4-acryloylpiperazin-1-yl)ethyl)-5-((2-(6-(2,2,2-trifluoroethyl)quinazolin-4-yl)-2,7-diazaspiro[3.5]nonan-7-yl)methyl)-1H-indole-2-carbonitrile |
| **87** | 4-methyl-1-[(5-oxo-1,4-oxazepan-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **88** | 4-methyl-1-[2-(4-methylsulfonylpiperazin-1-yl)propyl]-5-[2-[2-[6-(2,2,2-trifluoroethyl)quinazolin-4-yl]-2,7-diazaspiro[3.5]nonan-7-yl]ethyl]indole-2-carbonitrile |
| **89** | 4-methyl-1-[2-(4-methylsulfonylpiperazin-1-yl)propyl]-5-[[2-[6-(2,2,2-trifluoroethyl)pyrido[2,3-d]pyrimidin-4-yl]-2,7-diazaspiro[3.5]nonan-7-yl]methyl]indole-2-carbonitrile |
| **90** | 4-methyl-1-[2-(4-methylsulfonylpiperazin-1-yl)propyl]-5-[[3-[6-(2,2,2-trifluoroethyl)quinazolin-4-yl]-3,9-diazaspiro[5.5]undecan-9-yl]methyl]indole-3-carbonitrile |
| **91** | 1-(2-(4-(methylsulfonyl)piperazin-1-yl)ethyl)-5-((2-(6-(2,2,2-trifluoroethyl)quinazolin-4-yl)-2,7-diazaspiro[3.5]nonan-7-yl)methyl)-1H-indole-2-carbonitrile |
| **92** | 4-methyl-1-[2-(3-oxopiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **93** | 4-methyl-1-[2-(3-oxopiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **94** | N-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]methanesulfonamide |
| **95** | N-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]formamide |
| **96** | rac-(R)-4-methyl-1-(2-(4-(methylsulfonyl)piperazin-1-yl)propyl)-5-((2-(6-(2,2,2-trifluoroethyl)quinazolin-4-yl)-2,7-diazaspiro[3.5]nonan-7-yl)methyl)-1H-indole-2-carbonitrile |
| **97** | 4-methyl-1-(2-morpholinoethyl)-5-((2-(6-(2,2,2-trifluoroethyl)quinazolin-4-yl)-2,7-diazaspiro[3.5]nonan-7-yl)methyl)-1H-indole-2-carbonitrile |
| **98** | 5-((2-(6-(2,2,2-trifluoroethyl)quinazolin-4-yl)-2,7-diazaspiro[3.5]nonan-7-yl)methyl)-1H-indole-2-carbonitrile |
| **99** | (E)-1-(2-(4-(4-(dimethylamino)but-2-enoyl)piperazin-1-yl)ethyl)-5-((2-(6-(2,2,2-trifluoroethyl)quinazolin-4-yl)-2,7-diazaspiro[3.5]nonan-7-yl)methyl)-1H-indole-2-carbonitrile |
| **100** | 4-methyl-1-[[(2R,3S)-6-oxo-2-phenyl-3-piperidyl]methyl]-5-[[2-[6-(2,2,2-trifluoroethyl)quinazolin-4-yl]-2,7-diazaspiro[3.5]nonan-7-yl]methyl]indole-2-carbonitrile |
| **101** | 4-methyl-1-[[(2R)-5-oxomorpholin-2-yl]methyl]-5-[[2-[6-(2,2,2-trifluoroethyl)quinazolin-4-yl]-2,7-diazaspiro[3.5]nonan-7-yl]methyl]indole-2-carbonitrile |
| **102** | 4-methyl-1-[[(2S)-5-oxomorpholin-2-yl]methyl]-5-[[2-[6-(2,2,2-trifluoroethyl)quinazolin-4-yl]-2,7-diazaspiro[3.5]nonan-7-yl]methyl]indole-2-carbonitrile |
| **103** | 4-((2-(6-(2,2,2-trifluoroethyl)quinazolin-4-yl)-2,7-diazaspiro[3.5]nonan-7-yl)methyl)-1H-indole-2-carbonitrile |
| **104** | 1-[(4-ethyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **105** | 4-methyl-1-[[(2S)-5-oxomorpholin-2-yl]methyl]-5-[[2-[2-oxo-6-(2,2,2-trifluoroethyl)-1H-quinazolin-4-yl]-2,7-diazaspiro[3.5]nonan-7-yl]methyl]indole-2-carbonitrile |

In some embodiments, the compound is a neutral form (i.e., not a salt) of any one of the compounds described in **Table 5**.

In some embodiments, the compound is a pharmaceutically acceptable salt of any one of the compounds described in **Table 5**.

In some embodiments, the compound is a lithium salt, sodium salt, potassium salt, calcium salt, or magnesium salt of any one of the compounds described in **Table 5**.

In some embodiments, the compound is a sodium salt or potassium salt of any one of the compounds described in **Table 5**.

In some embodiments, the compound is a sodium salt of any one of the compounds described in **Table 5**.

In some embodiments, the compound is a potassium salt of any one of the compounds described in **Table 5**.

In some aspects, the present disclosure provides a compound being an isotopic derivative (e.g., isotopically labeled compound) of any one of the compounds of the Formulae disclosed herein.

In some embodiments, the compound is an isotopic derivative of any one of the compounds described in **Table 5** and prodrugs and pharmaceutically acceptable salts thereof.

In some embodiments, the compound is an isotopic derivative of any one of the compounds described in **Table 5** and pharmaceutically acceptable salts thereof.

In some embodiments, the compound is an isotopic derivative of any one of prodrugs of the compounds described in **Table 5** and pharmaceutically acceptable salts thereof.

In some embodiments, the compound is an isotopic derivative of any one of the compounds described in **Table 5**.

It is understood that the isotopic derivative can be prepared using any of a variety of art-recognized techniques. For example, the isotopic derivative can generally be prepared by carrying out the procedures disclosed in the Schemes and/or in the Examples described herein, by substituting an isotopically labeled reagent for a non-isotopically labeled reagent.

In some embodiments, the isotopic derivative is a deuterium labeled compound.

In some embodiments, the isotopic derivative is a deuterium labeled compound of any one of the compounds of the Formulae disclosed herein.

The term "isotopic derivative", as used herein, refers to a derivative of a compound in which one or more atoms are isotopically enriched or labelled. For example, an isotopic derivative of a compound of Formula (I) is isotopically enriched with regard to, or labelled with, one or more isotopes as compared to the corresponding compound of Formula (I). In some embodiments, the isotopic derivative is enriched with regard to, or labelled with, one or more atoms selected from ²H, ¹³C, ¹⁴C, ¹⁵N, ¹⁸O, ²⁹Si, ³¹P, and ³⁴S. In some embodiments, the isotopic derivative is a deuterium labeled compound (*i.e.*, being enriched with ²H with regard to one or more atoms thereof).

In some embodiments, the compound is a deuterium labeled compound of any one of the compounds described in **Table 5** and prodrugs and pharmaceutically acceptable salts thereof.

In some embodiments, the compound is a deuterium labeled compound of any one of the compounds described in **Table 5** and pharmaceutically acceptable salts thereof.

In some embodiments, the compound is a deuterium labeled compound of any one of the prodrugs of the compounds described in **Table 5** and pharmaceutically acceptable salts thereof.

In some embodiments, the compound is a deuterium labeled compound of any one of the compounds described in **Table 5**.

It is understood that the deuterium labeled compound comprises a deuterium atom having an abundance of deuterium that is substantially greater than the natural abundance of deuterium, which is 0.015%.

In some embodiments, the deuterium labeled compound has a deuterium enrichment factor for each deuterium atom of at least 3500 (52.5% deuterium incorporation at each deuterium atom), at least 4000 (60% deuterium incorporation), at least 4500 (67.5% deuterium incorporation), at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). As used herein, the term "deuterium enrichment factor" means the ratio between the deuterium abundance and the natural abundance of a deuterium.

It is understood that the deuterium labeled compound can be prepared using any of a variety of art-recognized techniques. For example, the deuterium labeled compound can generally be prepared by carrying out the procedures disclosed in the Schemes and/or in the Examples described herein, by substituting a deuterium labeled reagent for a non-deuterium labeled reagent.

A compound of the disclosure or a pharmaceutically acceptable salt or solvate thereof that contains the aforementioned deuterium atom(s) is within the scope of the disclosure. Further, substitution with deuterium (*i.e.*, ²H) may afford certain therapeutic advantages resulting from greater metabolic stability, e.g., increased *in vivo* half-life or reduced dosage requirements.

In some embodiments, the compound is a ¹⁸F labeled compound.

In some embodiments, the compound is a ¹²³I labeled compound, a ¹²⁴I labeled compound, a ¹²⁵I labeled compound, a ¹²⁹I labeled compound, a ¹³¹I labeled compound, a ¹³⁵I labeled compound, or any combination thereof.

In some embodiments, the compound is a ³³S labeled compound, a ³⁴S labeled compound, a ³⁵S labeled compound, a ³⁶S labeled compound, or any combination thereof.

It is understood that the ¹⁸F, ¹²³I, ¹²⁴I, ¹²⁵I, ¹²⁹I, ¹³¹I, ¹³⁵I, ³S, ³⁴S, ³⁵S, and/or ³⁶S labeled compound, can be prepared using any of a variety of art-recognized techniques. For example, the deuterium labeled compound can generally be prepared by carrying out the procedures disclosed in the Schemes and/or in the Examples described herein, by substituting a ¹⁸F, ¹²³I, ¹²⁴I, ¹²⁵I, ¹²⁹I, ¹³¹I, ¹³⁵I, ³S, ³⁴S, ³⁵S, and/or ³⁶S labeled reagent for a non-isotope labeled reagent.

A compound of the disclosure or a pharmaceutically acceptable salt or solvate thereof that contains one or more of the aforementioned ¹⁸F, ¹²³I, ¹²⁴I, ¹²⁵I, ¹²⁹I, ¹³¹I, ¹³⁵I, ³S, ³⁴S, ³⁵S, and ³⁶S atom(s) is within the scope of the disclosure. Further, substitution with isotope (e.g., ¹⁸F, ¹²³I, ¹²⁴I, ¹²⁵I, ¹²⁹I, ¹³¹I, ¹³⁵I, ³S, ³⁴S, ³⁵S, and/or ³⁶S) may afford certain therapeutic advantages resulting from greater metabolic stability, e.g., increased *in vivo* half-life or reduced dosage requirements.

For the avoidance of doubt, it is to be understood that, where in this specification a group is qualified by "described herein", the said group encompasses the first occurring and broadest definition as well as each and all of the particular definitions for that group.

The various functional groups and substituents making up the compounds of the Formula (I) are typically chosen such that the molecular weight of the compound does not exceed 1000 daltons. More usually, the molecular weight of the compound will be less than 900, for example less than 800, or less than 750, or less than 700, or less than 650 daltons. More conveniently, the molecular weight is less than 600 and, for example, is 550 daltons or less.

A suitable pharmaceutically acceptable salt of a compound of the disclosure is, for example, an acid-addition salt of a compound of the disclosure, which is sufficiently basic, for example, an acid-addition salt with, for example, an inorganic or organic acid, for example hydrochloric, hydrobromic, sulfuric, phosphoric, trifluoroacetic, formic, citric methane sulfonate or maleic acid. In addition, a suitable pharmaceutically acceptable salt of a compound of the disclosure which is sufficiently acidic is an alkali metal salt, for example a sodium or potassium salt, an alkaline earth metal salt, for example a calcium or magnesium salt, an ammonium salt or a salt with an organic base which affords a pharmaceutically acceptable cation, for example a salt with methylamine, dimethylamine, diethylamine, trimethylamine, piperidine, morpholine or tris-(2-hydroxyethyl)amine.

It will be understood that the compounds of any one of the Formulae disclosed herein and any pharmaceutically acceptable salts thereof, comprise stereoisomers, mixtures of stereoisomers, polymorphs of all isomeric forms of said compounds.

As used herein, the term "isomerism" means compounds that have identical molecular formulae but differ in the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images of each other are termed "enantiomers" or sometimes optical isomers. A mixture containing equal amounts of individual enantiomeric forms of opposite chirality is termed a "racemic mixture."

As used herein, the term "chiral center" refers to a carbon atom bonded to four nonidentical substituents.

As used herein, the term "chiral isomer" means a compound with at least one chiral center. Compounds with more than one chiral center may exist either as an individual diastereomer or as a mixture of diastereomers, termed "diastereomeric mixture." When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the *Sequence Rule* of Cahn, Ingold and Prelog. (Cahn et al., Angew. Chem. Inter. Edit. 1966, 5, 385; errata 511; Cahn et al., Angew. Chem. 1966, 78, 413; Cahn and Ingold, J. Chem. Soc. 1951 (London), 612; Cahn et al., Experientia 1956, 12, 81; Cahn, J. Chem. Educ. 1964, 41, 116).

As used herein, the term "geometric isomer" means the diastereomers that owe their existence to hindered rotation about double bonds or a cycloalkyl linker (*e.g.*, 1,3-cyclobutyl). These configurations are differentiated in their names by the prefixes cis and trans, or Z and E, which indicate that the groups are on the same or opposite side of the double bond in the molecule according to the Cahn-Ingold-Prelog rules.

It is to be understood that the compounds of the present disclosure may be depicted as different chiral isomers or geometric isomers. It is also to be understood that when compounds have chiral isomeric or geometric isomeric forms, all isomeric forms are intended to be included in the scope of the present disclosure, and the naming of the compounds does not exclude any isomeric forms, it being understood that not all isomers may have the same level of activity.

It is to be understood that the structures and other compounds discussed in this disclosure include all atropic isomers thereof. It is also to be understood that not all atropic isomers may have the same level of activity.

As used herein, the term "atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

As used herein, the term "tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerization is possible, a chemical equilibrium of the tautomers will be reached. The exact ratio of the tautomers depends on several factors, including temperature, solvent, and pH. The concept of tautomers that are interconvertible by tautomerisations is called tautomerism. Of the various types of tautomerism that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ring-shaped) form as exhibited by glucose.

It is to be understood that the compounds of the present disclosure may be depicted as different tautomers. It should also be understood that when compounds have tautomeric forms, all tautomeric forms are intended to be included in the scope of the present disclosure, and the naming of the compounds does not exclude any tautomer form. It will be understood that certain tautomers may have a higher level of activity than others.

Compounds of any one of the Formulae disclosed herein may exist in a number of different tautomeric forms and references to compounds of Formula (I) include all such forms. For the avoidance of doubt, where a compound can exist in one of several tautomeric forms, and only one is specifically described or shown, all others are nevertheless embraced by Formula (I) or (II). Examples of tautomeric forms include keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro.

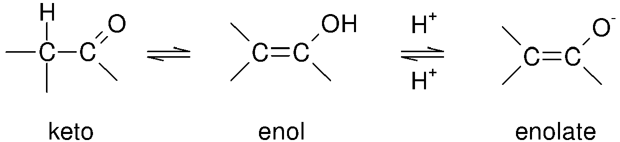

As a non-limiting example of tautomerism of the compound of Formula (I) may be presented by compound with substituent R¹ presented by OH:

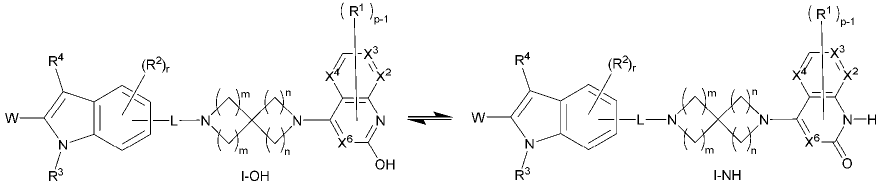

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric centre, for example, it is bonded to four different groups, a pair of enantiomers is possible. An enantiomer can be characterised by the absolute configuration of its asymmetric centre and is described by the R- and S-sequencing rules of Cahn and Prelog, or by the manner in which the molecule rotates the plane of polarised light and designated as dextrorotatory or levorotatory (*i.e.*, as (+) or (-)-isomers respectively). A chiral compound can exist as either individual enantiomer or as a mixture thereof. A mixture containing equal proportions of the enantiomers is called a "racemic mixture".

The compounds of this disclosure may possess one or more asymmetric centres; such compounds can therefore be produced as individual (R)- or (S)-stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the disclosure may have geometric isomeric centres (*E*- and *Z*- isomers). It is to be understood that the present disclosure encompasses all optical, diastereoisomers and geometric isomers and mixtures thereof that possess inflammasome inhibitory activity.

The present disclosure also encompasses compounds of the disclosure as defined herein which comprise one or more isotopic substitutions.

It is to be understood that the compounds of any Formula described herein include the compounds themselves, as well as their salts, and their solvates, if applicable. A salt, for example, can be formed between an anion and a positively charged group (*e.g.*, amino) on a substituted compound disclosed herein. Suitable anions include chloride, bromide, iodide, sulfate, bisulfate, sulfamate, nitrate, phosphate, citrate, methanesulfonate, trifluoroacetate, glutamate, glucuronate, glutarate, malate, maleate, succinate, fumarate, tartrate, tosylate, salicylate, lactate, naphthalenesulfonate, and acetate (*e.g.*, trifluoroacetate).

As used herein, the term "pharmaceutically acceptable anion" refers to an anion suitable for forming a pharmaceutically acceptable salt. Likewise, a salt can also be formed between a cation and a negatively charged group (*e.g.*, carboxylate) on a substituted compound disclosed herein. Suitable cations include sodium ion, potassium ion, magnesium ion, calcium ion, and an ammonium cation such as tetramethylammonium ion or diethylamine ion. The substituted compounds disclosed herein also include those salts containing quaternary nitrogen atoms.

It is to be understood that the compounds of the present disclosure, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

As used herein, the term "solvate" means solvent addition forms that contain either stoichiometric or non-stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as H₂O.

As used herein, the term "analog" refers to a chemical compound that is structurally similar to another but differs slightly in composition (as in the replacement of one atom by an atom of a different element or in the presence of a particular functional group, or the replacement of one functional group by another functional group). Thus, an analog is a compound that is similar or comparable in function and appearance, but not in structure or origin to the reference compound.

As used herein, the term "derivative" refers to compounds that have a common core structure and are substituted with various groups as described herein.

As used herein, the term "bioisostere" refers to a compound resulting from the exchange of an atom or of a group of atoms with another, broadly similar, atom or group of atoms. The objective of a bioisosteric replacement is to create a new compound with similar biological properties to the parent compound. The bioisosteric replacement may be physicochemically or topologically based. Examples of carboxylic acid bioisosteres include, but are not limited to, acyl sulfonamides, tetrazoles, sulfonates and phosphonates. See*, e.g.*, Patani and LaVoie, Chem. Rev. 96, 3147-3176, 1996.

It is also to be understood that certain compounds of any one of the Formulae disclosed herein may exist in solvated as well as unsolvated forms such as, for example, hydrated forms. A suitable pharmaceutically acceptable solvate is, for example, a hydrate such as hemi-hydrate, a monohydrate, a di-hydrate, or a tri-hydrate. It is to be understood that the disclosure encompasses all such solvated forms that possess inflammasome inhibitory activity.

It is also to be understood that certain compounds of any one of the Formulae disclosed herein may exhibit polymorphism, and that the disclosure encompasses all such forms, or mixtures thereof, which possess inflammasome inhibitory activity. It is generally known that crystalline materials may be analysed using conventional techniques such as X-Ray Powder Diffraction analysis, Differential Scanning Calorimetry, Thermal Gravimetric Analysis, Diffuse Reflectance Infrared Fourier Transform (DRIFT) spectroscopy, Near Infrared (NIR) spectroscopy, solution and/or solid state nuclear magnetic resonance spectroscopy. The water content of such crystalline materials may be determined by Karl Fischer analysis.

Compounds of any one of the Formulae disclosed herein containing an amine function may also form *N-*oxides. A reference herein to a compound of Formula (I) or (II) that contains an amine function also includes the *N-*oxide. Where a compound contains several amine functions, one or more than one nitrogen atom may be oxidised to form an *N-*oxide. Particular examples of *N-*oxides are the *N-*oxides of a tertiary amine or a nitrogen atom of a nitrogen-containing heterocycle. *N-*oxides can be formed by treatment of the corresponding amine with an oxidising agent such as hydrogen peroxide or a peracid (*e.g.* a peroxycarboxylic acid), see for example Advanced Organic Chemistry, by Jerry March, 4th Edition, Wiley Interscience, pages. More particularly, *N-*oxides can be made by the procedure of L. W. Deady (Syn. Comm. 1977, 7, 509-514) in which the amine compound is reacted with meta-chloroperoxybenzoic acid (mCPBA), for example, in an inert solvent such as dichloromethane.

The compounds of any one of the Formulae disclosed herein may be administered in the form of a prodrug which is broken down in the human or animal body to release a compound of the disclosure. A prodrug may be used to alter the physical properties and/or the pharmacokinetic properties of a compound of the disclosure. A prodrug can be formed when the compound of the disclosure contains a suitable group or substituent to which a property-modifying group can be attached. Examples of prodrugs include derivatives containing *in vivo* cleavable alkyl or acyl substituents at the ester or amide group in any one of the Formulae disclosed herein.

Accordingly, the present disclosure includes those compounds of any one of the Formulae disclosed herein as defined hereinbefore when made available by organic synthesis and when made available within the human or animal body by way of cleavage of a prodrug thereof. Accordingly, the present disclosure includes those compounds of any one of the Formulae disclosed herein that are produced by organic synthetic means and also such compounds that are produced in the human or animal body by way of metabolism of a precursor compound, that is a compound of any one of the Formulae disclosed herein may be a synthetically produced compound or a metabolically-produced compound.

A suitable pharmaceutically acceptable prodrug of a compound of any one of the Formulae disclosed herein is one that is based on reasonable medical judgment as being suitable for administration to the human or animal body without undesirable pharmacological activities and without undue toxicity. Various forms of prodrug have been described, for example in the following documents: a) Methods in Enzymology, Vol. 42, p. 309-396, edited by K. Widder, et al. (Academic Press, 1985); b) Design of Pro-drugs, edited by H. Bundgaard, (Elsevier, 1985); c) A Textbook of Drug Design and Development, edited by Krogsgaard-Larsen and H. Bundgaard, Chapter 5 "Design and Application of Pro-drugs", by H. Bundgaard p. 113-191 (1991); d) H. Bundgaard, Advanced Drug Delivery Reviews, 8, 1-38 (1992); e) H. Bundgaard, et al., Journal of Pharmaceutical Sciences, 77, 285 (1988); f) N. Kakeya, et al., Chem. Pharm. Bull., 32, 692 (1984); g) T. Higuchi and V. Stella, "Pro-Drugs as Novel Delivery Systems", A.C.S. Symposium Series, Volume 14; and h) E. Roche (editor), "Bioreversible Carriers in Drug Design", Pergamon Press, 1987.

A suitable pharmaceutically acceptable prodrug of a compound of any one of the Formulae disclosed herein that possesses a hydroxy group is, for example, an *in vivo* cleavable ester or ether thereof. An *in vivo* cleavable ester or ether of a compound of any one of the Formulae disclosed herein containing a hydroxy group is, for example, a pharmaceutically acceptable ester or ether which is cleaved in the human or animal body to produce the parent hydroxy compound. Suitable pharmaceutically acceptable ester forming groups for a hydroxy group include inorganic esters such as phosphate esters (including phosphoramidic cyclic esters). Further suitable pharmaceutically acceptable ester forming groups for a hydroxy group include C₁-C₁₀ alkanoyl groups such as acetyl, benzoyl, phenylacetyl and substituted benzoyl and phenylacetyl groups, C₁-C₁₀ alkoxycarbonyl groups such as ethoxycarbonyl, *N*,*N*-(C₁-C₆ alkyl)₂carbamoyl, 2-dialkylaminoacetyl and 2-carboxyacetyl groups. Examples of ring substituents on the phenylacetyl and benzoyl groups include aminomethyl, N-alkylaminomethyl, *N,N-*dialkylaminomethyl, morpholinomethyl, piperazin-1-ylmethyl and 4-(C₁-C₄ alkyl)piperazin-1-ylmethyl. Suitable pharmaceutically acceptable ether forming groups for a hydroxy group include α-acyloxyalkyl groups such as acetoxymethyl and pivaloyloxymethyl groups.

A suitable pharmaceutically acceptable prodrug of a compound of any one of the Formulae disclosed herein that possesses a carboxy group is, for example, an *in vivo* cleavable amide thereof, for example an amide formed with an amine such as ammonia, a C₁₋₄alkylamine such as methylamine, a (C₁-C₄ alkyl)₂amine such as dimethylamine, *N-*ethyl*-N-*methylamine or diethylamine, a C₁-C₄ alkoxy-C₂-C₄ alkylamine such as 2-methoxyethylamine, a phenyl-C₁-C₄ alkylamine such as benzylamine and amino acids such as glycine or an ester thereof.

A suitable pharmaceutically acceptable prodrug of a compound of any one of the Formulae disclosed herein that possesses an amino group is, for example, an *in vivo* cleavable amide derivative thereof. Suitable pharmaceutically acceptable amides from an amino group include, for example an amide formed with C₁-C₁₀ alkanoyl groups such as an acetyl, benzoyl, phenylacetyl and substituted benzoyl and phenylacetyl groups. Examples of ring substituents on the phenylacetyl and benzoyl groups include aminomethyl, *N-*alkylaminomethyl, *N*,*N-*dialkylaminomethyl,morpholinomethyl,piperazin-1-ylmethyl and 4-(C₁-C₄ alkyl)piperazin-1-ylmethyl.

The *in vivo* effects of a compound of any one of the Formulae disclosed herein may be exerted in part by one or more metabolites that are formed within the human or animal body after administration of a compound of any one of the Formulae disclosed herein. As stated hereinbefore, the *in vivo* effects of a compound of any one of the Formulae disclosed herein may also be exerted by way of metabolism of a precursor compound (a prodrug).

### Method of Synthesizing the Compounds

The compounds of the present invention may be made by a variety of methods, including standard chemistry. Suitable synthetic routes are depicted in the Schemes given below.

The compounds of Formula (I) may be prepared by methods known in the art of organic synthesis as set forth in part by the following synthetic schemes. In the schemes described below, it is well understood that protecting groups for sensitive or reactive groups are employed where necessary in accordance with general principles or chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of those skilled in the art will recognize if a stereocenter exists in the compounds of Formula (I). Accordingly, the present invention includes both possible stereoisomers (unless specified in the synthesis) and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley-Interscience, 1994).

The compounds described herein may be made from commercially available starting materials or synthesized using known organic, inorganic, and/or enzymatic processes.

### Preparation of Compounds

The compounds of the present invention can be prepared in a number of ways well known to those skilled in the art of organic synthesis. By way of example, compounds of the present invention can be synthesized using the methods described below, together with synthetic methods known in the art of synthetic organic chemistry, or variations thereon as appreciated by those skilled in the art. Suitable methods include but are not limited to those methods described below. Compounds of the present invention can be synthesized by following the steps outlined in General Procedures A or B which comprise different sequences of assembling intermediates or compounds. Starting materials are either commercially available or made by known procedures in the reported literature or as illustrated below.

### GENERAL PROCEDURE

In general, the compound of the Formula (I) can be prepared using reaction of reductive amination of carbonyl compound (A) with amine (B) in the presence of appropriate reducing agent ([H]):

A-C(O)H + B-NH₂ + [H] → Compound of Formula (I)

In more specific aspect, the compound of the Formula (I) can be obtained according to the scheme presented below:

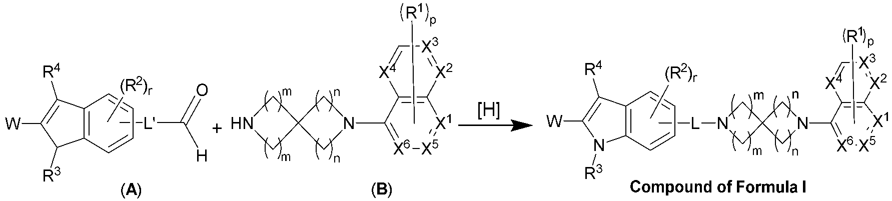

Reagents (A) and (B) may be commercially available compounds itself or products of synthesis from commercially available reagents. For compounds (A) and (B) preparation may be used one step or multistep synthetic procedures, including but not limited procedures described herein in preparative part.

As not limiting examples of the preparation of the compound of Formula (I) can be presented by reaction of any one of compound (A) with any one of compound (B) presented in the **Table 6**, according to reaction scheme presented in paragraph [**1028**] herein in the conditions described herein or any other conditions of reductive amination known from the art.

**Table 6.**

| 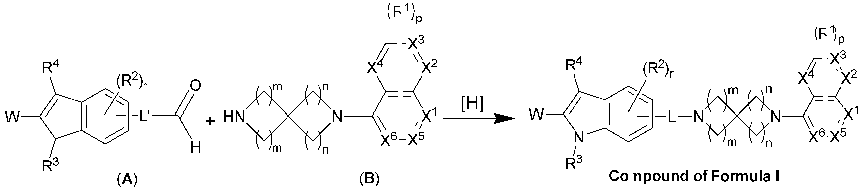 | |
|---|---|
| **(A)** | **(B)** |
| 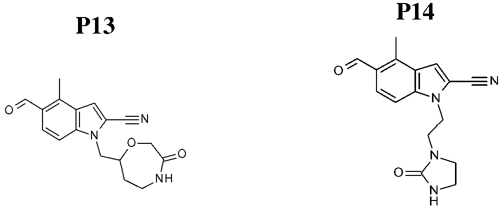 | 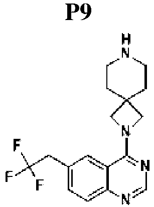 |
| 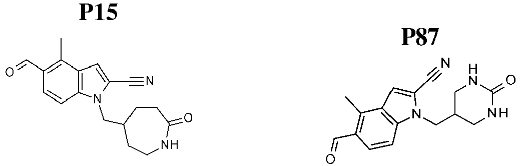 | 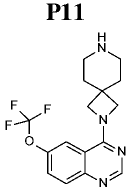 |
| 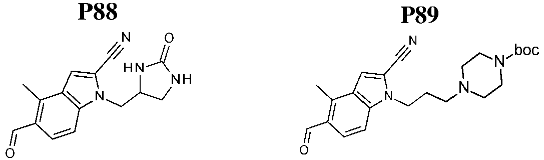 | 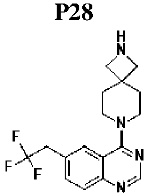 |
| 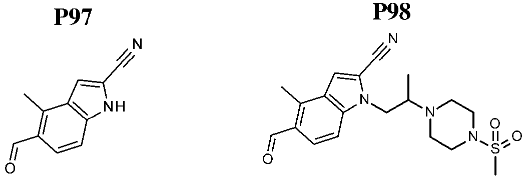 | 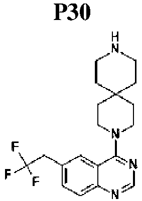 |
| 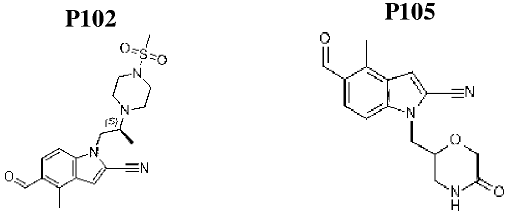 | 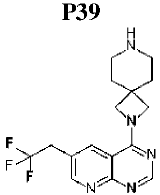 |
| 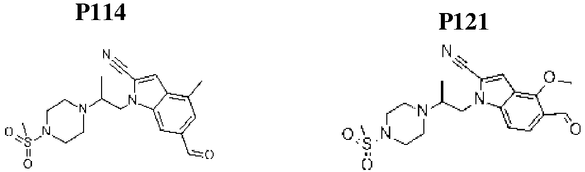 | 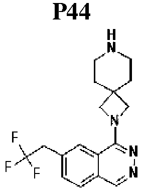 |
| 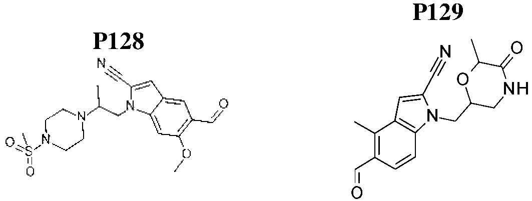 | 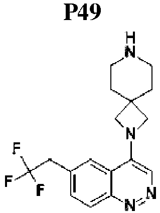 |
| 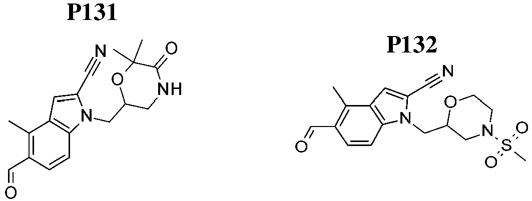 | 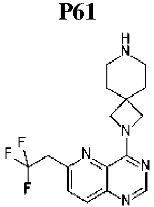 |
| 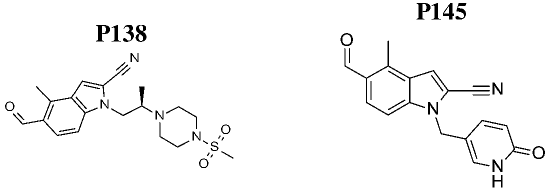 | 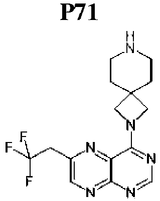 |
| 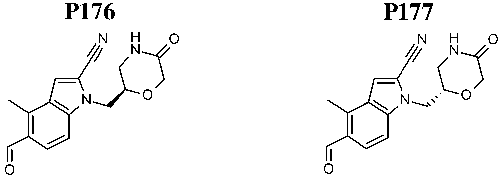 | 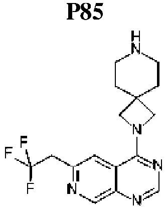 |
| 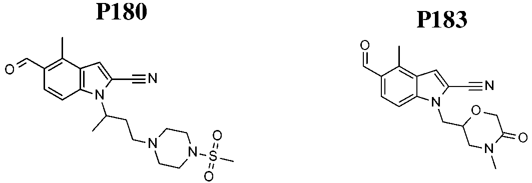 | 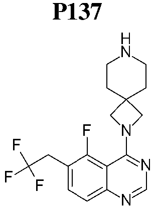 |
| 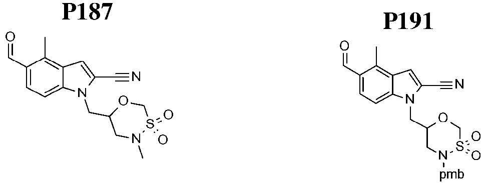 | 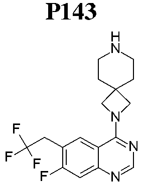 |
| 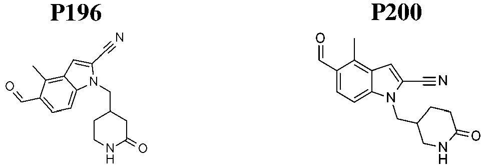 | 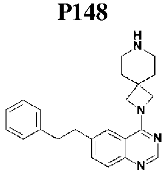 |
| 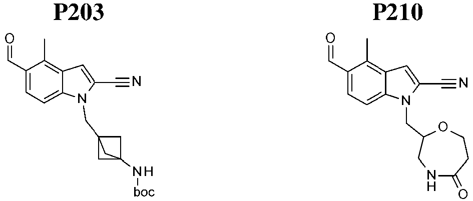 | 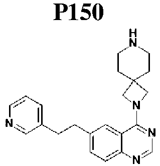 |
| 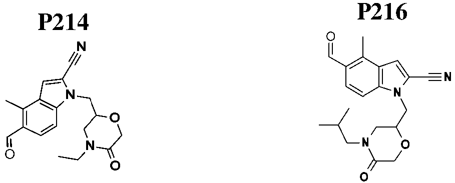 | 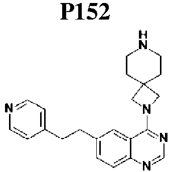 |
| 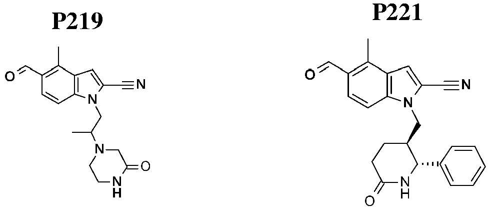 | 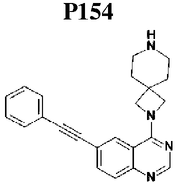 |
| 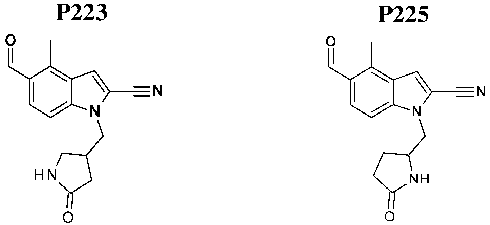 | 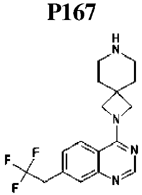 |
| 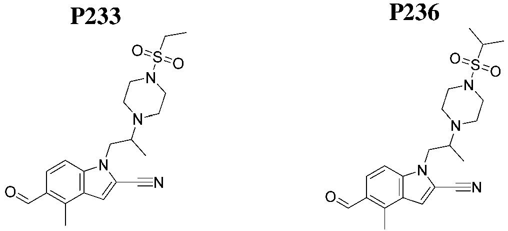 | 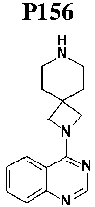 |
| 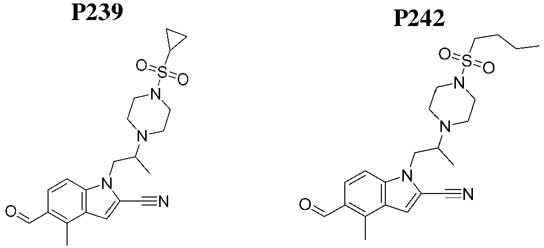 | 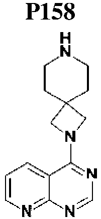 |
| 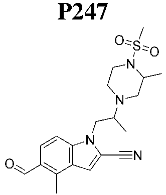 | 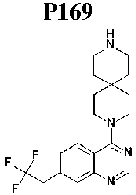 |
| 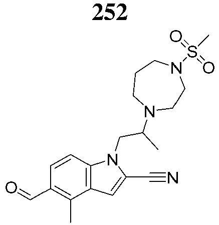 | 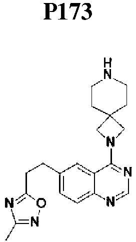 |
| 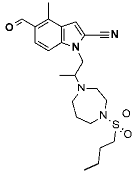 | 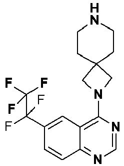 |
| 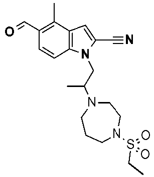 | 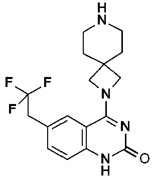 |

It should be obvious for specialist in this field that any of compound of Formula (I) obtained according to the procedures described above may be a subject for further transformation and modification that will led to obtain other compound of Formula (I).

As a specific non-limiting example of further transformation of Compound of Formula (I) can be presented by the reaction of preparation of the compound **97** (***Example 15***) from the compound **68** (***Example 9***):

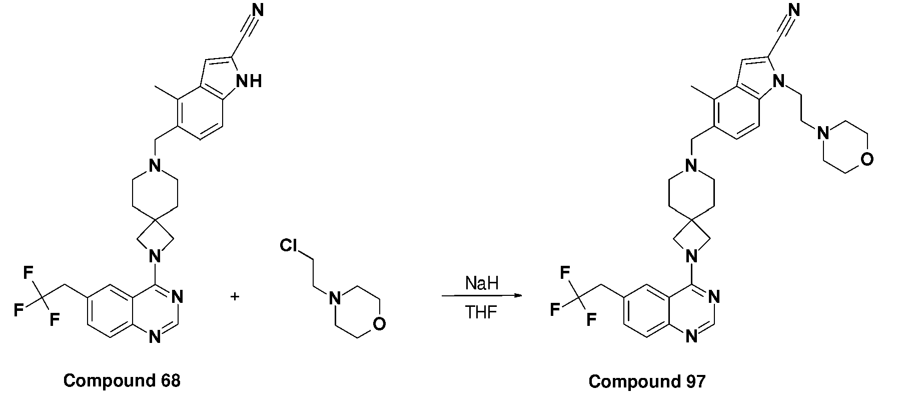

### Biological Assays

Compounds designed, selected and/or optimized by methods described above, once produced, can be characterized using a variety of assays known to those skilled in the art to determine whether the compounds have biological activity. For example, the molecules can be characterized by conventional assays, including but not limited to those assays described below, to determine whether they have a predicted activity, binding activity and/or binding specificity.

Furthermore, high-throughput screening can be used to speed up analysis using such assays. As a result, it can be possible to rapidly screen the molecules described herein for activity, using techniques known in the art. General methodologies for performing high-throughput screening are described, for example, in Devlin (1998) High Throughput Screening, Marcel Dekker; and U.S. Patent No. 5,763,263. High-throughput assays can use one or more different assay techniques including, but not limited to, those described below.

Various *in vitro* or *in vivo* biological assays may be suitable for detecting the effect of the compounds of the present disclosure. These *in vitro* or *in vivo* biological assays can include, but are not limited to, enzymatic activity assays, electrophoretic mobility shift assays, reporter gene assays, *in vitro* cell viability assays, and the assays described herein.

### Pharmaceutical Compositions

In some aspects, the present disclosure provides a pharmaceutical composition comprising a compound of the present disclosure as an active ingredient. In some embodiments, the present disclosure provides a pharmaceutical composition comprising at least one compound of each of the formulae described herein, or a pharmaceutically acceptable salt or solvate thereof, and one or more pharmaceutically acceptable carriers or excipients. In some embodiments, the present disclosure provides a pharmaceutical composition comprising at least one compound selected from **Table 5**.

As used herein, the term "composition" is intended to encompass a product comprising the specified ingredients in the specified amounts, as well as any product which results, directly or indirectly, from combination of the specified ingredients in the specified amounts.

The compounds of present disclosure can be formulated for oral administration in forms such as tablets, capsules (each of which includes sustained release or timed release formulations), pills, powders, granules, elixirs, tinctures, suspensions, syrups and emulsions. The compounds of present disclosure on can also be formulated for intravenous (bolus or in-fusion), intraperitoneal, topical, subcutaneous, intramuscular or transdermal (*e.g.*, patch) administration, all using forms well known to those of ordinary skill in the pharmaceutical arts.

The formulation of the present disclosure may be in the form of an aqueous solution comprising an aqueous vehicle. The aqueous vehicle component may comprise water and at least one pharmaceutically acceptable excipient. Suitable acceptable excipients include those selected from the group consisting of a solubility enhancing agent, chelating agent, preservative, tonicity agent, viscosity/suspending agent, buffer, and pH modifying agent, and a mixture thereof.

Any suitable solubility enhancing agent can be used. Examples of a solubility enhancing agent include cyclodextrin, such as those selected from the group consisting of hydroxypropyl-β-cyclodextrin, methyl-β-cyclodextrin, randomly methylated-β-cyclodextrin, ethylated-β-cyclodextrin, triacetyl-β-cyclodextrin, peracetylated-β-cyclodextrin, carboxymethyl-β-cyclodextrin, hydroxyethyl-β-cyclodextrin, 2-hydroxy-3-(trimethylammonio)propyl-β-cyclodextrin, glucosyl-β-cyclodextrin, sulfated β-cyclodextrin (S-β-CD), maltosyl-β-cyclodextrin, β-cyclodextrin sulfobutyl ether, branched-β-cyclodextrin, hydroxypropyl-γ-cyclodextrin, randomly methylated-γ-cyclodextrin, and trimethyl-γ-cyclodextrin, and mixtures thereof.

Any suitable chelating agent can be used. Examples of a suitable chelating agent include those selected from the group consisting of ethylenediaminetetraacetic acid and metal salts thereof, disodium edetate, trisodium edetate, and tetrasodium edetate, and mixtures thereof.

Any suitable preservative can be used. Examples of a preservative include those selected from the group consisting of quaternary ammonium salts such as benzalkonium halides (preferably benzalkonium chloride), chlorhexidine gluconate, benzethonium chloride, cetyl pyridinium chloride, benzyl bromide, phenylmercury nitrate, phenylmercury acetate, phenylmercury neodecanoate, merthiolate, methylparaben, propylparaben, sorbic acid, potassium sorbate, sodium benzoate, sodium propionate, ethyl p-hydroxybenzoate, propylaminopropyl biguanide, and butyl-p-hydroxybenzoate, and sorbic acid, and mixtures thereof.

In some embodiments, examples of a preservative include those selected from the group consisting of quaternary ammonium salts such as benzalkonium halides (preferably benzalkonium chloride), chlorhexidine gluconate, benzethonium chloride, cetyl pyridinium chloride, benzyl bromide, phenylmercury nitrate, merthiolate, methylparaben, propylparaben, sorbic acid, potassium sorbate, sodium benzoate, sodium propionate, ethyl p-hydroxybenzoate, propylaminopropyl biguanide, and butyl-p-hydroxybenzoate, and sorbic acid, and mixtures thereof.

The aqueous vehicle may also include a tonicity agent to adjust the tonicity (osmotic pressure). The tonicity agent can be selected from the group consisting of a glycol (such as propylene glycol, diethylene glycol, triethylene glycol), glycerol, dextrose, glycerin, mannitol, potassium chloride, and sodium chloride, and a mixture thereof. In some embodiments, the tonicity agent is selected from the group consisting of a glycol (such as propylene glycol, triethylene glycol), glycerol, dextrose, glycerin, mannitol, potassium chloride, and sodium chloride, and a mixture thereof.

The aqueous vehicle may also contain a viscosity/suspending agent. Suitable viscosity/suspending agents include those selected from the group consisting of cellulose derivatives, such as methyl cellulose, ethyl cellulose, hydroxyethylcellulose, polyethylene glycols (such as polyethylene glycol 300, polyethylene glycol 400), carboxymethyl cellulose, hydroxypropylmethyl cellulose, and cross-linked acrylic acid polymers (carbomers), such as polymers of acrylic acid cross-linked with polyalkenyl ethers or divinyl glycol (Carbopols - such as Carbopol 934, Carbopol 934P, Carbopol 971, Carbopol 974 and Carbopol 974P), and a mixture thereof.

In order to adjust the formulation to an acceptable pH (typically a pH range of about 5.0 to about 9.0, more preferably about 5.5 to about 8.5, particularly about 6.0 to about 8.5, about 7.0 to about 8.5, about 7.2 to about 7.7, about 7.1 to about 7.9, or about 7.5 to about 8.0), the formulation may contain a pH modifying agent. The pH modifying agent is typically a mineral acid or metal hydroxide base, selected from the group of potassium hydroxide, sodium hydroxide, and hydrochloric acid, and mixtures thereof, and preferably sodium hydroxide and/or hydrochloric acid. These acidic and/or basic pH modifying agents are added to adjust the formulation to the target acceptable pH range. Hence it may not be necessary to use both acid and base - depending on the formulation, the addition of one of the acid or base may be sufficient to bring the mixture to the desired pH range.

The aqueous vehicle may also contain a buffering agent to stabilize the pH. When used, the buffer is selected from the group consisting of a phosphate buffer (such as sodium dihydrogen phosphate and disodium hydrogen phosphate), a borate buffer (such as boric acid, or salts thereof including disodium tetraborate), a citrate buffer (such as citric acid, or salts thereof including sodium citrate), and ε-aminocaproic acid, and mixtures thereof.

The formulation may further comprise a wetting agent. Suitable classes of wetting agents include those selected from the group consisting of polyoxypropylene-polyoxyethylene block copolymers (poloxamers), polyethoxylated ethers of castor oils, polyoxyethylenated sorbitan esters (polysorbates), polymers of oxyethylated octyl phenol (Tyloxapol), polyoxyl 40 stearate, fatty acid glycol esters, fatty acid glyceryl esters, sucrose fatty esters, and polyoxyethylene fatty esters, and mixtures thereof.

Oral compositions generally include an inert diluent or an edible pharmaceutically acceptable carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

According to a further aspect of the disclosure there is provided a pharmaceutical composition which comprises a compound of the disclosure as defined hereinbefore, or a pharmaceutically acceptable salt, hydrate or solvate thereof, in association with a pharmaceutically acceptable diluent or carrier.

In some embodiments, a pharmaceutical composition described herein may further comprise one or more additional pharmaceutically active agents.

The compositions of the disclosure may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular, intraperitoneal or intramuscular dosing or as a suppository for rectal dosing).

The compositions of the disclosure may be obtained by conventional procedures using conventional pharmaceutical excipients, well known in the art. Thus, compositions intended for oral use may contain, for example, one or more coloring, sweetening, flavoring and/or preservative agents.

A therapeutically effective amount of a compound of the present disclosure for use in therapy is an amount sufficient to treat or prevent a MLL related condition referred to herein, slow its progression and/or reduce the symptoms associated with the condition.

A therapeutically effective amount of a compound of the present disclosure for use in therapy is an amount sufficient to treat an MLL related condition referred to herein, slow its progression and/or reduce the symptoms associated with the condition.

The size of the dose for therapeutic or prophylactic purposes of a compound of Formula (I) will naturally vary according to the nature and severity of the conditions, the age and sex of the animal or subject and the route of administration, according to well-known principles of medicine.

### Methods of Use

In some aspects, the present disclosure provides a method of inhibiting the interaction of menin with MLL (e.g., *in vitro* or *in vivo*), comprising contacting a cell with a therapeutically effective amount of a compound of the present disclosure or a pharmaceutically acceptable salt thereof.

In some aspects, the present disclosure provides a method of treating or preventing a disease or disorder disclosed herein in a subject in need thereof, comprising administering to the subject a therapeutically effective amount of a compound of the present disclosure or a pharmaceutically acceptable salt thereof, or a pharmaceutical composition of the present disclosure.

In some aspects, the present disclosure provides a method of treating a disease or disorder disclosed herein in a subject in need thereof, comprising administering to the subject a therapeutically effective amount of a compound of the present disclosure or a pharmaceutically acceptable salt thereof, or a pharmaceutical composition of the present disclosure.

In some embodiments, the disease or disorder is associated with MLL. In some embodiments, the disease or disorder is a disease or disorder in which menin-MLL binding is implicated.

The compounds of the invention are inhibitors of the interaction of menin with MLL and MLL fusion proteins. In some embodiments, the present invention is directed to a method of inhibiting the interaction between menin and MLL or an MLL fusion protein by contacting menin and MLL or the MLL fusion protein with a compound of the invention. The contacting can be carried out in vitro or in vivo. In some embodiments, the compounds of the invention can bind to menin, thereby interfering with the binding of MLL to menin. In some embodiments, the present invention provides a method of inhibiting the activity of menin by contacting menin with a compound of the invention in the presence of MLL or an MLL fusion protein. In further embodiments, the present invention provides a method of inhibiting the binding of MLL or an MLL fusion protein to menin, comprising contacting menin with a compound of the invention in the presence of the MLL or MLL fusion protein.

The compounds of the invention are also useful in treating diseases associated with the menin-MLL interaction or menin-MLL fusion protein interaction. For example, diseases and conditions treatable according to the methods of the invention include cancer, such as leukemia, and other diseases or disorders mediated by the menin-MLL interaction or menin- MLL fusion protein interaction such as diabetes.

In some embodiments, the disease or disorder is selected from the group consisting of a leukemia, hematologic malignancy, solid tumor cancer, prostate cancer, breast cancer, liver cancer, brain tumor, and diabetes. In some embodiments, the leukemia is selected from the group consisting of AML, ALL, Mixed Lineage Leukemia, and a leukemia with Partial Tandem Duplications of MLL.

In some embodiments, the disease or disorder is a cancer.

In some embodiments, the cancer is selected from hematological cancer (e.g., leukemia and lymphoma), bladder cancer, brain cancer (e.g., glioma), diffuse intrinsic pontine glioma (DIPG)), breast cancer (e.g., triple-negative breast cancer), colorectal cancer, cervical cancer, gastrointestinal cancer (e.g., colorectal carcinoma, gastric cancer), genitourinary cancer, head and neck cancer, liver cancer, lung cancer, melanoma, ovarian cancer, pancreatic cancer, prostate cancer, renal cancer (e.g., renal cell carcinoma), skin cancer, thyroid cancer (e.g., papillary thyroid carcinoma), testicular cancer, sarcoma (e.g., Ewing's sarcoma), and AIDS- related cancers. In some embodiments, cancer is selected from cardiac cancers, such as for example, sarcoma (e.g., angiosarcoma, fibrosarcoma, rhabdomyosarcoma, and liposarcoma), myxoma, rhabdomyoma, fibroma, lipoma and teratoma; lung cancers, including, for example, bronchogenic carcinoma (e.g., squamous cell, undifferentiated small cell, undifferentiated large cell, and adenocarcinoma), alveolar and bronchiolar carcinoma, bronchial adenoma, sarcoma, lymphoma, chondromatous hamartoma, mesothelioma, non-small cell lung cancer, small cell lung cancer, bronchial adenomas/carcinoids, and pleuropulmonary blastoma; gastrointestinal cancer, including, for example, cancers of the esophagus (e.g., squamous cell carcinoma, adenocarcinoma, leiomyosarcoma, and lymphoma), cancers of the stomach (e.g., carcinoma, lymphoma, and leiomyosarcoma), cancers of the pancreas (e.g., ductal adenocarcinoma, insulinoma, glucagonoma, gastrinoma, carcinoid tumors, and vipoma), cancers of the small bowel (e.g., adenocarcinoma, lymphoma, carcinoid tumors, Kaposi's sarcoma, leiomyoma, hemangioma, lipoma, neurofibroma, and fibroma), cancers of the large bowel or colon, (e.g., adenocarcinoma, tubular adenoma, villous adenoma, hamartoma, and leiomyoma), and other cancers of the digestive tract (e.g., anal cancer, anorectal cancer, appendix cancer, cancer of the anal canal, cancer of the tongue, gallbladder cancer, gastrointestinal stromal tumor (GIST), colon cancer, colorectal cancer, extrahepatic bile duct cancer, intrahepatic bile duct cancer, rectal cancer, and small intestine cancer); genitourinary tract cancers, including, for example, cancers of the kidney (e.g., adenocarcinoma, Wilm's tumor (nephroblastoma), lymphoma, and leukemia), cancers of the bladder and urethra (e.g., squamous cell carcinoma, transitional cell carcinoma, and adenocarcinoma), cancers of the prostate (e.g., adenocarcinoma and sarcoma), cancers of the testis, (e.g., seminoma, teratoma, embryonal carcinoma, teratocarcinoma, choriocarcinoma, sarcoma, interstitial cell carcinoma, fibroma, fibroadenoma, adenomatoid tumors, and lipoma), as well as transitional cell cancer, transitional cell cancer of the renal pelvis and ureter and other urinary organs, urethral cancer, and urinary bladder cancer; liver cancers, including, for example, hepatoma (e.g., hepatocellular carcinoma), cholangiocarcinoma, hepatoblastoma, angiosarcoma, hepatocellular adenoma, and hemangioma; bone cancers, including, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochrondroma (osteocartilaginous exostoses), benign chondroma, chondroblastoma, chondromyxofibroma, osteoid osteoma and giant cell tumors; nervous system cancers, including, for example, cancers of the skull (e.g., osteoma, hemangioma, granuloma, xanthoma, and osteitis deformans); cancers of the meninges (e.g., meningioma, meningiosarcoma, and gliomatosis); cancers of the brain (e.g., astrocytoma, medulloblastoma, glioma, ependymoma, germinoma (pinealoma), glioblastoma multiforme, oligodendroglioma, schwannoma, retinoblastoma, and congenital tumors); cancers of the spinal cord (e.g., neurofibroma, meningioma, glioma, and sarcoma), and other nervous system cancers (e.g., brain stem glioma, diffuse intrinsic pontine glioma (DIPG), brain tumor, central nervous system cancer, cerebellar astrocytoma, cerebral astrocytoma/malignant glioma, childhood cerebellar astrocytoma, childhood cerebral astrocytoma, primary central nervous system lymphoma, visual pathway and hypothalamic glioma, nervous system lymphoma, supratentorial primitive neuroectodeimal tumors, pineoblastoma and supratentorial primitive neuroectodermal tumors); gynecological cancers, including, for example, cancers of the uterus (e.g., endometrial carcinoma), cancers of the cervix (e.g., cervical carcinoma, and pre tumor cervical dysplasia), cancers of the ovaries (e.g., ovarian carcinoma, including serous cystadenocarcinoma, mucinous cystadenocarcinoma, unclassified carcinoma, granulosa thecal cell tumors, Sertoli Leydig cell tumors, dysgerminoma, and malignant teratoma), cancers of the vulva (e.g., squamous cell carcinoma, intraepithelial carcinoma, adenocarcinoma, fibrosarcoma, and melanoma), cancers of the vagina (e.g., clear cell carcinoma, squamous cell carcinoma, botryoid sarcoma, and embryonal rhabdomyosarcoma), and cancers of the fallopian tubes (e.g., carcinoma); other reproductive tract cancers, including, for example, endometrial cancer, endometrial uterine cancer, germ cell tumor, gestational trophoblastic tumor, gestational trophoblastic tumor glioma, ovarian epithelial cancer, ovarian germ cell tumor, ovarian low malignant potential tumor, penile cancer, vaginal cancer, vulvar cancer, extracranial germ cell tumor, extragonadal germ cell tumor, uterine cancer, uterine corpus cancer, uterine sarcoma; lymphatic and hematologic cancers, including, for example, cancers of the blood (e.g., acute myeloid leukemia (AML), chronic myeloid leukemia (CML), acute lymphoblastic leukemia (ALL), chronic lymphoblastic leukemia, chronic lymphocytic leukemia, myeloproliferative diseases, multiple myeloma, and myelodysplastic syndrome, Hodgkin's lymphoma, non-Hodgkin's lymphoma (malignant lymphoma) and Waldenstrom's macroglobulinemia), and other lymphatic or hematologic cancers including, for example, childhood leukemia, myeloproliferative disorders (e.g., primary myelofibrosis), plasma cell neoplasm/multiple myeloma, myelodysplasia, myelodysplastic syndrome, cutaneous T-cell lymphoma, lymphoid neoplasm, AIDS-related lymphoma, thymoma, thymoma and thymic carcinoma, mycosis fungoides, and Sezary Syndrome; skin cancers, including, for example, malignant melanoma, basal cell carcinoma, squamous cell carcinoma, Kaposi's sarcoma, moles dysplastic nevi, lipoma, angioma, dermatofibroma, keloids, psoriasis, merkel cell carcinoma, merkel cell skin carcinoma, melanoma, and carcinoid tumor; adrenal gland cancers, including, for example, neuroblastoma; other cancers associated with the endocrine system including, for example, adrenocortical carcinoma, multiple endocrine neoplasia (e.g., multiple endocrine neoplasia type I), multiple endocrine neoplasia syndrome, parathyroid cancer, pituitary tumor, pheochromocytoma, islet cell pancreatic cancer, and islet cell tumors); connective tissue cancer (e.g., bone cancer, bone and joint cancer, osteosarcoma and malignant fibrous histiocytoma); cancer associated with the head, neck, and mouth (e.g., head and neck cancer, paranasal sinus and nasal cavity cancer, metastatic squamous neck cancer, mouth cancer, throat cancer, esophageal cancer, laryngeal cancer, pharyngeal cancer, hypopharyngeal cancer, lip and oral cavity cancer, nasopharyngeal cancer, oral cancer, oropharyngeal cancer, and salivary gland cancer); and cancer associated with the eye (e.g., ocular cancer, intraocular melanoma). In some embodiments, the cancer is Ewing' s sarcoma.

In some embodiments, the cancer is a hematological cancer such as leukemia or lymphoma. Example leukemia and lymphomas treatable by the compounds of the invention include mixed lineage leukemia (MLL), MLL-related leukemia, MLL-associated leukemia, MLL-positive leukemia, MLL-induced leukemia, rearranged mixed lineage leukemia (MLL- r), leukemia associated with a MLL rearrangement or a rearrangement of the LJ gene, acute leukemia, chronic leukemia, indolent leukemia, lymphoblastic leukemia, lymphocytic leukemia, myeloid leukemia, myelogenous leukemia, childhood leukemia, acute lymphocytic leukemia (ALL) (also referred to as acute lymphoblastic leukemia or acute lymphoid leukemia), acute myeloid leukemia (AML) (also referred to as acute myelogenous leukemia or acute myeloblastic leukemia), acute granulocytic leukemia, acute nonlymphocytic leukemia, chronic lymphocytic leukemia (CLL) (also referred to as chronic lymphoblastic leukemia), chronic myelogenous leukemia (CML) (also referred to as chronic myeloid leukemia), therapy related leukemia, myelodysplastic syndrome (MDS), myeloproliferative disease (MPD) (such as primary myelofibrosis (PMF)), myeloproliferative neoplasia (MPN), plasma cell neoplasm, multiple myeloma, myelodysplasia, cutaneous T-cell lymphoma, lymphoid neoplasm, AIDS-related lymphoma, thymoma, thymic carcinoma, mycosis fungoides, Alibert-Bazin syndrome, granuloma fungoides, Sezary Syndrome, hairy cell leukemia, T-cell prolymphocytic leukemia (T-PLL), large granular lymphocytic leukemia, meningeal leukemia, leukemic leptomeningitis, leukemic meningitis, multiple myeloma, Hodgkin's lymphoma, non-Hodgkin's lymphoma (malignant lymphoma), and Waldenstrom's macroglobulinemia.

In some embodiments, diseases and conditions treatable with compounds of the invention include insulin resistance, pre-diabetes, diabetes (e.g., Type 2 diabetes or Type 1 diabetes), and risk of diabetes. In some embodiments, diseases and conditions treatable with compounds of the invention include hyperglycemia. In some embodiments, the hyperglycemia is associated with diabetes, such as Type 2 diabetes. In some embodiments, compounds of the invention are used to treat loss of response to other anti-diabetic agents and/or reduced beta cell function in a patient or subject. In some embodiments, compounds of the invention are used to restore response to other anti -diabetic agents and/or to restore beta cell function and/or to reduce the need for insulin in a patient or subject. In some embodiments, compounds of the invention are used to reduce insulin resistance, reduce the risk of diabetes, or reduce increases in blood glucose caused by a statin in a subject taking a statin. In some embodiments, compounds of the invention are used to treat diabetes in a subject taking a statin or to prevent diabetes in a subject taking a statin. Methods of the invention include decreasing, reducing, inhibiting, suppressing, limiting or controlling in the subject elevated blood glucose levels. In further aspects, methods of the invention include increasing, stimulating, enhancing, promoting, inducing or activating in the subject insulin sensitivity. Statins include, but are not limited to atorvastatin, cerivastatin, fluvastatin, lovastatin, mevastatin, pitavastatin, pravastatin, rosuvastatin and simvastatin.

In some aspects, the present disclosure provides a method of treating or preventing a cancer in a subject in need thereof, comprising administering to the subject a therapeutically effective amount of a compound of the present disclosure or a pharmaceutically acceptable salt thereof, or a pharmaceutical composition of the present disclosure.

In some aspects, the present disclosure provides a method of treating a cancer in a subject in need thereof, comprising administering to the subject a therapeutically effective amount of a compound of the present disclosure or a pharmaceutically acceptable salt thereof, or a pharmaceutical composition of the present disclosure.

In some aspects, the present disclosure provides a compound of the present disclosure or a pharmaceutically acceptable salt thereof for use in inhibiting the interaction of menin with MLL (e.g., *in vitro* or *in vivo*).

In some aspects, the present disclosure provides a compound of the present disclosure or a pharmaceutically acceptable salt thereof for use in treating or preventing a disease or disorder disclosed herein.

In some aspects, the present disclosure provides a compound of the present disclosure or a pharmaceutically acceptable salt thereof for use in treating a disease or disorder disclosed herein.

In some aspects, the present disclosure provides a compound of the present disclosure or a pharmaceutically acceptable salt thereof for use in treating or preventing a cancer in a subject in need thereof.

In some aspects, the present disclosure provides a compound of the present disclosure or a pharmaceutically acceptable salt thereof for use in treating a cancer in a subject in need thereof.

In some aspects, the present disclosure provides use of a compound of the present disclosure or a pharmaceutically acceptable salt thereof in the manufacture of a medicament for inhibiting the interaction of menin with MLL (e.g., *in vitro* or *in vivo*).

In some aspects, the present disclosure provides use of a compound of the present disclosure or a pharmaceutically acceptable salt thereof in the manufacture of a medicament for treating or preventing a disease or disorder disclosed herein.

In some aspects, the present disclosure provides use of a compound of the present disclosure or a pharmaceutically acceptable salt thereof in the manufacture of a medicament for treating a disease or disorder disclosed herein.

In some aspects, the present disclosure provides use of a compound of the present disclosure or a pharmaceutically acceptable salt thereof in the manufacture of a medicament for treating or preventing a cancer in a subject in need thereof.

In some aspects, the present disclosure provides use of a compound of the present disclosure or a pharmaceutically acceptable salt thereof in the manufacture of a medicament for treating a cancer in a subject in need thereof.

The present disclosure provides compounds that function as inhibitors of the interaction of menin with MLL (e.g., *in vitro* or *in vivo*). The present disclosure therefore provides a method of inhibiting the interaction of menin with MLL *in vitro* or *in vivo*, said method comprising contacting a cell with a therapeutically effective amount of a compound, or a pharmaceutically acceptable salt thereof, as defined herein.

In some embodiments, the menin/MLL interaction inhibitor is a compound of the present disclosure.

Effectiveness of compounds of the disclosure can be determined by industry-accepted assays/ disease models according to standard practices of elucidating the same as described in the art and are found in the current general knowledge.

The present disclosure also provides a method of treating a disease or disorder in which interaction of menin with MLL is implicated in a subject in need of such treatment, said method comprising administering to said subject a therapeutically effective amount of a compound, or a pharmaceutically acceptable salt thereof, or a pharmaceutical composition as defined herein.

In some embodiments, the subject is a mammal. In some embodiments, the subject is a human.

### Routes of Administration

The compounds of the disclosure or pharmaceutical compositions comprising these compounds may be administered to a subject by any convenient route of administration, whether systemically/ peripherally or topically (i.e., at the site of desired action).

Routes of administration include, but are not limited to, oral (e.g. by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eye drops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intra-arterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

Abbreviations used in the following examples and elsewhere herein are:
- ACN: acetonitrile
- AcOH: acetic acid
- anh.: anhydrous
- aq.: aqueous
- Boc₂O: di-*tert-*butyl dicarbonate
- br.: broad
- BSA: bovine serum albumin
- d: duplet
- DBU: 1,8-diazabicyclo[5.4.0]undec-7-ene
- DCE: dichloroethane
- DCM: dichloromethane
- DIPEA: *N,N-*diisopropylethylamine
- DMAA: 1,3-dimethylamylamine
- DMAP: 4-(dimethylamino)pyridine
- DMEM: Dulbecco's modified Eagle's medium
- DMF: *N,N-*dimethyl formamide
- DMSO: dimethyl sulfoxide
- DTT: dithiothreitol
- FBS: fetal bovine serum
- h: hour(s)
- HATU: (1-[bis(dimethylamino)methylene]-1*H-*1,2,3-triazolo[4,5-*b*]pyridinium 3-oxide hexafluorophosphate
- HPLC: high pressure (or performance) liquid chromatography
- IPA: *iso*-propyl alcohol
- LAH: lithium aluminium hydride
- LCMS: liquid chromatography mass spectrometry
- m: multiplet
- M: molar
- MHz: megahertz
- min: minutes
- Ms: Mesyl
- NBS: *N-*bromosuccinimide
- NMR: nuclear magnetic resonance
- Pd₂(dba)₃: tris(dibenzylideneacetone)dipalladium(0)
- Pd(dppf)Cl₂: [1,1'-bis(diphenylphosphino)ferrocene]dichloropalladium(II)
- PyBOP q: quadruplet
- rt: room temperature
- s: singlet
- sat.: saturated
- STAB: sodium triacetoxyborohydride
- t: temperature, triplet
- T3P: Propylphosphonic anhydride
- TEA: Triethyl amine
- TFA: trifluoroacetic acid
- THF: tetrahydrofuran
- TLC: thin layer chromatography
- TRIS: 2-amino-2-(hydroximethyl)propane-1,3-diol
- TsOH: toluenesulfonic acid (e.g., *p*-TsOH)

### EXAMPLES

### General synthetical procedures and examples of the compound's preparation.

### Synthesis of Building Blocks

### Synthesis of 4-chloro-6-(2,2,2-trifluoroethyl)quinazoline (P7)

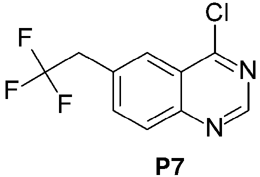

### Preparation 1. Methyl 5-bromo-2-[(tert-butoxycarbonyl)amino]benzoate (P1)

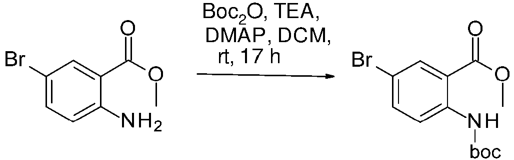

To solution of methyl 2-amino-5-bromobenzoate (25 g, 0.109 mol) in DCM (250 ml) was added Boc₂O (26.5 g, 0.122 mol), triethylamine (62 g, 0.61 mol) and DMAP (4 g, 0.033 mol). The solution was stirred at ambient temperature for 17 h. Then 250 ml of water was added, and potassium hydrosulfate was added in small portions with stirring until pH reached 3. Water phase was washed with dichloromethane (200 ml), organic solution was concentrated, and residue was purified using silica gel column chromatography (30% dichloromethane in hexane). 5-Bromo-2-[(*tert*-butoxycarbonyl)amino]benzoate (**P1**) was isolated as white solid (14.3 g, 40%). ¹H NMR (400 MHz, DMSO-*d₆*), δ: 10.02 (s, 1H), 8.10 (d, *J* = 9.0 Hz, 1H), 7.98 (d, *J* = 2.4 Hz, 1H), 7.76 (d, *J* = 9.0, 2.4 Hz, 1H), 3.84 (s, 3H), 1.43 (s, 9H).

### Preparation 2. Methyl 2-[(tert-butoxycarbonyl)amino]-5-(4,4,5,5-tetramethyl-1,3,2-dioxaborolan-2-yl)benzoate (P2)

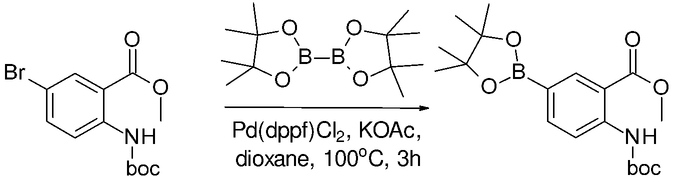

5-Bromo-2-[(*tert*-butoxycarbonyl)amino]benzoate (**P1**, 14.3 g, 0.043 mol) and bis(pinacolato)diboron (22 g, 0.087 mol) were dissolved in dioxane (120 ml). Potassium acetate (12.6 g, 0.129 mol) was added to the solution. The mixture was stirred for 30 min at 70°C under argon atmosphere. Then Pd(dppf)Cl₂ (3.2 g) was added, and reaction mixture was stirred at 100°C for additional 3 h (TLC control). The mixture was cooled down to rt and filtered through a pad of celite. Filtrate was then concentrated, and residue was purified by silica gel column chromatography (dichloromethane) to give the product (**P2**) as a white solid (16 g, 99 % yield). ¹H NMR (400 MHz, DMSO-*d₆*), δ: 10.31 (s, 1H), 8.26 (d, J = 8.8 Hz, 2H), 7.83 (d, J = 8.4 Hz, 1H), 3.87 (s, 3H), 1.48 (s, 9H), 1.29 (s, 12H).

### Preparation 3. Methyl 2-[(tert-butoxycarbonyl)amino]-5-(2,2,2-trifluoroethyl)benzoate (P3)

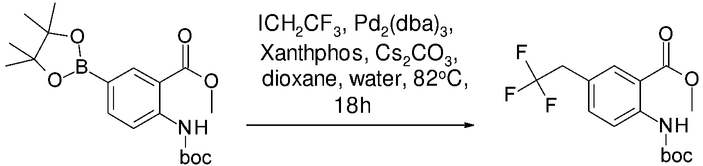

Methyl 2-[(*tert*-butoxycarbonyl)amino]-5-(4,4,5,5-tetramethyl-1,3,2-dioxaborolan-2-yl)benzoate (**P2**, 11.2 g, 30 mmol) was dissolved in dioxane (100 ml), cesium carbonate (32.6 g, 118 mmol) in water (8 ml) was added. The mixture was stirred 30 min at 70°C under argon atmosphere. Then 1,1,1-trifluoro-2-iodo-ethane (18.6 g, 89 mmol) , Pd₂(dba)₃ (2.6 g), and Xantphos (2.6 g) were added. The reaction mixture was stirred at 82°C for 18 h, then the mixture was cooled down and filtered through a pad of celite. The filtrate was concentrated. The residue was purified by silica gel column chromatography (dichloromethane-hexane - 1 : 1) to give the product **(P3)** as a white solid (5.85 g, 59% yield). ¹H NMR (400 MHz, DMSO-*d₆*), δ: 10.10 (s, 1H), 8.20 (t, *J* = 20.8 Hz, 1H), 7.92 (s, 1H), 7.50 (d, *J* = 23.8 Hz, 1H), 3.86 (s, 3H), 3.74 - 3.48 (m, 2H), 1.48 (s, 9H).

### Preparation 4. Methyl 2-amino-5-(2,2,2-trifluoroethyl)benzoate (P4)

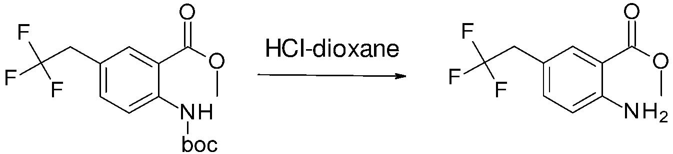

3M Solution of HCl in dioxane was added to methyl 2-[(tert-butoxycarbonyl)amino]-5-(2,2,2-trifluoroethyl)benzoate **(P3,** 5.85 g, 17.6 mmol). Reaction mixture was stirred at rt for 2 h (NMR control) and concentrated to dryness to give the product **(P4,** 4.6 g, 100 %) as hydrochloride. ¹H NMR (400 MHz, DMSO-*d₆*), δ: 7.62-7.70 (m, 1H), 7.19-7.27 (m, 1H), 6.86-6.84 (m, 1 H), 3.79 (s, 3 H), 4.03 - 3.58 (m, 2H).

### Preparation 5. 2-Amino-5-(2,2,2-trifluoroethyl)benzoic acid (P5)

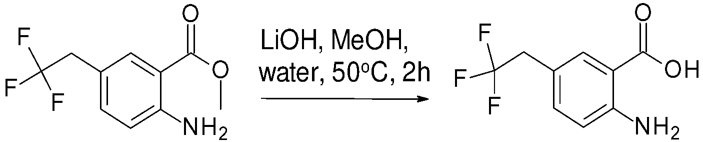

Lithium hydroxide (2 g, 83 mmol) in water (35 ml) was added to the solution of methyl 2-amino-5-(2,2,2-trifluoroethyl)benzoate (**P4**, 4.6 g, 17.6 mmol) in methanol (100 ml). The reaction mixture was stirred at 50°C for 2 h. Then reaction mixture was cooled, concentrated to dryness, and water (50 ml) was added. Concentrated HCl was added to the obtained solution with stirring and cooling with cold water up to pH=3. Obtained precipitate was filtered off, washed with water (15 ml) and dried to give the product **P5** as a white solid (3 g , 78%). ¹H NMR (400 MHz, DMSO-*d₆*), δ: 7.62-7,70 (s, 1H), 7.23 (d, *J* = 8.3 Hz, 1H), 6.81 (d, *J* = 8.1 Hz, 1H), 3.55 - 3.33 (m, 2H).

### Preparation 6. 6-(2,2,2-Trifluoroethyl)quinazolin-4(3H)-one (P6)

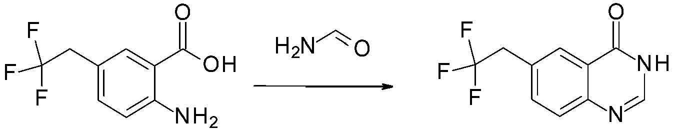

2-Amino-5-(2,2,2-trifluoroethyl)benzoic acid (**P5**, 3 g, 13.7 mmol) and formamide (2.2 g, 49 mmol) were stirred in vial at 155°C for 1.5 h, and then at 165°C for 40 min. The reaction mixture was cooled, saturated solution of sodium bicarbonate (50 ml) was added, and the mixture stirred at rt for 30 min. Formed precipitate was filtered off , washed with water (55 ml) and dried to give the product **P6** as a white solid (2.52 g, 85 %). ¹H NMR (400 MHz, DMSO-*d₆*), δ: 12.30 (s, 1H), 8.4 - 8,16 (m, 1H), 8,07-8,16 (m, 1H), 7.78 (d, *J* = 8.6 Hz, 1H),7.68 (d, J=7.8 Hz, 1H), 3.79 - 3.90 (m, 2H).

### Preparation 7. 4-Chloro-6-(2,2,2-trifluoroethyl)quinazoline (P7)

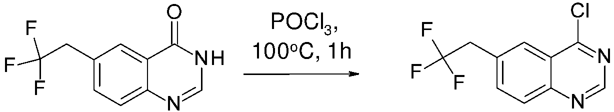

POCl₃ (35 ml) was added to 6-(2,2,2-trifluoroethyl)quinazolin-4(3*H*)-one (**P6**, 2.52 g, 11 mmol), the reaction mixture was stirred and refluxed for 1 h. The solution was cooled down and poured to ice-cold water solution of sodium bicarbonate. The mixture was stirred for 30 min, then extracted with DCM (2x50 ml). After evaporation of dichloromethane, residue was purified by silica gel column chromatography (dichloromethane-ethyl acetate - 10 : 1) to give the product (**P7**, 1.9 g 70 %) as a brown solid. ¹H NMR (400 MHz, CDCl₃), δ: 9.12-9.07 (m, 1H), 8.25-8.22 (m, 1H), 8.12 (d, *J =* 8.3 Hz, 1H), 7.93 (d, *J =* 8.1, Hz, 1H), 3.70 - 3.60 (m, 2H).

### Synthesis of 4-(2,7-diazaspiro[3.5]non-2-yl)-6-(2,2,2-trifluoroethyl)quinazoline (P9)

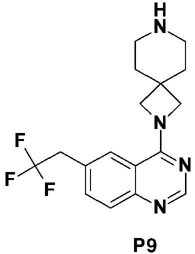

### Preparation 8. tert-Butyl 2-[6-(2,2,2-trifluoroethyl)quinazolin-4-yl]-2,7-diazaspiro[3.5]nonane-7-carboxylate (P8)

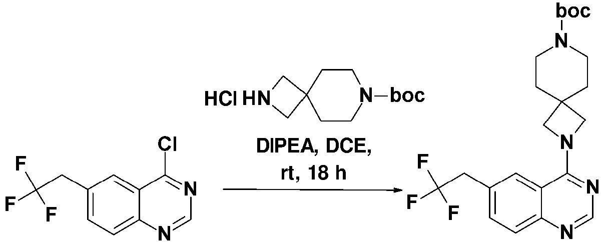

DIPEA (7/9 g, 61 mmol) and *tert-butyl* 2,7-diazaspiro[3.5]nonane-7-carboxylate hydrochloride (3.2 g, 12.2 mmol) were added to a solution of 4-chloro-6-(2,2,2-trifluoroethyl)quinazoline **(P7,** 3 g, 12.2 mmol) in dichloroethane (60 ml). The reaction solution was stirred at rt for 18 h (LCMS control). Then product was purified by column chromatography on silica (ethyl acetate - methanol 10:1) to give **P8** as a white solid (5.1 g, 96%). ¹H NMR (400 MHz, DMSO-*d₆*), δ: 8.47-8.43 (m, 1H), 7.99-7.95 (m, 1H), 7.68 (m, 2H), 4.65-3.96 (m, 4H), 3.96-3.75 (m, 2H), 3.48-3.40 (m, 4H), 2.63-2.48 (m,4H),1.86 - 1.63 (m, 4H), 1.41 (s, 9H).

### Preparation 9. 4-(2,7-Diazaspiro[3.5]non-2-yl)-6-(2,2,2-trifluoroethyl)quinazoline (P9)

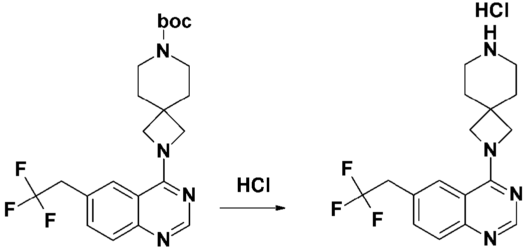

To *tert-*butyl 2-[6-(2,2,2-trifluoroethyl)quinazolin-4-yl]-2,7-diazaspiro[3.5]nonane-7-carboxylate (**P8**, 5.1g, 11.7 mmol) was added dioxane-HCl solution (3 M, 120 ml). The reaction mixture was stirred at rt for 3 h (LCMS control), then evaporated under vacuum to dryness to give 4-(2,7-diazaspiro[3.5]non-2-yl)-6-(2,2,2-trifluoroethyl)quinazoline hydrochloride (**P9**, 4.4 g, 100%) as a white solid. ¹H NMR (400 MHz, DMSO-*d₆*), δ: 9.44 (s,1H), 9.29 (s,1H), 8.85-8.80 (m, 1H), 8.14-8.08 (m, 1H), 8.06-7.98 (m, 2H), 4.90-4.76 (m, 2H), 4.38-4.24 (m,2H), 4.03-3.89 (m,2H), 3.18-2.97 (m, 4H), 2.20-2.02 (m, 4H).

### Synthesis of 4-(2,7-diazaspiro[3.5]nonan-2-yl)-6-(trifluoromethoxy)quinazoline (P11)

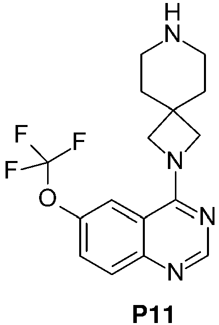

### Preparation 10. tert-Butyl 2-[6-(trifluoromethoxy)quinazolin-4-yl]-2,7-diazaspiro[3.5]nonane-7-carboxylate (P10)

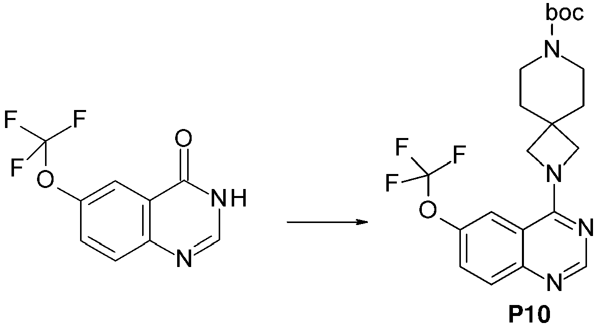

To a solution of 6-(trifluoromethoxy)quinazolin-4(3*H*)-one (230 mg, 1 mmol, prepared as described in US2005/54626, 2005, A1) and *tert-*butyl 2,7-diazaspiro[3.5]nonane-7-carboxylate (263 mg, 1 mmol) in DMAA (4 ml) were added DBU (610 mg, 4 mmol), and PyBOP (564 mg, 1.5 mmol). Reaction mixture was stirred at 50°C for 48 h, then water (30 ml) was added, and product was extracted with ethylacetate (2x5 ml). Extract was washed with brine, concentrated. Flash chromatography on silica gel with ethylacetate gives *tert-*butyl 2-[6-(trifluoromethoxy)quinazolin-4-yl]-2,7-diazaspiro[3.5]nonane-7-carboxylate (**P10**, 350 mg, 80%). ¹H NMR (400 MHz, DMSO-*d₆*), δ: 8.45 (s, 1H), 7.88 (m, 3H), 3.33 (m, 6H), 2.08 (m, 2H), 1.75 (m, 4H). LCMS (ESI) [MH]⁺: 439.

### Preparation 11. [4-({2-[6-(2,2,2-Trifluoroethyl)quinazolin-4-yl]-2,7-diazaspiro[3.5]non-7-yl}methyl)phenyl]amine (P11)

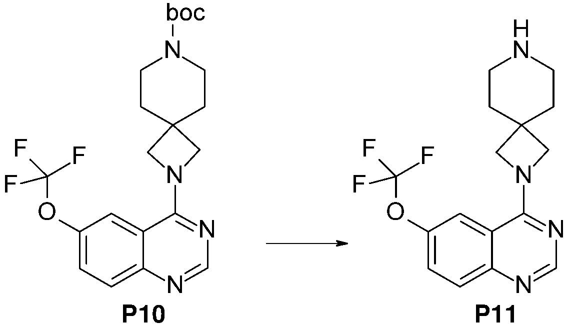

To *tert-*butyl 2-[6-(trifluoromethoxy)quinazolin-4-yl]-2,7-diazaspiro[3.5]nonane-7-carboxylate (**P10**, 350 mg, 0.8 mmol) was added TFA (3 ml). The reaction mixture was stirred at 20°C for 1 h, then concentrated water solution of sodium bicarbonate (20 ml) was added, and the mixture was extracted with DCM (2x10 ml), DCM was evaporated to dryness to yield 4-(2,7-diazaspiro[3.5]non-2-yl)-6-(trifluoromethoxy)quinazoline (**P11**, 270 mg, 100%).¹H NMR (400 MHz, DMSO-*d₆*), δ: 8.87 (s, 1H), 8.13 (m, 2H), 7.94 (m, 1H), 4.84 (m, 3H), 3.90 (m, 2H), 3.85 (m, 2H), 3.70 (m, 2H), 2.08 (m, 4H). LCMS (ESI) [MH]⁺: 339.

### Synthesis of 5-formyl-4-methyl-1-[(3-oxo-1,4-oxazepan-7-yl)methyl]indole-2-carbonitrile (P13)

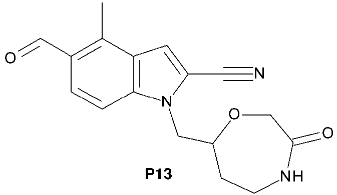

### Preparation 12. 7-(Hydroxymethyl)-1,4-oxazepan-3-one (P12)

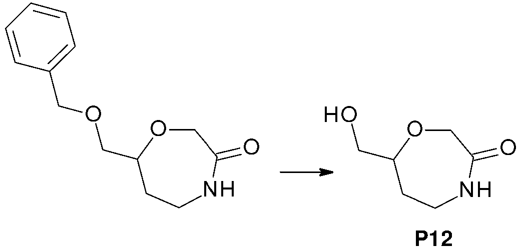

7-[(benzyloxy)methyl]-1,4-oxazepan-3-one (2,25 g, 10 mmol, prepared according to WO2016/75240, 2016, A1) dissolved in MeOH (70 ml) and stirred with Pd-C 10% (0.5 g) under hydrogen (70 bar) for 18 h. The reaction mixture was filtered, evaporated to dryness to yield 7-(hydroxymethyl)-1,4-oxazepan-3-one (**P12**, 1.45 g, 100%). ¹H NMR (400 MHz, DMSO-*d₆*), δ: 10.2 (s, 1H), 7.63 (s, 1H), 4.70 (m, 1H), 4.40 (m, 2H), 3.55 (m, 1H), 3.38 (m, 1H), 3.19 (m, 2H), 1.87 (m, 1H), 1.53 (m, 1H).

### Preparation 13. 5-Formyl-4-methyl-1-[(3-oxo-1,4-oxazepan-7-yl)methyl]indol-2-carbonitrile (P13)

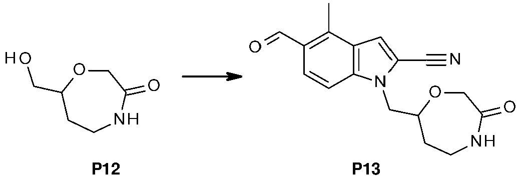

To a solution of 7-(hydroxymethyl)-1,4-oxazepan-3-one (**P12**, 123 mg, 0.85 mmol) in DMAA (2 ml) were added DIPEA (131 mg, 1.02 mmol) and MsCl (116 mg, 1.02 mmol). The mixture was stirred at 50°C for 45 min, then 2-cyano-4-methyl-5-formylindole (**P97**, 78 mg, 0.42 mmol) and potassium carbonate (300 mg, 2.2 mmol) were added, and mixture stirred at 90°C for 48 h, the product was extracted with ethyl acetate (3x5 ml) from water (10 ml), ethyl acetate evaporated to dryness. The residue was washed with dry ethyl acetate (4 ml), filtered, dried to yield pure product (**P13**, 53 mg, 41%). ¹H NMR (400 MHz, DMSO-*d₆*), δ: 10.38 (s, 1H), 7.82 (m, 2H), 7.70 (m, 2H), 4.45 (m, 1H), 4.41 (m, 1H), 3.98 (m, 2H), 3.30 (m, 3H), 3.20 (m, 2H), 2.86 (m, 3H). LCMS (ESI) [MH]⁺: 312.

### Synthesis of 2-ethynyl-4-methyl-1-[2-(2-oxoimidazolidin-1-yl)ethyl]-1H-indole-5-carbaldehyde (P14)

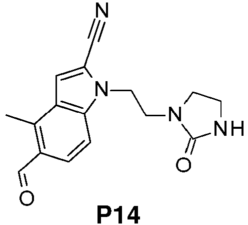

### Preparation 14. 2-Ethynyl-4-methyl-1-[2-(2-oxoimidazolidin-1-yl)ethyl]-1H-indole-5-carbaldehyde (P14)

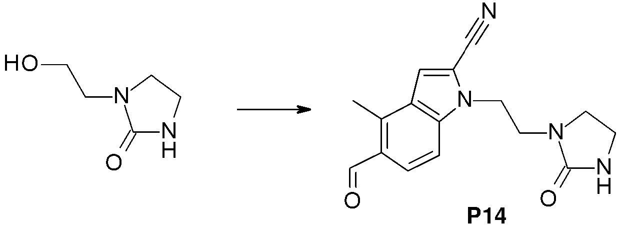

To solution of 1-(2-hydroxyethyl)imidazolidin-2-one (130 mg, 1 mmol) in DMAA (3 ml) DIPEA (258 mg, 2 mmol) and MsCl (132 mg, 1.15 mmol) were added. The mixture was stirred at 50°C for 45 min, then 2-cyano-4-methyl-5-formylindole (**P97**, 92 mg, 0.5 mmol) and potassium carbonate (400 mg,2.9 mmol) were added, and the mixture was stirred at 90°C for 48 h. The product was extracted with ethyl acetate (3x5 ml) from water (40 ml), ethyl acetate was evaporated to dryness. The residue was washed with dry ethyl acetate (4 ml), filtered, dried to yield pure 2-ethynyl-4-methyl-1-[2-(2-oxoimidazolidin-1-yl)ethyl]-1H-indole-5-carbaldehyde (**P14**, 47 mg, 32%). ¹H NMR (400 MHz, CDCl₃), δ: (8.9 (s, 1H), 6.85 (n, 1H), 6.40 (m, 3H), 3.46 (m, 3H), 3.02 (m, 2H), 2.20 (m, 2H), 1.75 (m, 4H). LCMS (ESI) [MH]⁺: 297.

### Synthesis of 5-formyl-4-methyl-1-[(7-oxoazepan-4-yl)methyl]-1H-indole-2-carbonitrile (P15)

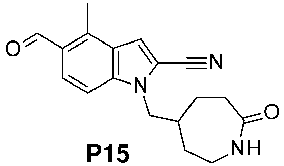

### Preparation 15. 5-Formyl-4-methyl-1-[(7-oxoazepan-4-yl)methyl]-1H-indole-2-carbonitrile (P15)

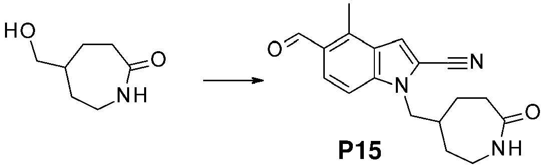

To a solution of 5-(hydroxymethyl)azepan-2-one (312 mg, 2.2 mmol, prepared as described in CN113277991, 2021, A) in DMAA (3 ml) DIPEA (405 mg, 3.14 mmol) and MsCl (300 mg, 2.02 mmol) were added. The mixture was stirred at 50°C for 45 min, then 2-cyano-4-methyl-5-formylindole (**P97**, 92 mg, 0.5 mmol) and potassium carbonate (400 mg, 2.9 mmol) were added, and the mixture was stirred at 90°C for additional 48 h. After completing of the reaction water was added (40 ml) and product was extracted with ethyl acetate (3x5 ml). Ethyl acetate was evaporated to dryness. The residue was washed with dry ethyl acetate (4 ml), filtered, and dried to yield pure 5-formyl-4-methyl-1-[(7-oxoazepan-4-yl)methyl]-1*H*-indole-2-carbonitrile (**P15**, 128 mg, 83%). ¹H NMR (400 MHz, CDCl₃), δ: 10.45 (s, 1H), 7.85 (m, 1H), 7.40 (m,1H), 6.15 (m, 1H), 4.18 (m,1H), 3.45 (m,2H), 3.2 (m, 2H), 2.95 (m, 3H), 2.45 (m,2H), 1.35 (m, 4H). LCMS (ESI) [MH]⁺: 310.

### Synthesis of 1-(2-piperazin-1-ylethyl)-5-({2-[6-(2,2,2-trifluoroethyl)quinazolin-4-yl]-2,7-diazaspiro[3.5]non-7-yl}methyl)-1H-indole-2-carbonitrile (P17)

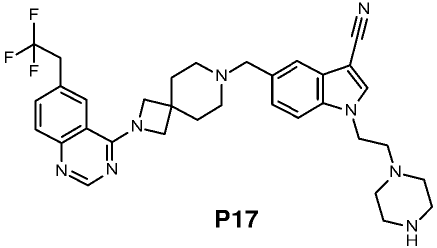

### Preparation 16. tert-Butyl 4-{2-[2-cyano-5-({2-[6-(2,2,2-trifluoroethyl)quinazolin-4-yl]-2,7-diazaspiro[3.5]non-7-yl}methyl)-1H-indol-1-yl]ethyl}piperazine-1-carboxylate (P16)

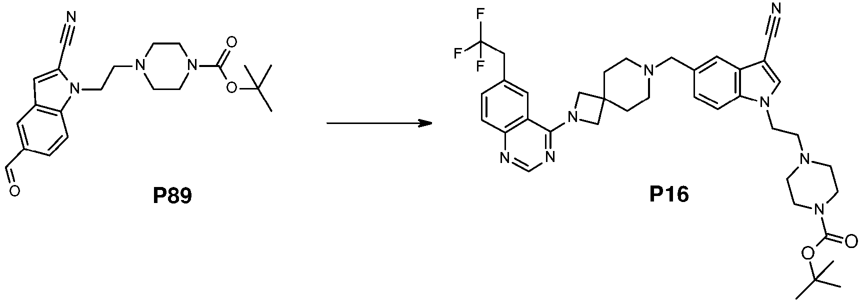

A mixture of 4-(2,7-diazaspiro[3.5]non-2-yl)-6-(2,2,2-trifluoroethyl)quinazoline hydrochloride (**P9**, 200 mg, 0.535 mmol), *tert-*butyl 4-[2-(2-cyano-5-formyl-1*H*-indol-1-yl)ethyl]piperazine-1-carboxylate (**P89**, 306 mg, 0.8 mmol), and triacethoxyborohydride (227 mg, 1.07 mmol) in DCM (10 ml) stirred at rt for 24 h (LCMS control). Then a water solution of sodium bicarbonate was added with stirring. The mixture was stirred for 30 min and was separated. Water phase was extracted with DCM (100 ml). Combined DCM extract was concentrated, and residue was purified by column chromatography on silica with ethyl acetate - methanol (10:2) to yield *tert-*butyl 4-{2-[2-cyano-({2-[6-(2,2,2-trifluoroethyl)quinazolin-4-yl]-2,7-diazaspiro[3.5]non-7-yl}-1*H*-indol-1-yl]ethyl}piperazine-1-carboxylate (**P16**, 190 mg, 51%) as a white solid. LCMS (ESI) [MH]⁺: 703.

### Preparation 17. 1-(2-Piperazin-1-ylethyl)-5-({2-[6-(2,2,2-trifluoroethyl)quinazolin-4-yl]-2,7-diazaspiro[3.5]non-7-yl}methyl)-1H-indole-2-carbonitrile (P17)

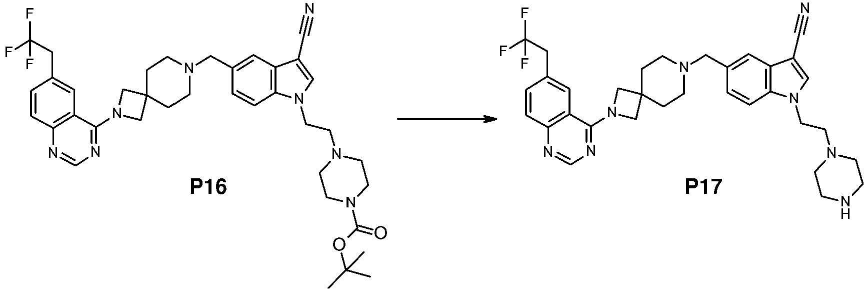

*tert*-Butyl 4-{2-[2-cyano-5-({2-[6-(2,2,2-trifluoroethyl)quinazolin-4-yl]-2,7-diazaspiro[3.5]non-7-yl}methyl)-1*H*-indol-1-yl]ethyl}piperazine-1-carboxylate (**P16**, 190 mg, 0.27 mmol) was added to dioxane solution of HCl (3M, 12 ml). Reaction mixture was stirred at rt for 3 h (LCMS control), then evaporated under vacuum to dryness to yield 1-(2-piperazin-1-ylethyl)-5-({2-[6-(2,2,2-trifluoroethyl)quinazolin-4-yl]-2,7-diazaspiro[3.5]non-7-yl}methyl)-1*H*-indole-2-carbonitrile hydrochloride (**P17**, 172 mg, 100%) as a white solid. LCMS (ESI) [MH]⁺: 603.

### Synthesis of 4-methyl-1-(2-piperazin-1-ylpropyl)-5-[[2-[6-(2,2,2-trifluoroethyl)quinazolin-4-yl]-2,7-diazaspiro[3.5]nonan-7-yl]methyl]indole-2-carbonitrile (P20)

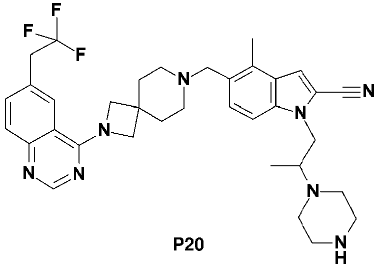

### Preparation 18. 5-Formyl-4-methyl-1-{2-[4-(tert-butylcarboxlato)piperazin-1-yl]propyl}-1H-indole-2-carbonitrile (P18)

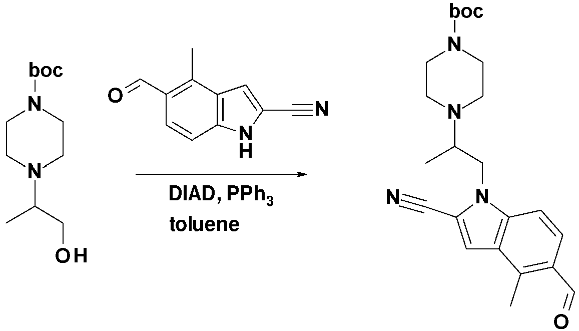

To a mixture of 2-[4-(*tert*-butylcarboxlato)piperazin-1-yl]propan-1-ol (200 mg, 0.9 mmol), 5-formyl-4-methyl-1H-indole-2-carbonitrile **P97** (165 mg, 0.9 mmol) and triphenyl phosphine (354 mg, 1.35 mmol) in toluene (5 ml) DIAD (273 mg, 1.35 mmol) was added, and the resulting mixture was stirred at ambient temperature overnight. The solvent was evaporated. The residue after evaporation was subjected to column chromatography on silica gel eluting with dichloromethane/ethyl acetate (0 → 20%) to afford 5-formyl-4-methyl-1-{2-[4-(*tert*-butylcarboxlato)piperazin-1-yl]propyl}-1H-indole-2-carbonitrile (**P18**, 130 mg, 37%). LCMS [MH⁺]: 411. ¹H NMR (400 MHz, DMSO-*d₆*), δ: 10.37 (s, 1H), 7.82-7.78 (m, 2H), 7.63 (d, J=7.0 Hz, 1H), 4.82-4.73 (m, 1H), 4.39-4.31 (m, 1H), 4.27-4.20 (m, 1H), 3.25-3.10 (m, 4H), 2.85 (s, 3H), 2.64-2.52 (m, 2H), 2.25-2.19 (m, 2H), 1.38 (s, 9H), 1,18-1,12 (m, 3H).

### Preparation 19. 4-Methyl-1-{2-[4-( tert-butylcarboxlato)piperazin-1-yl]propyl}-5-({2-[6-(2,2,2-trifluoroethyl)quinazolin-4-yl]-2,7-diazaspiro[3.5]non-7-yl}methyl)-1H-indole-2-carbonitrile (P19)

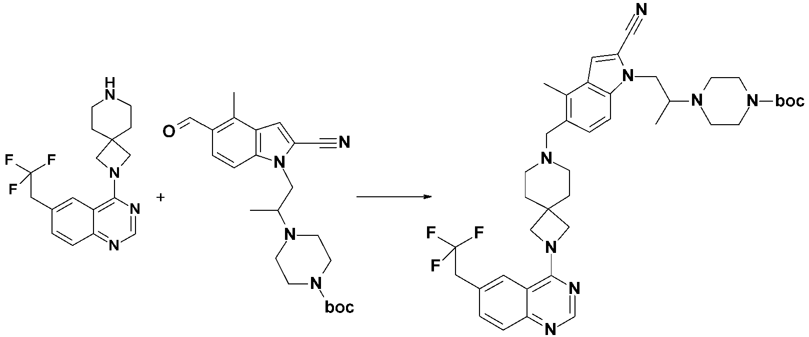

Molecular sieves, 3Å (200 mg) was added to a mixture of 5-formyl-4-methyl-1-{2-[4-(*tert-*butylcarboxlato)piperazin-1-yl]propyl}-1H-indole-2-carbonitrile (**P18**, 130 mg, 0.34 mmol) and 4-(2,7-diazaspiro[3.5]non-2-yl)-6-(2,2,2-trifluoroethyl)quinazoline (**P9**, 113 mg, 0.34 mmol) in methanol (1 ml). Then NaBH₃CN (42 mg, 0.67 mmol) was added, and the reaction mixture was stirred at ambient temperature overnight. Saturated aqueous solution of sodium bicarbonate, and dichloromethane were added, and the organic layer was separated, washed with brine, dried over sodium sulfate, filtered and the filtrate was evaporated. The residue after evaporation was subjected to HPLC to afford the target compound 4-methyl-1-{2-[4-(*tert*-butylcarboxlato)piperazin-1-yl]propyl}-5-({2-[6-(2,2,2-trifluoroethyl)quinazolin-4-yl]-2,7-diazaspiro[3.5]non-7-yl}methyl)-1H-indole-2-carbonitrile (**P19**, 39 mg, 17%). LCMS (ESI) [MH⁺]: 731.

### Preparation 20. 4-Methyl-1-(2-piperazin-1-ylpropyl)-5-[[2-[6-(2,2,2-trifluoroethyl)quinazolin-4-yl]-2,7-diazaspiro[3.5]nonan-7-yl]methyl]indole-2-carbonitrile (P20)

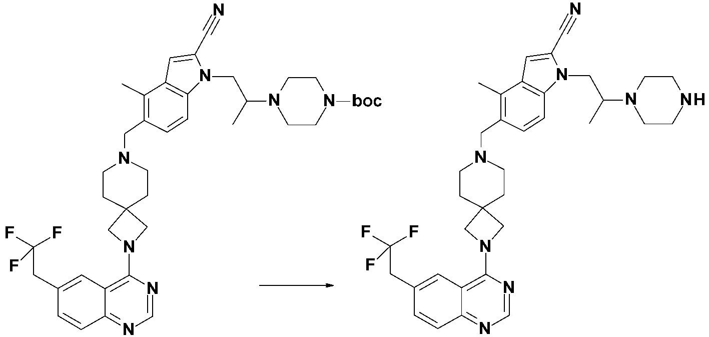

To 4-methyl-1-{2-[4-(*tert*-butylcarboxlato)piperazin-1-yl]propyl}-5-({2-[6-(2,2,2-trifluoroethyl)quinazolin-4-yl]-2,7-diazaspiro[3.5]non-7-yl}methyl)-1H-indole-2-carbonitrile (**P19**, 39 mg, 5.8 mmol) was added TFA (1ml). The solution stirred at rt for 2 h (LCMS control), then evaporated to dryness, water solution of sodium bicarbonate (5 ml) was added, product extracted with DCM (3x5 ml). Organic phase dried with sodium sulphate, concentrated to dryness yielded the product **P20** (31 mg, 100%). LCMS (ESI) [MH⁺]: 631.

### Synthesis of 4-(7-{[2-ethynyl-4-methyl-1-(4-piperazin-1-ylbutyl)-1H-indol-5-yl]methyl}-2,7-diazaspiro[3.5]non-2-yl)-6-(2,2,2-trifluoroethyl)quinazoline (P24)

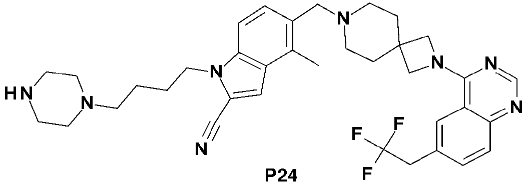

### Preparation 21. 1-(4-Chlorobutyl)-5-formyl-4-methyl-1H-indole-2-carbonitrile (P21)

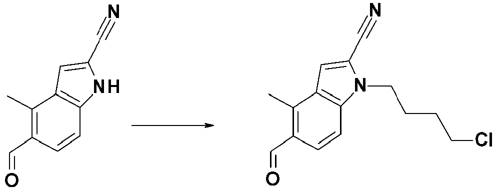

To solution of 5-formyl-4-methyl-1H-indole-2-carbonitrile **P97** (184 mg, 1.0 mmol) in 10 ml of MeCN was added 1,4-dichlorobutane (1.27 g, 10 mmol), K₂CO₃ (2 g) and NaI (0.5 g). The reaction mixture was stirred at 80°C for 24 h, then evaporated to dryness, washed with water (15 ml), dried under vacuum, and washed with hexane (3 ml) to yield 1-(4-chlorobutyl)-5-formyl-4-methyl-1*H*-indole-2-carbonitrile (**P21**, 244 mg, 89%). ¹H NMR (400 MHz, DMSO-*d₆*), δ: 10.37 (s, 1H), 7.89-7.81 (m, 2H), 7.68 (d, J=6.8 Hz, 1H), 4.42 (t, J=7.0 Hz, 2H), 3.64 (t, J=6.8 Hz, 2H), 2.85 (s, 3H), 1.95-1.79 (m, 2H), 1.79-1.63 (m,2H).

### Preparation 22. tert-Butyl 4-[4-(2-cyano-5-formyl-4-methyl-1H-indol-1-yl)butyl]piperazine-1-carboxylate (P22)

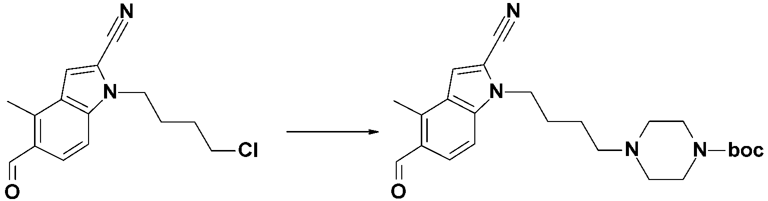

To a solution of 1-(4-chlorobutyl)-5-formyl-4-methyl-1*H*-indole-2-carbonitrile (**P21**, 244 mg, 0.89 mmol) in MeCN (3 ml) was added Boc-piperazine (215 mg, 1.16 mmol) and K₂CO₃ (0.4 g). The mixture was stirred at 80°C for 24h, then filtered, concentrated. The residue after evaporation was subjected to column chromatography on silica gel eluting with dichloromethane/ethyl acetate (0 → 50%) to afford the title compound **P22** (238 mg, 63%). LCMS [MH⁺]: 425. ¹H NMR (400 MHz, CDCl₃), δ: 10.44 (s, 1H), 7.91 (d, J=9.0 Hz, 1H), 7.39 (s, 1H), 7.33 (d, J=8.2 Hz, 1H), 4,36 (t, J=7.1 Hz, 2H), 3.48-3.34 (m, 4H), 2.90 (s, 3H), 2.43-2.29 (m, 5H), 1.98-1.89 (m, 2H), 1.60-1.52 (m, 2H), 1.43 (s, 9H).

### Preparation 23. tert-Butyl 4-{4-[2-ethynyl-4-methyl-5-({2-[6-(2,2,2-trifluoroethyl)quinazolin-4-yl]-2,7-diazaspiro[3.5]non-7-yl}methyl)-1H-indol-1-yl]butyl}piperazine-1-carboxylate (P23)

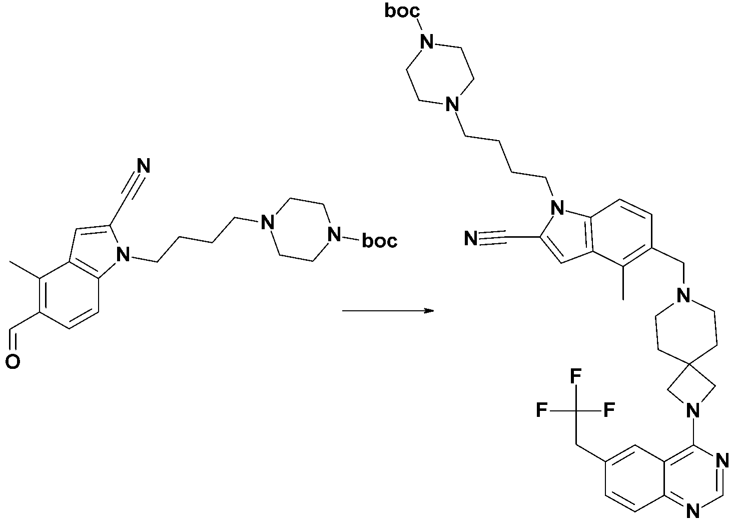

To a solution of 4-(2,7-diazaspiro[3.5]non-2-yl)-6-(2,2,2-trifluoroethyl)quinazoline (**P9**, 182 mg, 0.54 mmol), *tert*-butyl 4-[4-(2-cyano-5-formyl-4-methyl-1*H*-indol-1-yl)butyl]piperazine-1-carboxylate (**P22**, 230 mg, 0.54 mmol) in DCM (5 ml) were added DIPEA (210 mg, 1.63 mmol) and STAB (345 mg, 1.63 mmol). Reaction mixture stirred at rt for 24 h (LCMS control), then washed with saturated water solution of NaHCO₃, extracted with DCM (2x5 ml). The residue after evaporation was subjected to column chromatography on silica gel eluting with ethyl acetate-methanol (0 → 30%) to afford the title compound **P23** (167 mg, 43%). LCMS (ESI) [MH⁺]: 745. ¹H NMR (400 MHz, CDCl₃), δ: 8.59 (s, 1H), 7.83-7.72 (m, 2H), 7.60 (d, J=6.0 Hz, 1H), 7.34 (d, J=9.6 Hz, 1H), 7.23-7.11 (m, 2H), 4.39-4.17 (m, 6H), 4.17-4.08 (m, 2H), 2.54 (s, 3H), 2.49-2.33 (m, 2H), 2.33-2.27 (m, 6H), 1.95-1.82 (m, 6H), 1.57-1.47 (m, 2H), 1.43 (s, 9H).

### Preparation 24. 4-(7-{[2-Ethynyl-4-methyl-1-(4-piperazin-1-ylbutyl)-1H-indol-5-yl]methyl}-2,7-diazaspiro[3.5]non-2-yl)-6-(2,2,2-trifluoroethyl)quinazoline (P24)

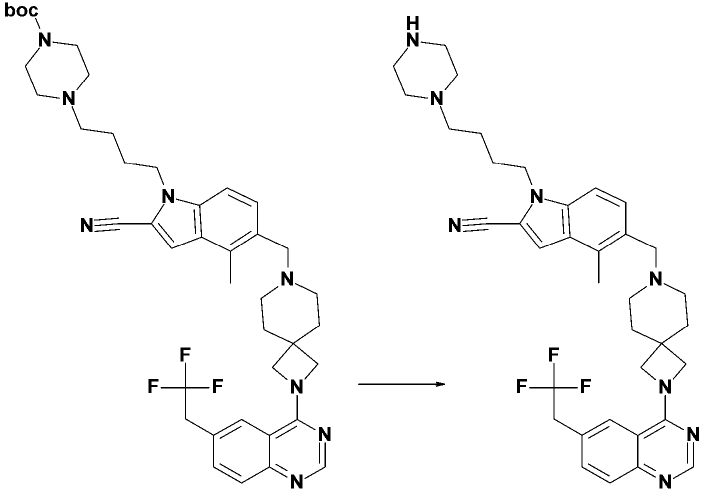

A solution of *tert*-butyl 4-{4-[2-ethynyl-4-methyl-5-({2-[6-(2,2,2-trifluoroethyl)quinazolin-4-yl]-2,7-diazaspiro[3.5]non-7-yl}methyl)-1*H*-indol-1-yl]butyl}piperazine-1-carboxylate (**P23**, 167 mg, 0.224 mmol) in TFA (1ml) was stirred at rt for 2 h (LCMS control) , then evaporated to dryness, water solution of sodium bicarbonate (5 ml) was added, product was extracted with dichloromethane (3x5 ml). Organic phase dried with sodium sulphate, concentrated to dryness yield the target compound **P24** (144 mg, 100%). LCMS (ESI) [MH⁺]: 644. ¹H NMR (400 MHz, DMSO-*d₆*), δ: 8.44 (s, 1H), 8.20-7.95 (m, 1H), 7.74-7.68 (m, 2H), 7.42 (d, J=6.0 Hz, 1H), 7.31 (d, J=9.5 Hz, 1H), 4.35-4.28 (m, 3H), 3.92-3.81 (m, 2H), 3.54-3.48 (m, 2H), 3.43-3.31 (m, 4H), 2.64-2.56 (m, 4H), 2.45-2.30 (m, 4H), 2.25-2.16 (m, 6H), 1.82-1.73 (m, 6H), 1.42-1.31 (m, 2H).

### Synthesis of 1-[(trans-4-aminocyclohexyl)methyl]- 4-methyl-5-[[2-[6-(2,2,2-trifluoroethyl)quinazolin-4-yl]-2,7-diazaspiro[3.5]nonan-7-yl]methyl]-1H-indole-2-carbonitrile hydrochloride (P26)

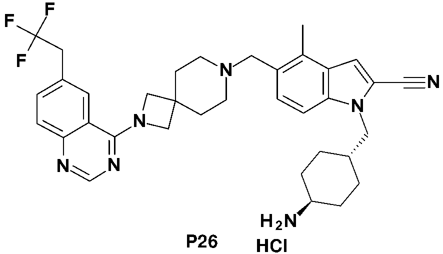

### Preparation 25. tert-Butyl (trans-4-{[2-cyano-5-({2-[6-(2,2-dimethylpropyl)quinazolin-4-yl]-2,7-diazaspiro[3.5]non-7-yl}methyl)-4-methyl-1H-indol-1-yl]methyl}cyclohexyl)carbamate (P25)

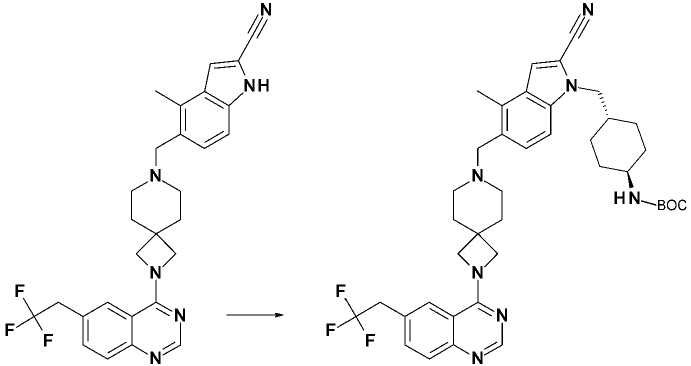

To a solution of {trans-4-[(*tert*-butoxycarbonyl)amino]cyclohexyl}methyl methanesulfonate (118 mg, 0.4 mmol), prepared according to **WO2018/67422, 2018, A1**, and 4-methyl-5-[[2-[6-(2,2,2-trifluoroethyl)quinazolin-4-yl]-2,7-diazaspiro[3.5]nonan-7-yl]methyl]-1H-indole-2-carbonitrile (150 mg, 0.30 mmol) in MeCN (3 ml) was added potassium carbonate (138 mg, 1 mmol). Reaction mixture was stirred at 50°C for 24 h, then filtered and concentrated. The residue after evaporation was subjected to column chromatography on silica gel eluting with ethyl acetate-methanol (0 → 30%) to afford the title compound **P25** (70 mg, 30%). LCMS (ESI) [MH⁺]: 716.

### Preparation 26. 1-[(trans-4-Aminocyclohexyl)methyl]- 4-methyl-5-[[2-[6-(2,2,2-trifluoroethyl)quinazolin-4-yl]-2,7-diazaspiro[3.5]nonan-7-yl]methyl]-1H-indole-2-carbonitrile hydrochloride (P26)

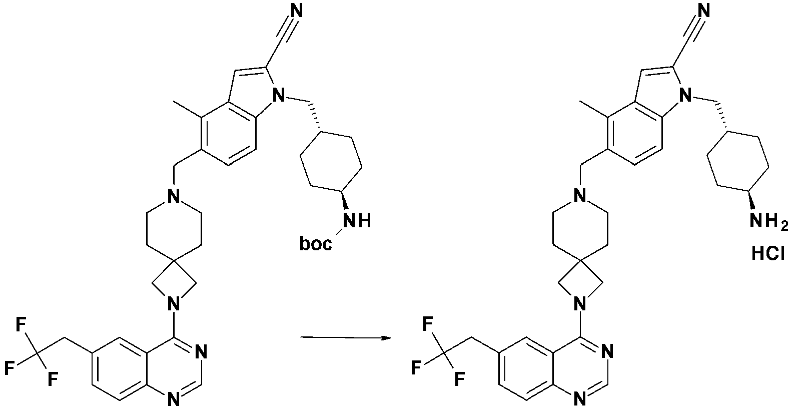

To *tert*-butyl (*trans*-4-{[2-cyano-5-({2-[6-(2,2-dimethylpropyl)quinazolin-4-yl]-2,7-diazaspiro[3.5]non-7-yl}methyl)-4-methyl-1*H*-indol-1-yl]methyl}cyclohexyl)carbamate **P25** (70 mg, 0.1 mmol) 2 ml 3 M solution HCl in dioxane was added. The reaction mixture was stirred at rt for 2 h (LCMS control), then evaporated to dryness to yield **P26** (68 mg, 100%). LCMS (ESI) [MH⁺]: 616.

### Synthesis of 4-(2,7-diazaspiro[3.5]non-7-yl)-6-(2,2,2-trifluoroethyl)quinazoline (P28)

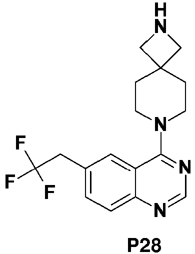

### Preparation 27. tert-Butyl 7-[6-(2,2,2-trifluoroethyl)quinazolin-4-yl]-2,7-diazaspiro[3.5]nonane-2-carboxylate (P27)

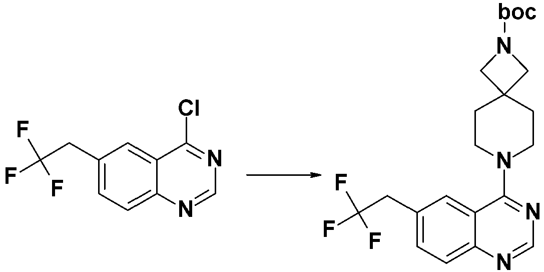

To a solution of 4-chloro-6-(2,2,2-trifluoroethyl)quinazoline (**P7**, 3 g, 12.2 mmol) in DCM (60 ml) was added DIPEA (7.9 g, 61 mmol) and *tert*-butyl 2,7-diazaspiro[3.5]nonane-2-carboxylate hydrochloride (3.2 g, 12.2 mmol). The reaction mixture was stirred at rt for 18 h (LCMS control). After completing of the reaction, the solvent was evaporated, and residue was purified by column chromatography on silica with ethyl acetate-methanol (10:1) to yield 5.1 g of the product **P27** as a white solid. LCMS (ESI) [MH]⁺: 437.

### Preparation 28. 4-(2,7-Diazaspiro[3.5]non-7-yl)-6-(2,2,2-trifluoroethyl)quinazoline (P28)

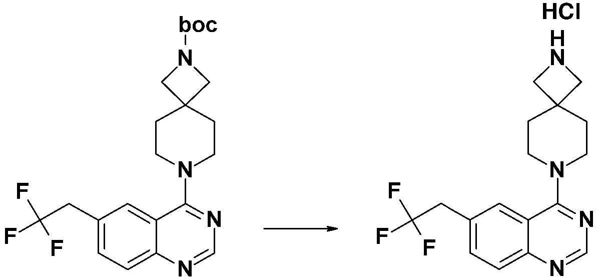

*tert*-Butyl 7-[6-(2,2,2-trifluoroethyl)quinazolin-4-yl]-2,7-diazaspiro[3.5]nonane-2-carboxylate (**P27**, 5.1 g, 11.7 mmol) was added to dioxane-HCl solution (3M, 120 ml). The reaction mixture was stirred at rt for 3 h (LCMS control), then evaporated under reduce pressure to dryness to yield 4-(2,7-diazaspiro[3.5]non-7-yl)-6-(2,2,2-trifluoroethyl)quinazoline hydrochloride (**P28**, 4.4 g, 100%) as a white solid. LCMS (ESI) [MH]⁺: 337.

### Synthesis of 3-[6-(2,2,2-trifluoroethyl)quinazolin-4-yl]-3,9-diazaspiro[5.5]undecane (P30)

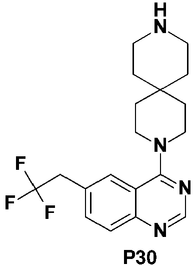

### Preparation 29. tert-Butyl 9-[6-(2,2,2-trifluoroethyl)quinazolin-4-yl]-3,9-diazaspiro[5.5]undecane-3-carboxylate (P29)

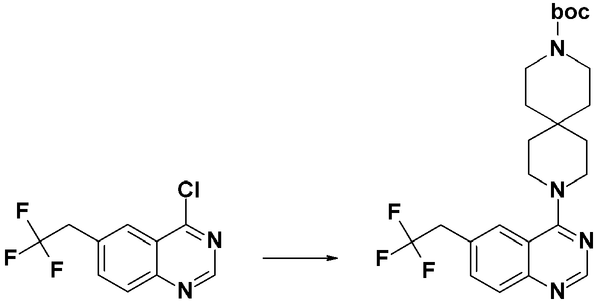

To a solution of 4-chloro-6-(2,2,2-trifluoroethyl)quinazoline (**P7**, 3 g, 12.2 mmol) in dichloroethane (60 ml) were added DIPEA (7.9 g, 61 mmol), and *tert*-butyl 3,9-diazaspiro[5.5]undecane-3-carboxylate (3.5 g, 12.2 mmol). The reaction mixture was stirred at rt for 18 h (LCMS control). The residue after solvent evaporation was purified by column chromatography on silica with ethyl acetate - methanol (10:1) to yield the product **P29** as a white solid (5.1 g, 92%). LCMS (ESI) [MH]⁺: 465.

### Preparation 30. 3-[6-(2,2,2-Trifluoroethyl)quinazolin-4-yl]-3,9-diazaspiro[5.5]undecane (P30)

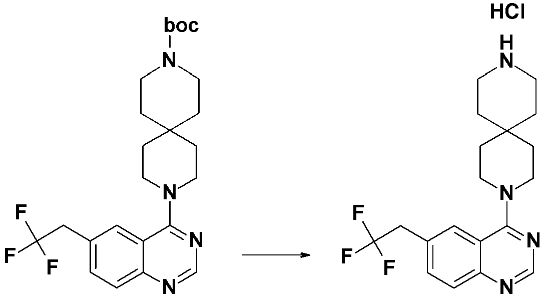

*tert*-Butyl 9-[6-(2,2,2-trifluoroethyl)quinazolin-4-yl]-3,9-diazaspiro[5.5]undecane-3-carboxylate (**P29**, 5.1 g, 11.7 mmol) was added to HCl/dioxane solution (3M, 120 ml). The reaction mixture was stirred at rt for 3 h (LCMS control), then solvent was evaporated under reduce pressure to dryness to yield 3-[6-(2,2,2-trifluoroethyl)quinazolin-4-yl]-3,9-diazaspiro[5.5]undecane (**P30**, 4.4 g, 100%) as a white solid. LCMS (ESI) [MH]⁺: 364.

### Synthesis of 4-(2,7-diazaspiro[3.5]non-2-yl)-6-(2,2,2-trifluoroethyl)pyrido[2,3-d]pyrimidine (P39)

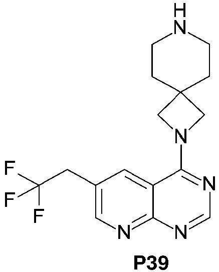

### Preparation 31. Methyl 2-amino-5-bromonicotinate (P31)

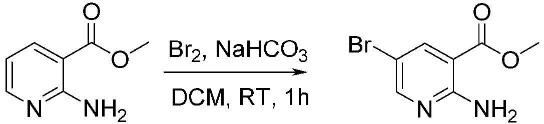

To a stirred solution of methyl 2-aminonicotinate (2 g, 13.15 mmol) and sodium bicarbonate (2.2 g, 26.31 mmol) in DCM (30 ml) was added a solution of bromine (1.01 ml, 39 mmol) in DCM (20 ml) dropwise at 0°C. The reaction mixture was stirred at room temperature for 1 h. The reaction mixture was quenched with sodium bisulfate solution (50 ml) and extracted with DCM (2x40 ml). The combined organic layers are washed with brine (40 ml), dried over sodium sulfate, filtered, and evaporated under reduced pressure to give the title compound (**P31**) as a yellow solid (2.76 g, 91%). ¹H NMR (400 MHz, DMSO-*d₆*), δ: 8.29 (d, J = 2.5 Hz, 1H), 8.12 (d, J = 2.5 Hz, 1H), 7.32 (s, 2H), 3.82 (s, 3H).

### Preparation 32. 2-Amino-5-bromonicotinic acid (P32)

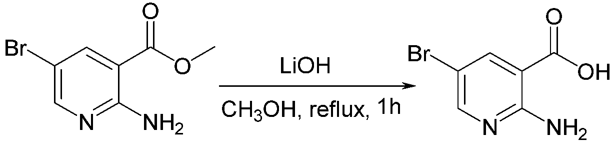

To a solution of **P31** (2.76 g, 11.95 mmol) in DCM (100ml) was added a solution LiOH (1.72 g, 71.7 mmol) in water, the reaction mixture was refluxed for 1 hour, then evaporated under reduced pressure and dissolved in water, pH was adjusted to ~3 with HCl. The residue was filtered and washed with water to give the title compound (**P32**) as a white solid (2.05 g, 79%). ¹H NMR (400 MHz, DMSO-*d₆*), δ: 13.30 (s, 1H), 8.25 (d, J = 2.5 Hz, 1H), 8.09 (d, J = 2.5 Hz, 1H), 7.38 (s, 1H).

### Preparation 33. 6-Bromopyrido[2,3-d]pyrimidin-4(3H)-one (P33)

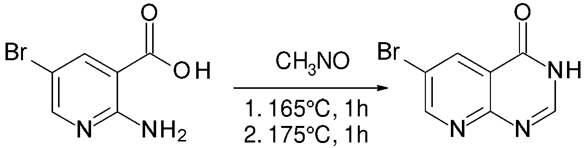

The solution of **P32** (2 g, 9.22 mmol) in formamide (1.66 g, 36.88 mmol) was refluxed at 165°C for 1 h and then at 175°C at 1 h. The residue was washed with water and added to a solution of sodium bicarbonate (30 ml) and stirred to shredding the residue. Then the precipitate was filtered and washed with water to give the title compound (**P33**) as a white solid (2.03 g, 97%). ¹H NMR (400 MHz, DMSO-*d₆*), δ: 12.69 (s, 1H), 9.03 (d, J = 2.6 Hz, 1H), 8.60 (d, J = 2.6 Hz, 1H), 8.35 (s, 1H).

### Preparation 34. tert-Butyl-2-(6-bromopyrido[2,3-d]pyrimidin-4-yl)-2,7-diazaspiro[3.5]nonane-7-carboxylate (P34)

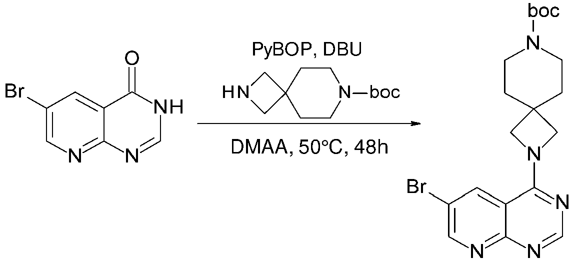

A mixture of **P33** (144 mg, 0.64 mmol), amine (205 mg, 0.78 mmol), PyBOP (416 mg, 1.11 mmol) and DBU (474 mg, 3.12 mmol) in DMAA (2 ml). The reaction mixture was stirred at 60°C for 48 h. Then the reaction mixture was cooled to rt, extracted with EtOAc (2x10 ml), washed with brine (40 ml), dried over sodium sulfate, filtered, concentrated, and purified by silica gel column chromatography (gradient from EtOAc to 1% MeOH in EtOAc) to give the title compound (**P34**) as a white solid (150 mg, 44%). ¹H NMR (400 MHz, DMSO-*d₆*), δ: 9.03 (d, J = 2.4 Hz, 1H), 8.59 (s, 1H), 8.46 (d, J = 2.5 Hz, 1H), 3.35 (br. s, 2H), 3.29 (br. s, 2H), 2.51 (br. s, 2H), 2.49 (br. s, 2H), 1.74 (s, 4H), 1.40 (s, 9H).

### Preparation 35. tert-Butyl-2-(6-vinylpyrido[2,3-d]pyrimidin-4-yl)-2,7-diazaspiro[3.5]nonane-7-carboxylate (P35)

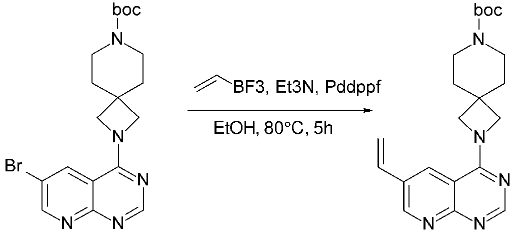

To a solution of **P34** (150 mg, 0.35 mmol) in EtOH (2 ml) DIPEA (87 mg, 0.75 mmol) and vinyl-BF3 (92 mg, 0.70 mmol) were added. Then the mixture was filled with argon and Pddppf (30 mg) was added. The reaction mixture was stirred at 80°C for 5 h. Then the reaction mixture was cooled to room temperature, extracted with EtOAc and water, dried over sodium sulfate, filtered, concentrated, and purified by silica gel column chromatography (gradient from EtOAc to 1% MeOH in EtOAc) to give the title compound (**P35**) as a white solid (130 mg, 99%). LCMS (ESI) [MH]⁺: 382.

### Preparation 36. tert-Butyl-2-(6-formylpyrido[2,3-d]pyrimidin-4-yl)-2,7-diazaspiro[3.5]nonane-7-carboxylate (P36)

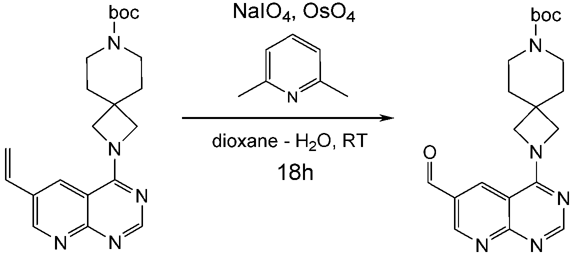

To the solution of **P35** (130 mg, 0.3 mmol), 2,6-lutidine (58 mg, 0.48 mmol) and NaIO₄ (241 mg, 0,99 mmol) in mixture of dioxane (6 ml) and water (2 ml) that filled by argon was added the solution of OsO₄ (17 mg, 0.06 mmol) in *t*-BuOH (1 ml). The reaction mixture was stirred at room temperature for 20 h. The reaction mixture was extracted with DCM (2x10 ml) and purified by silica gel column chromatography (gradient from EtOAc to 1% MeOH in EtOAc) to give the title compound (**P36**) as a white solid (110 mg, 84%). ¹H NMR (400 MHz, CDCl₃), δ: 10.19 (s, 1H), 9.46 (d, *J* = 2.2 Hz, 1H), 8.83 (s, 1H), 8.65 (d, *J* = 2.2 Hz, 1H), 4.36 (br. s, 2H), 3.17 (d, *J* = 3.4 Hz, 2H), 1.89 (t, *J* = 5.4 Hz, 4H), 1.72 (s, 2H), 1.62 (s, 2H), 1.49 (s, 9H).

### Preparation 37. tert-Butyl-2-{6-[(E)-hydrazonomethyl]pyrido[2,3-d]pyrimidin-4-yl}-2,7-diazaspiro[3.5]nonane-7-carboxylate (P37)

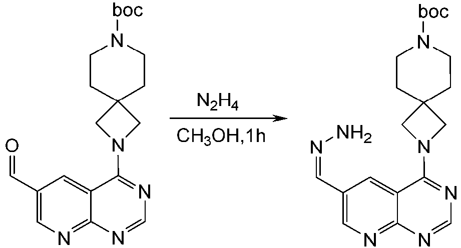

To a stirred solution of **P36** (100 mg, 0.26 mmol) in MeOH (1 ml) was added dropwise aqua solution of hydrazine hydrate (39 mg, 0.72 mmol). The reaction mixture was stirred at rt for 1 h. The reaction mixture was evaporated under reduced pressure to give the title compound (**P37**) as white solid (87 mg, 84%). LCMS (ESI) [MH]⁺: 398.

### Preparation 38. tert-Butyl-2-[6-(2,2,2-trifluoroethyl)pyrido[2,3-d]pyrimidin-4-yl]-2,7-diazaspiro[3.5]nonane-7-carboxylate (P38)

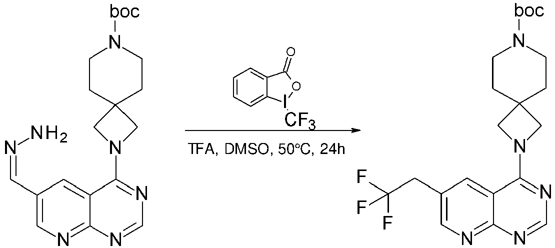

A solution of **P37** (87 mg, 0.22 mmol), 3-(trifluoromethyl)-2-benzofuran-1(*3H*)-one (97 mg, 0.31 mmol) and TFA (25 mg, 0.22 mmol) in DMSO (2 ml) was filled with argon. The reaction mixture was stirred at 50°C for 24 h. The reaction mixture was extracted with EtOAc, combined organic layers were evaporated under reduced pressure and purified by silica gel column chromatography (gradient from EtOAc to 50% MeOH in EtOAc) to give the title compound **P38** as white solid (39 mg, 41%). ¹H NMR (400 MHz, CDCl₃), δ: 8.95 (s, 1H), 8.78 (s, 1H), 8.12 (s, 1H), 4.29 (s, 4H), 3.55 (d, *J* = 10.4 Hz, 2H), 3.47 (s, 4H), 1.94 - 1.84 (m, 4H), 1.48 (s, 9H).

### Preparation 39. 4-(2,7-Diazaspiro[3.5]non-2-yl)-6-(2,2,2-trifluoroethyl)pyrido[2,3-d]pyrimidine (P39)

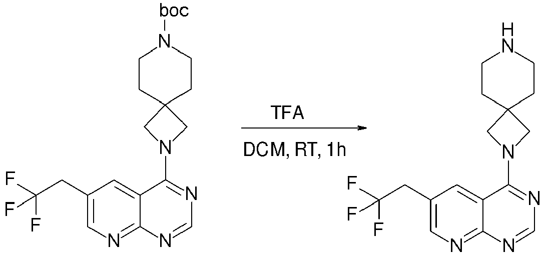

A solution of **P38** (39 mg, 0.09 mmol) and TFA (3 ml) in DCM (1 ml) was stirred at rt for 1 h. The reaction mixture was evaporated under reduced pressure, then aqua solution of potassium carbonate was added and extracted with DCM (2x10 ml), dried over sodium sulfate, filtered, and concentrated to give the title compound (**P39**) as a white solid (25 mg, 83 %). ¹H NMR (400 MHz, CDCl₃), δ: 8.94 (d, *J =* 1.8 Hz, 1H), 8.77 (s, 1H), 8.14 - 8.08 (m, 1H), 4.27 (s, 4H), 3.58 - 3.49 (m, 2H), 3.17 (d, *J =* 3.0 Hz, 1H), 2.89 (s, 4H), 1.93 - 1.86 (m, 4H).

### Synthesis of 1-(2,7-diazaspiro[3.5]non-2-yl)-7-(2,2,2-trifluoroethyl)phthalazine (P44)

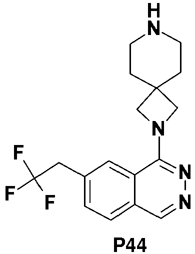

### Preparation 40. tert-Butyl 2-[7-bromophthalazin-1-yl]-2,7-diazaspiro[3.5]nonane-7-carboxylate (P40)

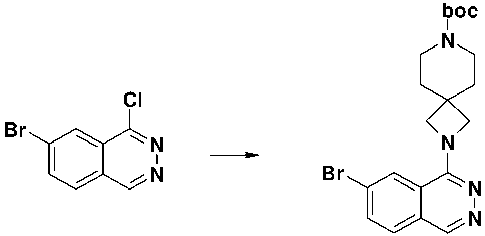

To solution of crude 7-bromo-1-chloriphtalazine (1.55 g, 6.37 mmol) (synthesized according to US2015/259331, 2015) in dichloroethane (40 ml) was added DIPEA (7.9 g, 61 mmol) and *tert-butyl* 2,7-diazaspiro[3.5]nonane-7-carboxylate hydrochloride (1.67 g, 6.37 mmol). Reaction solution was stirred at rt for 18 h (LCMS control). Then product was purified by column chromatography on silica with ethyl acetate-methanol (10-1) to yield the title compound **P40** (1.3 g, 47%) as a white solid. LCMS (ESI) [MH⁺]: 434.

### Preparation 41. tert-Butyl 2-(7-vinylphthalazin-1-yl)-2,7-diazaspiro[3.5]nonane-7-carboxylate (P41)

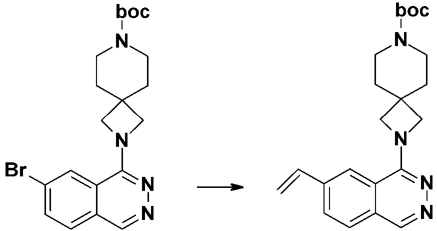

To a solution of *tert*-butyl 2-[7-bromophthalazin-1-yl]-2,7-diazaspiro[3.5]nonane-7-carboxylate (**P40**, 0.30 g, 0.69 mmol) in EtOH (10 ml), water (0.5 ml), TEA (140 mg, 1.39 mmol) was added. Potassium vinyltrifluoroborate (140 mg, 1.03 mmol) and dichloro[l,l'-bis(diphenylphosphino)ferrocene]palladium(H) dichloromethane adduct (0.17 g, 0.21 mmol) were then added, and the reaction mixture was stirred at 80°C for 24 h. The reaction mixture was worked up with EtOAc and H₂O, and the layers were separated. The organic layer was washed with brine, dried over MgSO₄, filtered and concentrated. The crude material was purified on silica (gradient elution, 0-40% EtOAc/hexanes) to yield the title compound **P41** as white solid (210 mg, 80 %). LCMS (ESI) [MH⁺]: 381.

### Preparation 42. tert-Butyl 2-(7-formylphthalazin-1-yl)-2,7-diazaspiro[3.5]nonane-7-carboxylate (P42)

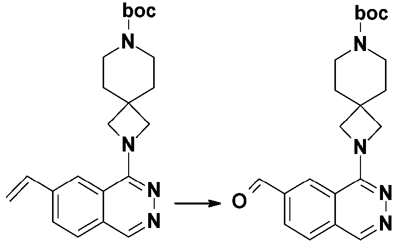

To a solution of *tert*-butyl 2-(7-vinylphthalazin-1-yl)-2,7-diazaspiro[3.5]nonane-7-carboxylate (**P41**, 210 mg, 0.55 mmol) in dioxane (2 ml) and water (0.42 ml) were added 2,6-lutidine (113 mg, 1.1 mmol), solution of osmium tetraoxide (10 mg) in *tert*-BuOH (0.5 ml) and sodium periodate (354 mg, 1.65 mmol). The reaction mixture was stirred at rt for 24 h, after reaction completed, solution was cooled and added to ice-cold water solution of sodium bicarbonate. The mixture was stirred at 15°C for 30 min, then extracted with DCM (2x50 ml), DCM was evaporated, residue was purified by silica gel column chromatography (DCM-ethyl acetate - 1:1) to give title product **P42** (145 mg, 65 %) as a brown solid. LCMS (ESI) [MH⁺]: 383.

### Preparation 43. tert-Butyl 2-[7-(2,2,2-trifluoroethyl)phthalazin-1-yl]-2,7-diazaspiro[3.5]nonane-7-carboxylate (P43)

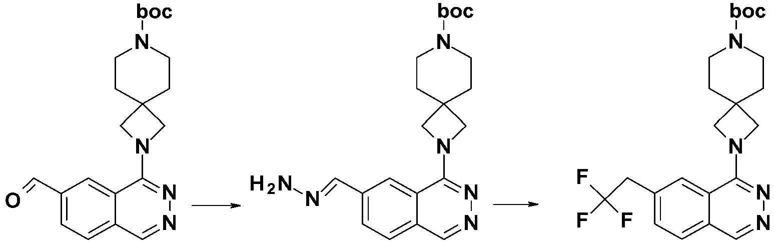

To solution of *tert*-butyl 2-(7-formylphthalazin-1-yl)-2,7-diazaspiro[3.5]nonane-7-carboxylate (**P42**, 145 mg, 0.38 mmol) in MeOH (1 ml) was added hydrazine hydrate (38 mg, 0.76 mmol). The solution was stirred at rt for 2 h (TLC control), then reaction mixture was evaporated to dryness to yield crude hydrazone (150 mg) used in the next stage without purification. The hydrazone (150 mg, 0.38 mmol) dissolved in DMSO (1 ml) under argon atmosphere, then Togni's reagent (144 mg, 0.456 mmol) and TFA (43 mg, 0.38 mmol) were added. The resulting mixture was stirred for 24 h at 50°C. After cooling to rt, the reaction mixture was extracted with ethyl acetate (3x25 ml) and the combine organic layers were washed with water (3x60 ml) and brine (25 ml) and dried over Na₂SO₄. The organic layers were concentrated, and the residue separated by HPLC (silica C-18, MeCN-H₂O) to yield **P43** (75 mg, 45%). LCMS (ESI) [MH⁺]: 437. ¹H NMR (400 MHz, CDCl₃), δ: 9.96 (s, 1H), 7.78-7.82 (m, 2H), 7.71 (d, J=10.6 Hz, 1H), 4.33-4,26 (m, 4H), 3.64-3.54 (m, 2H), 3.53-3.42 (m, 4H), 1.94-1.84 (m, 4H), 1.48 (s, 9H).

### Preparation 44. 1-(2,7-Diazaspiro[3.5]non-2-yl)-7-(2,2,2-trifluoroethyl)phthalazine (P44)

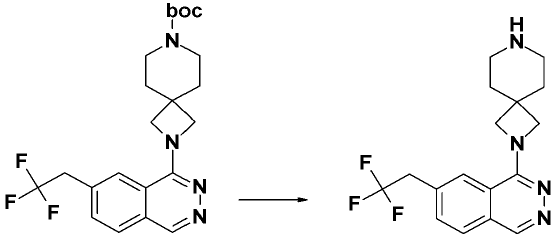

To *tert*-butyl 2-[7-(2,2,2-trifluoroethyl)phthalazin-1-yl]-2,7-diazaspiro[3.5]nonane-7-carboxylate (**P43**, 75 mg, 0.17 mmol) was added TFA (1ml). The solution stirred at rt for 2 h (LCMS control), then evaporated to dryness, water solution of sodium bicarbonate (5 ml) was added, product extracted with DCM (3x5 ml). Organic phase was dried with Na₂SO₄, concentrated to dryness to yield 1-(2,7-diazaspiro[3.5]non-2-yl)-7-(2,2,2-trifluoroethyl)phthalazine **P44** (57 mg, 100%). LCMS (ESI) [MH⁺]: 337. ¹H NMR (400 MHz, DMSO-*d₆*) δ 9.20 (s, 1H), 8.25-8.17(m, 2H), 8.17-8.08 (m, 1H), 4.60-4.40 (m, 4H), 4.11-3.98 (m, 2H), 3.17-3,08 (m, 4H), 2.12-2.03 (m, 4H).

### Synthesis of 4-(2,7-diazaspiro[3.5]non-2-yl)-6-(2,2,2-trifluoroethyl)cinnoline (P49)

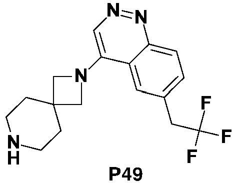

### Preparation 45. tert-Butyl 2-(6-bromocinnolin-4-yl)-2,7-diazaspiro[3.5]nonane-7-carboxylate (P45)

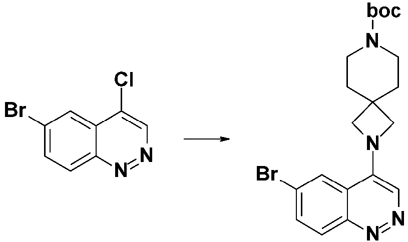

To solution of crude 6-Bromo-4-chlorocinnoline (prepared according to the procedure, described in US2015/259331, 2015, A1; 1.55 g, 6.37 mmol) in dichloroethane (40 ml) was added DIPEA (7.9 g, 61 mmol) and *tert*-butyl 2,7-diazaspiro[3.5]nonane-7-carboxylate hydrochloride (1.67 g, 6.37 mmol). Reaction solution was stirred at rt for 18 h (LCMS control). The product was purified by column chromatography on silica with ethyl acetate-methanol (10:1) yield the title compound **P45** (1.3 g, 47%), as a white solid. LCMS (ESI) [MH]⁺: 434.

### Preparation 46. tert-Butyl 2-(6-vinilcinnolin-4-yl)-2,7-diazaspiro[3.5]nonane-7-carboxylate (P46)

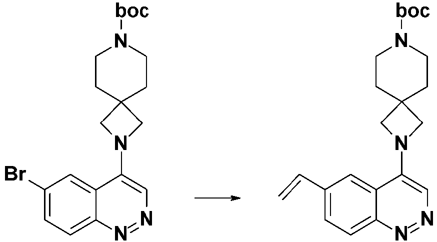

To a solution of *tert*-butyl 2-(6-bromocinnolin-4-yl)-2,7-diazaspiro[3.5]nonane-7-carboxylate (**P45**, 0.30 g, 0.69 mmol) in EtOH (10 ml) water (0.5 ml) and TEA (140 mg, 1.39 mmol) were added. Then potassium vinyltrifluoroborate (140 mg, 1.03 mmol) and dichloro[l,l'-bis(diphenylphosphino)ferrocene]palladium(H) dichloromethane adduct (0.17 g, 0.21 mmol) were added, and the reaction mixture was stirred at 80°C for 24 hours. The reaction mixture was worked up with EtOAc and H₂O, and the layers were separated. The organic layer was washed with brine, dried over MgSO₄, filtered and concentrated. The crude material was purified on silica (gradient elution, 0-40% EtOAc/hexanes) to yield the title compound **P46** as white solid (210 mg, 80%). LCMS (ESI) [MH]⁺: 381.

### Preparation 47. tert-Butyl 2-(6-fopmylcinnolin-4-yl)-2,7-diazaspiro[3.5]nonane-7-carboxylate (P47)

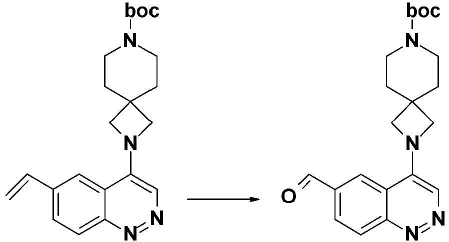

To a solution of *tert*-butyl 2-(6-vinilcinnolin-4-yl)-2,7-diazaspiro[3.5]nonane-7-carboxylate (**P46**, 210 mg, 0.55 mmol) in a mixture of dioxane (2 ml) and water (0.42 ml) 2,6-lutidine (113 mg, 1.1 mmol), solution of osmium tetraoxide (10 mg) in *tert*-BuOH (0.5 ml) and sodium periodate (354 mg, 1.65 mmol) were added. The reaction mixture was stirred at rt for 24 h, after reaction completed, the solution cooled and added to ice-cold water solution of sodium bicarbonate. The mixture was stirred at 15°C for 30 min, then extracted with DCM (2x50 ml), DCM was evaporated, residue was purified by silica gel column chromatography (DCM-ethyl acetate - 1:1) to give the title product **P47** (145 mg, 65%) as a brown solid. LCMS (ESI) [MH]⁺: 383.

### Preparation 48. tert-Butyl 2-(6-(2,2,2-trifluoroethyl)cinnolin-4-yl)-2,7-diazaspiro[3.5]nonane-7-carboxylate (P48)

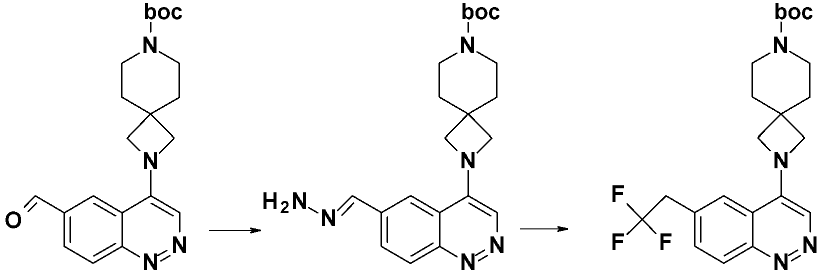

To a solution of *tert*-butyl 2-(6-fopmylcinnolin-4-yl)-2,7-diazaspiro[3.5]nonane-7-carboxylate (**P47**, 145 mg, 0.38 mmol) in MeOH (1 ml) hydrazine hydrate (38 mg, 0.76 mmol) was added. The reaction mixture was stirred at rt for 2 h (TLC control), then reaction mixture was evaporated to dryness yield crude hydrazone (150 mg) used in the next step without purification. The hydrazone (150 mg, 0.38 mmol) was dissolved in DMSO (1 ml) under argon atmosphere, Togni's reagent (144 mg, 0.456 mmol) and TFA (43 mg, 0.38 mmol) were added. The resulting mixture was stirred for 24 h at 50°C. After cooling to rt, the mixture was extracted with ethyl acetate (3x25 ml), and the combined organic layers were washed with water (3x60 ml) and brine (25 ml) and dried under Na₂SO₄. The organic layers were concentrated, and the residue separated by HPLC (silica C-18, MeCN-H₂O) to yield **P48** (75 mg, 45%). LCMS (ESI) [MH]⁺: 437. ¹H NMR (400 MHz, CDCl₃), δ: 8.35 (s, 1H), 8.29 (d, J=5.2 Hz, 1H), 7.80 (s, 1H), 7.62 (d, J=9.4 Hz, 1H), 4.29-4.17 (m, 4H), 3.60-3.43 (m, 6H), 1.92-1.85 (m, 4H), 1.49 (s, 9H).

### Preparation 49. 4-(2,7-Diazaspiro[3.5]non-2-yl)-6-(2,2,2-trifluoroethyl)cinnoline (P49)

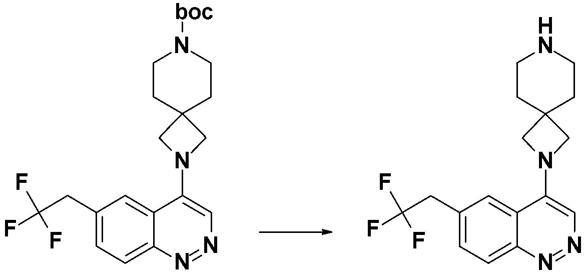

To *tert*-butyl 2-(6-(2,2,2-trifluoroethyl)cinnolin-4-yl)-2,7-diazaspiro[3.5]nonane-7-carboxylate (**P48**, 75 mg, 0.17 mmol) was added TFA (1ml). The solution was stirred at rt for 2 h (LCMS control), then evaporated to dryness. A water solution of sodium bicarbonate (5 ml) was added to the residue, product was extracted with DCM (3x5 ml). The extract was dried with sodium sulphate, concentrated to dryness yield 4-(2,7-Diazaspiro[3.5]non-2-yl)-6-(2,2,2-trifluoroethyl)cinnoline **P49** (57 mg, 100%). LCMS (ESI) [MH]⁺: 337. ¹H NMR (400 MHz, DMSO-*d₆*), δ: 8.35 (s, 1H), 8.14-8.07 (m, 1H), 8.03 (s, 1H), 7.73-7.66 (m, 1H), 4.25-4.14 (m, 4H), 4.09-3.82 (m, 6H), 2.10-1.94 (m, 4H).

### Synthesis of 4-(2,7-diazaspiro[3.5]non-2-yl)-6-(2,2,2-trifluoroethyl)pyrido[3,2-d]pyrimidine (P61)

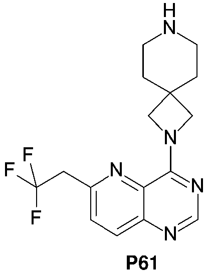

### Preparation 50. Methyl 3-aminopicolinate (P50)

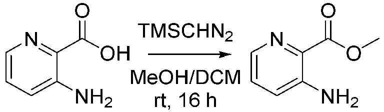

To a solution of 3-aminopicolinic acid (1.00 g, 7.24 mmol, 1.0 eq) in methanol (4.0 ml) and DCM (16 ml) was added trimethylsilyldiazomethane (7.23 ml, 14.5 mmol, 2.0 eq) at rt. Then the mixture solution was stirred at rt for 16 h, the solution was treated with water and extracted with EtOAc. The organic layers were collected, washed with brine, dried over MgSO₄(s), filtered, and concentrated in vacuo to afford **P50** (555 mg, 50%) as an orange solid. ¹H NMR (400 MHz, CDCl₃), δ: 8.06 (dd, J = 4.0, 1.4 Hz, 1H), 7.22 (dd, J = 8.4, 4.0 Hz, 1H), 7.05 (dd, J = 8.4, 1.4 Hz, 1H), 5.73 (br. s, 2H), 3.97 (s, 3H).

### Preparation 51. Methyl 3-amino-6-bromopicolinate (P51)

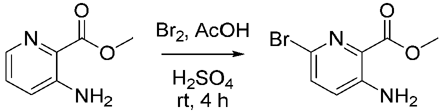

To a solution of **P50** (5.00 g, 32.8 mmol, 1.0 eq) in 2M of sulfuric acid (101 ml) was added a solution of bromine (1.68 ml, 32.8 mmol, 1.0 eq) in acetic acid (12.6 ml) at rt. After the mixture solution was stirred at rt for 4 h, the solution was treated with Na₂S₂O_{3(aq)} and extracted with EtOAc. The organic layers were collected, washed with brine, dried over MgSO₄₍ₛ₎, filtered, and concentrated in vacuo. The residue was purified by flash column chromatography (EtOAc : *n*-hexane = 1:3) to afford **P51** (5.56 g, 73%) as an orange solid. ¹H NMR (400 MHz, CDCl₃), δ: 7.34 (d, *J* = 8.6 Hz, 1H), 6.94 (d, *J* = 8.6 Hz, 1H), 5.82 (br. s, 2H), 3.95 (s, 3H).

### Preparation 52. Methyl 3-amino-6-vinylpicolinate (P52)

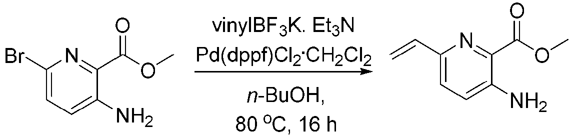

To a stirred solution of **P51** (500 mg, 2.16 mmol, 1.0 eq) in *n*-butanol (10 ml) was added potassium vinyltrifluoroborate (435 mg, 3.25 mmol, 1.5 eq) and trimethylamine (1.51 ml, 10.8 mmol, 5.0 eq) at rt. The solution was degassed with argon, and then was added by 1,1'-bis(diphenylphosphino)ferrocene-palladium(II)dichloride dichloromethane complex. Then the reaction mixture was stirred at 80°C for 16 h, it was cooled to rt and filtered through with a pad of celite. The solution was treated with water and extracted with EtOAc. The organic layers were collected, washed with brine, dried over MgSO₄(s), filtered, and concentrated in vacuo. The residue was purified by flash column chromatography (EtOAc : *n*-hexane = 1:2) to afford **P52** (230 mg, 60%) as a yellow solid. ¹H NMR (400 MHz, CDCl₃), δ: 7.47 (d, J = 8.8 Hz, 1H), 7.04 (d, J = 8.8 Hz, 1H), 6.81 (dd, J = 17.6, 10.8 Hz, 1H), 5.86 (dd, J = 17.6, 0.8 Hz, 1H), 5.79 (br. s, 2 H), 5.36 (dd, J = 10.8, 0.8 Hz, 1H), 3.97 (s, 3H); LCMS (ESI) [MH]⁺: 179.1.

### Preparation 53. Methyl 3-(di-tert-butoxycarbonylamino)-6-vinylpicolinate (P53)

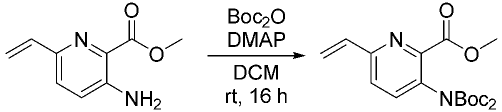

To a solution of **P52** (230 mg, 1.29 mmol, 1.0 eq) in DCM (10 ml) was added di-*tert*-butyl dicarbonate (0.890 ml, 3.87 mol, 3.0 eq) and 4-dimethylaminopyridine (15.7 mg, 0.129 mmol, 0.1 eq) at rt. After the reaction mixture was stirred at rt for 16 h, it was concentrated in vacuo. The residue was purified by flash column chromatography (EtOAc : *n*-hexane = 1:3) to afford **P53** (432 mg, 88%) as a white solid. ¹H NMR (400 MHz, CDCl₃), δ: 7.54 (m, 2H), 6.91 (dd, J = 17.6, 11.2 Hz, 1H), 6.22 (d, J = 17.6 Hz, 1H), 5.60 (d, J = 11.2 Hz, 1H), 3.94 (s, 3H), 1.38 (s, 18H); LCMS (ESI) [MH]⁺: 379.2.

### Preparation 54. Methyl 3-(di-tert-butoxycarbonylamino)-6-formylpicolinate (P54)

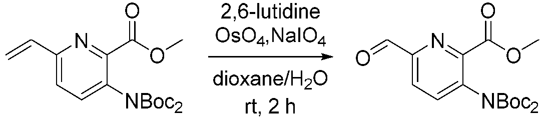

To a solution of **P53** (432 mg, 1.14 mmol, 1.00 eq) in 1,4-dioxane (10 ml) and water (2.5 ml) was added 2.5% osmium tetroxide in *tert*-butanol (0.226 ml, 0.0228 mmol, 0.02 eq), sodium periodate (977 mg, 4.56 mmol, 4.00 eq), 2,6-lutidine (265 ml, 2.28 mmol, 2.00 eq) at room temperature and stirred at room temperature for 2 h. The mixture solution was filtered through celite and extracted with EtOAc. The organic layers were collected, washed with brine, dried over MgSO₄₍ₛ₎, filtered, and concentrated in vacuo. The residue was purified by flash column chromatography (EtOAc : dichloromethane = 1:4) to afford **P54** (402 mg, 93%) as a yellow solid. ¹H NMR (400 MHz, CDCl₃) δ 10.15 (s, 1H), 8.13 (d, *J* = 8.0 Hz, 1H), 7.77 (dd, *J* = 8.0 Hz, 1H), 4.00 (s, 3H), 1.39 (s, 18H).

### Preparation 55. Methyl (E)-3-(di-tert-butoxycarbonylamino)-6-(hydrazineylidenemethyl)-picolinate (P55)

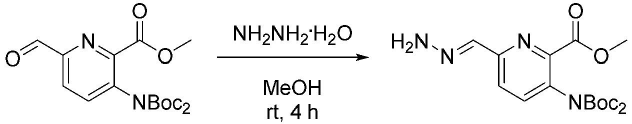

To a solution of **P54** (402 mg, 1.06 mmol, 1.0 eq) in methanol (9 ml) was added hydrazine monohydrate (63.5 mg, 1.27 mol, 1.2 eq) at rt and the mixture solution was stirred at room temperature for 4 h. The solution was concentrated in vacuo to afford **P55** (428 mg) as a yellow solid which was used in next step without purification.

### Preparation 56. Methyl 3-(di-tert-butoxycarbonylamino)-6-(2,2,2-trifluoroethyl)picolinate (P56)

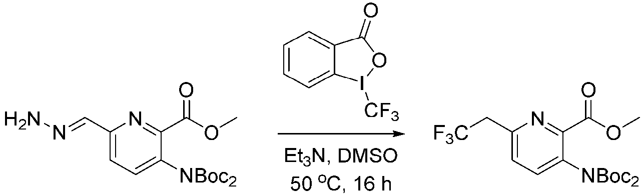

A solution of **P55** (428 mg, 1.08 mmol, 1.0 eq) in DMSO (10 ml) was added 1-trifluoromethyl-1,2-benziodoxol-3-(1*H*)-one (377 mg, 1.19 mmol, 1.1 eq) and the mixture was stirred for 10 min. Triethylamine (0.197 ml, 1.41 mmol, 1.3 eq) was added to the reaction mixture and the solution was stirred at 50°C for 16 h. After the solution was cooled to rt, it was diluted with water, and extracted with EtOAc. The organic layers were washed with brine, dried over MgSO₄₍ₛ₎, filtered, and concentrated in vacuo. The residue was purified by flash column chromatography (EtOAc : *n*-hexane = 1:2) to afford **P56** (258 mg, crude) as a yellow solid. ¹H NMR (400 MHz, CDCl₃), δ: 7.60 (d, *J* = 8.0 Hz, 1H), 7.53 (d, *J* = 8.0 Hz, 1H), 3.95 (s, 3H), 2.80 (q, *J* = 10.4 Hz, 2H), 1.38 (s, 18H); LRMS (ESI) [MH]⁺: 435.1.

### Preparation 57. Methyl 3-amino-6-(2,2,2-trifluoroethyl)picolinate (P57)

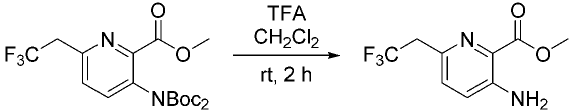

To a solution of **P56** (3.6 g, 8.29 mmol, 1.0 eq) in DCM (20 ml) was added trifluoroacetic acid (10 ml) and the mixture was stirred at rt for 2 h. The solution was concentrated in vacuo and purified by flash column chromatography (EtOAc : *n*-hexane = 1:1) to afford **P57** (1.9 g, 98%) as a yellow solid. ¹H NMR (400 MHz, CDCl₃), δ: 7.28 (d, *J* = 8.6 Hz, 1H), 7.07 (d, *J* = 8.6 Hz, 1H), 3.97 (s, 3H), 3.57 (q, *J* = 10.8 Hz, 2H); LCMS (ESI) [MH]⁺: 235.1.

### Preparation 58. 3-Amino-6-(2,2,2-trifluoroethyl)picolinamide (P58)

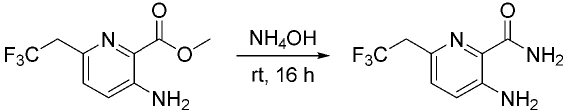

A solution of **P57** (1.90 g, 8.11 mmol, 1.0 eq) in 28% of ammonia in water (50 ml) was stirred at rt for 16 h. The solution was diluted with water and extracted with EtOAc. The organic layers were collected, washed with brine, dried over MgSO₄(s), filtered, and concentrated in vacuo to afford **P58** (1.45 g, 81%) as a yellow solid. ¹H NMR (400 MHz, CDCl₃), δ: 7.82 (br. s, 1H), 7.19 (d, J = 8.4 Hz, 1H), 7.00 (d, J = 8.4 Hz, 1H), 5.98 (br. s, 2H), 5.42 (br. s, 1H), 3.45 (q, J = 10.8 Hz, 2H).

### Preparation 59. 6-(2,2,2-Trifluoroethyl)pyrido[3,2-d]pyrimidin-4(3H)-one (P59)

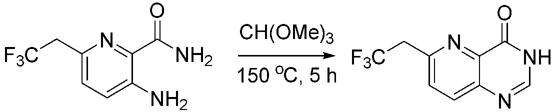

A solution of **P58** (50.0 mg, 0.114 mmol, 1.0 eq) in trimethyl orthoformate (5.0 ml) in sealed tube was stirred at 150°C for 5 h. After the solution was cooled to rt, it was diluted with water and extracted with EtOAc. The organic layers were collected, washed with brine, dried over MgSO₄₍ₛ₎, filtered, and concentrated in vacuo. The residue was purified by flash column chromatography (methanol : DCM = 1 : 10) to afford **P59** (38 mg, 73%) as a pale-yellow solid. ¹H NMR (400 MHz, CDCl₃), δ: 12.30 (br. s, 1H), 8.23 (s, 1H), 8.17 (d, *J* = 8.4 Hz, 1H), 7.82 (d, *J* = 8.4 Hz, 1H), 3.91 (q, *J* = 10.4 Hz, 2H); LCMS (ESI) [MH]⁺: 230.1.

### Preparation 60. tert-Butyl 2-(6-(2,2,2-trifluoroethyl)pyrido[3,2-d]pyrimidin-4-yl)-2,7-diazaspiro[3.5]nonane-7-carboxylate (P60)

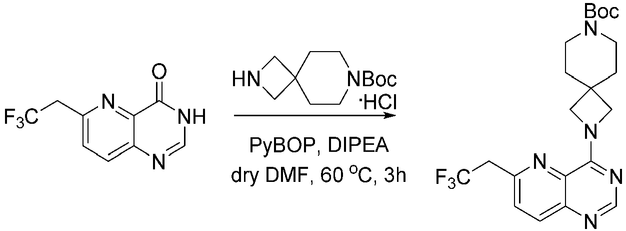

To a solution of **P59** (410 mg, 1.79 mmol, 1.0 eq) in anhydrous *N*,*N*-dimethylformamide (10 ml) was added *tert*-butyl2,7-diazaspiro[3.5]nonane-7-carboxylatehydrochloride (564 mg, 2.15 mmol, 1.2 eq), benzotriazol-1-yl-oxytripyrrolidinophosphonium hexafluorophosphate (1.86 g, 3.58 mmol, 2.0 eq) and *N*,*N*-diisopropylethylamine (0.934 mg, 5.36 mmol, 3.0 eq). After the reaction mixture was stirred at 60°C for 3 h, it was cooled to rt and directly purified by C18 flash column chromatography (0-100% methanol in H₂O) to afford **P60** (580 mg, 74%) as an orange solid. ¹H NMR (400 MHz, CDCl₃), δ: 8.55 (s, 1H), 8.05 (d, *J* = 8.6 Hz, 1H), 7.58 (d, *J* = 8.6 Hz, 1H), 4.61 (s, 2H), 4.08 (s, 2H), 3.69 (q, *J* = 10.4 Hz, 2H), 3.53-3.48 (m, 2H), 3.41-3.36 (m, 2H), 1.85-1.83 (m, , 4H), 1.47 (s, 9H); LCMS (ESI) [MH]⁺: 438.2.

### Preparation 61. 4-(2,7-Diazaspiro[3.5]non-2-yl)-6-(2,2,2-trifluoroethyl)pyrido[3,2-d]pyrimidine (P61)

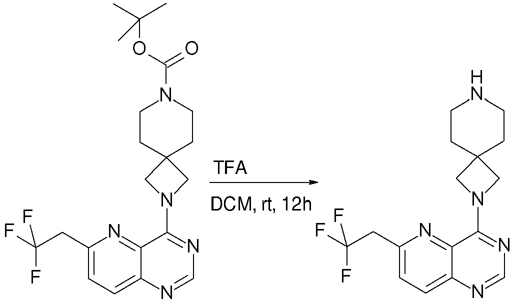

To a stirred solution of **P60** (330 mg, 0.68 mmol) in DCM (3 ml) was added TFA (0.860 g, 10 eq). After the reaction was stirred at rt overnight and the solution was treated with sat. NaHCO₃ and extracted with EtOAc. The combined organic layers were dried over with MgSO₄(s), filtered, and concentrated to give the product **P61** as yellow solid (240 mg, 94%). LCMS (ESI) [MH]⁺: 338.4.

### Synthesis of 4-(2,7-diazaspiro[3.5]non-2-yl)-6-(2,2,2-trifluoroethyl)pteridine (P71)

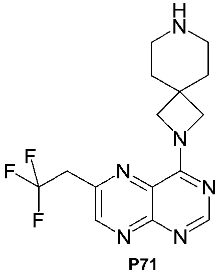

### Preparation 62. Methyl 3-amino-6-vinyl-pyrazine-2-carboxylate (P62)

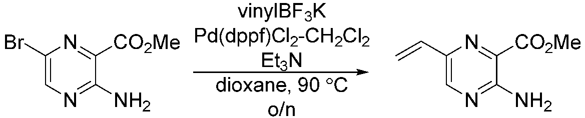

To a stirred solution of methyl 3-amino-6-bromopyrazine-2-carboxylate (12.0 g, 51.72 mmol) in dioxane (120 ml) was added potassium vinyltrifluoroborate (10.4 g, 77.6 mmol) and triethylamine (37.7 ml, 259 mmol). The solution was purged with argon for 30 min, and then [1,1'-bis(diphenylphosphino)ferrocene]dichloropalladium(II) dichloromethane complex (4.23 g, 5.17 mmol) was added and purged again. The mixture was stirred at reflux for 16 h. Monitoring by TLC showed the reaction was completed. The solution was treated with ethyl acetate and washed with NaHCO₃(aq) and water. The organic phase was dried over with MgSO₄(s), filtered, and concentrated in vacuo to give a crude product. The residue was purified by silica gel column chromatography (25 % EtOAc in *n*-hexane) to give methyl 3-amino-6-vinylpyrazine-2-carboxylate (**P62**, 7.5 g, 75% yield) as a yellow solid.

### Preparation 63. Methyl 3-(di(tert-butoxycarbonyl)amino)-6-vinylpyrazine-2-carboxylate (P63)

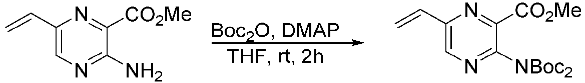

To a solution of **P62** (7.5 g, 41.9 mmol) in tetrahydrofuran (100 ml) was added di-*tert-*butyl dicarbonate (48.2 ml, 209.5 mmol) and 4-dimethylaminopyridine (512 mg, 4.19 mmol).The reaction was stirred at rt for overnight. It showed starting materials was consumed. It was concentrated to remove solvent. The mixture was extracted with EtOAc/Water and purified by silica-gel column chromatography (25% EtOAc in *n-*Hexane) to give **P63** (14.57 g, 92% yield) as a yellow solid. ¹H NMR (400 MHz, CDCl₃), δ: 8.67 (s, 1H), 6.92 (dd, J = 17.6 Hz, 11.0 Hz, 1H), 6.41 (d, J = 17.6 Hz, 1H), 5.75 (d, J = 11.0 Hz, 1H), 3.98 (s, 3H), 1.39 (s, 18H).

### Preparation 64. Methyl 3-(di(tert-butoxycarbonyl)amino)-6-formylpyrazine-2-carboxylate (P64)

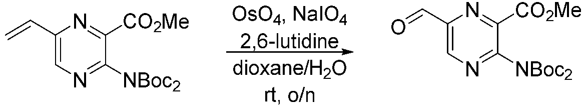

To a stirred solution of **P63** (14.0 g, 36.9 mmol) in dioxane (400 ml) and H₂O 100 ml) 2,6-lutidine (8.6 ml, 73.8 mmol), a solution of 2.5% osmium tetroxide in *tert*-butanol (188 mg, 0.74 mmol), and sodium periodate (31.59 g, 147.6 mmol) were added at rt. The mixture was stirred at rt for 16 h. Monitoring by TLC showed the reaction was completed. The resulting mixture was filtered, and the filtrate was treated with water and extracted with EtOAc. The organic layer was dried over with MgSO₄(s), filtered and concentrated in vacuo to give a crude product. The residue was purified by silica gel column chromatography (25 % EtOAc in *n*-Hexane) to give **P64** (9.96 g, 71% yield) as a brown solid. ¹H NMR (400 MHz, CDCl₃), δ: 10.23 (s, 1H), 9.18 (s, 1 H), 4.03 (s, 3H), 1.42 (s, 18H).

### Preparation 65. Methyl (E)-3-(di(tert-butoxycarbonyl)amino)-6-(hydrazineylidenemethyl)pyrazine-2-carboxylate (P65)

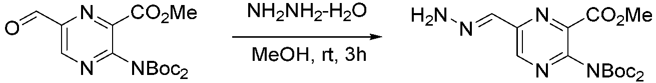

To a stirred solution of **P64** (9.96 g, 26.1 mmol) in methanol (50 ml) was added hydrazine monohydrate (1.52 ml, 31.3 mmol) at 0°C and stirred for 10 min. The solution was warmed to rt and stirred for another 5 h. The solution was concentrated to give **P65** as a crude product which was used in next step without further purification. ¹H NMR (400 MHz, CDCl₃), δ: 9.11 (s, 1H), 7.86 (s, 1 H), 6.11 (br. s, 2H), 3.98 (s, 3H), 1.39 (s, 18H).

### Preparation 66. Methyl 3-(di(tert-butoxycarbonyl)amino)-6-(2,2,2-trifluoroethyl)pyrazine-2-carboxylate (P66)

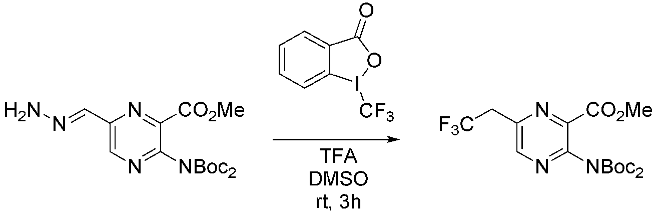

To a stirred solution of **P65** (26.1 mmol) in DMSO (70 ml) was added 1-(trifluoromethyl)-1,2-benziodoxol-3(1H)-one (1.52 ml, 31.3 mmol) and TFA (2.0 ml) at 0°C and stirred for 10 min. The solution was warmed to rt and stirred for 3 h. The mixture was treated with water and extracted with EtOAc. The combined organic layers were dried over with MgSO₄(s), filtered, and concentrated to give a residue. The residue was purified by silica-gel column chromatography (20-33% EtOAc in *n*-hexane) to give **P66** (4.54 g, 40% yield) as a white solid. ¹H NMR (400 MHz, CDCl₃), δ: 8.68 (s, 1H), 3.99 (s, 3 H), 3.80 (q, J = 10.4 Hz, 2H), 1.40 (s, 18H).

### Preparation 67. Methyl 3-amino-6-(2,2,2-trifluoroethyl)pyrazine-2-carboxylate (P67)

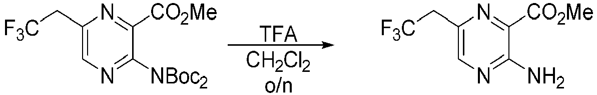

To a stirred solution of **P66** (4.54 g, 10.43 mmol) in DCM (20 ml) was added trifluoroacetic acid (10.0 ml) at rt and stirred for overnight. The mixture was concentrated to give **P67** as a product which was used in next step without further purification. ¹H NMR (400 MHz, DMSO-*d₆*), δ: 8.30 (s, 1H), 7.43 (br. s, 2H), 3.84 (s, 3 H), 3.71 (q, J = 11.2 Hz, 2H).

### Preparation 68. 3-Amino-6-(2,2,2-trifluoroethyl)pyrazine-2-carboxamide (P68)

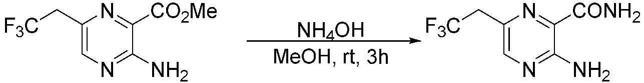

To a stirred solution of **P67** (11.28 mmol) in methanol (40 ml) 33% ammonium hydroxide solution (10 ml) was added at rt and stirred for 3 h. The solution was concentrated to give **P68** as a yellow solid which was used in next step without further purification. ¹H NMR (400 MHz, DMSO-*d₆*), δ: 8.23 (s, 1H), 7.89 (br. s, 1H), 7.68 (br. s, 1 H), 3.68 (q, J = 11.2 Hz, 2H).

### Preparation 69. 6-(2,2,2-Trifluoroethyl)pteridin-4(3H)-one (P69)

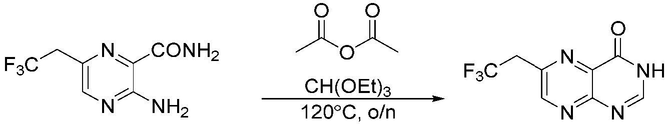

To a stirred solution of **P68** (11.28 mmol) in triethyl orthoformate (24.4 ml) acetic anhydride (12.3 ml) was added at rt and heated to 120°C. After the mixture was stirred for 3 h at 120°C, the solution was cooled to rt and concentrated. The crude product was treated with isopropanol. The resulting precipitate was collected by filtration and dried under air to give **P69** (1.96 g, 76%) as an apricot solid. ¹H NMR (400 MHz, DMSO-*d₆*), δ: 12.89 (br. s, 1H), 9.04 (s, 1H), 8.37 (s, 1 H), 4.12 (q, J = 11.4 Hz, 2 H); HPLC purity: 100.0 %, Rt = 8.434 min.

### Preparation 70. tert-Butyl 2-(6-(2,2,2-trifluoroethyl)pteridin-4-yl)-2,7-diazaspiro[3.5]nonane-7-carboxylate (P70)

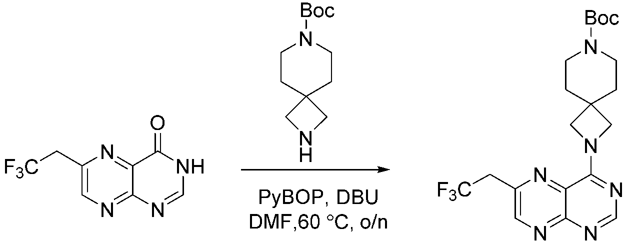

To a stirred solution of **P69** (800 mg, 3.48 mmol) in *N*,*N*-dimethylformamide (100 ml) was added *tert*-butyl 2,7-diazaspiro[3.5]nonane-7-carboxylate hydrochloride (1.094 g, 4.18 mmol), *N*,*N*-diisopropylethylamine (1.82 ml) and (benzotriazol-1-yloxy)tripyrrolidinophosphonium hexafluorophosphate (5.44 g, 10.44 mmol). After the reaction was stirred at 60°C for overnight and cooled to rt, the solution was treated with water and extracted with EtOAc. The combined organic layers were dried over with MgSO₄(s), filtered, and concentrated to give a residue. The residue was purified by C18 reverse phase column (0-75% MeOH in water) to give a crude solid. The solid was suspended in a solution (10% EtOAc in *n*-hexane). The resulting precipitate was collected by filtration to afford **P70** (799 mg, 52 % yield) as a yellow solid. ¹H NMR (400 MHz, DMSO-*d₆*), δ: 8.92 (s, 1H), 8.71 (s, 1H), 4.57 (s, 2 H), 4.13 (s, 2 H), 3.76 (q, J = 10.4 Hz, 2H), 3.53-3.48 (m, 2H), 3.42-3.35 (m, 2H), 1.86-1.84 (m, 4H), 1.47 (s, 9H); LCMS (ESI) [MH]⁺: 439.2; HPLC purity: 98.7 %, Rt = 20.806 min.

### Preparation 71. 4-(2,7-Diazaspiro[3.5]non-2-yl)-6-(2,2,2-trifluoroethyl)pteridine (P71)

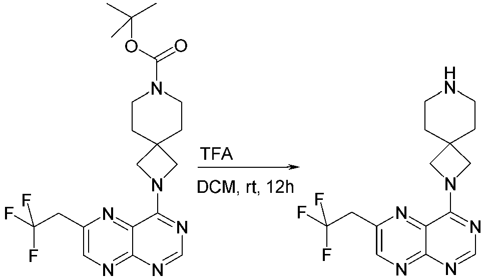

To a stirred solution of **P70** (300 mg, 0.68 mmol) in DCM (3 ml) was added TFA (0.78 g, 10 eq). After the reaction was stirred at rt overnight and the solution was treated with sat. NaHCO₃ and extracted with EtOAc. The combined organic layers were dried over with MgSO₄(s), filtered, and concentrated to give the product **P71** as yellow solid (224mg, 97%). LCMS (ESI) [MH]⁺: 339.2.

### Synthesis of 4-(2,7-diazaspiro[3.5]non-2-yl)-6-(2,2,2-trifluoroethyl)pyrido[3,4-d]pyrimidine (P85)

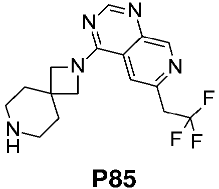

### Preparation 72. 2-Bromo-5-nitroisonicotinic acid (P72)

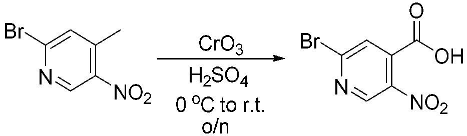

To a mixture of 2-bromo-4-methyl-5-nitro-pyridine (10.0 g, 46.5 mmol) and sulfuric acid (100 ml) was added chromium trioxide (15.5 g, 153 mmol) slowly in ice bath. The mixture was stirred at rt for overnight. The mixture was quenched by water slowly in ice bath. The resulting solid was collected by filtration and washed with water to afford **P72** (9.15 g, 80%) as a white solid. ¹H NMR (400 MHz, DMSO-*d₆*), δ: 9.10 (s, 1H), 8.13 (s, 1H), 4.59 (br. s, 1H).

### Preparation 73. Methyl 2-bromo-5-nitroisonicotinate (P73)

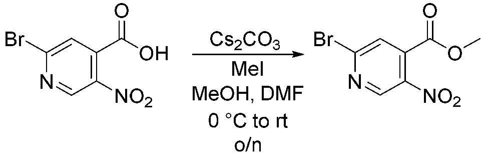

A mixture of **P72** (9.15 g, 37.2 mmol) and cesium carbonate (6.04 g, 18.6 mmol) in MeOH (75 ml) was stirred for 30 min, and then the solution was concentrated. The mixture was treated with DMF (75 ml) and methyl iodide (2.80 ml, 44.6 mmol) in ice bath. The reaction was stirred at rt for overnight. The mixture was quenched by water slowly in ice bath. The resulting solid was collected by filtration and washed with water to afford **P73** (9.20 g, 95%) as a white solid. ¹H NMR (400 MHz, DMSO-*d*₆), δ: 9.17 (d, *J* = 0.4 Hz, 1H), 8.22 (d, *J* = 0.4 Hz, 1H), 3.90 (s, 3H).

### Preparation 74. Methyl 5-amino-2-bromoisonicotinate (P74)

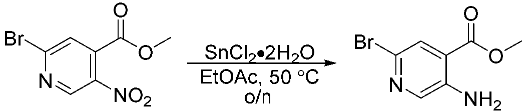

A mixture of **P73** (9.20 g, 35.4 mmol) and tin(II) chloride dihydrate (39.91 g, 176.9 mmol) in EtOAc (118 ml) was stirred at 50°C for overnight. The mixture was quenched by NaHCO_{3(aq)} in ice bath. The solution was filtered through a pad of celite and washed with EtOAc. The filtrate was extracted with EtOAc. The combined organic layers were dried over MgSO₄₍ₛ₎, filtered, and concentrated under reduced pressure to afford **P74** (8.00 g, 98%) as a yellow solid which was used in next step without further purification. ¹H NMR (400 MHz, DMSO-*d*₆), δ: 8.04 (s, 1H), 7.59 (s, 1H), 6.79 (s, 2H, NH₂), 3.28 (s, 3H).

### Preparation 75. Methyl 5-amino-2-vinylisonicotinate (P75)

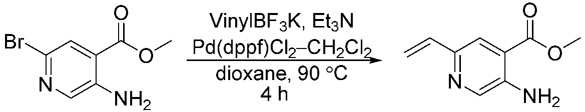

A mixture of **P74** (8.00 g, 30.7 mmol), potassium vinyltrifluoroborate (6.16 g, 46.0 mmol) and triethylamine (21.4 ml, 15.3 mmol) in dioxane (123 ml) was degassed with argon for 15 min. The mixture was treated with 1,1'-bis(diphenylphosphino)ferrocene-palladium(II)dichloride dichloromethane complex (2.48 g, 3.07 mmol). The reaction mixture was stirred at 90°C for 4 h. The mixture was filtered with a pad of celite and concentrated under reduced pressure. The residue was purified by silica gel column chromatography (0-50% EtOAc in *n*-hexane) to afford **P75** (5.8 g, 94%) as a yellow solid. ¹H NMR (400 MHz, CDCl₃), δ: 8.16 (s, 1H), 7.64 (s, 1H), 6.70 (dd, *J* = 17.6, 10.8 Hz, 1H), 5.98 (dd, *J* = 17.4, 1.2 Hz, 1H), 5.68 (s, 2H), 5.26 (dd, *J* = 10.8, 1.2 Hz, 1H), 3.91 (s, 3H).

### Preparation 76. Methyl 5-(di-tert-butoxycarbonylamino)-2-vinylisonicotinate (P76)

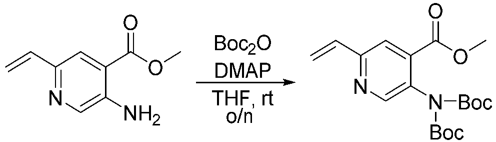

A mixture of **P75** (5.80 g, 32.6 mmol), di-*tert*-butyl dicarbonate (28.4 g, 130 mmol) and DMAP (0.40 g, 3.3 mmol) in THF (129 ml) was stirred at rt for overnight. The mixture was concentrated under reduced pressure. The mixture was treated with water and extracted with EtOAc. The combined organic layers were dried over MgSO₄₍ₛ₎, filtered, and concentrated under reduced pressure. The residue was purified by silica gel column chromatography (0-50% EtOAc in *n*-hexane) to afford **P76** (8.3 g, 69%) as a yellow solid. ¹H NMR (400 MHz, CDCl₃), δ: 8.42 (s, 1H), 7.81 (s, 1H), 6.83 (dd, *J* = 17.4, 10.8 Hz, 1H), 6.29 (dd, *J* = 17.4, 1.0 Hz, 1H), 5.58 (dd, *J* = 10.8, 1.0 Hz, 1H), 3.90 (s, 3H), 1.36 (s, 18H).

### Preparation 77. Methyl 5-(di-tert-butoxycarbonylamino)-2-formylisonicotinate (P77)

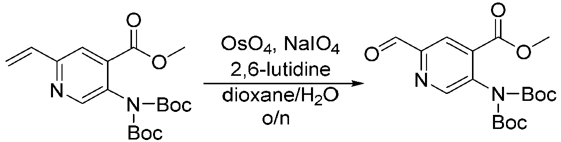

To a mixture of **P76** (8.30 g, 22.5 mmol) in dioxane (180 ml) and water (45 ml) was added sodium periodate (19.2 g, 89.8 mmol), 2,6-lutidine (5.2 ml, 44.92 mmol) and osmium tetroxide (2.5% in *tert*-BuOH, 4.5 ml, 0.45 mmol). After the solution was stirred at rt for overnight, the mixture was filtered and washed with EtOAc. The organic layer was dried over MgSO₄₍ₛ₎, filtered, and concentrated under reduced pressure. The residue was purified by silica gel column chromatography (0-35% EtOAc in *n*-hexane) to afford **P77** (6.23 g, 74%) as a yellow solid. ¹H NMR (400 MHz, DMSO-*d*₆): δ 10.02 (s, 1H), 8.92 (d, *J* = 0.8 Hz, 1H), 8.23 (d, *J* = 0.8 Hz, 1H), 3.87 (s, 3H), 1.31 (s, 18H); LCMS (ESI) [MH]⁺: 381.2.

### Preparation 78. Methyl 5-(di-tert-butoxycarbonylamino)-(E)-2-(hydrazonomethyl)isonicotinate (P78)

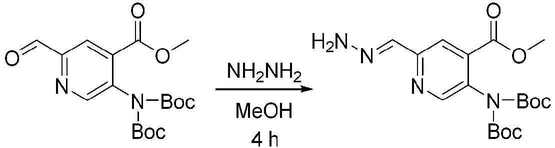

A solution of **P77** (6.23 g, 16.4 mmol) and hydrazine (0.90 ml, 20 mmol) in MeOH (126 ml) was stirred at rt for 4 h. The solution was concentrated under reduced pressure to afford **P78** (6.50 g, crude) as a yellow oil which was used in next step without further purification. ¹H NMR (400 MHz, DMSO-*d₆*), δ: 8.41 (s, 1H), 8.05 (s, 1H), 7.72 (s, 1H), 7.58 (s, 2H), 3.82 (s, 3H), 1.31 (s, 18H).

### Preparation 79. Methyl 5-(di-tert-butoxycarbonylamino)-2-(2,2,2-trifluoroethyl)isonicotinate (P79)

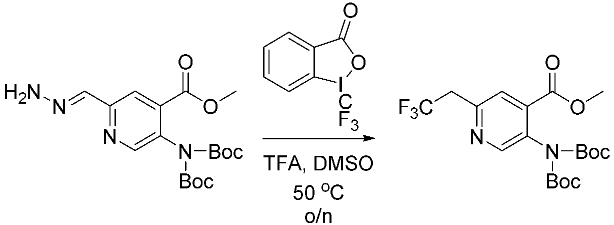

To a solution of **P78** (6.50 g, 16.4 mmol) and TFA (1.20 ml, 16.44 mmol) in DMSO (63.5 ml) was added 1-(trifluoromethyl)-1,2 benziodaoxol-3(1H)-one (5.69 g, 18.0 mmol) in ice bath. After the reaction was stirred at rt for overnight, the mixture was treated with NaHCO₃ (aq.) and extracted with EtOAc. The combined organic layers were dried over MgSO₄(s), filtered, and concentrated under reduced pressure. The residue was purified by silica gel column chromatography (0-50% EtOAc in *n*-hexane) to afford **P79** (4.3 g, 60%) as a yellow solid. ¹H NMR (400 MHz, CDCl₃), δ: 8.48 (s, 1H), 7.83 (s, 1H), 3.91 (s, 3H), 3.68 (q, J = 10.4 Hz, 2H), 1.37 (s, 18H).

### Preparation 80. Methyl 5-amino-2-(2,2,2-trifluoroethyl)isonicotinate (P80)

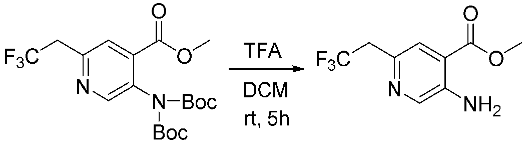

A mixture of **P79** (4.30 g, 16.4 mmol) and TFA (16.5 ml) in DCM (33 ml) was stirred at rt for 5 h. The mixture was treated NaHCO_{3(aq} and extracted with DCM. The combined organic layers were dried over MgSO₄₍ₛ₎, filtered, and concentrated under reduced pressure to afford **P80** (2.18 g, crude) as a yellow oil which was used in next step without further purification. ¹H NMR (400 MHz, DMSO-*d₆*), δ: 8.21 (s, 1H), 7.55 (s, 1H), 6.74 (s, 2H), 3.83 (s, 3H), 3.61 (q, *J* = 11.6 Hz, 2H).

### Preparation 81. 5-Amino-2-(2,2,2-trifluoroethyl)isonicotinic acid (P81)

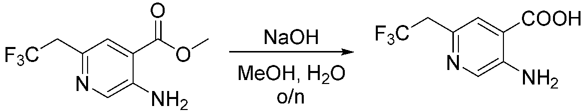

A solution of **P80** (2.18 g, 9.31 mmol) and sodium hydroxide (1M solution in water, 25.9 ml, 25.9 mmol) in MeOH (26 ml) was stirred at rt for overnight. Dowax H⁺ was added to the mixture. The solution was filtered, washed with EtOAc and MeOH, and concentrated under reduced pressure to afford **P81** (1.94 g) as a yellow oil which was used in next step without further purification. ¹H NMR (400 MHz, DMSO-*d₆*), δ: 8.03 (s, 1H), 7.56 (s, 1H), 3.54 (q, *J* = 11.6 Hz, 2H); LRMS (ESI) [MH]⁺: 221.1.

### Preparation 82. 5-Amino-2-(2,2,2-trifluoroethyl)isonicotinamide (P82)

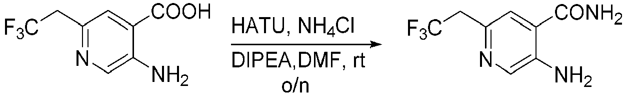

A mixture of **P81** (1.94 g, 8.82 mmol), ammonium chloride (1.40 g, 26.45 mmol), HATU (6.70 g, 17.64 mmol), and DIPEA (3.1 ml, 18 mmol) in DMF (38 ml) was stirred at rt for overnight. The mixture was treated with water and extracted with EtOAc. The combined organic layers were dried over MgSO₄₍ₛ₎, filtered, and concentrated under reduced pressure. The residue was purified by silica gel column chromatography (0-75% EtOAc in *n*-hexane containing 1% of triethylamine) to afford **P82** (1.0 g, 51%) as a yellow solid. ¹H NMR (400 MHz, DMSO-*d*₆), δ: 8.09 (s, 1H), 7.99 (br. s, 1H), 7.45 (s, 2H), 6.62 (s, 2H), 3.50 (q, *J* = 11.2 Hz, 2H).

### Preparation 83. 6-(2,2,2-Trifluoroethyl)pyrido[3,4-d]pyrimidin-4(3H)-one (P83)

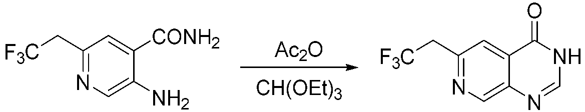

A mixture of **P82** (1.0 g, 4.7 mmol) and acetic anhydride (6.5 ml, 69 mmol) in triethyl orthoformate (13 ml) was stirred at 120°C for 6 h. The mixture was concentrated under reduced pressure. The residue was purified by silica gel column chromatography (0-100% EtOAc in *n*-hexane with 1% of triethylamine) to afford **P83** (0.80 g, 74%) as a yellow solid. ¹H NMR (400 MHz, DMSO-*d₆*), δ: 9.03 (s, 1H), 8.24 (s, 1H), 8.05 (s, 1H), 3.98 (q, *J* = 11.2 Hz, 2H); LRMS (ESI) [MH]⁺: 230.1; HPLC purity: 91.5%, *t*_{R} = 11.00 min.

### Preparation 84. tert-Butyl 2-(6-(2,2,2-trifluoroethyl)pyrido[3,4-d]pyrimidin-4-yl)-2,7-diazaspiro[3.5]nonane-7-carboxylate (P84)

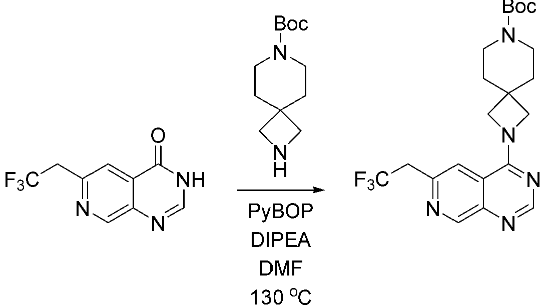

A mixture of **P83** (0.30 g, 1.3 mmol), *tert*-butyl 2,7-diazaspiro[3.5]nonane-7-carboxylate (549 mg, 2.10 mmol), benzotriazol-1-yloxytripyrrolidinophosphonium hexafluorophosphate (1.09 g, 2.10 mmol) and DIPEA (1.2 ml, 6.5 mmol) in DMF (6.0 ml) was stirred at 130°C for 6 h. The mixture was treated with water and extracted with EtOAc. The organic layers were washed with brine, dried over Na₂SO₄₍ₛ₎, filtered, and concentrated under reduced pressure. The residue was purified by silica gel column chromatography (0-100% EtOAc in *n*-hexane with 1% of triethylamine) to afford **P84** (169 mg, 30%) as a yellow solid. ¹H NMR (400 MHz, CDCl₃), δ: 9.23 (s, 1H), 8.69 (s, 1H), 7.62 (s, 1H), 4.29 (s, 4H), 3.74 (q, *J* = 10.6 Hz, 2H), 3.46 (s, 4H), 1.88-1.85 (m, 4H), 1.48 (s, 9H); LRMS (ESI) [MH]⁺: 438.2; HPLC purity: 97.07%, *t*_{R} = 19.55 min.

### Preparation 85. 4-(2,7-Diazaspiro[3.5]non-2-yl)-6-(2,2,2-trifluoroethyl)pyrido[3,4-d]pyrimidine (P85)

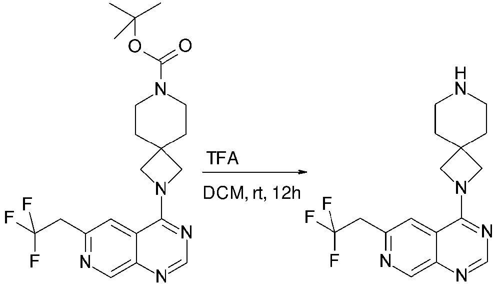

To a stirred solution of **P84** (150 mg, 0.34 mmol) in DCM (3 ml) was added TFA (0.391 g, 10 eq). After the reaction was stirred at rt overnight and the solution was treated with sat. NaHCO₃ and extracted with EtOAc. The combined organic layers were dried over with MgSO4(s), filtered, and concentrated to give the product **P85** as yellow solid (85 mg, 73%). LCMS (ESI) [MH]⁺: 338.3.

### Synthesis of 5-formyl-4-methyl-1-[(2-oxohexahydropyrimidin-5-yl)methyl]indole-2-carbonitrile (P87)

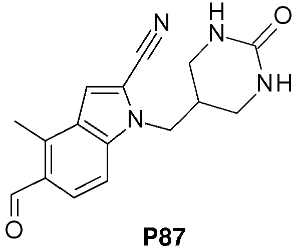

### Preparation 86. 5-(Hydroxymethyl)tetrahydropyrimidin-2(1H)-one (P86)

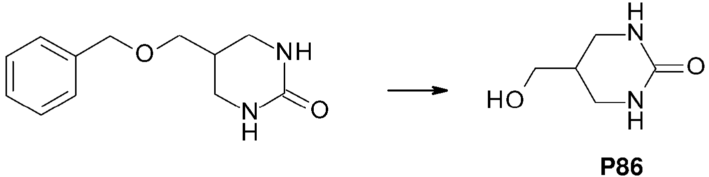

A solution of 5-[(benzyloxy)methyl]tetrahydropyrimidin-2(1H)-one (229 mg, 1.04 mmol, prepared using the procedure described in WO2015/62486, 2015, A1) in MeOH (30 ml) was stirred with Pd-C 10% (0.25 g) under hydrogen (70 bar) for 18 h. Then the reaction mixture was filtered, evaporated to dryness to yield 5-(hydroxymethyl)tetrahydropyrimidin-2(1H)-one (**P86**, 130 mg, 98%). ¹H NMR (400 MHz, DMSO-*d₆*), δ: 6.02 (s, 2H), 4.67 (m, 1H), 3.14 (m, 2H), 2.85 (m, 2H), 2.07 (m,2H), 1.85 (m, 1H).

### Preparation 87. 2-Ethynyl-4-methyl-1-[(2-oxohexahydropyrimidin-5-yl)methyl]-1H-indole-5-carbaldehyde (P87)

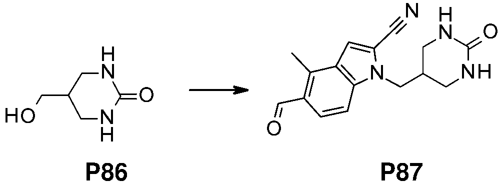

To a solution of 5-(hydroxymethyl)tetrahydropyrimidin-2(1*H*)-one (**P86**, 130 mg, 1 mmol) in DMAA (3 ml) DIPEA (258 mg, 2 mmol) and MsCl (132 mg, 1.15 mmol) were added. The mixture was stirred at 50°C for 45 min, then 2-cyano-4-methyl-5-formylindole (92 mg, 0.5 mmol) and potassium carbonate (400 mg, 2.9 mmol) were added, and mixture was stirred at 90°C for 48 h, then the product was extracted with ethyl acetate (3x5 ml) from water (40 ml), ethyl acetate was evaporated to dryness. The residue was washed with dry ethyl acetate (4 ml), filtered, and dried to yield pure 2-ethynyl-4-methyl-1-[(2-oxohexahydropyrimidin-5-yl)methyl]-1*H*-indole-5-carbaldehyde (**P87**, 28 mg, 9.5%) ¹H NMR (400 MHz, DMSO-*d₆*), δ: 10.40 (s, 1H), 7.85 (m, 3H), 6.15 (m, 2H), 4.46 (m, 2H), 2.90 (m, 6H), 2.44 (m, 2H). LCMS (ESI) [MH]⁺: 297.

### Synthesis of 5-formyl-4-methyl-1-[(2-oxoimidazolidin-4-yl)methyl]-1H-indole-2-carbonitrile (P88)

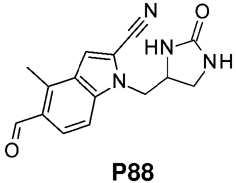

### Preparation 88. 5-Formyl-4-methyl-1-[(2-oxoimidazolidin-4-yl)methyl]-1H-indole-2-carbonitrile (P88)

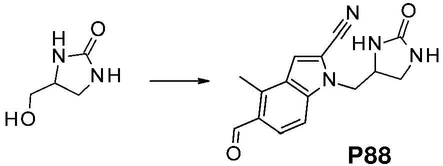

To a solution of 4-(hydroxymethyl)imidazolidin-2-one (116 mg, 1 mmol, prepared as described in US2015/284405, 2015, A1) in DMAA (6 ml) DIPEA (194 mg, 1.5 mmol) and MsCl (137 mg, 1.2 mmol) were added. The mixture was stirred at 50°C for 45 min, then 2-cyano-4-methyl-5-formylindole (**P97**, 92 mg, 0.5 mmol) and potassium carbonate (400 mg, 2.9 mmol) were added, and the mixture was stirred at 90°C for additional 48 h, then reaction mixture was quenched with water (40 ml) and product was extracted with ethyl acetate (3x5 ml). Organic solvent was evaporated to dryness and residue was washed with dry ethyl acetate (4 ml), filtered, and dried to give pure 5-formyl-4-methyl-1-[(2-oxoimidazolidin-4-yl)methyl]-1*H*-indole-2-carbonitrile (**P88**, 28 mg, 20 %). ¹H NMR (400 MHz, DMSO-*d₆*), δ: 10.38 (s, 1H), 8.05 (m, 1H), 7.73 (m, 1H), 6.60 (m, 1H), 6.00 (m, 1H), 5.55 (m, 1H), 4.38 (m, 1H), 4,35 (m, 2H), 3.0 (m, 3H). LCMS (ESI) [MH]⁺: 283.

### Synthesis of tert-butyl 4-[2-(2-cyano-5-formyl-1H-indol-1-yl)ethyl]piperazine-1-carboxylate (P89)

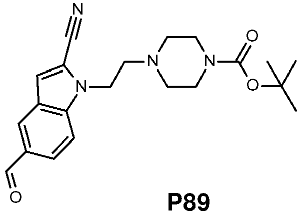

### Preparation 89. tert-Butyl 4-[2-(2-cyano-5-formyl-1H-indol-1-yl)ethyl]piperazine-1-carboxylate (P89)

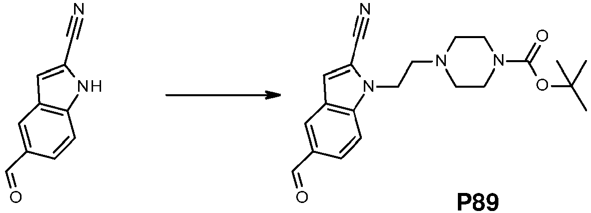

To a solution of *tert*-butyl 4-{2-[(methylsulfonyl)oxy]ethyl}piperazine-1-carboxylate (330 mg, 1.07 mmol) and 2-cyano-5-formylindole in MeCN (10 ml) was added potassium carbonate (490 mg, 3.55 mmol), and the mixture was stirred at 60°C for 20 h (LCMS control). After reaction completed the mixture was filtered, filtrate was concentrated and residue was purified by column chromatography on silica with ethyl acetate - DCM (1:1) to yield *tert*-butyl 4-[2-(2-cyano-5-formyl-1H-indol-1-yl)ethyl]piperazine-1-carboxylate (**P89**, 380 mg, 93%). LCMS (ESI) [MH]⁺: 383.

### Synthesis of 5-formyl-4-methyl-1H-indole-2-carbonitrile (P97)

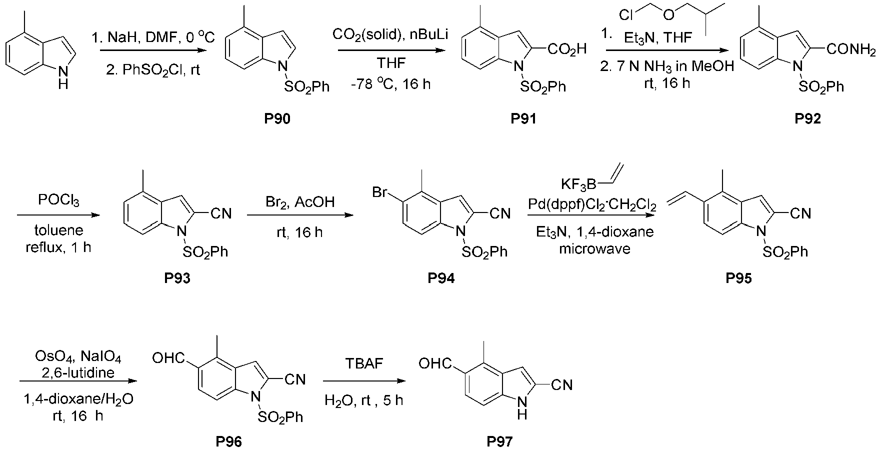

### Preparation 90. 4-Methyl-1-(phenylsulfonyl)-1H-indole (P90)

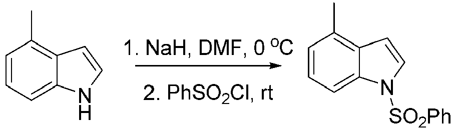

To a solution of 4-methyl-1*H*-indole (69.0 g, 0.525 mol, 1.0 eq) in anhydrous DMF (600 ml) was added 60 % sodium hydride in mineral oil (31.5 g, 0.789 mol, 1.5 eq) at 0°C. After stirred at 0°C for 1 h, benzenesulfonyl chloride was added to the mixture at 0°C. After the solution was stirred at 0°C for 16 h, it was quenched with H₂O and extracted with EtOAc. The organic layers were collected, washed with brine, dried over MgSO₄₍ₛ₎, filtered, and concentrated in vacuo to afford **P90** (169 g, crude) as a pink solid which was used in next step without further purification. ¹H NMR (400 MHz, DMSO-*d₆*), δ: 7.89-7.87 (m, 2H), 7.83 (d, *J* = 8.4 Hz, 1H), 7.56 (d, *J* = 3.6 Hz, 1H), 7.54-7.50 (m, 1H), 7.45-7.41 (m, 2H), 7.23-7.19 (m, 1H), 7.04-7.01 (m, 1H), 6.69 (d, *J* = 3.6 Hz, 1H), 2.47 (s, 3H); LRMS (ESI) [MH]⁺: 272.1.

### Preparation 91. 4-Methyl-1-(phenylsulfonyl)-1H-indole-2-carboxylic acid (P91)

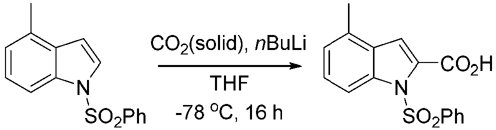

To a solution of **P90** (10.0 g, 36.9 mmol, 1.00 eq) in anhydrous THF (100 ml) was added dropwise with a 2.5 M solution of *n*-butyllithium in hexane (22.1 ml, 55.3 mmol, 1.5 eq) at -78°C. After it was stirred at room temperature for 1 h, the mixture solution was cooled to -78°C and an excess of CO₂₍ₛ₎ was added. After the solution was stirred at -78°C for 10 min, the mixture was warmed to rt gradually and stirred for 16 h. The mixture solution was quenched with water and acidified by 1N HCl_{(aq.)}. The solution was extracted with EtOAc, and the organic layers were collected, dried over MgSO₄₍ₛ₎, filtered, and concentrated to afford **P91** (10.2 g, 88%) as a purple solid which was used in next step without further purification. ¹H NMR (400 MHz, DMSO-*d₆*), δ: 13.58 (s, 1H), 8.01-7.99 (m, 2H), 7.81 (d, *J* = 8.4 Hz, 1H), 7.72-7.68 (m, 1H), 7.62-7.58 (m, 2H), 7.38 (s, ¹H ), 7.36-7.32 (m, 1H), 7.12-7.10 (m, 1H), 2.43 (s, 3H); LRMS (ESI) [MH]⁺: 316.1.

### Preparation 92. 4-Methyl-1-(phenylsulfonyl)-1H-indole-2-carboxamide (P92)

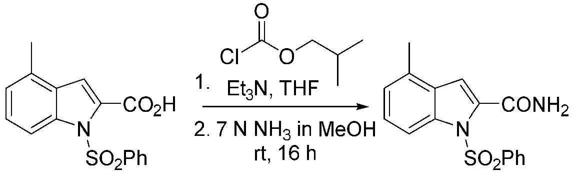

To a stirred solution of **P91** (10.2 g, 32.3 mmol, 1.0 eq) in anhydrous THF (170 ml) was added isobutyl carbonochloridate (4.62 ml, 35.6 mmol, 1.1 eq) and TEA (4.96 ml, 35.6 mmol, 1.1 eq) at rt. After the solution was stirred at rt for 1 h, a solution of 7 N ammonia in methanol (23.1 ml, 161 mmol, 5.0 eq) was added. After the mixture solution was stirred at rt for 16 h, it was concentrated and extracted with EtOAc. The organic layers were collected, washed with brine, dried over MgSO₄₍ₛ₎, filtered, and concentrated in vacuo. The residue was purified by flash column chromatography (EtOAc : *n*-hexane = 2 : 1) to afford **P92** (6.3 g, 62%) as a white solid. ¹H NMR (400 MHz, CDCl₃), δ: 8.20 (br s, 1H), 8.13-8.11 (m, 2H), 7.76 (d, *J* = 8.4 Hz, 1H), 7.70-7.66 (m, 2H), 7.60-7.56 (m, 2H), 7.28-7.24 (m, 1H), 7.08-7.06 (m, 2H), 2.41 (s, 3H); LRMS (ESI) [MH]⁺: 315.1.

### Preparation 93. 4-Methyl-1-(phenylsulfonyl)-1H-indole-2-carbonitrile (P93)

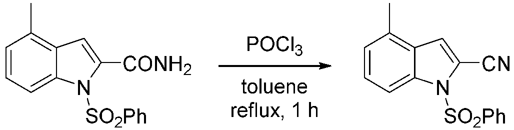

To a solution of **P92** (42.9 g, 0.136 mol, 1.0 eq) in toluene (750 ml) was added phosphoryl chloride (44.6 ml, 0.477 mol, 3.5 eq) at rt and stirred at reflux for 1 h. After the solution was cooled to rt, it was quenched with water and neutralized with saturated NaHCO_{3(aq.)}. The mixture solution was extracted with EtOAc. The organic layers were collected, washed with brine, dried over MgSO₄₍ₛ₎, filtered, and concentrated in vacuo. The residue was purified by flash column chromatography (EtOAc : *n*-hexane = 1 : 3) to give **P93** (35.8 g, 88%) as a pale-yellow solid. ¹H NMR (400 MHz, CDCl₃), δ: 8.11 (s, 1H), 7.98-7.92 (m, 3H), 7.79-7.73 (m, 1H), 7.67-7.62 (m, 2H), 7.53-7.49 (m, 1H), 7.22-7.20 (m, 1H), 2.45 (s, 3H); LRMS (ESI) [MH]⁺: 297.1.

### Preparation 94. 5-Bromo-4-methyl-1-(phenylsulfonyl)-1H-indole-2-carbonitrile (P94)

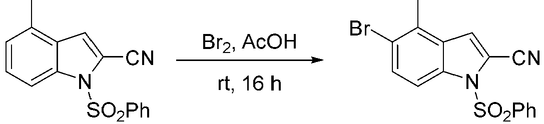

To a solution of **P93** (4.17 g, 14.1 mmol, 1.0 eq) in acetic acid (60 ml) was added bromine (1.44 ml, 28.1 mol, 2.0 eq) at rt and the mixture was stirred at rt for 16 h. The mixture solution was diluted with water and extracted with EtOAc. The organic layers were collected, washed with Na₂S₂O_{3(aq.)}, dried over MgSO₄₍ₛ₎, filtered, and concentrated in vacuo. The residue was purified by flash column chromatography (EtOAc : *n*-hexane = 1 : 5) to give **P94** (4.81 g, 91%) as a yellow solid. ¹H NMR (400 MHz, DMSO-*d₆*), δ: 8.02-8.00 (m, 2H), 7.95-7.93 (m, 1H), 7.68-7.61 (m, 1H), 7.53-7.49 (m, 2H), 7.39 (s, 1H), 2.51 (s, 3H).

### Preparation 95. 4-Methyl-1-(phenylsulfonyl)-5-vinyl-1H-indole-2-carbonitrile (P95)

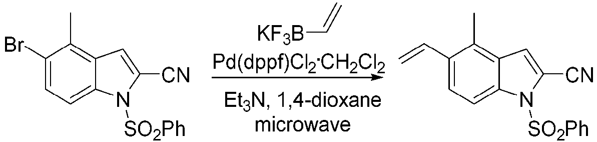

To a solution of **P94** (4.00 g, 10.6 mmol, 1.0 eq) in 1,4-dioxane (35 ml) was added potassium vinyltrifluoroborate (2.14 g, 15.9 mmol, 1.5 eq) and TEA (7.42 ml, 53.3 mmol, 5.0 eq) at rt. After the solution was degassed with Argon, 1,1'-bis(diphenylphosphino)ferrocene-palladium(II)dichloride dichloromethane complex (0.870 mg, 1.06 mmol, 0.1 eq) was added. The solution was reacted in a microwave reactor by using a condition of 800 W and 110°C for 2 h. After the solution was cooled to rt, the mixture was diluted with EtOAc, filtered through a pad of Celite, and concentrated in vacuo. The residue was purified by flash column chromatography (EtOAc : *n*-hexane = 1 : 6) to give **P95** (3.12 g, 91%) as an orange solid. ¹H NMR (400 MHz, DMSO-*d₆*), δ: 8.04-8.01 (m, 3H), 7.67 (d, *J* = 8.8 Hz, 1H), 7.63-7.59 (m, 1H), 7.52-7.48 (m, 2H), 7.42 (d, *J* = 1.2 Hz, 1H), 6.99 (dd, *J* = 17.6, 11.2 Hz, 1H), 5.67 (dd, *J* = 17.6, 1.2 Hz, 1H), 5.37 (dd, *J* = 11.2, 1.2 Hz, 1H), 2.45 (s, 3H); LRMS (ESI) [MH]⁺: 323.0.

### Preparation 96. 5-Formyl-4-methyl-1-(phenylsulfonyl)-1H-indole-2-carbonitrile (P96)

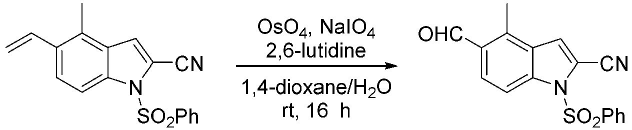

To a solution of **P95** (3.12 g, 9.68 mmol, 1.0 eq) in 1,4-dioxane/H₂O (20/5 ml) was added a solution of 2.5% OsO₄ in *t*-butanol (1.97 ml, 0.193 mmol, 0.02 eq), sodium periodate (8.28 g, 38.7 mmol, 4.0 eq), 2,6-lutidine (2.25 g, 19.3 mmol, 2.0 eq) at rt and stirred at rt for 16 h. The mixture solution was filtered through a pad of Celite and extracted with EtOAc. The organic layers were collected, washed with brine, dried over MgSO₄₍ₛ₎, filtered, and concentrated in vacuo. The residue was purified by flash column chromatography (EtOAc : *n*-hexane = 1 : 2) to give **P96** (1.46 g, 46%) as a yellow solid. ¹H NMR (400 MHz, DMSO-*d*₆), δ: 10.38 (s, 1H), 8.20 (d, *J* = 8.8 Hz, 1H), 8.07-8.05 (m, 2H), 8.01 (d, *J* = 8.8 Hz, 1H), 7.68-7.63 (m, 1H), 7.56-7.52 (m, 3H), 2.82 (s, 3H); LRMS (ESI) [MH]⁺: 325.1.

### Preparation 97. 5-Formyl-4-methyl-1H-indole-2-carbonitrile (P97)

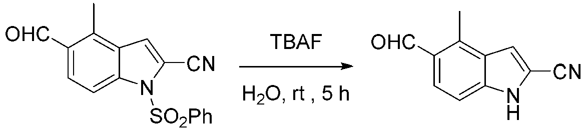

To a solution of **P96** (1.46 g, 4.50 mmol, 1.0 eq) in THF (22 ml) was added 1 M tetra-*n-*butylammonium fluoride in THF (5.40 ml, 5.40 mmol, 1.2 eq) and stirred at rt for 5 h. The mixture solution was filtered through a pad of Celite and extracted with EtOAc. The organic layers were collected, washed with brine, dried over MgSO₄₍ₛ₎, filtered, and concentrated in vacuo to give a crude solid. The crude solid was washed with methanol and collected by filtration to give **P97** (672 mg, 81%) as a pale-yellow solid. ¹H NMR (400 MHz, DMSO-*d*₆), δ: 12.93 (br. s, 1H), 10.35 (s, 1H), 7.78-7.75 (m, 2H), 7.42 (d, *J* = 8.4 Hz, 1H), 2.85 (s, 3H); LRMS (ESI) [MH]⁺: 185.1.

### Synthesis of 5-formyl-4-methyl-1-{2-[4-(methylsulfonyl)piperazin-1-yl]propyl}-1H-indole-2-carbonitrile (P98)

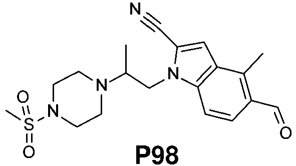

### Preparation 98. 5-Formyl-4-methyl-1-{2-[4-(methylsulfonyl)piperazin-1-yl]propyl}-1H-indole-2-carbonitrile (P98)

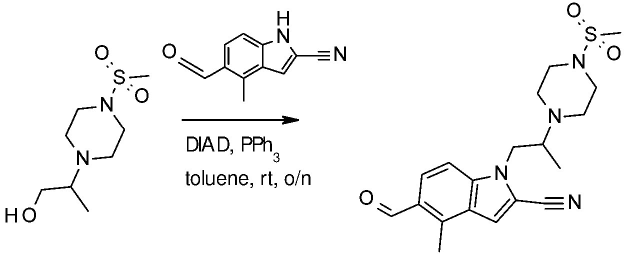

A solution of 5-formyl-4-methyl-1H-indole-2-carbonitrile **P97** (3.0 g, 16.3 mmol), 2-(4-methylsulfonylpiperazin-1-yl)propan-1-ol (3.6 g, 16.3 mmol), and triphenylphosphine (6.4 g, 24.4 mmol) in anhydrous THF (50 ml) was stirred in ice bath for 15 min. DIAD (4.8 ml, 24.4 mmol) was added to the solution dropwise. The reaction was stirred at rt for overnight. The mixture was treated with water and extracted with dichloromethane. The combined organic layers were dried over Na₂SO₄, filtered and concentrated. The residue was purified by silica gel chromatography (0-20 % EtOAc in dichloromethane) to give compound **P98** (2.6 g, 42 %) as a white solid. LCMS (ESI) [MH]⁺: 389.

### Synthesis of (S)-2-(4-(methylsulfonyl)piperazin-1-yl)propan-1-ol (P101)

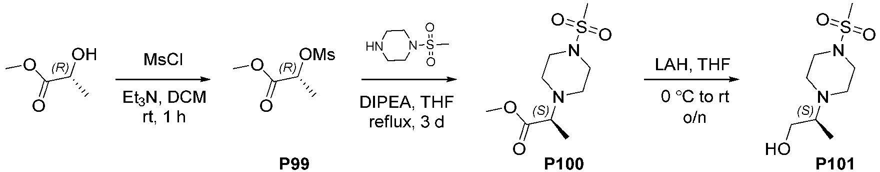

### Preparation 99. Methyl (R)-2-((methylsulfonyl)oxy)propanoate (P99)

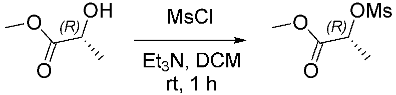

To a solution of methyl (*R*)-(+)-lactate (25.00 g, 0.24 mol) in DCM (400 ml) was added triethylamine (67.0 ml) in ice bath. The mixture was stirred in ice bath for 15 mins, and then methanesulfonyl chloride (22.5 ml) was added to the mixture. After the reaction was stirred at rt for 1 h, the mixture was treated with DCM and water and extracted with DCM. The combined organic layers were washed with brine, dried over Na₂SO₄, filtered, and concentrated to give **P99** (45.57 g) as a crude yellow liquid which was used in next step without further purification. ¹H NMR (400 MHz, CDCl₃), δ: 5.14 (q, *J* = 7.2 Hz, 1H), 3.81 (s, 3H), 3.15 (s, 3H), 1.62 (d, *J* = 7.2 Hz, 3H).

### Preparation 100. Methyl (S)-2-(4-(methylsulfonyl)piperazin-1-yl)propanoate (P100)

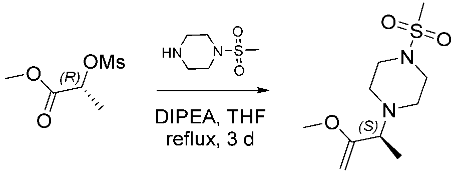

To a solution of **P99** (33.95 g, 0.19 mmol) and 1-methylsulfonyl 1-piperazine (25.50 g, 0.160 mmol) in THF (anhydrous, 450 ml), was added DIPEA (54.1 ml). The reaction was stirred at 70°C for 3 days. Monitoring by LCMS showed that 1-methylsulfonyl 1-piperazine was consumed, the solution was then concentrated. The residue was treated with ethyl acetate and water. The organic layer was dried over Na₂SO₄, filtered, and concentrated to give a residue. The residue was purified by silica gel chromatography (EtOAc : *n*-hexane = 1 : 2 to 4 : 1) to give **P100** (20.15 g, 58%) as a white sold. ¹H NMR (400 MHz, CDCl₃), δ: 3.71 (s, 3H), 3.40 (q, *J* = 7.2 Hz, 1H), 3.28- 3.23 (m, 4H), 2.77 (s, 3H), 2.75- 2.67 (m, 4H), 1.13 (d, *J* = 7.2 Hz, 3H).

### Preparation 101. (S)-2-(4-(Methylsulfonyl)piperazin-1-yl)propan-1-ol (P101)

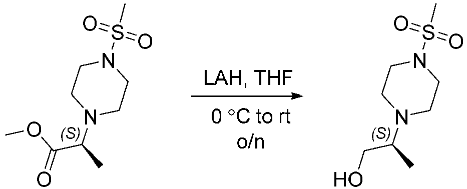

A solution of **P100** (17.00 g, 67.92 mmol) in anhydrous THF (120 ml) was added a solution of LAH in THF (1M, 102 ml) dropwise in ice bath. The mixture was warmed up slowly to rt and stirred for overnight. The mixture was treated with water (4.0 ml) and 15% of NaOH_{(aq.)} (4.0 ml) in ice bath. The mixture was diluted with DCM, and it was filtered through a pad of celite. The filtrate was dried over Na₂SO₄, filtered, and concentrated to give **P101** (14.78 g, 98%) as a white solid. ¹H NMR (400 MHz, CDCl₃), δ: 3.47-3.21 (m, 6H), 2.99-2.75 (m, 7H), 2.57-2.51 (m, 2H), 0.93 (d, *J* = 7.2 Hz, 3H); LCMS (ESI) [MH]⁺: 223.1.

### Synthesis of 5-formyl-4-methyl-1-{(2S)-2-[4-(methylsulfonyl)piperazin-1-yl]propyl}-1H-indole-2-carbonitrile (P102)

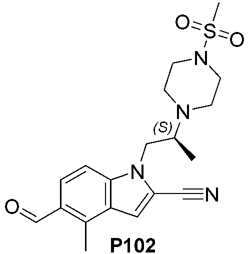

### Preparation 102. 5-Formyl-4-methyl-1-{(2S)-2-[4-(methylsulfonyl)piperazin-1-yl]propyl}-1H-indole-2-carbonitrile (P102)

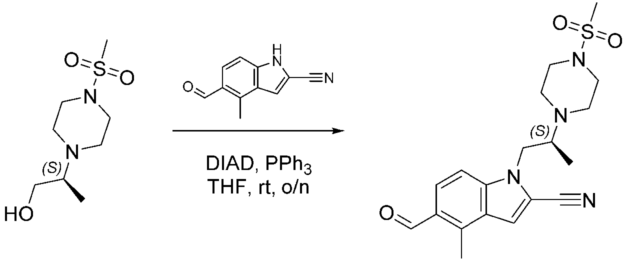

A solution of **P97** (672 mg, 3.65 mmol), **P101** (892 mg, 4.01 mmol), and triphenylphosphine (1.244 g, 4.74 mmol) in anhydrous THF (17 ml) was stirred in ice bath for 15 min. DIAD (0.934 ml) was added dropwise in ice bath and the reaction was stirred at rt for overnight. The mixture was treated with water and extracted with DCM. The combined organic layers were dried over Na₂SO₄, filtered and concentrated. The residue was purified by silica gel chromatography (0-20 % EtOAc in DCM) to give **P102** (682 mg, 48%) as a white solid. ¹H NMR (400 MHz, CDCl₃), δ: 10.43 (s, 1H), 7.89 (d, *J* = 8.8 Hz, 1H), 7.36 (s, 1H), 7.24 (d, *J* = 8.8 Hz, 1H), 4.30 (dd, *J* = 14.8, 8.8 Hz, 1H), 4.12 (dd, *J* = 14.8, 4.8 Hz, 1H), 3.23- 3.11 (m, 5H), 2.90 (s, 3H), 2.86-2.80 (m, 2H), 2.75 (s, 3H), 1.11 (d, *J* = 6.8 Hz, 3H); LCMS (ESI) [MH]⁺: 389.2.

### Synthesis of 5-formyl-4-methyl-1-[(5-oxomorpholin-2-yl)methyl]-1H-indole-2-carbonitrile (P105)

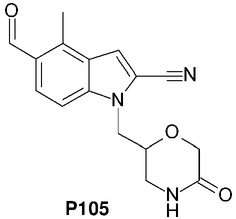

### Preparation 103. 2-Chloro-N-((S)-2,3-dihydroxypropyl)acetamide (P103)

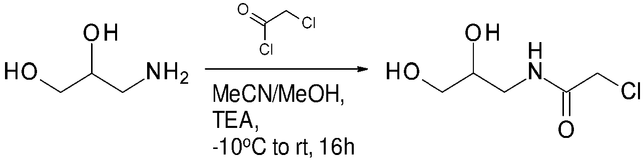

To a solution of 3-amino-1,2-propanediol (5.24 g, 57.5 mmol) in a mixture CH₃CN/MeOH (190/33 ml) at -10°C was added triethylamine (9.60 ml, 1.2 eq). Chloroacetyl chloride (5.10 ml, 1.1 eq) was then added dropwise at -10°C during 1.5 h under nitrogen. The reaction mixture was allowed to reach rt and stirred overnight (16 h). The crude was concentrated under vacuum and purified by flash chromatography on silica gel with MeOH/EtOAc (8:92) to provide product **P103** as a white solid (7 g, 73%).

### Preparation 104. 6-(Hydroxymethyl)morpholin-3-one (P104)

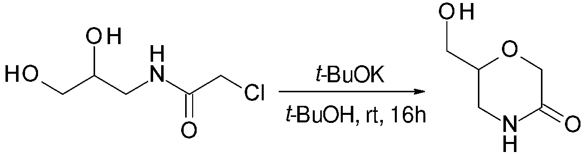

To a stirred solution of potassium *tert*-butoxide (8.37 g, 2.5 eq) in 150 ml *tert*-butyl alcohol at rt was added 2-chloro-*N*-((S)-2,3-dihydroxypropyl)acetamide (**P103**, 5 g, 29.8 mmol) in 150 ml *tert*-butyl alcohol over 2 h under Ar. After one more hour, MeOH (50 ml) and H₂O (3 ml) were added, and the reaction mixture was stirred overnight. The crude was concentrated under vacuum and purified by flash chromatography on silica gel with MeOH/EtOAc (20:80) to provide **P104** as a white solid (2.7 g, 69%). ¹H NMR (400 MHz, DMSO-*d₆*) δ 7.95 (s, 1H), 4.89 (s, 1H), 4.01 - 3.97 (m, 2H), 3.68 - 3.60 (m, 1H), 3.39 (d, 2H), 3.21 - 3.14 (m, 1H), 3.09 - 3.01 (m, 1H).

### Preparation 105. 5-Formyl-4-methyl-1-[(5-oxomorpholin-2-yl)methyl]-1H-indole-2-carbonitrile (P105)

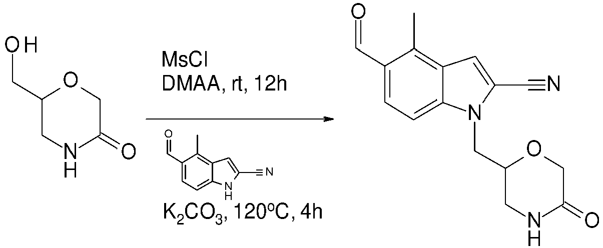

To a solution of 6-(hydroxymethyl)morpholin-3-one (**P104**, 200 mg, 15 mmol) and DIPEA (256 mg, 1.2 eq) in DMAA (5 ml) at rt MsCl (192 mg, 1.1 eq) was added. The reaction mixture was stirred for 16 h at rt. Then K₂CO₃ (600 mg, 4 eq) and 5-formyl-4-methyl-1H-indole-2-carbonitrile **P97** (200 mg, 1 eq) were added, and mixture was heated to 140°C for 4 h. The reaction mixture was diluted with water and extracted with EtOAc. The residue after evaporation was subjected to column chromatography on silica gel eluting with dichloromethane/MeOH (0 - 20%) to afford product **P105** (50 mg, 16%) as a yellow solid. LCMS (ESI) [MH]⁺: 298. ¹H NMR (400 MHz, CDCl₃), δ: 10.35 (s, 1H), 7.83 (d, J=7.4 Hz, 1H), 7.39-7.31 (m, 2H), 4.45-4.39 (m, 2H), 4.21-4.18 (m, 1H), 4.18-4.12 (m, 2H), 4.10-4.03 (m, 1H), 3.95-3.86 (m, 1H), 2.82 (s, 3H).

### Synthesis of 2-(4-(methylsulfonyl)piperazin-1-yl)propan-1-ol (P107)

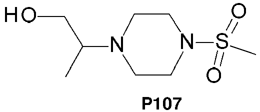

### Preparation 106. Methyl 2-(4-methylsulfonylpiperazin-1-yl)propanoate (P106)

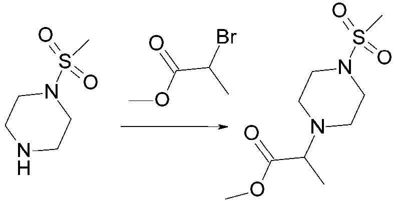

A solution of 1-(methylsulfonyl)piperazine (10.0 g, 61.0 mmol) and K₂CO₃ (4.2 g, 30.5 mmol) in CH₃CN (anhydrous, 100 ml) methyl 2-bromopropanoate (10.2 g, 61.0 mmol) was added. The reaction was stirred at 80⁰C for overnight. Monitoring by LCMS showed that 1-methylsulfonyl 1-piperazine was consumed. The mixture was filtered, and the filtrate was concentrated. The residue was purification by re-crystallization from ethyl acetate/hexane to give **P106** (12.0 g, 77%) as a white sold. ¹H NMR (400 MHz, CDCl₃), δ: 3.71 (s, 3H), 3.40 (q, *J* = 7.2 Hz, 1H), 3.28- 3.23 (m, 4H), 2.77 (s, 3H), 2.75- 2.67 (m, 4H), 1.13 (d, *J* = 7.2 Hz, 3H).

### Preparation 107. 2-(4-(Methylsulfonyl)piperazin-1-yl)propan-1-ol (P107)

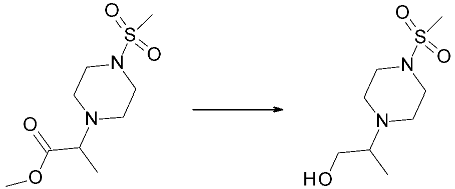

To a solution of LAH (1.8 g, 48.0 mmol) in anhydrous THF (100 ml) was added a solution of **P106** (12.0 g, 48.0 mmol) in THF (50 ml) dropwise in ice bath. The mixture was warmed up slowly to rt and stirred for 1 h. The mixture was treated with water (2.0 ml) and 10% of NaOH_{(aq.)} (6.0 ml) in ice bath. The mixture was diluted with DCM, and it was filtered through a pad of celite. The filtrate was dried over Na₂SO₄, filtered, and concentrated to give **P107** (10.0 g, 94%) as a white solid. ¹H NMR (400 MHz, DMSO-*d₆*), δ: 4.30 (s, 1H), 3.44 (d, J = 4.6 Hz, 1H), 3.29 - 3.22 (m, 1H), 2.64-2.53 (m, 4H), 2.84 (s, 3H), 3.12-2.98 (m, 4H), 0.92 (d, J = 6.3 Hz, 3H).

### Synthesis of 6-formyl-4-methyl-1-[(5-oxomorpholin-2-yl)methyl]-1H-indole-2-carbonitrile (P114)

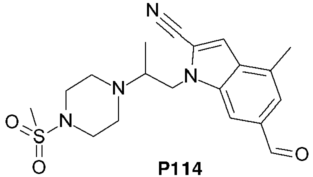

### Preparation 108. Ethyl-2-azido-3-(4-bromo-2-methylphenyl)acrylate (P108)

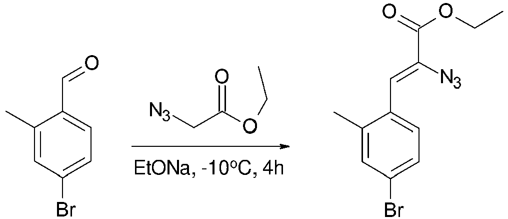

NaH (3.62 g, 60% dispersion in mineral oil, 3 eq) was added in portions to 60 ml of ethanol and stirred until clear solution formed. The solution then was cooled to -20°C and solution of 2-methyl-4-bromobenzaldehyde (6 g, 30 mmol) and ethyl azidoacetate (11.7 g, 3 eq) in 60 ml ethanol was added dropwise at that temperature. The reaction mixture was stirred 2 h at -10°C and 2 more at rt. Then the reaction mixture was diluted with sat. NH₄Cl and precipitate was filtered off washed with water and dried under vacuum to give product P108 as yellowish solid (8 g, 85%).

### Preparation 109. Ethyl 6-bromo-4-methyl-1H-indole-2-carboxylate (P109)

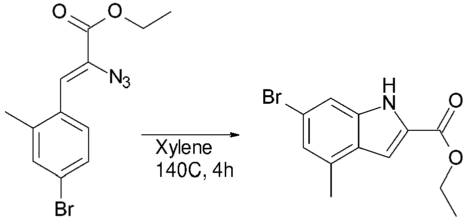

A solution of ethyl-2-azido-3-(4-bromo-2-methylphenyl)acrylate (**P108,** 8 g, 15 mmol) in xylene was refluxed for 4 h. The reaction mass was cooled to ~-15°C and precipitate was filtered off yielding product **P109** as yellow solid (1.56 g, 21%). ¹H NMR (400 MHz, CDCl₃) δ 7.92 - 7.84 (m, 1H), 7.37 (s, 1H), 7.03 (s, 1H), 4.40 (q, J = 7.1 Hz, 2H), 2.35 (s, 3H), 1.47 - 1.35 (m, 3H).

### Preparation 110. 6-Bromo-4-methyl-1H-indole-2-carboxylic acid (P110)

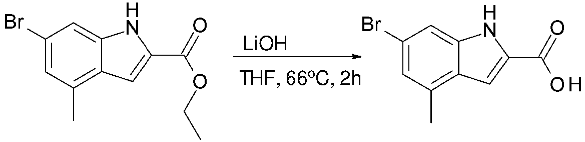

To a THF solution of ethyl 6-bromo-4-methyl-1H-indole-2-carboxylate (**P109**, 1 g, 3.54 mmol) was added lithium hydroxide (127 mg, 1.5 eq) in H₂O. The mixture was heated under reflux for 2 h. To the cooled mixture was added a 1 M hydrochloric acid solution until the pH is 3. The acidic mixture was poured into water and the organic layer was separated. The aqueous layer was extracted with CHCl₃.The combined organic layer was dried over MgSO₄. The solvent was removed under reduced pressure to give product **P110** as off-white solid (890 mg, 98%).

### Preparation 111. 6-Bromo-4-methyl-1H-indole-2-carboxamide (P111)

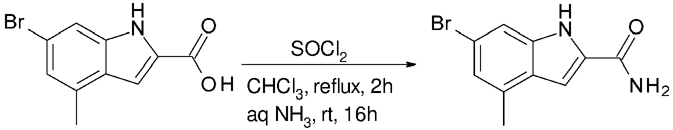

A mixture of 6-bromo-4-methyl-1H-indole-2-carboxylic acid (**P110**, 890 mg, 3.5 mmol), thionyl chloride (542 mg, 1.3 eq) in chloroform and 1 drop of dimethylformamide was refluxed for 2 h. The reaction mixture was cooled to 20°C, poured into a mixture of 10 ml of 25 % ammonia solution and 5 g of ice, then stirred for 16 h. The precipitate was filtered off and washed with water to give product **P111** as yellow solid (630 mg, 71%). ¹H NMR (400 MHz, DMSO-*d₆*), δ: 11.63 (s, 1H), 7.97 (s, 1H), 7.40 (s, 1H), 7.37 (s, 1H), 7.18 (s, 1H), 6.99 (s, 1H), 2.47 (s, 3H).

### Preparation 112. 6-Bromo-4-methyl-1H-indole-2-carbonitrile (P112)

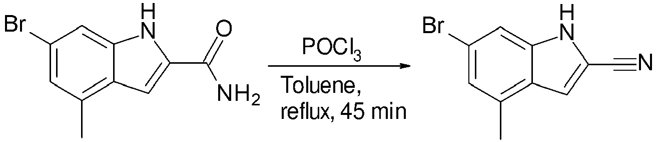

Phosphorous oxychloride was added to a suspension of 6-bromo-4-methyl-1H-indole-2-carboxamide (**P111**, 630 mg, 2.5 mmol) in toluene (6 ml) and the mixture was refluxed for 45 min. On cooling, the mixture was poured into an aqueous Na₂CO₃ solution, and the mixture stirred until effervescence had subsided. The layers were separated, the aqueous phase extracted with EtOAc, and the combined organic layers dried (MgSO₄) and evaporated to dryness. The crude material was purified by column chromatography to give product **P112** as yellow solid (460 mg 79%). ¹H NMR (400 MHz, CDCl₃), δ: 8.56 (s, 1H), 7.44 (s, 1H), 7.20 (s, 1H), 7.16 (s, 1H), 2.54 (s, 3H).

### Preparation 113. 6-Formyl-4-methyl-1H-indole-2-carbonitrile (P113)

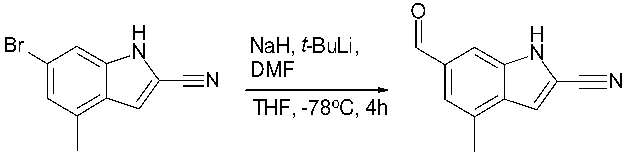

To a solution of 6-bromo-4-methyl-1H-indole-2-carbonitrile (**P112**, 430 mg, 1.8 mmol) in 20 ml dry THF, NaH (146 mg, 60% dispersion in mineral oil, 2 eq) was added in portions at rt. After stirring for 15 min the reaction mixture was cooled on acetone/dry ice bath (yellowish precipitate formed). Then *tert*-BuLi (2.17 ml, 1.7 M in pentane, 2 eq) was added dropwise and after stirring for 20 min dry DMF (0.850 ml, 6 eq) was added. The reaction mixture was stirred at -78°C for 4 h and then was quenched with sat. NH₄Cl solution. Phases were separated, aqueous phase was extracted with EtOAc (20 ml x 3). Organic phases were combined, washed with water and brine, dried over Na₂SO₄, and concentrated under vacuum. The residue was purified by silica gel column chromatography (CH₂Cl₂/Et₂O - 30/1) to yield product **P113** as off-white solid (220 mg, 65%). ¹H NMR (400 MHz, DMSO-*d₆*), δ: 12.92 (s, 1H), 10.03 (s, 1H), 7.90 (s, 1H), 7.54 (s, J = 12.7 Hz, 1H), 2.55 (s, 3H).

### Preparation 114. 6-Formyl-4-methyl-1-{2-[4-(methylsulfonyl)piperazin-1-yl]propyl}-1H-indole-2-carbonitrile (P114)

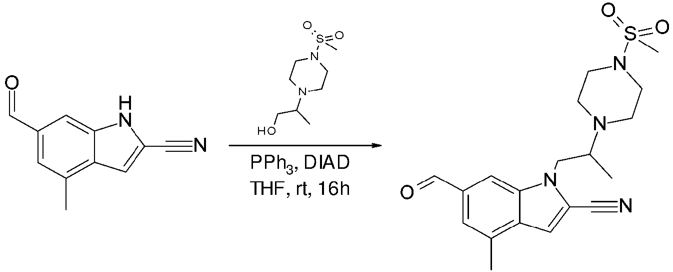

To a mixture of 2-[4-(methylsulfonyl)piperazin-1-yl]propan-1-ol (**P107**, 125 mg, 1.3 eq), 6-formyl-4-methyl-1H-indole-2-carbonitrile (**P113**, 80 mg, 0.43 mmol) and triphenyl phosphine (145 mg, 1.3 eq) in THF (5 ml) DIAD (114 mg, 1.3 eq) was added, and the resulting mixture was stirred at ambient temperature overnight. The solvent was evaporated. The residue after evaporation was subjected to column chromatography on silica gel eluting with dichloromethane/ethyl acetate (0 - 20%) to afford product **P114** (84 mg, 50%) as a yellow solid. LCMS (ESI) [MH⁺]: 389.

### Synthesis of 5-formyl-4-methoxy-1-{2-[4-(methylsulfonyl)piperazin-1-yl]propyl}-1H-indole-2-carbonitrile (P121)

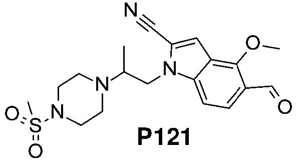

### Preparation 115. 4-Methoxy-1-(phenylsulfonyl)-1H-indole (P115)

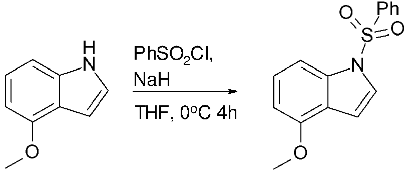

To a solution of 4-methoxy-1H-indole (2.8 g, 19 mmol) in THF, NaH (0.837 g, 60% in mineral oil, 1.1 eq) and PhSO₂Cl (3.7 g, 1.1 eq) was sequentially added at 0°C. After stirring at 25 °C for 4 h, the reaction solution was poured into ice water. The precipitated solid was collected by filtration and re-dissolved in EtOAc. The organic layer was washed with saturated aqueous NaHCO₃ solution, brine, dried over Na₂SO₄ and concentrated. The solid was then slurried in petroleum ether (2.0 L) and filtered to afford product **P115** as a dark yellow solid (4.2 g, 77%).

### Preparation 116. 4-Methoxy-1-(phenylsulfonyl)-1H-indole-2-carbaldehyde (P116)

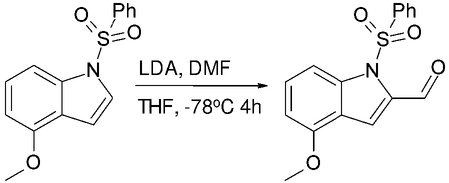

Under an Ar atmosphere, *n*-BuLi (5.85 ml, 1.05 eq) was added to a THF solution of diisopropylamine (1.55 g, 1.1 eq) at -78°C. The reaction mixture was stirred for 10 min. A THF solution of 4-methoxy-1-(phenylsulfonyl)-1H-indole **P115** (4 g, 14 mmol) was added dropwise to the LDA solution. The whole was stirred for 10 min (the color of solution turned yellow) and then DMF (4.2 ml, 3 eq) was added to the mixture. Whole was stirred for 4 h and poured into water. The organic layer was separated, and the aqueous layer was extracted with CHCl₃. The combined organic layer was washed with water and dried over MgSO₄. The solvent was removed under reduced pressure. The residue was slurried in hexane and solids were filtered off to give product **P116** as yellow solid (2.9 g, 66%). ¹H NMR (400 MHz, CDCl₃), δ: 10.30 (s, 1H), 7.92 (d, J = 7.5 Hz, 2H), 7.70 (d, J = 8.3 Hz, 2H), 7.63 - 7.53 (m, 4H), 6.89 (d, J = 8.0 Hz, 1H), 3.88 (s, 3H).

### Preparation 117. 4-Methoxy-1-(phenylsulfonyl)-1H-indole-2-carbonitrile (P117)

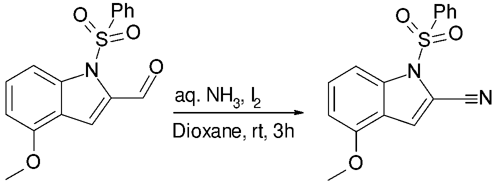

4-Methoxy-1-(phenylsulfonyl)-1H-indole-2-carbaldehyde (**P116**, 2.9 g, 9.2 mmol) was dissolved in dioxane/THF (20/9 ml) and aq. NH₃ (10ml) was added. The mixture was stirred at rt for 15 min, cooled on ice bath and I₂ (2.8 g, 1.2 eq) was added in portions. The reaction mixture was stirred on ice bath for 20 min, ice bath was removed, and reaction mixture was stirred at rt until completion. Then the reaction mixture was diluted with sat Na₂S₂O₃, organic layer was separated, concentrated and re-slurried in Et₂O/Hex 1/1 with obtaining of yellow solid of **P117** (2.21 g, 77%). ¹H NMR (400 MHz, DMSO-*d₆*), δ: 7.96 (dd, J = 7.5, 6.2 Hz, 1H), 7.76 (t, J = 7.5 Hz, 1H), 7.66 (dd, J = 15.8, 7.9 Hz, 1H), 7.55 (dd, J = 17.8, 9.4 Hz, 1H), 6.92 (d, J = 8.0 Hz, 1H), 3.86 (d, J = 15.8 Hz, 1H).

### Preparation 118. 5-Bromo-4-methoxy-1-(phenylsulfonyl)-1H-indole-2-carbonitrile (P118)

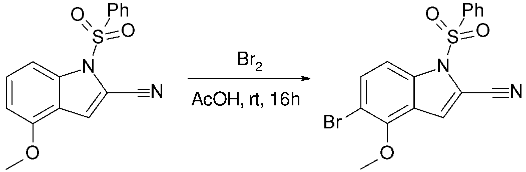

The 4-methoxy-1-(phenylsulfonyl)-1H-indole-2-carbonitrile **P117** (250 mg, 0.8 mmol) was dissolved in acetonitrile (25 ml) and *N*-Bromosuccinimide (171 mg, 1.2 eq) was added in portions. The mixture was stirred at rt for 16 h. The reaction mixture was diluted with sat. Na₂SO₃ and extracted with EtOAc, organic layer was separated, concentrated and product was purified using column chromatography. Product **P118** was obtained as a yellow solid (200 mg, 64%). ¹H NMR (300 MHz, DMSO-*d₆*), δ: 8.27 (s, 1H), 8.04 - 7.97 (m, 2H), 7.79 (s, 3H), 7.68 (t, J = 7.6 Hz, 2H), 4.02 (s, 3H).

### Preparation 119. 5-Bromo-4-methoxy-1H-indole-2-carbonitrile (P119)

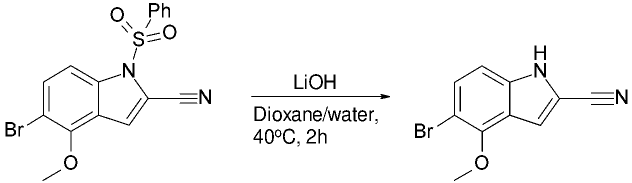

To suspension of 5-bromo-4-methoxy-1-(phenylsulfonyl)-1H-indole-2-carbonitrile **P118** (200 mg, 0.5 mmol) in dioxane (4 ml), solution of LiOH (37 mg, 3 eq) in water (1 ml) was added. The reaction mixture was stirred at 40°C for 2 h. The reaction mixture turned from milky suspension to yellow solution. The reaction mixture was diluted with water and precipitate was filtered off, washed with hexane, and dried to collect product **P119** as off-white solid (110 mg, 86%). ¹H NMR (400 MHz, DMSO-*d₆*), δ: 7.58 (s, 1H), 7.42 (d, J = 8.8 Hz, 1H), 7.13 (d, J = 8.8 Hz, 1H), 4.04 (s, 3H).

### Preparation 120. 5-Formyl-4-methoxy-1H-indole-2-carbonitrile (P120)

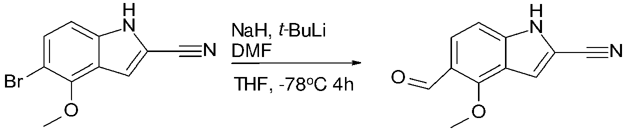

To a solution of 5-bromo-4-methoxy-1H-indole-2-carbonitrile **P119** (90 mg, 0.36 mmol) in 5 ml of dry THF, NaH (16 mg, 60% dispersion in mineral oil, 1.1 eq) was added in portions at rt. After stirring for 15 min the reaction mixture was cooled on acetone/dry ice bath (yellowish precipitate formed). Then *tert*-BuLi (0.425 ml, 1.7 M in pentane, 2 eq) was added dropwise and after stirring for 20 min dry DMF (0.166 ml, 6 eq) was added. The reaction mixture was stirred at -78°C for 4 h and then was quenched with sat. NH₄Cl solution. Phases were separated, aqueous phase was extracted with EtOAc (20 ml x 3). Organic phases were combined, washed with water and brine, dried over Na₂SO_{4,} and concentrated under vacuum. The residue was purified by silica gel column chromatography (CH₂Cl₂/Et₂O - 30/1) to yield the product **P120** as off-white solid (49 mg, 68%). ¹H NMR (400 MHz, DMSO-*d₆*), δ: 10.36 (s, 1H), 7.91 (s, 1H), 7.64 (d, J = 8.7 Hz, 1H), 7.18 (d, J = 8.7 Hz, 1H), 4.28 (s, 3H).

### Preparation 121. 5-Formyl-4-methoxy-1-{2-[4-(methylsulfonyl)piperazin-1-yl]propyl}-1H-indole-2-carbonitrile (P121)

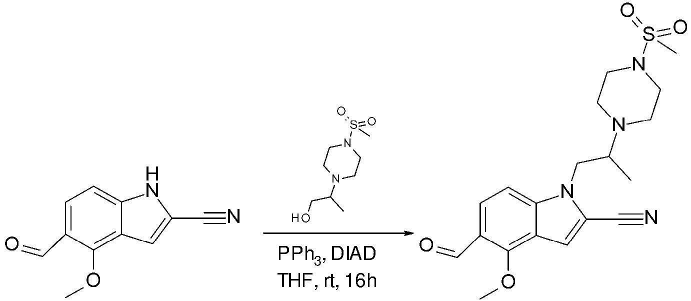

To a mixture of 2-[4-(methylsulfonyl)piperazin-1-yl]propan-1-ol **P107** (44 mg, 1.1 eq), 5-formyl-4-methoxy-1H-indole-2-carbonitrile **P120** (45 mg, 0.225 mmol) and triphenyl phosphine (65 mg, 1.1 eq) in THF (2 ml) DIAD (50 mg, 1.1eq) was added, and the resulting mixture was stirred at ambient temperature overnight. The solvent was evaporated. The residue after evaporation was subjected to column chromatography on silica gel eluting with dichloromethane/ethyl acetate (0 - 20%) to afford 5-formyl-4-methoxy-1-{(2S)-2-[4-(methylsulfonyl)piperazin-1-yl]propyl}-1H-indole-2-carbonitrile **P121** (45 mg, 49%) as a yellow solid. LCMS (ESI) [MH]⁺: 405.

### Synthesis of 5-formyl-6-methoxy-1-{2-[4-(methylsulfonyl)piperazin-1-yl]propyl}-1H-indole-2-carbonitrile (P128)

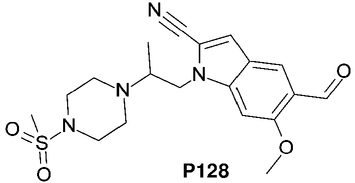

### Preparation 122. Ethyl 2-azido-3-(3-bromo-4-methoxyphenyl)acrylate (P122)

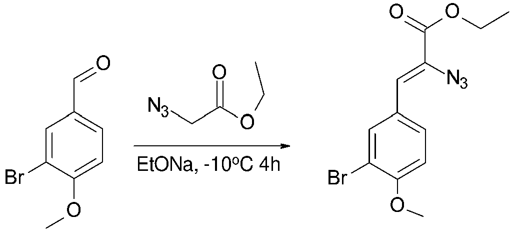

NaH was added in portions to 60 ml of ethanol and stirred until clear solution formed. The solution then was cooled to -20°C and solution of 3-bromo-4-methoxybenzaldehyde (7 g, 33 mmol) and ethyl azidoacetate (12.6 g, 3 eq) in 60ml ethanol was added dropwise at that temperature. The reaction mixture was stirred for2 h at -10°C and for 2 more hours at rt. Then the reaction mixture was diluted with sat. NH₄Cl and precipitate was filtered off, washed with water, and dried under vacuum to give product **P122** as yellowish solid (6.5 g, 61%).

### Preparation 123. Ethyl 5-bromo-6-methoxy-1H-indole-2-carboxylic acid (P123)

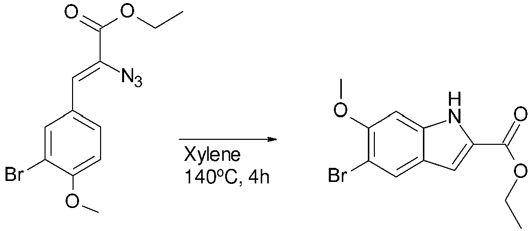

A solution of ethyl 2-azido-3-(3-bromo-4-methoxyphenyl)acrylate **P122** (5 g, 15 mmol) in xylene was refluxed for 4 h. The reaction mixture was cooled to ~-15°C and precipitate was filtered off yielding product **P123** as yellow solid (600 mg, 13%). ¹H NMR (400 MHz, DMSO-*d₆*), δ: 11.86 (s, 1H), 7.89 (s, 1H), 7.06 (d, J = 1.8 Hz, 1H), 6.99 (s, 1H), 4.32 (q, J = 7.1 Hz, 2H), 1.32 (t, J = 7.1 Hz, 3H).

### Preparation 124. 5-Bromo-6-methoxy-1H-indole-2-carboxylic acid (P124)

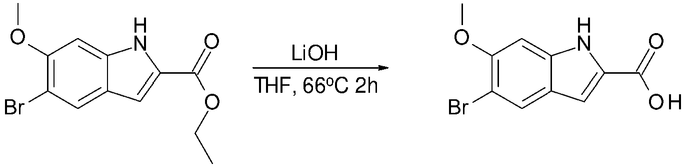

To a THF solution of ethyl 5-bromo-6-methoxy-1H-indole-2-carboxylate **P123** (300 mg, 1 mmol) was added lithium hydroxide (48 mg, 2 eq) in H₂O. The mixture was heated under reflux for 2 h. To the cooled mixture was added a 1 M hydrochloric acid solution until the pH is 3. The acidic mixture was poured into water and the organic layer was separated. The aqueous layer was extracted with CHCl₃.The combined organic layer was dried over MgSO₄. The solvent was removed under reduced pressure to give product **P124** as off-white solid (250 mg, 92%). ¹H NMR (400 MHz, DMSO-*d₆*), δ: 12.88 (s, 1H), 11.74 (s, 1H), 7.87 (s, 1H), 7.03 - 6.95 (m, 2H), 3.85 (s, 3H).

### Preparation 125. 5-Bromo-6-methoxy-1H-indole-2-carboxamide (P125)

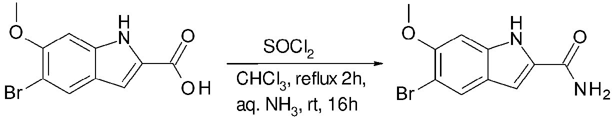

A mixture of 5-bromo-6-methoxy-1H-indole-2-carboxylic acid **P124** (250 mg, 0.9 mmol), thionyl chloride (138 mg, 1.2 eq) in chloroform and 1 drop of dimethylformamide was refluxed for 2 h. The reaction mixture was cooled to 20°C, poured into a mixture of 10 ml of 25 % ammonia solution and 5g of ice, then stirred for 16 h. The precipitate was filtered off and washed with water to give product **P125** as yellow solid (200 mg, 80%). ¹H NMR (400 MHz, DMSO-*d₆*), δ: 11.54 (s, 1H), 7.91 (s, 1H), 7.83 (s, 1H), 7.05 - 6.96 (m, 3H), 3.84 (s, 3H).

### Preparation 126. 5-Bromo-6-methoxy-1H-indole-2-carbonitrile (P126)

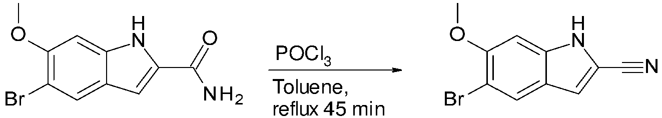

Phosphorous oxychloride was added to a suspension of 5-bromo-6-methoxy-1H-indole-2-carboxamide (**P125**, 200 mg, 0.74 mmol) in toluene (2 ml) and the mixture was refluxed for 45 min. On cooling, the mixture was poured into an aq. Na₂CO₃ solution, and the mixture stirred until effervescence had subsided. The layers were separated, the aqueous phase extracted with EtOAc, and the combined organic layers dried (MgSO₄) and evaporated to dryness. The crude material was purified by column chromatography to give product **P126** as yellow solid (160 mg, 86%). ¹H NMR (400 MHz, DMSO-*d₆*), δ: 12.32 (s, 1H), 7.91 (s, 1H), 7.26 (s, 1H), 7.03 (s, 1H), 3.89 (s, 3H).

### Preparation 127. 5-Formyl-6-methoxy-1H-indole-2-carbonitrile (P127)

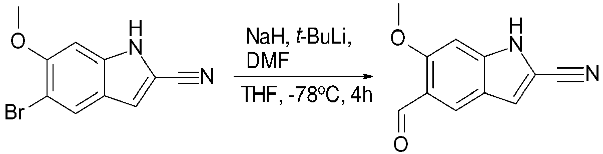

To a solution of 5-bromo-6-methoxy-1H-indole-2-carbonitrile **(P126,** 150 mg, 0.6 mmol) in 3 ml of dry THF, NaH (27mg, 60% dispersion in mineral oil, 1.2eq) was added in portions at rt. After stirring for 15 min the reaction mixture was cooled on acetone/dry ice bath (yellowish precipitate formed). Then *tert*-BuLi (0.708 ml, 1.7 M in pentane, 2 eq) was added dropwise and after stirring for 20 min dry DMF (0.278 ml, 6 eq) was added. The reaction mixture was stirred at -78°C for 4 h and then was quenched with sat. NH₄Cl solution. Phases were separated, aqueous phase was extracted with EtOAc (20 ml x 3). Organic phases were combined, washed with water and brine, dried over Na₂SO₄, and concentrated under vacuum. The residue was purified by silica gel column chromatography (CH₂Cl₂/Et₂O - 30/1) to yield the product **P127** as off-white solid (88 mg, 74%).

### Preparation 128. 5-Formyl-6-methoxy-1-{2-[4-(methylsulfonyl)piperazin-1-yl]propyl}-1H-indole-2-carbonitrile (P128)

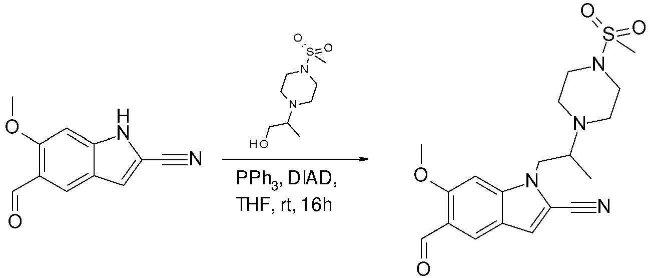

To a mixture of 2-[4-(methylsulfonyl)piperazin-1-yl]propan-1-ol **P107** (97 mg, 1.1 eq), 5-formyl-6-methoxy-1H-indole-2-carbonitrile (**P127**, 80 mg, 0.225 mmol) and triphenyl phosphine (126 mg, 1.2 eq) in THF (2 ml) DIAD (97 mg, 1.2 eq) was added, and the resulting mixture was stirred at ambient temperature overnight. The solvent was evaporated. The residue after evaporation was subjected to column chromatography on silica gel eluting with dichloromethane/ethyl acetate (0 - 20%) to afford product **P128** (90 mg, 56%) as a yellow solid. LCMS (ESI) [MH]⁺: 405.

### Synthesis of 5-formyl-4-methyl-1-[(6-methyl-5-oxomorpholin-2-yl)methyl]-1H-indole-2-carbonitrile (P129)

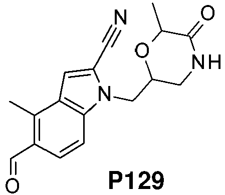

### Preparation 129. 5-Formyl-4-methyl-1-[(6-methyl-5-oxomorpholin-2-yl)methyl]-1H-indole-2-carbonitrile (P129)

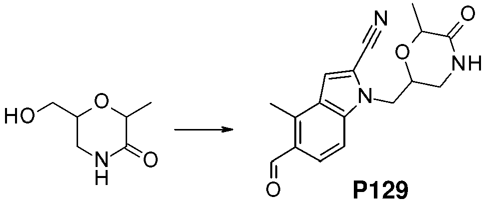

To a solution of 6-(hydroxymethyl)-2-methylmorpholin-3-one (145 mg, 1 mmol, prepared as described in Danklmaier, Johann; Hoenig, Helmut; Liebigs Annalen der Chemie, 1988, p. 1149 - 1154) in DMAA (6 ml) DIPEA (194 mg, 1.5 mmol) and MsCl (137 mg, 1.2 mmol) were added. The mixture was stirred at 50°C for 45 min, then 2-cyano-4-methyl-5-formylindole (**P97**, 92 mg, 0.5 mmol) and potassium carbonate (400 mg, 2.9 mmol) were added, and the mixture was stirred at 90°C for 48 h. After water (40 ml) was added the product was extracted with ethyl acetate (3x5 ml), ethyl acetate was evaporated to dryness. The residue was washed with dry ethyl acetate (4 ml), filtered, and dried to yield pure 5-formyl-4-methyl-1-[(6-methyl-5-oxomorpholin-2-yl)methyl]-1*H*-indole-2-carbonitrile (**P128**, 78 mg, 50 %). ¹H NMR (400 MHz, DMSO-*d₆*), δ: 10.4 (s, 1H), 7.88 (m, 3H), 7.65 (m, 1H), 4.62 (m, 1H), 4.43 (m, 1H), 4.26 (m, 2H), 3.26 (m, 3H), 3.10 (m, 1H), 2.87 (m, 3H). LCMS (ESI) [MH]⁺: 312.

### Synthesis of 1-[(6,6-dimethyl-5-oxomorpholin-2-yl)methyl]-5-formyl-4-methyl-1H-indole-2-carbonitrile (P131)

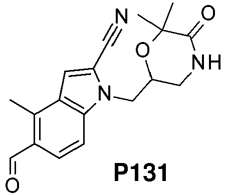

### Preparation 130. 6-(Hydroxymethyl)-2,2-dimethylmorpholin-3-one (P130)

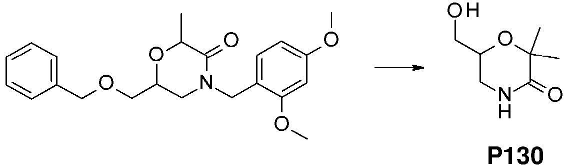

To a solution of 6-[(benzyloxy)methyl]-4-(2,4-dimethoxybenzyl)-2-methylmorpholin-3-one (3.85 g, 10 mmol, prepared according to procedure described in US2015/105370, 2015, A1) in THF (120 ml) under nitrogen a solution of LDA (12 mmol) was added with stirring at temperature -75°C. The reaction mixture was stirred at the same temperature for 30 min, then methyl iodide (1.7 g, 12 mmole) was added. The reaction mixture was allowed to reach rt and water solution of ammonium chloride was added. The product was extracted with ethylacetate (2x70 ml), organic phase was evaporated to dryness. The residue was dissolved in acetic acid (70 ml) and stirred under hydrogen pressure 70 bar with PdCl₂ (0.35 g) for 18 h at 50°C. The mixture evaporated to dryness, and the residue was purified by flash chromatography on silica gel with MeOH-EtOAc (15-85) to provide 6-(hydroxymethyl)-2,2-dimethylmorpholin-3-one (**P130**, 0.52 g, 36 %). ¹H NMR (400 MHz, DMSO-*d₆*), δ: 7.83 (s, 1H), 4.80 (m, 1H), 3.75 (m, 1H), 3.44 (m, 1H), 3.15 (m, 1H), 3.06 (m, 1H), 1.27 (m, 6H).

### Preparation 131. 1-[(6,6-Dimethyl-5-oxomorpholin-2-yl)methyl]-5-formyl-4-methyl-1H-indole-2-carbonitrile (P131)

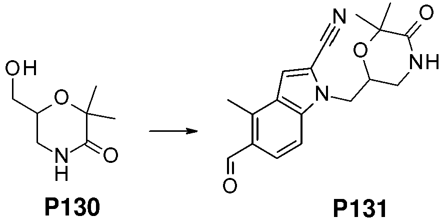

To a solution of 6-(hydroxymethyl)-2,2-dimethylmorpholin-3-one (**P130**, 159 mg, 1 mmol) in DMAA (6 ml) DIPEA (194 mg, 1.5 mmol) and MsCl (137 mg, 1.2 mmol) were added. The mixture was stirred at 50°C for 45 min, then 2-cyano-4-methyl-5-formylindole (**P97**, 92 mg, 0.5 mmol) and potassium carbonate (400 mg, 2.9 mmol) were added, and the mixture was stirred at 90°C for 48 h, after water was added (40 ml) the product was extracted with ethyl acetate (3x5 ml), and ethyl acetate was evaporated to dryness. The residue was washed with dry ethyl acetate (4 ml), filtered, and dried to yield pure 5-formyl-4-methyl-1-[(6-methyl-5-oxomorpholin-2-yl)methyl]-1*H*-indole-2-carbonitrile (**P131**, 78 mg, 48 %). ¹H NMR (400 MHz, CDCl₃), δ: 10.3 (s, 1H), 7.84 (m, 3H), 7.3 (m, 2H), 4.48 (m, 1H), 4.25 (m, 2H), 3.15 (m, 1H), 3.0 (m, 3H), 1.25 (m, 6H). LCMS (ESI) [MH]⁺: 326.

### Synthesis of 5-formyl-4-methyl-1-{[4-(methylsulfonyl)morpholin-2-yl]methyl}-1H-indole-2-carbonitrile (P132)

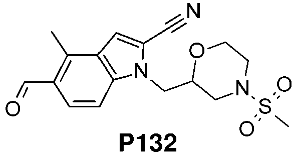

### Preparation 132. 5-Formyl-4-methyl-1-{[4-(methylsulfonyl)morpholin-2-yl]methyl}-1H-indole-2-carbonitrile (P132)

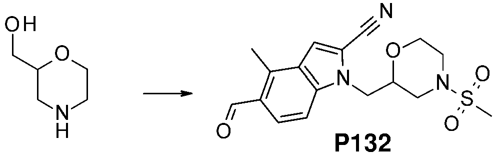

To a solution of morpholin-2-ylmethanol (117 mg, 1 mmol, was prepared according to WO2004/33440, 2004, A1) in DMAA (6 ml) DIPEA (387 mg, 3 mmol) and MsCl (239 mg, 2.1 mmol) were added. The mixture was stirred at 20°C for 45 min, then 2-cyano-4-methyl-5-formylindole (**P97**, 92 mg, 0.5 mmol) and potassium carbonate (600 mg, 4.35 mmol) were added, and the mixture was stirred at 90°C for 48 h. Water (40 ml) was added to the reaction mixture and product was extracted with ethyl acetate (3x5 ml), ethyl acetate was evaporated to dryness. The residue was washed with dry ethyl acetate (4 ml), filtered, and dried to yield pure 5-formyl-4-methyl-1-{[4-(methylsulfonyl)morpholin-2-yl]methyl}-1*H*-indole-2-carbonitrile (**P132**, 57 mg, 62 %). ¹H NMR (400 MHz, DMSO-*d₆*), δ: 10.4 (s, 1H), 8.14 (m, 1H), 7.51 (m, 1H), 6.55 (m, 1H), 4.22 (m, 1H), 3.88 (m, 2H), 3.75 (m, 4H), 3.58 (m, 2H), 2.95 (m, 3H). LCMS (ESI) [MH]⁺: 362.

### Synthesis of 4-(2,7-diazaspiro[3.5]non-2-yl)-5-fluoro-6-(2,2,2-trifluoroethyl)quinazoline (P137)

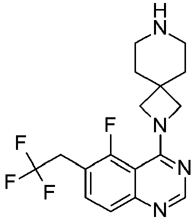

### Preparation 133. tert-Butyl 2-(6-bromo-5-fluoroquinazolin-4-yl)-2,7-diazaspiro[3.5]nonane-7-carboxylate (P133)

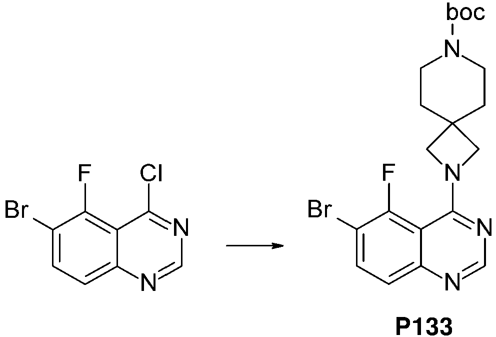

To solution of crude 3-chloro-6-bromo-5-fluoroquinazolin (WO2021/250521, 2021, A1) in DCE (40 ml) DIPEA (7.9 g, 61 mmol) and *tert-*butyl 2,7-diazaspiro[3.5]nonane-7-carboxylate hydrochloride (1.67 g, 6.37 mmol) were added. The reaction solution was stirred at rt for 18 h (LCMS control). Then product was purified by column chromatography on silica with ethylacetate-hexane (1-31) to yield the title compound **P133** (1.3 g, 47%), as a white solid. ¹H NMR (400 MHz, CDCl₃), δ: 8.72 (s, 1H), 7.82 (m,1H), 7.55 (m, 1H), 4.12 (m, 4H), 3.42 (m, 4H), 1.84 (m, 4H), 1.45 (s, 9H). LCMS (ESI) [MH]⁺: 452.

### Preparation 134. tert-Butyl 2-(5-fluoro-6-vinylquinazolin-4-yl)-2,7-diazaspiro[3.5]nonane-7-carboxylate (P134)

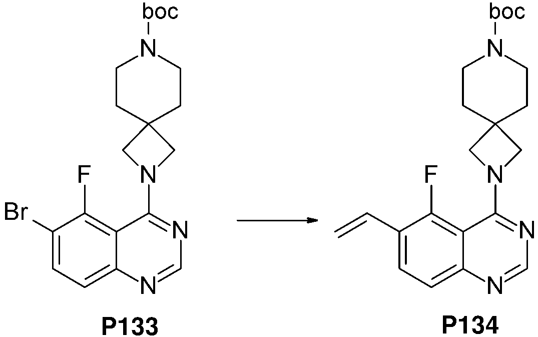

To a solution of *tert*-butyl 2-[5-fluoro-7-bromoquinazolin-1-yl]-2,7-diazaspiro[3.5]nonane-7-carboxylate (**P133**, 0.30 g, 0.69 mmol) in EtOH (10 ml), water (0.5 ml), and TEA (140 mg, 1.39 mmol) were added. Potassium vinyltrifluoroborate (140 mg, 1.03 mmol) and dichloro[l,l' - bis(diphenylphosphino)ferrocene]palladium(H) dichloromethane adduct (0.17 g, 0.21 mmol) then were added, and the reaction mixture was stirred at 80°C for 24 h. The reaction mixture was worked up with EtOAc and H₂O, and the layers were separated. The organic layer was washed with brine, dried over MgSO₄, filtered and concentrated. The crude material was purified on silica (gradient elution, 0-40% EtOAc/hexanes) to yield **P134** (210 mg, 80 %) as white solid. LCMS (ESI) [MH]⁺: 399.

### Preparation 135. tert-Butyl 2-(5-fluoro-6-formylquinazolin-4-yl)-2,7-diazaspiro[3.5]nonane-7-carboxylate (P135)

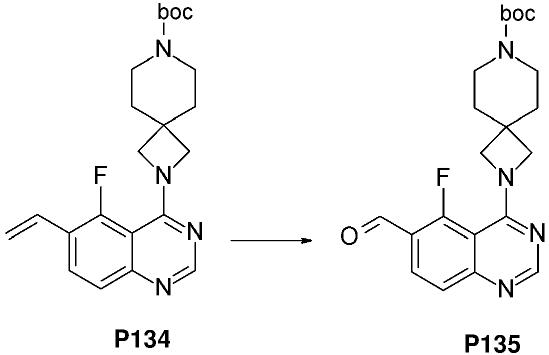

To a solution of *tert*-butyl 2-(7-vinylquinazolin-1-yl)-2,7-diazaspiro[3.5]nonane-7-carboxylate (**P134**, 210 mg, 0.55 mmol) in dioxane (2 ml) and water (0.42 ml) 2,6-lutidine (113 mg, 1.1 mmol), solution of osmium tetraoxide (10 mg) in *tert*-BuOH (0.5 ml) and sodium periodate (354 mg, 1.65 mmol) were added. The reaction mixture was stirred at rt for 24 h, after reaction complete, solution was cooled and added to ice-cold water solution of sodium bicarbonate. The mixture was stirred at 15°C for 30 min, then extracted with DCM (2x50 ml), DCM was concentrated, residue was purified by silica gel column chromatography (dichloromethane-ethylacetate - 1:1) to give **P135** (145 mg, 65 %) as a brown solid. ¹H NMR (400 MHz, CDCl₃), δ: 10.54 (s, 1H), 8.65 (s, 1H), 8.14 (m, 1H), 7.62 (m, 1H), 4.12 (m, 4H), 3.42 (m, 4H), 1.84 (m, 4H), 1.45 (s, 9H). LCMS (ESI) [MH]⁺: 401.

### Preparation 136. tert-Butyl 2-[5-fluoro-6-(2,2,2-trifluoroethyl)quinazolin-4-yl]-2,7-diazaspiro[3.5]nonane-7-carboxylate (P136)

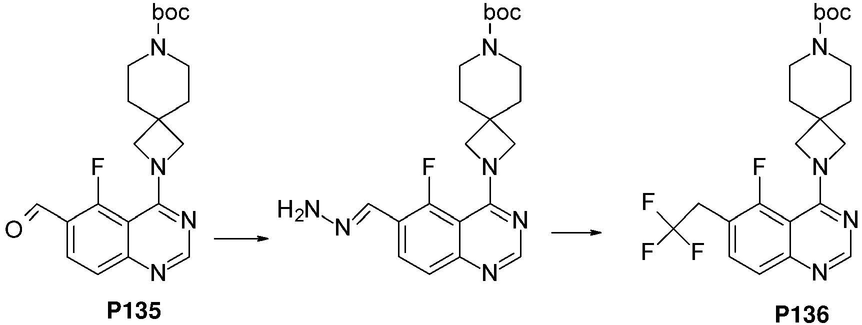

To a solution of *tert*-butyl 2-(7-formylquinazolin-1-yl)-2,7-diazaspiro[3.5]nonane-7-carboxylate (**P135**, 145 mg, 0.38 mmol) in MeOH (1 ml) hydrazine hydrate (38 mg, 0.76 mmol) was added. The solution was stirred at rt for 2 h (TLC control), then the reaction mixture was evaporated to dryness to yield crude hydrazone (150 mg) used in the next stage without purification. The hydrazone (150 mg, 0.38 mmol) was dissolved in DMSO (1 ml) under argon atmosphere, then Togni"s reagent (144 mg, 0.456 mmol) and TFA (43 mg, 0.38 mmol) were added. The resulting mixture was stirred at 50°C for 24 h. After cooling to rt, the mixture was extracted with ethylacetate (3x25 ml) and the combined organic layers were washed with water (3x60 ml) and brine (25 ml), and dried under Na₂SO₄. The organic layers were concentrated, and the residue was separated by HPLC (silica C18, MeCN-H₂O) to yield **P136** (75 mg, 45%). ¹H NMR (400 MHz, CDCl₃), δ: 8.55 (s, 1H), 7.85 (m, 1H), 7.55 (m, 1H), 4.32 (m, 4H), 3.62 m, 2H), 3.51 (m, 4H), 1.83 (m, 4H), 1.45 (s, 9H). LCMS (ESI) [MH]⁺: 455.

### Preparation 137. 4-(2,7-Diazaspiro[3.5]non-2-yl)-5-fluoro-6-(2,2,2-trifluoroethyl)quinazoline (P137)

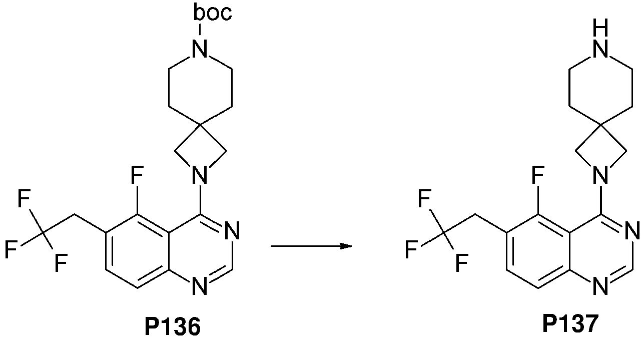

To *tert*-butyl 2-[7-(2,2,2-trifluoroethyl) quinazolin-1-yl]-2,7-diazaspiro[3.5]nonane-7-carboxylate (75 mg, 0.17 mmol) was added TFA (1ml). The solution was stirred at rt for 2 h (LCMS control), then evaporated to dryness, water solution of sodium bicarbonate (5 ml) was added, product was extracted with DCM (3x5 ml). Organic phase was dried with anh. sodium sulphate, concentrated to dryness to yield 1-(2,7-diazaspiro[3.5]non-2-yl)-7-(2,2,2-trifluoroethyl) quinazoline (57 mg, 100%). ¹H NMR (400 MHz, DMSO-*d₆*), δ: 8.64 (s, 1H), 7.74 (m, 2H), ), 4.32 (m, 4H), 3.62 m, 2H), 3.51 (m, 4H), 1.83 (m, 4H). LCMS (ESI) [MH]⁺: 355.

### Synthesis of 5-formyl-4-methyl-1-{(2S)-2-[4-(methylsulfonyl)piperazin-1-yl]propyl}-1H-indole-2-carbonitrile (P138)

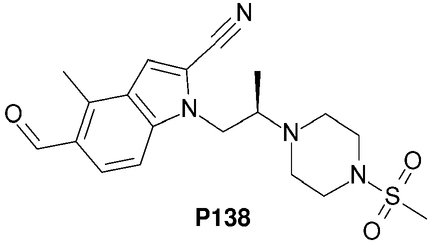

### Preparation 138. 5-Formyl-4-methyl-1-{(2S)-2-[4-(methylsulfonyl)piperazin-1-yl]propyl}-1H-indole-2-carbonitrile (P138)

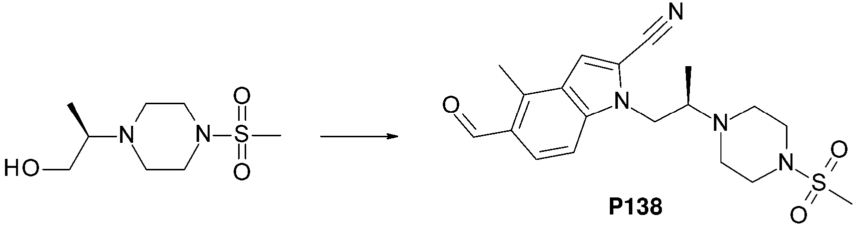

To a mixture of 2-[4-(tert-butylcarboxlato)piperazin-1-yl]propan-1-ol (200 mg, 0.9 mmol), 5-formyl-4-methyl-1H-indole-2-carbonitrile (165 mg, 0.9 mmol) and triphenyl phosphine (354 mg, 1.35 mmol) in toluene (5 ml) DIAD (273 mg, 1.35 mmol) was added, and the resulting mixture was stirred at ambient temperature overnight. The solvent was evaporated. The residue after evaporation was subjected to column chromatography on silica gel eluting with dichloromethane/ethyl acetate (0 → 20%) to afford **P138** (130 mg, 37%). ¹H NMR (400 MHz, DMSO-*d₆*), δ: 10.37 (s, 1H), 7.82-7.78 (m, 2H), 7.63 (d, J=7.0 Hz, 1H), 4.82-4.73 (m, 1H), 4.39-4.31 (m, 1H), 4.27-4.20 (m, 1H), 3.25-3.10 (m, 4H), 2.85 (s, 3H), 2.64-2.52 (m, 2H), 2.25-2.19 (m, 2H), 1.38 (s, 9H), 1,18-1,12 (m, 3H). LCMS (ESI) [MH]⁺: 411.

### Synthesis of 4-(2,7-diazaspiro[3.5]non-2-yl)-7-fluoro-6-(2,2,2-trifluoroethyl)quinazoline (P143)

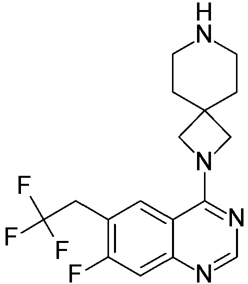

### Preparation 139. tert-Butyl 2-(6-bromo-7-fluoroquinazolin-4-yl)-2,7-diazaspiro[3.5]nonane-7-carboxylate (P139)

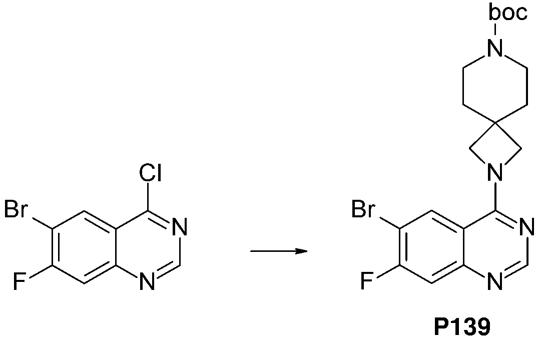

To solution of crude 3-chloro-6-bromo-7-fluoroquinazolin (1.6 g, synthesized according to WO2011/131741, 2011, A1) in DCM (40 ml) DIPEA (7.9 g, 61 mmol) and *tert-*butyl 2,7-diazaspiro[3.5]nonane-7-carboxylate hydrochloride (1.67 g, 6.37 mmol) were added. The reaction solution was stirred at rt for 18 h (LCMS control). Then product was purified by column chromatography on silica with ethylacetate-hexane (1-31) yield **P139** (1.3 g, 47%), as a white solid. ¹H NMR (400 MHz, DMSO-*d₆*), δ: 8.42 (s, 1H), 8.18 (m, 1H), 7.65 (m, 1H), 3.38 (m, 4H), 2.50 (m, 4H), 1.68 (m, 4H), 1.44 (s, 9H). LCMS (ESI) [MH]⁺: 451.

### Preparation 140. tert-Butyl 2-(7-fluoro-6-vinylquinazolin-4-yl)-2,7-diazaspiro[3.5]nonane-7-carboxylate (P140)

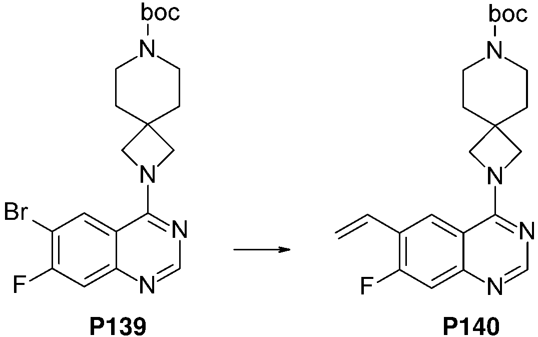

To a solution of *tert-*butyl 2-[6-bromo-7-fluoroquinazolin quinazolin-1-yl]-2,7-diazaspiro[3.5]nonane-7-carboxylate (**P139**, 0.30 g, 0.69 mmol) in EtOH (10 ml), water (0.5 ml) and TEA (140 mg, 1.39 mmol) were added. Potassium vinyltrifluoroborate (140 mg, 1.03 mmol) and dichloro[1,1'-bis(diphenylphosphino)ferrocene]palladium(H) dichloromethane adduct (0.17 g, 0.21 mmol) then were added, and the reaction mixture was stirred at 80°C for 24 h. The reaction mixture was worked up with EtOAc and H₂O, and the layers were separated. The organic layer was washed with brine, dried over MgSO₄, filtered and concentrated. The crude material was purified on silica (gradient elution, 0-40% EtOAc/hexanes) to yield **P140** (210 mg, 80 %) as white solid. ¹H NMR (400 MHz, CDCl₃), δ: 8.56 (s, 1H), 7.90 (m, 1H), 7.45 (m, 1H), 6.86 (m, 1H), 5.79 (m,1H), 5.43 (m, 1H), 4.2 (m, 4H), 3.47 (m, 4H), 1.82 (m, 4H), 145 (m, 9H). LCMS (ESI) [MH]⁺: 399.

### Preparation 141. tert-Butyl 2-(7-fluoro-6-formylquinazolin-4-yl)-2,7-diazaspiro[3.5]nonane-7-carboxylate (P141)

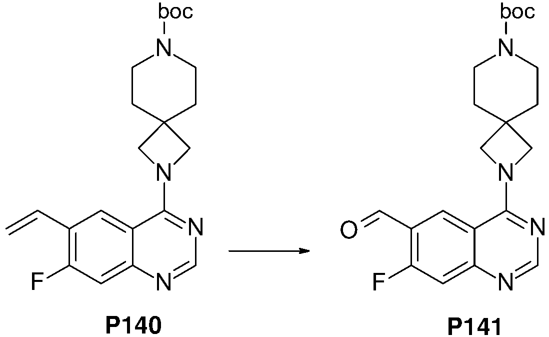

To a solution of *tert*-butyl 2-(6-vinyl-7-fluoroquinazolin-1-yl)-2,7-diazaspiro[3.5]nonane-7-carboxylate (**P140**, 210 mg, 0.55 mmol) in dioxane (2 ml) and water (0.42 ml) 2,6-lutidine (113 mg, 1.1 mmol), solution of osmium tetraoxide (10 mg) in *tert*-BuOH (0.5 ml) and sodium periodate (354 mg, 1.65 mmol) were added. The reaction mixture was stirred at rt for 24 h, after reaction completed, the solution was cooled and added to ice-cold water solution of sodium bicarbonate. The mixture was stirred at 15°C for 30 min, then extracted with DCM (2x50 ml), DCM was concentrated, the residue was purified by silica gel column chromatography (dichloromethane-ethylacetate - 1:1) to give **P141** (145 mg, 65 %) as a brown solid. ¹H NMR (400 MHz, CDCl₃), δ: 10.37 (s, 1H), 8.60 (s, 1H), 8.40 (m, 1H), 7.56 (m, 1H), 4.32 (m, 4H), 3.48 (m, 4H), 1.85 (m, 4H), 1.44 (s, 9H). LCMS (ESI) [MH]⁺: 401.

### Preparation 142. tert-Butyl 2-[7-fluoro-6-(2,2,2-trifluoroethyl)quinazolin-4-yl]-2,7-diazaspiro[3.5]nonane-7-carboxylate (P142)

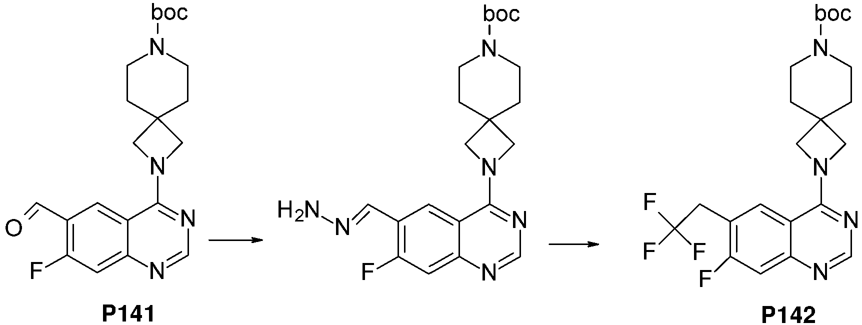

To a solution of *tert*-butyl 2-(6-formy-7-fluoroquinazolin-1-yl)-2,7-diazaspiro[3.5]nonane-7-carboxylate (**P141**, 145 mg, 0.38 mmol) in MeOH (1 ml) hydrazine hydrate (38 mg, 0.76 mmol) was added. The solution was stirred at rt for 2 h (TLC control), then the reaction mixture was evaporated to dryness to yield crude hydrazone (150 mg) used in the next stage without purification. The hydrazone (150 mg, 0.38 mmol) was dissolved in DMSO (1 ml) under argon atmosphere, then Togni"s reagent (144 mg, 0.456 mmol) and TFA (43 mg, 0.38 mmol) were added. The resulting mixture was stirred at 50°C for 24 h. After cooling to rt, the mixture was extracted with ethylacetate (3x25 ml) and the combine organic layers were washed with water (3x60 ml) and brine (25 ml) and dried under anh. Na₂SO₄. The organic layers were concentrated, and the residue was separated by HPLC (silica C18, MeCN-H₂O) to yield **P142** (75 mg, 45%). ¹H NMR (400 MHz, CDCl₃), δ: 8.66 (s, 1H), 7.83 (m, 1H), 7,50 (m, 1H), 4.25 (m, 4H), 3.58 (m, 2H), 3.43 (m, 4H), 1.85 (m, 4H), 1.44 (s, 9H). LCMS (ESI) [MH]⁺: 455.

### Preparation 143. 4-(2,7-Diazaspiro[3.5]non-2-yl)-7-fluoro-6-(2,2,2-trifluoroethyl)quinazoline (P143)

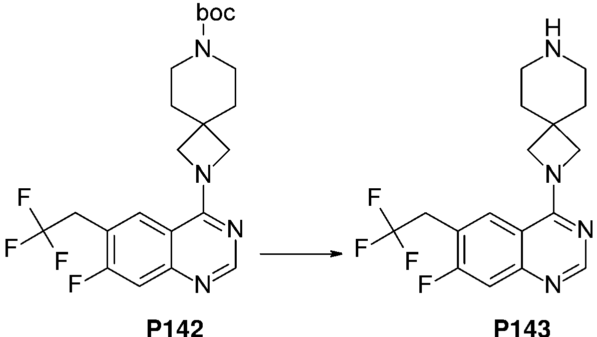

To *tert-*butyl 2-[6-(2,2,2-trifluoroethyl)-7-fluoroquinazolin-1-yl]-2,7-diazaspiro[3.5]nonane-7-carboxylate (**P142**, 75 mg, 0.17 mmol) TFA (1ml) was added. The solution was stirred at rt for 2 h (LCMS control), then evaporated to dryness, water solution of sodium bicarbonate (5 ml) was added, product was extracted with DCM (3x5 ml). Organic phase was dried with anh. sodium sulphate, concentrated to dryness to yield **P143** (57 mg, 100%). LCMS (ESI) [MH]⁺: 355.

### Synthesis of 5-formyl-4-methyl-1-[(6-oxo-1,6-dihydropyridin-3-yl)methyl]-1H-indole-2-carbonitrile (P145)

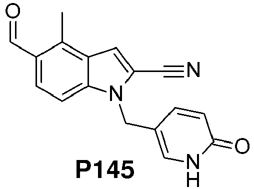

### Preparation 144. 5-(Hydroxymethyl)pyridin-2(1H)-one (P144)

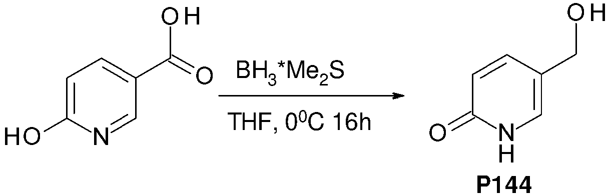

To a dispersion of 6-hydroxynicotinic acid (1.0 g, 7.2 mmol) in 20 ml of MeOH BH₃*Me₂S (2M, 5eq) was added dropwise at 0°C. The reaction mixture was stirred at rt for 16 h. The reaction mixture was cooled to 0°C and MeOH (100 ml) was slowly added. The reaction mixture was stirred for 15 min, and then concentrated to dryness. The residue was suspended in 20% MeOH-80% CHCl₃, stirred for 5 min, and solids were filtered-off. Filtrate was concentrated to give product **P144** as white solid (800mg, 89%).

### Preparation 145. 5-Formyl-4-methyl-1-[(6-oxo-1,6-dihydropyridin-3-yl)methyl]-1H-indole-2-carbonitrile (P145)

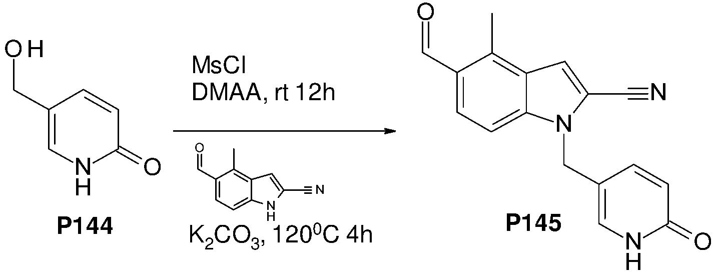

To a solution of 5-(hydroxymethyl)pyridin-2(1H)-one (**P144**, 100 mg, 0.8 mmol) and DIPEA (1.2 eq) in DMAA (5 ml) at rt MsCl (1.1 eq) was added. The reaction mixture was stirred at rt for 16 h. Then K₂CO₃ (4eq) and 5-formyl-4-methyl-1H-indole-2-carbonitrile (**P97**, 1eq) were added and mixture was heated to 140°C for 4h. The reaction mixture was diluted with water and extracted with EtOAc. The residue after evaporation was subjected to column chromatography on silica gel eluting with dichloromethane-MeOH (0 - 20%) to afford product **P145** (110 mg, 47%) as a yellow solid. ¹H NMR (400 MHz, DMSO-*d₆*), δ: 11.53 (s, 1H), 10.36 (s, 1H), 7.86 (d, J = 12.0 Hz, 2H), 7.79 (d, J = 8.9 Hz, 1H), 7.44 (s, 1H), 7.23 (dd, J = 9.4, 2.3 Hz, 1H), 6.29 (d, J = 9.5 Hz, 1H), 5.36 (s, 2H), 2.85 (s, 3H). LCMS (ESI) [MH]⁺: 292.

### Synthesis of 4-(2,7-Diazaspiro[3.5]non-2-yl)-6-(2-phenylethyl)quinazoline (P148)

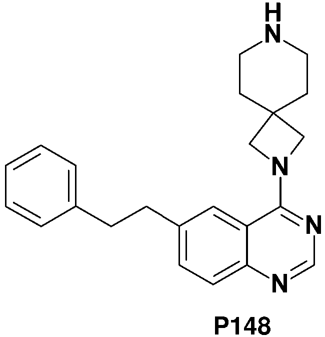

### Preparation 146. tert-Butyl 2-(6-bromoquinazolin-4-yl)-2,7-diazaspiro[3.5]nonane-7-carboxylate (P146)

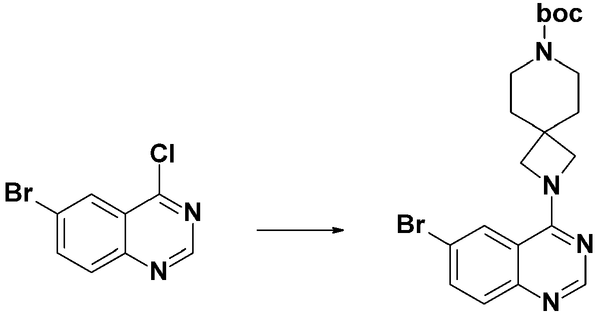

To a solution 6-bromo-4-chloroquinazoline prepared according to WO2013/57711, 2013, A1 (3 g, 12.3 mmol) in DCM (60 ml) were added DIPEA (7.9 g, 61 mmol) and *tert*-butyl 2,7-diazaspiro[3.5]nonane-7-carboxylate hydrochloride (3.2 g, 12.2 mmol). The reaction solution was stirred at rt for 18 h (LCMS control). Then obtained product was purified by column chromatography on silica with ethyl acetate-methanol (10:1) to yield 5.1 g (96%) of **P146** as a white solid. LCMS (ESI) [MH]⁺: 434.

### Preparation 147. of tert-Butyl 2-[6-(2-phenylethyl)quinazolin-4-yl]-2,7-diazaspiro[3.5]nonane-7-carboxylate (P147)

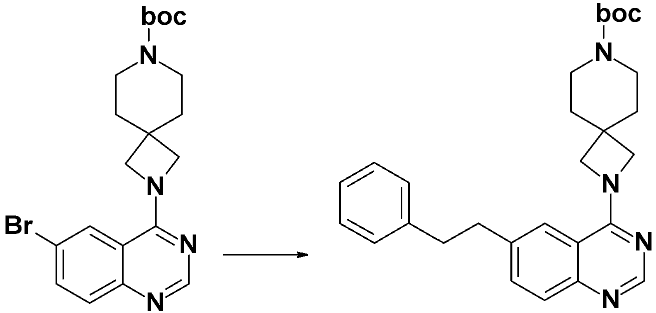

To a solution of *tert*-butyl 2-(6-bromoquinazolin-4-yl)-2,7-diazaspiro[3.5]nonane-7-carboxylate (**P146**, 200 mg 0.46 mmol) in DMAA (2 ml) under argon atmosphere were added TEA (186 mg, 1.84 mmol), styrene (144 mg, 1.48 mmol) and Pd(PPh₃)₄ (20 mg). The mixture was stirred at 90°C for 16 h (LCMS control). After reaction completed Pd-C (30 mg) was added and the mixture was stirred at rt under hydrogen atmosphere until reaction completed (LCMS control). Then the mixture was filtered, washed with brine (40 ml) and extracted with ethyl acetate (2x5 ml), concentrated and residue was purified by silica gel column chromatography (dichloromethane-ethyl acetate - 1:1) to give the title product **P147** (145 mg, 69%). LCMS (ESI) [MH]⁺: 459.

### Preparation 148. 4-(2,7-Diazaspiro[3.5]non-2-yl)-6-(2-phenylethyl)quinazoline (P148)

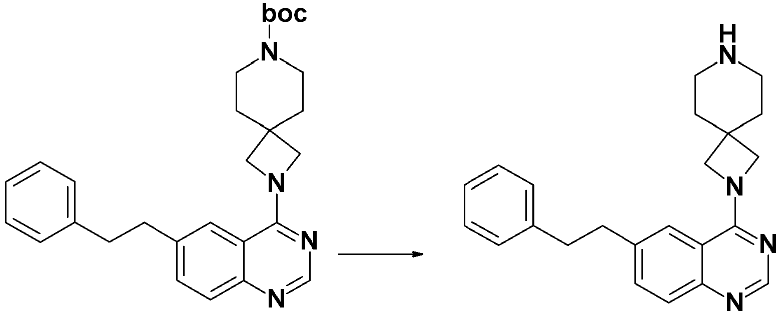

To *tert*-butyl 2-[6-(2-phenylethyl)quinazolin-4-yl]-2,7-diazaspiro[3.5]nonane-7-carboxylate (**P147**, 145 mg, 0.316 mmol) was added TFA (1 ml). The solution was stirred at rt for 2 h (LCMS control), then the reaction mixture was evaporated to dryness, water solution of sodium bicarbonate (5 ml) was added, product extracted with DCM (3x5 ml). Combined organic extract was dried with sodium sulphate, concentrated to dryness to yield the title product **P148** (113 mg, 100%). LCMS (ESI) [MH]⁺: 359.

### Synthesis of 4-(2,7-diazaspiro[3.5]non-2-yl)-6-(2-pyridin-3-ylethyl)quinazoline (P150)

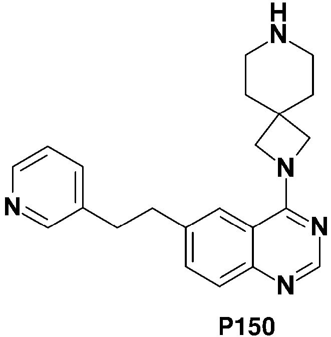

### Preparation 149. tert-Butyl 2-[6-(2-pyridin-3-ylethyl)quinazolin-4-yl]-2,7-diazaspiro[3.5]nonane-7-carboxylate (P149)

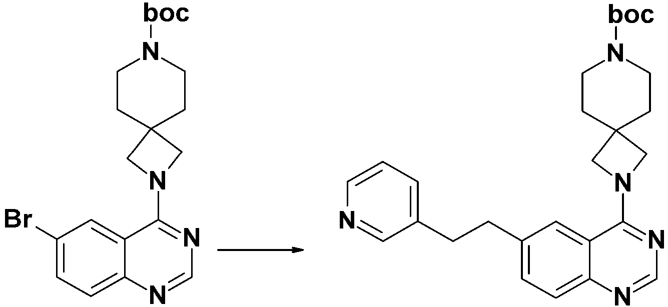

To a solution of *tert*-butyl 2-(6-bromoquinazolin-4-yl)-2,7-diazaspiro[3.5]nonane-7-carboxylate (**P146**, 200 mg 0.46 mmol) in DMAA (2 ml) TEA (186 mg, 1.84 mmol), 3-vinylpyridine (144 mg, 1.48 mmol) and Pd(PPh₃)₄ (20 mg) were added under argon atmosphere. The mixture was stirred at 90°C for 16 h (LCMS control). After reaction completed Pd-C (30 mg) was added and mixture was stirred at rt under hydrogen atmosphere until reaction complete (LCMS control). Then the mixture was filtered, washed with brine (40 ml) and extracted with ethyl acetate (2x5 ml), concentrated and residue was purified by silica gel column chromatography (DCM-ethyl acetate - 1:1) to give the title product **P149** (145 mg, 69 %). LCMS (ESI) [MH]⁺: 460.

### Preparation 150. 4-(2,7-Diazaspiro[3.5]non-2-yl)-6-(2-pyridin-3-ylethyl)quinazoline (P150)

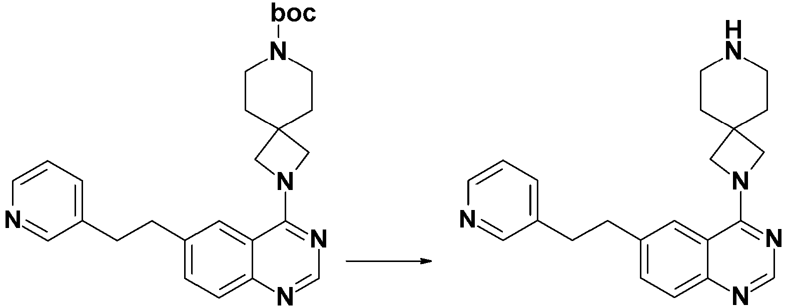

To *tert*-butyl 2-[6-(2-pyridin-3-ylethyl)quinazolin-4-yl]-2,7-diazaspiro[3.5]nonane-7-carboxylate (**P149**, 145 mg, 0.316 mmol) was added TFA (1 ml). The solution was stirred at rt for 2 h (LCMS control), then evaporated to dryness, water solution of sodium bicarbonate (5 ml) was added, product was extracted with DCM (3x5 ml). Organic phase dried with sodium sulphate, concentrated to dryness yield title product **P150** 113 mg, 100%. LCMS (ESI) [MH]⁺: 360.

### Synthesis of 4-(2,7-diazaspiro[3.5]non-2-yl)-6-(2-pyridin-4-ylethyl)quinazoline (P152)

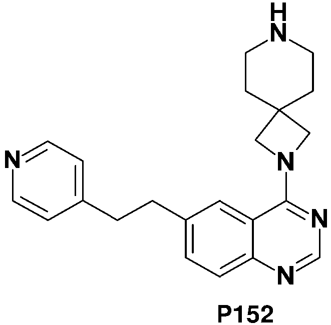

### Preparation 151. tert-Butyl 2-[6-(2-pyridin-4-ylethyl)quinazolin-4-yl]-2,7-diazaspiro[3.5]nonane-7-carboxylate (P151)

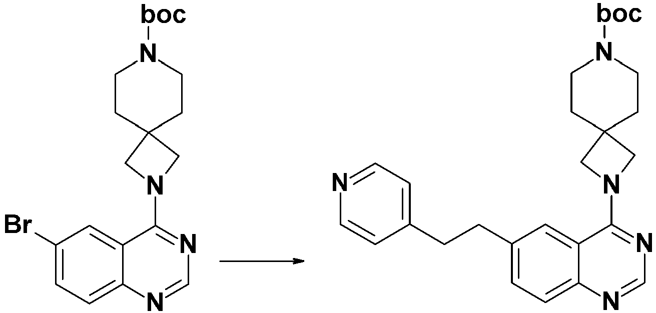

To a solution of *tert*-butyl 2-(6-bromoquinazolin-4-yl)-2,7-diazaspiro[3.5]nonane-7-carboxylate (**P146**, 200 mg 0.46 mmol) in DMAA (2 ml) TEA (186 mg, 1.84 mmol), 4-vinylpyridine (144 mg, 1.48 mmol) and Pd(PPh₃)₄ (20 mg) were added under argon atmosphere. The mixture was stirred at 90°C for 16 h (LCMS control). After reaction completed Pd/C (30 mg) was added and the mixture was stirred at rt under hydrogen atmosphere until reaction was completed (LCMS control). Then mixture was filtered, washed with brine (40 ml) and extracted with ethyl acetate (2x5 ml), concentrated and residue was purified by silica gel column chromatography (dichloromethane-ethyl acetate - 1:1) to give title product **P151** (145 mg, 69%). LCMS (ESI) [MH]⁺: 460.

### Preparation 152. 4-(2,7-Diazaspiro[3.5]non-2-yl)-6-(2-pyridin-4-ylethyl)quinazoline (P152)

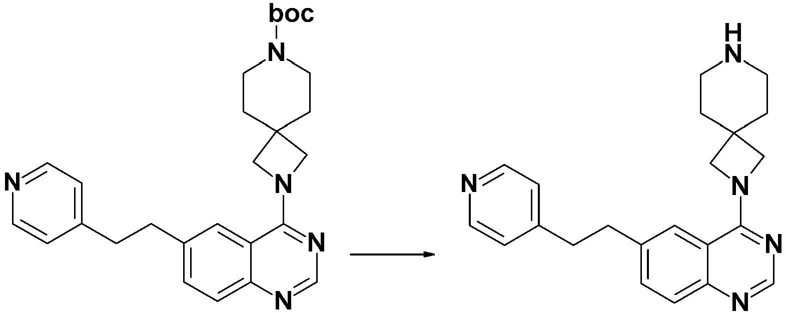

To *tert*-butyl 2-[6-(2-pyridin-4-ylethyl)quinazolin-4-yl]-2,7-diazaspiro[3.5]nonane-7-carboxylate (**P151**, 145 mg, 0.316 mmol) was added TFA (1 ml). The solution was stirred at rt for 2 h (LCMS control), then evaporated to dryness, water solution of sodium bicarbonate (5 ml) was added, the product was extracted with DCM (3x5 ml). Organic phase was dried with sodium sulphate, concentrated to dryness to yield the title compound **P152** (113 mg, 100%). LCMS (ESI) [MH]⁺: 360

### Synthesis of 4-(2,7-diazaspiro[3.5]non-2-yl)-6-(phenylethynyl)quinazoline (P154)

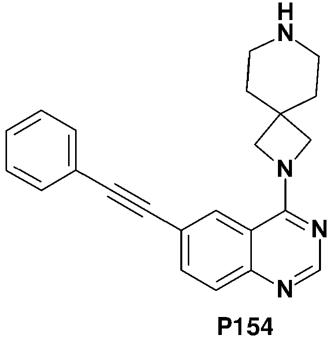

### Preparation 153. tert-Butyl 2-[6-(phenylethynyl)quinazolin-4-yl]-2,7-diazaspiro[3.5]nonane-7-carboxylate (P153)

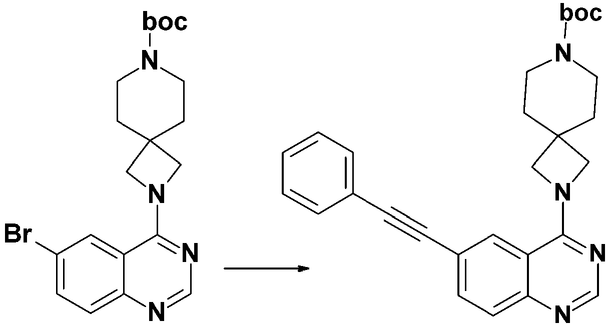

*tert*-Butyl 2-(6-bromoquinazolin-4-yl)-2,7-diazaspiro[3.5]nonane-7-carboxylate (**P146**, 80 mg, 0.185 mmol) was dissolved in DMF (1 mL) and purged with N₂ on an oil bath at 80°C for 10 minutes. Then bis(triphenylphosphine)palladium(II) dichloride (11 mg, 0.016 mmol), triphenylphosphine (10 mg, 0.037 mmol) and copper iodide ( 7 mg, 0.037 mmol) were added. After 5 minutes of purging with N₂, diethylamine (0.5 ml, 5 mmol) was added followed by the addition of phenylacetylene (0.03 ml, 0.27 mmol). The vessel was closed, and the reaction stirred at 80°C for 16 hours. The reaction mixture was poured into ice water, and the precipitate was isolated by filtration, washed with water, and dried under vacuum. The product was stirred in DCM for 30 minutes. The precipitate was isolated by filtration, washed with DCM and diisopropyl ether and dried under vacuo at 50°C to obtain *tert*-butyl 2-[6-(phenylethynyl)quinazolin-4-yl]-2,7-diazaspiro[3.5]nonane-7-carboxylate (**P153**, 56 mg, 70%). LCMS (ESI) [MH]⁺: 455.

### Preparation 154. 4-(2,7-Diazaspiro[3.5]non-2-yl)-6-(phenylethynyl)quinazoline (P154)

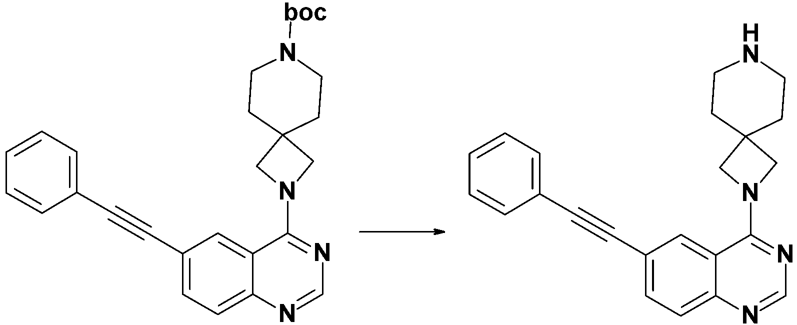

To *tert*-butyl 2-[6-(phenylethynyl)quinazolin-4-yl]-2,7-diazaspiro[3.5]nonane-7-carboxylate (**P153**, 56 mg, 0.123 mmol) was added TFA (1 ml). The solution stirred at rt for 2 h (LCMS control), then evaporated to dryness, water solution of sodium bicarbonate (5 ml) was added, product extracted with DCM (3x5 ml). Organic phase dried with sodium sulphate, concentrated to dryness yield 1-(2,7-diazaspiro[3.5]non-2-yl)-7-(2,2,2-trifluoroethyl)phthalazine (**P154**, 44 mg, 100%). LCMS (ESI) [MH]⁺: 355.

### Synthesis of 4-(2,7-diazaspiro[3.5]non-2-yl)quinazoline (P156)

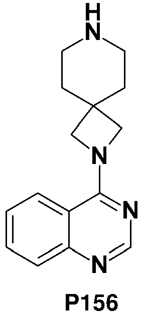

### Preparation 155. tert-Butyl 2-(quinazolin-4-yl)-2,7-diazaspiro[3.5]nonane-7-carboxylate (P155)

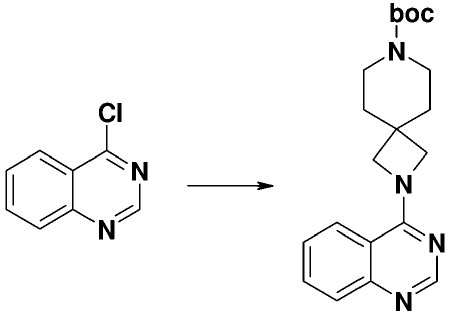

To a solution 4-chloroquinazoline prepared according to WO2007/38387, 2007, A2 (3 g, 18.2 mmol) in DCM (60 ml) was added DIPEA (7/9 g, 61 mmol) and *tert-*butyl *2,7-*diazaspiro[3.5]nonane-7-carboxylate hydrochloride (4.8 g, 18.2 mmol). The reaction solution was stirred at rt for 18 h (LCMS control). Then product was purified by column chromatography on silica with ethyl acetate-methanol (10:1) to yield **P155** (6.2 g, 96%) as a white solid. LCMS (ESI) [MH]⁺: 355.

### Preparation 156. 4-(2,7-Diazaspiro[3.5]non-2-yl)quinazoline (P156)

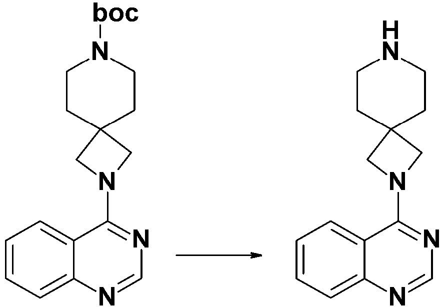

To *tert*-butyl 2-(quinazolin-4-yl)-2,7-diazaspiro[3.5]nonane-7-carboxylate (6.2 g, 17.5 mmol) was added TFA (60 ml). The solution was stirred at rt for 2 h (LCMS control) , then evaporated to dryness, water solution of sodium bicarbonate (100 ml) was added, product was extracted with DCM (3x50 ml). Organic phase was dried with sodium sulphate and concentrated to dryness to yield the title compound **P156** (4.44 g, 100%). LCMS (ESI) [MH]⁺: 255.

### Synthesis of 4-(2,7-diazaspiro[3.5]non-2-yl)pyrido[2,3-d]pyrimidine (P158)

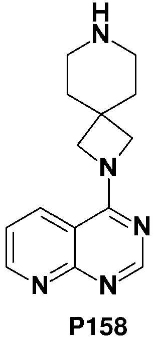

### Preparation 157. tert-Butyl 2-pyrido[2,3-d]pyrimidin-4-yl-2,7-diazaspiro[3.5]nonane-7-carboxylate (P157)

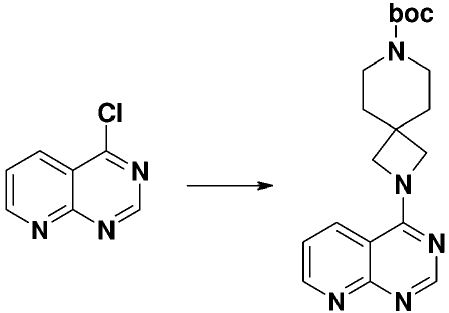

To a solution of 4-chloro-pyrido[2,3-d]pyrimidine prepared according to the procedure described by Robins; Hitchings [Journal of the American Chemical Society, 1955, vol. 77, p. 2256,2259] (3 g, 18.2 mmol) in dichloroethane (60 ml) was added DIPEA (7.9 g, 61 mmol) and *tert*-butyl 2,7-diazaspiro[3.5]nonane-7-carboxylate hydrochloride (4.8 g, 18.2 mmol). The reaction solution was stirred at rt for 18 h (LCMS control). After evaporation of the solvent the product was purified by column chromatography on silica with ethyl acetate-methanol (10:1) to yield **P157** (6.2 g, 96%) as a white solid. LCMS (ESI) [MH]⁺: 356.

### Preparation 158. 4-(2,7-Diazaspiro[3.5]non-2-yl)pyrido[2,3-d]pyrimidine (P158)

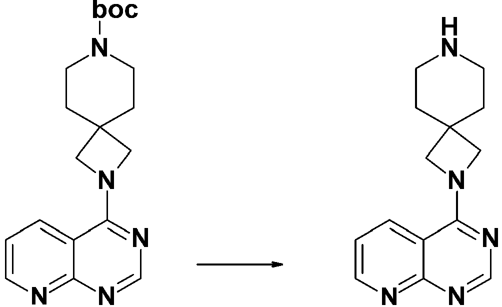

To *tert*-butyl 2-pyrido[2,3-d]pyrimidin-4-yl-2,7-diazaspiro[3.5]nonane-7-carboxylate (**P157**, 6.2 g,17.5 mmol) was added TFA (60 ml). The solution was stirred at rt for 2 h (LCMS control), then evaporated to dryness, water solution of sodium bicarbonate (100 ml) was added, product was extracted with DCM (3x50 ml). Organic phase dried with sodium sulphate, concentrated to dryness to yield the title compound **P158** (4.44 g, 100%). LCMS (ESI) [MH]⁺: 256.

### Synthesis of 4-chloro-7-(2,2,2-trifluoroethyl)quinazoline (P165)

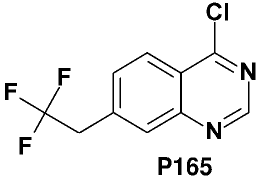

### Preparation 159. Methyl 4-bromo-2-[(tert-butoxycarbonyl)amino]benzoate (P159)

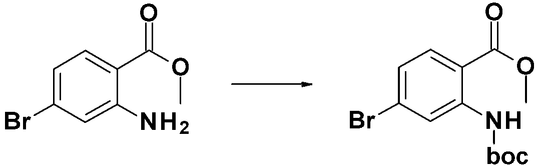

To a solution of methyl 2-amino-4-bromobenzoate (25 g, 0.109 mol) in DCM (250 ml) Boc₂O (26.5 g, 0.122 mol), triethylamine (62 g, 0.61 mol) and DMAP (4 g, 33 mmol) were added. The solution was stirred at ambient temperature for 18 h, then water (250 ml) was added and with stirring potassium hydrosulfate was added small portions up to pH=3. Water phase was extracted with DCM (200 ml), organic solution was concentrated, and residue was purified with silica gel column chromatography (30% DCM in hexane) to yield methyl 4-bromo-2-[(tert-butoxycarbonyl)amino]benzoate **P159** as white solid (14.3 g, 40 %). LCMS (ESI) [MH]⁺: 331.

### Preparation 160. Methyl 2-[(tert-butoxycarbonyl)amino]-4-(4,4,5,5-tetramethyl-1,3,2-dioxaborolan-2-yl)benzoate (P160)

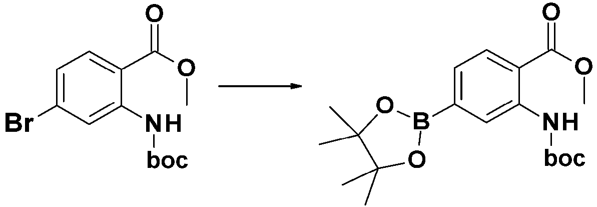

Methyl 4-bromo-2-[(tert-butoxycarbonyl)amino]benzoate (**P159**, 14.3 g, 43 mmol), and bis(pinacolato)diboron (22 g, 87 mmol) were dissolved in dioxane (120 ml), then potassium acetate (12.6 g, 0.129 mol) was added. The mixture was stirred for 30 min at 70°C under argon atmosphere, Pd(dppf)Cl₂ (3.2 g) was added, and reaction mixture was stirred at 100°C for 3 h (TLC control), the mixture was cooled and filtered through a pad of celite. The filtrate was then concentrated. The residue was purified by silica gel column chromatography (DCM) to give the title product **P160** (16 g, 99%) as a white solid. LCMS (ESI) [MH]⁺: 378.

### Preparation 161. Methyl 2-[(tert-butoxycarbonyl)amino]-4-(2,2,2-trifluoroethyl)benzoate (P161)

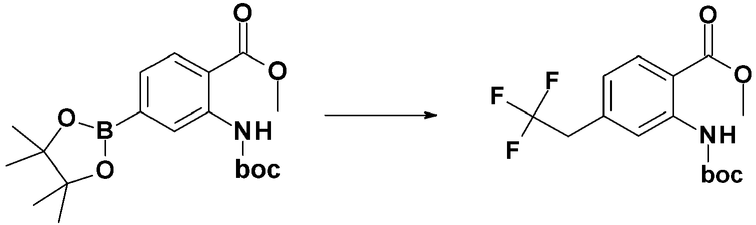

Methyl 2-[(*tert*-butoxycarbonyl)amino]-4-(4,4,5,5-tetramethyl-1,3,2-dioxaborolan-2-yl)benzoate (**P160**, 11.2 g, 30 mmol) was dissolved in dioxane (100 ml), cesium carbonate (32.6 g, 118 mmol) and water (8 ml) were added. The mixture was stirred for 30 min at 70°C under argon atmosphere, then iodotrifluoroethane (18.6 g, 89 mmol) , Pd₂(dba)₃ (2.6 g), and Xantphos (2.6 g) were added. Reaction mixture was stirred at 82°C for 20 h, then the mixture was cooled and filtered through a pad of celite. The filtrate was concentrated. The residue was purified by silica gel column chromatography (DCM-Hexane - 1:1) to give the title product **P161** (5.85 g 59 %) as a white solid. LCMS (ESI) [MH]⁺: 334.

### Preparation 162. Methyl 2-amino-4-(2,2,2-trifluoroethyl)benzoate (P162)

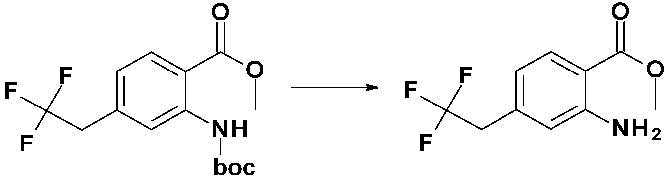

To methyl 2-[(tert-butoxycarbonyl)amino]-4-(2,2,2-trifluoroethyl)benzoate (**P161**, 5.85 g, 17.6 mmol) solution 3 M HCl in dioxane was added. The reaction mixture was stirred at rt for 2 h (NMR control) and concentrated to dryness to yield the title product **P162** (4.6 g, 100%) as hydrochloride. LCMS (ESI) [MH]⁺: 234.

### Preparation 163. 2-Amino-4-(2,2,2-trifluoroethyl)benzoic acid (P163)

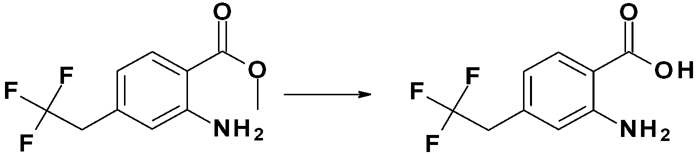

To a solution of methyl 2-amino-4-(2,2,2-trifluoroethyl)benzoate (**P162**, 4.6 g, 17.6 mmol) in methanol (100 ml) lithium hydroxide (2 g, 83 mmol) and water (35 ml) were added. the mixture was stirred at 50°C for 1h (TLC control) then cooled, concentrated to dryness, and water (50 ml) was added . To the solution with stirring and cooling with cold water concentrated HCl was added up to pH=3, precipitate was filtered off, washed with water (15 ml) and dried to yield the title product **P163** (3 g, 78%) as a white solid. LCMS (ESI) [MH]⁺: 220.

### Preparation 164. 7-(2,2,2-Trifluoroethyl)quinazolin-4(3H)-one (P164)

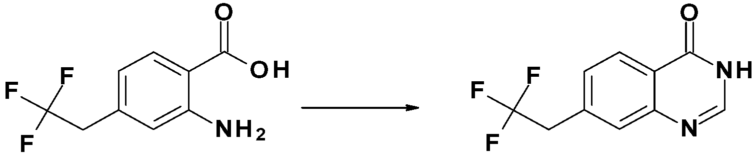

A mixture of 2-amino-4-(2,2,2-trifluoroethyl)benzoic acid (**P163**, 3 g, 13.7 mmol) and formamide (2.2 g, 49 mmol) were stirred in vial at 155°C for 1.5 h, and at 165°C for 40 min, cooled, and saturated solution of sodium bicarbonate (50 ml) was added. The mixture was stirred at rt for 30 min. Product was filtered off, washed with water (55 ml) and dried to yield the title product **P164** (2.52 g, 85%) as a white solid. LCMS (ESI) [MH]⁺: 229.

### Preparation 165. 4-Chloro-7-(2,2,2-trifluoroethyl)quinazoline (P165)

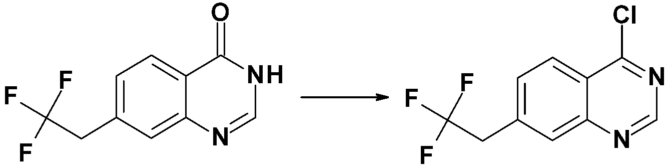

To 7-(2,2,2-trifluoroethyl)quinazolin-4(3H)-one (**P164**, 2.52 g, 11 mmol) POCl₃ (35 ml) was added, the reaction mixture was stirred and refluxed for 45 min (TLC control), after the reaction completed, the solution was cooled and added to ice-cold water solution of sodium bicarbonate. The mixture was stirred at 15°C for 30 min, then extracted with DCM (2x50 ml), DCM was concentrated, residue was purified by silica gel column chromatography (dichloromethane-ethyl acetate - 10:1) to give the title product **P165** (1.9 g 70 %) as a brown solid. LCMS (ESI) [MH]⁺: 247.

### Synthesis of 4-(2,7-diazaspiro[3.5]non-2-yl)-7-(2,2,2-trifluoroethyl)quinazoline (P167)

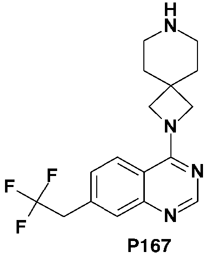

### Preparation 166. tert-Butyl 2-[7-(2,2,2-trifluoroethyl)quinazolin-4-yl]-2,7-diazaspiro[3.5]nonane-7-carboxylate (P166)

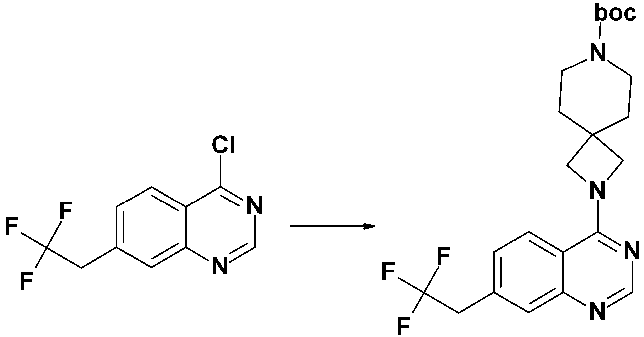

To a solution of 4-chloro-7-(2,2,2-trifluoroethyl)quinazoline (**P165**, 3 g, 12.2 mmol) in dichloroethane (60 ml) was added DIPEA (7.9 g, 61 mmol) and *tert*-butyl 2,7-diazaspiro[3.5]nonane-7-carboxylate hydrochloride (3.2 g,12.2 mmol). The reaction solution was stirred at rt for 18 h (LCMS control). The product was purified by column chromatography on silica with ethyl acetate-methanol (10:1) to yield the title product **P166** (5.1 g, 96%) as a white solid. LCMS (ESI) [MH]⁺: 437.

### Preparation 167. 4-(2,7-Diazaspiro[3.5]non-2-yl)-7-(2,2,2-trifluoroethyl)quinazoline (P167)

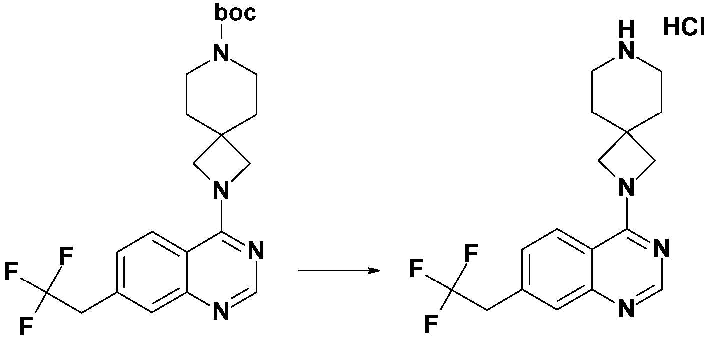

To *tert*-butyl 2-[7-(2,2,2-trifluoroethyl)quinazolin-4-yl]-2,7-diazaspiro[3.5]nonane-7-carboxylate (**P166**, 5.1 g, 11.7 mmol) solution HCl in dioxan (3M, 120 ml) was added. The reaction mixture was stirred at rt for 3 h (LCMS control), then evaporated under vacuum to dryness to yield 4-(2,7-diazaspiro[3.5]non-2-yl)-7-(2,2,2-trifluoroethyl)quinazoline hydrochloride (**P167**, 4.4 g, 100%) as a white solid. LCMS (ESI) [MH]⁺: 337.

### Synthesis of 3-[7-(2,2,2-trifluoroethyl)quinazolin-4-yl]-3,9-diazaspiro[5.5]undecane (P169)

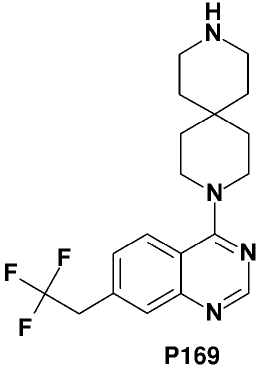

### Preparation 168. tert-Butyl 9-[7-(2,2,2-trifluoroethyl)quinazolin-4-yl]-3,9-diazaspiro[5.5]undecane-3-carboxylate (P168)

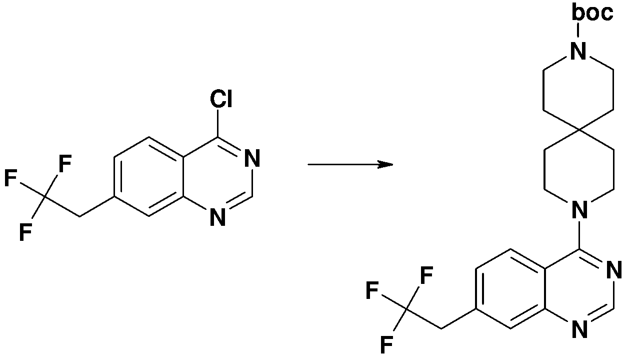

To a solution of 4-chloro-7-(2,2,2-trifluoroethyl)quinazoline (**P165**, 3 g, 12.2 mmol) in dichloroethane (60 ml) was added DIPEA (7.9 g, 61 mmol) and *tert-*butyl 3,9-diazaspiro[5.5]undecane-3-carboxylate (3.2 g,12.0 mmol). The reaction solution was stirred at rt for 18 h (LCMS control). Then product was purified by column chromatography on silica with ethyl acetate-methanol (10:1) to yield the title product **P168** (5.1 *g,* 93%) as a white solid. LCMS (ESI) [MH]⁺: 465.

### Preparation 169. 3-[7-(2,2,2-Trifluoroethyl)quinazolin-4-yl]-3,9-diazaspiro[5.5]undecane (P169)

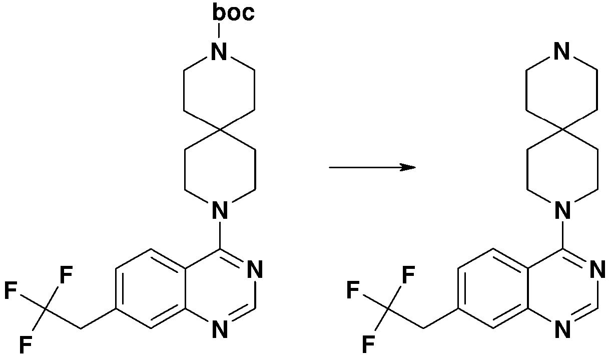

To a solution of *tert-*butyl 9-[7-(2,2,2-trifluoroethyl)quinazolin-4-yl]-3,9-diazaspiro[5.5]undecane-3-carboxylate (**P168**, 5.1 g, 11 mmol) in 50 ml of DCM TFA (60 ml) was added. The solution was stirred at rt for 2 h (LCMS control) , then evaporated to dryness, water solution of sodium bicarbonate (100 ml) was added, product was extracted with DCM (3x50 ml). Organic phase was dried with sodium sulphate, concentrated to dryness to yield the title compound **P169** (4.0 g, 100%). LCMS (ESI) [MH]⁺: 365.

### Synthesis of 4-(2,7-diazaspiro[3.5]non-2-yl)-6-[2-(3-methyl-1,2,4-oxadiazol-5-yl)ethyl]quinazoline (P173)

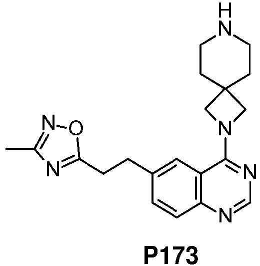

### Preparation 170. tert-Butyl 2-[6-(3-ethoxy-3-oxopropyl)quinazolin-4-yl]-2,7-diazaspiro[3.5]nonane-7-carboxylate (P170)

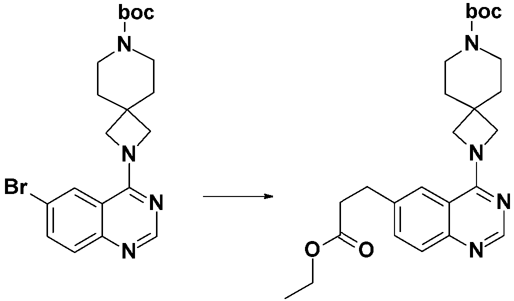

To a solution of *tert*-butyl 2-(6-bromoquinazolin-4-yl)-2,7-diazaspiro[3.5]nonane-7-carboxylate (**P146**, 200 mg 0.46 mmol) in DMAA (2 ml) under argon atmosphere TEA (186 mg, 1.84 mmol), ethyl acrylate (148 mg, 1.48 mmol) and Pd(PPh₃)₄ (20 mg) were added. The mixture was stirred at 90°C for 16 h (LCMS control). After reaction completed Pd/C (30 mg) was added and the mixture was stirred at rt under hydrogen atmosphere until reaction completed (LCMS control). Then the mixture was filtered, washed with brine (40 ml) and extracted with ethyl acetate (2x5 ml), concentrated and residue was purified by silica gel column chromatography (DCM-ethyl acetate - 1:1) to give title product **P170 (**145 mg, 69%). LCMS (ESI) [MH]⁺: 455.

### Preparation 171. 3-{4-[7-(tert-Butoxycarbonyl)-2,7-diazaspiro[3.5]non-2-yl]quinazolin-6-yl}propanoic acid (P171)

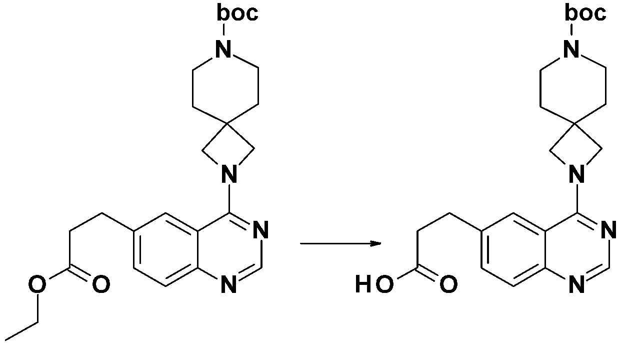

To a solution of *tert*-butyl 2-[6-(3-ethoxy-3-oxopropyl)quinazolin-4-yl]-2,7-diazaspiro[3.5]nonane-7-carboxylate (**P170**, 145 mg, 0.53 mmol) in MeOH (10 ml) solution of NaOH (64 mg, 1.6 mmol) in water (1 ml) was added. The mixture stirred at rt for 4 h (TLC control), then concentrated, dissolved in water (8 ml), HCl was added up to pH = 4, water phase was extracted with DCM (3x30 ml). Organic phase was evaporated to dryness to yield the title compound **P171** (115 mg, 84%). LCMS (ESI) [MH]⁺: 427.

### Preparation 172. tert-Butyl 2-{6-[2-(3-methyl-1,2,4-oxadiazol-5-yl)ethyl]quinazolin-4-yl}-2,7-diazaspiro[3.5]nonane-7-carboxylate (P172)

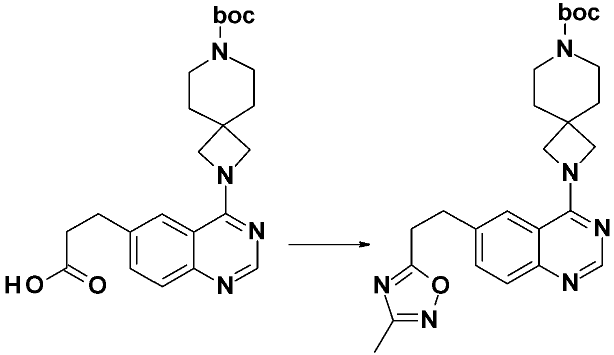

The acid prepared on the previous stage (**P171**, 115 mg, 0.27 mmol) was dissolved in DMAA (1 ml), CDI (66 mg, 0.41 mmol) was added, the mixture was stirred at 50°C for 4h, then *N*-hydroxyethanimidamide (25 mg, 0.41 mmol) was added and the mixture was stirred at 90°C for 24 h. After reaction completed (LCMS control) the reaction mixture cooled, brine (20 ml) was added, product was extracted with ethyl acetate (2x5 ml). Organic phase evaporated to dryness and residue was separated by silica gel column chromatography (dichloromethane-ethyl acetate - 1:1) to give title product **P172** (81 mg, 65%). LCMS (ESI) [MH]⁺: 465.

### Preparation 173. 4-(2,7-Diazaspiro[3.5]non-2-yl)-6-[2-(3-methyl-1,2,4-oxadiazol-5-yl)ethyl]quinazoline (P173)

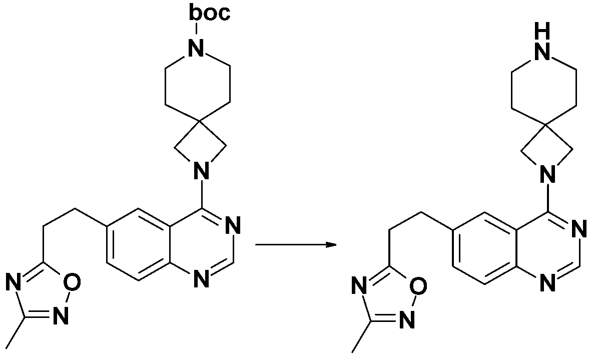

A solution of *tert*-butyl 2-{6-[2-(3-methyl-1,2,4-oxadiazol-5-yl)ethyl]quinazolin-4-yl}-2,7-diazaspiro[3.5]nonane-7-carboxylate (**P172**, 81 mg, 17.6 mmol) in TFA (1 ml) was stirred at rt for 2 h (LCMS control), then evaporated to dryness, water solution of sodium bicarbonate (10 ml) was added, product was extracted with DCM (3x5 ml). Organic phase was dried with sodium sulphate, concentrated to dryness to yield the title compound **P173** (64 mg, 100%). LCMS (ESI) [MH]⁺: 365.

### Synthesis of 5-formyl-4-methyl-1-[[(2R)-5-oxomorpholin-2-yl]methyl]indole-2-carbonitrile (P176)

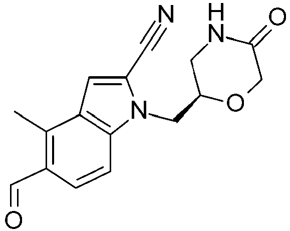

### Preparation 174. 2-Chloro-N-[(2R)-2,3-dihydroxypropyl]acetamide (P174)

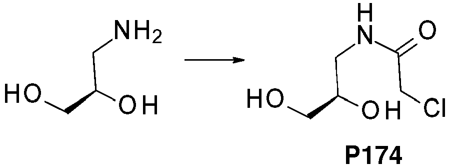

To a solution of (2*R*)-3-aminopropane-1,2-diol (10 g, 0.110 mol) in a mixture of MeCN (330 ml) and MeOH (63 ml) triethylamine (13.32 g, 0.132 mol) was added. Chloroacetyl chloride (13.77 g, 0.122 mol) was then added dropwise with stirring at temperature -20°C during 1 h under argon. The reaction mixture was allowed to reach rt and stirred overnight. The reaction mixture was concentrated to dryness, shaken with dry ethyl acetate (0.5 L), solution filtered and dried. Ethyl acetate was evaporated to give pure 2-chloro-*N*-[(2*R*)-2,3-dihydroxypropyl]acetamide **P174** (9.2 g, 50 %), as a beige oil. ¹H NMR (400 MHz, DMSO-*d₆*), δ: 8.02(s, 1H), 4.27 (s,1H), 4.48 (s, 1H), 4.06 (s, 2H), 3.53 (m, 1H), 3.31 (m, 3H), 3.03 (m, 1H).

### Preparation 175. (6R)-6-(Hydroxymethyl)morpholin-3-one (P175)

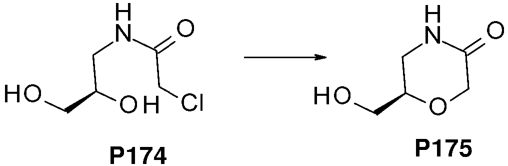

Potassium *tert*-butoxide (15.4 g, 0.138 mol) was dissolved in *tert*-BuOH (100 ml), then solution of 2-chloro-*N*-[(2*R*)-2,3-dihydroxypropyl]acetamide (**P174**, 9.2 g, 0.055 mol) in *tert*-BuOH (220 ml) was added with stirring at temperature 30°C over 1 h under argon. The reaction mixture was stirred for 1 h, then MeOH (50 ml) and water (3 ml) were added and the reaction was stirred for an additional 15 min. The crude was concentrated and purified by flash chromatography on silica gel with MeOH/EtOAc (30:70) to provide (6*R*)-6-, (hydroxymethyl)morpholin-3-one (**P175**, 3.4 g, 47 %) as a white solid. ¹H NMR (400 MHz, DMSO-*d₆*), δ: 7.93 (s, 1H), 4.85 (t, J=5.5 Hz, 1H), 4.00 (m, 2H), 3.65 (m, 1H), 3.45 (m, 2H) 3.18 (m, 1H), 3.07 (m, 1H).

### Preparation 176. 5-Formyl-4-methyl-1-{[(2R)-5-oxomorpholin-2-yl]methyl}-1H-indole-2-carbonitrile (P176)

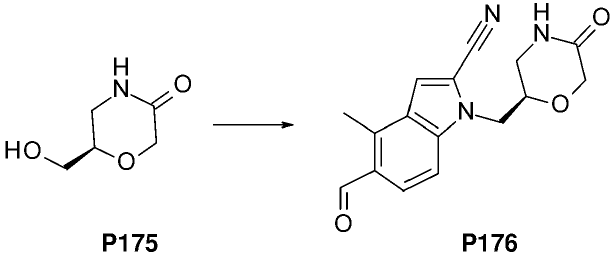

To a solution of (6*R*)-6-(hydroxymethyl)morpholin-3-one (**P175**, 1.07 g, 8.15 mmol) in DMAA (20 ml) DIPEA (1.6 g, 12.25 mmol) and MsCl (1.21 g, 10.6 mmol) were added. The mixture was stirred at 50°C for 45 min. Then 2-cyano-4-methyl-5-formylindole (**P97**, 0.5 g, 2.72 mmol) and potassium carbonate (3 g, 22 mmol) were added, and the mixture was stirred at 100°C for additional 18 h and quenched with water (100 ml). After extraction with ethyl acetate (3x100 ml) and evaporation to dryness obtained residue was washed with dry ethyl acetate (150 ml), filtered, and dried to give 5-formyl-4-methyl-1-{[(2*R*)-5-oxomorpholin-2-yl]methyl}-1*H*-indole-2-carbonitrile (**P176**, 245 mg, 30 %). ¹H NMR (400 MHz, DMSO-*d₆*), δ: 12.37 (s, 1H), 8.03 (m, 1H), 7.84 (m, 2H), 7.69 (d, J=12.2 Hz, 1H), 4.63 (m, 1H), 4.44 (m, 1H), 4.05 (m, 1H), 3.90 (m, 2H), 3.40 (m, 1H), 3.17 (m, 1H), 2.84 (s, 3H).

### Synthesis of 5-formyl-4-methyl-1-[[(2S)-5-oxomorpholin-2-yl]methyl]indole-2-carbonitrile (P177)

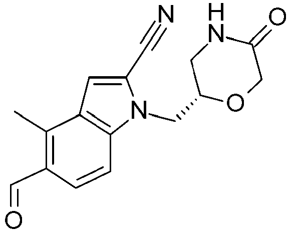

This compound **P177** was obtained using procedures described above for the synthesis of **P176** (***Preparations 174-176***), using (2*S*)-3-aminopropane-1,2-diol instead of (2*R*)-3-aminopropane-1,2-diol at the first step.

### Synthesis of 5-formyl-4-methyl-1-{1-methyl-3-[4-(methylsulfonyl)piperazin-1-yl]propyl}-1H-indole-2-carbonitrile (P180)

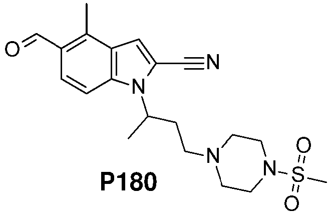

### Preparation 178. 1-(3-{[tert-Butyl(dimethyl)silyl]oxy}-1-methylpropyl)-5-formyl-4-methyl-1H-indole-2-carbonitrile (P178)

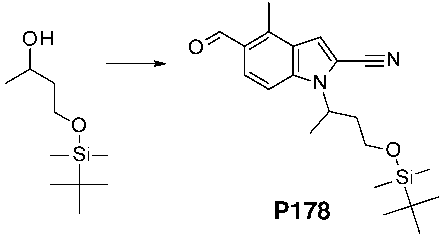

To a solution of 4-{[*tert*-butyl(dimethyl)silyl]oxy}butan-2-ol (816 mg, 4 mmol, was prepared as described in EP1466898, 2004, A1) in DMAA (20 ml) TEA (404 mg, 4 mmol) and MsCl (458 mg, 4 mmol) were added at rt. The mixture was stirred at 20°C for 45 min, then 2-cyano-4-methyl-5-formylindole (552 mg, 3 mmol) and potassium carbonate (1600 mg, 11.6 mmol) were added, and the mixture was stirred at 100°C for 48 h. The water (40 ml) was added, and the product was extracted with ethyl acetate (3x50 ml), ethyl acetate was evaporated to dryness. The residue was washed with dry ethyl acetate (10 ml), filtered, dried, to yield 1-(3-{[*tert*-butyl(dimethyl)silyl]oxy}-1-methylpropyl)-5-formyl-4-methyl-1*H*-indole-2-carbonitrile (**P178**, 480 mg, 43%). ¹H NMR (400 MHz, CDCl₃), δ: 10.38 (s, 1H), 8.17 (m, 1H), 7.50 (m, 1H), 6.55 (m, 1H), 4.70 (m, 1H), 3.34 (m, 2H), 3.0 (m, 3H), 2.48 (m, 2H), 2.29 (m, 2H), 1,33 (m, 3H), 0.82 (s, 9H), 0.05 (s, 6H). LCMS (ESI) [MH]⁺: 371.

### Preparation 179. 5-Formyl-1-(3-hydroxy-1-methylpropyl)-4-methyl-1H-indole-2-carbonitrile (P179)

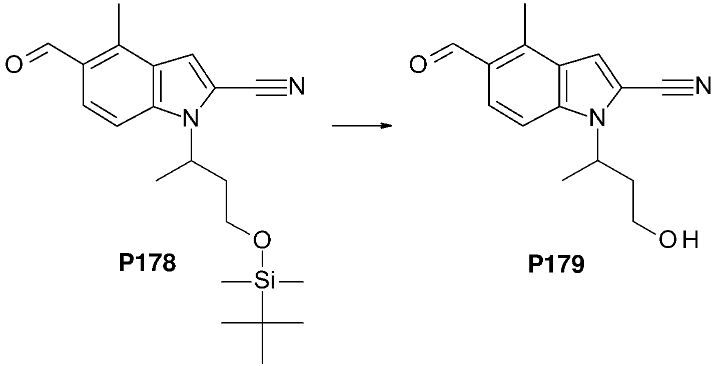

To a solution of 1-(3-{[*tert*-butyl(dimethyl)silyl]oxy}-1-methylpropyl)-5-formyl-4-methyl-1*H*-indole-2-carbonitrile (**P178**, 480 mg, 1.29 mmol) in MeOH (30 ml) was added HCl (0.5 ml). The mixture was stirred at 20°C for 45 min (TLC control), then aqueous solution of sodium bicarbonate and DCM were added, and the organic layer was separated, washed with brine, dried over sodium sulfate, filtered and the filtrate was evaporated. The residue after evaporation to dryness gave **P179** (307 mg, 93%). ¹H NMR (400 MHz, DMSO-*d₆*), δ: 10.41 (s, 1H), 7.91 (m, 2H), 7.68 (m, 1H), 4.70 (m, 1H), 3.34 (m, 2H), 3.0 (m, 3H), 2.48 (m, 2H), 2.29 (m, 2H), 1,33 (m, 3H). LCMS (ESI) [MH]⁺: 257.

### Preparation 180. 5-Formyl-4-methyl-1-{1-methyl-3-[4-(methylsulfonyl)piperazin-1-yl]propyl}-1H-indole-2-carbonitrile (P180)

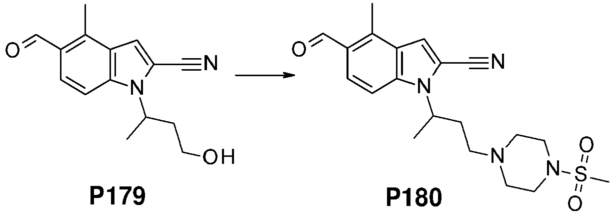

To a solution of 5-formyl-1-(3-hydroxy-1-methylpropyl)-4-methyl-1H-indole-2-carbonitrile (**P179**, 307 mg, 1.2 mmol) in MeCN (15 ml) DIPEA (155 mg, 1.2 mmol) and MsCl (137 mg, 1.2 mmol) were added. The mixture was stirred at 20°C for 45 min (TLC control), then 1-(methylsulfonyl)piperazine (394, 2.4 mmol) and K₂CO₃ (1.38 g, 10 mmol) were added. The mixture was stirred at 80°C for 18 h, cooled, filtered. The residue after evaporation was subjected to HPLC purification to afford the target compound 5-formyl-4-methyl-1-{ 1-methyl-3-[4-(methylsulfonyl)piperazin-1-yl]propyl}-1H-indole-2-carbonitrile (**P180**, 96 mg, 19%). LCMS (ESI) [MH]⁺: 425.

### Synthesis of 5-formyl-4-methyl-1-[(4-methyl-5-oxomorpholin-2-yl)methyl]-1H-indole-2-carbonitrile (P183)

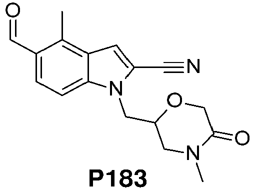

### Preparation 181. 2-Chloro-N-(2,3-dihydroxypropyl)-N-methylacetamide (P181)

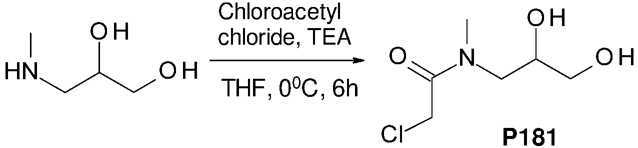

To a solution of 3-(methylamino)propane-1,2-diol (2.0g, 19 mmol) and TEA (1.2 eq) in ACN/MeOH (84/16 ml), chloroacetyl chloride (1.1eq) was added dropwise at 0°C. After stirring at 25°C for 6 h, the solvent was removed under reduced pressure. The residue was purified by column chromatography (EtOAc/MeOH, 80/20 v/v) to afford product **P181** as a clear oil (2.5 g, 72%). LCMS (ESI) [MH]⁺: 183.

### Preparation 182. 5-(Hydroxymethyl)-1-methylpiperidin-2-one (P182)

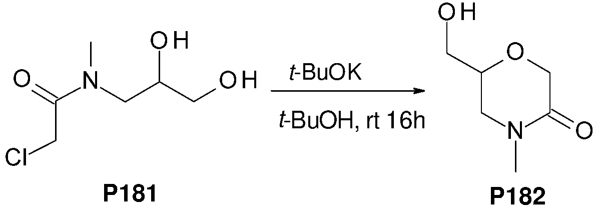

To a stirred solution of potassium *tert*-butoxide (2.5 eq) in 75 ml of *tert*-butyl alcohol at rt was added 2-chloro-N-(2,3-dihydroxypropyl)-N-methylacetamide (**P181**, 2.5 g, 13.8 mmol) in 5 ml of *tert*-butyl alcohol over 2 h under nitrogen. After one more hour, MeOH (50 ml) and H₂O (3 ml) were added, and the reaction mixture was stirred for an additional 20 min. The crude was concentrated under vacuum and purified by flash chromatography on silica gel with MeOH/EtOAc (20:80) to provide **P182** as a clear oil (1.0 g, 50%).

### Preparation 183. 5-Formyl-4-methyl-1-[(4-methyl-5-oxomorpholin-2-yl)methyl]-1H-indole-2-carbonitrile (P183)

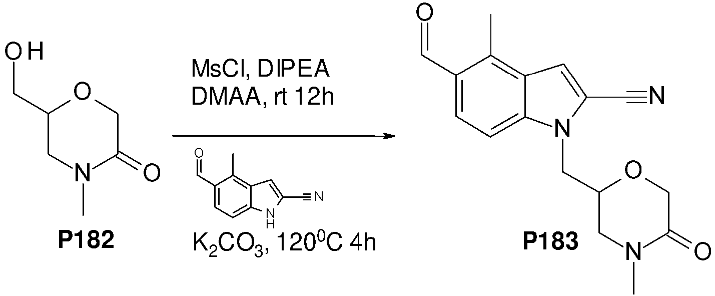

To a solution of 5-(hydroxymethyl)-1-methylpiperidin-2-one (**P182**, 79 mg, 0.54 mmol) and DIPEA (1.2 eq) in DMAA (5 ml) at rt MsCl (1.1 eq) was added. The reaction mixture was stirred at rt for 12 h Then K₂CO₃ (4 eq) and 5-formyl-4-methyl-1H-indole-2-carbonitrile (1 eq) were added and the mixture was heated at 140°C for 4 h. The reaction mixture was diluted with water and extracted with EtOAc. The residue after evaporation was subjected to column chromatography on silica gel eluting with DCM/MeOH (0 - 20%) to afford product **P183** (38 mg, 22%) as a yellow solid. ¹H NMR (400 MHz, DMSO-*d₆*), δ: 10.37 (s, 1H), 7.83 (d, J = 6.6 Hz, 2H), 7.68 (d, J = 8.8 Hz, 1H), 4.63 (d, J = 15.1 Hz, 1H), 4.55 - 4.37 (m, 1H), 4.18 (s, 1H), 4.12 - 3.82 (m, 3H), 3.32 (s, 3H). LCMS (ESI) [MH]⁺: 312.

### Synthesis of 5-formyl-4-methyl-1-[(4-methyl-3,3-dioxido-1,3,4-oxathiazinan-6-yl)methyl]-1H-indole-2-carbonitrile (P187)

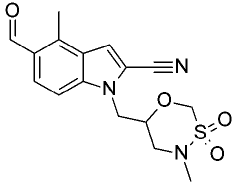

### Preparation 184. N-(3-{[tert-Butyl(dimethyl)silyl]oxy}-2-hydroxypropyl)-1-chloro-N-methylmethanesulfonamide (P184)

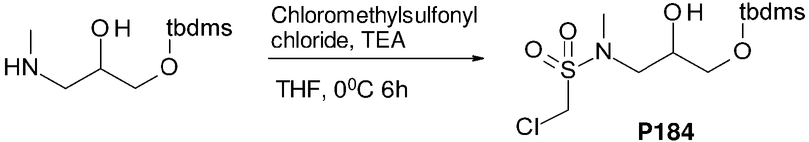

To a solution of 1-{ [*tert*-butyl(dimethyl)silyl]oxy}-3-(methylamino)propan-2-ol (1.0 g, 4.6 mmol) and TEA (1.2 eq) in THF (30 ml), Chloromethylsulfonyl chloride (1.1 eq) was added dropwise at 0°C. After stirring at 25 °C for 6 h, the solvent was removed under reduced pressure. The residue was purified by column chromatography (EtOAc/MeOH, 80/20 v/v) to afford product **P184** as clear oil (0.9 g, 59%). LCMS (ESI) [MH]⁺: 333.

### Preparation 185. 6-({[tert-Butyl(dimethyl)silyl]oxy}methyl)-4-methyl-1,3,4-oxathiazinane 3,3-dioxide (P185)

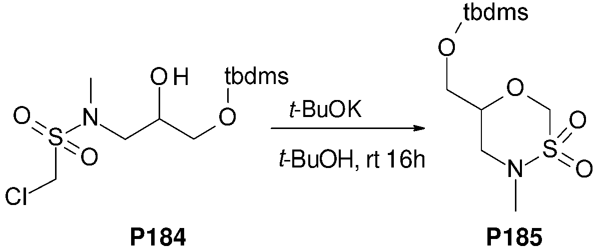

To a stirred solution of potassium *tert*-butoxide (2.5 eq) in 30 ml *tert*-butyl alcohol at rt was added *N*-(3-{[tert-butyl(dimethyl)silyl]oxy}-2-hydroxypropyl)-1-chloro-N-methylmethanesulfonamide (**P184**, 0.5 g, 1.5 mmol) in 30 ml *tert*-butyl alcohol over 2 h under nitrogen. After one more hour, MeOH (50 ml) and H₂O (3 ml) were added, and the reaction mixture was stirred for an additional 20 min. The crude was concentrated under vacuum and purified by flash chromatography on silica gel with MeOH/EtOAc (20:80) to provide **P185** as clear oil (0.29 g, 65%). ¹H NMR (400 MHz, CDCl₃), δ: 4.62 (s, 2H), 4.02 - 3.89 (m, 1H), 3.77 (dd, J = 10.6, 5.1 Hz, 1H), 3.62 - 3.54 (m, 2H), 2.84 (s, 3H), 0.89 (s, 9H), 0.07 (s, 6H). LCMS (ESI) [MH]⁺: 297.

### Preparation 186. (4-Methyl-3,3-dioxido-1,3,4-oxathiazinan-6-yl)methanol (P186)

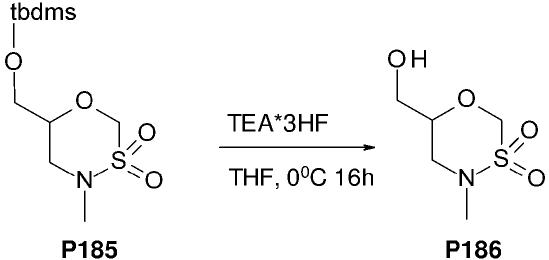

To a solution of 6-({[tert-butyl(dimethyl)silyl]oxy}methyl)-4-methyl-1,3,4-oxathiazinane 3,3-dioxide (**P185**, 0.29 g, 0.98 mmol) in 20 ml of anh. THF triethylamine trihydrofluoride (7 eq) at 0°C was added under N₂ atmosphere. The solution was stirred at rt for 12 h and all volatiles were removed using a rotary evaporator. The residue was dissolved in EtOAc and washed with cold saturated NaHCO₃ solution. The filtrate was purified by column chromatography (DCM/MeOH=10:1 v/v) to give **P186** (150 mg, 84%).

### Preparation 187. 5-Formyl-4-methyl-1-[(4-methyl-3,3-dioxido-1,3,4-oxathiazinan-6-yl)methyl]-1H-indole-2-carbonitrile (P187)

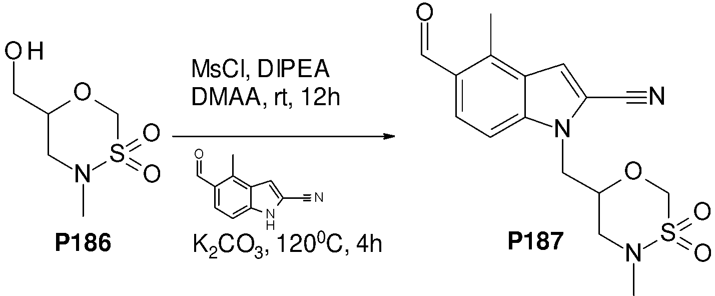

To a solution of (4-methyl-3,3-dioxido-1,3,4-oxathiazinan-6-yl)methanol (**P186**, 98 mg, 0.54 mmol) and DIPEA (1.2 eq) in DMAA (5ml) at rt MsCl (1.1 eq) was added. The reaction mixture was stirred at rt for 12 h. Then K₂CO₃ (4 eq) and 5-formyl-4-methyl-1H-indole-2-carbonitrile (1 eq) were added and the mixture was heated to 140°C for 4 h. The reaction mixture was diluted with water and extracted with EtOAc. The residue after evaporation was subjected to column chromatography on silica gel eluting with DCM/MeOH (0 - 10%) to afford product **P187** (60 mg, 32%) as a yellow solid. LCMS (ESI) [MH]⁺: 348.

### Synthesis of 1-{[4-(4-methoxybenzyl)-3,3-dioxido-1,3,4-oxathiazinan-6-yl]methyl}-4-methyl-5-({2-[6-(2,2,2-trifluoroethyl)quinazolin-4-yl]-2,7-diazaspiro[3.5]non-7-yl}methyl)-1H-indole-2-carbonitrile (P192)

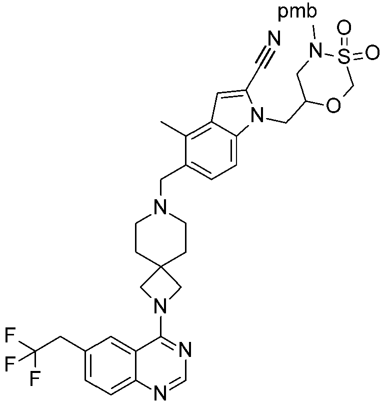

### Preparation 188. N-[3-(Benzyloxy)-2-hydroxypropyl]-1-chloro-N-(4-methoxybenzyl)methanesulfonamide (P188)

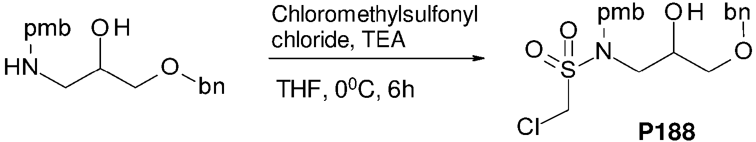

To a solution of 1-(benzyloxy)-3-[(4-methoxybenzyl)amino]propan-2-ol (0.5 g, 1.66 mmol) and TEA (1.2 eq) in THF (10 ml), Chloromethylsulfonyl chloride (1.1 eq) was added dropwise at 0°C. After stirring at 25°C for 6 h, the solvent was removed under reduced pressure. The residue was purified by column chromatography (EtOAc/MeOH, 80/20 v/v) to afford product **P188** as clear oil (0.52 g, 76%). ¹H NMR (400 MHz, CDCl₃), δ: 7.45 - 7.20 (m, 7H), 6.89 (t, J = 9.1 Hz, 2H), 4.80 - 4.68 (m, 2H), 4.64 - 4.47 (m, 3H), 4.46 - 4.36 (m, 1H), 4.00 (s, 1H), 3.81 (s, 3H), 3.59 (ddd, J = 19.0, 13.3, 7.4 Hz, 1H), 3.56 - 3.14 (m, 4H). LCMS (ESI) [MH]⁺: 415.

### Preparation 189. 6-[(Benzyloxy)methyl]-4-(4-methoxybenzyl)-1,3,4-oxathiazinane 3,3-dioxide (P189)

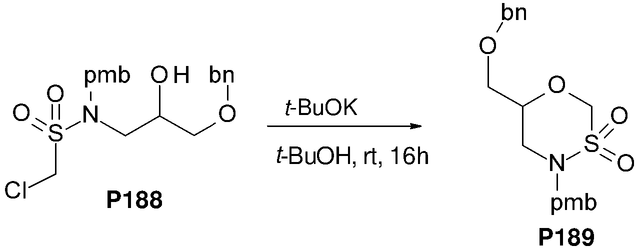

To a stirred solution of potassium *tert*-butoxide (2.5 eq) in 30 ml *tert-*butyl alcohol at rt *N-*[3-(benzyloxy)-2-hydroxypropyl]-1-chloro-*N*-(4-methoxybenzyl)methanesulfonamide (**P188**, 0.5 g, 1.2 mmol) in 30 ml of *tert*-butyl alcohol was added over 2 h under nitrogen. After one more hour, MeOH (50 ml) and H₂O (3 ml) were added, and the reaction mixture was stirred for an additional 20 min. The crude was concentrated under vacuum and purified by flash chromatography on silica gel with MeOH/EtOAc (20:80) to provide **P189** as clear oil (0.3 g, 66%). ¹H NMR (400 MHz, CDCl₃), δ: 7.31 (ddd, J = 22.0, 15.8, 7.4 Hz, 7H), 6.96 - 6.82 (m, 2H), 4.77 - 4.60 (m, 2H), 4.52 (d, J = 12.4 Hz, 2H), 4.13 (p, J = 7.2 Hz, 1H), 4.04 (d, J = 14.0 Hz, 1H), 4.00 - 3.86 (m, 1H), 3.82 (d, J = 4.9 Hz, 3H), 3.63 - 3.29 (m, 3H), 3.12 (dd, J = 13.7, 2.1 Hz, 1H). LCMS (ESI) [MH]⁺: 379.

### Preparation 190. [4-(4-Methoxybenzyl)-3,3-dioxido-1,3,4-oxathiazinan-6-yl]methanol (P190)

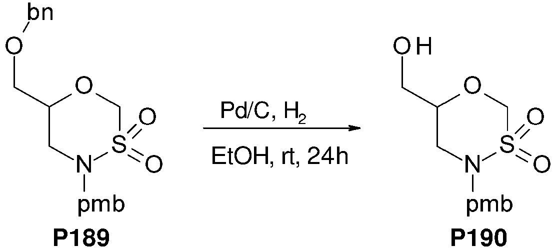

To a EtOH solution of ethyl 6-[(benzyloxy)methyl]-4-(4-methoxybenzyl)-1,3,4-oxathiazinane 3,3-dioxide (**P189**, 150 mg, 0.4 mmol) Pd/C (10%, 150 mg) was added. The mixture was stirred at rt under H₂ atmosphere for 24 h. Pd/C was filtered off, filtrate was concentrated under vacuum and purified by column chromatography to give product **P190** as clear oil (60 mg, 53%). ¹H NMR (400 MHz, DMSO-*d₆*), δ: 7.26 (d, J = 8.6 Hz, 2H), 6.93 (d, J = 8.6 Hz, 2H), 4.93 - 4.81 (m, 3H), 4.33 (d, J = 14.3 Hz, 1H), 4.15 (t, J = 12.1 Hz, 1H), 3.98 - 3.85 (m, 1H), 3.75 (s, 3H), 3.43 (dt, J = 11.2, 5.4 Hz, 1H), 3.36 - 3.29 (m, 3H), 3.13 (dd, J = 14.0, 1.9 Hz, 1H). LCMS (ESI) [MH]⁺: 288.

### Preparation 191. 5-Formyl-1-{[4-(4-methoxybenzyl)-3,3-dioxido-1,3,4-oxathiazinan-6-yl]methyl}-4-methyl-1H-indole-2-carbonitrile (P191)

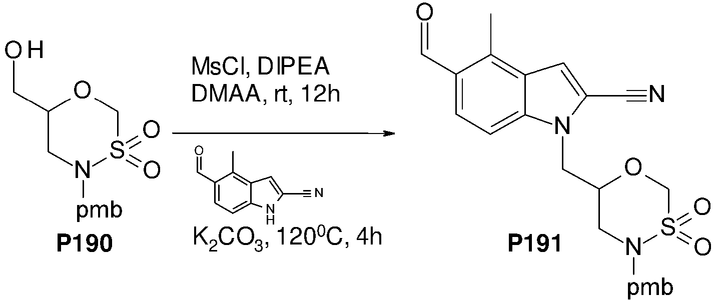

To a solution of [4-(4-methoxybenzyl)-3,3-dioxido-1,3,4-oxathiazinan-6-yl]methanol (**P190**, 50 mg, 0.17 mmol) and DIPEA (1.2 eq) in DMAA (5 ml) at rt MsCl (1.1eq) was added. The reaction mixture was stirred at rt for 12 h. Then K₂CO₃ (4 eq) and 5-formyl-4-methyl-1H-indole-2-carbonitrile (1 eq) were added and the mixture was heated to 140°C for 4 h. The reaction mixture was diluted with water and extracted with EtOAc. The residue after evaporation was subjected to column chromatography on silica gel eluting with dichloromethane/EtOAc (0 - 20%) to afford product **P191** (48 mg, 61%) as a yellow solid. ¹H NMR (400 MHz, DMSO-*d₆*), δ: 10.37 (s, 1H), 7.85 (d, J = 7.1 Hz, 2H), 7.65 (d, J = 8.8 Hz, 1H), 7.26 (d, J = 8.5 Hz, 2H), 6.93 (d, J = 8.5 Hz, 2H), 4.80 (dd, J = 27.0, 11.9 Hz, 2H), 4.55 (d, J = 15.3 Hz, 1H), 4.46 - 4.17 (m, 4H), 3.75 (d, J = 6.2 Hz, 3H), 3.56 - 3.38 (m, 2H), 2.85 (s, 3H). LCMS (ESI) [MH]⁺: 455.

### Preparation 192. 1-{[4-(4-Methoxybenzyl)-3,3-dioxido-1,3,4-oxathiazinan-6-yl]methyl}-4-methyl-5-({2-[6-(2,2,2-trifluoroethyl)quinazolin-4-yl]-2,7-diazaspiro[3.5]non-7-yl}methyl)-1H-indole-2-carbonitrile (P192)

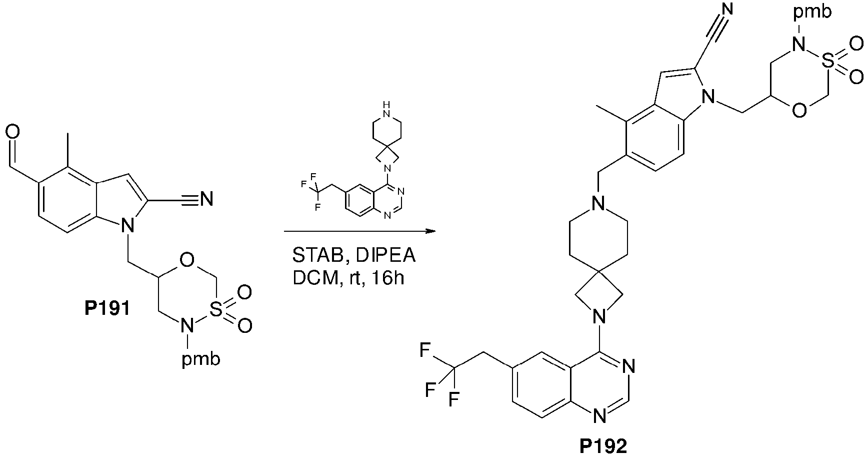

A mixture of 5-formyl-1-{[4-(4-methoxybenzyl)-3,3-dioxido-1,3,4-oxathiazinan-6-yl]methyl}-4-methyl-1H-indole-2-carbonitrile (**P191**, 43 mg, 0.1 mmol), triacetoxyborohydride (3 eq) and 4-(2,7-diazaspiro[3.5]non-2-yl)-6-(2,2,2-trifluoroethyl)quinazoline (1.2 eq) in DCM (2 ml) was stirred at rt (LCMS control), then water solution of sodium bicarbonate was added with stirring. The mixture was stirred 30 min, water phase was extracted with DCM (30 ml), DCM was concentrated, and residue was purified by HPLC to give yellow solid of **P192** (23 mg, 31%). LCMS (ESI) [MH]⁺: 775.

### Synthesis of 5-formyl-4-methyl-1-[(2-oxopiperidin-4-yl)methyl]-1H-indole-2-carbonitrile (P196)

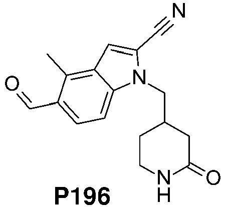

### Preparation 193. 2-Oxopiperidine-4-carboxylic acid (P193)

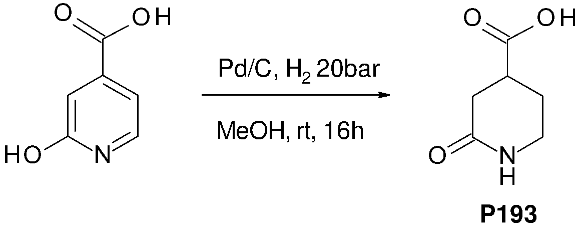

A dispersion of 2-hydroxyisonicotinic acid (2.8 g, 20 mmol) in 50 ml of MeOH was placed in autoclave with Pd/C (10%, 1 g). The reaction mixture was stirred at rt under 20 bars of H₂ for 16 h. After reaction completed solids were filtered off, filtrate was concentrated under vacuum to give product **P193** as white solid (1.63 g, 63%). ¹H NMR (500 MHz, DMSO-*d₆*), δ: 12.37 (br. s, 1H), 7.47 (s, 1H), 3.24-3.12 (m, 2H), 2.81-2.72 (m, 1H), 2.34-2.22 (m, 2H), 2.03-1.91 (m, 1H), 1.76-1.58 (m, 1H).

### Preparation 194. Methyl 2-oxopiperidine-4-carboxylate (P194)

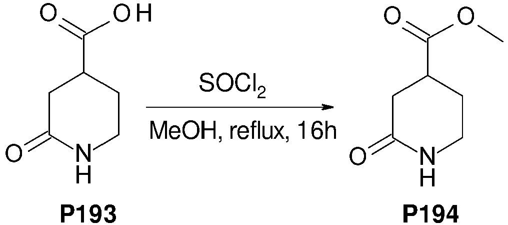

To a solution of 2-oxopiperidine-4-carboxylic acid (**P193**, 1.6 g, 11 mmol) in 20ml of MeOH SOCl₂ (1.2 eq) was added dropwise. The reaction mixture was refluxed for 16 h. The residue after evaporation was subjected to column chromatography eluting with DCM/EtOAc (0 - 10%) to afford product **P194** (1.4 g, 80%) as an off-white solid.¹H NMR (400 MHz, CDCl₃), δ: 3.68 (s, 3H), 3.28 (m, 2H), 2.79 (m, 1H), 2.53 (m, 2H), 2.45 - 2.24 (m, 1H), 2.13 - 2.02 (m, 1H), 1.89 - 1.76 (m, 1H).

### Preparation 195. Methyl 2-oxopiperidine-4-carboxylate (P195)

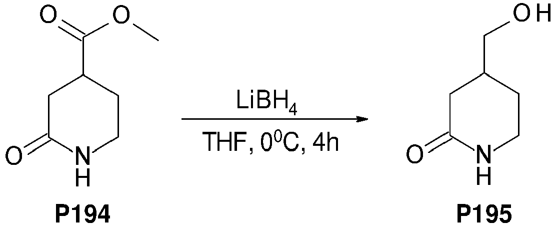

To a solution of methyl 2-oxopiperidine-4-carboxylate (**P194**, 0.5 g, 2.9 mmol) and dry MeOH (3 eq) in 20 ml anh. THF LiBH₄ (3 eq) was added in portions at 0°C. The reaction mixture was stirred at 0°C for 4 h. The mixture was quenched by addition of NaOH (1 M) and then allowed to warm to rt. EtOAc was added and the separated aqueous phase was extracted with EtOAc. The residue after evaporation of combined organic phases was subjected to column chromatography eluting with DCM/MeOH (0 - 10%) to afford **P195** (0.31 g, 82%) as a clear oil. ¹H NMR (400 MHz, DMSO-*d₆*), δ: 4.45 (s, 1H), 3.64 (t, J = 6.7 Hz, 2H), 3.44 (d, J = 18.4 Hz, 2H), 1.76 (dd, J = 14.3, 7.1 Hz, 2H), 1.52 (dt, J = 13.4, 6.6 Hz, 2H).

### Preparation 196. 5-Formyl-4-methyl-1-[(2-oxopiperidin-4-yl)methyl]-1H-indole-2-carbonitrile (P196)

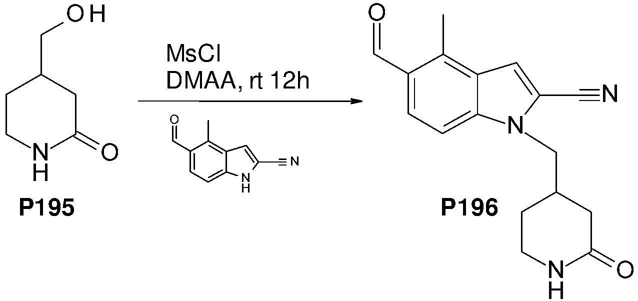

To a solution of 4-(hydroxymethyl)piperidin-2-one (**P195**, 50 mg, 0.4 mmol) and DIPEA (1.2 eq) in DMAA (5 ml) at rt MsCl (1.1 eq) was added. The reaction mixture was stirred at rt for 16 h. Then K₂CO₃ (4 eq) and 5-formyl-4-methyl-1H-indole-2-carbonitrile (1 eq) were added and mixture was heated to 140°C for 4h. The reaction mixture was diluted with water and extracted with EtOAc. The residue after evaporation was subjected to column chromatography on silica gel eluting with DCM/MeOH (0 - 20%) to afford **P196** (28 mg, 24%) as a yellow solid. LCMS (ESI) [MH]⁺: 296.

### Synthesis of 5-formyl-4-methyl-1-[(6-oxopiperidin-3-yl)methyl]-1H-indole-2-carbonitrile (P200)

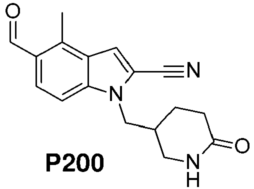

### Preparation 197. 6-Oxopiperidine-3-carboxylic acid (P197)

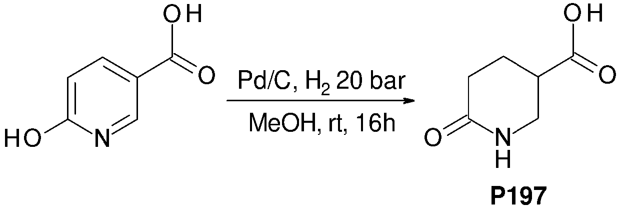

A dispersion of 6-hydroxynicotinic acid (2.0 g, 14 mmol) in 50 ml of MeOH was placed in autoclave with Pd/C (10%, 1 g). The reaction mixture was stirred at rt under 20 bars of H₂ for 16 h. Solids was filtered off, filtrate was concentrated under vacuum to give **P197** as a white solid (1.3 g, 63%). ¹H-NMR (DMSO-*d₆*), δ: 1.75-1.88 (m, 1H), 1.91-2.01 (m, 1H), 2.11-2.24 (m, 2H), 2.66-2.73 (m, 1H), 3.21-3.32 (m, 2H), 7.45 (s, 1H), 12.51 (br. s, 1H).

### Preparation 198. Methyl 6-oxopiperidine-3-carboxylate (P198)

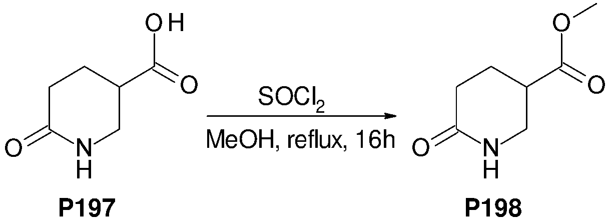

To a solution of 6-oxopiperidine-3-carboxylic acid (**P197**, 1.3 g, 9 mmol) in 20 ml of MeOH SOCl₂ (1.2 eq) was added dropwise. The reaction mixture was refluxed for 16 h. The residue after evaporation was subjected to column chromatography eluting with DCM/EtOAc (0 - 10%) to afford **P198** (1.2 g, 84%) as an off-white solid.

### Preparation 199. 5-(Hydroxymethyl)piperidin-2-one (P199)

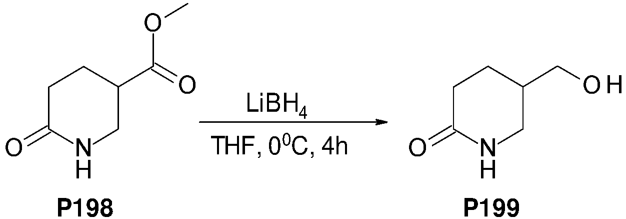

To a solution of methyl 6-oxopiperidine-3-carboxylate (**P198**, 0.5 g, 2.9 mmol) and dry MeOH (3 eq) in 20 ml THF LiBH₄ (3 eq) was added in portions at 0°C. The reaction mixture was stirred at 0°C for 4 h. The mixture was quenched by addition of NaOH (1 M) and then allowed to warm to rt. EtOAc was added and the separated aqueous phase was extracted with EtOAc. The residue after evaporation was subjected to column chromatography eluting with DCM/MeOH (0 - 10%) to afford **P199** (0.3 g, 80%) as a clear oil. ¹H NMR (400 MHz, DMSO-*d₆*), δ: 7.40 (s, 1H), 4.75 (s, 1H), 3.43 (s, 2H), 3.37 - 3.25 (m, 2H), 3.22 - 3.16 (m, 1H), 2.15 - 2.07 (m, 2H), 1.83 - 1.68 (m, 2H).

### Preparation 200. 5-Formyl-4-methyl-1-[(6-oxopiperidin-3-yl)methyl]-1H-indole-2-carbonitrile (P200)

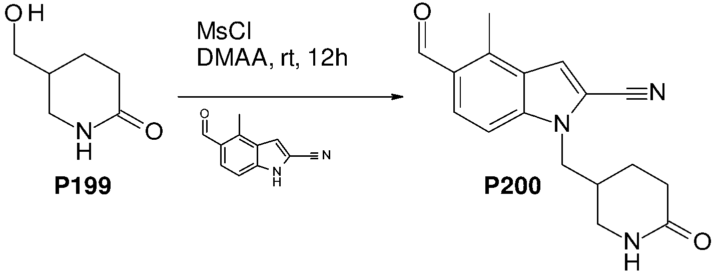

To a solution of 5-(hydroxymethyl)piperidin-2-one (**P199**, 100 mg, 0.8 mmol) and DIPEA (1.2 eq) in DMAA (5 ml) at rt MsCl (1.1 eq) was added. The reaction mixture was stirred at rt for 16 h. Then K₂CO₃ (4 eq) and 5-formyl-4-methyl-1H-indole-2-carbonitrile (**P97**, 1 eq) were added and the mixture was heated to 140°C for 4 h. The reaction mixture was diluted with water and extracted with EtOAc. The residue after evaporation of the organic phase was subjected to column chromatography on silica gel eluting with DCM/MeOH (0 - 20%) to afford **P200** (50 mg, 22%) as a yellow solid. ¹H NMR (400 MHz, DMSO-*d₆*), δ: 10.37 (s, 1H), 7.93 - 7.81 (m, 2H), 7.71 (d, J = 8.9 Hz, 1H), 7.41 (s, 1H), 4.50 - 4.28 (m, 2H), 2.97 (d, J = 20.8 Hz, 2H), 2.87 (d, J = 12.8 Hz, 3H), 2.32 - 2.05 (m, 3H), 1.74 - 1.45 (m, 2H). LCMS (ESI) [MH]⁺: 296.

### Synthesis of 1-[(3-aminobicyclo[1.1.1]pent-1-yl)methyl]-4-methyl-5-({2-[6-(2,2,2-trifluoroethyl)quinazolin-4-yl]-2,7-diazaspiro[3.5]non-7-yl}methyl)-1H-indole-2-carbonitrile (P205)

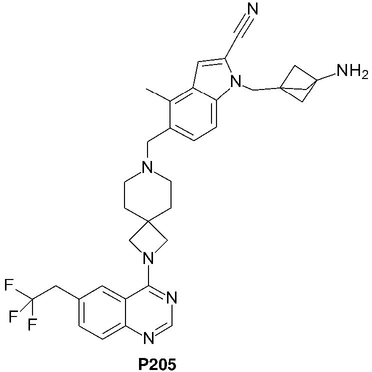

### Preparation 201. Methyl 3-[(tert-butoxycarbonyl)amino]bicyclo[1.1.1]pentane-1-carboxylate (P201)

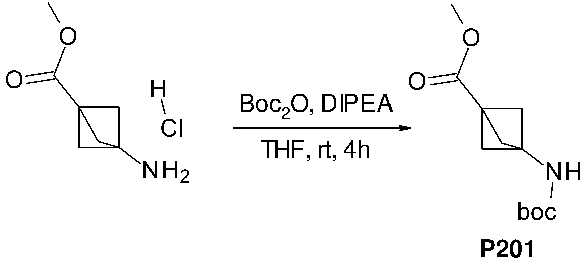

Methyl 3-aminobicyclo[1.1.1]pentane-l-carboxylate hydrochloride 27 (0.8 g, 4.5 mmol), Boc-anhydride (1.1 eq) and DIPEA (5 eq) were combined in THF (20 ml). The reaction mixture was stirred at rt for 4 h, concentrated under vacuo, redissolved in EtOAc, and washed with an aqueous solution of sat. NaHCO₃, then with a 3% citric acid solution. The organic phase was concentrated, and the residue was purified by column chromatography (Hexane/EtOAc 10%) to afford **P201** (1.07 g, 98%) as a white solid. ¹H NMR (400 MHz, CDCl₃), δ: 3.69 (s, 1H), 2.29 (s, 2H), 1.46 (s, 3H). LCMS (ESI) [MH]⁺: 242.

### Preparation 202. tert-Butyl [3-(hydroxymethyl)bicyclo[1.1.1]pent-1-yl]carbamate (P202)

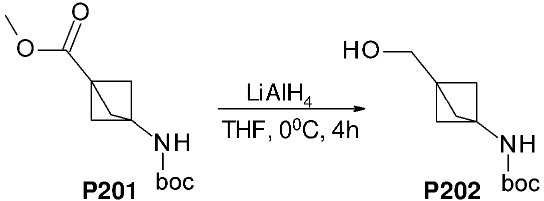

To a solution of methyl 3-[(*tert*-butoxycarbonyl)amino]bicyclo[1.1.1]pentane-1-carboxylate (**P201**, 500 mg, 2 mmol) in THF (20 ml) was added LiAlH₄ (1.5 eq) in small portions at 0°C. The reaction was stirred at 0°C for 4 h. The reaction mixture was quenched by addition of wet EtOAc and partitioned between EtOAc and H₂0. The organic phase was concentrated, and the residue was purified by column chromatography (DCM/MeOH 10%) to afford **P202** (380 mg, 86%) as a white solid. LCMS (ESI) [MH]⁺: 214.

### Preparation 203. tert-Butyl N-[3-[(2-cyano-5-formyl-4-methyl-indol-1-yl)methyl]-1-bicyclo[1.1.1]pentanyl]carbamate (P203)

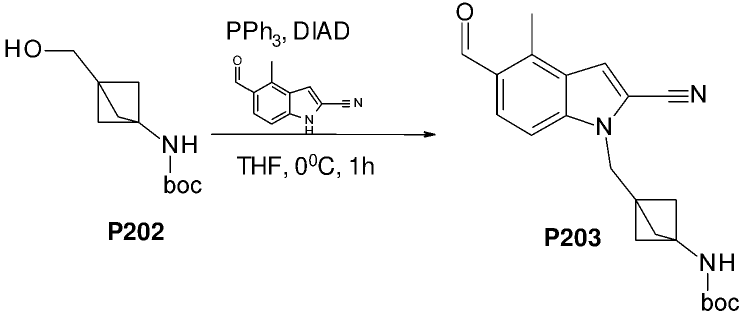

To a solution of *tert-*butyl [3-(hydroxymethyl)bicyclo[1.1.1]pent-1-yl]carbamate (**P202**, 350 mg, 1.6 mmol), 6-formyl-4-methyl-1H-indole-2-carbonitrile (1.1 eq) and triphenyl phosphine (1.3 eq) in THF (5 ml) DIAD (1.3 eq) was added dropwise at 0°C, and the resulting mixture was stirred at 0°C for 1 h. The solvent was evaporated. The residue after evaporation was subjected to column chromatography DCM/ethyl acetate (0 - 20%) to afford **P203** (185 mg, 30%) as a yellow solid. ¹H NMR (400 MHz, DMSO-*d₆*), δ: 10.36 (s, 1H), 7.90 - 7.78 (m, 2H), 7.61 (d, J = 8.8 Hz, 1H), 7.43 (s, 1H), 4.55 (s, 2H), 2.86 (s, 3H), 2.13 (s, 1H), 1.73 (s, 6H), 1.32 (s, 9H). LCMS (ESI) [MH]⁺: 380.

### Preparation 204. tert-Butyl (3-{[2-cyano-4-methyl-5-({2-[6-(2,2,2-trifluoroethyl)quinazolin-4-yl]-2,7-diazaspiro[3.5]non-7-yl}methyl)-1H-indol-1-yl]methyl}bicyclo[1.1.1]pent-1-yl)carbamate (P204)

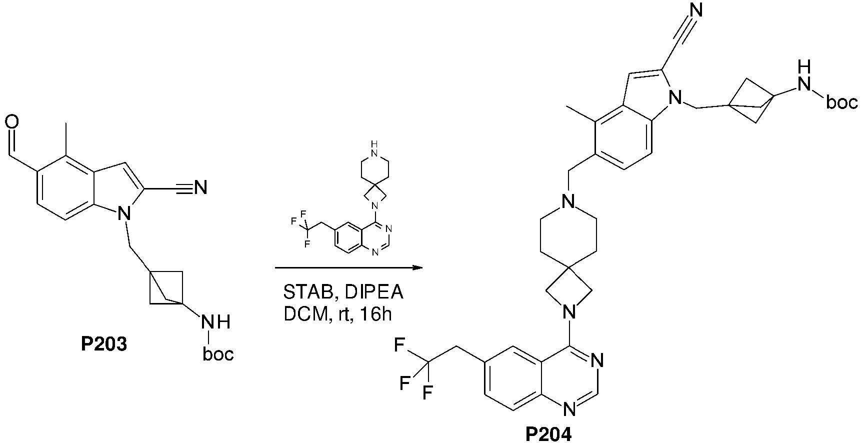

A mixture of *tert**-***butyl *N*-[3-[(2-cyano-5-formyl-4-methyl-indol-1-yl)methyl]-1-bicyclo[1.1.1]pentanyl]carbamate (**P203**, 90 mg, 0.24 mmol), triacetoxyborohydride (3 eq) and 4-(2,7-diazaspiro[3.5]non-2-yl)-6-(2,2,2-trifluoroethyl)quinazoline (1.2 eq) in DCM (5 ml) stirred at rt (LCMS control), then with stirring water solution of sodium bicarbonate was added. The mixture was stirred for 30 min, organic layer was separated, water phase was extracted with DCM (30 ml), DCM was concentrated, and residue was purified by HPLC to give **P204** (87 mg, 52%). LCMS (ESI) [MH]⁺: 701.

### Preparation 205. 1-[(3-Aminobicyclo[1.1.1]pent-1-yl)methyl]-4-methyl-5-({2-[6-(2,2,2-trifluoroethyl)quinazolin-4-yl]-2,7-diazaspiro[3.5]non-7-yl}methyl)-1H-indole-2-carbonitrile (P205)

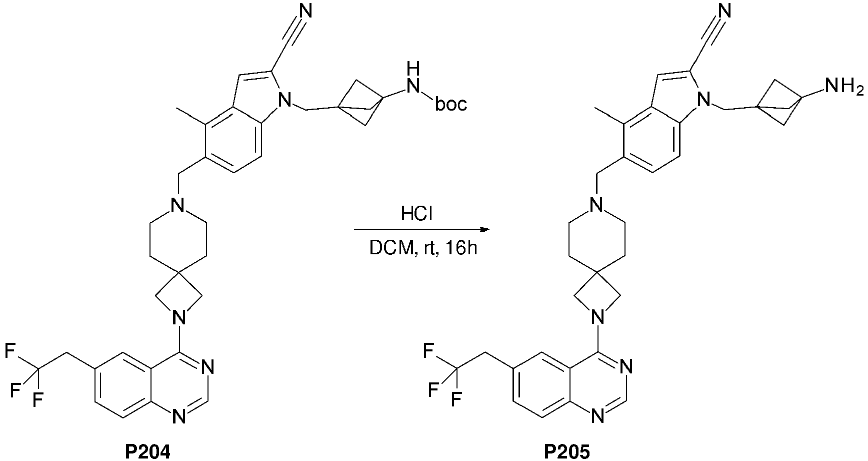

To a solution of *tert*-butyl (3-{[2-cyano-4-methyl-5-({2-[6-(2,2,2-trifluoroethyl)quinazolin-4-yl]-2,7-diazaspiro[3.5]non-7-yl}methyl)-1H-indol-1-yl]methyl}bicyclo[1.1.1]pent-1-yl)carbamate (**P205**, 200 mg, 0.03 mmol) in 20 ml of dry THF, HCl (3M in dioxane, 10 eq) was added. The reaction mixture was stirred at rt for 16 h. Reaction was quenched with sat. NaHCO₃ and extracted with EtOAc. The organic phase was concentrated, and the residue was purified by column chromatography (EtOAc/MeOH 20%) to afford product **P205** (70 mg, 94%) as a yellow solid. LCMS (ESI) [MH]⁺: 601.

### Synthesis of 5-formyl-4-methyl-1-[(5-oxo-1,4-oxazepan-2-yl)methyl]-1H-indole-2-carbonitrile (P210)

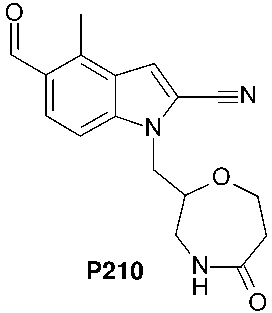

### Preparation 206. 1-{[tert-Butyl(dimethyl)silyl]oxy}-3-(dibenzylamino)propan-2-ol (P206)

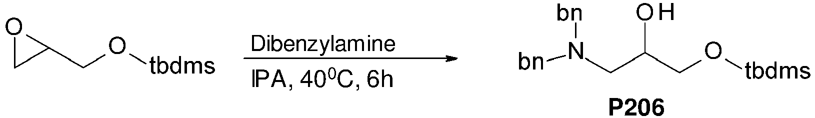

To a solution of *tert*-butyl(dimethyl)(oxiran-2-ylmethoxy)silane (5 g, 27 mmol) in IPA (20 ml), dibenzylamine (1.1 eq) was added. After stirring at rt for 6 h, the solvent was removed under reduced pressure. The residue was purified by column chromatography (EtOAc/MeOH, 80/20 v/v) to afford **P206** as yellow oil (6.5 g, 63%). LCMS (ESI) [MH]⁺: 387.

### Preparation 207. Ethyl 3-{2-{[tert-butyl(dimethyl)silyl]oxy}-1-[(dibenzylamino)methyl]ethoxy}propanoate (P207)

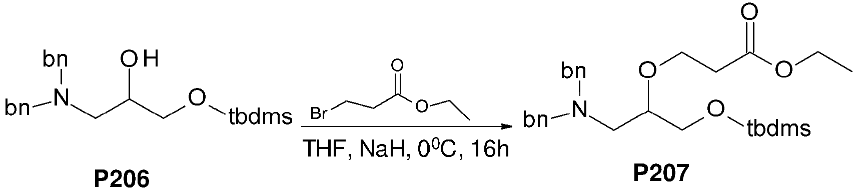

To a solution of 1-{[tert-butyl(dimethyl)silyl]oxy}-3-(dibenzylamino)propan-2-ol (**P206**, 5 g, 13 mmol) in THF (100 ml), NaH (1.1 eq) was added in portions at 0°C. After stirring for 1 h, ethyl 3-bromopropanoate (1.1 eq) in THF (20 ml) was added dropwise. After stirring at rt for 6 h, the solvent was removed under reduced pressure. The residue was purified by column chromatography (DCM/EtOAc, 0-10%) to afford **P207** as yellow oil (2.7 g, 42%). LCMS (ESI) [MH]⁺: 487.

### Preparation 208. 2-({[tert-Butyl(dimethyl)silyl]oxy}methyl)-1,4-oxazepan-5-one (P208)

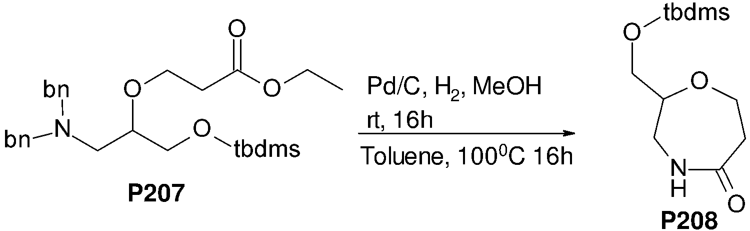

A solution of ethyl 3-{2-{[tert-butyl(dimethyl)silyl]oxy}-1-[(dibenzylamino)methyl]ethoxy}propanoate (**P207**, 1 g, 2 mmol) and Pd/C (10%, 500 mg) in MeOH (20 ml), was stirred at rt under H₂ for 16 h. The solvent was removed under reduced pressure and the residue was redissolved in toluene (50 ml) and stirred at 100°C for 16 h. The solvent was removed under reduced pressure. The residue was purified by column chromatography (DCM/EtOAc, 0-10%) to afford product **P208** as yellow oil (280 mg, 66%). LCMS (ESI) [MH]⁺: 260.

### Preparation 209. 2-(Hydroxymethyl)-1,4-oxazepan-5-one (P209)

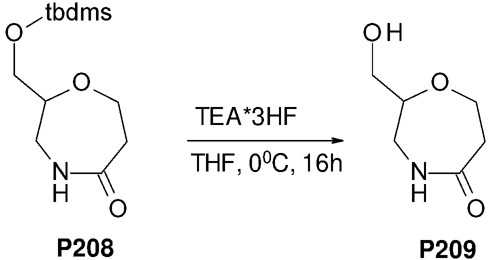

To a solution of 2-({[tert-butyl(dimethyl)silyl]oxy}methyl)-1,4-oxazepan-5-one (**P208**, 560 mg, 2.2 mmol) in 20 ml of anh. THF triethylamine trihydrofluoride (7 eq) at 0°C under N₂ atmosphere was added. The solution was stirred at rt for 12 h and all volatiles were removed using a rotary evaporator. The residue was dissolved in EtOAc and washed with saturated NaHCO₃ solution. The filtrate was purified by column chromatography (DCM/MeOH=10:1 v/v) to give **P209** (250 mg, 80%).

### Preparation 210. 5-Formyl-4-methyl-1-[(5-oxo-1,4-oxazepan-2-yl)methyl]-1H-indole-2-carbonitrile (P210)

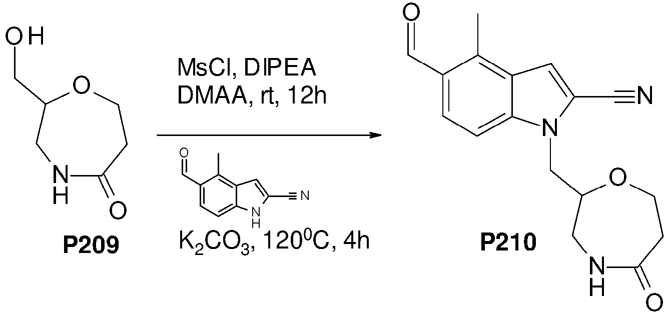

To a solution of 2-(hydroxymethyl)-1,4-oxazepan-5-one (**P209**, 100 mg, 0.7 mmol) and DIPEA (1.2 eq) in DMAA (5 ml) at rt MsCl (1.1 eq) was added. The reaction mixture was stirred at rt for 12 h. Then K₂CO₃ (4 eq) and 5-formyl-4-methyl-1H-indole-2-carbonitrile (**P97**, 1 eq) were added and the mixture was heated to 140°C for 4 h. The reaction mixture was diluted with water and extracted with EtOAc. The residue after evaporation was subjected to column chromatography on silica gel eluting with DCM/MeOH (0 - 20%) to afford **P210** (90 mg, 23%) as a yellow solid. LCMS (ESI) [MH]⁺: 312.

### Synthesis of 1-[(4-ethyl-5-oxomorpholin-2-yl)methyl]-5-formyl-4-methyl-1H-indole-2-carbonitrile (P214)

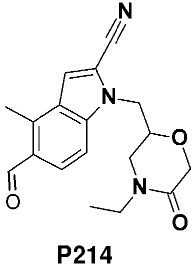

### Preparation 211. 6-({[tert-Butyl(dimethyl)silyl]oxy}methyl)morpholin-3-one (P211)

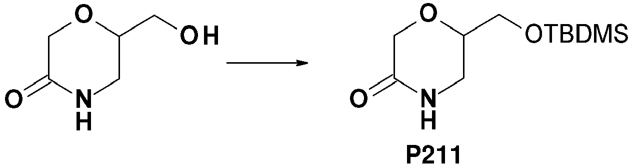

To a solution of 6-(hydroxymethyl)morpholin-3-one (1.2 g, 9 mmol) in 10 ml of DMF imidazole (0.623 g, 9 mmol) and *tert*-butyldimethylsilyl chloride (1.37 g, 9 mmol) were added. The solution was stirred at rt for 18 h. The solvent then was removed in vacuo and the residue was dissolved in 150 ml of ethyl acetate. The solution was washed with water and the organic layer was dried over MgSO₄. After removing the solvent, the solid was purified on silica gel column chromatography (gradient elution, 0-10% EtOAc/MeOH) to yield **P211** (1.75 g, 78 %). ¹H NMR (400 MHz, DMSO-*d₆*), δ: 7.93 (s, 1H), 4.03-3.98 (m, 2H), 3.72-3.66 (m, 2H), 3.63-3.57 (m, 1H), 3.22-3.07 (m, 2H), 0.86 (s, 9H), 0.04 (s, 6H).

### Preparation 212. 6-({[tert-Butyl(dimethyl)silyl]oxy}methyl)-4-ethylmorpholin-3-one (P212)

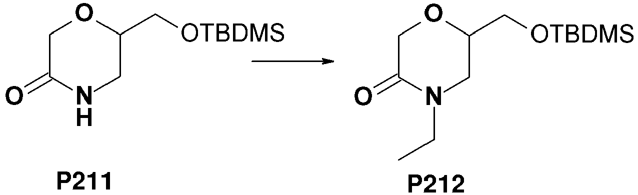

To a solution of 6-({[tert-butyl(dimethyl)silyl]oxy}methyl)morpholin-3-one (**P211**, 0.3 g, 2.3 mmol) in 10 ml of DMAA sodium hydride (0.04 g) was added and the mixture was stirred at rt for 30 min. Ethyl iodide (0.3 g, 2.3 mmol) was added and formed solution was stirred at rt for 18 h (TLC monitoring). The reaction mixture was diluted with water (100 ml) and EtOAc (100 ml). The organic layer was separated, washed with brine, dried over Na₂SO₄, filtered, and concentrated under reduced pressure. The residue was subjected to silica flash chromatography eluting with a EtOAc to yield **P212** (0.35 g, 97 %). ¹H NMR (400 MHz, DMSO-*d₆*), δ: 4.08-3.98 (m, 2H), 3.86-3.77 (m, 1H), 3.72-3.61 (m, 2H), 3.34-3.20 (m, 4H), 3.09 (t, 3H, *J* = 7.2), 0.87 (s, 9H), 0.05 (s, 6H).

### Preparation 213. 4-Ethyl-6-(hydroxymethyl)morpholin-3-one (P213)

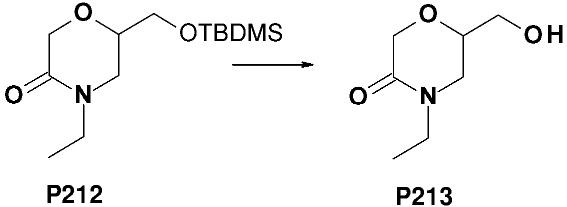

A mixture of 6-({[tert-butyl(dimethyl)silyl]oxy}methyl)-4-ethylmorpholin-3-one (**P212**, 0.35 g, 2.3 mmol), HCl (2 ml), and water (2 ml) in THF was stirred at rt for 3 h and then concentrated under reduced pressure. The mixture was purified on silica gel column chromatography (gradient elution, 0-10% EtOAc/MeOH) to yield **P213** (183 mg, 92 %). ¹H NMR (400 MHz, CD₃CN), δ: 4.15-4.03 (m, 2H), 3.86-3.76 (m, 1H), 3.62-3.51 (m, 2H), 3.45-3.19 (m, 4H), 3.01-2.88 (m, 1H), 1.11 (t, 3H, *J* = 7.2).

### Preparation 214. 1-[(4-Ethyl-5-oxomorpholin-2-yl)methyl]-5-formyl-4-methyl-1H-indole-2-carbonitrile (P214)

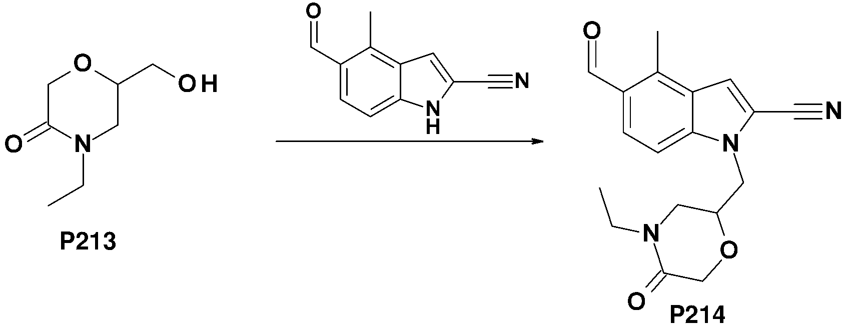

To a solution of 4-ethyl-6-(hydroxymethyl)morpholin-3-one (**P213**, 0.19 g, 1.2 mmol) in 5 ml DMAA K₂CO₃ (0.825 g, 6 mmol) and methanesulfonyl chloride (0.275 g, 2.4 mmol) were added at 0°C. After 30 min 5-formyl-4-methyl-1H-indole-2-carbonitrile (**P97**, 0.220 g, 1.2 mmol) was added. The mixture was stirred at 80°C for 24 h. After cooling to rt, the mixture was extracted with ethylacetate (3x25 ml), and the combine organic layers were washed with water (3x60 ml) and brine (25 ml) and dried under Na₂SO₄. After removing the solvent, the solid was purified on silica gel column chromatography (gradient elution, 0-50% DCM/EtOAc) to yield **P214** (0.102 g, 26%). ¹H NMR (400 MHz, DMSO-*d*₆), δ: 10.36 (s, 1H), 7.84 (d, *J* = 8.6 Hz, 1H), 7.78 (d, *J* = 8.6 Hz, 1H), 7.51 (s, 1H), 4.15 (m, 2H), 3.87 (m, 3H), 3.39 (m, 2H), 2.85 (s, 3H), 3.37 (m, 2H), 1.06 (t, *J* = 7.1 Hz, 3H). LCMS (ESI) [MH]⁺: 326.

### Synthesis of 5-formyl-1-[(4-isobutyl-5-oxomorpholin-2-yl)methyl]-4-methyl-1H-indole-2-carbonitrile (P216)

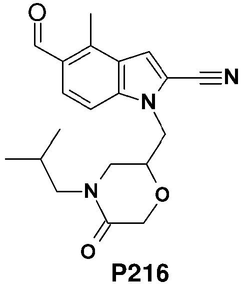

### Preparation 215. 5-(Dimethoxymethyl)-4-methyl-1-[(5-oxomorpholin-2-yl)methyl]-1H-indole-2-carbonitrile (P215)

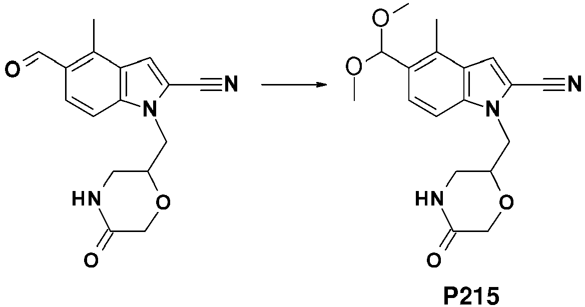

1-[(5-oxomorpholin-2-yl)methyl]-5-formyl-4-methyl-1H-indole-2-carbonitrile (0.1 g, 0.33 mmol) and trimethyl orthoformate (0.054 g, 0.51 mmol) were added to 10 ml of methanol. Then 10 µl of concentrated sulfuric acid was added dropwise. After stirring at 60°C for 3 h, the reaction was stopped. Then 30% aqueous NaHCO₃ solution (10.0 ml) was added to the reaction mixture. The mixture was extracted with DCM (3x25 ml) and the combined organic layers were washed with water (3x60 ml) and brine (25 ml) and dried under Na₂SO₄. The solvent was distilled off under reduced pressure to yield **P215** (0.087 g, 76 %). ¹H NMR (400 MHz, DMSO-*d₆*), δ: 8.01 (s, 1H), 7.58 (s, 1H), 7.53 - 7.46 (m, 2H), 7.53 (s, 1H), 4.60 - 4.52 (m, 1H), 4.42 - 4.31 (m, 1H), 4.09 - 4.03 (m, 1H), 4.03 - 3.86 (m, 2H), 3.29 (s, 3H), 3.25 (s, 6H), 2.94 (s, 1H), 2.78 (s, 1H). LCMS (ESI) [MH]⁺: 326.

### Preparation 216. 5-(Dimethoxymethyl)-4-methyl-1-[(5-oxomorpholin-2-yl)methyl]-1H-indole-2-carbonitrile (P216)

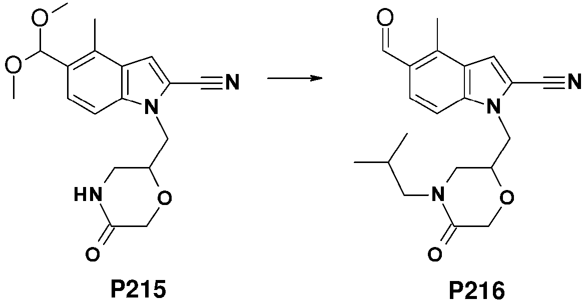

To a solution of 5-(dimethoxymethyl)-4-methyl-1-[(5-oxomorpholin-2-yl)methyl]-1H-indole-2-carbonitrile (**P215**, 0.087 g, 0.25 mmol) in 5 ml of DMAA sodium hydride (0.013 g) was added and the mixture was stirred at rt for 30 min. Isobutyl bromide (30 µl) was added and solution was stirred at rt for additional 18 h (TLC monitoring). The reaction mixture was diluted with water (100 ml) and EtOAc (100 ml). The organic layer was separated, washed with brine, dried over Na₂SO₄, filtered, and concentrated under reduced pressure. The residue was subjected to silica flash chromatography eluting with a EtOAc to yield **P216** (0.06 g, 67 %). ¹H NMR (400 MHz, CDCl₃), δ: 10.43 (s, 1H), 7.93 (d, *J* = 8.9 Hz, 1H), 7.45 (s, 1H), 7.40 (d, *J* = 8.9 Hz, 1H), 4.5 - 4.45 (m, 1H), 4.28 (d, *J* = 16.5 Hz, 1H), 4.22 - 4.14 (m, 1H), 4.08 (d, *J* = 16.7 Hz, 1H), 3.47 - 3.38 (m, 1H), 3.37 - 3.26 (m, 2H), 3.16 - 3.11 (m, 1H), 2.91 (s, 3H), 2.08 - 1.93 (m, 1H), 1.69 - 1.53 (m, 1H), 0.99 - 0.82 (m, 6H). LCMS (ESI) [MH]⁺: 354.

### Synthesis of 5-formyl-4-methyl-1-[2-(3-oxopiperazin-1-yl)propyl]-1H-indole-2-carbonitrile (P219)

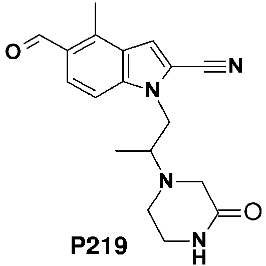

### Preparation 217. Methyl 2-(3-oxopiperazin-1-yl)propanoate (P217)

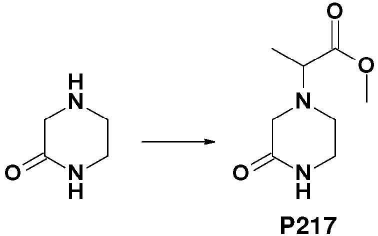

To a solution of piperazin-2-one (1.5 g, 15. mmol) and methyl 2-bromopropanoate (2.76 g, 16.5 mmol) in 15 ml of ACN was added K₂CO₃ (6.21 g, 45 mmol) and the mixture was stirred at 80°C for overnight (TLC monitoring). The reaction mixture was diluted with water (100 ml) and EtOAc (100 ml). The organic layer was separated, washed with brine, dried over Na₂SO₄, filtered, and concentrated under reduced pressure. The residue was subjected to silica flash chromatography eluting with EtOAc to give **P217** as yellow oil (2.43 g, 86 %). ¹H NMR (400 MHz, DMSO-*d₆*), δ: 7.74 (s, 1H), 3.36 (s, 3H), 3.45 (q, *J* = 7.1 Hz, 1H), 3.10 (m, 4H), 2.78 - 2.70 (m, 1H), 2.67 - 2.61 (m, 1H), 1.20 (d, *J* = 7.1 Hz, 3H). LCMS (ESI) [MH]⁺: 187.

### Preparation 218. 4-(2-Hydroxy-1-methylethyl)piperazin-2-one (P218)

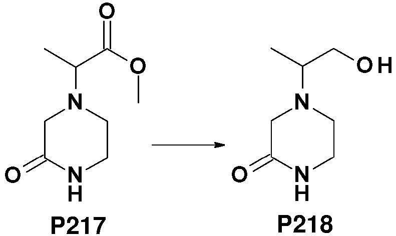

To a solution of methyl 2-(3-oxopiperazin-1-yl)propanoate (**P217**, 1 g, 5 mmol) in 15 ml of THF LiBH₄ (0.237 g, 10 mmol) was added, and the reaction mixture was stirred at rt for overnight (TLC monitoring). The reaction mixture was diluted with water (100 ml) and EtOAc (100 ml). The organic layer was separated, washed with brine, dried over Na₂SO₄, filtered, and concentrated under reduced pressure. The residue was subjected to silica flash chromatography eluting with a EtOAc to yield **P218** (0.515 g, 66%). ¹H NMR (400 MHz, CDCl₃), δ: 6.21 (s, 1H), 3.55 - 3.45 (m, 1H), 3.43 - 3.30 (m, 4H), 3.24 - 3.20 (m, 1H), 2.97 - 2.76 (m, 3H), 1.84 - 1.61 (m, 1H), 0.97 (d, J = 6.7 Hz, 3H). LCMS (ESI) [MH]⁺: 145.

### Preparation 219. 5-Formyl-4-methyl-1-[2-(3-oxopiperazin-1-yl)propyl]-1H-indole-2-carbonitrile (P219)

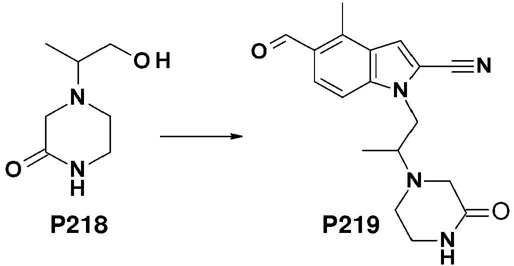

To a solution of 4-(2-hydroxy-1-methylethyl)piperazin-2-one (**P218**, 0.25 g, 1.6 mmol) in 5 ml DMAA K₂CO₃ (1.09 g, 8 mmol) and methanesulfonyl chloride (0.362 g, 3.2 mmol) were added at 0°C. After 30 min 5-formyl-4-methyl-1H-indole-2-carbonitrile (**P97**, 0.220 g, 1.2 mmol) was added. The mixture was stirred at 80°C for 24 h. After cooling to rt, the mixture was extracted with ethylacetate (3x25 ml) and the combine organic layers were washed with water (3x60 ml) and brine (25 ml) and dried under Na₂SO₄. After removing the solvent, the solid was purified on silica gel column chromatography (gradient elution, 0-50% DCM/ EtOAc) to yield **P219** (0.082 g, 16%). ¹H NMR (400 MHz, CDCl₃), δ: 10.44 (s, 1H), 7.92 (d, *J* = 8.7 Hz, 1H), 7.43 (s, 1H), 7.38 (d, *J* = 8.7 Hz, 1H), 3.44 - 3.38 (m, 3H), 3.37 - 3.26 (m, 2H), 3.24 - 3.17 (m, 1H), 2.81- 2.73 (m, 3H), 2.65 - 2.58 (m, 2H), 1.30 - 1.21 (m, 2H), 1.12 (d, *J* = 6.8 Hz, 3H). LCMS (ESI) [MH]⁺: 325.

### Synthesis of 5-formyl-4-methyl-1-{[(2R,3S)-6-oxo-2-phenylpiperidin-3-yl]methyl}-1H-indole-2-carbonitrile (P221)

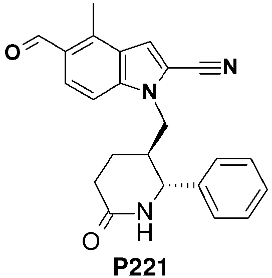

### Preparation 220. (5S,6S)-5-(Hydroxymethyl)-6-phenylpiperidin-2-one (P220)

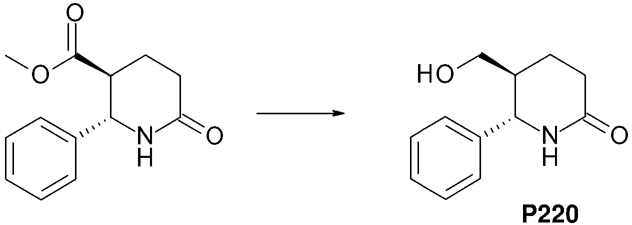

To a solution of methyl (2S,3S)-6-oxo-2-phenylpiperidine-3-carboxylate (0.5 g, 2 mmol) in 10 ml of THF LiBH₄ (0.09 g, 4 mmol) was added, and the mixture was stirred at rt for overnight (TLC monitoring). The reaction mixture was diluted with water (100 ml) and EtOAc (100 ml). The organic layer was separated, washed with brine, dried over Na₂SO₄, filtered, and concentrated under reduced pressure. The residue was subjected to silica flash chromatography eluting with a EtOAc to yield **P220** (0.16 g, 36 %). ¹H NMR (400 MHz, DMSO-*d₆*), δ: 7.57 (s, 1H), 7.41 - 7.32 (m, 2H), 7.30 - 7.22 (m, 3H), 4.65 - 4.62 (m, 1H), 4.27 (d, J = 7.2 Hz, 1H), 3.39 - 3.30 (m, 1H), 3.28 - 3.14 (m, 1H), 2.30 - 2.19 (m, 2H), 1.89 - 1.78 (m, 1H), 1.75 - 1.65 (m, 1H), 1.63 - 1.54 (m, 1H). LCMS (ESI) [MH]⁺: 206.

### Preparation 221. 5-Formyl-4-methyl-1-{[(2R,3S)-6-oxo-2-phenylpiperidin-3-yl]methyl}-1H-indole-2-carbonitrile (P221)

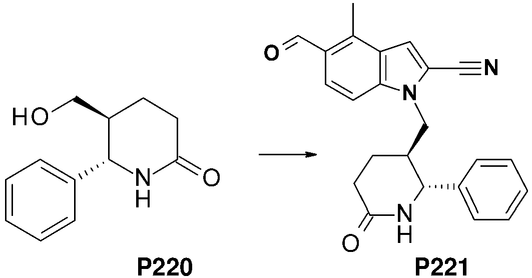

To a solution of (5S,6S)-5-(hydroxymethyl)-6-phenylpiperidin-2-one (**P220**, 0.16 g, 0.8 mmol) in 5 ml DMAA DIPEA (720 µL) and methanesulfonyl chloride (0.07 g, 0.9 mmol) were added at 0°C. After 30 min 5-formyl-4-methyl-1H-indole-2-carbonitrile (**P97**, 0.072 g, 0.4 mmol) was added. The mixture was stirred at 80°C for 24 h. After cooling to rt, the mixture was extracted with ethylacetate (3x25 ml) and the combine organic layers were washed with water (3x60 ml) and brine (25 ml) and dried under Na₂SO₄. After removing the solvent, the solid was purified on silica gel column chromatography (gradient elution, 0-50% DCM/EtOAc) to yield **P221** (0.1 g, 34%). ¹H NMR (400 MHz, CDCl₃), δ: 10.40 (s, 1H), 7.80 (d, *J* = 8.8 Hz, 1H), 7.54 - 7.41 (m, 2H), 7.43 - 7.37 (m, 2H), 7.36 - 7.32 (m, 2H),6.70 (d, *J* = 8.8 Hz, 1H), 5.79 (s, 1H), 4.38 - 4.30 (m, 1H), 4.25 - 4.08 (m, 2H), 2.86 (s, 3H), 2.62 - 2.52 (m, 2H), 2.45 - 2.32 (m, 1H), 1.95 - 1.72 (m, 1H), 1.35 - 1.24 (m, 1H). LCMS (ESI) [MH]⁺: 372.

### Synthesis of 5-formyl-4-methyl-1-[(5-oxopyrrolidin-3-yl)methyl]-1H-indole-2-carbonitrile (P223)

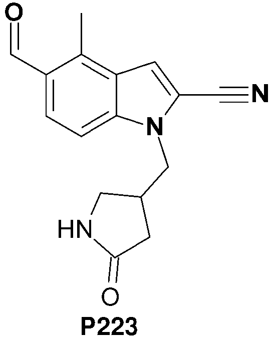

### Preparation 222. 4-(Hydroxymethyl)pyrrolidin-2-one (P222)

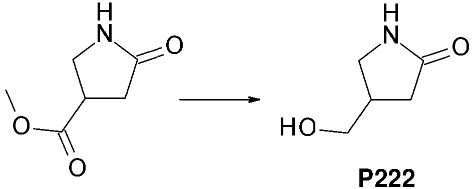

To a solution of methyl 5-oxopyrrolidine-3-carboxylate (1.1 g, 7.7 mmol) in 15 ml of THF LiBH₄ (0.34 g, 15 mmol) was added and stirred at rt for overnight (TLC monitoring). The reaction mixture was diluted with water (100 ml) and EtOAc (100 ml). The organic layer was separated, washed with brine, dried over Na₂SO₄, filtered, and concentrated under reduced pressure. The residue was subjected to silica flash chromatography eluting with a EtOAc to yield **P222** (0.5 g, 57%). ¹H NMR (400 MHz, DMSO-*d₆*), δ: 7.44 (s, 1H), 4.75 (t, *J* = 5.2 Hz, 1H), 3.38 - 3.30 (m, 3H), 3.28 - 3.23 (m, 1H), 2.47 - 2.38 (m, 1H), 2.23 - 2.11 (m, 1H), 1.92 - 1.83 (m, 1H). LCMS (ESI) [MH]⁺: 116.

### Preparation 223. 5-Formyl-4-methyl-1-[(5-oxopyrrolidin-3-yl)methyl]-1H-indole-2-carbonitrile (P223)

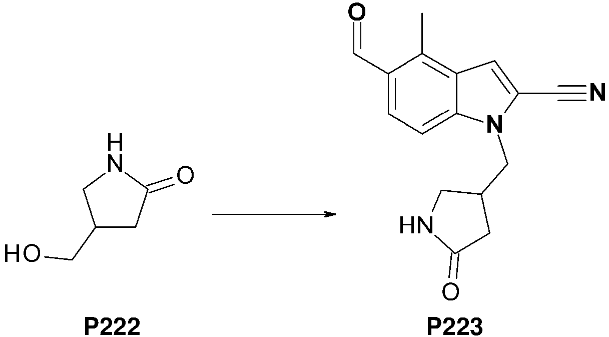

To a solution of 4-(hydroxymethyl)pyrrolidin-2-one (**P222**, 0.3 g, 2.6 mmol) in 5 ml of DMAA DIPEA (2 ml) and methanesulfonyl chloride (0.358 g, 3.1 mmol) were added at 0°C. After 30 min 5-formyl-4-methyl-1H-indole-2-carbonitrile (**P97**, 0.072 g, 0.4 mmol) was added. The mixture was stirred at 80°C for 24 h. After cooling to rt, the mixture was extracted with ethylacetate (3x25 ml) and the combine organic layers were washed with water (3x60 ml) and brine (25 ml) and dried under Na₂SO₄. After removing the solvent, the solid was purified on silica gel column chromatography (gradient elution, 0-50% CH₂Cl₂/ EtOAc) to yield **P223** (16 mg, 2%). ¹H NMR (400 MHz, CDCl₃), δ: 10.41 (s, 1H), 7.94 (d, *J* = 8.5 Hz, 1H), 7.44 (s, 1H), 7.32 (d, *J* = 8.5 Hz, 1H), 5.93 (s, 1H), 4.44 - 4.30 (m, 1H), 3.69 - 3.53 (m, 2H), 2.91 (s, 3H), 2.62 - 2.45 (m, 2H), 2.28 - 2.14 (m, 1H), 1.69 - 1.60 (m, 1H). LCMS (ESI) [MH]⁺: 282.

### Synthesis of 5-formyl-4-methyl-1-[(5-oxopyrrolidin-2-yl)methyl]indole-2-carbonitrile (P225)

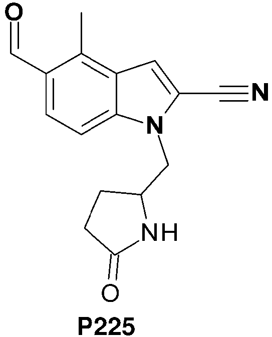

### Preparation 224. 5-(Hydroxymethyl)pyrrolidin-2-one (P224)

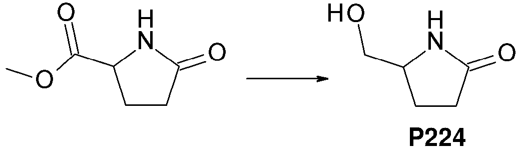

To a solution of methyl 5-oxopyrrolidine-3-carboxylate (2.5 g, 19.5 mmol) in 15 ml of THF LiBH₄ (0.772 g, 39 mmol) was added, and the reaction mixture was stirred at rt for overnight (TLC monitoring). The reaction mixture was diluted with water (100 ml) and EtOAc (100 ml). The organic layer was separated, washed with brine, dried over Na₂SO₄, filtered, and concentrated under reduced pressure. The residue was subjected to silica flash chromatography eluting with a EtOAc to yield **P224** (1.3 g, 65%). ¹H NMR (400 MHz, DMSO-*d₆*), δ: 7.53 (s, 1H), 4.76 (t, J = 5.4 Hz, 1H), 3.53 - 3.45 (m, 1H), 3.34 - 3.29 (m, 2H), 2.18 - 1.94 (m, 3H), 1.77 - 1.60 (m, 1H). LCMS (ESI) [MH]⁺: 116.

### Preparation 225. 5-Formyl-4-methyl-1-[(5-oxopyrrolidin-2-yl)methyl]indole-2-carbonitrile (P225)

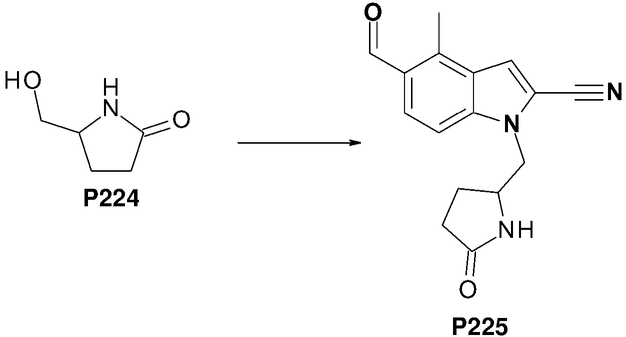

To a solution of 5-(hydroxymethyl)pyrrolidin-2-one (**P224**, 0.3 g, 2.6 mmol) in 5 ml of DMAA DIPEA (2 ml) and methanesulfonyl chloride (0.358 g, 3.1 mmol) were added at 0°C. After 30 min 5-formyl-4-methyl-1H-indole-2-carbonitrile (**P97**, 0.072 g, 0.4 mmol) was added. The mixture was stirred at 80°C for 24 h. After cooling to rt, the mixture was extracted with ethylacetate (3x25 ml), and the combine organic layers were washed with water (3x60 ml) and brine (25 ml) and dried under Na₂SO₄. After removing the solvent, the solid was purified on silica gel column chromatography (gradient elution, 0-50% DCM/ EtOAc) to yield **P225** (40 mg, 5%). ¹H NMR (400 MHz, CDCl₃), δ: 10.37 (s, 1H), 7.86 (d, *J* = 8.7 Hz, 1H), 7.58 (s, 1H), 7.20 (d, *J* = 8.7 Hz, 1H), 7.03 (s, 1H), 4.41 - 4.44 (m, 1H), 3.97 - 3.86 (m, 1H), 2.83 (s, 3H), 2.76 - 2.66 (m, 2H), 2.47 - 2.45 (m, 1H), 1.97 - 1.86 (m, 2H). LCMS (ESI) [MH]⁺: 282.

### Synthesis of tert-butyl N-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]sulfamoyl]carbamate (P226)

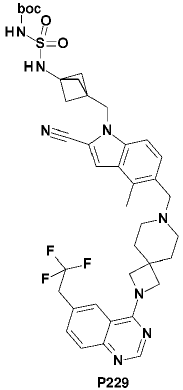

### Preparation 226. tert-Butyl N-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]sulfamoyl]carbamate hydrochloride (P226)

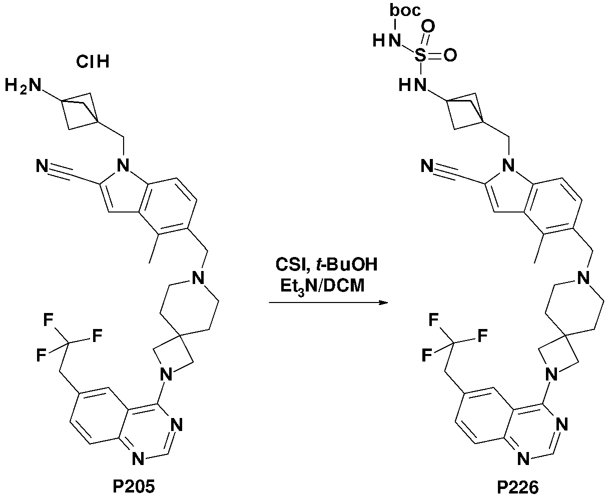

To a stirred solution of chlorosulfonyl isocyanate (50 mg, 0.35 mmol) in anhydrous DCM (2 ml) t-BuOH (26 mg, 0.35 mmol) was added dropwise at 0°C. After that 1-[(3-amino-1-bicyclo[1.1.1]pentanyl)methyl]-4-methyl-5-[[2-[6-(2,2,2-trifluoroethyl)quinazolin-4-yl]-2,7-diazaspiro[3.5]nonan-7-yl]methyl]indole-2-carbonitrile hydrochloride (**P205**, 150 mg, 0.24 mmol) in anhydrous DCM (5 ml) was added at 0°C. The resulting solution was allowed to warm up to rt for over a period of 1 h. The reaction mixture was diluted with water. The organic layer was separated, washed with brine, dried over sodium sulfate, filtered, and the filtrate was evaporated to afford the *tert-*butyl *N*-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]sulfamoyl]carbamate (**P226**, 120 mg, 67%). LCMS (ESI) [MH]⁺: 779.

### Synthesis of ethyl 2-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]-sulfamoyl-amino]acetate (P227)

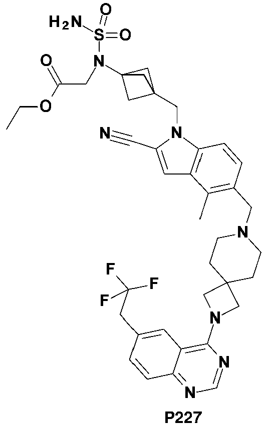

### Preparation 227. Ethyl 2-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]-sulfamoyl-amino]acetate (P227)

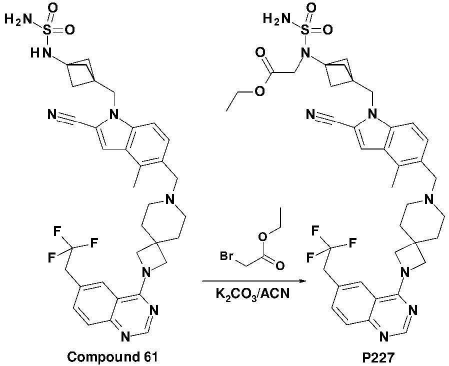

To a stirred mixture of 4-[7-[[2-cyano-4-methyl-1-[[3-(sulfamoylamino)-1-bicyclo[1.1.1]pentanyl]methyl]indol-5-yl]methyl]-2,7-diazaspiro[3.5]nonan-2-yl]-6-(2,2,2-trifluoroethyl)quinazoline trifluoroacetate (compound **61**, 80 mg, 0.1 mmol), K₂CO₃ (41 mg, 0.3 mmol) in ACN (2 ml) methyl bromoacetate (17 mg, 0.1 mmol) was added and the reaction mixture was stirred at rt for 12 h. Water (5 ml) and EtOAc (5 ml) were added. The organic layer was separated, washed with brine, dried over sodium sulfate, filtered, and the filtrate was evaporated. The ethyl 2-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]-sulfamoyl-amino]acetate was used in the next step without additional purification. LCMS (ESI) [MH]⁺: 765.

### Synthesis of 1-(2-(4-(ethylsulfonyl)piperazin-1-yl)propyl)-5-formyl-4-methyl-1H-indole-2-carbonitrile (P233)

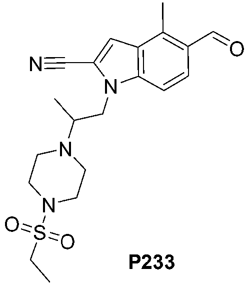

### Preparation 228. Methyl 2-((methylsulfonyl)oxy)propanoate (P228)

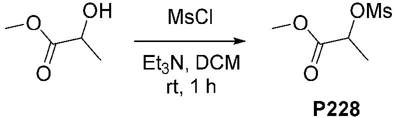

To a stirred solution of methyl 2-hydroxypropanoate (25 ml, 0.26 mol) in DCM (435 ml) was added triethylamine (72 ml, 0.52 mol) in ice bath. The mixture was stirred in ice bath for 15 min, and then methanesulfonyl chloride (24 ml, 0.31 mol) was added to the mixture. After the reaction was stirred at rt for 1 h, the mixture was treated with water and extracted with DCM. The combined organic layers were washed with brine, dried over Na₂SO₄₍ₛ₎, filtered, and concentrated to give **P231** (45.0 g) as an orange oil which was used in next step without further purification. ¹H NMR (400 MHz, CDCl₃), δ: 5.14 (q, *J* = 7.0 Hz, 1H), 3.80 (s, 3H), 3.15 (s, 3H), 1.61 (d, *J* = 7.0 Hz, 3H); LCMS (ESI) [MH]⁺: 183.0.

### Preparation 229. tert-Butyl 4-(1-methoxy-1-oxopropan-2-yl)piperazine-1-carboxylate (P229)

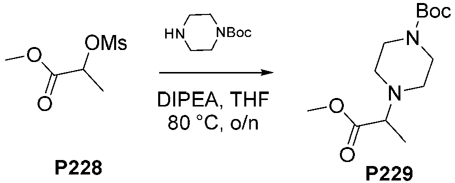

To a stirred solution of **P228** (45.0 g, 248 mmol) and 1-*boc*-piperazine (41 g, 220 mmol) in anh. THF (740 ml) was added *N*,*N*-diisopropylethylamine (76 ml). The reaction was stirred at 80°C for overnight. The solution was concentrated. The residue was treated with water and extracted with EtOAc. The combined organic layers were dried over Mg₂SO₄, filtered, and concentrated. The residue was purified by silica gel chromatography (0-40 % EtOAc in *n*-hexane) to give **P229** (49.5 g, 70%) as an orange oil. ¹H NMR (400 MHz, CDCl₃), δ: 5.13 (q, *J* = 7.0 Hz, 1H), 3.69 (s, 3H), 3.44-3.41 (m, 4H), 3.14 (s, 3H), 2.54-2.51 (m, 4H), 1.29 (d, *J* = 7.0 Hz, 3H).

### Preparation 230. Methyl 2-(piperazin-1-yl)propanoate hydrochloride (P230)

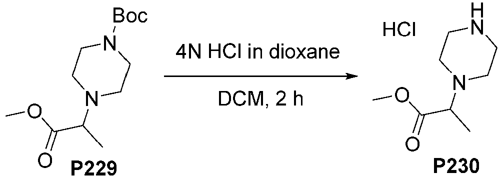

To a stirred solution of **P229** (5.0 g, 18.4 mmol) in DCM (15 ml) was added a solution of 4N HCl in dioxane (15 ml). The mixture was stirred at rt for 2 h. The solution was treated with water and extracted with EtOAc. The water layer was concentrated to give **P230** (3.6 g) as an orange oil which was used in next step without further purification. ¹H NMR (400 MHz, CD₃OD), δ: 4.52 (q, *J* = 7.2 Hz, 1H), 3.89 (s, 3H), 3.85-3.79 (m, 4H), 3.73-3.70 (m, 4H), 1.71 (d, *J* = 7.2 Hz, 3H).

### Preparation 231. Methyl 2-(4-(ethylsulfonyl)piperazin-1-yl)propanoate (P231)

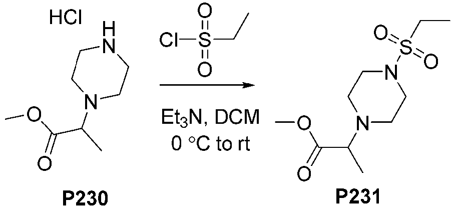

To a stirred solution of **P230** (3.60 g, 20.9 mmol) in DCM (52 ml) was added TEA (9.3 ml, 66.9 mmol) at 0°C and stirred for 15 min. After ethanesulfonyl chloride (2.4 ml, 25.1 mmol) was added to the solution at 0°C, the solution was stirred at rt for overnight. The solution was treated with water and extracted with EtOAc. The combined organic layers were washed with NaHCO_{3(aq)}, dried over with MgSO₄₍ₛ₎, filtered, and concentrated in vacuo to give a residue. The residue was purified by silica gel column chromatography (80% EtOAc in *n*-hexane) to give **P231** (2.53 g) as a colorless oil. ¹H NMR (400 MHz, CDCl₃), δ: 3.70 (s, 3H), 3.37-3.29 (m, 5H), 2.93 (q, *J* = 7.2 Hz, 2H), 2.69-2.66 (m, 4H), 1.36 (t, J= 7.2 Hz, 3H), 1.30 (d, *J* = 7.2 Hz, 3H). LCMS (ESI) [MH]⁺: 265.1.

### Preparation 232. 2-(4-Ethylsulfonylpiperazin-1-yl)propan-1-ol (P232)

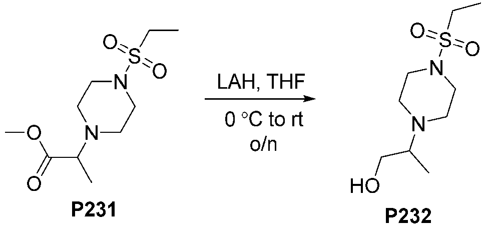

To a solution of **P231** (2.53 g, 9.57 mmol) in anh. THF (17 ml) was added LAH (1M solution in THF, 14.4 ml) dropwise in ice bath. The mixture was stirred at rt for overnight. The mixture was treated with water and 15% NaOH_{(aq)} in ice bath. The mixture was filtered through a pad of celite, and the filtrate was concentrated. The residue was dissolved in DCM, dried over Na₂SO₄₍ₛ₎, filtered, and concentrated. The residue was purified by silica gel chromatography (0-10 % MeOH in DCM) to give **P232** (1.2 g, 55%) as a colorless oil. ¹H NMR (400 MHz, CDCl₃), δ: 3.45-3.41 (m, 1H), 3.38-3.27 (m, 5H), 2.96 (q, *J* = 7.6 Hz, 2H), 2.91-2.82 (m, 1H), 2.77-2.72 (m, 2H), 2.53-2.47 (m, 2H), 1.37 (t, *J =* 7.6 Hz, 3H), 0.92 (d, *J* = 6.4 Hz, 3H) ; LCMS (ESI) [MH]⁺: 237.1.

### Preparation 233. 1-(2-(4-(Ethylsulfonyl)piperazin-1-yl)propyl)-5-formyl-4-methyl-1H-indole-2-carbonitrile (P233)

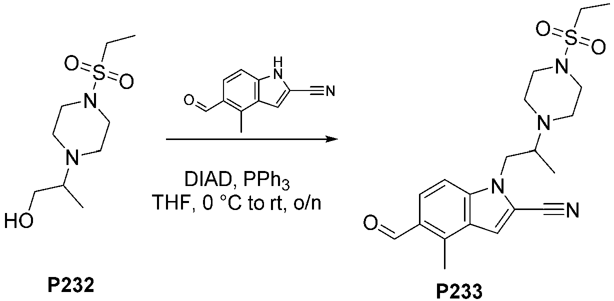

A solution of **P97** (364 mg, 1.98 mmol), **P232** (480 mg, 1.82 mmol), and triphenylphosphine (567 mg, 2.16 mmole) in anh. THF (5.0 ml) was stirred in ice bath for 20 min. After diisopropyl azodicarboxylate (437 mg, 2.16 mmol) was added to the reaction dropwise in ice bath, the reaction was stirred at rt for overnight. The solution was concentrated to remove solvent. The residue was purified by silica-gel column chromatography (50% EtOAc in *n*-hexane). The isolated product was treated with methanol and stirred for a period of time. The resulting solid was collected by filtration to give **P233** (208 mg, 31%) as a white solid. ¹H NMR (400 MHz, CDCl₃), δ: 10.43 (s, 1H), 7.89 (d, *J* = 8.8 Hz, 1H), 7.36 (s, 1H), 7.24 (d, *J* = 8.8 Hz, 1H), 4.29 (dd, *J* = 8.8, 14.4 Hz, 1H), 4.12 (dd, *J* = 5.2, 14.8 Hz, 1H), 3.29-3.17 (m, 4H), 3.16-3.10 (m, 1H), 2.95-2.91 (m, 2H), 2.90 (s, 3H), 2.81-2.78 (m, 2H), 2.44-2.42 (m, 2H), 1.35 (t, *J* = 7.6 Hz, 3H), 1.10 (d, *J* = 6.8 Hz, 3H); LCMS (ESI) [MH]⁺: 403.2. HPLC purity: 95.4 %, *t*_{R} = 20.213 min.

### Synthesis of 5-formyl-1-(2-(4-(isopropylsulfonyl)piperazin-1-yl)propyl)-4-methyl-1H-indole-2-carbonitrile (P236)

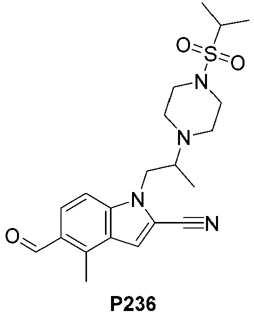

### Preparation 234. Methyl 2-(4-(iso-propylsulfonyl)piperazin-1-yl)propanoate (P234)

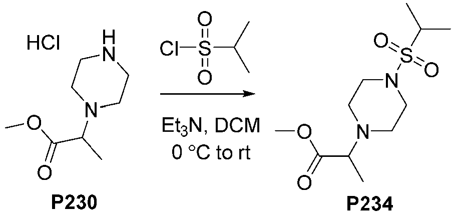

To a solution of **P230** (1.80 g, 8.60 mmol, 1.0 eq) in DCM (22 ml) were added TEA (4.0 ml, 27.5 mmol, 3.2 eq) and propane-2-sulfonyl chloride (1.90 ml, 17.2 mmol, 2.0 eq) at 0°C. The reaction was stirred at rt for 2 h. This mixture was treated with water and extracted with DCM. The organic layers were dried over anh. MgSO_{4,} filtered, and concentrated. The residue was purified by silica gel column (15-50% EtOAc in *n*-hexane) to afford **P234** (250 mg, 22%) as brown oil. ¹H NMR (400 MHz, chloroform-*d*), δ: 3.71 (s, 3H), 3.39-6.20 (m, 5H), 3.20-3.13 (m, 1H), 2.69-2.61 (m, 4H), 1.34 (d, *J* = 6.8 Hz, 6H), 1.30 (d, *J* = 6.8 Hz, 3H).

### Preparation 235. Methyl 2-(4-(iso-propylsulfonyl)piperazin-1-yl)propanoate (P235)

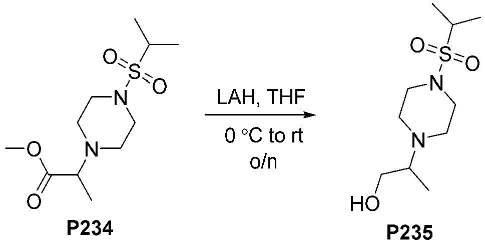

To a solution of **P234** (1.2 g, 4.4 mmol, 1.0 eq) in THF (11 ml) was added 1M LAH in THF solution (6.6 ml, 6.6 mmol, 1.5 eq). The reaction was stirred at rt for overnight. The mixture was quenched with 15% NaOH_{(aq)}, filtered through a pad of celite, and washed with DCM. The filtrate was dried over anh. MgSO₄, filtered, and evaporated. The resulting crude material was purified by C18 reverse phase column (0-100% MeOH in water) to afford **P235** (930 mg, 84%) as a colorless oil. ¹H NMR (400 MHz, chloroform-*d*), δ: 3.44-3.31 (m, 6H), 3.23-3.13 (m, 1H), 3.06 (br. s, 1H), 2.90-2.80 (m, 1H), 2.73-2.67 (m, 2H), 2.49-2.44 (m, 2H), 1.34 (d, *J* = 6.8 Hz, 6H), 0.91 (d, *J* = 6.8 Hz, 3H).

### Preparation 236. 5-Formyl-1-(2-(4-(isopropylsulfonyl)piperazin-1-yl)propyl)-4-methyl-1H-indole-2-carbonitrile (P236)

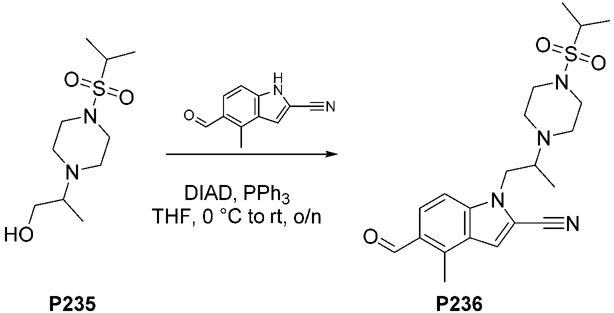

A solution of **P97** (336 mg, 1.82 mmol), **P235** (500 mg, 2.00 mmol), and triphenylphosphine (619 mg, 2.36 mmol) in anh. THF (6 ml) was stirred in ice bath for 20 min. Diisopropyl azodicarboxylate (477 mg, 2.36 mmol) was added to the reaction in ice bath. After the reaction was stirred at rt for overnight, the solution was concentrated to remove solvent. The residue was purified by silica-gel column chromatography (50% EtOAc in *n*-hexane). The isolated product was treated with methanol and stirred for a period of time. The resulting solid was collected by filtration to give **P236** (210 mg, 28%) as a white solid. ¹H NMR (400 MHz, CDCl₃), δ: 10.43 (s, 1H), 7.89 (d, *J* = 9.0 Hz, 1H), 7.36 (s, 1H), 7.24 (d, *J* = 9.0 Hz, 1H), 4.30 (dd, *J* = 14.8, 8.8 Hz, 1H), 4.11 (dd, *J* = 14.8, 5.2 Hz, 1H), 3.25-3.27 (m, 4H), 3.18-3.10 (m, 2H), 2.90 (s, 3H), 2.79-2.75 (m, 2H), 2.42-2.36 (m, 2H), 1.32 (d, *J* = 3.6 Hz, 3H), 1.30 (d, *J* = 3.6 Hz, 3H), 1.09 (d, *J* = 6.8 Hz, 3H); LCMS (ESI) [MH]⁺: 417.2. HPLC purity: 91 %, *t*_{R} = 21.361 min.

### Synthesis of 1-(2-(4-(cyclopropylsulfonyl)piperazin-1-yl)propyl)-5-formyl-4-methyl-1H-indole-2-carbonitrile (P239)

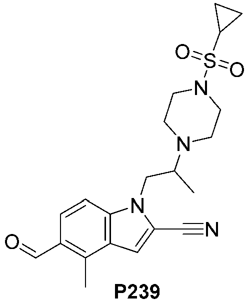

### Preparation 237. Methyl 2-(4-(cyclopropylsulfonyl)piperazin-1-yl)propanoate (P237)

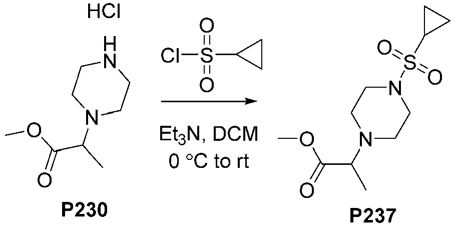

To a stirred solution of **P230** (3.70 g, 17.8 mmol) in DCM (45 ml) was added triethylamine (8.1 ml, 57 mmol) at 0°C and stirred for 10 minutes. After cyclopropanesulfonyl chloride (5.0 g, 36 mmol) was added to the solution at 0°C, the solution was stirred at rt for 3 h. The solution was treated with water and extracted with DCM. The combined organic layers were washed with NaHCO_{3(aq)}, dried over with MgSO₄₍ₛ₎, filtered, and concentrated in vacuo to give a residue. The residue was purified by silica gel column chromatography (80% EtOAc in *n*-hexane) to give **P237** (1.77 g, 36% yield) as a yellow oil. ¹H NMR (400 MHz, CDCl₃), δ: 3.72 (s, 3H), 3.34-3.32 (m, 5H), 2.71-2.69 (m,4 H), 2.28-2.22 (m,1 H), 1.31 (d, *J* = 3.6 Hz, 3 H), 1.19-1.15 (m, 2 H), 1.00-0.95 (m, 2H).

### Preparation 238. 2-(4-(Cyclopropylsulfonyl)piperazin-1-yl)propan-1-ol (P238)

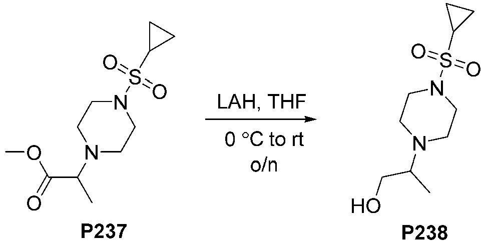

To a solution of **P237** (1.77 g, 6.4 mmol) in anh. THF (15 ml) was added a solution of LAH (2M in THF, 4.8 ml, 9.6 mmol) dropwise in ice bath. The mixture was stirred at rt for overnight. The mixture was treated with water and 15% NaOH_{(aq)} in ice bath. The mixture was filtered through a pad of celite, and the filtrate was concentrated. The residue was dissolved in DCM, dried over Na₂SO₄₍ₛ₎, filtered, and concentrated. The residue was purified by reverse phase column (30% methanol in water) to give **P238** (960 mg, 60%) as a white solid. ¹H NMR (400 MHz, CDCl₃), δ: 3.46-3.42 (m, 1H), 3.38-3.31 (br. m, 5H), 2.92-2.83 (m, 1H), 2.79-2.74 (m, 2H), 2.55-2.50 (m, 2H), 2.30-2.23 (m, 1H), 1.20-1.16 (m, 2H), 1.03-0.97 (m, 2H), 0.93 (d, *J* = 6.8 Hz, 3H).

### Preparation 239. 1-(2-(4-(Cyclopropylsulfonyl)piperazin-1-yl)propyl)-5-formyl-4-methyl-1H-indole-2-carbonitrile (P239)

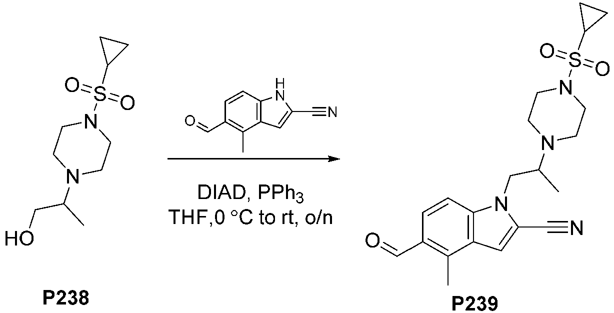

A solution of **P97** (364 mg, 1.98 mmol), **P238** (540 mg, 2.17 mmol), and triphenylphosphine (675 mg, 2.57 mmole) in anh. THF (8.0 ml) was stirred in ice bath for 20 min. After diisopropyl azodicarboxylate (0.51 ml, 2.6 mmol) was added to the reaction dropwise in ice bath, the reaction was stirred at rt for overnight. The solution was concentrated to remove solvent. The residue was purified by silica-gel column chromatography (50% EtOAc in *n*-hexane) to **P238** (208 mg, 23%) as a white solid. ¹H NMR (400 MHz, CDCl₃), δ: 10.43 (s, 1H), 7.89 (d, *J* = 8.8 Hz, 1H), 7.36 (s, 1H), 7.25 (d, *J* = 8.8 Hz, 1H), 4.31 (dd, *J* = 8.8 Hz, 14.8 Hz, 1H), 4.12 (dd, *J* = 5.6 Hz, 14.8 Hz, 1H), 3.30-3.20 (m, 4H), 3.14-3.11 (m, 1H), 2.88 (s, 3H), 2.84-2.79 (m, 2H), 2.48-2.42 (m, 2H), 2.25-2.20 (m, 1H), 1.17-1.12 (m, 2H), 1.10 (d, J= 6.8 Hz, 3H), 1.00-0.94 (m, 2H). LCMS (ESI) [MH]⁺: 415.2. HPLC purity: 90 %, *t*_{R} = 20.906 min.

### Synthesis of 1-(2-(4-(cyclopropylsulfonyl)piperazin-1-yl)propyl)-5-formyl-4-methyl-1H-indole-2-carbonitrile (P242)

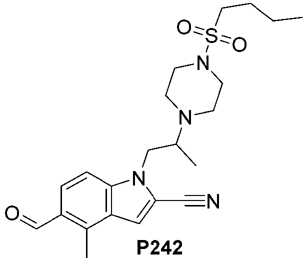

### Preparation 240. Methyl 2-(4-(butylsulfonyl)piperazin-1-yl)propanoate (P240)

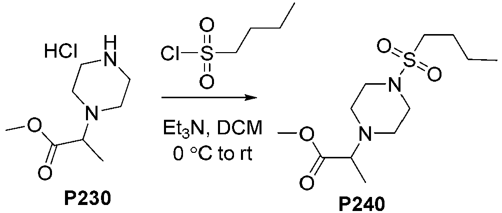

To a stirred solution of **P230** (6.00 g, 28.8 mmol) in DCM (72 ml) was added TEA (13 ml, 92 mmol) at 0°C, and it was stirred for 10 min. After butane-1-sulfonyl chloride (7.50 ml, 57.5 mmol) was added to the reaction at 0°C, the solution was stirred at rt for 4 h. The solution was treated with water and extracted with DCM. The organic layers were washed with NaHCO_{3(aq)}, dried over with MgSO₄₍ₛ₎, filtered, and concentrated in vacuo to give a residue. The residue was purified by silica gel column chromatography (9 % MeOH in DCM) to give **P240** (3.57 g, 42% yield) as a white solid. ¹H NMR (400 MHz, CDCl₃), δ: 3.71 (s, 3H), 3.38-3.29 (m, 5H), 2.91-2.87 (m, 2H), 2.73-2.64 (m, 4H),1.83-1.75 (m, 2H), 1.47-1.41 (m, 2H), 1.30 (d, *J* = 7.2 Hz, 3 H), 0.94 (t, *J* = 7.2 Hz, 3H).

### Preparation 241. 2-(4-(Butylsulfonyl)piperazin-1-yl)propan-1-ol (P241)

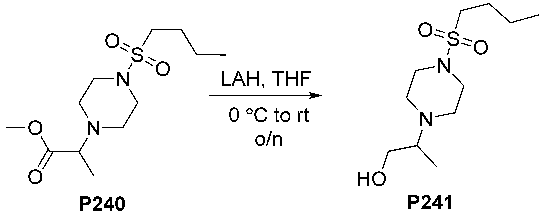

To a solution of **P240** (3.5 g, 12 mmol) in anhydrous THF (30 ml) was added LAH (2M solution in THF, 9.0 ml, 18 mmol) in ice bath. The mixture was stirred at rt for overnight. The mixture was treated with water and 15% NaOH_{(aq)} in ice bath. The mixture was filtered through a pad of celite. The filtrate was concentrated. The residue was dissolved in DCM, dried over Na₂SO₄₍ₛ₎, purified by C18 reverse phase column (30% MeOH in water) to give **P241** (1.4 g, 44%) as a colorless oil. ¹H NMR (400 MHz, CDCl₃), δ: 3.45-3.42 (m, 1H), 3.37-3.26 (br. m, 5H), 2.93-2.82 (m, 3H), 2.77-2.72 (m, 2H), 2.53-2.48 (m, 2H), 1.84-1.76 (m, 2H), 1.50-1.41 (m, 2H), 0.97-0.92 (m, 6H).

### Preparation 242. 1-(2-(4-(Butylsulfonyl)piperazin-1-yl)propyl)-5-formyl-4-methyl-1H-indole-2-carbonitrile (P242)

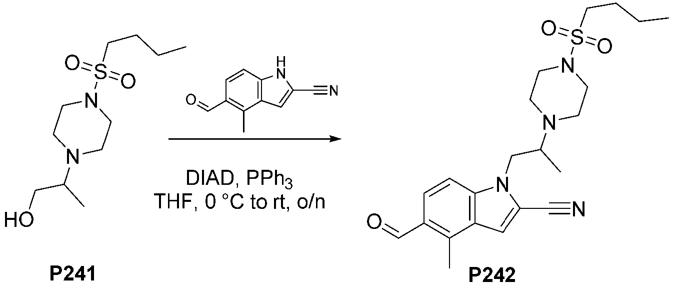

A solution of **P97** (320 mg, 1.72 mmol), **P241** (500 mg, 1.89 mmol), and triphenylphosphine (588 mg, 2.24 mmol) in anh. THF (7.0 ml) was stirred in ice bath for 20 min. After diisopropyl azodicarboxylate (453 mg, 2.24 mmol) was added to the reaction in ice bath, the reaction was stirred at rt for overnight and concentrated. The residue was purified by silica-gel column chromatography (50% EtOAc in *n*-hexane, and then 25 % EtOAc in DCM). The isolated product was treated with methanol and stirred for a period of time. The resulting solid was collected by filtration to give **P242** (193 mg, 26%) as a white solid. ¹H NMR (400 MHz, CDCl₃), δ: 10.43 (s, 1H), 7.89 (d, *J* = 9.0 Hz, 1H), 7.36 (s, 1H), 7.24 (d, *J* = 9.0 Hz, 1H), 4.30 (dd, *J* = 14.8, 8.8 Hz, 1H), 4.12 (dd, *J* = 14.8, 5.2 Hz, 1H), 3.26-3.09 (m, 5H), 2.90 (s, 3H), 2.89-2.84 (m, 2H), 2.83-2.79 (m, 2H), 2.46-2.40 (m, 2H), 1.81-1.70 (m, 2H), 1.49-1.40 (m, 2 H), 1.10 (d, *J* = 6.8 Hz, 3H), 0.95 (t, *J* = 7.6 Hz, 3H); LCMS (ESI) [MH]⁺: 431.2. HPLC purity: 94 %, *t*_{R} = 23.189 min.

### Synthesis of 1-(2-(4-(cyclopropylsulfonyl)piperazin-1-yl)propyl)-5-formyl-4-methyl-1H-indole-2-carbonitrile (P247)

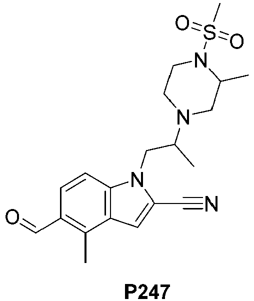

### Preparation 243. tert-Butyl 4-(1-methoxy-1-oxopropan-2-yl)-2-methylpiperazine-1-carboxylate (P243)

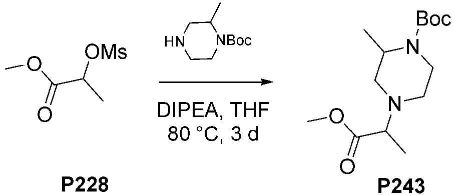

A solution of **P228** (5.46 g, 30.0 mmol) and 1-*boc*-2-methylpiperazine (5.00 g, 25.0 mmol) in anh. THF (85 ml) was added DIPEA (8.70 ml). The reaction was stirred at 80°C for 3 days. The solvent was removed under reduced pressure. The residue was treated with water and extracted with EtOAc. The combined organic layers were dried over Na₂SO₄, filtered, and concentrated to give **P243** (8.98 g) as an orange oil which was used to next step without further purification. ¹H NMR (400 MHz, CDCl₃): δ 4.19 (br. s, 1H), 3.78 (br. s, 1H), 3.68 (s, 3H), 3.32-3.30 (m, 1H), 3.12-2.99 (m, 1H), 2.79-2.70 (m, 1H), 2.61-2.55 (m, 1H), 2.44-2.33 (m, 1H), 1.63 (d, *J* = 6.8 Hz, 3H), 1.45 (s, 9H), 1.27 (d, *J* = 7.0 Hz, 3H); LCMS (ESI) [MH]⁺: 287.2.

### Preparation 244. Methyl 2-(3-methylpiperazin-1-yl)propanoate hydrochloride (P244)

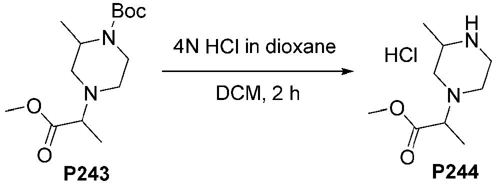

A solution of **P243** (8.98 g, 31.4 mmol) in DCM (27 ml) was added a solution of 4N HCl in dioxane (27 ml). The mixture was stirred at room temperature for 2 h. The solution was washed with water. The water layer was concentrated to give **P244** (6.20 g) as an orange oil which was used in next step without further purification. ¹H NMR (400 MHz, CD₃OD), δ: 4.51 (q, *J* = 7.2 Hz, 1H), 3.98-3.90 (m, 2H), 3.89 (s, 3H), 3.88-3.46 (m, 6H), 1.71 (d, *J* = 7.2 Hz, 3H), 1.50- 1.47 (m, 3H); LCMS (ESI) [MH]⁺: 187.1.

### Preparation 245. Methyl 2-(3-methyl-4-(methylsulfonyl)piperazin-1-yl)propanoate (P245)

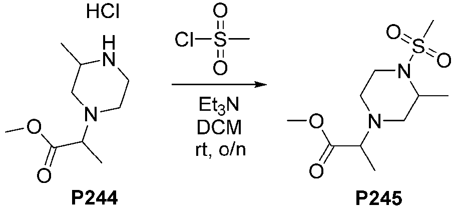

To a stirred solution of **P244** (1.50 g, 8.05 mmol) in anh. DCM (20 ml) was added TEA (3.60 ml, 25.8 mmol) at 0°C and stirred for 15 min. After methanesulfonyl chloride (0.75 ml, 9.7 mmol) was added to the solution at 0°C, the solution was stirred at rt for overnight. The solution was treated with water and extracted with EtOAc. The combined organic layers were washed with NaHCO_{3(aq)}, dried over with MgSO₄₍ₛ₎, filtered, and concentrated in vacuo to give a residue. The residue was purified by silica gel column chromatography (0-65 % EtOAc in *n*-hexane) to give **P245** (930 mg) as a yellow solid. ¹H NMR (400 MHz, CDCl₃), δ: 4.07-3.99 (br. m, 1H), 3.68 (s, 3H), 3.53-3.48 (m, 1H), 3.37-3.30 (m, 1H), 3.29-3.19 (m, 1H), 2.84 (s, 3H), 2.71-2.53 (m, 3H), 2.48-2.41 (m, 1H), 1.33 (t, *J* = 7.2 Hz, 3H), 1.28-1.25 (m, 3H); LCMS (ESI) [MH]⁺: 265.1.

### Preparation 246. 2-(3-Methyl-4-(methylsulfonyl)piperazin-1-yl)propan-1-ol (P246)

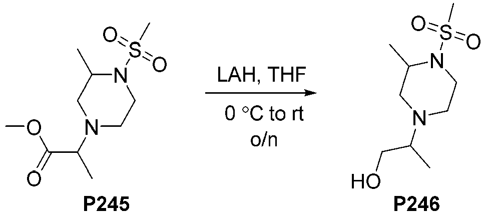

A solution of **P245** (930 mg, 3.52 mmol) in anh. THF (5.9 ml) was added LAH (1M solution in THF, 5.3 ml) dropwise in ice bath. The mixture was stirred at rt for overnight. The mixture was treated with H₂O (0.20 ml) and then 15% NaOH_{(aq)} (0.20 ml) in ice bath. The mixture was filtered through a pad of celite, and the filtrate was concentrated. The residue was dissolved in DCM, dried over Na₂SO₄₍ₛ₎, filtered, and concentrated to give **P246** (680.0 mg) as a yellow oil which was used in next step without further purification. ¹H NMR (400 MHz, CD₃OD), δ: 4.00-3.95 (br. m, 1H), 3.59-3.39 (m, 3H), 2.89 (s, 3H), 2.80-2.61 (m, 4H), 2.59-2.50 (m, 1H), 2.43-2.37 (m, 1H), 1.36-1.33 (m, 3H), 0.98-0.96 (m, 3H). LCMS (ESI) [MH]⁺: 237.1.

### Preparation 247. 5-Formyl-4-methyl-1-(2-(3-methyl-4-(methylsulfonyl)piperazin-1-yl)propyl)-1H-indole-2-carbonitrile (P247)

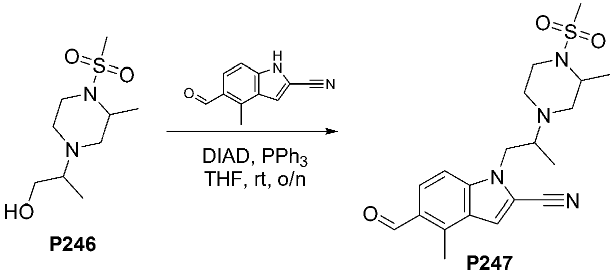

A solution of **P97** (389 mg, 2.11 mmol), **P246** (549 mg, 2.32 mmol), and triphenylphosphine (722 mg, 2.75 mmol) in anh. THF (7.0 ml) was stirred in ice bath for 20 min. After diisopropyl azodicarboxylate (0.55 ml, 2.75 mmol) was added to the reaction dropwise in ice bath, the reaction was stirred at rt for overnight. The solution was concentrated to remove solvent. The residue was purified by silica-gel column chromatography (50% EtOAc in *n*-hexane, and then 25% EtOAc in DCM). The isolated product was treated with methanol and stirred for a period of time. The resulting solid was collected by filtration to give **P247** (236 mg, 28 %) as a white solid. ¹H NMR (400 MHz, CDCl₃), δ: 10.42 (s, 2H), 7.89 (d, *J* = 8.8 Hz, 2H), 7.39-7.36 (m, 2H), 7.29 (d, *J* = 8.8 Hz, 1H), 7.23 (d, *J* = 8.8 Hz, 1H), 4.37 (dd, *J* = 7.2 Hz, 14.8 Hz, 1H), 4.28 (dd, *J* = 10 Hz, 14.8 Hz, 1H), 4.16-4.08 (m, 2H), 4.04-4.02 (m, 1H), 3.91-3.90 (m, 1H), 3.55-3.45 (m, 2H), 3.33-3.30 (m, 1H), 3.26-3.13 (m, 2H), 3.08-3.02 (m, 1H), 2.95-2.92(m, 1H), 2.89 (s, 6H), 2.81 (s, 3H), 2.80 (s, 3H), 2.78-2.74 (m, 2H), 2.67-2.56 (m, 3H), 2.40-2.33 (m, 1H), 2.13-2.10 (m, 1H), 1.20 (d, *J* = 6.8 Hz, 3H), 1.11 (d, *J* = 6.8 Hz, 3H), 1.00 (d, *J* = 6.8 Hz, 3H), 0.89 (d, *J* = 6.8 Hz, 3H). LCMS (ESI) [MH]⁺: 403.20. HPLC purity: 91.1 %, *t*_{R} = 20.644 min.

### Synthesis of 1-(2-(4-(cyclopropylsulfonyl)piperazin-1-yl)propyl)-5-formyl-4-methyl-1H-indole-2-carbonitrile (P252)

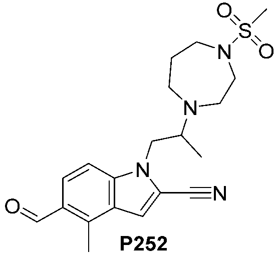

### Preparation 251. tert-Butyl 4-(1-methoxy-1-oxopropan-2-yl)-1,4-diazepane-1-carboxylate (P251)

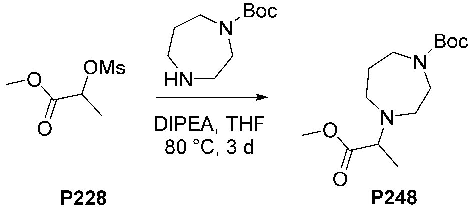

A solution of **P228** (11 g, 59.9 mmol) and *tert*-butyl 1,4-diazepane-1-carboxylate (10 g, 49.9 mmol) in anh. THF (171 ml) was added DIPEA (17.4 ml). The reaction was stirred at 80 °C for 3 days. The solution was treated with water and extracted with EtOAc. The combined organic layers were dried over Na₂SO₄, filtered, and concentrated to give **P248** (15.3 g) as an orange oil which was used to next step without further purification. ¹H NMR (400 MHz, CDCl₃), δ: 3.68 (d, *J* = 2.0 Hz, 3H), 3.48-3.38(m, 4H), 2.85-2.58(m, 4H), 1.79-1.72 (m, 2H), 1.64 (br. s, 1H), 1.44 (s, 9H), 1.30-1.28 (m, 3H).

### Preparation 249. Methyl 2-(1,4-diazepan-1-yl)propanoate hydrochloride (P249)

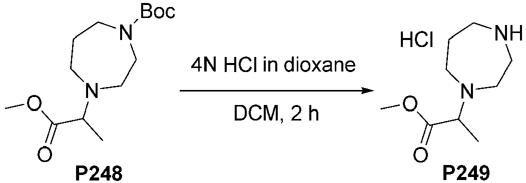

A solution of **P248** (15.3 g, 53.4 mmol) in dichloromethane (46 ml) was added a solution of 4N HCl in dioxane (46 ml). The mixture was stirred at rt for 2 h. The solution was extracted with water. The combined water layers were concentrated to give **P249** (11.4 g) as an orange oil which was used in next step without further purification. ¹H NMR (400 MHz, CD₃OD), δ: 4.53 (q, *J* = 7.2 Hz, 1H), 3.94-3.89 (m, 2H), 3.88 (s, 3H), 3.78-3.74 (m, 2H), 3.72-3.64 (m, 2H), 3.52-3.50 (m, 2H), 2.43- 2.37 (m, 2H), 1.70 (d, *J* = 7.2 Hz, 3H).

### Preparation 250. Methyl 2-(4-(methylsulfonyl)-1,4-diazepan-1-yl)propanoate (P250)

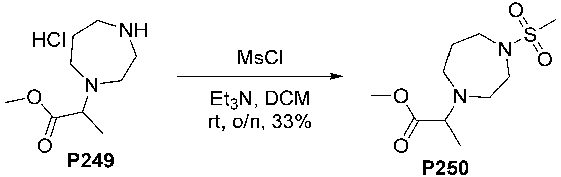

To a solution of **P249** (1.00 g, 5.37 mmol) in anh. DCM (13 ml) was added triethylamine (2.40 ml, 17.2 mmol) in ice bath. The mixture was stirred in ice bath for 15 min, and then methanesulfonyl chloride (0.50 ml, 6.4 mmol) was added to the mixture. After the reaction was stirred at rt for 16 h, the mixture was treated with water and extracted with DCM. The combined organic layers were washed with brine, dried over MgSO₄₍ₛ₎, filtered and concentrated. The residue was purified by silica gel chromatography (0-50 % EtOAc in *n-*hexane) to give **P250** (517 mg) as a colorless liquid. ¹H NMR (400 MHz, CDCl₃), δ: 3.71 (s, 3H), 3.54-3.31 (m, 5H), 2.96-2.73 (m, 7H), 1.93-1.75 (m, 2H), 1.30 (d, *J* = 7.2 Hz, 1H); LCMS (ESI) [MH]⁺: 265.1.

### Preparation 251. 2-(4-(Methylsulfonyl)-1,4-diazepan-1-yl)propan-1-ol (P251)

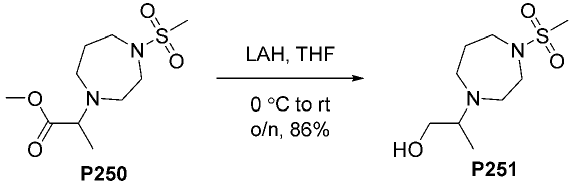

To a solution of **P250** (515 mg, 1.95 mmol) in anh. THF (5 ml) was added LAH (1M solution in THF, 2.9 ml) dropwise in ice bath. The mixture was stirred at rt for overnight. The mixture was quenched by H₂O (2.0 ml) and then 15% NaOH_{(aq)} (2.0 ml) in ice bath. The mixture was filtered through a pad of celite, and the filtrate was concentrated. The residue was purified by C18 reverse phase chromatography (0-100 % MeOH in H₂O) to give **P251** (394 mg) as a colorless gum. ¹H NMR (400 MHz, CDCl₃), δ: 3.53-3.34 (m, 5H), 3.28 (dd, *J* = 10.4, 10.8 Hz, 1H), 3.00-2.84 (m, 3H), 2.83 (s, 3H), 2.68-2.53 (m, 2H), 2.00-1.78 (m, 3H), 0.90 (d, *J* = 6.8 Hz, 3H); LCMS (ESI) [MH]⁺: 237.2.

### Preparation 252. 5-Formyl-4-methyl-1-(2-(4-(methylsulfonyl)-1,4-diazepan-1-yl)propyl)-1H-indole-2-carbonitrile (P252)

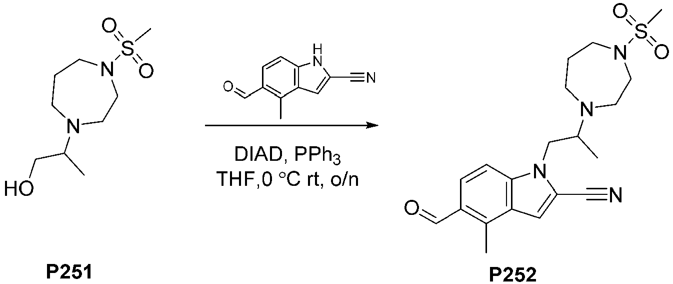

A solution of **P97** (567 mg, 3.07 mmol), **P251** (800 mg, 3.39 mmol), and triphenylphosphine (1210 mg, 4.61 mmol) in anh. THF (11 ml) was stirred in ice bath for 20 min. After diisopropyl azodicarboxylate (0.91 ml, 4.61 mmol) was added to the reaction dropwise in ice bath, the reaction was stirred at rt for overnight. The solution was concentrated to remove solvent. The residue was purified by silica-gel column chromatography (50% EtOAc in *n*-hexane, 25 % EtOAc in DCM, 30% EtOAc in *n-*hexane). The isolated product was treated with methanol and stirred for a period of time. The resulting solid was collected by filtration to give **P252** (258 mg, 19 %) as a white solid. ¹H NMR (400 MHz, CDCl₃), δ: 10.43 (s, 1H), 7.89 (d, *J* = 8.6 Hz, 1H), 7.37 (s, 1H), 7.26 (d, *J* = 8.6 Hz, 1H), 4.31 (dd, *J* = 8.4 Hz, 14.8 Hz, 1H), 4.11 (dd, *J* = 6.4 Hz, 14.8 Hz, 1H), 3.41-3.31 (m, 2H), 3.30-3.25 (m, 3H), 2.92 (s, 3H), 2.91-2.84 (m, 2H), 2.75 (s, 3H), 2.89-2.59 (m, 2H), 1.79-1.68 (m, 2H), 1.09 (d, *J* = 6.8 Hz, 3H); LCMS (ESI) [MH]⁺: 403.2. HPLC purity: 98 %, *t*_{R} = 15.648 min.

In the **Table 7** presented certain examples of key intermediates.

**Table 7. Certain examples of key intermediates.**

| **#** | **Structure** | **IUPAC Name** | **[MH]⁺ Calc.** | **[MH]⁺ Found** |
|---|---|---|---|---|
| **P7** | 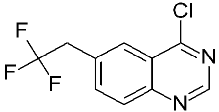 | 4-chloro-6-(2,2,2-trifluoroethyl)quinazoline | 247.03 | 247 |
| **P17** | 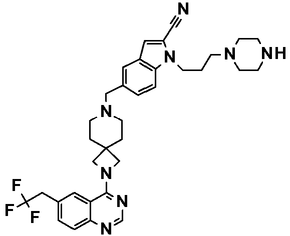 | 1-(3-piperazin-1-ylpropyl)-5-[[2-[6-(2,2,2-trifluoroethyl)quinazolin-4-yl]-2,7-diazaspiro[3.5]nonan-7-yl]methyl]indole-2-carbonitrile | 617.33 | 617 |
| **P20** | 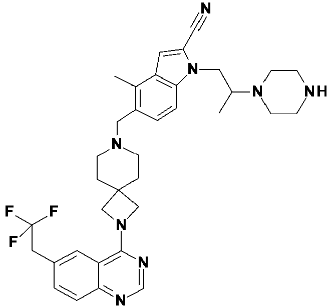 | 4-methyl-1-(2-piperazin-1-ylpropyl)-5-[[2-[6-(2,2,2-trifluoroethyl)quinazolin-4-yl]-2,7-diazaspiro[3.5]nonan-7-yl]methyl]indole-2-carbonitrile | 631.35 | 631 |
| **P24** | 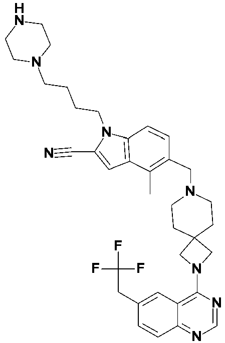 | 4-methyl-1-(4-piperazin-1-ylbutyl)-5-[[2-[6-(2,2,2-trifluoroethyl)quinazolin-4-yl]-2,7-diazaspiro[3.5]nonan-7-yl]methyl]indole-2-carbonitrile | 645.36 | 645 |
| **P26** | 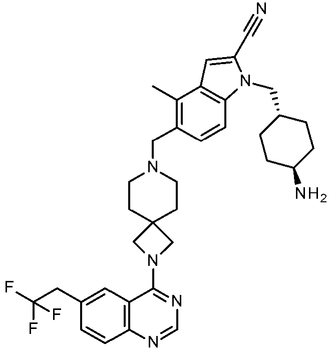 | 1-[(4-aminocyclohexyl)methyl]-4-methyl-5-[[2-[6-(2,2,2-trifluoroethyl)quinazolin-4-yl]-2,7-diazaspiro[3.5]nonan-7-yl]methyl]indole-2-carbonitrile | 616.338 | 616 |
| **P101** | 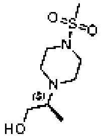 | (S)-2-(4-(Methylsulfonyl)piperazin-1-yl)propan-1-ol | 223 | 223.1 |
| **P107** | 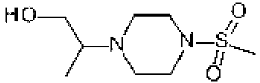 | 2-(4-(Methylsulfonyl)piperazin-1-yl)propan-1-ol | - | - |
| **P165** | 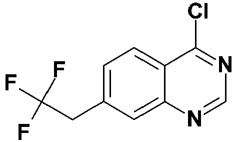 | 4-chloro-7-(2,2,2-trifluoroethyl)quinazoline | 247.03 | 247 |
| **P192** | 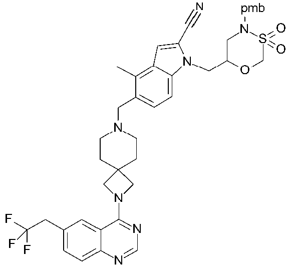 | 1-{[4-(4-Methoxybenzyl)-3,3-dioxido-1,3,4-oxathiazinan-6-yl]methyl}-4-methyl-5-({2-[6-(2,2,2-trifluoroethyl)quinazolin-4-yl]-2,7-diazaspiro[3.5]non-7-yl}methyl)-1H-indole-2-carbonitrile | | 775 |
| **P205** | 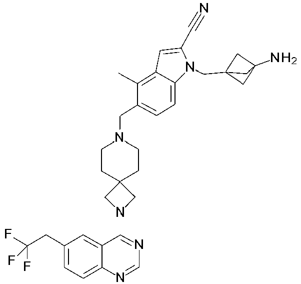 | 1-[(3-aminobicyclo[1.1.1]pent-1-yl)methyl]-4-methyl-5-({2-[6-(2,2,2-trifluoroethyl)quinazolin-4-yl]-2,7-diazaspiro[3.5]non-7-yl}methyl)-1H-indole-2-carbonitrile | 600.306 | 601 |
| **P228** | 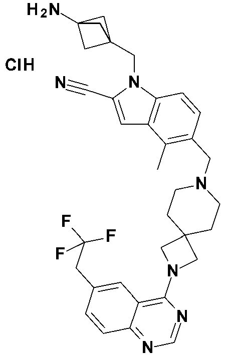 | 1-[(3-amino-1-bicyclo[1.1.1]pentanyl)methyl]-4-methyl-5-[[2-[6-(2,2,2-trifluoroethyl)quinazolin-4-yl]-2,7-diazaspiro[3.5]nonan-7-yl]methyl]indole-2-carbonitrile hydrochloride | 600.306 | 600 |
| **P229** | 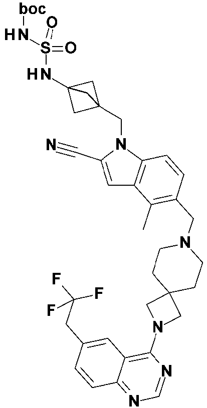 | *tert*-butyl *N*-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]sulfamo yl]carbamate | 779.331 | 779 |
| **P230** | 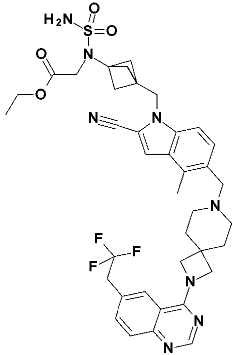 | ethyl 2-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]-sulfamoyl-amino] acetate | 765.316 | 765 |

### Examples of the Final Compound

**Table 8** presents certain non-limiting examples of a compound of Formula (I).

**Table 8. Selected examples of a compound of Formula (I)**

| **#** | **Structure** | **IUPAC Name** | **[MH]⁺ Calc.** | **[MH]⁺ Found** |
|---|---|---|---|---|
| **1** | 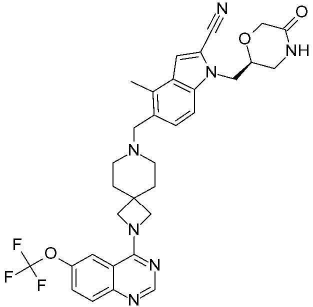 | 4-methyl-1-[[(2S)-5-oxomorpholin-2-yl]methyl]-5-[[2-[6-(trifluoromethoxy)quinazolin-4-yl]-2,7-diazaspiro[3.5]nonan-7-yl]methyl]indole-2-carbonitrile | 620.26 | 620 |
| | | **Example 3** | | |
| **2** | 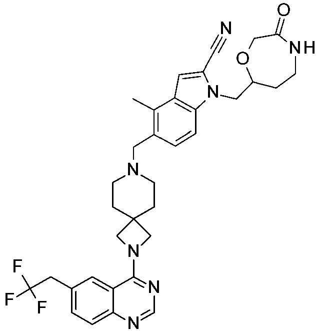 | 4-methyl-1-[(3-oxo-1,4-oxazepan-7-yl)methyl]-5-[[2-[6-(2,2,2-trifluoroethyl)quinazolin-4-yl]-2,7-diazaspiro[3.5]nonan-7-yl]methyl]indole-2-carbonitrile | 632.296 | 632 |
| | | **Example 4** | | |
| **3** | 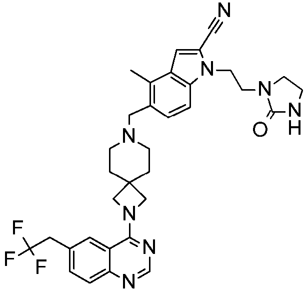 | 4-methyl-1-[2-(2-oxoimidazolidin-1-yl)ethyl]-5-[[2-[6-(2,2,2-trifluoroethyl)quinazolin-4-yl]-2,7-diazaspiro[3.5]nonan-7-yl]methyl]indole-2-carbonitrile | 617.296 | 617 |
| | | **Example 5** | | |
| **4** | 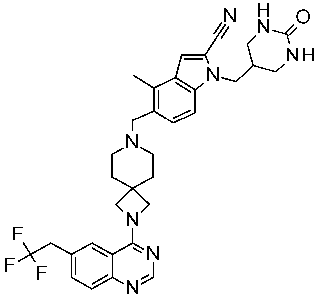 | 4-methyl-1-[(2-oxohexahydropyrimidin-5-yl)methyl]-5-[[2-[6-(2,2,2-trifluoroethyl)quinazolin-4-yl]-2,7-diazaspiro[3.5]nonan-7-yl]methyl]indole-2-carbonitrile | 617.296 | 617 |
| | | **Example 6** | | |
| **5** | 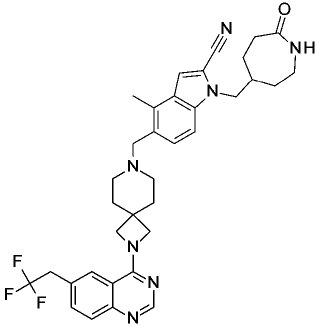 | 4-methyl-1-[(7-oxoazepan-4-yl)methyl]-5-[[2-[6-(2,2,2-trifluoroethyl)quinazolin-4-yl]-2,7-diazaspiro[3.5]nonan-7-yl]methyl]indole-2-carbonitrile | 630.317 | 630 |
| | | **Example 7** | | |
| **6** | 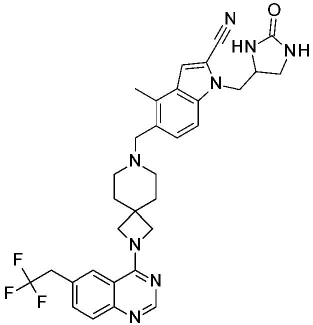 | 4-methyl-1-[(2-oxoimidazolidin-4-yl)methyl]-5-[[2-[6-(2,2,2-trifluoroethyl)quinazolin-4-yl]-2,7-diazaspiro[3.5]nonan-7-yl]methyl]indole-2-carbonitrile | 603.281 | 603 |
| | | **Example 8** | | |
| **7** | 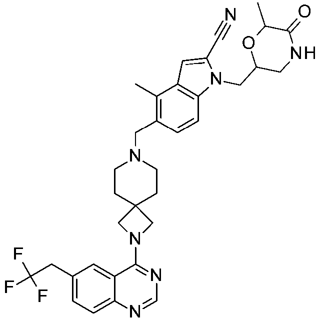 | 4-methyl-1-[(6-methyl-5-oxo-morpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile | 632.296 | 632 |
| | | **Example 11** | | |
| **8** | 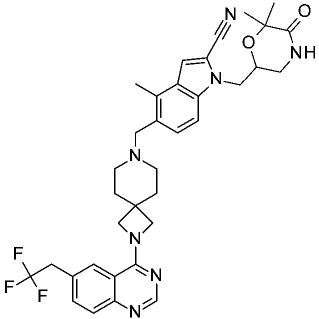 | 1-[(6,6-dimethyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile | 646.312 | 646 |
| | | **Example 12** | | |
| **9** | 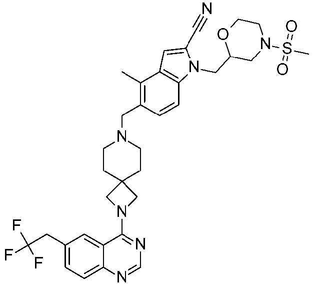 | 4-methyl-1-[(4-methylsulfonylmorpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile | 682.28 | 682 |
| | | **Example 13** | | |
| **10** | 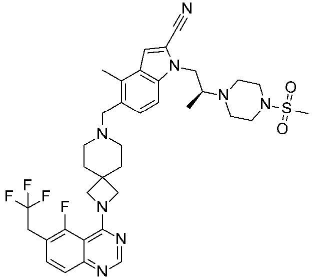 | 5-[[2-[5-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile | 727.317 | 727 |
| | | **Example 14** | | |
| **11** | 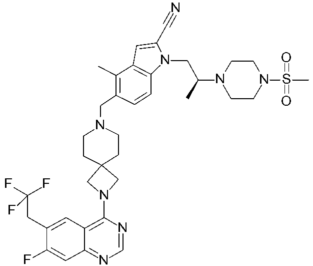 | 5-[[2-[7-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile | 727.317 | 727 |
| | | **Example 16** | | |
| **12** | 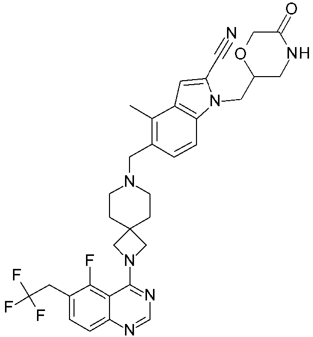 | 5-[[2-[5-fluoro-6-(2,2,2-trifluoroethyl)quinazolin-4-yl]-2,7-diazaspiro[3.5]nonan-7-yl]methyl]-4-methyl-1-[(5-oxomorpholin-2-yl)methyl]indole-2-carbonitrile | 636.271 | 636 |
| | | **Example 35** | | |
| **13** | 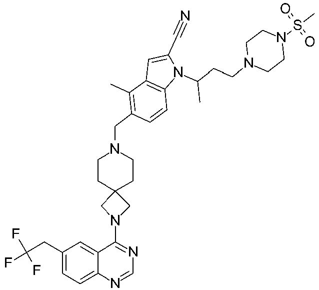 | 4-methyl-1-[1-methyl-3-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 723.342 | 723 |
| | | **Example 36** | | |
| **14** | 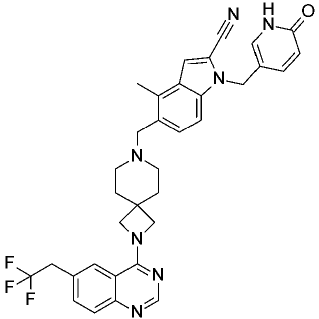 | 4-methyl-1-[(6-oxo-1H-pyridin-3-yl)methyl]-5-[[2-[6-(2,2,2-trifluoroethyl)quinazolin-4-yl]-2,7-diazaspiro[3.5]nonan-7-yl]methyl]indole-2-carbonitrile | 612.27 | 613 |
| | | **Example 37** | | |
| **15** | 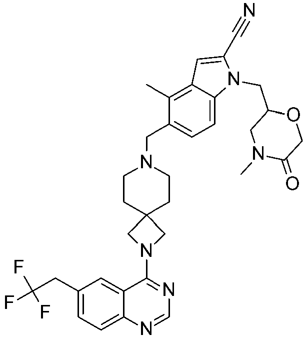 | 4-methyl-1-[(4-methyl-5-oxo-morpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile | 632.296 | 633 |
| | | **Example 38** | | |
| **16** | 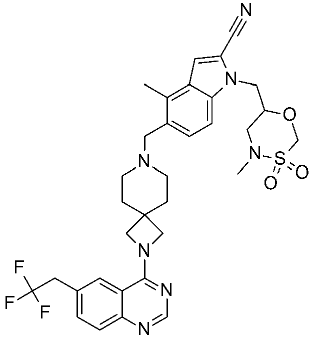 | 4-methyl-1-[(4-methyl-3,3-dioxo-1,3,4-oxathiazinan-6-yl)methyl]-5-[[2-[6-(2,2,2-trifluoroethyl)quinazolin-4-yl]-2,7-diazaspiro[3.5]nonan-7-yl]methyl]indole-2-carbonitrile | 668.263 | 668 |
| | | **Example 39** | | |
| **17** | 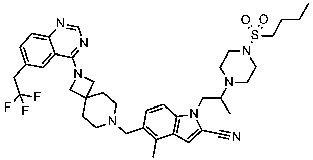 | 1-[2-(4-butylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile | 751.37 | 751 |
| **18** | 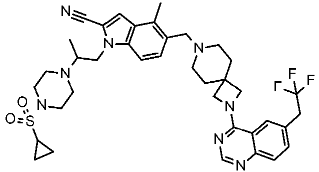 | 1-[2-(4-cyclopropylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile | 735.34 | 735 |
| **19** | 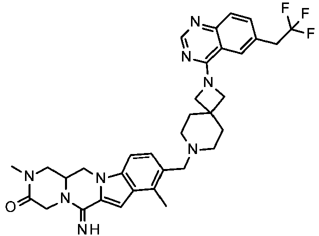 | 2-imino-6,15-dimethyl-14-[[2-[6-(2,2,2-trifluoroethyl)quinazolin-4-yl]-2,7-diazaspiro [3.5] nonan-7-yl]methyl]-3,6,10-triazatetracyclo[8.7.0.03,8.011,16]heptadec a-1(17),11(16),12,14-tetraen-5-one | 631.31 | 631 |
| **20** | 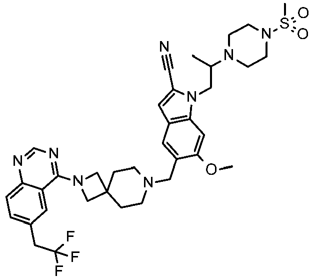 | 6-methoxy-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 725.32 | 725 |
| | | **Example 30** | | |
| **21** | 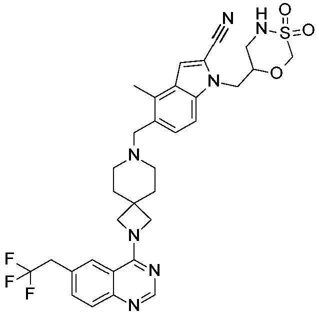 | 1-[(3,3-dioxo-1,3,4-oxathiazinan-6-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile | 654.247 | 654 |
| | | **Example 40** | | |
| **22** | 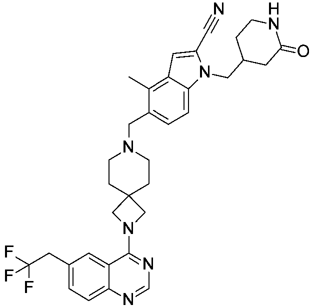 | 4-methyl-1-[(2-oxo-4-piperidyl)methyl]-5-[[2-[6-(2,2,2-trifluoroethyl)quinazolin-4-yl]-2,7-diazaspiro[3.5]nonan-7-yl]methyl]indole-2-carbonitrile | 616.301 | 617 |
| | | **Example 41** | | |
| **23** | 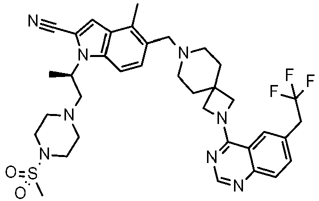 | 4-methyl-1-[(1R)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile | 709.33 | 709 |
| | | **Example 20** | | |
| **24** | 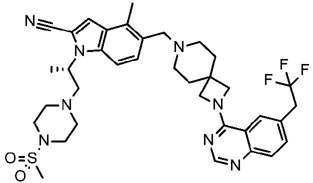 | 4-methyl-1-[(1S)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile | 709.33 | 709 |
| | | **Example 20** | | |
| **25** | 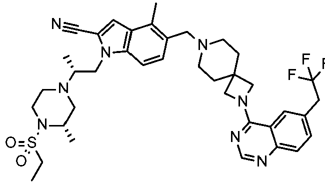 | 1-[(2R)-2-[(3S)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile | 737.36 | 737 |
| **26** | 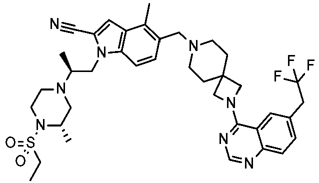 | 1-[(2S)-2-[(3S)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile | 737.36 | 738 |
| | | **Example 49** | | |
| **27** | 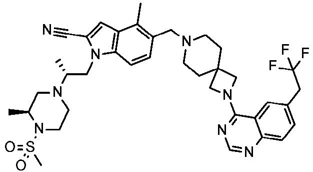 | 4-methyl-1-[(2R)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile | 723.34 | 724 |
| **28** | 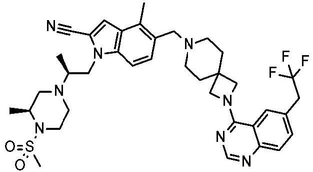 | 4-methyl-1-[(2S)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile | 723.34 | 724 |
| **29** | 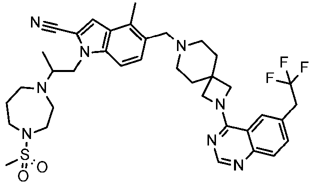 | 4-methyl-1-[2-(4-methylsulfonyl-1,4-diazepan-1-yl)propyl]-5-[[2-[6-(2,2,2-trifluoroethyl)quinazolin-4-yl]-2,7-diazaspiro[3.5]nonan-7-yl]methyl]indole-2-carbonitrile | 723.34 | 723 |
| **30** | 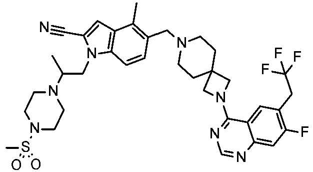 | 5-[[2-[7-fluoro-6-(2,2,2-trifluoroethyl)quinazolin-4-yl]-2,7-diazaspiro[3.5]nonan-7-yl]methyl]-4-methyl-1-[2-(4-methylsulfonylpiperazin-1-yl)propyl]indole-2-carbonitrile | 727.32 | 727 |
| **31** | 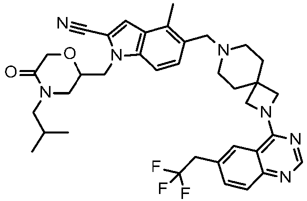 | 1-[(4-isobutyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile | 674.34 | 674 |
| | | **Example 52** | | |
| **32** | 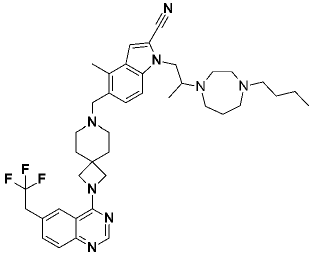 | 1-[2-(4-butyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile | 701.427 | 701 |
| **33** | 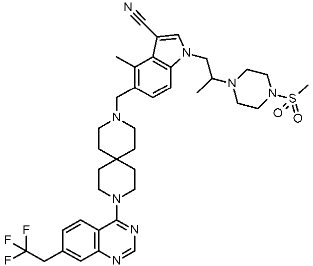 | 4-methyl-1-[2-(4-methylsulfonylpiperazin-1-yl)propyl]-5-[[3-[7-(2,2,2-trifluoroethyl)quinazolin-4-yl]-3,9-diazaspiro[5.5]undecan-9-yl]methyl]indole-3-carbonitrile | 757.357 | 757 |
| **34** | 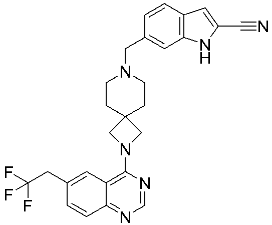 | 6-((2-(6-(2,2,2-trifluoroethyl)quinazolin-4-yl)-2,7-diazaspiro[3.5]nonan-7-yl)methyl)-1H-indole-2-carbonitrile | 491.22 | 491 |
| **35** | 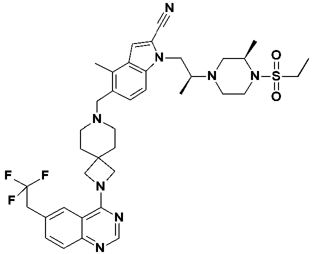 | 1-[(2S)-2-[(3R)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile | 737.36 | 738 |
| | | **Example 49** | | |
| **36** | 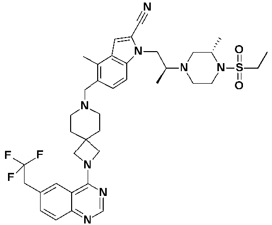 | 1-[(2S)-2-[(3S)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile | 737.36 | 738 |
| | | **Example 49** | | |
| **37** | 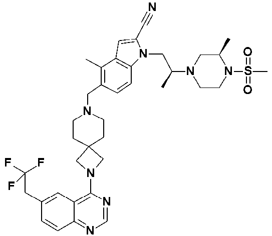 | 4-methyl-1-[(2R)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile | 723.342 | 724 |
| | | **Example 48** | | |
| **38** | 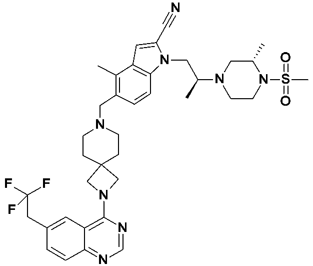 | 4-methyl-1-[(2S)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile | 723.342 | 724 |
| | | **Example 48** | | |
| **39** | 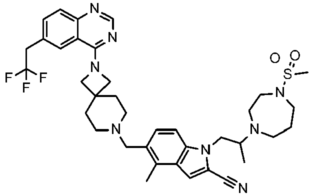 | 4-methyl-1-[2-(4-methylsulfonyl-1,4-diazepan-1-yl)propyl]-5-[[2-[6-(2,2,2-trifluoroethyl)quinazolin-4-yl]-2,7-diazaspiro[3.5]nonan-7-yl]methyl]indole-2-carbonitrile | 723.342 | 723 |
| | | **Example 50** | | |
| **40** | 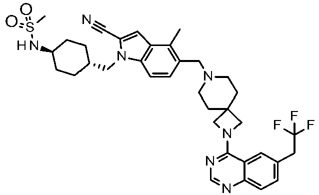 | N-(1R,4R)-[4-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]cyclohexyl]methanesulfonamide | 694.32 | 694 |
| | | **Example 26** | | |
| **41** | 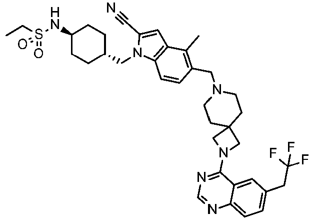 | *N*-(1R,4R)-[4-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]cyclohexyl]ethanesulfonamide | 708.33 | 708 |
| | | **Example 27** | | |
| **42** | 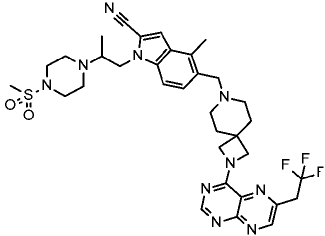 | 4-methyl-1-[2-(4-methylsulfonylpiperazin-1-yl)propyl]-5-[[2-[6-(2,2,2-trifluoroethyl)pteridin-4-yl]-2,7-diazaspiro[3.5]nonan-7-yl]methyl]indole-2-carbonitrile | 711.32 | 711.8 |
| | | **Example 32** | | |
| **43** | 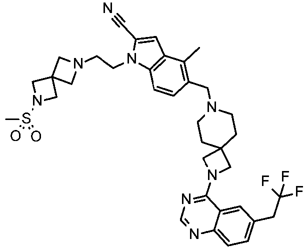 | 4-methyl-1-[2-(2-methylsulfonyl-2,6-diazaspiro[3.3]heptan-6-yl)ethyl]-5-[[2-[6-(2,2,2-trifluoroethyl)quinazolin-4-yl]-2,7-diazaspiro[3.5]nonan-7-yl]methyl]indole-2-carbonitrile | 707.31 | 707 |
| **44** | 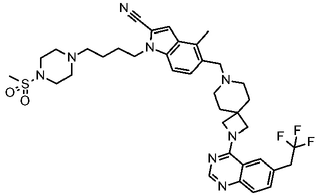 | 4-methyl-1-[4-(4-methylsulfonylpiperazin-1-yl)butyl]-5-[[2-[6-(2,2,2-trifluoroethyl)quinazolin-4-yl]-2,7-diazaspiro[3.5]nonan-7-yl]methyl]indole-2-carbonitrile | 723.34 | 723 |
| | | **Example 25** | | |
| **45** | 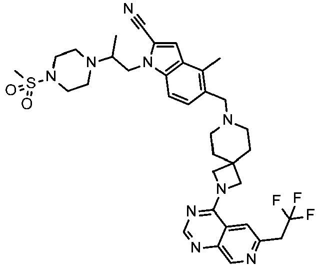 | 4-methyl-1-[2-(4-methylsulfonylpiperazin-1-yl)propyl]-5-[[2-[6-(2,2,2-trifluoroethyl)pyrido[3,4-d]pyrimidin-4-yl]-2,7-diazaspiro[3.5]nonan-7-yl]methyl]indole-2-carbonitrile | 710.32 | 710 |
| | | **Example 34** | | |
| **46** | 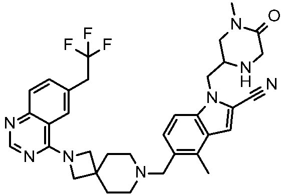 | 4-methyl-1-[(4-methyl-5-oxo-piperazin-2-yl)methyl]-5-[[2-[6-(2,2,2-trifluoroethyl)quinazolin-4-yl]-2,7-diazaspiro[3.5]nonan-7-yl]methyl]indole-2-carbonitrile | 631.31 | 631 |
| **47** | 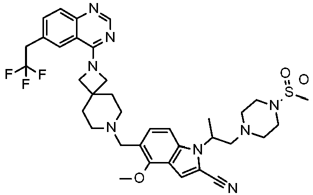 | 4-methoxy-1-[1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile | 725.321 | 725 |
| | | **Example 29** | | |
| **48** | 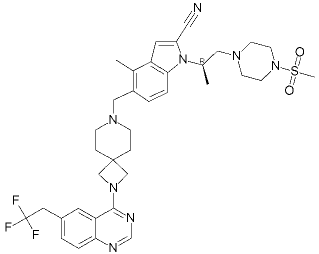 | 4-methyl-1-[(1R)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile | 709.33 | 709 |
| **49** | 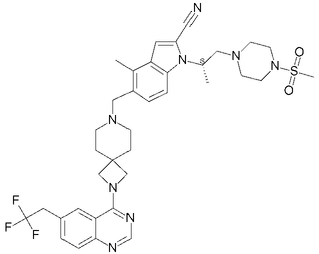 | 4-methyl-1-[(1S)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile | 709.33 | 709 |
| **50** | 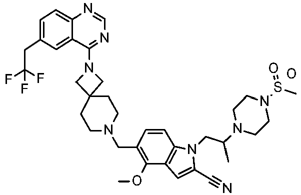 | 4-methoxy-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 725.321 | 725 |
| | | **Example 29** | | |
| **51** | 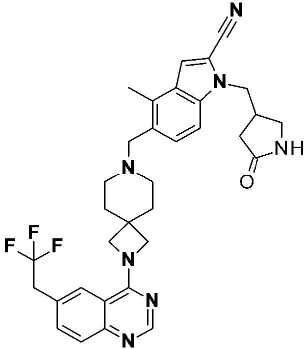 | 4-methyl-1-[(5-oxopyrrolidin-3-yl)methyl]-5-[[2-[6-(2,2,2-trifluoroethyl)quinazolin-4-yl]-2,7-diazaspiro[3.5]nonan-7-yl]methyl]indole-2-carbonitrile | 602.286 | 602 |
| | | **Example 58** | | |
| **52** | 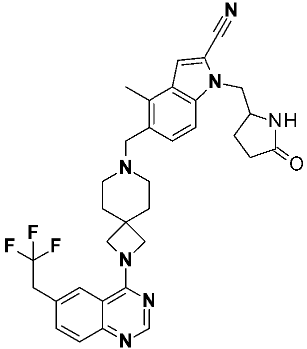 | 4-methyl-1-[(5-oxopyrrolidin-2-yl)methyl]-5-[[2-[6-(2,2,2-trifluoroethyl)quinazolin-4-yl]-2,7-diazaspiro[3.5]nonan-7-yl]methyl]indole-2-carbonitrile | 602.286 | 602 |
| | | **Example 59** | | |
| **53** | 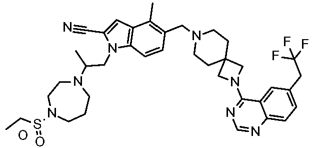 | 1-[2-(4-ethylsulfonyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile | 737.36 | 737 |
| | | **Example 55** | | |
| **54** | 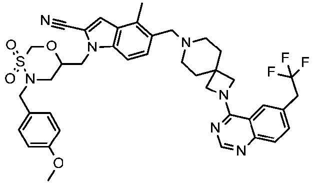 | 1-[[4-[(4-methoxyphenyl)methyl]-3,3-dioxo-1,3,4-oxathiazinan-6-yl]methyl]-4-methyl-5-[[2-[6-(2,2,2-trifluoroethyl)quinazolin-4-yl]-2,7-diazaspiro[3.5]nonan-7-yl]methyl]indole-2-carbonitrile | 774.31 | 774 |
| **55** | 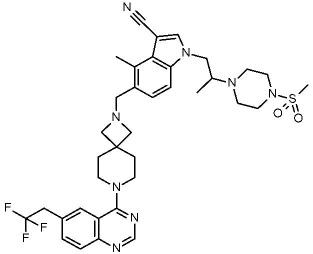 | 4-methyl-1-[2-(4-methylsulfonylpiperazin-1-yl)propyl]-5-[[7-[6-(2,2,2-trifluoroethyl)quinazolin-4-yl]-2,7-diazaspiro[3.5]nonan-2-yl]methyl]indole-3-carbonitrile | 709.326 | 709 |
| **56** | 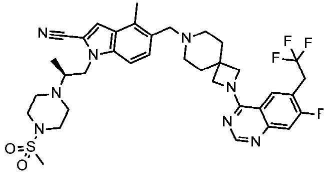 | 5-[[2-[7-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile | 727.32 | 727 |
| **57** | 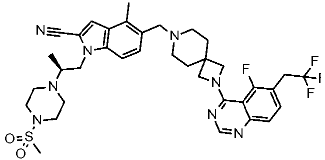 | 5-[[2-[5-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile | 727.32 | 727 |
| **58** | 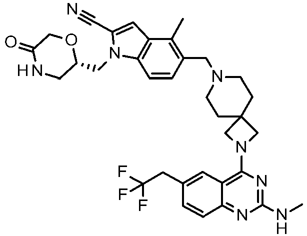 | 4-methyl-5-[[2-[2-(methylamino)-6-(2,2,2-trifluoroethyl)quinazolin-4-yl]-2,7-diazaspiro[3.5]nonan-7-yl]methyl]-1-[[(2S)-5-oxomorpholin-2-yl]methyl]indole-2-carbonitrile | 647.307 | 647 |
| **59** | 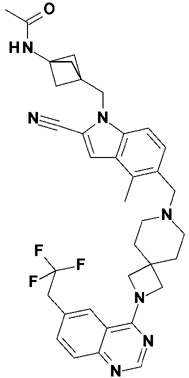 | N-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]acetamide | 642.317 | 642 |
| | | **Example 60** | | |
| **60** | 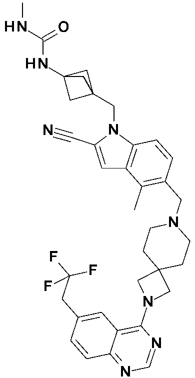 | 1-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]-3-methyl-urea | 657.328 | 657 |
| | | **Example 61** | | |
| **61** | 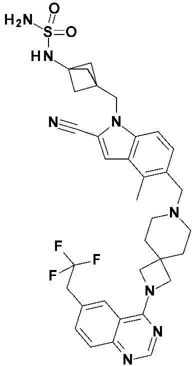 | 4-[7-[[2-cyano-4-methyl-1-[[3-(sulfamoylamino)-1-bicyclo[1.1.1]pentanyl]methyl]indol-5-yl]methyl]-2,7-diazaspiro[3.5]nonan-2-yl]-6-(2,2,2-trifluoroethyl)quinazoline | 679.279 | 679 |
| | | **Example 62** | | |
| **62** | 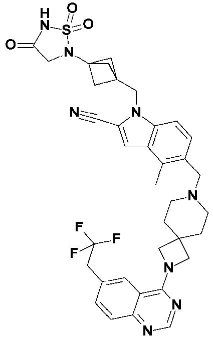 | 4-methyl-5-[[2-[6-(2,2,2-trifluoroethyl)quinazolin-4-yl]-2,7-diazaspiro[3.5]nonan-7-yl]methyl]-1-[[3-(1,1,4-trioxo-1,2,5-thiadiazolidin-2-yl)-1-bicyclo[1.1.1]pentanyl]methyl]indole-2-carbonitrile | 719.274 | 719 |
| | | **Example 63** | | |
| **63** | 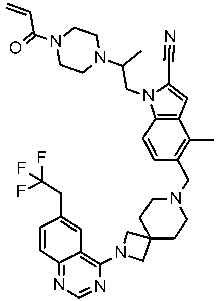 | 4-methyl-1-[2-(4-prop-2-enoylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 685.36 | 685 |
| | | **Example 23** | | |
| **64** | 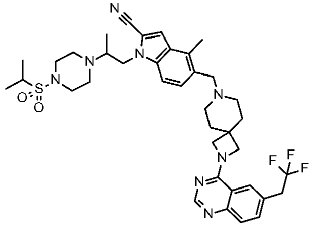 | 1-[2-(4-isopropylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile | 737.36 | 737 |
| **65** | 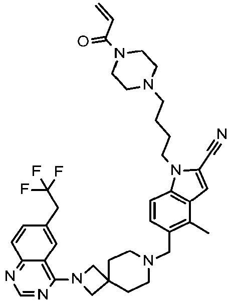 | 4-methyl-1-[4-(4-prop-2-enoylpiperazin-1-yl)butyl]-5-[[2-[6-(2,2,2-trifluoroethyl)quinazolin-4-yl]-2,7-diazaspiro[3.5]nonan-7-yl]methyl]indole-2-carbonitrile | 699.37 | 699 |
| | | **Example 24** | | |
| **66** | 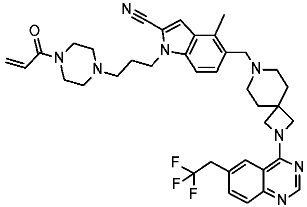 | 4-methyl-1-[3-(4-prop-2-enoylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 685.36 | 685 |
| **67** | 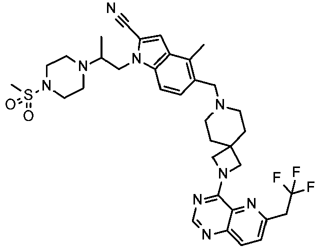 | 4-methyl-1-[2-(4-methylsulfonylpiperazin-1-yl)propyl]-5-[[2-[6-(2,2,2-trifluoroethyl)pyrido[3,2-d]pyrimidin-4-yl]-2,7-diazaspiro[3.5]nonan-7-yl]methyl]indole-2-carbonitrile | 710.32 | 710.8 |
| | | **Example 33** | | |
| **68** | 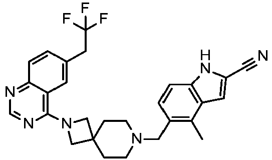 | 4-methyl-5-[[2-[6-(2,2,2-trifluoroethyl)quinazolin-4-yl]-2,7-diazaspiro[3.5]nonan-7-yl]methyl]-1H-indole-2-carbonitrile | 505.233 | 505 |
| | | **Example 9** | | |
| **69** | 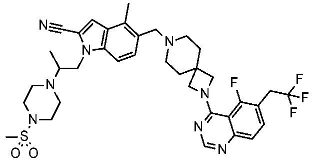 | 5-[[2-[5-fluoro-6-(2,2,2-trifluoroethyl)quinazolin-4-yl]-2,7-diazaspiro[3.5]nonan-7-yl]methyl]-4-methyl-1-[2-(4-methylsulfonylpiperazin-1-yl)propyl]indole-2-carbonitrile | 727.32 | 727 |
| **70** | 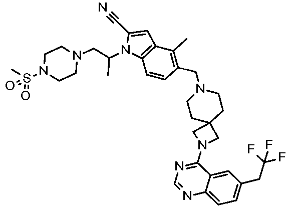 | 4-methyl-1-[1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile | 709.326 | 709 |
| | | **Example 19** | | |
| **71** | 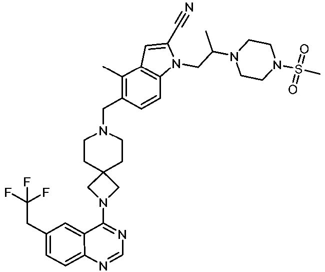 | 4-methyl-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 709.326 | 709 |
| | | **Example 19** | | |
| **72** | 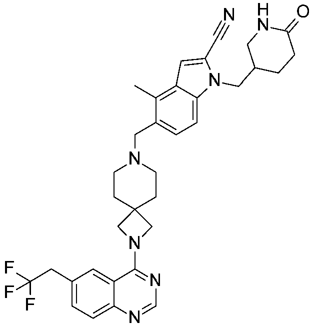 | 4-methyl-1-[(6-oxo-3-piperidyl)methyl]-5-[[2-[6-(2,2,2-trifluoroethyl)quinazolin-4-yl]-2,7-diazaspiro[3.5]nonan-7-yl]methyl]indole-2-carbonitrile | 616.301 | 617 |
| | | **Example 42** | | |
| **73** | 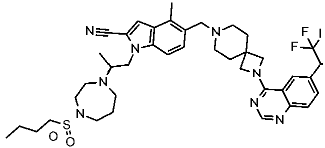 | 1-[2-(4-butylsulfonyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile | 765.39 | 765 |
| | | **Example 54** | | |
| **74** | 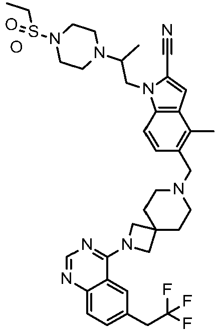 | 1-[2-(4-ethylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile | 723.34 | 723 |
| **75** | 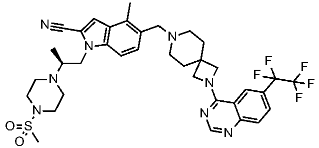 | 4-methyl-1-[(2S)-2-(4-methylsulfonylpiperazin-1-yl)propyl]-5-[[2-[6-(1,1,2,2,2-pentafluoroethyl)quinazolin-4-yl]-2,7-diazaspiro[3.5]nonan-7-yl]methyl]indole-2-carbonitrile | 745.31 | 745 |
| | | **Example 56** | | |
| **76** | 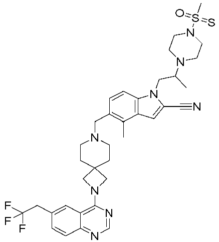 | 4-methyl-1-(2-(4-(methylsulfonothioyl)piperazin-1-yl)propyl)-5-((2-(6-(2,2,2-trifluoroethyl)quinazolin-4-yl)-2,7-diazaspiro[3.5]nonan-7-yl)methyl)-1H-indole-2-carbonitrile | 725.30 | 725 |
| **77** | 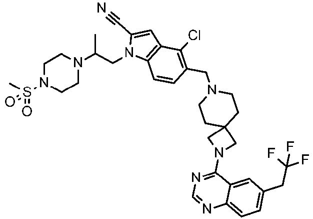 | 4-chloro-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 729.27 | 729 |
| **78** | 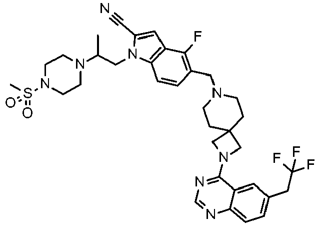 | 4-fluoro-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 713.30 | 713 |
| **79** | 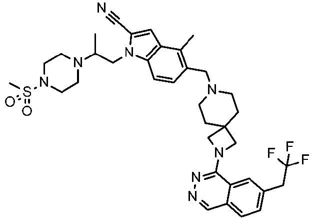 | 4-methyl-1-[2-(4-methylsulfonylpiperazin-1-yl)propyl]-5-[[2-[7-(2,2,2-trifluoroethyl)phthalazin-1-yl]-2,7-diazaspiro[3.5]nonan-7-yl]methyl]indole-2-carbonitrile | 709.33 | 709 |
| | | **Example 21** | | |
| **80** | 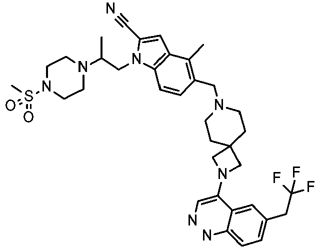 | 4-methyl-1-[2-(4-methylsulfonylpiperazin-1-yl)propyl]-5-[[2-[6-(2,2,2-trifluoroethyl)cinnolin-4-yl]-2,7-diazaspiro[3.5]nonan-7-yl]methyl]indole-2-carbonitrile | 709.33 | 709 |
| | | **Example 22** | | |
| **81** | 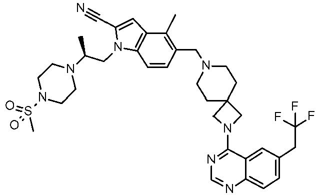 | 4-methyl-1-[(2S)-2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 709.33 | 709 |
| **82** | 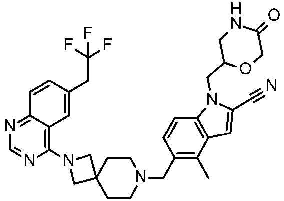 | 4-methyl-1-[(5-oxomorpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile | 618.28 | 618 |
| | | **Example 28** | | |
| **83** | 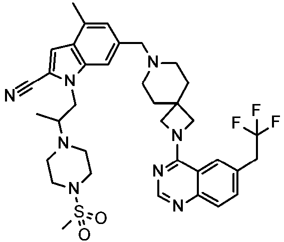 | 4-methyl-1-[2-(4-methylsulfonylpiperazin-1-yl)propyl]-6-[[2-[6-(2,2,2-trifluoroethyl)quinazolin-4-yl]-2,7-diazaspiro[3.5]nonan-7-yl]methyl]indole-2-carbonitrile | 709.33 | 709 |
| | | **Example 31** | | |
| **84** | 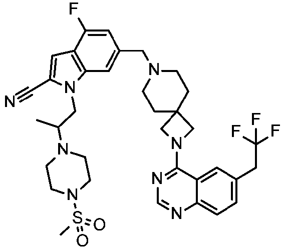 | 4-fluoro-1-[2-(4-methylsulfonylpiperazin-1-yl)propyl]-6-[[2-[6-(2,2,2-trifluoroethyl)quinazolin-4-yl]-2,7-diazaspiro[3.5]nonan-7-yl]methyl]indole-2-carbonitrile | 713.30 | 713 |
| **85** | 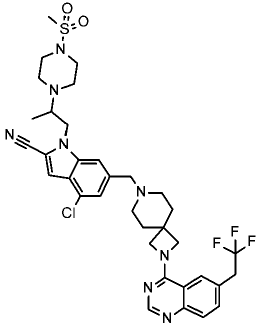 | 4-chloro-1-[2-(4-methylsulfonylpiperazin-1-yl)propyl]-6-[[2-[6-(2,2,2-trifluoroethyl)quinazolin-4-yl]-2,7-diazaspiro[3.5]nonan-7-yl]methyl]indole-2-carbonitrile | 729.27 | 729 |
| **86** | 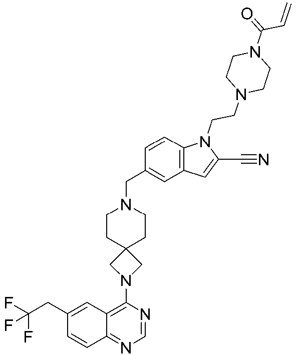 | 1-(2-(4-acryloylpiperazin-1-yl)ethyl)-5-((2-(6-(2,2,2-trifluoroethyl)quinazolin-4-yl)-2,7-diazaspiro[3.5]nonan-7-yl)methyl)-1H-indole-2-carbonitrile | 657.33 | 657 |
| **87** | 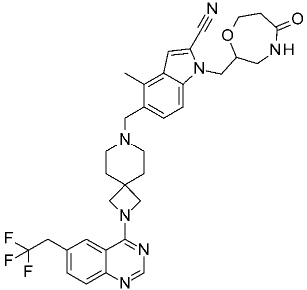 | 4-methyl-1-[(5-oxo-1,4-oxazepan-2-yl)methyl]-5-[[2-[6-(2,2,2-trifluoroethyl)quinazolin-4-yl]-2,7-diazaspiro[3.5]nonan-7-yl]methyl]indole-2-carbonitrile | 632.296 | 632 |
| | | **Example 47** | | |
| **88** | 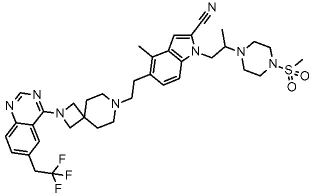 | 4-methyl-1-[2-(4-methylsulfonylpiperazin-1-yl)propyl]-5-[2-[2-[6-(2,2,2-trifluoroethyl)quinazolin-4-yl]-2,7-diazaspiro[3.5]nonan-7-yl]ethyl]indole-2-carbonitrile | 723.34 | 723 |
| **89** | 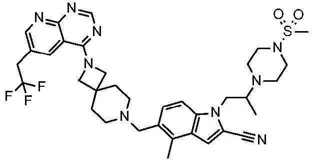 | 4-methyl-1-[2-(4-methylsulfonylpiperazin-1-yl)propyl]-5-[[2-[6-(2,2,2-trifluoroethyl)pyrido[2,3-d]pyrimidin-4-yl]-2,7-diazaspiro[3.5]nonan-7-yl]methyl]indole-2-carbonitrile | 710.32 | 710 |
| | | **Example 18** | | |
| **90** | 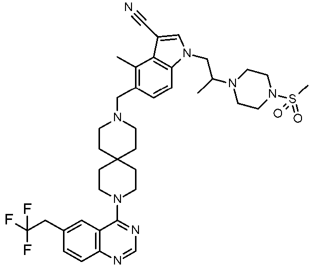 | 4-methyl-1-[2-(4-methylsulfonylpiperazin-1-yl)propyl]-5-[[3-[6-(2,2,2-trifluoroethyl)quinazolin-4-yl]-3,9-diazaspiro[5.5]undecan-9-yl]methyl]indole-3-carbonitrile | 737.357 | 737 |
| **91** | 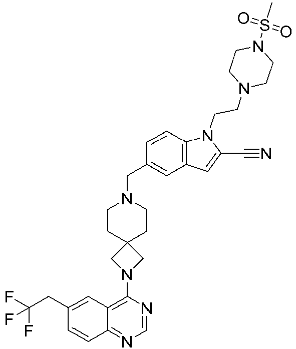 | 1-(2-(4-(methylsulfonyl)piperazin-1-yl)ethyl)-5-((2-(6-(2,2,2-trifluoroethyl)quinazolin-4-yl)-2,7-diazaspiro[3.5]nonan-7-yl)methyl)-1H-indole-2-carbonitrile | 681.29 | 681 |
| | | **Example 1** | | |
| **92** | 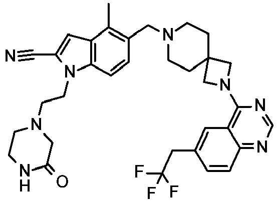 | 4-methyl-1-[2-(3-oxopiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile | 631.31 | 631 |
| **93** | 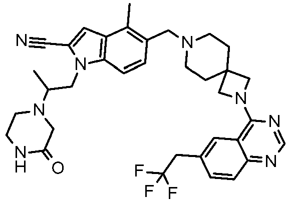 | 4-methyl-1-[2-(3-oxopiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile | 645.33 | 645 |
| | | **Example 53** | | |
| **94** | 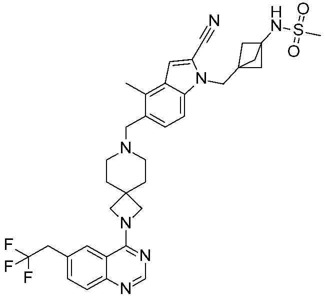 | N-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]methanesulfonamide | 678.284 | 678 |
| | | **Example 44** | | |
| **95** | 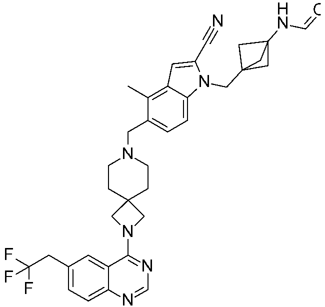 | N-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]formamide | 628.301 | 628 |
| | | **Example 43** | | |
| **96** | 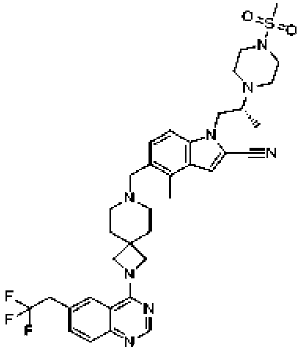 | rac-(R)-4-methyl-1-(2-(4-(methylsulfonyl)piperazin-1-yl)propyl)-5-((2-(6-(2,2,2-trifluoroethyl)quinazolin-4-yl)-2,7-diazaspiro[3.5]nonan-7-yl)methyl)-1H-indole-2-carbonitrile | 709.33 | 709 |
| | | **Example 17** | | |
| **97** | 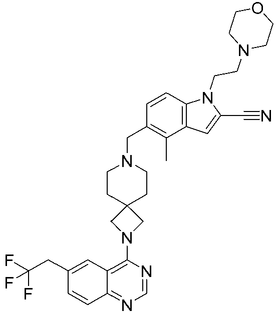 | 4-methyl-1-(2-morpholinoethyl)-5-((2-(6-(2,2,2-trifluoroethyl)quinazolin-4-yl)-2,7-diazaspiro[3.5]nonan-7-yl)methyl)-1H-indole-2-carbonitrile | 618.32 | 618 |
| | | **Example 15** | | |
| **98** | 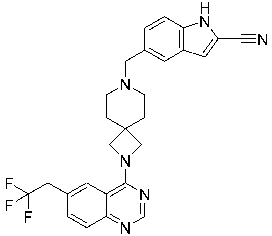 | 5-((2-(6-(2,2,2-trifluoroethyl)quinazolin-4-yl)-2,7-diazaspiro[3.5]nonan-7-yl)methyl)-1H-indole-2-carbonitrile | 491.22 | 491 |
| | | **Example 10** | | |
| **99** | 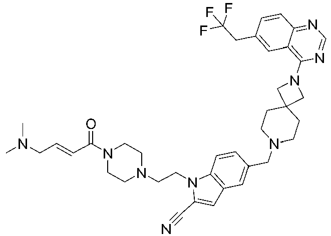 | (E)-1-(2-(4-(4-(dimethylamino)but-2-enoyl)piperazin-1-yl)ethyl)-5-((2-(6-(2,2,2-trifluoroethyl)quinazolin-4-yl)-2,7-diazaspiro[3.5]nonan-7-yl)methyl)-1H-indole-2-carbonitrile | 714.39 | 714 |
| | | **Example 2** | | |
| **100** | 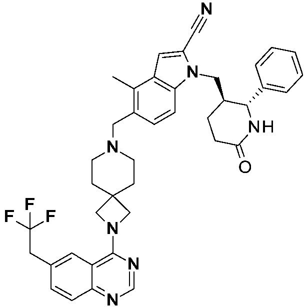 | 4-methyl-1-[[(2R,3S)-6-oxo-2-phenyl-3-piperidyl]methyl]-5-[[2-[6-(2,2,2-trifluoroethyl)quinazolin-4-yl]-2,7-diazaspiro[3.5]nonan-7-yl]methyl]indole-2-carbonitrile | 692.332 | 692 |
| | | **Example 57** | | |
| **101** | 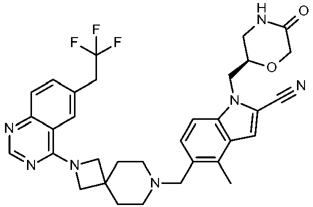 | 4-methyl-1-[[(2R)-5-oxomorpholin-2-yl]methyl]-5-[[2-[6-(2,2,2-trifluoroethyl)quinazolin-4-yl]-2,7-diazaspiro[3.5]nonan-7-yl]methyl]indole-2-carbonitrile | 618.28 | 618 |
| | | **Example 45** | | |
| **102** | 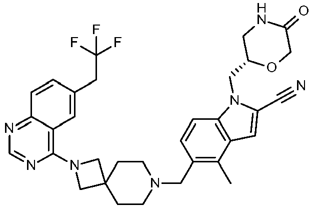 | 4-methyl-1-[[(2S)-5-oxomorpholin-2-yl]methyl]-5-[[2-[6-(2,2,2-trifluoroethyl)quinazolin-4-yl]-2,7-diazaspiro[3.5]nonan-7-yl]methyl]indole-2-carbonitrile | 618.28 | 618 |
| | | **Example 46** | | |
| **103** | 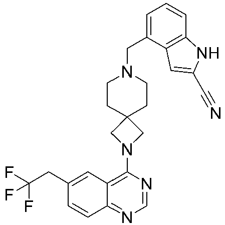 | 4-((2-(6-(2,2,2-trifluoroethyl)quinazolin-4-yl)-2,7-diazaspiro[3.5]nonan-7-yl)methyl)-1H-indole-2-carbonitrile | 491.22 | 491 |
| **104** | 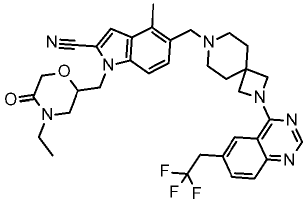 | 1-[(4-ethyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile | 646.31 | 646 |
| | | **Example 51** | | |
| **105** | 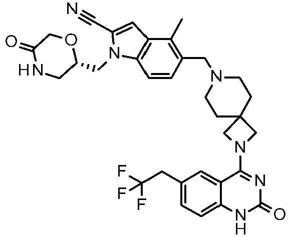 | 4-methyl-1-[[(2S)-5-oxomorpholin-2-yl]methyl]-5-[[2-[2-oxo-6-(2,2,2-trifluoroethyl)-1H-quinazolin-4-yl]-2,7-diazaspiro[3.5]nonan-7-yl]methyl]indole-2-carbonitrile | 634.275 | 634 |

### Synthesis of the Representative Examples of the compound

### Example 1. 1-{2-[4-(Methylsulfonyl)piperazin-1-yl]ethyl}-5-({2-[6-(2,2,2-trifluoroethyl)quinazolin-4-yl]-2,7-diazaspiro[3.5]non-7-yl}methyl)-1H-indole-2-carbonitrile (Compound 91)

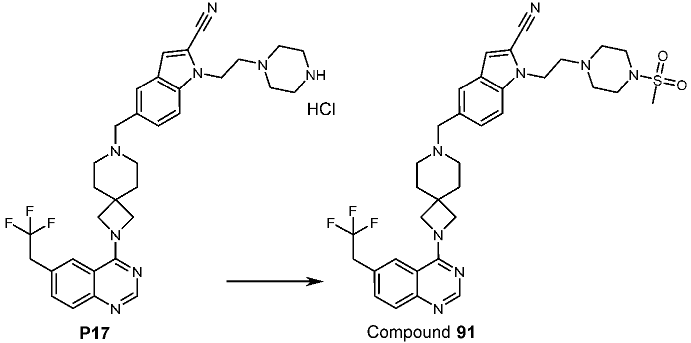

The mixture of 1-(2-piperazin-1-ylethyl)-5-({2-[6-(2,2,2-trifluoroethyl)quinazolin-4-yl]-2,7-diazaspiro[3.5]non-7-yl}methyl)-1*H*-indole-2-carbonitrile hydrochloride (**P17**, 60 mg, 0.094 mmol), ethanesulfonyl chloride (18 mg, 0.14 mmol) and DIPEA (97 mg, 0.75 mmol) in DCM (5 ml) was stirred at rt for 24 h (LCMS control). Then water solution of sodium bicarbonate was added with stirring. The mixture was stirred for 30 min, and layers were separated. The water phase was extracted with DCM (100 ml). Combined organic extract was concentrated and residue was purified by column chromatography on silica with ethyl acetate-methanol (10:2) to yield the Compound **91** (18 mg, 28 %). ¹H NMR (400 MHz, DMSO-*d₆*), δ: 8.43 (s, 1H), 7.96 (s, 1H), 7.73-7.68 (m, 2H), 7.65-7.55 (m, 2H), 7.45-7.34 (m, 2H), 4.40 (t, J=5.4 Hz, 2H), 4.20 (.s, 4H), 3.84 (q, J = 11.7 Hz, 2H), 3.54 (s, 2H), 3.04 (t, J = 4.9 Hz, 4H), 2.84 (s, 3H), 2.70 (t, J = 5.6 Hz, 2H), 2.53 (s, 2H), 2.65-2.47 (m, 4H), 2.37 (s, 2H), 1.83-1.72 (m, 4H). LCMS (ESI) [MH]⁺: 681.

### Example 2. 1-(2-{4-[(2E)-3-(Dimethylamino)prop-2-enoyl]piperazin-1-yl}ethyl)-5-({2-[6-(2,2,2-trifluoroethyl)quinazolin-4-yl]-2,7-diazaspiro[3.5]non-7-yl}methyl)-1H-indole-2-carbonitrile (Compound 99)

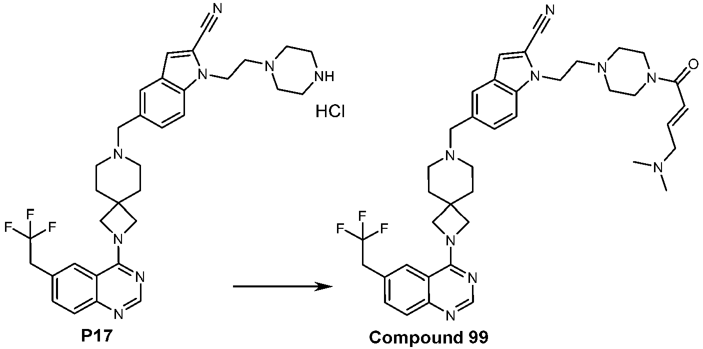

To the mixture of 1-(2-piperazin-1-ylethyl)-5-({2-[6-(2,2,2-trifluoroethyl)quinazolin-4-yl]-2,7-diazaspiro[3.5]non-7-yl}methyl)-1*H*-indole-2-carbonitrile hydrochloride (**P17**, 60 mg, 0.094 mmol), (2*E*)-3-(dimethylamino)acrylic acid (18 mg, 0.14 mmol), and DIPEA (97 mg, 0.75 mmol) in DCM (5 ml) a solution of T3P in ethyl acetate (0.15 mmol) was added. The reaction mixture was stirred at rt for 24 h (LCMS control). Then a water solution of sodium bicarbonate was added to the reaction mixture with stirring. The mixture was stirred for 30 min and layers were separated. The water phase was extracted with DCM (100 ml). Combined DCM extract was concentrated, and residue purified by column chromatography on silica with ethyl acetate-methanol (10:2) to yield the target compound (Compound **99**, 30 mg, 45 %). ¹H NMR (400 MHz, DMSO-*d₆*), δ: 8.83 (s, 1H), 7.98-8.08 (m, 2H), 7.84-7.94 (m, 3H), 7.7-7.41 (m, 1H), 7.60 (s, 1H),6.96 (d, J= 15.2 Hz, 1H), 6.75-6.62 (m, 1H), 4.91-4.73 (m, 4H), 4.43-4.31 (m, 6H), 3.96-3.81 (m, 2H), 3.40-3-20 (m, 6H), 3.20-2.77 (m, 4H), 2.82-2.67 (m, 6H), 2.3 4-2.04 (m, 4H). LCMS (ESI) [MH]⁺: 714.

### Example 3. 4-Methyl-1-[[(2S)-5-oxomorpholin-2-yl]methyl]-5-[[2-[6-(trifluoromethoxy)quinazolin-4-yl]-2,7-diazaspiro[3.5]nonan-7-yl]methyl]indole-2-carbonitrile (Compound 1)

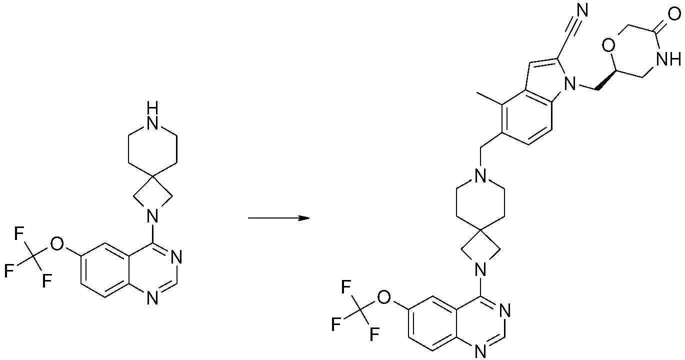

A mixture of 5-formyl-4-methyl-1-{[(2S)-5-oxomorpholin-2-yl]methyl}-1H-indole-2-carbonitrile (**P177,** 147 mg, 0.8 mmol), 4-(2,7-diazaspiro[3.5]non-2-yl)-6-(2,2,2-trifluormethoxy)quinazoline (**P11**, 270 mg, 0.8 mmol), DIPEA (1.03 g, 8 mmol) and STAB (1.7 g, 8 mmol) in DCM (30 ml) was stirred for 24 h, at rt (LCMS control), then water solution of sodium bicarbonate was added with stirring. The mixture was stirred for 30 min, water phase was extracted with DCM (2x20 ml), evaporated to dryness. Residue was refluxed with MeCN (5 ml) for 2h, cooled, filtered, washed with MeCN (1 ml), and dried to yield pure product (Compound 1, 25 mg, 5%)*.* ¹ H NMR (400 MHz, DMSO-*d₆*), δ: 8.48 (s, 1H), 8.01 (d, J = 4.1 Hz, 1H), 7.82 (m, 3H), 7.52 (s, 1H), 7.46 (d, J = 8.6 Hz, 1H), 7.31 (d, J = 8.7 Hz, 1H), 4.54 (dd, J = 15.4, 3.3 Hz, 1H), 4.37 (dd, J = 15.4, 7.7 Hz, 1H), 4.22 (s, 4H), 3.94 (m, 3H), 3.51 (s, 2H), 3.31 (m, 1H), 3.16 (t, J = 11.3 Hz, 1H), 2.50 (s, 3H), 2.37 (s, 4H), 1.78 (s, 4H). LCMS (ESI) [MH]⁺: 620.

### Example 4. 4-Methyl-1-[(3-oxo-1,4-oxazepan-7-yl)methyl]-5-[[2-[6-(2,2,2-trifluoroethyl)quinazolin-4-yl]-2,7-diazaspiro[3.5]nonan-7-yl]methyl]indole-2-carbonitrile (Compound 2)

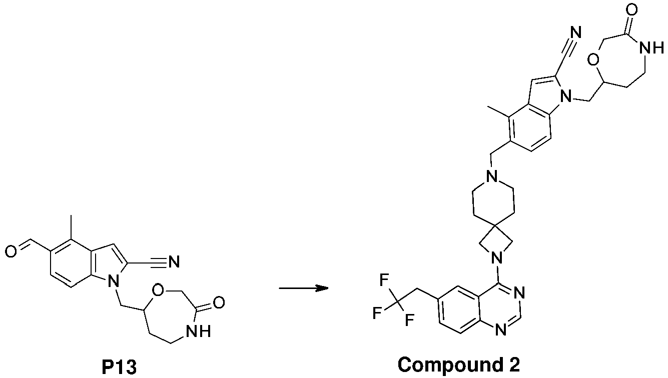

To mixture of 5-formyl-4-methyl-1-[(3-oxo-1,4-oxazepan-7-yl)methyl]indol-2-carbonitrile (**P13**, 53 mg, 0.17 mmol) and 4-(2,7-diazaspiro[3.5]non-2-yl)-6-(2,2,2-trifluoroethyl)quinazoline (**P9**, 57 mg, 0.17 mmol) DIPEA (219 mg, 1.7 mmol) and STAB (360 mg 1.7 mmol) in DCM (20 ml) were added. The reaction mixture was stirred for 24 h, at rt (LCMS control), then water solution of sodium bicarbonate was added with stirring. The mixture was stirred for 30 min, water phase was extracted with DCM (2x20 ml), and extract was evaporated to dryness. The residue was refluxed with MeCN (1 ml) for 2 h, cooled, filtered, washed with MeCN (1 ml), and dried to yield pure product (Compound **2**, 40 mg, 37%). ¹H NMR (400 MHz, DMSO-*d₆*), δ: 8.47 (s, 1H), 8.45 (s, 1H), 7.98 (s, 1H), 7.86 (d, J = 1.6 Hz, 1H), 7.72 (m, 2H), 7.54 (s, 1H), 7.38 (d, J = 8.7 Hz, 1H), 7.30 (d, J = 8.6 Hz, 1H), 4.50 (m, 2H), 4.15 (s, 4H), 3.86 (m, 2H), 3.52 (s, 2H), 2.50 (s, 3H), 2.38 (s, 4H), 1.83 (s, 6H), 1.80 (s, 4H). LCMS (ESI) [MH]⁺: 632.

### Example 5. N-[trans-4-({9-[6-(2,2,2-Trifluoroethyl)quinazolin-4-yl]-3,9-diazaspiro[5.5]undec-3-yl}methyl)cyclohexyl]ethanesulfonamide (Compound 3)

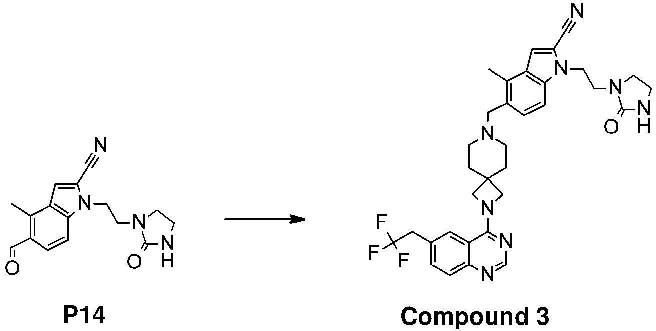

To mixture of 2-ethynyl-4-methyl-1-[2-(2-oxoimidazolidin-1-yl)ethyl]-1*H*-indole-5-carbaldehyde (**P14**, 47 mg, 0.16 mmol) and 4-(2,7-diazaspiro[3.5]non-2-yl)-6-(2,2,2-trifluoroethyl)quinazoline (**P9**, 53 mg, 0.16 mmol) DIPEA (219 mg, 1.7 mmol) and STAB (360 mg 1.7 mmol) in DCM (20 ml) were added. The reaction mixture was stirred for 48 h at rt (LCMS control), then water solution of sodium bicarbonate was added with stirring. The mixture was stirred for 30 min, water phase extracted with DCM (2x20 ml), and extract was evaporated to dryness. The residue was refluxed with MeCN (1 ml) for 2 h, cooled, filtered, washed with MeCN (1 ml), and dried to yield pure product (Compound **3**, 20 mg, 20%). ¹H NMR (400 MHz, DMSO-*d₆*), δ: 8.44 (s, 1H), 7.98 (s, 1H), 7.70 (m, 2H), 7.50 (s, 1H), 7.42 (d, *J* = 8.6 Hz, 1H), 7.32 (d, *J* = 8.6 Hz, 1H), 6.31 (s, 1H), 4.38 (t, *J* = 5.7 Hz, 2H), 4.21 (s, 4H), 3.86 (m, 2H), 3.52 (s, 2H), 3.40 (t, *J* = 5.6 Hz, 2H), 3.20 (m, 2H), 3.12 (m, 2H), 2.51 (s, 3H), 2.38 (s, 4H), 1.78 (s, 4H). LCMS (ESI) [MH]⁺: 617.

### Example 6. 4-Methyl-1-[(2-oxohexahydropyrimidin-5-yl)methyl]-5-[[2-[6-(2,2,2-trifluoroethyl)quinazolin-4-yl]-2,7-diazaspiro[3.5]nonan-7-yl]methyl]indole-2-carbonitrile (Compound 4)

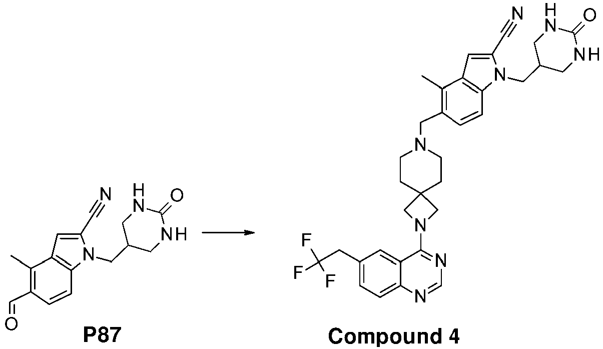

To mixture of 2-ethynyl-4-methyl-1-[(2-oxohexahydropyrimidin-5-yl)methyl]-1*H*-indole-5-carbaldehyde (**P87**, 26 mg, 0.088 mmol) and 4-(2,7-diazaspiro[3.5]non-2-yl)-6-(2,2,2-trifluoroethyl)quinazoline (**P9**, 29.5 mg, 0.088 mmol) DIPEA (57 mg, 0.44 mmol) and STAB (93 mg 0.44 mmol) in DCM (5 ml) were added. The reaction mixture was stirred for 48 h at rt (LCMS control), then water solution of sodium bicarbonate was added with stirring. The mixture was stirred for 30 min, water phase extracted with DCM (2x10 ml), the extract was evaporated to dryness and the residue was refluxed with MeCN (0.5 ml) for 2 h, cooled, filtered, washed with MeCN (1 ml),and dried to yield pure product (Compound **4**, 22 mg, 41 %). ¹H NMR (400 MHz, DMSO-*d₆*), δ: 8.44 (s, 1H), 7.98 (s, 1H), 7.71 (m, 2H), 7.58 (s, 1H), 7.46 (d, *J* = 8.6 Hz, 1H), 7.33 (d, *J* = 8.9 Hz, 1H), 6.14 (s, 2H), 4.33 (d, *J* = 7.3 Hz, 2H), 4.22 (s, 5H), 3.86 (m, 2H), 3.52 (s, 2H), 3.03 (m, 2H), 2.94 (m, 2H), 2.50 (s, 3H), 2.37 (s, 4H), 1.79 (s, 4H). LCMS (ESI) [MH]⁺: 617.

### Example 7. 4-Methyl-1-[(7-oxoazepan-4-yl)methyl]-5-[[2-[6-(2,2,2-trifluoroethyl)quinazolin-4-yl]-2,7-diazaspiro[3.5]nonan-7-yl]methyl]indole-2-carbonitrile (Compound 5)

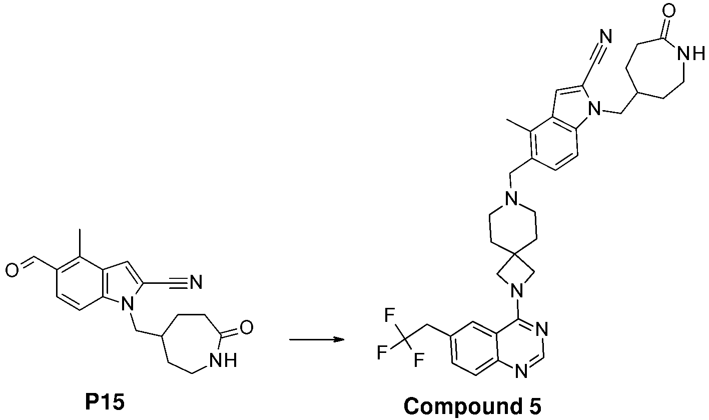

To a mixture of 5-formyl-4-methyl-1-[(7-oxoazepan-4-yl)methyl]-1*H*-indole-2-carbonitrile (**P15**, 108 mg, 0.35 mmol) and 4-(2,7-diazaspiro[3.5]non-2-yl)-6-(2,2,2-trifluoroethyl)quinazoline (**P9**, 117 mg, 0.35 mmol) DIPEA (452 mg, 3.5 mmol) and STAB (742 mg 3.5 mmol) in DCM (40 ml) were added. The reaction mixture was stirred for 48 h, at rt (LCMS control), then water solution of sodium bicarbonate was added with stirring. The mixture was stirred for additional 30 min, water phase was extracted with DCM (2x30 ml), and organic solvent was evaporated to dryness. The residue was refluxed with MeCN (15 ml) for 2 h, cooled, filtered, washed with MeCN (5 ml), and dried to give pure product (Compound **5**, 56 mg, 26 %). ¹H NMR (400 MHz, DMSO-*d₆*), δ: 8.45 (s, 1H), 7.98 (s, 1H), 7.71 (m, 2H), 7.54 (s, 1H), 7.45 (m, 2H), 7.31 (d, *J* = 8.7 Hz, 1H), 4.18 (m, 7H), 3.86 (m, 2H), 3.51 (s, 2H), 3.05 (m, 2H), 2.50 (s, 3H), 2.40 (s, 4H), 2.13 (m, 2H), 1.78 (s, 4H), 1.58 (m, 2H), 1.23 (m, 2H). LCMS (ESI) [MH]⁺: 630.

### Example 8. 4-Methyl-1-[(2-oxoimidazolidin-4-yl)methyl]-5-({2-[6-(2,2,2-trifluoroethyl)quinazolin-4-yl]-2,7-diazaspiro[3.5]non-7-yl}methyl)-1H-indole-2-carbonitrile (Compound 6)

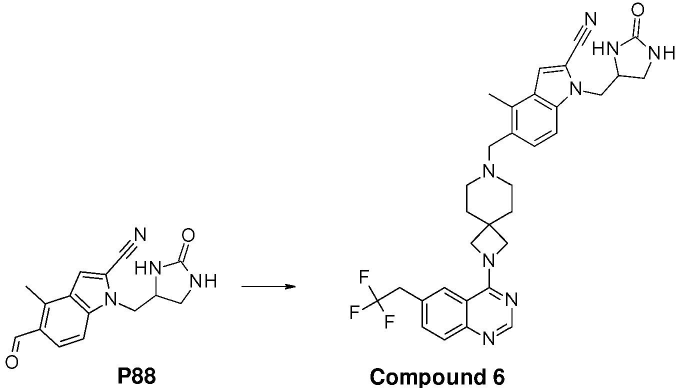

To a mixture of 5-formyl-4-methyl-1-[(2-oxoimidazolidin-4-yl)methyl]-1*H*-indole-2-carbonitrile (**P88**, 28 mg, 0.1 mmol) and 4-(2,7-diazaspiro[3.5]non-2-yl)-6-(2,2,2-trifluoroethyl)quinazoline (**P9**, 33 mg, 0.1 mmol) DIPEA (129 mg, 1 mmol) and STAB (212 mg 1 mmol) in DCM (10 ml) were added and the mixture was stirred for 48 h, at rt (LCMS control), then a water solution of sodium bicarbonate was added with stirring. The mixture was stirred for additional 30 min, water phase was extracted with DCM (2x10 ml), DCM was evaporated to dryness. The residue was refluxed with MeCN (0.5 ml) for 2 h, cooled, filtered, washed with MeCN (0.5 ml), and dried to give pure product (Compound **6**, 15 mg, 24 %). LCMS (ESI) [MH]⁺: 603.

### Example 9. 4-Methyl-5-({2-[6-(2,2,2-trifluoroethyl)quinazolin-4-yl]-2,7-diazaspiro[3.5]non-7-yl}methyl)-1H-indole-2-carbonitrile (Compound 68)

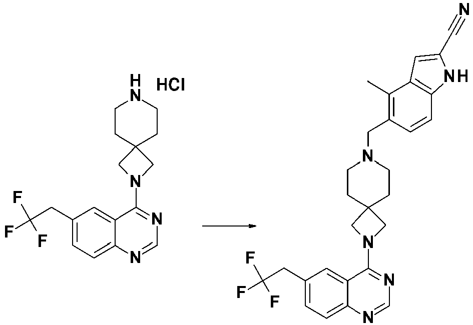

Compound **68** was prepared using the procedure described in ***Example 6*** and appropriate substrates: 2-cyano-4-metyl-5-formylindole (**P97**) and 4-(2,7-diazaspiro[3.5]non-2-yl)-6-(2,2,2-trifluoroethyl)quinazoline hydrochloride (**P9**). Yield 31%. ¹H NMR (400 MHz, DMSO-*d₆*), δ: 12.24 (s, 1H), 8.43 (s, 1H), 7.97 (s, 1H), 7.70 (s, 2H), 7.43 (s, 1H), 7.30-7.14 (m, 2H), 4.20 (s, 4H), 3.86 (q, J=11.3 Hz, 2H), 3.50 (s, 2H), 2.53 (s, 2H), 2.37 (s, 4H), 1.77 (br. s, 4H). LCMS (ESI) [MH]⁺: 505.

### Example 10. 4-Methyl-5-({2-[6-(2,2,2-trifluoroethyl)quinazolin-4-yl]-2,7-diazaspiro[3.5]non-7-yl}methyl)-1H-indole-2-carbonitrile (Compound 98)

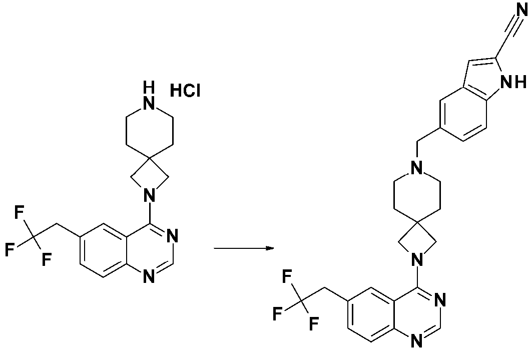

Compound **98** was prepared using the procedure described in ***Example 6*** and appropriate substrates: 2-cyano-5-formylindole and 4-(2,7-diazaspiro[3.5]non-2-yl)-6-(2,2,2-trifluoroethyl)quinazoline hydrochloride (**P9**). Yield 27%. ¹H NMR (400 MHz, DMSO-*d₆*), δ: 12.31 (s, 1H), 8.43 (s, 1H), 7.96 (s, 1H), 7.73-7.48 (m, 2H), 7.56 (s, 1H), 7.43-7.36 (m, 1H), 7.42-7.21 (m, 2H), 4.50-4.00 (m, 4H), 3.86 (q, J = 11.7 Hz, 2H), 3.52 (s, 2H), 2.53 (s, 2H), 2.53-2.16 (m, 4H), 1.90-1.75 (m, 4H). LCMS (ESI) [MH]⁺: 491.

### Example 11. 4-Methyl-1-[(6-methyl-5-oxo-morpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile (Compound 7)

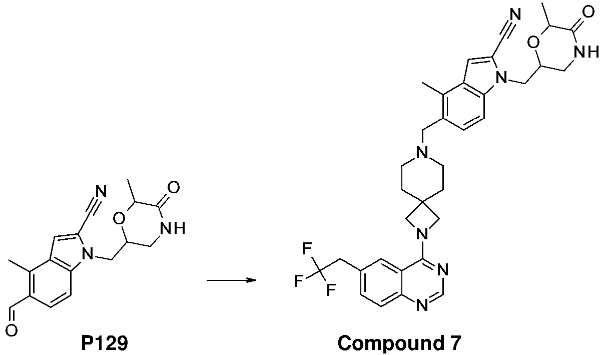

To a mixture of 5-formyl-4-methyl-1-[(6-methyl-5-oxomorpholin-2-yl)methyl]-1*H-*indole-2-carbonitrile (**P129**, 33 mg, 0.106 mmol) and 4-(2,7-diazaspiro[3.5]non-2-yl)-6-(2,2,2-trifluoroethyl)quinazoline (**P9**, 36 mg, 0.1 mmol) DIPEA (129 mg, 1 mmol) and STAB (212 mg 1 mmol) in DCM (10 ml) were added and the mixture was stirred for 48 h at rt (LCMS control), then water solution of sodium bicarbonate was added with stirring. The mixture was stirred for additional 30 min, water phase was extracted with DCM (2x10 ml), and organic solvent was evaporated to dryness. The residue was refluxed with MeCN (0.5 ml) for 2 h, cooled, filtered, washed with MeCN (0.5 ml), and dried to give pure Compound **7** (46 mg, 73 %). ¹H NMR (400 MHz, DMSO-*d₆*), δ: 8.44 (s, 1H), 7.98 (s, 1H), 7.86 (d, *J* = 4.2 Hz, 1H), 7.72 (m, 2H), 7.53 (m, 1H), 7.47 (d, *J* = 8.6 Hz, 1H), 7.31 (d, *J* = 8.7 Hz, 1H), 4.52 (dd, *J* = 15.3, 3.6 Hz, 1H), 4.36 (dd, *J* = 15.3, 7.5 Hz, 1H), 4.22 (s, 4H), 4.07 (m, 1H), 4.00 (q, *J* = 6.8 Hz, 1H), 3.86 (m, 2H), 3.52 (s, 2H), 3.32 (m, 1H), 3.15 (m, 1H), 2.50 (s, 3H), 2.38 (s, 4H), 1.78 (s, 4H), 1.20 (d, *J* = 6.8 Hz, 3H). LCMS (ESI) [MH]⁺: 632.

### Example 12. 1-[(6,6-Dimethyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile (Compound 8)

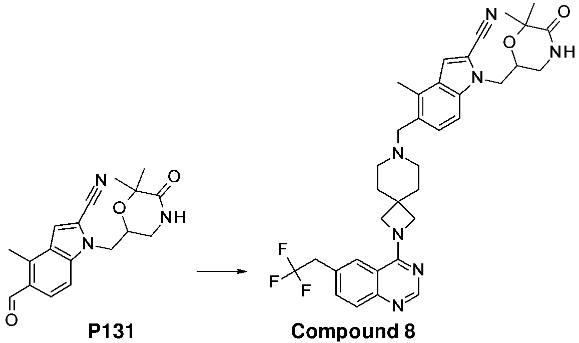

To a mixture of 5-formyl-4-methyl-1-[(6-methyl-5-oxomorpholin-2-yl)methyl]-1*H-*indole-2-carbonitrile (**P131**, 78 mg, 0.24 mmol) and 4-(2,7-diazaspiro[3.5]non-2-yl)-6-(2,2,2-trifluoroethyl)quinazoline (**P9**, 81 mg, 0.24 mmol) DIPEA (129 mg, 1 mmol) and STAB (212 mg 1 mmol) in DCN (10 ml) were added. The reaction mixture was stirred for 48 h at rt (LCMS control), then water solution of sodium bicarbonate was added with stirring. The mixture was stirred for additional 30 min, water phase was extracted with DCM (2x10 ml) and evaporated to dryness. The residue was refluxed with MeCN (0.5 ml) for 2 h, cooled, filtered, washed with MeCN (0.5 ml), and dried to give Compound **8** (46 mg, 21 %). ¹H NMR (400 MHz, DMSO-*d₆*), δ: 8.44 (s, 1H), 7.98 (s, 1H), 7.79 (d, *J* = 4.6 Hz, 1H), 7.71 (m, 2H), 7.53 (s, 1H), 7.47 (d, *J* = 8.6 Hz, 1H), 7.31 (d, *J* = 8.6 Hz, 1H), 4.50 (dd, *J* = 15.2, 4.0 Hz, 1H), 4.35 (dd, *J* = 15.2, 6.6 Hz, 1H), 4.22 (s, 4H), 4.15 (ddt, *J* = 10.1, 6.9, 3.5 Hz, 1H), 3.86 (m, 2H), 3.52 (m, 2H), 3.23 (m, 1H), 3.09 (t, *J* = 11.2 Hz, 1H), 2.50 (s, 3H), 2.38 (s, 4H), 1.78 (s, 4H), 1.24 (s, 3H), 1.11 (s, 3H). LCMS (ESI) [MH]⁺: 646.

### Example 13. 4-Methyl-1-{[4-(methylsulfonyl)morpholin-2-yl]methyl}-5-({2-[7-(2,2,2-trifluoroethyl)-1-naphthyl]-2,7-diazaspiro[3.5]non-7-yl}methyl)-1H-indole-2-carbonitrile (Compound 9)

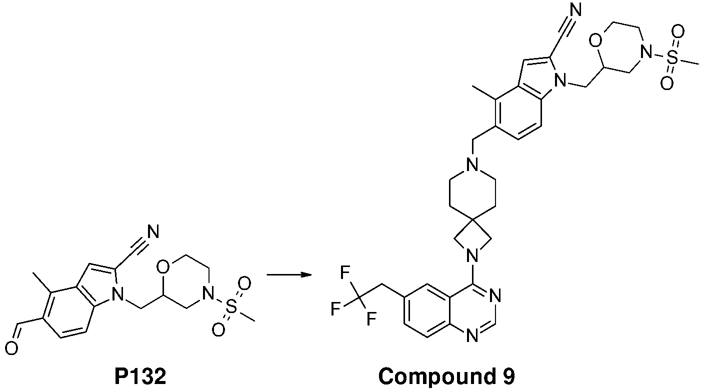

To a mixture of 5-formyl-4-methyl-1-{[4-(methylsulfonyl)morpholin-2-yl]methyl}-1*H-*indole-2-carbonitrile (**P132**, 57 mg, 0.31 mmol) and 4-(2,7-diazaspiro[3.5]non-2-yl)-6-(2,2,2-trifluoroethyl)quinazoline (**P9**, 104 mg, 0.31 mmol) DIPEA (400 mg, 3.1 mmol) and STAB (657 mg 3.1 mmol) in DCM (30 ml) were added. The reaction mixture was stirred at rt for 48 h (LCMS control), then water solution of sodium bicarbonate was added with stirring. The mixture was stirred for 30 min, water phase was extracted with DCM (2x20 ml), organic solvent was evaporated to dryness. The residue was refluxed with MeCN (0.3 ml) for 2 h, cooled, filtered, washed with MeCN (0.5 ml), and dried to yield pure Compound **9** (34 mg, 16 %). ¹H NMR (400 MHz, DMSO-*d₆*), δ: 8.44 (s, 1H), 7.97 (s, 1H), 7.70 (m, 2H), 7.51 (m, 2H), 7.31 (d, *J* = 8.6 Hz, 1H), 4.57 (dd, *J* = 15.2, 3.1 Hz, 1H), 4.27 (dd, *J* = 15.2, 8.5 Hz, 1H), 4.13 (s, 4H), 3.85 (m, 4H), 3.67 (d, *J* = 11.3 Hz, 1H), 3.52 (s, 2H), 3.38 (td, *J* = 11.6, 2.2 Hz, 1H), 3.29 (m, 1H), 2.91 (s, 3H), 2.83 (td, *J* = 11.6, 3.1 Hz, 1H), 2.67 (t, *J* = 10.9 Hz, 1H), 2.51 (s, 3H), 2.38 (s, 4H), 1.78 (s, 4H). LCMS (ESI) [MH]⁺: 682.

### Example 14. 5-({2-[5-Fluoro-6-(2,2,2-trifluoroethyl)quinazolin-4-yl]-2,7-diazaspiro[3.5]non-7-yl}methyl)-4-methyl-1-{(2S)-2-[4-(methylsulfonyl)piperazin-1-yl]propyl}-1H-indole-2-carbonitrile (Compound 10)

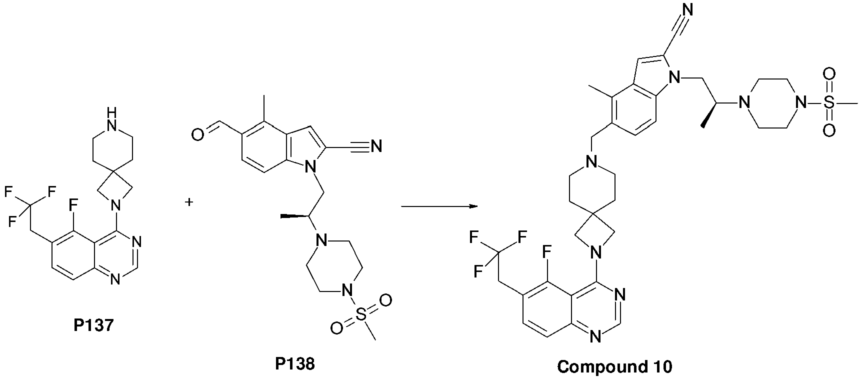

Molecular sieves 3Å (200 mg) were added to a mixture of 5-formyl-4-methyl-1-{(2*S*)-2-[4-(methylsulfonyl)piperazin-1-yl]propyl}-1*H*-indole-2-carbonitrile (**P138**, 130 mg, 0.34 mmol) and 4-(2,7-diazaspiro[3.5]non-2-yl)-5-fluoro-6-(2,2,2-trifluoroethyl)quinazoline (**P137**, 113 mg, 0.34 mmol) in methanol (1 ml). Then NaBH₃CN (42 mg, 0.67 mmol) was added, and the reaction mixture was stirred at ambient temperature overnight. Saturated aqueous solution of sodium bicarbonate and DCM were added, and the organic layer was separated, washed with brine, dried over anh. sodium sulfate, filtered and the filtrate was evaporated. The residue after evaporation was subjected to HPLC purification to afford the target compound **10** (39 mg, 17%). ¹H NMR (400 MHz, DMSO-*d*₆), δ: 8.49 (s, 1H), 7.80 (m, 1H), 7.58 (d, *J* = 8.6 Hz, 1H), 7.50 (s, 1H), 7.39 (d, *J* = 8.6 Hz, 1H), 7.30 (d, *J* = 8.6 Hz, 1H), 4.32 (dd, *J* = 14.9, 8.1 Hz, 1H), 4.17 (dd, *J* = 14.8, 5.8 Hz, 1H), 4.05 (s, 4H), 3.84 (m, 2H), 3.50 (s, 2H), 3.05 (m, 5H), 2.82 (s, 3H), 2.77 (m, 2H), 2.50 (s, 3H), 2.41 (m, 6H), 1.72 (s, 4H), 0.98 (d, *J* = 6.7 Hz, 3H). LCMS (ESI) [MH]⁺: 727.

### Example 15. 4-Methyl-1-(2-morpholin-4-ylethyl)-5-({2-[6-(2,2,2-trifluoroethyl)quinazolin-4-yl]-2,7-diazaspiro[3.5]non-7-yl}methyl)-1H-indole-2-carbonitrile (Compound 97)

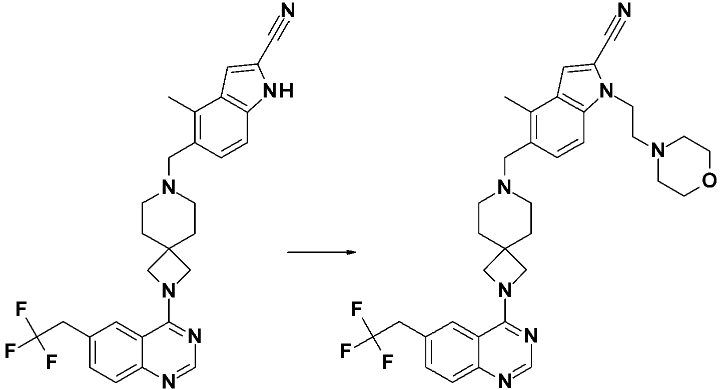

To a solution of 4-methyl-5-({2-[6-(2,2,2-trifluoroethyl)quinazolin-4-yl]-2,7-diazaspiro[3.5]non-7-yl}methyl)-1*H*-indole-2-carbonitrile (Compound **68,** 73 mg, 0.145 mmol) in dry THF (3ml) sodium hydride (60 %, 15 mg, 0.36 mmol) was added under atmosphere of Argon. The mixture was stirred at rt for 1 h, then 4-(2-chloroethyl)morpholine hydrochloride (27 mg, 0.145 mmol) was added. The reaction mixture was stirred at rt for additional 20 h. Then a water solution of sodium bicarbonate was added to the reaction mixture with stirring. The mixture was stirred for 30 min and layers were separated. The water phase was extracted with DCM (100 ml). Combined DCM extract was concentrated, and residue purified by HPLC silica-C18, MeCN-H₂O to yield the product (Compound **97**, 27 mg, 30 %). ¹H NMR (400 MHz, DMSO-*d₆*), δ: 8.84 (s, 1H), 8.06 (s, 1H), 8.15-7.94 (m, 1H), 7.89 -7.80 (m, 1H), 7.80 (s, 2H), 7.74 (s, 1H), 4.82-4.77 (m, 4H),4.42- 4.37 (m, 2H), 3.95 (q, J=11.2 Hz, 2H), 3.56 (s, 2H), 3.49-3.34 (m, 4H), 2.65 (s, 4H), 2.53 (s, 3H), 2.41-2.16 (m, 4H). LCMS (ESI) [MH]⁺: 618.

### Example 16. 7-({2-[6-(2,2,2-Trifluoroethyl)quinazolin-4-yl]-2,7-diazaspiro[3.5]non-7-yl}methyl)-2H-1,4-benzoxazin-3(4H)-one (Compound 11)

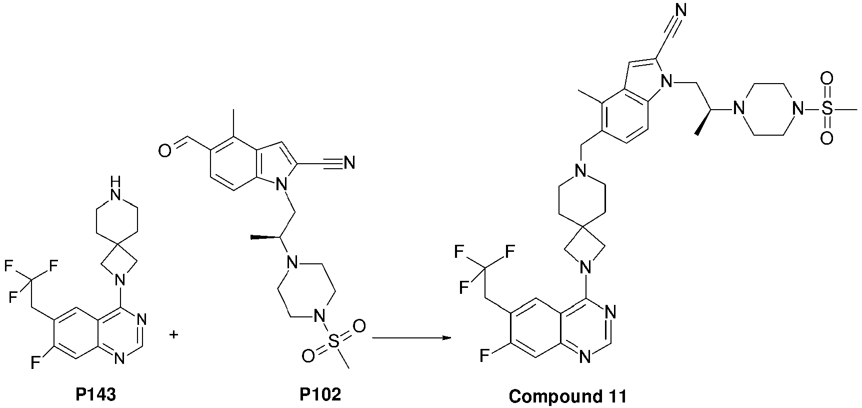

Molecular sieves, 3Å (200 mg) was added to a mixture of 5-formyl-4-methyl-1-{(2S)-2-[4-( tert-butylcarboxlato)piperazin-1-yl]propyl}-1H-indole-2-carbonitrile (**P102**, 130 mg, 0.34 mmol) and 4-(2,7-diazaspiro[3.5]non-2-yl)-6-(2,2,2-trifluoroethyl)-7-fluoroquinazolin (**P143**, 113 mg, 0.34 mmol) in methanol (1 ml). Then NaBH₃CN (42 mg, 0.67 mmol) was added, and the reaction mixture was stirred at ambient temperature overnight. Saturated aqueous solution of sodium bicarbonate and DCM were added, and the organic layer was separated, washed with brine, dried over anh. sodium sulfate, filtered and the filtrate was evaporated. The residue after evaporation was subjected to HPLC purification to afford the compound **11** (39 mg, 17%). ¹H NMR (400 MHz, DMSO-*d₆*), δ: 8.44 (s, 1H), 8.10 (d, *J* = 7.8 Hz, 1H), 7.50 (m, 2H), 7.40 (d, *J* = 8.7 Hz, 1H), 7.31 (d, *J* = 8.6 Hz, 1H), 4.32 (dd, *J* = 14.9, 8.0 Hz, 1H), 4.23 (s, 4H), 4.17 (dd, *J* = 14.8, 5.9 Hz, 1H), 3.92 (m, 2H), 3.51 (s, 2H), 3.05 (m, 5H), 2.82 (s, 3H), 2.77 (m, 2H), 2.51 (s, 3H), 2.40 (m, 6H), 1.79 (s, 4H), 0.98 (d, *J* = 6.7 Hz, 3H). LCMS (ESI) [MH]⁺: 727.

### Example 17. 4-Methyl-1-{2-[4-(methylsulfonyl)piperazin-1-yl]propyl}-5-({2-[6-(2,2,2-trifluoroethyl)quinazolin-4-yl]-2,7-diazaspiro[3.5]non-7-yl}methyl)-1H-indole-2-carbonitrile (Compound 96)

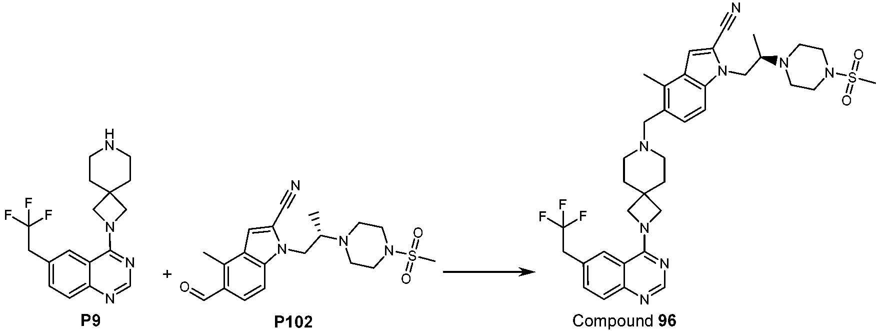

Molecular sieves, 3Å (200 mg) was added to a mixture of 5-formyl-4-methyl-1-{(2S)-2-[4-(methylsulfonyl)piperazin-1-yl]propyl}-1H-indole-2-carbonitrile (**P102**, 130 mg, 0.34 mmol) and 4-(2,7-diazaspiro[3.5]non-2-yl)-6-(2,2,2-trifluoroethyl)quinazoline (**P9**, 113 mg, 0.34 mmol) in methanol (1 ml). Then NaBH₃CN (42 mg, 0.67 mmol) was added, and the reaction mixture was stirred at ambient temperature overnight. Saturated aqueous solution of sodium bicarbonate, and dichloromethane were added, and the organic layer separated, washed with brine, dried over sodium sulfate, filtered and the filtrate was evaporated. The residue after evaporation was subjected to HPLC to afford the target compound **96** (39 mg, 17 %). ¹H NMR (400 MHz, DMSO-*d₆*), δ: 8.44 (s, 1H), 7.98 (s, 1H), 7.70 (t, J = 5.9 Hz, 2H), 7.50 (s, 1H), 7.38 (s, 1H), 7.31 (d, J = 8.6 Hz, 1H), 4.38 - 4.09 (m, 5H), 3.86 (q, J = 11.4 Hz, 2H), 3.59 - 3.45 (m, 2H), 3.10-2.85 (m, 5H), 2.82 (s, 3H), 2.80-2.70 (m, 2H), 2.45-2.40 (m, 4H), 1.90-1.70 (m, 4H), 0.98 (d, J = 6.7 Hz, 3H). LCMS (ESI) [MH]⁺: 709.

### Example 18. 4-Methyl-1-{2-[4-(methylsulfonyl)piperazin-1-yl]propyl}-5-({2-[6-(2,2,2-trifluoroethyl)pyrido[2,3-d]pyrimidin-4-yl]-2,7-diazaspiro[3.5]non-7-yl}methyl)-1H-indole-2-carbonitrile (Compound 89)

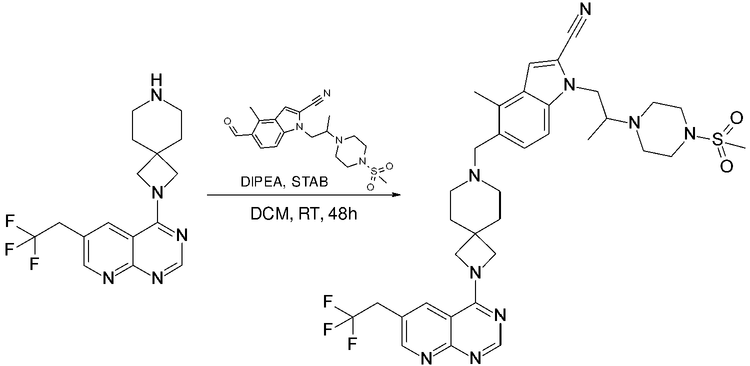

To a solution of **P39** (20 mg, 0.06 mmol), aldehyde **P98** (23 mg, 0.06 mmol) and DIPEA (38 mg, 0.3 mmol) in DCM (1 ml) STAB (50 mg, 0.24 mmol) was added. The reaction mixture was stirred at rt for 48 h. Water solution of sodium bicarbonate were added, the mixture was stirred for 15 minutes, and extracted with DCM (2x5 ml), concentrated extract was purified by silica gel column chromatography (gradient from EtOAc to 50% MeOH in EtOAc) to give the title Compound **89** as white solid (13 mg, 31%). LCMS (ESI) [MH]⁺: 710.

### Example 19. 4-Methyl-1-{2-[4-(methylsulfonyl)piperazin-1-yl]propyl}-5-({2-[6-(2,2,2-trifluoroethyl)quinazolin-4-yl]-2,7-diazaspiro[3.5]non-7-yl}methyl)-1H-indole-2-carbonitrile (Compound 71) and 4-methyl-1-{1-methyl-2-[4-(methylsulfonyl)piperazin-1-yl]ethyl}-5-({2-[6-(2,2,2-trifluoroethyl)quinazolin-4-yl]-2,7-diazaspiro[3.5]non-7-yl}methyl)-1H-indole-2-carbonitrile (Compound 70)

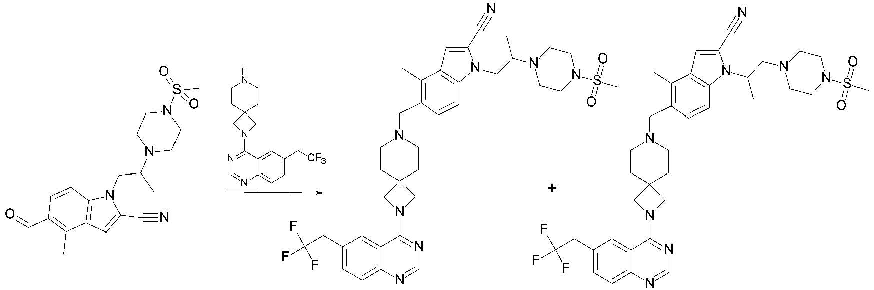

To a solution of compound **P98** (2.6 g, 6.7 mmol), Et₃N (2.9 ml, 20.1 mmol) and compound **P9** (2.25 g, 6.7 mmol) dissolved dichloromethane (50 ml), and STAB (4.26 g, 20.1 mmol) was added. The reaction mixture was stirred at rt for 18 h (LCMS control), concentrated, residue was purified by silica gel column chromatography in THF and purification by HPLC C-18 to give Compound **71** (2.7 g, 57 % yield) and Compound **70** (95 mg, 2% yield). Compound **71**: LCMS (ESI) [MH]⁺: 709. Compound **70**: ¹H NMR (400 MHz, DMSO-*d₆*), δ: 8.44 (s, 1H), 7.97 (s, 1H), 7.71 (m, 2H), 7.49 (s, 1H), 7.39 (d, *J* = 8.6 Hz, 1H), 7.31 (d, *J* = 8.6 Hz, 1H), 4.32 (dd, *J* = 14.7, 8.1 Hz, 1H), 4.19 (d, *J* = 6.1 Hz, 5H), 3.86 (q, *J* = 11.4 Hz, 2H), 3.52 (m, 2H), 3.02 (m, 5H), 2.82 (s, 3H), 2.76 (m, 2H), 2.51 (s, 3H), 2.41 (s, 6H), 1.79 (s, 4H), 0.98 (d, *J* = 6.6 Hz, 3H). LCMS (ESI) [MH]⁺: 709.

### Example 20. 4-Methyl-1-{(1R)-1-methyl-2-[4-(methylsulfonyl)piperazin-1-yl]ethyl}-5-({2-[6-(2,2,2-trifluoroethyl)quinazolin-4-yl]-2,7-diazaspiro[3.5]non-7-yl}methyl)-1H-indole-2-carbonitrile (Compound 23) and 4-methyl-1-{(1S)-1-methyl-2-[4-(methylsulfonyl)piperazin-1-yl]ethyl}-5-({2-[6-(2,2,2-trifluoroethyl)quinazolin-4-yl]-2,7-diazaspiro[3.5]non-7-yl}methyl)-1H-indole-2-carbonitrile (Compound 24)

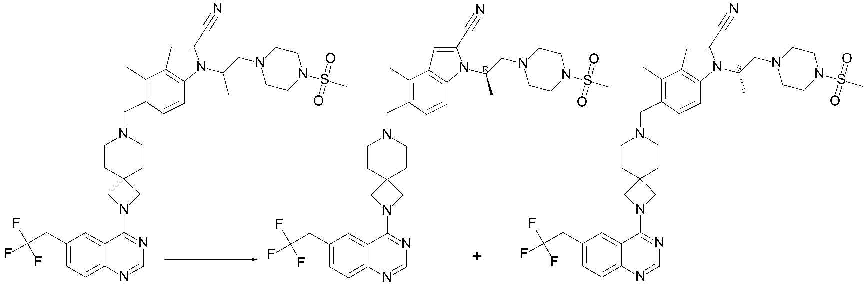

Compounds **23** and **24** were obtained by separation of enantiomers mixture (Compound **70**) using Phenomenex Cell-4 45mm and mobile phase MeCN/EtOH/HCOOH 90/10/0.1 at 23°C with UV/CD 254 nm detector and mobile phase flow as 1 ml/min. It was obtained Compound **23** (37 mg) and Compound **24** (30 mg). Compound **23**: ¹H NMR (400 MHz, DMSO-*d₆*), δ: 8.44 (s, 1H), 7.97 (s, 1H), 7.71 (m, 2H), 7.49 (s, 1H), 7.39 (d, J = 8.6 Hz, 1H), 7.31 (d, J = 8.6 Hz, 1H), 4.32 (dd, J = 14.7, 8.1 Hz, 1H), 4.19 (d, J = 6.1 Hz, 5H), 3.86 (q, J = 11.4 Hz, 2H), 3.52 (m, 2H), 3.02 (m, 5H), 2.82 (s, 3H), 2.76 (m, 2H), 2.51 (s, 3H), 2.41 (s, 6H), 1.79 (s, 4H), 0.98 (d, J = 6.6 Hz, 3H). LCMS (ESI) [MH]⁺: 709. Compound **24**: ¹H NMR (400 MHz, DMSO-*d₆*), δ: 8.44 (s, 1H), 7.97 (s, 1H), 7.71 (m, 2H), 7.49 (s, 1H), 7.39 (d, J = 8.6 Hz, 1H), 7.31 (d, J = 8.6 Hz, 1H), 4.32 (dd, J = 14.7, 8.1 Hz, 1H), 4.19 (d, J = 6.1 Hz, 5H), 3.86 (q, J = 11.4 Hz, 2H), 3.52 (m, 2H), 3.02 (m, 5H), 2.82 (s, 3H), 2.76 (m, 2H), 2.51 (s, 3H), 2.41 (s, 6H), 1.79 (s, 4H), 0.98 (d, J = 6.6 Hz, 3H). LCMS (ESI) [MH]⁺: 709.

### Example 21. 4-Methyl-1-[2-(4-methylsulfonylpiperazin-1-yl)propyl]-5-[[2-[7-(2,2,2-trifluoroethyl)phthalazin-1-yl]-2,7-diazaspiro[3.5]nonan-7-yl]methyl]indole-2-carbonitrile (Compound 79)

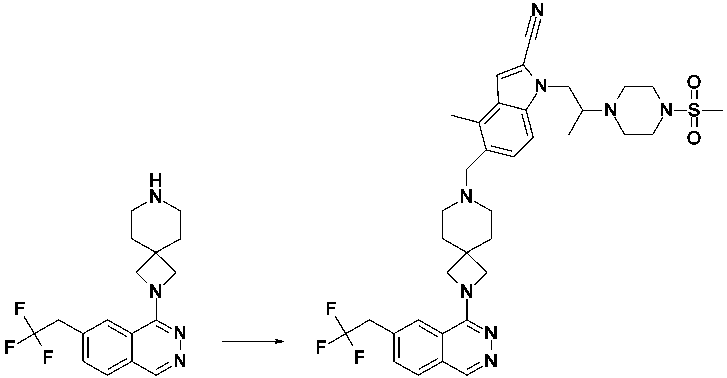

A mixture of 1-(2,7-diazaspiro[3.5]non-2-yl)-7-(2,2,2-trifluoroethyl)phthalazine **P44** (37 mg, 0.1 mmol), 5-formyl-4-methyl-1-{2-[4-(methylsulfonyl)piperazin-1-yl]propyl}-1*H-*indole-2-carbonitrile **P98** (39 mg, 0.1 mmol), DIPEA (65 mg, 0.5 mmol) and STAB (106 mg, 0.5 mmol) in DCM (4 ml) was stirred at rt for 24 h (LCMS control), then washed with water solution of NaHCO₃, extracted with DCM (2x5 ml). The solvent was evaporated, and residue was subjected to column chromatography on silica gel eluting with ethyl acetate-methanol (0 → 30%) to afford the title Compound **79** (36 mg, 50 %). ¹H NMR (400 MHz, DMSO-*d*₆) δ 8.99 (s, 1H), 8.03 (s, 1H), 7.99 (d, *J* = 8.3 Hz, 1H), 7.85 (d, *J* = 8.3 Hz, 1H), 7.50 (s, 1H), 7.40 (d, *J* = 8.7 Hz, 1H), 7.32 (d, *J* = 8.7 Hz, 1H), 4.32 (dd, *J* = 14.8, 8.0 Hz, 1H), 4.17 (m, 5H), 3.98 (q, *J* = 11.4 Hz, 2H), 3.52 (m, 2H), 3.05 (m, 5H), 2.83 (s, 3H), 2.78 (m, 2H), 2.51 (s, 3H), 2.40 (s, 6H), 1.79 (s, 4H), 0.98 (d, *J* = 6.7 Hz, 3H). LCMS (ESI) [MH]⁺: 709.

### Example 22. 4-Methyl-1-[2-(4-methylsulfonylpiperazin-1-yl)propyl]-5-[[2-[6-(2,2,2-trifluoroethyl)cinnolin-4-yl]-2,7-diazaspiro[3.5]nonan-7-yl]methyl]indole-2-carbonitrile (Compound 80)

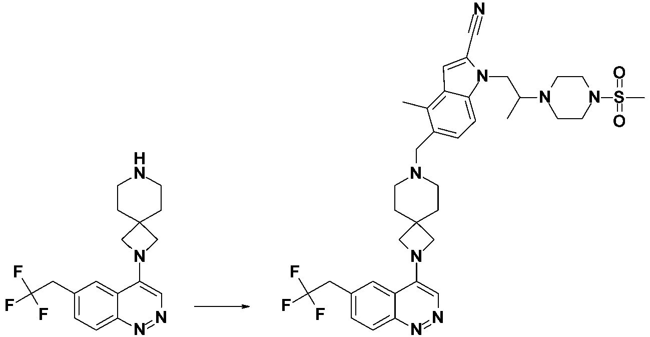

A mixture of 4-(2,7-diazaspiro[3.5]non-2-yl)-6-(2,2,2-trifluoroethyl)cinnoline (**P49**, 37 mg, 0.1 mmol), 5-formyl-4-methyl-1-{2-[4-(methylsulfonyl)piperazin-1-yl]propyl}-1*H-*indole-2-carbonitrile (**P98**, 39 mg, 0.1 mmol), DIPEA (65 mg, 0.5 mmol) and STAB (106 mg, 0.5 mmol) in DCM (4 ml) was stirred at rt for 24 h (LCMS control), then washed with water solution of NaHCO₃, extracted with DCM (2x5 ml). The residue after evaporation was subjected to column chromatography on silica gel eluting with ethyl acetate-methanol (0 → 30%) to afford the title compound **80** (36 mg, 50 %). ¹H NMR (400 MHz, DMSO-*d*₆) δ 8.34 (s, 1H), 8.10 (d, *J* = 8.8 Hz, 1H), 8.02 (s, 1H), 7.71 (d, *J* = 8.8 Hz, 1H), 7.50 (s, 1H), 7.40 (d, *J* = 8.7 Hz, 1H), 7.31 (d, *J* = 8.7 Hz, 1H), 4.33 (dd, *J* = 14.8, 8.0 Hz, 1H), 4.21 (m, 5H), 3.91 (q, *J* = 11.4 Hz, 2H), 3.52 (m, 2H), 3.03 (m, 5H), 2.83 (s, 3H), 2.78 (m, 2H), 2.51 (s, 3H), 2.40 (m, 6H), 1.80 (s, 4H), 0.98 (d, *J* = 6.7 Hz, 3H). LCMS (ESI) [MH]⁺: 709.

### Example 23. 1-[2-(4-Acryloylpiperazin-1-yl)propyl]-4-methyl-5-({2-[6-(2,2,2-trifluoroethyl)quinazolin-4-yl]-2,7-diazaspiro[3.5]non-7-yl}methyl)-1H-indole-2-carbonitrile (Compound 63)

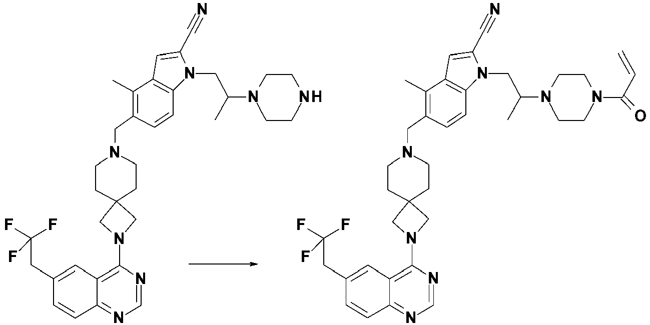

A mixture of 4-methyl-1-(2-piperazin-1-ylpropyl)-5-[[2-[6-(2,2,2-trifluoroethyl)quinazolin-4-yl]-2,7-diazaspiro[3.5]nonan-7-yl]methyl]indole-2-carbonitrile (**P20**, 50 mg, 0.079 mmol), acrylic acid chloride (11 mg, 0.12 mmol) and K₂CO₃ (28 mg, 0.2 mmol) in MeCN (2 ml) was stirred at rt for 24 h. The reaction mixture was concentrated, and the residue was separated by HPLC (silica C-18, MeCN-H₂O) to yield title Compound **63** (19 mg, 30%). LCMS (ESI) [MH]⁺: 685.

### Example 24. 1-[4-(4-Acryloylpiperazin-1-yl)butyl]-4-methyl-5-({2-[6-(2,2,2-trifluoroethyl)quinazolin-4-yl]-2,7-diazaspiro[3.5]non-7-yl}methyl)-1H-indole-2-carbonitrile (Compound 65)

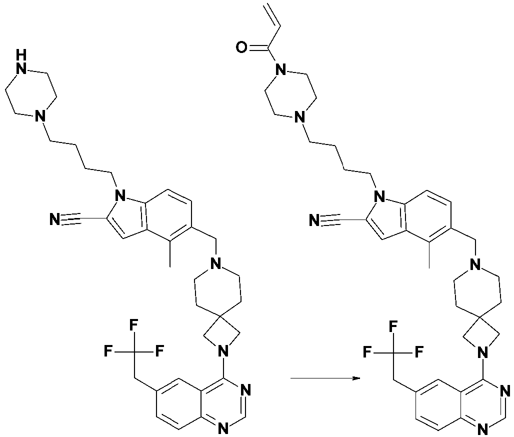

The mixture of 4-(7-{[2-ethynyl-4-methyl-1-(4-piperazin-1-ylbutyl)-1*H*-indol-5-yl]methyl}-2,7-diazaspiro[3.5]non-2-yl)-6-(2,2,2-trifluoroethyl)quinazoline (**P24**, 50 mg, 0.077 mmol), acrylic acid chloride (11 mg, 0.12 mmol) and K₂CO₃ (28 mg, 0.2 mmol) in MeCN (2 ml) stirred at rt for 24 h. The reaction mixture was concentrated, and the residue was separated by HPLC (silica C-18, MeCN-H₂O) to yield title Compound **65** (21 mg, 32%). ¹H NMR (300 MHz, DMSO-*d₆*), δ: 8.44 (s, 1H), 7.97 (s, 1H), 7.71 (m, 2H), 7.52 (s, 1H), 7.42 (d, *J* = 8.6 Hz, 1H), 7.32 (d, *J* = 8.6 Hz, 1H), 6.76 (dd, *J* = 16.6, 10.5 Hz, 1H), 6.07 (dd, *J* = 16.6, 2.3 Hz, 1H), 5.64 (dd, *J* = 10.3, 2.3 Hz, 1H), 4.32 (t, *J* = 7.0 Hz, 2H), 4.20 (m, 4H), 3.86 (q, *J* = 11.3 Hz, 2H), 3.50 (m, 6H), 2.50 (s, 3H), 2.38 (m, 3H), 2.27 (m, 7H), 1.78 (m, 6H), 1.41 (p, *J* = 7.9 Hz, 2H). LCMS (ESI) [MH]⁺: 699.

### Example 25. 4-Methyl-1-{4-[4-(methylsulfonyl)piperazin-1-yl]butyl}-5-({2-[6-(2,2,2-trifluoroethyl)quinazolin-4-yl]-2,7-diazaspiro[3.5]non-7-yl}methyl)-1H-indole-2-carbonitrile (Compound 44)

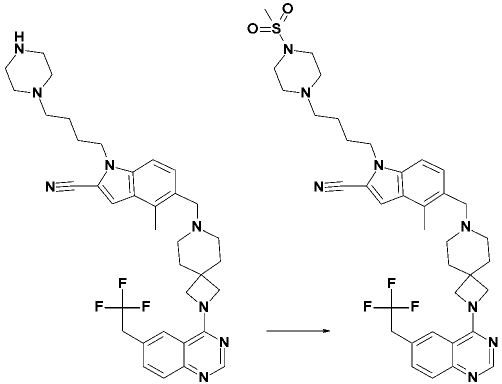

### A mixture of 4-(7-{[2-ethynyl-4-methyl-1-(4-piperazin-1-ylbutyl)-1H-indol-5-yl]methyl}-2,7-diazaspiro[3.5]non-2-yl)-6-(2,2,2-trifluoroethyl)quinazoline (P24, 50 mg, 0.077 mmol), MsCl (13 mg, 0.12 mmol) and DIPEA (50 mg, 0.4 mmol) in MeCN (2 ml) was stirred at rt for 24 h. The reaction mixture was concentrated, and obtained residue was purified by HPLC (silica C-18, MeCN-H₂O) to yield the title Compound 44 (39 mg, 70 %). LCMS (ESI) [MH]⁺: 723.

### Example 26. N-(trans-4-{[2-Cyano-4-methyl-5-({2-[6-(2,2,2-trifluoroethyl)quinazolin-4-yl]-2,7-diazaspiro[3.5]non-7-yl}methyl)-1H-indol-1-yl]methyl}cyclohexyl)methanesulfonamide (Compound 40)

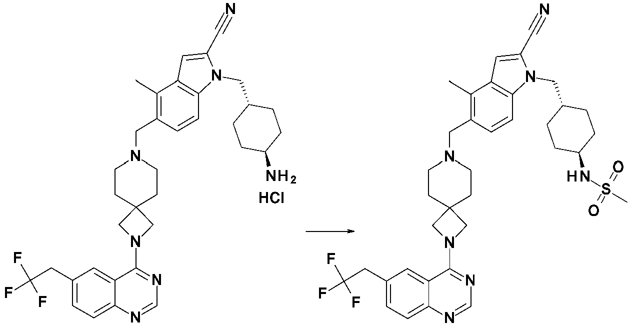

A mixture of 1-[(*trans*-4-aminocyclohexyl)methyl]-4-methyl-5-[[2-[6-(2,2,2-trifluoroethyl)quinazolin-4-yl]-2,7-diazaspiro[3.5]nonan-7-yl]methyl]-1H-indole-2-carbonitrile hydrochloride **P26** (34 mg, 0.053 mmol), MsCl (7 mg, 0.06 mmol) and DIPEA (39 mg, 0.30 mmol) in DCM (1 ml) was stirred at rt for 24 h. The reaction mixture was concentrated, and the residue was separated by HPLC (silica C-18, MeCN-H₂O) to yield the title compound **40** (29 mg, 80%). ¹H NMR (400 MHz, DMSO-*d₆*), δ: 8.45 (s, 1H), 7.98 (s, 1H), 7.72 (m, 2H), 7.52 (s, 1H), 7.42 (d, *J* = 8.6 Hz, 1H), 7.31 (d, *J* = 8.6 Hz, 1H), 6.96 (d, *J* = 7.4 Hz, 1H), 4.20 (s, 4H), 4.13 (d, *J* = 7.4 Hz, 2H), 3.86 (q, *J* = 11.5 Hz, 2H), 3.52 (s, 2H), 3.06 (m, 1H), 2.88 (s, 3H), 2.51 (s, 3H), 2.39 (m, 4H), 1.88 (m, 2H), 1.79 (m, 5H), 1.52 (m, 2H), 1.14 (m, 4H). LCMS (ESI) [MH]⁺: 694.

### Example 27. 5-({2-[5-Fluoro-6-(2,2,2-trifluoroethyl)quinazolin-4-yl]-2,7-diazaspiro[3.5]non-7-yl}methyl)-4-methyl-1-[(5-oxomorpholin-2-yl)methyl]-1H-indole-2-carbonitrile (Compound 41)

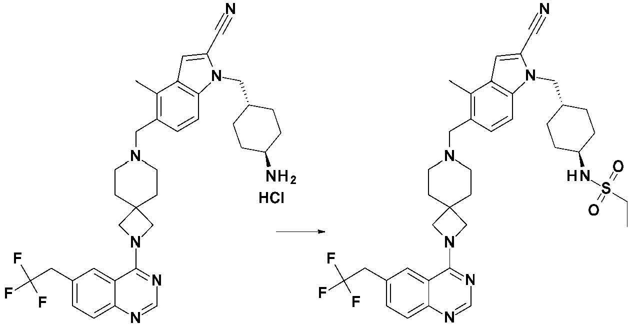

The mixture of 1-[(trans-4-aminocyclohexyl)methyl]- 4-methyl-5-[[2-[6-(2,2,2-trifluoroethyl)quinazolin-4-yl]-2,7-diazaspiro[3.5]nonan-7-yl]methyl]-1H-indole-2-carbonitrile hydrochloride **P26** (34 mg, 0.053 mmol), ethylsulfochloride (7 mg, 0.06 mmol) and DIPEA (39 mg, 0.30 mmol) in DCM (1 ml) was stirred at rt for 24 h. The reaction mixture was concentrated, and the residue was separated by HPLC (silica C-18, MeCN-H₂O) to yield the title Compound **41** (29 mg, 80%). ¹H NMR (400 MHz, DMSO-*d₆*), δ: 8.45 (s, 1H), 7.98 (s, 1H), 7.72 (m, 2H), 7.52 (s, 1H), 7.42 (d, J = 8.6 Hz, 1H), 7.31 (d, J = 8.6 Hz, 1H), 6.98 (d, J = 7.4 Hz, 1H), 4.21 (s, 4H), 4.13 (d, J = 7.2 Hz, 2H), 3.86 (q, J = 11.5 Hz, 2H), 3.52 (s, 2H), 3.01 (s, 1H), 2.96 (q, J = 7.3 Hz, 2H), 2.51 (s, 3H), 2.39 (m, 4H), 1.82 (m, 7H), 1.51 (m, 2H), 1.15 (m, 7H). LCMS (ESI) [MH]⁺: 708.

### Example 28. 4-Methyl-1-[(5-oxomorpholin-2-yl)methyl]-5-({2-[6-(2,2,2-trifluoroethyl)quinazolin-4-yl]-2,7-diazaspiro[3.5]non-7-yl}methyl)-1H-indole-2-carbonitrile (Compound 82)

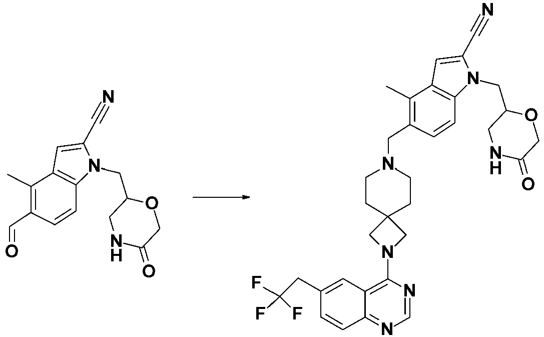

A mixture of 4-(2,7-diazaspiro[3.5]non-2-yl)-6-(2,2,2-trifluoroethyl)quinazoline (**P9**, 87 mg, 0.26 mmol), 5-formyl-4-methyl-1-[(5-oxomorpholin-2-yl)methyl]-1*H*-indole-2-carbonitrile (**P105**, 76 mg, 0.26 mmol), DIPEA (168 mg, 1.3 mmol) and STAB (276 mg, 1.3 mmol) in DCM (7 ml) was stirred at rt for 24 h (LCMS control), then washed with water solution of NaHCO₃, extracted with DCM (2x10 ml). The residue after solvent evaporation was subjected to column chromatography on silica gel eluting with ethyl acetate-methanol (0 → 30%) to afford the title Compound **82** (46 mg, 29 %). LCMS (ESI) [MH]⁺: 618.

### Example 29. 4-Methoxy-1-{2-[4-(methylsulfonyl)piperazin-1-yl]propyl}-5-({2-[6-(2,2,2-trifluoroethyl)quinazolin-4-yl]-2,7-diazaspiro[3.5]non-7-yl}methyl)-1H-indole-2-carbonitrile (Compound 50) and 4-methoxy-1-{1-methyl-2-[4-(methylsulfonyl)piperazin-1-yl]ethyl}-5-({2-[6-(2,2,2-trifluoroethyl)quinazolin-4-yl]-2,7-diazaspiro[3.5]non-7-yl}methyl)-1H-indole-2-carbonitrile (Compound 47)

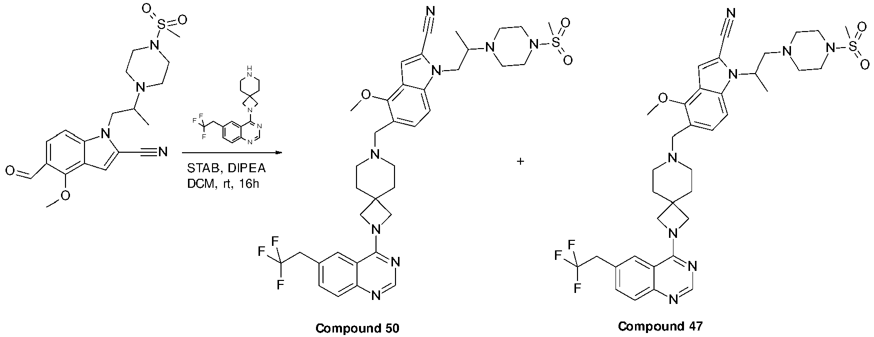

A mixture of 5-formyl-4-methoxy-1-{2-[4-(methylsulfonyl)piperazin-1-yl]propyl}-1H-indole-2-carbonitrile (**P121**, 45 mg, 0.11 mmol), triacethoxyborohydride (70 mg, 3 eq) and 4-(2,7-diazaspiro[3.5]non-2-yl)-6-(2,2,2-trifluoroethyl)quinazoline hydrochloride (**P9**, 45 mg, 1.2 eq) in DCM (2 ml) stirred at rt (LCMS control), then with stirring water solution of sodium bicarbonate was added. The mixture was stirred 30 min, water phase extracted with DCM (30 ml), DCM was evaporated, and residue purified by column chromatography on silica with mixture ethyl acetate-methanol (10-2). Two products (Compounds **50** and **47**) were separated using HPLC. Compound **50**: 14 mg, ¹H NMR (400 MHz, DMSO-*d*₆), δ: 8.44 (s, 1H), 7.98 (s, 1H), 7.71 (m, 2H), 7.56 (s, 1H), 7.37 (d, *J* = 8.6 Hz, 1H), 7.31 (d, *J* = 8.6 Hz, 1H), 4.32 (dd, *J* = 15.0, 8.1 Hz, 1H), 4.22 (s, 4H), 4.17 (dd, *J* = 14.8, 5.9 Hz, 1H), 3.99 (s, 3H), 3.86 (q, *J* = 11.5 Hz, 2H), 3.53 (m, 2H), 3.02 (m, 5H), 2.83 (s, 3H), 2.77 (m, 2H), 2.41 (s, 6H), 1.80 (s, 4H), 0.99 (d, *J* = 6.7 Hz, 3H). Compound **47**: 2.1 mg. LCMS (ESI) [MH]⁺: 725.

### Example 30. 6-Methoxy-1-{2-[4-(methylsulfonyl)piperazin-1-yl]propyl}-5-({2-[6-(2,2,2-trifluoroethyl)quinazolin-4-yl]-2,7-diazaspiro[3.5]non-7-yl}methyl)-1H-indole-2-carbonitrile (Compound 20)

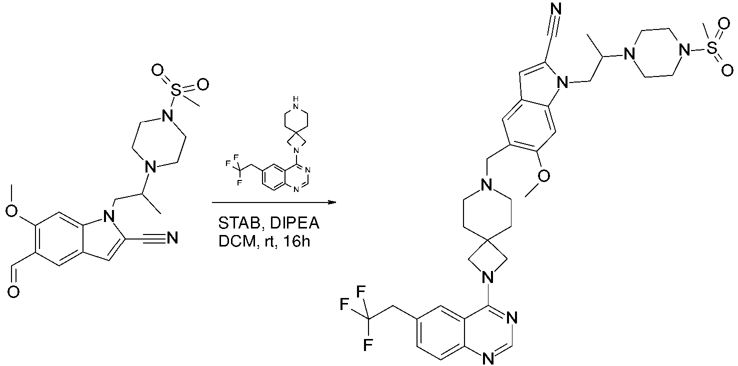

A mixture of 5-formyl-6-methoxy-1-{2-[4-(methylsulfonyl)piperazin-1-yl]propyl}-1H-indole-2-carbonitrile (**P128**, 90 mg, 0.11 mmol), triacethoxyborohydride (142 mg, 3 eq) and 4-(2,7-diazaspiro[3.5]non-2-yl)-6-(2,2,2-trifluoroethyl)quinazoline (**P9**, 113 mg, 1.5 eq) in DCM (2 ml) was stirred at rt (LCMS control), then with stirring water solution of sodium bicarbonate was added. The mixture was stirred for 30 min, water phase extracted with DCM (30 ml), after concentration of organic phase residue was purified by HPLC to afford yellow oil (Compound **20**, 68 mg, 42%). ¹H NMR (400 MHz, DMSO-*d₆*), δ: 8.44 (s, 1H), 7.98 (s, 1H), 7.71 (m, 2H), 7.58 (s, 1H), 7.32 (s, 1H), 7.08 (s, 1H), 4.26 (m, 6H), 3.89 (s, 3H), 3.86 (q, J = 11.5 Hz, 2H),3.51 (s, 2H), 3.12 (m, 1H), 3.01 (m, 4H), 2.82 (s, 3H), 2.77 (m, 2H), 2.44 (m, 6H), 1.83 (s, 4H), 0.99 (d, J = 6.6 Hz, 3H). LCMS (ESI) [MH]⁺: 725.

### Example 31. 4-Methyl-1-{2-[4-(methylsulfonyl)piperazin-1-yl]propyl}-6-({2-[6-(2,2,2-trifluoroethyl)quinazolin-4-yl]-2,7-diazaspiro[3.5]non-7-yl}methyl)-1H-indole-2-carbonitrile (Compound 83)

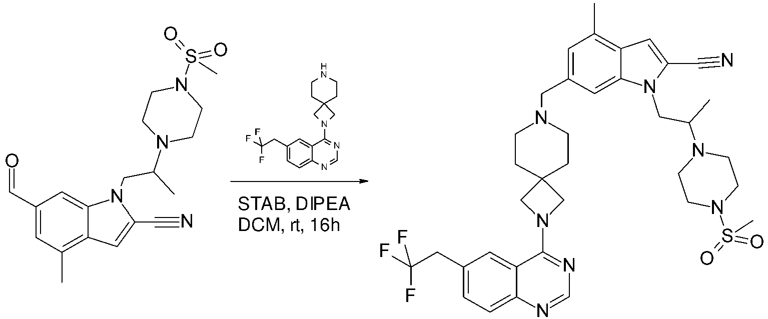

A mixture of 6-formyl-4-methyl-1-{2-[4-(methylsulfonyl)piperazin-1-yl]propyl}-1H-indole-2-carbonitrile (**P114**, 40 mg, 0.1 mmol), triacethoxyborohydride (65 mg, 3 eq) and 4-(2,7-diazaspiro[3.5]non-2-yl)-6-(2,2,2-trifluoroethyl)quinazoline (**P9**, 52 mg, 1.5 eq) in DCM (2 ml) was stirred at rt (LCMS control), then with stirring water solution of sodium bicarbonate was added. The mixture was stirred for additional 30 min, water phase extracted with DCM (30 ml), DCM was evaporated, and residue was purified by HPLC. Compound **83** was obtained as yellow oil (20 mg, 27%). ¹H NMR (400 MHz, DMSO-*d₆*), δ: 8.45 (s, 1H), 7.97 (s, 1H), 7.71 (m, 2H), 7.44 (s, 1H), 7.34 (s, 1H), 6.98 (s, 1H), 4.33 (dd, J = 14.8, 7.8 Hz, 1H), 4.19 (dd, J = 14.8, 5.9 Hz, 1H), 3.85 (q, J = 11.4 Hz, 2H), 3.56 (m, 2H), 3.02 (m, 6H), 2.82 (s, 3H), 2.76 (m, 2H), 2.51 (s, 3H), 2.42 (m, 7H), 1.83 (m, 4H), 1.25 (m, 2H), 1.00 (d, J = 6.7 Hz, 3H). LCMS (ESI) [MH]⁺: 709.

### Example 32. 4-Methyl-1-[2-(4-methylsulfonylpiperazin-1-yl)propyl]-5-[[2-[6-(2,2,2-trifluoroethyl)pteridin-4-yl]-2,7-diazaspiro[3.5]nonan-7-yl]methyl]indole-2-carbonitrile (Compound 42)

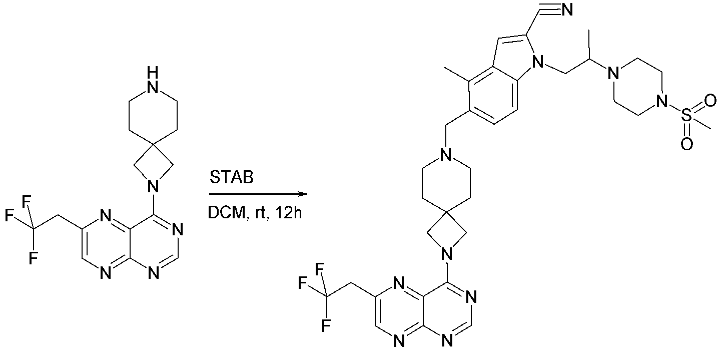

A mixture of 5-formyl-4-methyl-1-{2-[4-(methylsulfonyl)piperazin-1-yl]propyl}-1H-indole-2-carbonitrile (**P98**, 58 mg, 1 eq), sodium triacethoxyborohydride (78 mg, 2.5 eq) and 4-(2,7-diazaspiro[3.5]non-2-yl)-6-(2,2,2-trifluoroethyl)pteridine (**P71**, 50 mg, 0.15 mmol) in DCM (2 ml) stirred at rt (LCMS control), then with stirring water solution of sodium bicarbonate was added. Mixture was stirred for 30 min, water phase was extracted with DCM (30 ml), DCM concentrated, and residue purified by HPLC. Compound **42** was obtained as yellow oil (25mg, 24%). LCMS (ESI) [MH]⁺: 711.8.

### Example 33. 4-Methyl-1-{2-[4-(methylsulfonyl)piperazin-1-yl]propyl}-5-({2-[6-(2,2,2-trifluoroethyl)pyrido[3,2-d]pyrimidin-4-yl]-2,7-diazaspiro[3.5]non-7-yl}methyl)-1H-indole-2-carbonitrile (Compound 67)

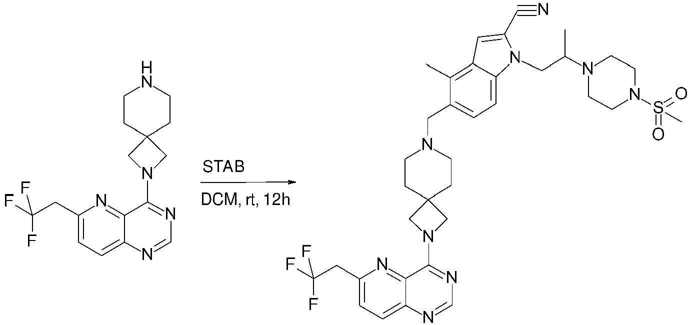

A mixture of 5-formyl-4-methyl-1-{2-[4-(methylsulfonyl)piperazin-1-yl]propyl}-1H-indole-2-carbonitrile (**P98**, 58 mg, 1 eq), sodium triacethoxyborohydride (78 mg, 2.5 eq) and 4-(2,7-diazaspiro[3.5]non-2-yl)-6-(2,2,2-trifluoroethyl)pyrido[3,2-d]pyrimidine (**P61**, 50 mg, 0.15 mmol) in DCM (2 ml) was stirred at rt (LCMS control), then with stirring water solution of sodium bicarbonate was added. The mixture was stirred for 30 min, water phase was extracted with DCM (30 ml), DCM concentrated, and residue purified by HPLC to give compound **67** as yellow oil (15 mg, 14%). LCMS (ESI) [MH]⁺: 710.8.

### Example 34. 4-Methyl-1-{2-[4-(methylsulfonyl)piperazin-1-yl]propyl}-5-({2-[6-(2,2,2-trifluoroethyl)pyrido[3,4-d]pyrimidin-4-yl]-2,7-diazaspiro[3.5]non-7-yl}methyl)-1H-indole-2-carbonitrile (Compound 45)

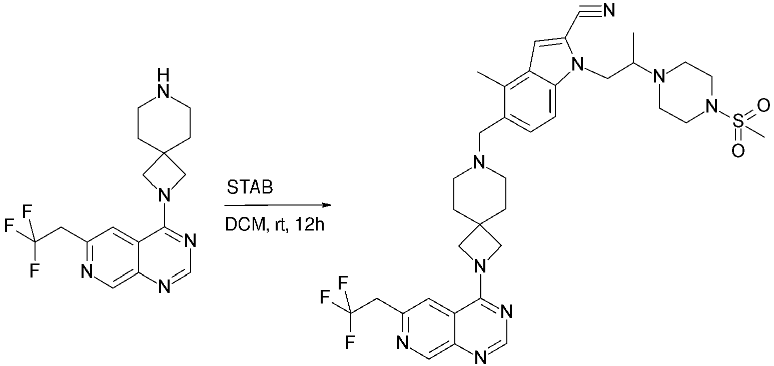

A mixture of 5-formyl-4-methyl-1-{2-[4-(methylsulfonyl)piperazin-1-yl]propyl}-1H-indole-2-carbonitrile (**P98**, 80 mg, 0.21 mmol), triacetoxyborohydride (131 mg, 3 eq) and 4-(2,7-diazaspiro[3.5]non-2-yl)-6-(2,2,2-trifluoroethyl)pyrido[3,4-d]pyrimidine (**P85**, 76 mg, 1.1 eq) in DCM (2 ml) was stirred at rt (LCMS control), then with stirring water solution of sodium bicarbonate was added. The mixture was stirred for additional 30 min, water phase extracted with DCM (30 ml), DCM was concentrated, and residue was purified by HPLC to give compound **45** as yellow oil (35mg, 24%). LCMS (ESI) [MH]⁺: 710.

### Example 35. 5-[[2-[5-Fluoro-6-(2,2,2-trifluoroethyl)quinazolin-4-yl]-2,7-diazaspiro[3.5]nonan-7-yl]methyl]-4-methyl-1-[(5-oxomorpholin-2-yl)methyl]indole-2-carbonitrile (Compound 12)

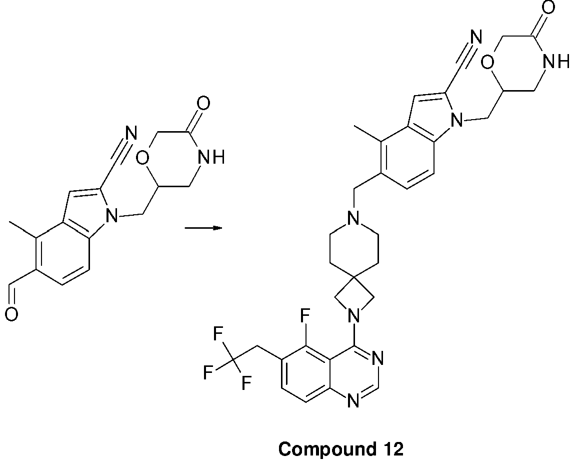

A mixture of 5-formyl-4-methyl-1-[(5-oxomorpholin-2-yl)methyl]-1H-indole-2-carbonitrile (**P105**, 28 mg, 0.1 mmol), 4-(2,7-diazaspiro[3.5]non-2-yl)-5-fluoro-6-(2,2,2-trifluoroethyl)quinazoline (**P137**, 37 mg, 0.1 mmol), DIPEA (130 mg, 1 mmol) and STAB (212 mg, 1 mmol) in DCM (10 ml) was stirred at rt for 48 h (LCMS control), then water solution of sodium bicarbonate was added with stirring. The mixture was stirred for additional 30 min, water phase was extracted with DCM (2x5 ml), the organic extract was evaporated to dryness. The residue was refluxed with MeCN (0.5 ml) for 2 h, cooled, filtered, washed with MeCN (0.5 ml) and dried to yield compound **12** (9 mg, 14 %). ¹H NMR (400 MHz, DMSO-*d₆*), δ: 8.49 (s, 1H), 8.01 (d, J = 4.1 Hz, 1H), 7.80 (t, J = 8.1 Hz, 1H), 7.58 (d, J = 8.6 Hz, 1H), 7.52 (s, 1H), 7.46 (d, J = 8.7 Hz, 1H), 7.30 (d, J = 7.6 Hz, 1H), 4.54 (d, J = 15.3 Hz, 1H), 4.36 (dd, J = 15.4, 7.7 Hz, 1H), 4.01 (m, 6H), 3.85 (m, 3H), 3.49 (s, 2H), 3.16 (t, J = 11.3 Hz, 1H), 2.50 (s, 3H), 2.33 (s, 5H), 1.71 (s, 4H). LCMS (ESI) [MH]⁺: 636.

### Example 36. 4-Methyl-1-{1-methyl-3-[4-(methylsulfonyl)piperazin-1-yl]propyl}-5-({2-[6-(2,2,2-trifluoroethyl)quinazolin-4-yl]-2,7-diazaspiro[3.5]non-7-yl}methyl)-1H-indole-2-carbonitrile (Compound 13)

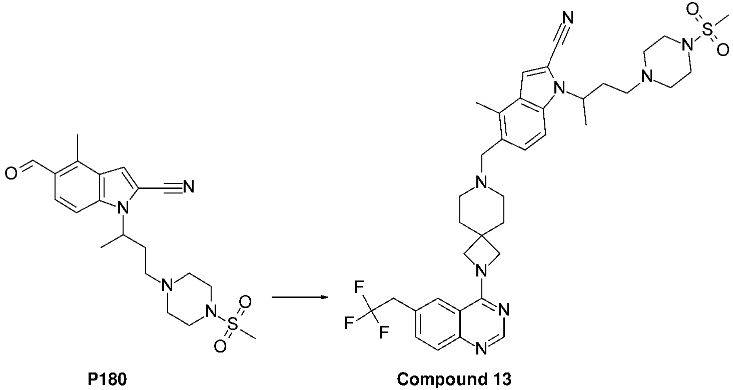

To a mixture of compound 5-formyl-4-methyl-1-{1-methyl-3-[4-(methylsulfonyl)piperazin-1-yl]propyl}-1*H*-indole-2-carbonitrile (**P180**, 96 mg, 0.23 mmol) and 4-(2,7-diazaspiro[3.5]non-2-yl)-6-(2,2,2-trifluoroethyl)quinazoline (**P9**, 77 mg, 0.23 mmol) DIPEA (297 mg, 2.3 mmol) and STAB (488 mg 2.3 mmol) in DCM (30 ml) were added. The mixture was stirred at rt for 48 h (LCMS control), then water solution of sodium bicarbonate was added with stirring. The mixture was stirred for 30 min, water phase was extracted with DCM (2x20 ml), and combined organic phase was evaporated to dryness. The residue was refluxed with MeCN (0.3 ml) for 2 h, cooled, filtered, washed with MeCN (0.5 ml), and dried to yield compound **13** (39 mg, 23 %). ¹H NMR (400 MHz, DMSO-*d*₆), δ: 8.45 (s, 1H), 7.98 (s, 1H), 7.71 (m, 2H), 7.54 (s, 1H), 7.48 (d, *J* = 8.7 Hz, 1H), 7.28 (d, *J* = 8.7 Hz, 1H), 4.87 (s, 1H), 4.21 (s, 4H), 3.86 (m, 2H), 3.51 (s, 2H), 3.29 (s, 2H), 2.95 (m, 4H), 2.83 (s, 3H), 2.50 (s, 3H), 2.36 (m, 5H), 2.09 (m, 5H), 1.78 (s, 4H), 1.62 (d, *J* = 6.9 Hz, 3H). LCMS (ESI) [MH]⁺: 723.

### Example 37. 4-Methyl-1-[(6-oxo-1,6-dihydropyridin-3-yl)methyl]-5-({2-[6-(2,2,2-trifluoroethyl)quinazolin-4-yl]-2,7-diazaspiro[3.5]non-7-yl}methyl)-1H-indole-2-carbonitrile (Compound 14)

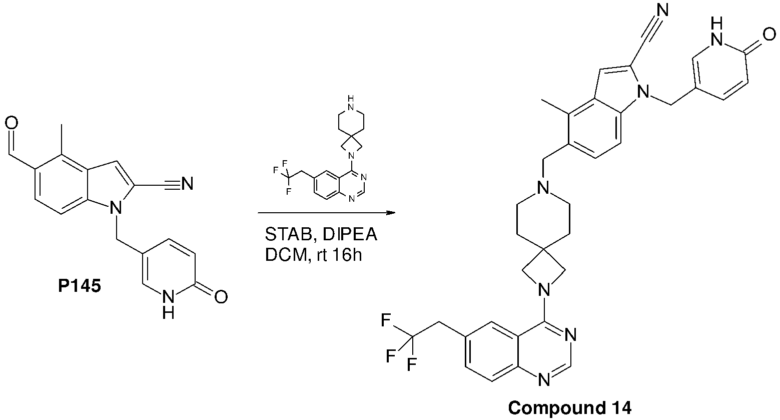

A mixture of 5-formyl-4-methyl-1-[(6-oxo-1,6-dihydropyridin-3-yl)methyl]-1H-indole-2-carbonitrile (**P145**, 100 mg, 0.34 mmol), triacetoxyborohydride (3 eq) and 4-(2,7-diazaspiro[3.5]non-2-yl)-6-(2,2,2-trifluoroethyl)quinazoline (**P9**, 1.2 eq) in DCM (1 ml) was stirred at rt (LCMS control), then with stirring a water solution of sodium bicarbonate was added. The mixture was stirred for additional 30 min, water phase was extracted with DCM (30 ml), DCM was concentrated, and residue was purified by HPLC to afford a yellow solid - compound **14** (33mg, 16%). ¹H NMR (400 MHz, DMSO-*d₆*), δ: 11.58 (s, 1H), 8.44 (s, 1H), 7.98 (s, 1H), 7.74 - 7.68 (m, 2H), 7.60 - 7.54 (m, 2H), 7.42 (s, 1H), 7.33 (d, J = 8.6 Hz, 1H), 7.24 (dd, J = 9.5, 2.6 Hz, 1H), 6.29 (d, J = 9.4 Hz, 1H), 5.28 (s, 2H), 4.18 (d, J = 23.8 Hz, 4H), 3.86 (q, J = 11.6 Hz, 2H), 3.51 (s, 2H), 2.30 (d, J = 59.7 Hz, 4H), 1.78 (s, 4H). LCMS (ESI) [MH]⁺: 613.

### Example 38. 4-Methyl-1-[(4-methyl-5-oxomorpholin-2-yl)methyl]-5-({2-[6-(2,2,2-trifluoroethyl)quinazolin-4-yl]-2,7-diazaspiro[3.5]non-7-yl}methyl)-1H-indole-2-carbonitrile (Compound 15)

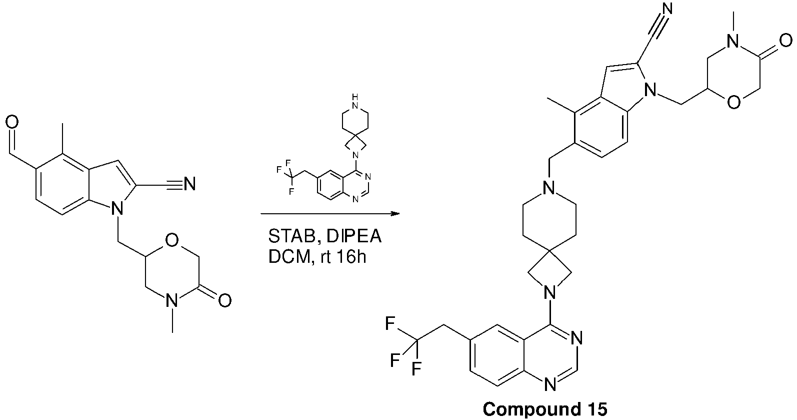

A mixture of 5-formyl-4-methyl-1-[(4-methyl-5-oxomorpholin-2-yl)methyl]-1H-indole-2-carbonitrile (**P183**, 31 mg, 0.1 mmol), triacetoxyborohydride (3eq) and 4-(2,7-diazaspiro[3.5]non-2-yl)-6-(2,2,2-trifluoroethyl)quinazoline (**P9**, 1.2 eq) in DCM (2 ml) was stirred at rt (LCMS control), then water solution of sodium bicarbonate was added with stirring. The mixture was stirred for 30 min more, and water phase was extracted with DCM (30 ml), DCM was concentrated, and residue was purified by HPLC to afford of yellow solid of compound **15** (10 mg, 16 %). ¹H NMR (400 MHz, DMSO-*d₆*), δ: 8.44 (s, 1H), 7.98 (s, 1H), 7.71 (m, 2H), 7.53 (s, 1H), 7.45 (d, J = 8.6 Hz, 1H), 7.32 (d, J = 8.6 Hz, 1H), 4.55 (dd, J = 15.4, 3.3 Hz, 1H), 4.38 (dd, J = 15.4, 7.9 Hz, 1H), 4.18 (m, 5H), 3.94 (m, 4H), 3.52 (s, 2H), 3.40 (m, 2H), 2.85 (s, 3H), 2.51 (m, 3H), 2.38 (s, 4H), 1.78 (s, 4H). LCMS (ESI) [MH]⁺: 633.

### Example 39. 4-Methyl-1-[(4-methyl-3,3-dioxido-1,3,4-oxathiazinan-6-yl)methyl]-5-({2-[6-(2,2,2-trifluoroethyl)quinazolin-4-yl]-2,7-diazaspiro[3.5]non-7-yl}methyl)-1H-indole-2-carbonitrile (Compound 16)

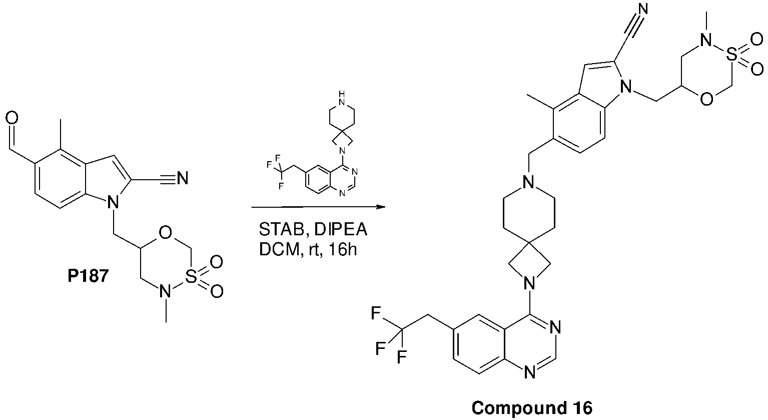

A mixture of 5-formyl-4-methyl-1-[(4-methyl-3,3-dioxido-1,3,4-oxathiazinan-6-yl)methyl]-1H-indole-2-carbonitrile (**P187,** 14 mg, 0.04 mmol), triacetoxyborohydride (3 eq) and 4-(2,7-diazaspiro[3.5]non-2-yl)-6-(2,2,2-trifluoroethyl)quinazoline **(P9,** 1.2 eq) in DCM (2 ml) was stirred at rt (LCMS control), then water solution of sodium bicarbonate was added with stirring. The mixture was stirred for 30 min, water phase was extracted with DCM (30 ml), DCM was concentrated, and residue was purified by HPLC to give yellow solid of compound **16** (10 mg, 37%). LCMS (ESI) [MH]⁺: 669.

### Example 40. 1-[(3,3-Dioxido-1,3,4-oxathiazinan-6-yl)methyl]-4-methyl-5-({2-[6-(2,2,2-trifluoroethyl)quinazolin-4-yl]-2,7-diazaspiro[3.5]non-7-yl}methyl)-1H-indole-2-carbonitrile (Compound 21)

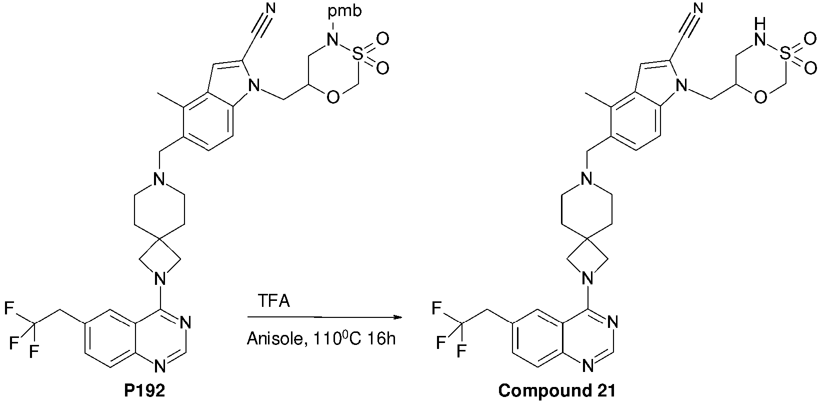

To a solution of 1-{[4-(4-methoxybenzyl)-3,3-dioxido-1,3,4-oxathiazinan-6-yl]methyl}-4-methyl-5-({2-[6-(2,2,2-trifluoroethyl)quinazolin-4-yl]-2,7-diazaspiro[3.5]non-7-yl}methyl)-1H-indole-2-carbonitrile (**P192**, 23 mg, 0.03 mmol) in 1 ml of anisole, TFA (10 eq) was added. And the reaction mixture was stirred at 110°C for 16 h. The reaction mixture was portioned between water and EtOAc. Organic phases were combined, washed with water and brine, dried over anh. Na₂SO₄ and concentrated under vacuum. The residue was purified by HPLC to yield compound **21** as off-white solid (10 mg, 51%). ¹H NMR (400 MHz, DMSO-*d₆*), δ: 8.46 (s, 1H), 7.97 (s, 1H), 7.71 (m, 2H), 7.54 (m, 1H), 7.45 (m, 1H), 7.34 (m, 1H), 7.28 (m, 1H), 4.82 (d, J = 11.8 Hz, 1H), 4.51 (m, 2H), 4.30 (m, 5H), 4.03 (m, 2H), 3.84 (m, 2H), 3.46 (m, 3H), 2.50 (s, 3H), 2.35 (m, 4H), 1.79 (s, 4H). LCMS (ESI) [MH]⁺: 654.

### Example 41. 4-Methyl-1-[(2-oxopiperidin-4-yl)methyl]-5-({2-[6-(2,2,2-trifluoroethyl)quinazolin-4-yl]-2,7-diazaspiro[3.5]non-7-yl}methyl)-1H-indole-2-carbonitrile (Compound 22)

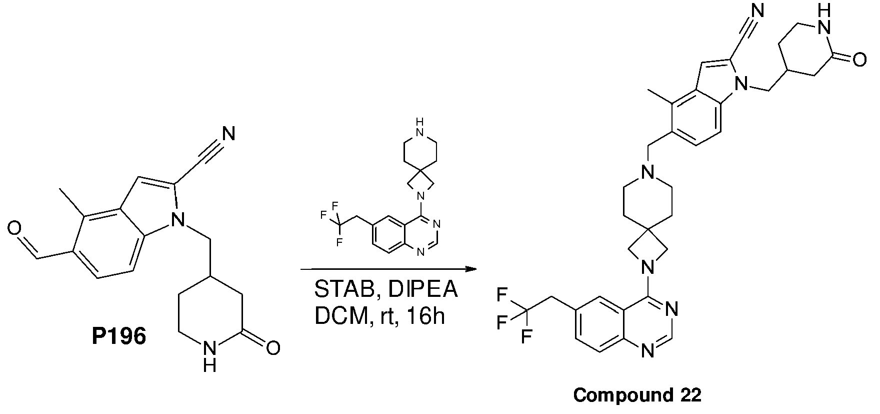

A mixture of 5-formyl-4-methyl-1-[(2-oxopiperidin-4-yl)methyl]-1H-indole-2-carbonitrile (**P196**, 28 mg, 0.1 mmol), triacetoxyborohydride (3 eq) and 4-(2,7-diazaspiro[3.5]non-2-yl)-6-(2,2,2-trifluoroethyl)quinazoline (**P9**, 1.2 eq) in DCM (1 ml) was stirred at rt (LCMS control), then with stirring water solution of sodium bicarbonate was added. Mixture was stirred for additional 30 min, organic phase was separated, water phase was extracted with DCM (30 ml), combined DCM phase was concentrated, and residue was purified by HPLC to afford a yellow oil of compound **22** (9 mg, 15%). LCMS (ESI) [MH]⁺: 617.

### Example 42. 4-Methyl-1-[(6-oxopiperidin-3-yl)methyl]-5-({2-[6-(2,2,2-trifluoroethyl)quinazolin-4-yl]-2,7-diazaspiro[3.5]non-7-yl}methyl)-1H-indole-2-carbonitrile (Compound 72)

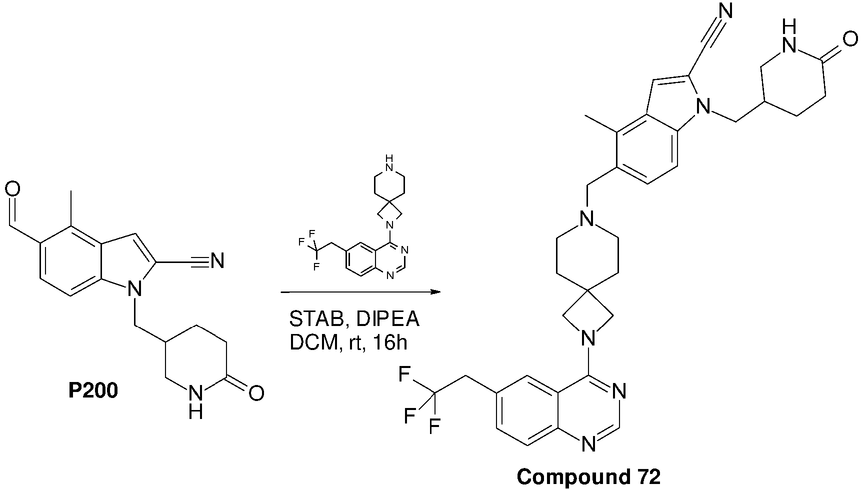

A mixture of 5-formyl-4-methyl-1-[(6-oxopiperidin-3-yl)methyl]-1H-indole-2-carbonitrile (**P200**, 50 mg, 0.17 mmol), triacetoxyborohydride (3 eq) and 4-(2,7-diazaspiro[3.5]non-2-yl)-6-(2,2,2-trifluoroethyl)quinazoline (**P9**, 1.2 eq) in DCM (1 ml) was stirred at rt (LCMS control), then with stirring water solution of sodium bicarbonate was added. The mixture was stirred for additional 30 min, then organic layer was separated, and water phase was extracted with DCM (30 ml), combined organic phase was concentrated and residue was purified by HPLC to give compound **72** as a yellow solid (15 mg, 14%). LCMS (ESI) [MH]⁺: 617.

### Example 43. (3-{[2-Cyano-4-methyl-5-({2-[6-(2,2,2-trifluoroethyl)quinazolin-4-yl]-2,7-diazaspiro[3.5]non-7-yl}methyl)-1H-indol-1-yl]methyl}bicyclo[1.1.1]pent-1-yl)formamide (Compound 95)

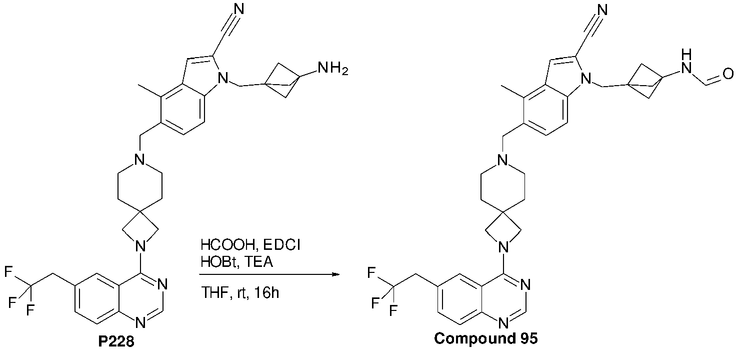

A solution of 1-[(3-amino-1-bicyclo[1.1.1]pentanyl)methyl]-4-methyl-5-[[2-[6-(2,2,2-trifluoroethyl)quinazolin-4-yl]-2,7-diazaspiro[3.5]nonan-7-yl]methyl]indole-2-carbonitrile (**P228,** 50 mg, 0.08 mmol), TEA (3 eq), HOBt (3 eq), HCOOH (3 eq) and EDCI (3 eq) was stirred at rt for 16 h. The solvent was removed under reduced pressure and the residue was purified by HPLC to afford compound **95** (10 mg, 19%) as a yellow solid. ¹H NMR (400 MHz, DMSO-*d₆*), δ: 8.47 (s, 1H), 8.45 (s, 1H), 7.98 (s, 1H), 7.86 (d, J = 1.6 Hz, 1H), 7.72 (m, 2H), 7.54 (s, 1H), 7.38 (d, J = 8.7 Hz, 1H), 7.30 (d, J = 8.6 Hz, 1H), 4.50 (m, 2H), 4.15 (s, 4H), 3.86 (m, 2H), 3.52 (s, 2H), 2.50 (s, 3H), 2.38 (s, 4H), 1.83 (s, 6H), 1.80 (s, 4H). LCMS (ESI) [MH]⁺: 628.

### Example 44. N-{trans-4-[(2-{6-[2-(3-Methyl-1,2,4-oxadiazol-5-yl)ethyl]quinazolin-4-yl}-2,7-diazaspiro[3.5]non-7-yl)methyl]cyclohexyl}ethane-sulfonamide (Compound 94)

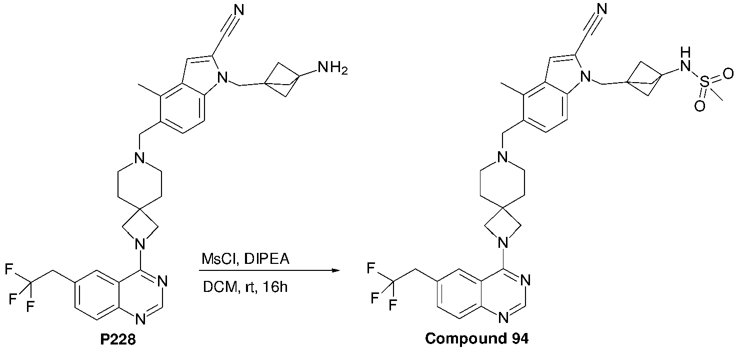

To a solution of 1-[(3-amino-1-bicyclo[1.1.1]pentanyl)methyl]-4-methyl-5-[[2-[6-(2,2,2-trifluoroethyl)quinazolin-4-yl]-2,7-diazaspiro[3.5]nonan-7-yl]methyl]indole-2-carbonitrile (P205, 20 mg, 0.05 mmol) and DIPEA (2 eq) MsCl (1.1eq) was added. The reaction mixture was stirred at rt for 16 h. The solvent was removed under reduced pressure and the residue was purified by HPLC to afford compound 94 (2 mg, 9%) as a yellow solid. LCMS (ESI) [MH]⁺: 678.

### Example 45. 4-Methyl-1-{[(2R)-5-oxomorpholin-2-yl]methyl}-5-({2-[6-(2,2,2-trifluoroethyl)quinazolin-4-yl]-2,7-diazaspiro[3.5]non-7-yl}methyl)-1H-indole-2-carbonitrile (Compound 101)

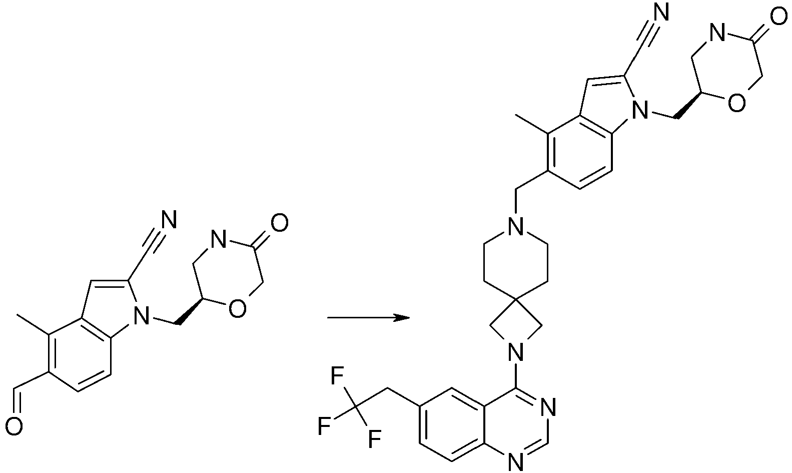

A mixture of 5-formyl-4-methyl-1-{[(2R)-5-oxomorpholin-2-yl]methyl}-1H-indole-2-carbonitrile **(P176,** 177 mg, 0.60 mmol), 4-(2,7-diazaspiro[3.5]non-2-yl)-6-(2,2,2-trifluoroethyl)quinazoline (**P9**, 200 mg, 0.6 mmol), DIPEA (384 mg, 2.98 mmol) and STAB (631 mg, 2.98 mmol) in DCM (30 ml) was stirred for 24 h, at rt (LCMS control), then with stirring a water solution of sodium bicarbonate was added. The mixture was stirred 30 min, water phase extracted with DCM (2x20 ml), evaporated to dryness. Residue was refluxed with MeCN (30 ml) for 2 h, cooled, filtered, washed with MeCN (10 ml), and dried to yield pure 4-methyl-1-{[(2R)-5-oxomorpholin-2-yl]methyl}-5-({2-[6-(2,2,2-trifluoroethyl)quinazolin-4-yl]-2,7-diazaspiro[3.5]non-7-yl}methyl)-1H-indole-2-carbonitrile (Compound **101,** 174 mg, 47 %). ¹H NMR (400 MHz, DMSO-*d₆*), δ: 8.46 (s,1H), 7.99 (m,2H), 7.73 (m,2H), 7.52 (m, 1H), 7.46 (d, J=5.6 Hz, 1H), 7.31 (d, J=4.8 Hz, 1H), 4.54 (m, 1H), 4.20 (m, 2H), 4.05 (m, 1H), 3.90 (m, 4H), 3.52 (m, 2H), 3.35 (m, 1H), 3.17 (m, 1H), 2.39 (m, 2H), 1.79 (m, 4H). LCMS (ESI) [MH]⁺: 618.

### Example 46. 4-Methyl-1-{[(2S)-5-oxomorpholin-2-yl]methyl}-5-({2-[6-(2,2,2-trifluoroethyl)quinazolin-4-yl]-2,7-diazaspiro[3.5]non-7-yl}methyl)-1H-indole-2-carbonitrile (Compound 102)

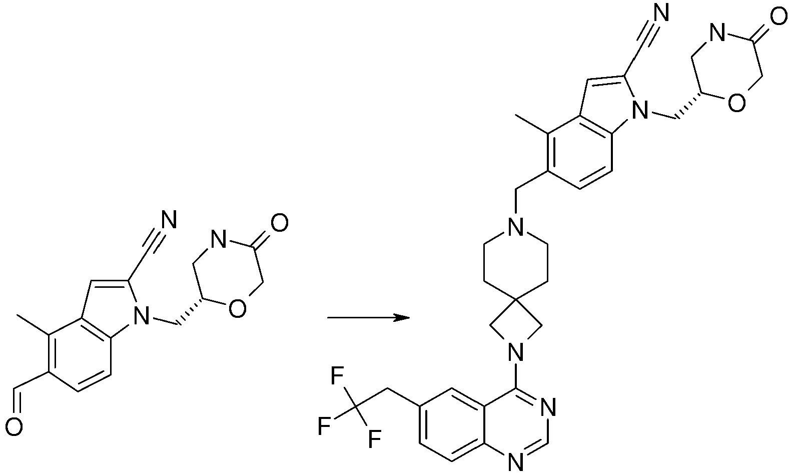

Compound was prepared using procedure described in the ***Example 45*** and 5-formyl-4-methyl-1-{[(2S)-5-oxomorpholin-2-yl]methyl}-1H-indole-2-carbonitrile **P177** instead of 5-formyl-4-methyl-1-{[(2R)-5-oxomorpholin-2-yl]methyl}-1H-indole-2-carbonitrile **P176.** Compound 102 was obtained with yield 49 %. ¹H NMR (400 MHz, DMSO-*d₆*), δ: 8.46 (s,1H), 7.99 (m,2H), 7.73 (m,2H), 7.52 (m, 1H), 7.46 (d, J=5.6 Hz, 1H), 7.31 (d, J=4.8 Hz, 1H), 4.54 (m, 1H), 4.20 (m, 2H), 4.05 (m, 1H), 3.90 (m, 4H), 3.52 (m, 2H), 3.35 (m, 1H), 3.17 (m, 1H), 2.39 (m, 2H), 1.79 (m, 4H). LCMS (ESI) [MH]⁺: 618.

### Example 47. 4-Methyl-1-[(5-oxo-1,4-oxazepan-2-yl)methyl]-5-({2-[6-(2,2,2-trifluoroethyl)quinazolin-4-yl]-2,7-diazaspiro[ 3.5 Jnon-7-yl }methyl)-1H-indole-2-carbonitrile (Compound 87)

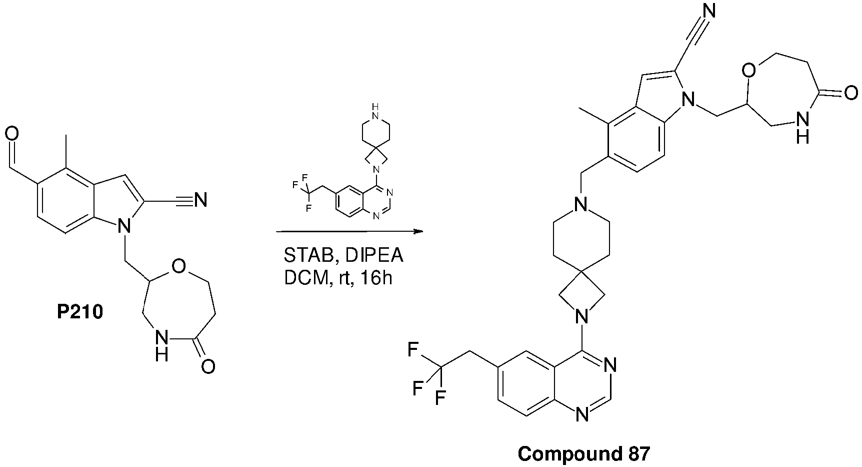

A mixture of 5-formyl-4-methyl-1-[(5-oxo-1,4-oxazepan-2-yl)methyl]-1H-indole-2-carbonitrile **(P210,** 50 mg, 0.16 mmol), triacetoxyborohydride (3 eq) and 4-(2,7-diazaspiro[3.5]non-2-yl)-6-(2,2,2-trifluoroethyl)quinazoline **(P9,** 1.2eq) in DCM (1 ml) was stirred at rt (LCMS control), then with stirring water solution of sodium bicarbonate was added. The mixture was stirred for additional 30 min, organic layer was separated, water phase was extracted with DCM (30 ml), combined DCM solution was concentrated, and residue was purified by HPLC to give a yellow solid of compound **87** (3 mg, 3%). LCMS (ESI) [MH]⁺: 635.

### Example 48. 1-{(2S)-2-[(3R/S)-4-(Methylsulfonyl)-3-methylpiperazin-1-yl]propyl}-4-methyl-5-({2-[6-(2,2,2-trifluoroethyl)quinazolin-4-yl]-2,7-diazaspiro[3.5]non-7-yl}methyl)-1H-indole-2-carbonitrile (Compound 37/38)

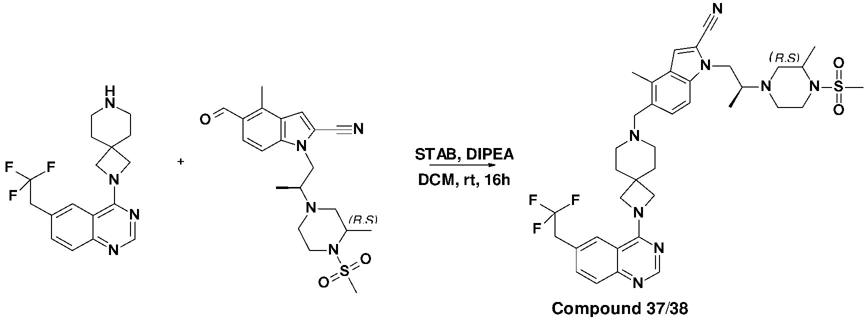

A mixture of 4-(2,7-diazaspiro[3.5]non-2-yl)-6-(2,2,2-trifluoroethyl)quinazoline (**P9**, 100 mg, 0.3 mmol), 1-{(2S)-2-[(3R/S)-4-(methylsulfonyl)-3-methylpiperazin-1-yl]propyl}-5-formyl-4-methyl-1H-indole-2-carbonitrile (120 mg, 0.3 mmol), DIPEA (194 mg, 1.5 mmol) and STAB (315 mg, 1.5 mmol) in DCM (10 ml) was stirred at rt for 16 h (LCMS control), then washed with aqueous NaHCO₃, and extracted with DCM (2x5 ml). *R-* and *S*- isomers were separated by HPLC (silica C-18, MeCN-H₂O). ***SR*-isomer** (Compound **37**): 50 mg (23%), ¹H NMR (400 MHz, CDCl₃), δ: 8.62 (s, 1H), 7.84 (d, J = 8.5 Hz, 1H), 7.78 (s, 1H), 7.64 (d, J = 8.7 Hz, 1H), 7.37 (d, J = 8.6 Hz, 1H), 7.23 (s, 1H), 7.16 (d, J = 8.7 Hz, 1H), 4.36 (dd, J = 14.6, 6.6 Hz, 1H), 4.26 (s, 4H), 4.09 (m, 2H), 3.52 (m, 5H), 3.32 (m, 1H), 3.22 (m, 1H), 2.84 (s, 3H), 2.68 (m, 4H), 2.57 (s, 3H), 2.48 (s, 4H), 1.92 (s, 4H), 1.25 (d, J = 6.7 Hz, 3H), 0.99 (d, J = 6.7 Hz, 3H). LCMS (ESI) [MH]⁺: 724. ***SS*-isomer** (Compound **38**): 60 mg (27%), ¹H NMR (400 MHz, CDCl₃), δ: 8.61 (s, 1H), 7.83 (d, J = 8.6 Hz, 1H), 7.79 (s, 1H), 7.64 (d, J = 8.5 Hz, 1H), 7.36 (d, J = 8.4 Hz, 1H), 7.21 (s, 1H), 7.09 (d, J = 8.4 Hz, 1H), 4.28 (d, J = 19.0 Hz, 5H), 4.08 (dd, J = 14.7, 4.9 Hz, 1H), 3.93 (s, 1H), 3.52 (q, J = 11.0 Hz, 5H), 3.27 (m, 1H), 3.08 (m, 1H), 2.94 (m, 1H), 2.82 (s, 3H), 2.77 (m, 1H), 2.56 (s, 3H), 2.43 (m, 5H), 2.21 (d, J = 11.2 Hz, 1H), 1.89 (s, 4H), 1.09 (d, J = 6.8 Hz, 3H), 0.97 (d, J = 6.6 Hz, 3H). LCMS (ESI) [MH]⁺: 724.

### Example 49. 1-{(2S)-2-[(3R/S)-4-(Ethylsulfonyl)-3-methylpiperazin-1-yl]propyl}-4-methyl-5-({2-[6-(2,2,2-trifluoroethyl)quinazolin-4-yl]-2,7-diazaspiro[3.5]non-7-yl}methyl)-1H-indole-2-carbonitrile (Compound 35/36)

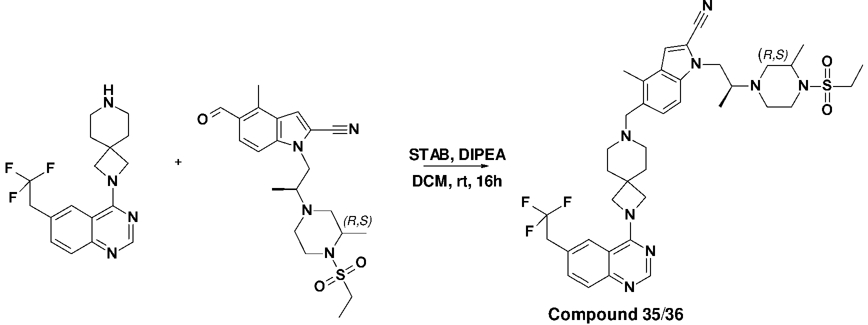

A mixture of 4-(2,7-diazaspiro[3.5]non-2-yl)-6-(2,2,2-trifluoroethyl)quinazoline (**P9**, 0.64 mg, 0.19 mmol), 1-{(2S)-2-[(3R/S)-4-(ethylsulfonyl)-3-methylpiperazin-1-yl]propyl}-5-formyl-4-methyl-1H-indole-2-carbonitrile (78 mg, 0.19 mmol), DIPEA (123 mg, 0.95 mmol) and STAB (200 mg, 0.95 mmol) in DCM (10 ml) was stirred at rt for 16 h (LCMS control), then washed with aqueous NaHCO₃, and extracted with DCM (2x5 ml). R- and S- isomers were separated by HPLC (silica C-18, MeCN-H₂O). ***SR*-isomer** (Compound **35):** 8 mg (14%), ¹H NMR (400 MHz, CDCl₃), δ: 8.61 (s, 1H), 7.83 (d, J = 8.6 Hz, 1H), 7.79 (s, 1H), 7.63 (d, J = 8.6 Hz, 1H), 7.36 (d, J = 8.6 Hz, 1H), 7.22 (s, 1H), 7.15 (d, J = 8.6 Hz, 1H), 4.36 (dd, J = 14.5, 6.5 Hz, 1H), 4.26 (s, 4H), 4.10 (dd, J = 14.6, 8.1 Hz, 1H), 4.01 (s, 1H), 3.52 (m, 5H), 3.29 (m, 2H), 2.95 (m, 2H), 2.69 (m, 2H), 2.60 (m, 2H), 2.56 (s, 3H), 2.46 (s, 4H), 1.89 (s, 4H), 1.34 (t, J = 7.4 Hz, 3H), 1.27 (d, J = 6.7 Hz, 3H), 0.98 (d, J = 6.7 Hz, 3H). LCMS (ESI) [MH]⁺: 738. **SS-isomer** (Compound **36):** 16 mg (28%), ¹H NMR (400 MHz, CDCl₃), δ: 8.61 (s, 1H), 7.83 (d, J = 8.6 Hz, 1H), 7.78 (s, 1H), 7.63 (d, J = 8.4 Hz, 1H), 7.35 (d, J = 8.6 Hz, 1H), 7.20 (s, 1H), 7.09 (d, J = 8.5 Hz, 1H), 4.28 (m, 5H), 4.07 (dd, J = 14.8, 5.1 Hz, 1H), 3.88 (s, 1H), 3.55 (m, 5H), 3.30 (t, J = 12.2 Hz, 1H), 3.07 (m, 1H), 2.93 (m, 3H), 2.75 (m, 1H), 2.56 (s, 3H), 2.46 (s, 4H), 2.35 (m, 1H), 2.21 (d, J = 11.1 Hz, 1H), 1.89 (s, 4H), 1.32 (t, J = 7.4 Hz, 3H), 1.08 (d, J = 6.8 Hz, 3H), 0.98 (d, J = 6.7 Hz, 3H). LCMS (ESI) [MH]⁺: 738.

### Example 50. 4-Methyl-1-{(2S)-2-[4-(methylsulfonyl)-1,4-diazepan-1-yl]propyl}-5-({2-[6-(2,2,2-trifluoroethyl)quinazolin-4-yl]-2,7-diazaspiro[3.5]non-7-yl}methyl)-1H-indole-2-carbonitrile (Compound 39)

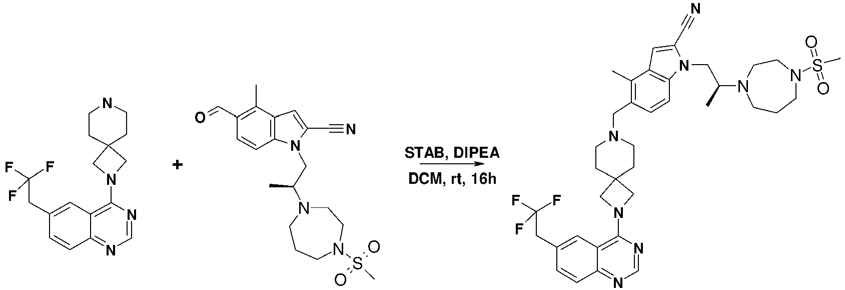

A mixture of 4-(2,7-diazaspiro[3.5]non-2-yl)-6-(2,2,2-trifluoroethyl)quinazoline **(P9,** 70 mg, 0.21 mmol), 5-formyl-4-methyl-1-[(2S)-2-(4-methylsulfonyl-1,4-diazepan-1-yl)propyl]indole-2-carbonitrile **(P252,** 84 mg, 0.21 mmol), DIPEA (150 mg, 1.0 mmol) and STAB (221 mg, 0.21 mmol) in DCM (10 ml) was stirred at rt for 16 h (LCMS control), then washed with water NaHCO₃, extracted with DCM (2x5 ml). The organic layer was dried over MgSO₄, filtered and concentrated. The crude material was purified on silica gel column chromatography (gradient elution, 0-40% EtOAc/MeOH) to yield Compound **39** (102 mg, 68 %) as white solid. ¹H NMR (400 MHz, DMSO-*d₆*), δ: 8.44 (s, 1H), 7.98 (s, 1H), 7.71 (m, 2H), 7.51 (s, 1H), 7.42 (d, *J* = 8.7 Hz, 1H), 7.31 (d, *J* = 8.6 Hz, 1H), 4.25 (m, 6H), 3.86 (m, 2H), 3.52 (m, 2H), 3.16 (m, 5H), 2.80 (m, 2H), 2.73 (s, 3H), 2.55 (m, 5H), 2.38 (m, 4H), 1.78 (s, 4H), 1.61 (m, 2H), 0.98 (d, *J* = 6.6 Hz, 3H). LCMS (ESI) [MH]⁺: 723.

### Example 51. 1-[(4-Ethyl-5-oxomorpholin-2-yl)methyl]-4-methyl-5-({2-[6-(2,2,2-trifluoroethyl)quinazolin-4-yl]-2,7-diazaspiro[3.5]non-7-yl}methyl)-1H-indole-2-carbonitrile (Compound 104)

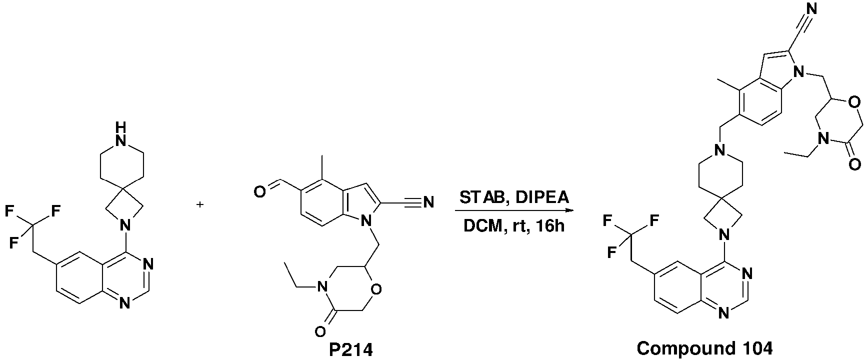

A mixture of 4-(2,7-diazaspiro[3.5]non-2-yl)-6-(2,2,2-trifluoroethyl)quinazoline (**P9**, 52 mg, 0.15 mmol), 1-[(4-ethyl-5-oxomorpholin-2-yl)methyl]-5-formyl-4-methyl-1H-indole-2-carbonitrile (**P214**, 50 mg, 0.15 mmol), DIPEA (98 mg, 0.75 mmol) and STAB (163 mg, 0.75 mmol) in DCM (10 ml) was stirred at rt for 16 h (LCMS control), then washed with aqueous NaHCO₃, extracted with DCM (2x15 ml). The organic layer was dried over MgSO₄, filtered and concentrated. The crude material was purified on silica gel column chromatography (gradient elution, 0-40% EtOAc/MeOH) to yield compound **104** (102 mg, 68 %) as a white solid. ¹H NMR (400 MHz, DMSO-*d₆*), δ: 8.45 (s, 1H), 7.97 (s, 1H), 7.71 (m, 2H), 7.54 (s, 1H), 7.48 (d, J = 8.6 Hz, 1H), 7.32 (d, J = 8.6 Hz, 1H), 4.56 (dd, J = 15.4, 3.0 Hz, 1H), 4.39 (dd, J = 15.4, 8.2 Hz, 1H), 4.23 (s, 4H), 4.15 (m, 1H), 4.02 (d, J = 16.3 Hz, 1H), 3.87 (m, 3H), 3.48 (m, 3H), 3.37 (m, 2H), 3.27 (dd, J = 13.6, 7.0 Hz, 1H), 2.51 (s, 3H), 2.35 (s, 4H), 1.79 (s, 4H), 1.06 (t, J = 7.1 Hz, 3H). LCMS (ESI) [MH]⁺: 646.

### Example 52. 1-[(4-Isobutyl-5-oxomorpholin-2-yl)methyl]-4-methyl-5-({2-[6-(2,2,2-trifluoroethyl)quinazolin-4-yl]-2,7-diazaspiro[3.5]non-7-yl}methyl)-1H-indole-2-carbonitrile (Compound 31)

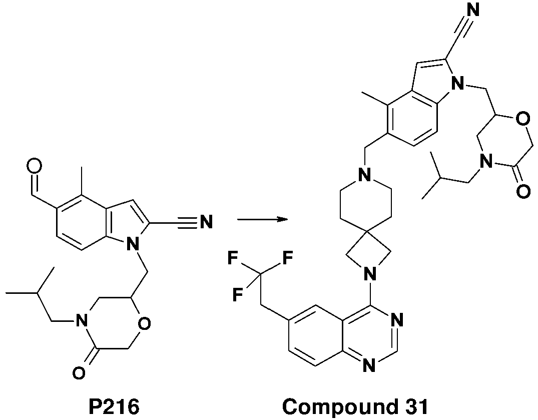

A mixture of 1 4-(2,7-diazaspiro[3.5]non-2-yl)-6-(2,2,2-trifluoroethyl)quinazoline (**P9**, 57 mg, 0.15 mmol), 5-formyl-1-[(4-isobutyl-5-oxomorpholin-2-yl)methyl]-4-methyl-1H-indole-2-carbonitrile (**P216**, 60 mg, 0.17 mmol), DIPEA (98 mg, 0.75 mmol) and STAB (163 mg, 0.75 mmol) in DCM (10 ml) was stirred at rt for 16 h (LCMS control), then it was washed with aq. NaHCO₃, extracted with DCM (2x15 ml). The organic layer was dried over MgSO₄, filtered and concentrated. The crude material was purified on silica gel column chromatography (gradient elution, 0-40% EtOAc/MeOH) to yield compound **31** (56 mg, 68%) as a white solid. ¹H NMR (400 MHz, DMSO-*d₆*), δ: 8.44 (s, 1H), 7.98 (s, 1H), 7.71 (m, 2H), 7.53 (s, 1H), 7.47 (d, *J* = 8.6 Hz, 1H), 7.32 (d, J = 8.6 Hz, 1H), 4.55 (dd, *J* = 15.5, 3.0 Hz, 1H), 4.39 (dd, J = 15.4, 8.1 Hz, 1H), 4.22 (s, 4H), 4.17 (m, 1H), 4.06 (d, *J* = 16.3 Hz, 1H), 3.94 (d, *J* = 16.3 Hz, 1H), 3.86 (m, 2H), 3.52 (s, 2H), 3.45 (dd, *J* = 12.1, 3.1 Hz, 1H), 3.35 (d, J = 11.3 Hz, 1H), 3.21 (dd, *J* = 13.2, 7.9 Hz, 1H), 3.07 (dd, *J* = 13.2, 7.1 Hz, 1H), 2.51 (s, 3H), 2.38 (s, 4H), 1.92 (m, 1H), 1.79 (s, 4H), 0.84 (m, 6H). LCMS (ESI) [MH]⁺: 674.

### Example 53. 4-Methyl-1-[2-(3-oxopiperazin-1-yl)propyl]-5-({2-[6-(2,2,2-trifluoroethyl)quinazolin-4-yl]-2,7-diazaspiro[3.5]non-7-yl}methyl)-1H-indole-2-carbonitrile (Compound 93)

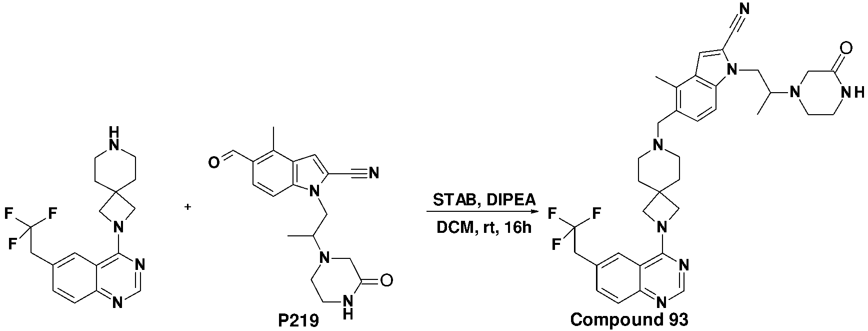

A mixture of 4-(2,7-diazaspiro[3.5]non-2-yl)-6-(2,2,2-trifluoroethyl)quinazoline **(P9,** 91 mg, 0.3 mmol), 5-formyl-4-methyl-1-[2-(3-oxopiperazin-1-yl)propyl]-1H-indole-2-carbonitrile **(P219,** 88 mg, 0.27 mmol), DIPEA (194 mg, 1.5 mmol) and STAB (315 mg, 1.5 mmol) in DCM (10 ml) was stirred at rt for 16 h (LCMS control), then washed with aq. NaHCO₃, extracted with DCM (2x5 ml). Crude product was separated by HPLC (silica C-18, ACN-H₂O) to yield compound **93** (23 mg, 13%). ¹H NMR (400 MHz, DMSO-*d₆*), δ: 8.44 (s, 1H), 7.98 (s, 1H), 7.69 (m, 3H), 7.49 (s, 1H), 7.41 (d, *J* = 8.6 Hz, 1H), 7.30 (d, *J =* 8.6 Hz, 1H), 4.34 (dd, J = 14.9, 8.2 Hz, 1H), 4.23 (s, 4H), 4.18 (dd, *J =* 15.0, 6.2 Hz, 1H), 4.02 (m, 1H), 3.86 (m, 2H), 3.51 (s, 2H), 3.11 (m, 4H), 2.84 (m, 2H), 2.44 (s, 3H), 2.38 (m, 4H), 1.78 (s, 4H), 0.97 (d, *J =* 6.7 Hz, 3H). LCMS (ESI) [MH]⁺: 645.

### Example 54. 1-{2-[4-(Butylsulfonyl)-1,4-diazepan-1-yl]propyl}-4-methyl-5-({2-[6-(2,2,2-trifluoroethyl)quinazolin-4-yl]-2,7-diazaspiro[3.5]non-7-yl}methyl)-1H-indole-2-carbonitrile (Compound 73)

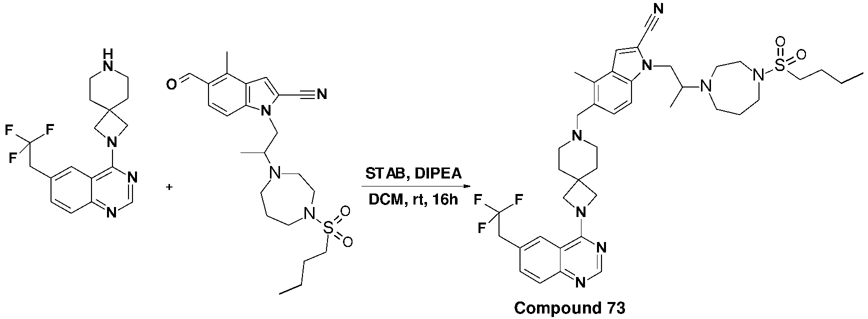

A mixture of 4-(2,7-diazaspiro[3.5]non-2-yl)-6-(2,2,2-trifluoroethyl)quinazoline **(P9,** 30 mg, 0.09 mmol), 1-[2-(4-butyl-1,4-diazepan-1-yl)propyl]-5-formyl-4-methyl-1H-indole-2-carbonitrile (34 mg, 0.09 mmol), DIPEA (65 mg, 0.5 mmol) and STAB (106 mg, 0.5 mmol) in DCM (4 ml) was stirred at rt for 24 h (LCMS control), then washed with aq. NaHCO₃, extracted with DCM (2x5 ml). The residue after evaporation was subjected to column chromatography on silica gel eluting with ethyl acetate-methanol (0 → 30%) to afford compound **73** (41 mg, 60%). ¹H NMR (400 MHz, DMSO-*d₆*), δ: 8.45 (s, 1H), 7.97 (s, 1H), 7.71 (m, 2H), 7.52 (s, 1H), 7.42 (d, *J* = 8.7 Hz, 1H), 7.31 (d, J = 8.9 Hz, 1H), 4.31 (dd, *J* = 14.8, 8.1 Hz, 1H), 4.20 (s, 4H), 4.15 (dd, *J* = 14.8, 6.2 Hz, 1H), 3.86 (m, 2H), 3.52 (s, 2H), 3.20 (m, 5H), 2.92 (m, 2H), 2.78 (m, 2H), 2.58 (m, 1H), 2.50 (s, 3H), 2.38 (s, 4H), 1.79 (s, 4H), 1.56 (m, 5H), 1.34 (h, *J* = 7.4 Hz, 2H), 0.98 (d, *J* = 6.6 Hz, 3H), 0.86 (t, *J* = 7.4 Hz, 3H). LCMS (ESI) [MH]⁺: 765.

### Example 55. 1-[2-(4-Ethylsulfonyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile (Compound 53)

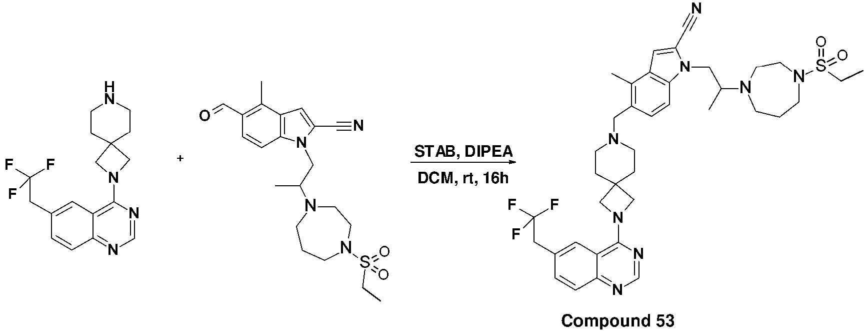

A mixture of 4-(2,7-diazaspiro[3.5]non-2-yl)-6-(2,2,2-trifluoroethyl)quinazoline (**P9**, 50 mg, 0.15 mmol), 1-[2-(4-ethylsulfonyl-1,4-diazepan-1-yl)propyl]-5-formyl-4-methylindole-2-carbonitrile (52 mg, 0.15 mmol), DIPEA (95 mg, 0.74 mmol) and STAB (157 mg, 0.74 mmol) in DCM (4 ml) was stirred at rt for 24 h (LCMS control), then washed with aq. NaHCO₃, extracted with DCM (2x5 ml). The residue after evaporation was subjected to column chromatography on silica gel eluting with ethyl acetate-methanol (0 → 30%) to afford compound **53** (63 mg, 67%). ¹H NMR (400 MHz, DMSO-*d₆*), δ: 8.44 (s, 1H), 7.98 (s, 1H), 7.72 (m, 2H), 7.52 (s, 1H), 7.42 (d, J = 8.6 Hz, 1H), 7.31 (d, J = 8.6 Hz, 1H), 4.31 (dd, J = 14.8, 8.2 Hz, 1H), 4.21 (s, 4H), 4.14 (dd, J = 14.9, 6.3 Hz, 1H), 3.86 (m, 2H), 3.52 (s, 2H), 3.19 (m, 6H), 2.90 (q, J = 7.3 Hz, 2H), 2.78 (m, 2H), 2.57 (m, 1H), 2.50 (s, 3H), 2.38 (s, 4H), 1.78 (s, 4H), 1.60 (m, 2H), 1.10 (t, J = 7.4 Hz, 3H), 0.98 (d, J = 6.7 Hz, 3H). LCMS (ESI) [MH]⁺: 737.

### Example 56. 4-Methyl-1-{(2S)-2-[4-(methylsulfonyl)piperazin-1-yl]propyl}-5-({2-[6-(pentafluoroethyl)quinazolin-4-yl]-2,7-diazaspiro[3.5]non-7-yl}methyl)-1H-indole-2-carbonitrile (Compound 75)

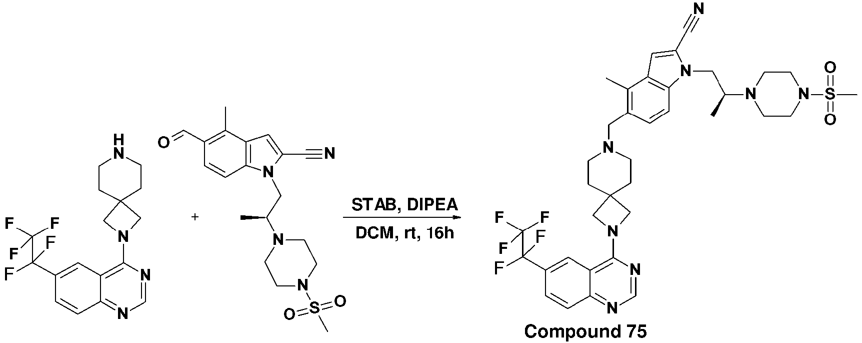

A mixture of 4-(2,7-diazaspiro[3.5]non-2-yl)-6-(pentafluoroethyl)quinazoline (5 mg, 0.013 mmol), 5-formyl-4-methyl-1-[(2S)-2-(4-methylsulfonylpiperazin-1-yl)propyl]indole-2-carbonitrile **(P138,** 5 mg, 0.013 mmol), DIPEA (8 mg, 0.067 mmol) and STAB (14 mg, 0.067 mmol) in DCM (4 ml) was stirred at rt for 24 h (LCMS control), then washed with aq. NaHCO₃, extracted with DCM (2x5 ml). The residue after evaporation was subjected to column chromatography on silica gel eluting with ethyl acetate-methanol (0 → 30%) to afford compound **75** (5 mg, 50 %). LCMS (ESI) [MH]⁺: 745.

### Example 57. 4-Methyl-1-{[(2R,3S)-6-oxo-2-phenylpiperidin-3-yl]methyl}-5-({2-[6-(2,2,2-trifluoroethyl)quinazolin-4-yl]-2,7-diazaspiro[3.5]non-7-yl}methyl)-1H-indole-2-carbonitrile (Compound 100)

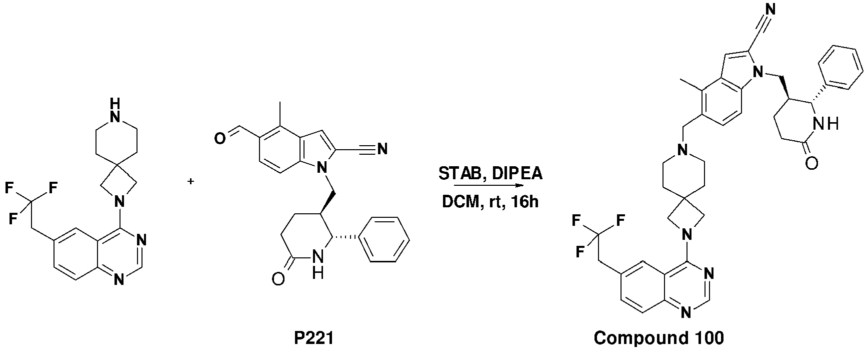

A mixture of 4-(2,7-diazaspiro[3.5]non-2-yl)-6-(2,2,2-trifluoroethyl)quinazoline (**P9**, 98 mg, 0.2 mmol), 5-formyl-4-methyl-1-{[(2*R*,3*S*)-6-oxo-2-phenylpiperidin-3-yl]methyl}-1H-indole-2-carbonitrile (**P221**, 100 mg, 0.2 mmol), DIPEA (250 µl) and STAB (0.285 mg, 1 mmol) in DCM (4 ml) stirred at rt for 24 h (LCMS control), then washed with aq. NaHCO₃, extracted with DCM (2x5 ml). The residue after evaporation was subjected to column chromatography on silica gel eluting with ethyl acetate-methanol (0 → 30%) to afford compound **100** (47 mg, 25%). ¹H NMR (400 MHz, DMSO-*d₆*), δ: 8.44 (s, 1H), 7.97 (s, 1H), 7.77 (s, 1H), 7.71 (m, 2H), 7.51 (s, 1H), 7.41 (m, 2H), 7.34 (m, 3H), 7.22 (d, *J* = 8.6 Hz, 1H), 6.86 (d, *J* = 8.6 Hz, 1H), 4.34 (d, *J* = 8.0 Hz, 1H), 4.27 (dd, *J* = 14.9, 9.8 Hz, 1H), 4.20 (s, 4H), 4.11 (dd, *J* = 15.0, 4.7 Hz, 1H), 3.86 (m, 2H), 3.48 (s, 2H), 2.51 (s, 3H), 2.34 (m, 4H), 2.26 (m, 3H), 1.77 (s, 4H), 1.63 (m, 1H), 1.49 (m, 1H). LCMS (ESI) [MH]⁺: 692.

### Example 58. 4-Methyl-1-[(5-oxopyrrolidin-3-yl)methyl]-5-({2-[6-(2,2,2-trifluoroethyl)quinazolin-4-yl]-2,7-diazaspiro[3.5]non-7-yl}methyl)-1H-indole-2-carbonitrile (Compound 51)

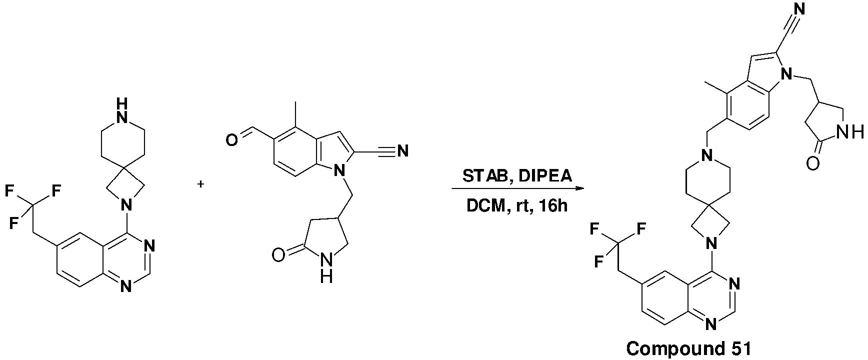

A mixture of 4-(2,7-diazaspiro[3.5]non-2-yl)-6-(2,2,2-trifluoroethyl)quinazoline **(P9,** 19 mg, 0.05 mmol), 5-formyl-4-methyl-1-[(5-oxopyrrolidin-3-yl)methyl]-1H-indole-2-carbonitrile **(P223,** 16 mg, 0.2 mmol), DIPEA (250 µl) and STAB (0.285 mg, 1 mmol) in DCM (4 ml) was stirred at rt for 24 h (LCMS control), then washed with aq. NaHCO₃, extracted with DCM (2x5 ml). The residue after evaporation was subjected to column chromatography on silica gel eluting with ethyl acetate-methanol (0 → 30%) to afford compound **51** (8 mg, 23%). LCMS (ESI) [MH]⁺: 602.

### Example 59. 4-Methyl-1-[(5-oxopyrrolidin-2-yl)methyl]-5-({2-[6-(2,2,2-trifluoroethyl)quinazolin-4-yl]-2,7-diazaspiro[3.5]non-7-yl}methyl)-1H-indole-2-carbonitrile (Compound 52)

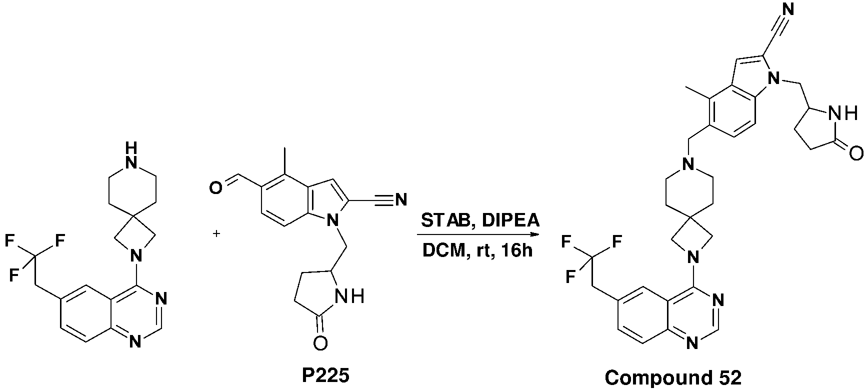

A mixture of 4-(2,7-diazaspiro[3.5]non-2-yl)-6-(2,2,2-trifluoroethyl)quinazoline **(P9,** 47 mg, 0.14 mmol), 5-formyl-4-methyl-1-[(5-oxopyrrolidin-2-yl)methyl]indole-2-carbonitrile (**P225**, 40 mg, 0.14 mmol), DIPEA (130 µl) and STAB (0.150 mg, 0.7 mmol) in DCM (4 ml) was stirred at rt for 24 h (LCMS control), then washed with water NaHCO₃, extracted with DCM (2x5 ml). Crude product was separated by HPLC (silica C-18, ACN-H₂O) to yield compound **52** (8 mg, 23%). LCMS (ESI) [MH]⁺: 602.

### Example 60. N-[3-[[2-Cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]acetamide (Compound 59)

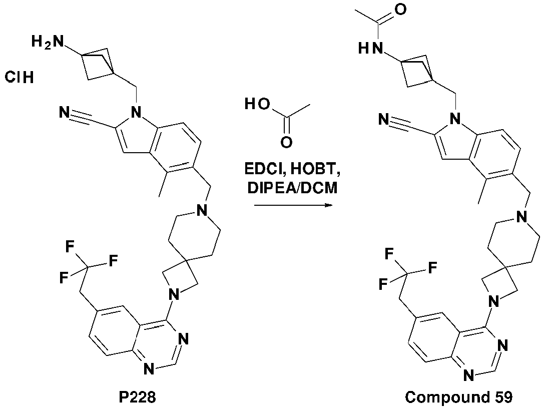

*N*,*N*-Diisopropylethylamine (30 mg, 0.24 mmol) was added to the solution of 1-[(3-amino-1-bicyclo[1.1.1]pentanyl)methyl]-4-methyl-5-[[2-[6-(2,2,2-trifluoroethyl)quinazolin-4-yl]-2,7-diazaspiro[3.5]nonan-7-yl]methyl]indole-2-carbonitrile hydrochloride (**P228**, 60 mg, 0.09 mmol), acetic acid (7 mg, 0.1 mol), 1-hydroxybenzotriazole hydrate (13 mg, 0.1 mmol) and *N*-(3-dimethylaminopropyl)-*N*'-ethylcarbodiimide hydrochloride (22 mg, 0.11 mmol) in DCM (1 ml). The reaction mixture was stirred at ambient temperature for 12 h. The reaction mixture was washed with 10% citric acid (10 ml), 10 % K₂CO₃ (10 ml) and water (10 ml). Organic layer was dried, filtrated and evaporated to dryness. The residue was purified by column chromatography on silica gel with DCM/methanol (0%→10%) as eluent, to afford Compound **59** (30 mg, 50%). ¹H NMR (400 MHz, DMSO-*d₆*), δ: 8.44 (s, 1H), 8.30 (s, 1H), 7.98 (s, 1H), 7.71 (m, 2H), 7.53 (s, 1H), 7.37 (d, J = 8.7 Hz, 1H), 7.30 (d, J = 8.6 Hz, 1H), 4.48 (s, 2H), 4.21 (s, 4H), 3.86 (m, 2H), 3.51 (s, 2H), 2.50 (s, 3H), 2.37 (s, 4H), 1.79 (s, 10H), 1.69 (s, 3H). LCMS (ESI) [MH]⁺: 642.

### Example 61. 1-[3-[[2-Cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]-3-methyl-urea (Compound 60)

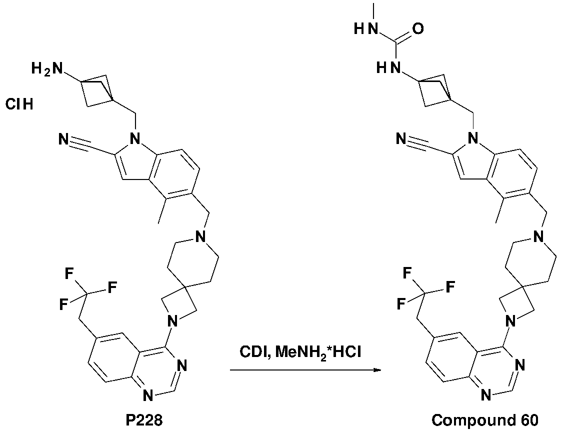

CDI (16 mg, 0.1 mmol) was added to the solution of 1-[(3-amino-1-bicyclo[1.1.1]pentanyl)methyl]-4-methyl-5-[[2-[6-(2,2,2-trifluoroethyl)quinazolin-4-yl]-2,7-diazaspiro[3.5]nonan-7-yl]methyl]indole-2-carbonitrile hydrochloride **(P228,** 60 mg, 0.09 mmol) and Et₃N (25 mg, 0.25 mmol) in DCM (1 ml). The reaction mixture was stirred at ambient temperature for 12 h. Then methanamine hydrochloride (7 mg, 0.1 mmol) was added. The reaction mixture was stirred at ambient temperature for 12 h. The reaction mixture was washed with 10% citric acid (10 ml), 10 % K₂CO₃ (10 ml) and water (10 ml). Organic layer was dried, filtrated and evaporated to dryness. The residue was purified by column chromatography on silica gel with DCM/methanol (0%→10%) as eluent, to afford compound **60** (30 mg, 50%). ¹H NMR (400 MHz, DMSO-*d₆*), δ: 8.45 (s, 1H), 7.98 (s, 1H), 7.71 (m, 2H), 7.53 (s, 1H), 7.38 (d, J = 8.5 Hz, 1H), 7.31 (d, J = 8.7 Hz, 1H), 6.46 (s, 1H), 5.52 (q, J = 4.3 Hz, 1H), 4.47 (s, 2H), 4.22 (s, 4H), 3.86 (m, 2H), 3.56 (s, 2H), 2.50 (s, 3H), 2.45 (d, J = 4.6 Hz, 3H), 2.40 (s, 4H), 1.81 (s, 4H), 1.75 (s, 6H). LCMS (ESI) [MH]⁺: 657.

### Example 62. 4-[7-[[2-cyano-4-methyl-1-[[3-(sulfamoylamino)-1-bicyclo[1.1.1]pentanyl]methyl]indol-5-yl]methyl]-2,7-diazaspiro[3.5]nonan-2-yl]-6-(2,2,2-trifluoroethyl)quinazoline (Compound 61)

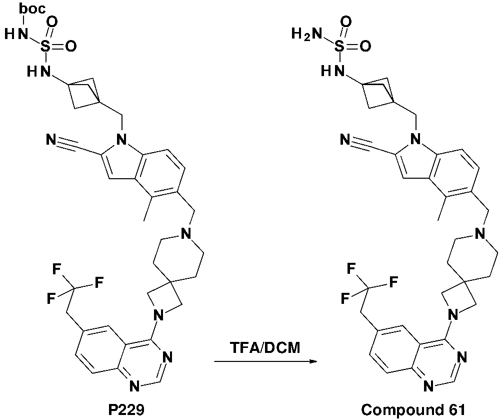

Trifluoroacetic acid (140 mg, 1.15 mmol) was added dropwise to a stirred solution of *tert-*butyl *N*-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]sulfamoyl]carbamate (**P229**, 120 mg, 0.15 mmol) in anhydrous DCM (5 ml) at 0°C. The solution was stirred at rt for 4 h. The reaction mixture was evaporated to afford the 4-[7-[[2-cyano-4-methyl-1-[[3-(sulfamoylamino)-1-bicyclo[1.1.1]pentanyl]methyl]indol-5-yl]methyl]-2,7-diazaspiro[3.5]nonan-2-yl]-6-(2,2,2-trifluoroethyl)quinazoline (compound **61**, 100 mg, 98%) as trifluoroacetate. ¹H NMR (400 MHz, DMSO-*d₆*), δ: 8.86 (s, 1H), 8.05 (m, 2H), 7.84 (m, 1H), 7.69 (s, 1H), 7.60 (d, J = 8.7 Hz, 1H), 7.51 (d, J = 8.7 Hz, 1H), 7.39 (s, 1H), 6.52 (s, 2H), 4.10-5.10 (m, 8H), 3.95 (m, 2H), 3.39 (m, 2H), 3.21 (m, 2H), 2.62 (s, 3H), 2.25 (m, 2H), 1.99 (m, 2H), 1.77 (s, 6H). LCMS (ESI) [MH]⁺: 679.

### Example 63. 4-Methyl-5-[[2-[6-(2,2,2-trifluoroethyl)quinazolin-4-yl]-2,7-diazaspiro[3.5]nonan-7-yl]methyl]-1-[[3-(1,1,4-trioxo-1,2,5-thiadiazolidin-2-yl)-1-bicyclo[1.1.1]pentanyl]methyl]indole-2-carbonitrile (Compound 62)

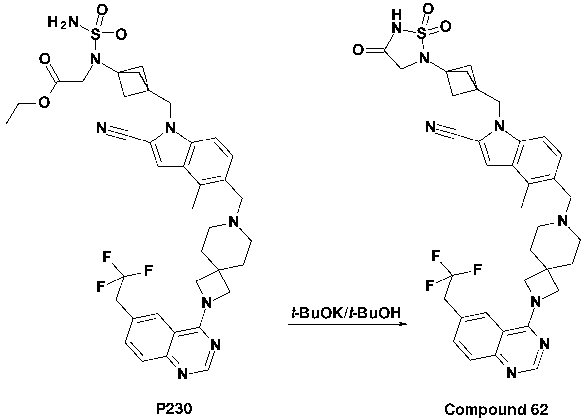

To ethyl 2-[[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]-sulfamoyl-amino]acetate (**P230**, 100 mg, 0.14 mmol), *tert*-BuOK (17 mg, 0.15 mmol) in THF (2 ml) was added and reaction mixture was stirred at rt for 12 h. Water (5 ml) and EtOAc (5 ml) were added. The organic layer was separated, washed with brine, dried over sodium sulfate, filtered, and the filtrate was evaporated. The residue after evaporation was subjected to HPLC to afford the target compound (**62**, 5 mg, 6%). LCMS (ESI) [MH]⁺: 719.

### Biological Assays

### Example A. Primary Assay Used to Determine Potency of MEN1 activity Inhibition.

Compound activity was determined using recombinant MEN1 protein (Creativebiomart, Cat# MEN1-35H) and a custom fluorescein-labeled MLL4-43 peptide (Eton Bioscience Inc.). Interaction between MEN1 and MLL4-43 in the presence of compounds was determined by fluorescence polarization assay using a Microplate Reader ClarioStar Plus. The reaction was carried out in assay buffer (50mM TRIS-HCl pH 7.4-7.6, 50 mM NaCl, 1 mM DTT, 0.1 mg/ml BSA). The compounds were dispensed on a 384 well Diamond Well Plate (Axigen, Cat# P-384-120SQ-C-S) using the Biomek FX liquid handling system at 100x solutions of compounds in DMSO. 2x MEN1 mix (final concentration of MEN1 10nM) was prepared in Assay buffer and 10 µl of mixture per well was added into 384w white Reaction plate with NBS (Corning, Cat#4513). 10 µl of Assay buffer w/o MEN1 was used for negative control. Plates were centrifuged for 1 min at 100 g. Next step the Compounds were added to Reaction plate using Biomek station via following steps: 3 µl of 100x compounds (in DMSO) were mixed thoroughly with 27 µl Assay Buffer, then 2 µl of this mixture was added to Reaction plate with 10 µl of MEN1 mix. Plates were centrifuged for 1 min at 100 g and incubated for 20 min at room temperature. Next 8 µL of MLL4-43 peptide per well was added to final concentration of MLL 0.5 nM. Plates were incubated for 1 hour at room temperature. Then fluorescence polarization was measured using Microplate Reader. The results of this assay are shown in the **Table A.** The values of EC₅₀ shown as a letters A-E, where: A ≤ 0.075 µM; 0.075 µM < B ≤ 0.5 µM; 0.5 µM < C ≤ 1 µM; 1 < D ≤ 5 µM; E >5.

**Table A: MEN1 Activity Inhibition Assays**

| **Compound Number** | **EC₅₀, uM^{*}** | **Compound Number** | **EC₅₀, uM^{*}** | **Compound Number** | **EC₅₀, uM^{*}** | **Compound Number** | **EC₅₀, uM^{*}** |
|---|---|---|---|---|---|---|---|
| **1** | A | **25** | A | **59** | A | **82** | A |
| **2** | A | **26** | A | **60** | A | **83** | E |
| **3** | A | **27** | A | **61** | A | **86** | B |
| **4** | A | **28** | A | **62** | A | **87** | A |
| **5** | A | **29** | A | **63** | A | **89** | A |
| **6** | D | **30** | A | **64** | A | **91** | A |
| **7** | A | **31** | B | **65** | A | **93** | A |
| **9** | B | **40** | A | **66** | A | **94** | A |
| **12** | A | **41** | B | **67** | A | **95** | A |
| **13** | A | **42** | A | **68** | A | **97** | A |
| **14** | A | **44** | A | **70** | A | **98** | A |
| **15** | A | **45** | A | **71** | A | **99** | A |
| **16** | A | **46** | D | **72** | A | **100** | B |
| **17** | A | **47** | A | **73** | A | **101** | A |
| **18** | A | **50** | A | **74** | A | **102** | A |
| **20** | A | **51** | A | **75** | A | **104** | A |
| **21** | A | **52** | A | **78** | B | **105** | C |
| **22** | A | **53** | A | **79** | E | | |
| **23** | A | **57** | A | **80** | A | | |
| **24** | A | **58** | A | **81** | A | | |

| | | | | | | | |
|---|---|---|---|---|---|---|---|
| *-EC₅₀: Half maximal effective concentration: the concentration of a compound which induces a response halfway between the baseline and maximum after a specific exposure time; ECso: A ≤ 0.075 µM; 0.075 µM < B ≤ 0.5 µM; 0.5 µM < C ≤ 1 µM; 1 < D ≤ 5 µM; E > 5 | | | | | | | |

### Example B. Cellular Growth Inhibition Assay.

HEK293 (Institute of Cytology Russian Academy of Science), MV4-11 (ATCC, CRL-9591), MOLM-13 (AcceGen, ABC-TC517S) were seeded at a density of 500 cells per well (HEK293) and 2000 cells per well (MV4-11, MOLM-13) in a 384-well clear bottom plate (Greiner Cat #781090) in 45 µl total volume of DMEM (PanEco, Cat# C420, Russia) or RPMI (PanEco, Cat# C330, Russia) with 10% FBS (HyClone Cat #SV30160.03). HEK293 were allowed to adhere overnight at 37°C, 5% CO₂. 500x compounds solutions in DMSO (Sigma Cat #D2650) were prepared into Cmpnds plate (Diamond Well Plate, Axigen, Cat#P-384-120SQ-C-S) and DMSO only control was included. 1 µl of 500x compounds (Cmpnds plate) was added to 49 µl of culture medium into Dilution plate (Diamond Well Plate, Axigen, Cat#P-384-120SQ-C-S), mixed and then 5 µl of 10x compounds solutions were transferred to cells followed by centrifugation at 100 g for 1 min. Final DMSO concentration was 0.2%. After 3 days of incubation, 10 µl of 1x compounds were added to cells. After 7 days of incubation, 12 µl of CellTiter-Glo (Promega, CAT#G7572) were added to the cells, plate was centrifuged at 100 g for 1 min and luminescence signal was measured using Microplate Reader (CLARIOStar). The results of these assays are shown in the **Tables B1, B2,** and **B3.**

**Table B1: MV4-11 Cellular Growth Inhibition Assay**

| Compound Number | MV4-11, CC_{50,}* µM | Compound Number | MV4-11, CC_{50,}* µM | Compound Number | MV4-11, CC₅₀,* µM | Compound Number | MV4-11, CC₅₀,* µM |
|---|---|---|---|---|---|---|---|
| **2** | B | **26** | A | **59** | A | **80** | B |
| **3** | B | **27** | A | **60** | A | **81** | A |
| **4** | B | **28** | A | **61** | A | **82** | A |
| **5** | B | **29** | A | **63** | B | **89** | A |
| **7** | A | **30** | A | **64** | A | **91** | C |
| **12** | B | **40** | B | **65** | B | **93** | A |
| **13** | A | **42** | B | **66** | B | **94** | B |
| **14** | A | **44** | D | **67** | B | **95** | A |
| **17** | B | **45** | B | **68** | B | **97** | C |
| **18** | A | **47** | B | **70** | A | **98** | C |
| **20** | B | **50** | A | **71** | A | **99** | D |
| **22** | B | **51** | B | **72** | B | **101** | B |
| **23** | A | **53** | A | **73** | A | **102** | A |
| **24** | B | **57** | A | **74** | A | | |
| **25** | B | **58** | B | **79** | C | | |

| | | | | | | | |
|---|---|---|---|---|---|---|---|
| *-CC₅₀: Cytotoxic Concentration: the extract concentration that reduced the cell viability by 50% when compared to untreated controls; MV4-11 CC₅₀: A ≤ 0.1µM; 0.1 µM < B ≤ 0.5 µM; 0.5 µM < C ≤ 1 µM; 1 < D ≤ 5 µM; E >5 | | | | | | | |

**Table B2: MOLM-13 Cellular Growth Inhibition Assay**

| Compound Number | MOLM-13 CC₅₀,^{a} µM | Compound Number | MOLM-13 CC₅₀,^{a} µM | Compound Number | MOLM-13 CC₅₀,^{a} µM | Compound Number | MOLM-13 CC₅₀,^{a} µM |
|---|---|---|---|---|---|---|---|
| **2** | D | **26** | C | **59** | A | **80** | D |
| **3** | D | **27** | B | **60** | A | **81** | B |
| **4** | C | **28** | B | **61** | B | **82** | B |
| **5** | C | **29** | B | **63** | B | **89** | C |
| **7** | B | **30** | B | **64** | C | **91** | D |
| **12** | C | **40** | D | **65** | D | **93** | C |
| **13** | C | **42** | B | **66** | B | **94** | B |
| **14** | A | **44** | E | **67** | B | **95** | B |
| **17** | D | **45** | D | **68** | C | **97** | D |
| **18** | C | **47** | D | **70** | B | **98** | D |
| **20** | D | **50** | D | **71** | B | **99** | D |
| **22** | C | **51** | D | **72** | C | **101** | D |
| **23** | D | **53** | B | **73** | C | **102** | B |
| **24** | D | **57** | B | **74** | B | | |
| **25** | C | **58** | B | **79** | D | | |

| | | | | | | | |
|---|---|---|---|---|---|---|---|
| *-CC₅₀: Cytotoxic Concentration: the extract concentration that reduced the cell viability by 50% when compared to untreated controls; MOLM-13 CC₅₀: A ≤ 0.1µM; 0.1 µM < B ≤ 0.5 µM; 0.5 µM < C ≤ 1 µM; 1 < D ≤ 5 µM; E >5 | | | | | | | |

**Table B3: HEK293 Cellular Growth Inhibition Assay**

| Compound Number | HEK293, CC₅₀,* µM | Compound Number | HEK293, CC₅₀,* µM | Compound Number | HEK293, CC₅₀,* µM | Compound Number | HEK293, CC₅ₒ, * µM |
|---|---|---|---|---|---|---|---|
| **2** | B | **26** | B | **59** | B | **80** | C |
| **3** | B | **27** | C | **60** | B | **81** | D |
| **4** | B | **28** | C | **61** | C | **82** | B |
| **5** | A | **29** | C | **63** | B | **89** | A |
| **7** | B | **30** | C | **64** | C | **91** | C |
| **12** | A | **40** | D | **65** | A | **93** | B |
| **13** | C | **42** | A | **66** | B | **94** | C |
| **14** | A | **44** | B | **67** | C | **95** | B |
| **17** | C | **45** | C | **68** | B | **97** | B |
| **18** | B | **47** | D | **70** | C | **98** | C |
| **20** | B | **50** | B | **71** | C | **99** | A |
| **22** | B | **51** | B | **72** | A | **101** | B |
| **23** | B | **53** | C | **73** | E | **102** | A |
| **24** | B | **57** | C | **74** | D | | |
| **25** | B | **58** | A | **79** | C | | |

| | | | | | | | |
|---|---|---|---|---|---|---|---|
| *-CC₅₀: Cytotoxic Concentration: the extract concentration that reduced the cell viability by 50% when compared to untreated controls; HEK293 CCso: A ≤ 2 µM; 2 µM < B ≤ 5 µM; 5 µM < C ≤ 10 µM; 10 < D ≤ 20 µM; E > 20 | | | | | | | |

### Equivalents

Those skilled in the art will recognize, or be able to ascertain, using no more than routine experimentation, numerous equivalents to the specific embodiments described specifically herein. Such equivalents are intended to be encompassed in the scope of the following claims.

### EMBODIMENTS

1. An embodiment of the present invention is a compound of Formula (I): 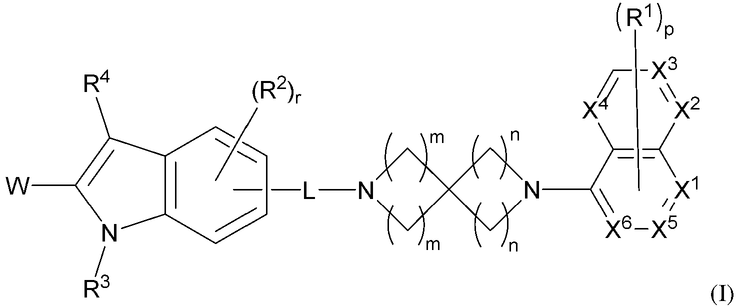
or a pharmaceutically acceptable salt, prodrug, stereoisomer, solvate, or tautomer thereof,
wherein
   X¹, X², X³, X⁴, X⁵, and X⁶ are each independently selected from CH or N;
   and at least one of X¹, X², X³, X⁴, X⁵, and X⁶ is N;
   W is -CN;
   or W and ring B together with the atoms to which they are attached and any intervening atoms, form a 5-10 membered heterocycle;
   each R¹ is independently selected from halogen, C₁-C₆ alkyl, C₁-C₆ alkoxy, C₃-C₁₀ cycloalkyl, C₂-C₆ alkenyl, C₂-C₆ alkynyl, heterocycle, aryl, heteroaryl, OH, NH₂, NHCH₃, and N(CH₃)₂ wherein the alkyl, alkoxy, alkenyl, alkynyl, heterocycle, cycloalkyl, aryl, or heteroaryl is optionally substituted with one or more substituents independently selected from halogen, OH, NR⁹R¹⁰, C₁-C₆ alkyl, C₁-C₆ alkoxy, C₃-C₁₀ cycloalkyl, C₂-C₆ alkenyl, C₂-C₆ alkynyl, heterocycle, aryl, heteroaryl;
   each R² is independently selected from halogen, OH, CN, C₁-C₆ alkyl, C₁-C₆ alkoxy, NR⁹R¹⁰, C₃-C₁₀ cycloalkyl, aryl, heterocyclyl, heteroaryl;
   L is selected from (CR⁵₂)_{q}, (CR⁵₂)_{q}O, (CR⁵₂)_{q}S(O)ₛ and (CR⁵₂)_{q}C(O);
   R³ is selected from hydrogen, deuterium, C₁-C₆ alkyl, C₃-C₁₀ cycloalkyl, heterocyclyl, aryl, heteroaryl and L⁴;
   L⁴ is 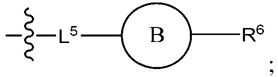 wherein,
      L⁵ is selected from (CR⁵₂)_{q}, (CR⁵₂)_{q}O, (CR⁵₂)_{q}S(O)ₛ and (CR⁵₂)_{q}C(O);
      Ring B is selected from C₃-C₁₄ cycloalkyl, 3- to 10-membered heterocycle, aryl, and heteroaryl, wherein cycloalkyl, heterocycle, aryl, or heteroaryl is optionally substituted with one or more substituents independently selected from halogen, CN, NO₂, oxo, OH, NR⁹R¹⁰, C₁-C₆ alkyl, C₁-C₆ alkoxy, cycloalkyl, heterocycle, aryl, and heteroaryl;
      R⁴ is selected from H, C₁-C₆ alkyl, C₂-C₆ alkenyl, C₂-C₆ alkynyl, C₃-C₁₀ cycloalkyl, C₁-C₆ alkyl-C₁-C₆ alkoxy, C(O)R⁷, heterocyclyl, aryl, or heteroaryl, wherein the alkyl, alkenyl, alkynyl, cycloalkyl, heterocyclyl, aryl, or heteroaryl is optionally substituted with one or more substituents independently selected from halogen, OH, NH₂, C₁-C₆ alkyl, and C₁-C₆ alkoxy;
      each R⁵ is independently selected from H, halogen, CN, OH, C₁-C₆ alkyl, C₂-C₆ alkenyl, C₂-C₆ alkynyl, C₃-C₁₀ cycloalkyl;
      R⁶ is selected from H, C₁-C₆ alkyl, -C(O)R⁷, -NHC(O)R⁷, S(O)ₛR¹¹, -NHS(O)ₛR¹¹, -NHS(O)₂NR⁹R¹⁰, 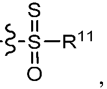 C₃-C₁₀ cycloalkyl, heterocyclyl, aryl, heteroaryl, C₁-C₆ alkanediyl C₃-C₁₀ cycloalkyl, C₁-C₆ alkanediyl heterocyclyl, C₁-C₆ alkanediyl aryl, and C₁-C₆ alkanediyl heteroaryl, wherein alkyl, cycloalkyl, aryl, or heteroaryl is optionally substituted with one or more substituents independently selected from halogen, CN, NO₂, oxo, OH, NH₂, C₁-C₆ alkyl, C₁-C₆ alkoxy, cycloalkyl, heterocycle, aryl, and heteroaryl;
      R⁷ is selected from R⁸, OR⁸, NR⁹R¹⁰,
      R⁸ is selected from H, C₁-C₆ alkyl, C₂-C₆ alkenyl, C₂-C₆ alkynyl, C₃-C₁₀ cycloalkyl, wherein alkyl, alkenyl, alkynyl or cycloalkyl is optionally substituted with one or more substituents independently selected from halogen, OH, CN, NO₂, NR⁹R¹⁰;
      each R⁹ and R¹⁰ is independently H, C₁-C₆ alkyl, C₂-C₆ alkenyl, C₂-C₆ alkynyl, C₃-C₁₀ cycloalkyl, aryl, heteroaryl, or heterocycle, wherein the alkyl, alkenyl, alkynyl, cycloalkyl, aryl, heteroaryl, or heterocycle is optionally substituted with one or more substituents independently selected from halogen, OH, NH₂, C₁-C₆ alkyl, and C₁-C₆ alkoxy;
      or R⁹ and R¹⁰ together with the atoms to which they are attached and any intervening atoms, form a 3-14 membered heterocycle;
      R¹¹ is selected from C₁-C₆ alkyl, C₃-C₁₀ cycloalkyl, C₁-C₆ alkoxy, NR⁹R¹⁰;
      m and n are each an integer independently selected from 1, 2, and 3;
      p is an integer selected from 0, 1, and 2;
      r is an integer selected from 0, 1, 2 and 3;
      q is an integer selected from 0, 1, 2, 3 and 4;
      s is an integer selected from 0, 1, and 2;
         wherein,
      cycloalkyl is a mono or polycyclic saturated carbon rings containing 3-18 carbon atoms;
      aryl is a cyclic, aromatic hydrocarbon groups that have 1 to 3 aromatic rings;
      heterocyclyl is a saturated or partially unsaturated 3-10 membered monocyclic, 7-12 membered bicyclic (fused, bridged, or spiro rings), or 11-14 membered tricyclic ring system (fused, bridged, or spiro rings) having one or more heteroatoms selected from O, N, S, P, Se, or B;
      heteroaryl is a monovalent monocyclic or a polycyclic aromatic radical of 5 to 24 ring atoms, containing one or more ring heteroatoms selected from N, O, S, P, or B, the remaining ring atoms being C.
2. A further embodiment of the present invention is the compound of embodiment 1, wherein the compound is of Formula (I'): 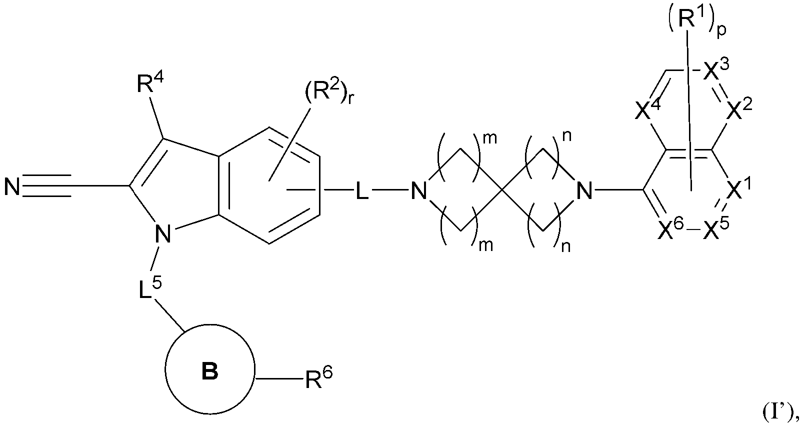 or a pharmaceutically acceptable salt, stereoisomer, solvate, or tautomer thereof.
3. A further embodiment of the present invention is the compound of any one of the preceding embodiments, wherein the compound is of Formula (I-A), (I-B), (I-C), or (I-D): 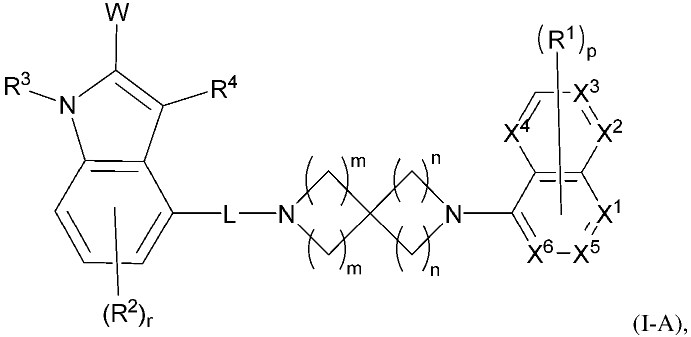 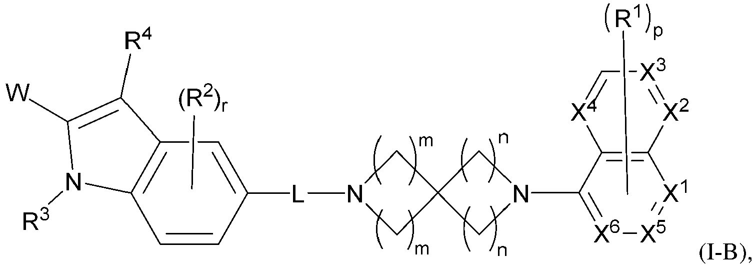 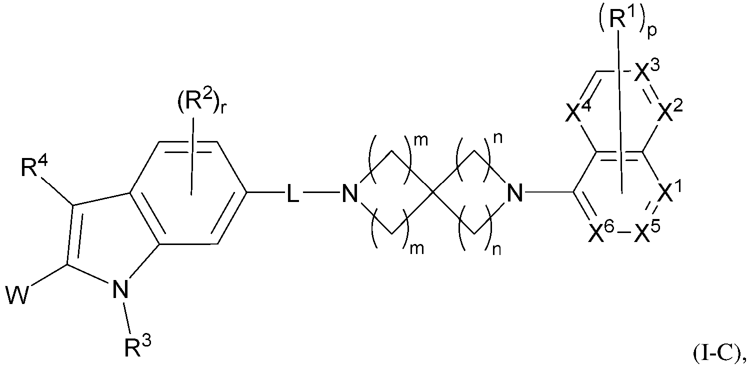 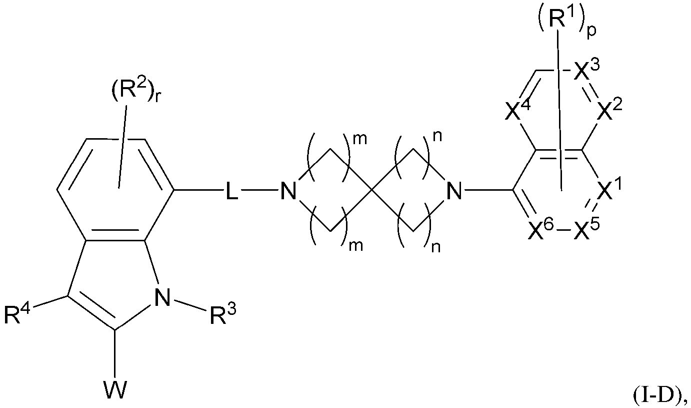 or a pharmaceutically acceptable salt, stereoisomer, solvate, or tautomer thereof.
4. A further embodiment of the present invention is the compound of any one of the preceding embodiments, wherein the compound is of Formula (I-A'), (I-B'), (I-C'), (I-D'): 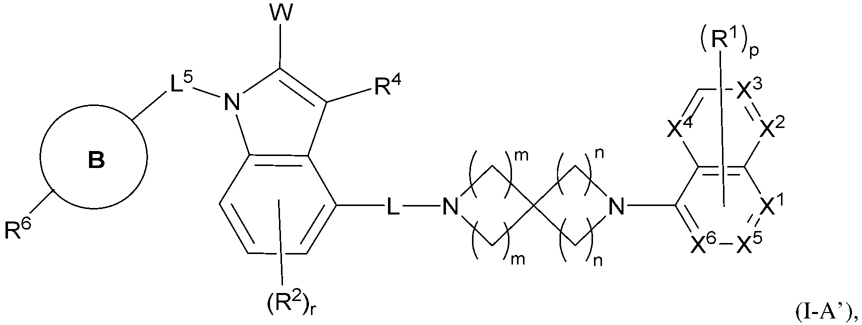 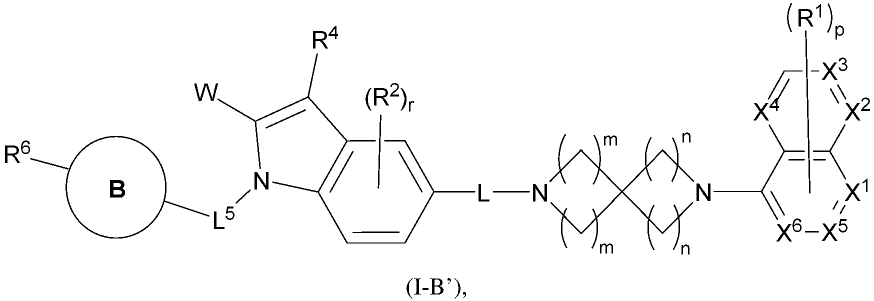 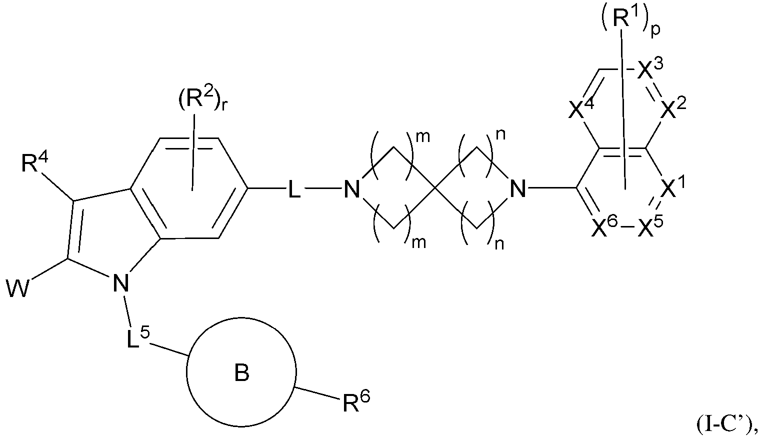 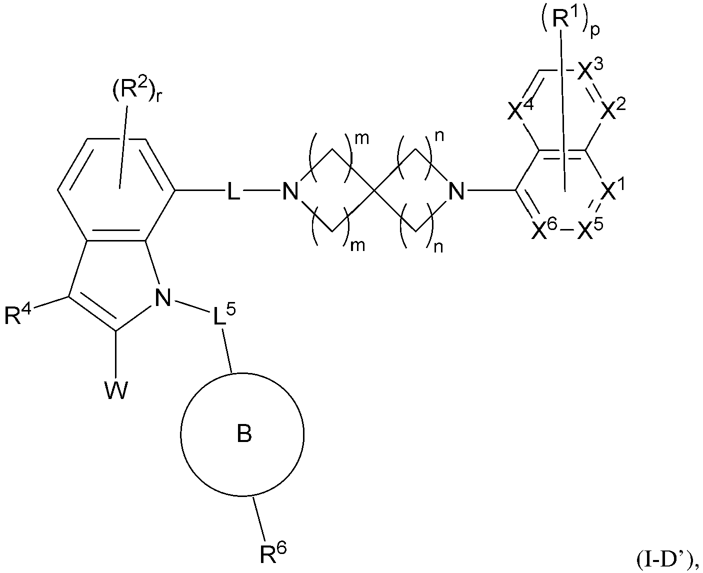 or a pharmaceutically acceptable salt, stereoisomer, solvate, or tautomer thereof.
5. A further embodiment of the present invention is the compound of any one of the preceding embodiments, wherein the compound is of Formula (I-I), (I-II), (I-III), (I-IV), (I-V), (I-VI) or (I-VII): 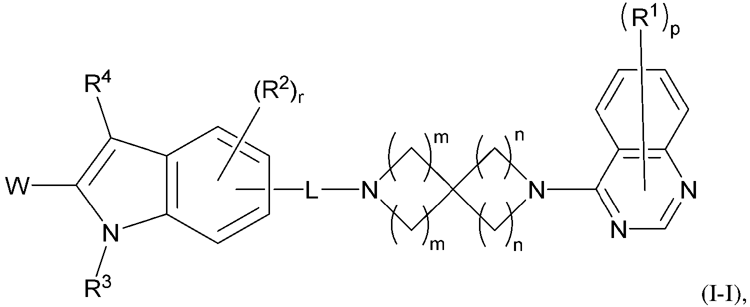 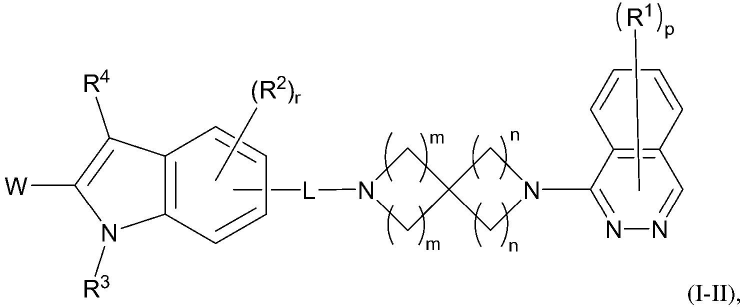 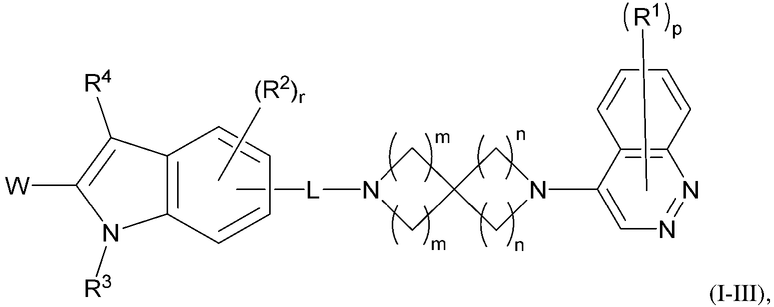 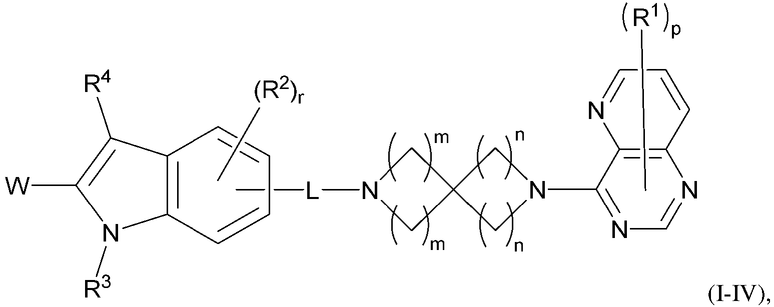 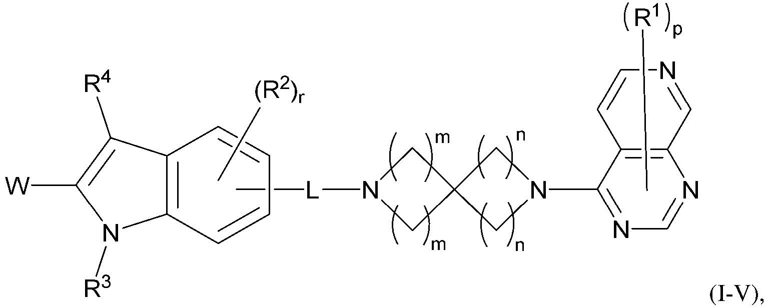 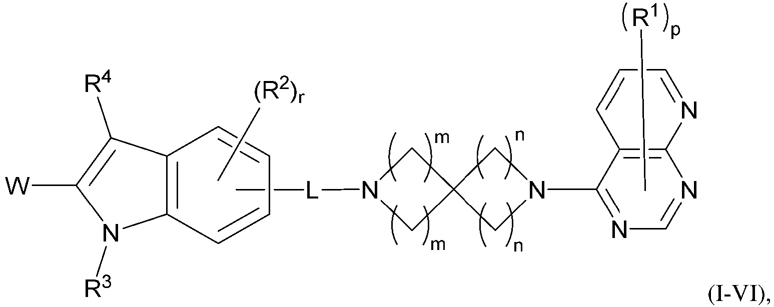 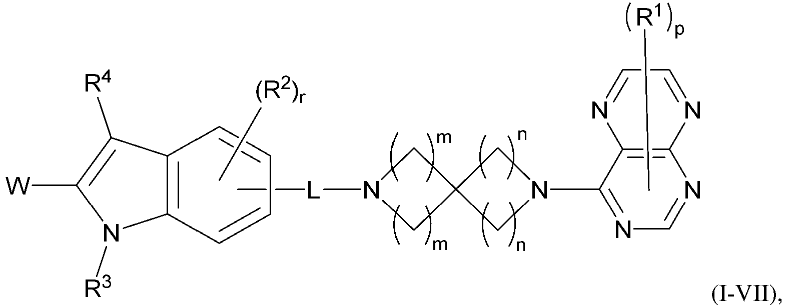 or a pharmaceutically acceptable salt, stereoisomer, solvate, or tautomer thereof.
6. A further embodiment of the present invention is the compound of any one of the preceding embodiments, wherein the compound is of Formula (I-I'), (I-II'), (I-III'), (I-IV'), (I-V'), (I-VI') or (I-VII'): 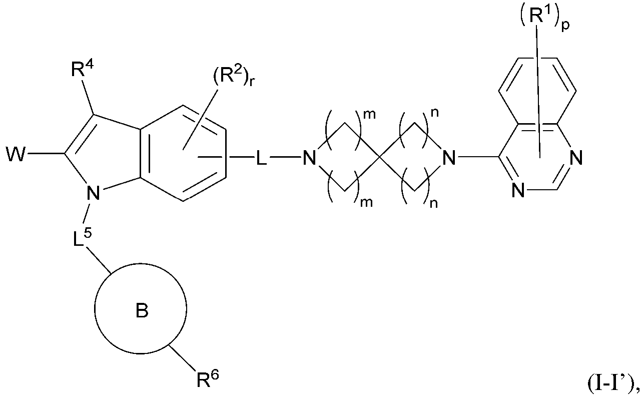 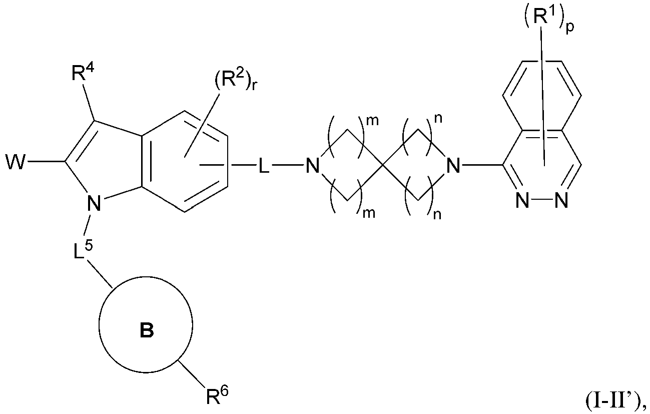 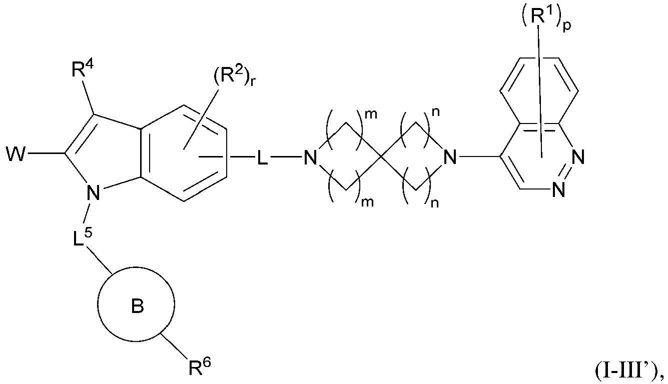 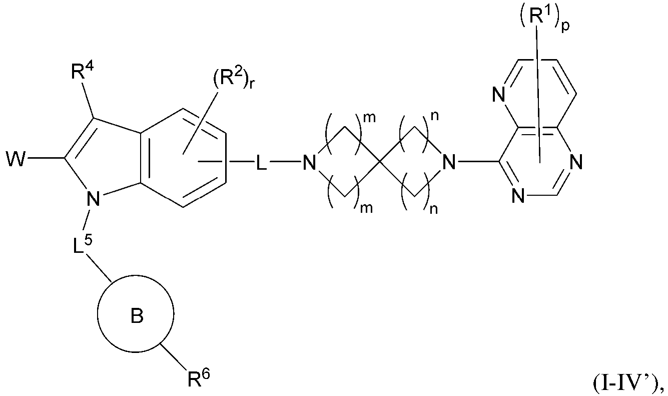 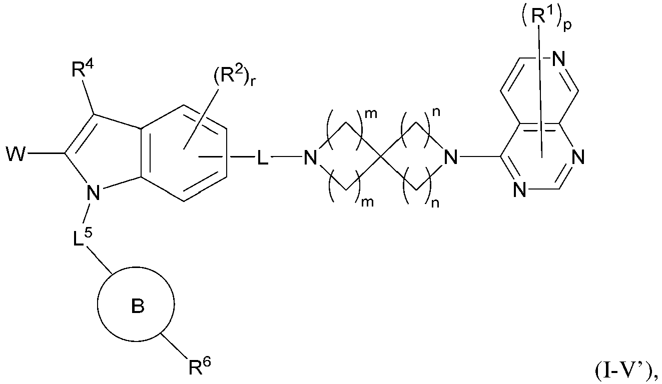 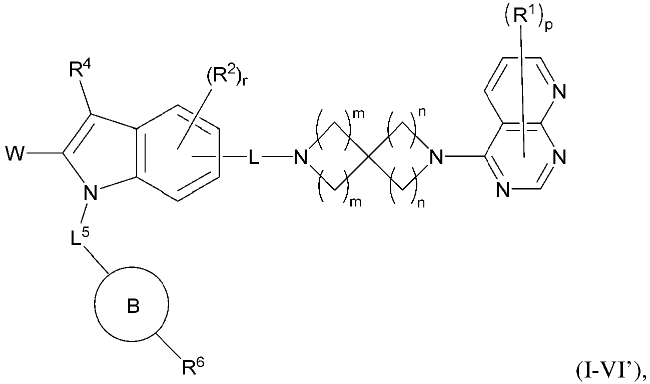 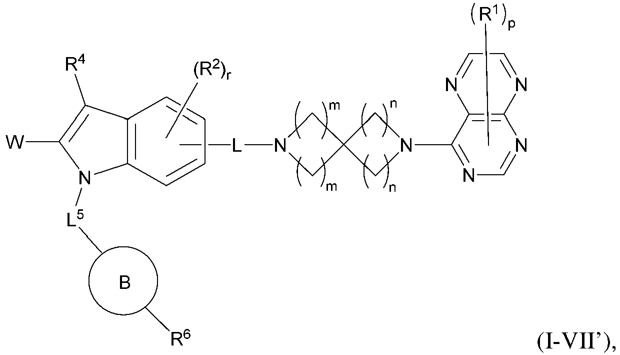 or a pharmaceutically acceptable salt, stereoisomer, solvate, or tautomer thereof.
7. A further embodiment of the present invention is the compound of embodiment 5, wherein R³ is selected from:

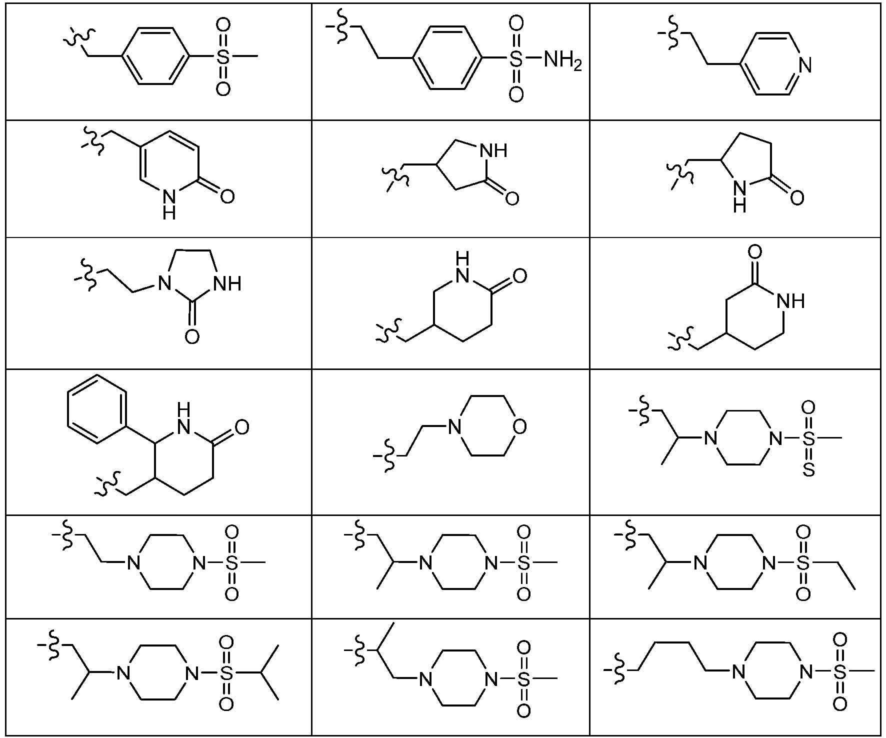

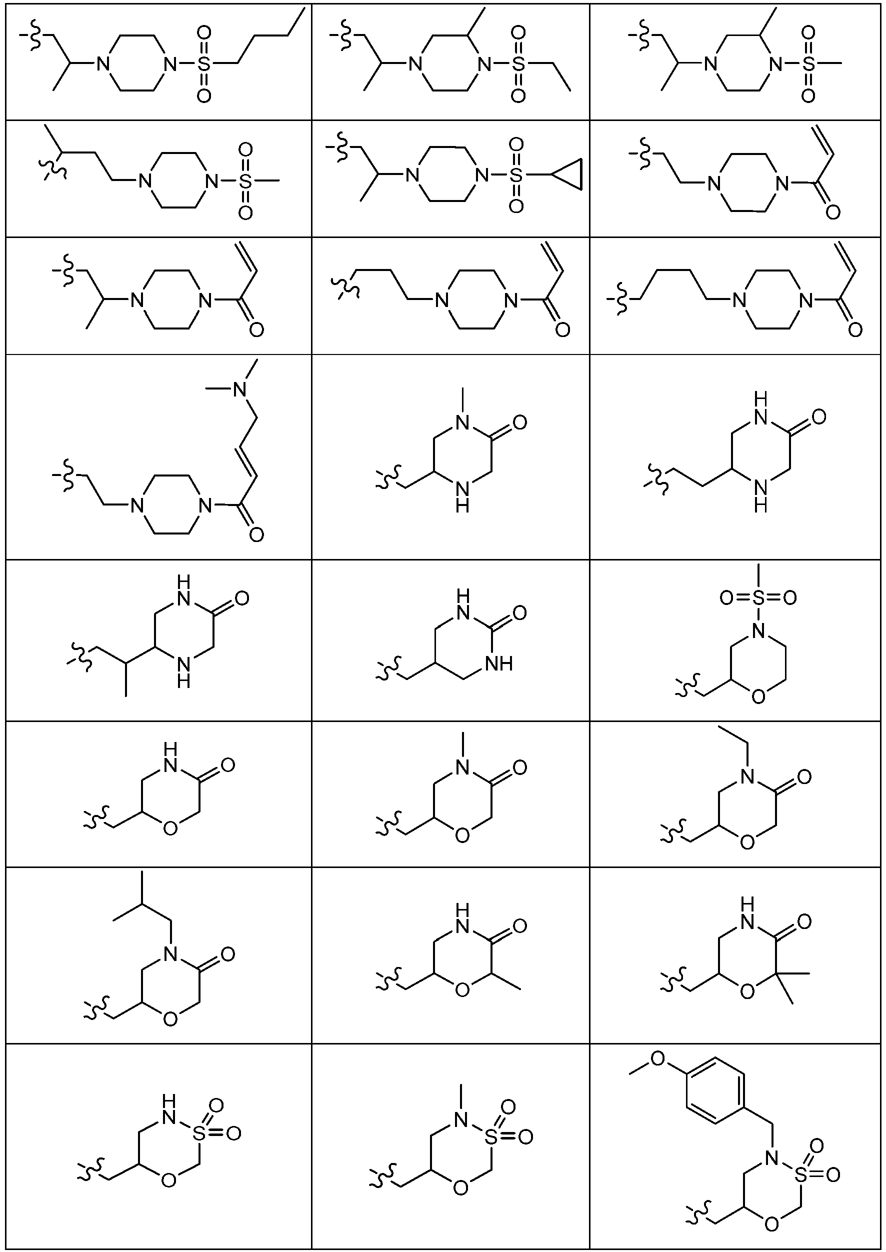

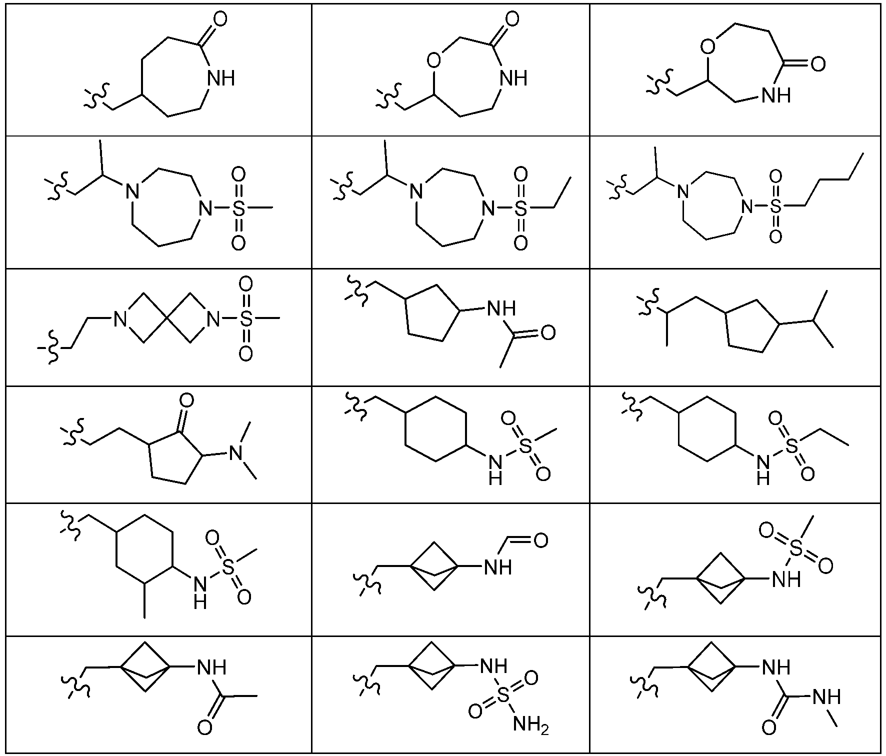

or a pharmaceutically acceptable salt, stereoisomer, solvate, or tautomer thereof.
8. A further embodiment of the present invention is the compound of embodiment 1, wherein the compound is of Formula (I-a), (I-b), (I-c), (I-d), (I-e), (I-f), (I-g), (I-h) or (I-i): 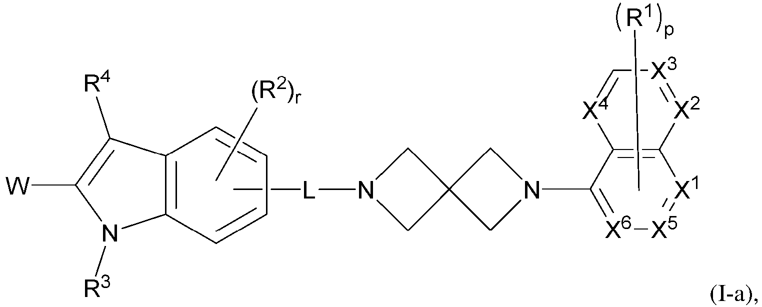 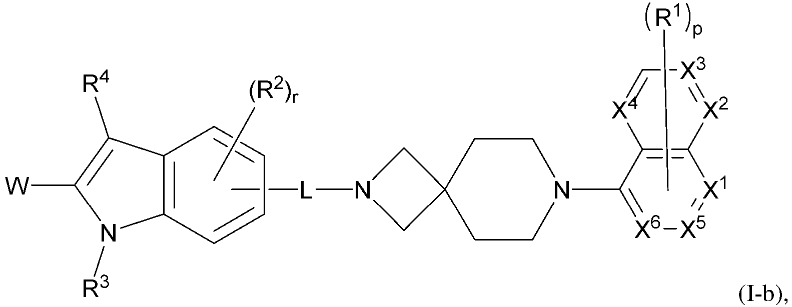 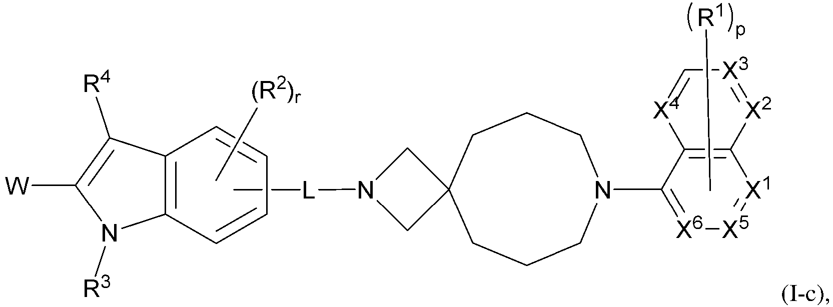 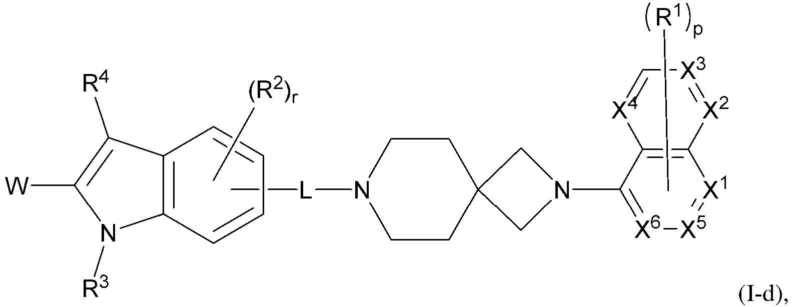 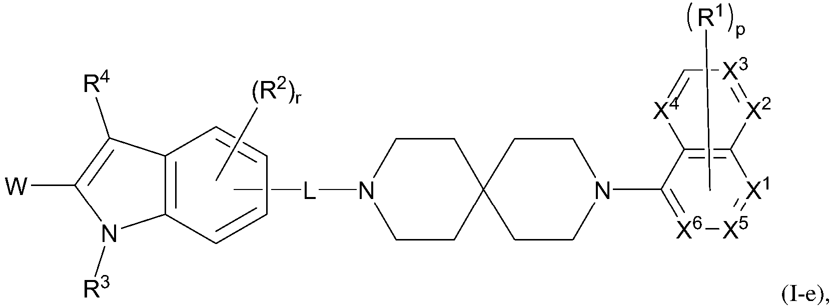 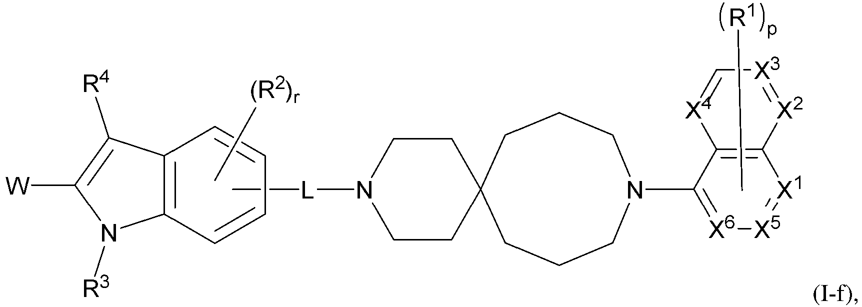 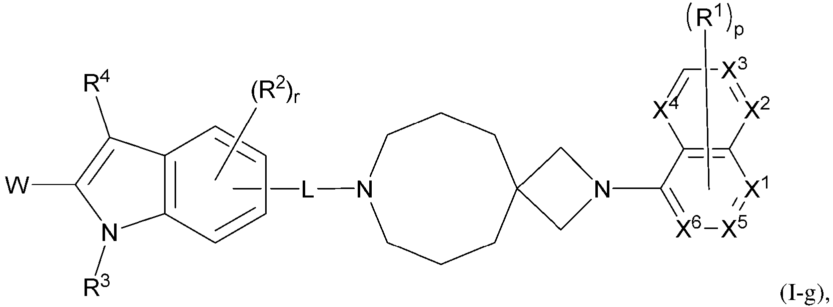 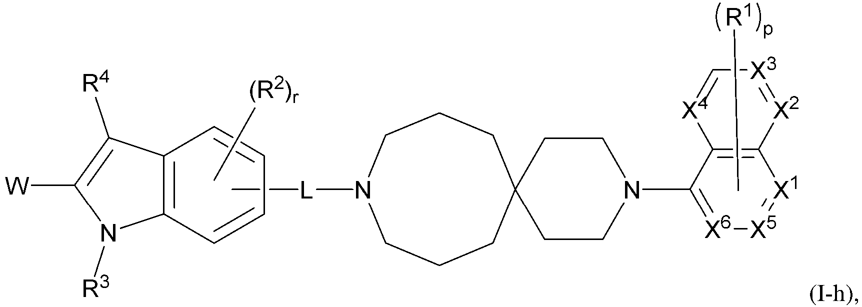 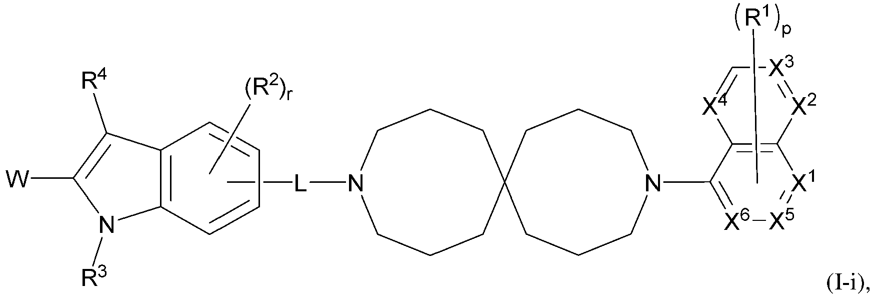 or a pharmaceutically acceptable salt, stereoisomer, solvate, or tautomer thereof.
9. A further embodiment of the present invention is a compound selected from:

| **#** | **Structure** | **IUPAC Name** |
|---|---|---|
| **1** | 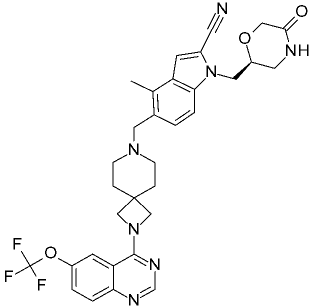 | 4-methyl-1-[[(2S)-5-oxomorpholin-2-yl]methyl]-5-[[2-[6-(trifluoromethoxy)quinazolin-4-yl]-2,7-diazaspiro[3.5]nonan-7-yl]methyl]indole-2-carbonitrile |
| **2** | 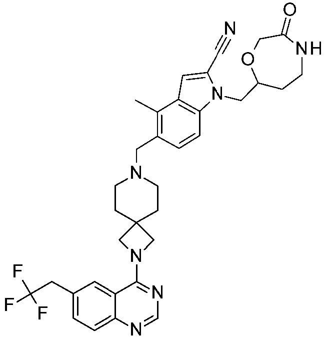 | 4-methyl-1-[(3-oxo-1,4-oxazepan-7-yl)methyl]-5-[[2-[6-(2,2,2-trifluoroethyl)quinazolin-4-yl]-2,7-diazaspiro[3.5]nonan-7-yl]methyl]indole-2-carbonitrile |
| **3** | 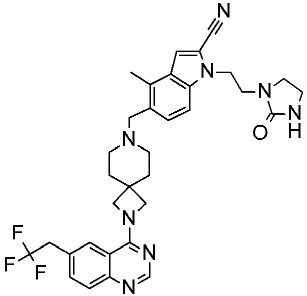 | 4-methyl-1-[2-(2-oxoimidazolidin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **4** | 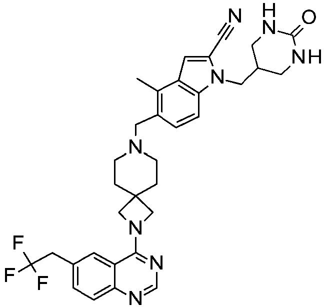 | 4-methyl-1-[(2-oxohexahydropyrimidin-5-yl)methyl]-5-[[2-[6-(2,2,2-trifluoroethyl)quinazolin-4-yl]-2,7-diazaspiro[3.5]nonan-7-yl]methyl]indole-2-carbonitrile |
| **5** | 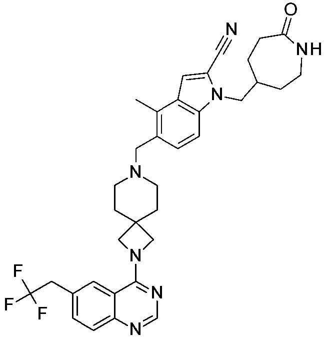 | 4-methyl-1-[(7-oxoazepan-4-yl)methyl]-5-[[2-[6-(2,2,2-trifluoroethyl)quinazolin-4-yl]-2,7-diazaspiro[3.5]nonan-7-yl]methyl]indole-2-carbonitrile |
| **6** | 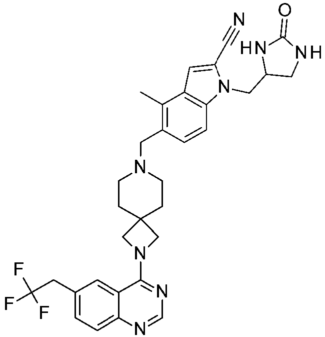 | 4-methyl-1-[(2-oxoimidazolidin-4-yl)methyl]-5-[[2-[6-(2,2,2-trifluoroethyl)quinazolin-4-yl]-2,7-diazaspiro[3.5]nonan-7-yl]methyl]indole-2-carbonitrile |
| **7** | 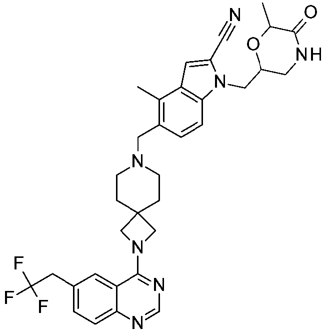 | 4-methyl-1-[(6-methyl-5-oxo-morpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **8** | 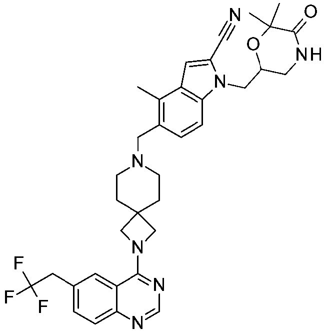 | 1-[(6,6-dimethyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **9** | 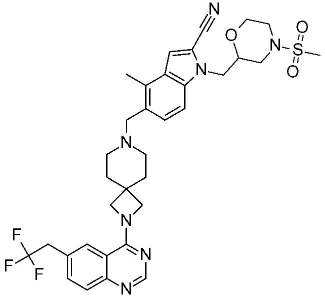 | 4-methyl-1-[(4-methylsulfonylmorpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **10** | 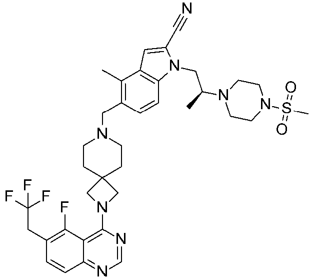 | 5-[[2-[5-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile |
| **11** | 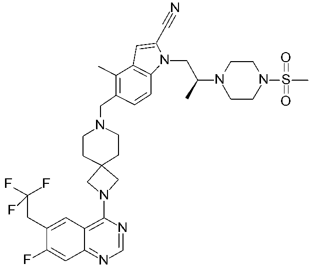 | 5-[[2-[7-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile |
| **12** | 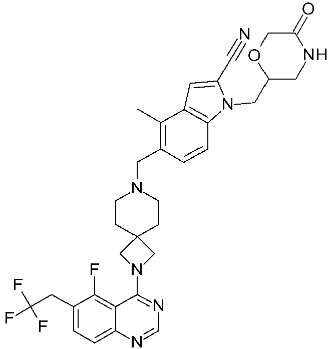 | 5-[[2-[5-fluoro-6-(2,2,2-trifluoroethyl)quinazolin-4-yl]-2,7-diazaspiro[3.5]nonan-7-yl]methyl]-4-methyl-1-[(5-oxomorpholin-2-yl)methyl]indole-2-carbonitrile |
| **13** | 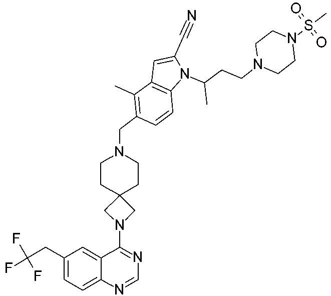 | 4-methyl-1-[1-methyl-3-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **14** | 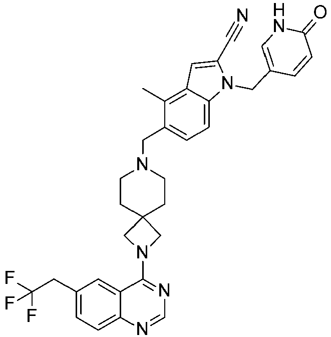 | 4-methyl-1-[(6-oxo-1H-pyridin-3-yl)methyl]-5-[[2-[6-(2,2,2-trifluoroethyl)quinazolin-4-yl]-2,7-diazaspiro[3.5]nonan-7-yl]methyl]indole-2-carbonitrile |
| **15** | 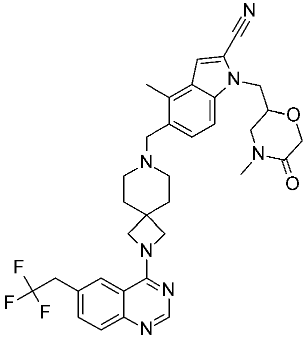 | 4-methyl-1-[(4-methyl-5-oxo-morpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **16** | 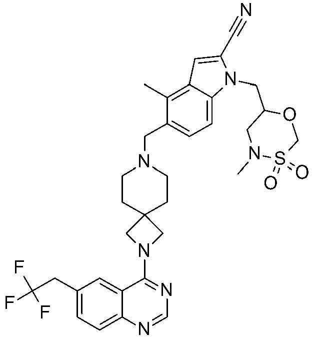 | 4-methyl-1-[(4-methyl-3,3-dioxo-1,3,4-oxathiazinan-6-yl)methyl]-5-[[2-[6-(2,2,2-trifluoroethyl)quinazolin-4-yl]-2,7-diazaspiro[3.5]nonan-7-yl]methyl]indole-2-carbonitrile |
| **17** | 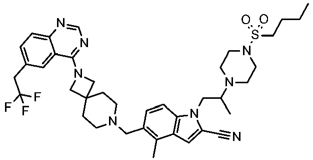 | 1-[2-(4-butylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **18** | 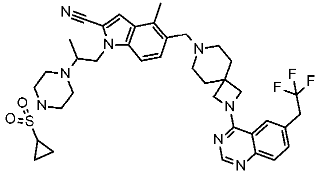 | 1-[2-(4-cyclopropylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **19** | 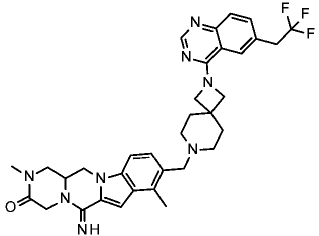 | 2-imino-6,15-dimethyl-14-[[2-[6-(2,2,2-trifluoroethyl)quinazolin-4-yl]-2,7-diazaspiro[3.5]nonan-7-yl]methyl]-3,6,10-triazatetracyclo[8.7.0.03,8.011,16]heptadeca-1(17),11(16),12,14-tetraen-5-one |
| **20** | 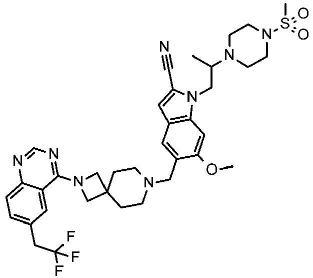 | 6-methoxy-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **21** | 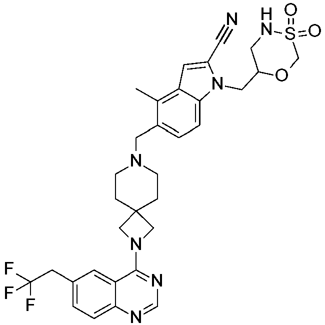 | 1-[(3,3-dioxo-1,3,4-oxathiazinan-6-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **22** | 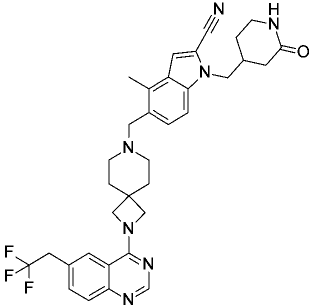 | 4-methyl-1-[(2-oxo-4-piperidyl)methyl]-5-[[2-[6-(2,2,2-trifluoroethyl)quinazolin-4-yl]-2,7-diazaspiro[3.5]nonan-7-yl]methyl]indole-2-carbonitrile |
| **23** | 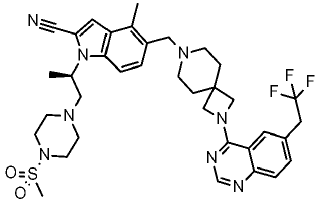 | 4-methyl-1-[(1R)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **24** | 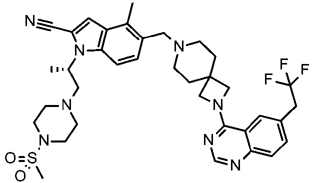 | 4-methyl-1-[(1S)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **25** | 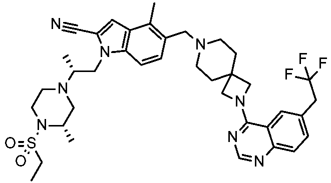 | 1-[(2R)-2-[(3S)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **26** | 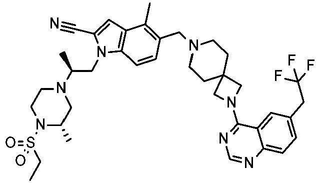 | 1-[(2S)-2-[(3S)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **27** | 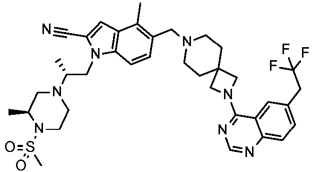 | 4-methyl-1-[(2R)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile |
| **28** | 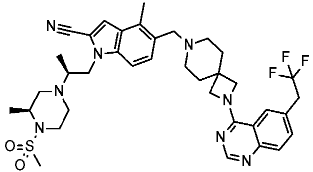 | 4-methyl-1-[(2S)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile |
| **29** | 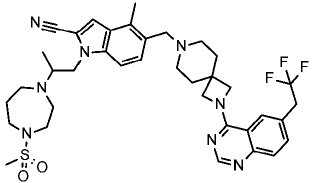 | 4-methyl-1-[2-(4-methylsulfonyl-1,4-diazepan-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **30** | 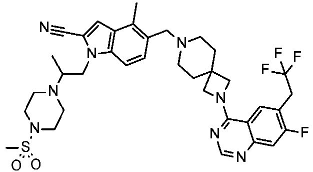 | 5-[[2-[7-fluoro-6-(2,2,2-trifluoroethyl)quinazolin-4-yl]-2,7-diazaspiro[3.5]nonan-7-yl]methyl]-4-methyl-1-[2-(4-methylsulfonylpiperazin-1-yl)propyl]indole-2-carbonitrile |
| **31** | 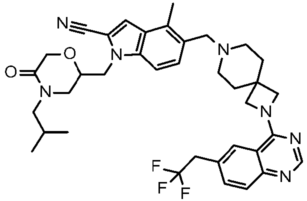 | 1-[(4-isobutyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **32** | 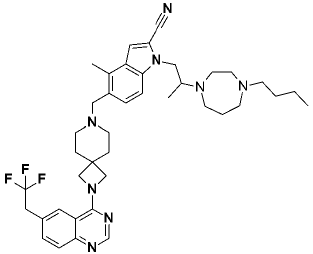 | 1-[2-(4-butyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **33** | 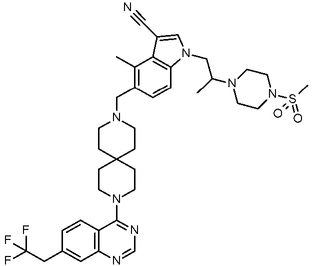 | 4-methyl-1-[2-(4-methylsulfonylpiperazin-1-yl)propyl]-5-[[3-[7-(2,2,2-trifluoroethyl)quinazolin-4-yl] -3,9-diazaspiro[5.5]undecan-9-yl]methyl]indole-3-carbonitrile |
| **34** | 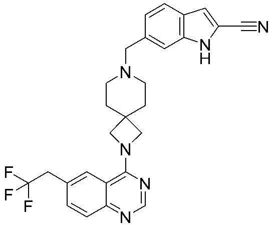 | 6-((2-(6-(2,2,2-trifluoroethyl)quinazolin-4-yl)-2,7-diazaspiro [3.5] nonan-7-yl)methyl)-1 H-indole-2-carbonitrile |
| **35** | 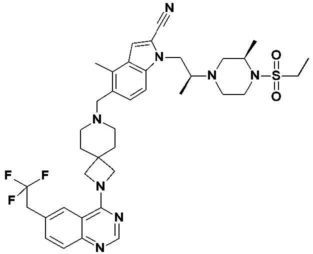 | 1-[(2S)-2-[(3R)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **36** | 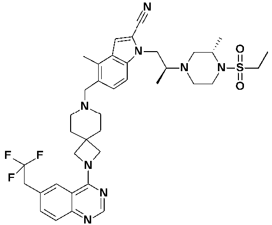 | 1-[(2S)-2-[(3S)-4-ethylsulfonyl-3-methyl-piperazin-1-yl]propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **37** | 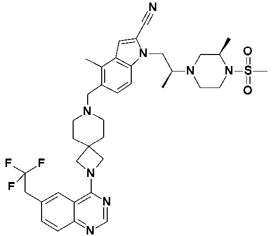 | 4-methyl-1-[(2R)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile |
| **38** | 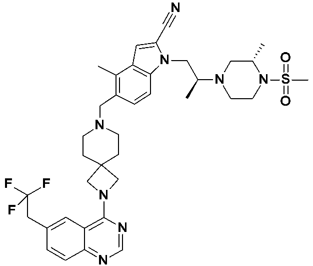 | 4-methyl-1-[(2S)-2-[(3S)-3-methyl-4-methylsulfonyl-piperazin-1-yl]propyl]-5-[[2-[6-(2,2,2-trifluoroethyl)quinazolin-4-yl]-2,7-diazaspiro[3.5]nonan-7-yl]methyl]indole-2-carbonitrile |
| **39** | 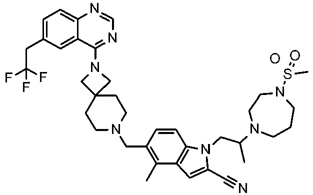 | 4-methyl-1-[2-(4-methylsulfonyl-1,4-diazepan-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **40** | 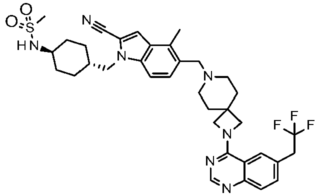 | N-(1R,4R)-[4-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]cyclohexyl]methanesulfonamide |
| **41** | 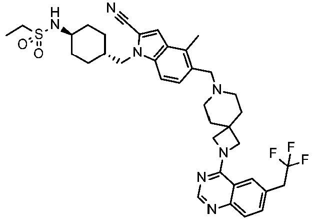 | N-(1R,4R)-[4-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]cyclohexyl]ethanesulfonamide |
| **42** | 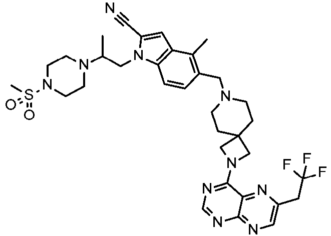 | 4-methyl-1-[2-(4-methylsulfonylpiperazin-1-yl)propyl]-5-[[2-[6-(2,2,2-trifluoroethyl)pteridin-4-yl]-2,7-diazaspiro[3.5]nonan-7-yl]methyl]indole-2-carbonitrile |
| **43** | 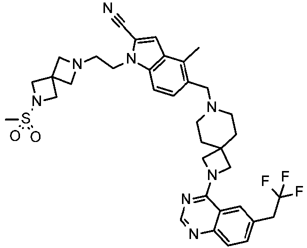 | 4-methyl-1-[2-(2-methylsulfonyl-2,6-diazaspiro[3.3]heptan-6-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **44** | 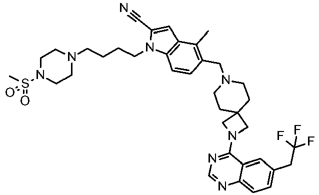 | 4-methyl-1-[4-(4-methylsulfonylpiperazin-1-yl)butyl]-5-[[2-[6-(2,2,2-trifluoroethyl)quinazolin-4-yl]-2,7-diazaspiro[3.5]nonan-7-yl]methyl]indole-2-carbonitrile |
| **45** | 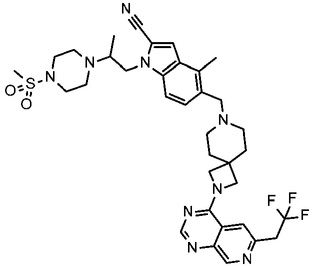 | 4-methyl-1-[2-(4-methylsulfonylpiperazin-1-yl)propyl]-5-[[2-[6-(2,2,2-trifluoroethyl)pyrido[3,4-d]pyrimidin-4-yl]-2,7-diazaspiro[3.5]nonan-7-yl]methyl]indole-2-carbonitrile |
| **46** | 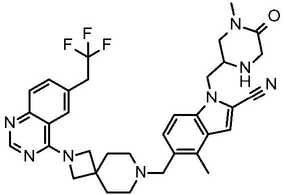 | 4-methyl-1-[(4-methyl-5-oxo-piperazin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **47** | 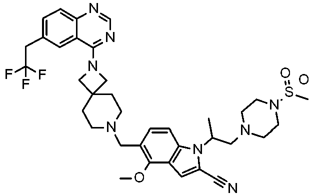 | 4-methoxy-1-[1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **48** | 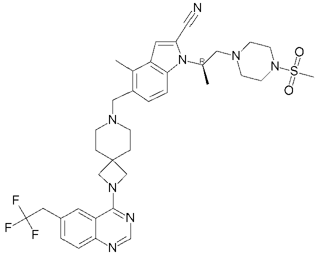 | 4-methyl-1-[(1R)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **49** | 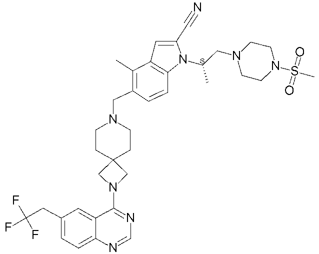 | 4-methyl-1-[(1S)-1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **50** | 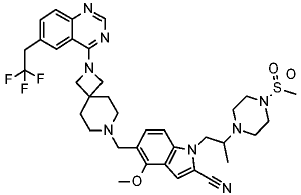 | 4-methoxy-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **51** | 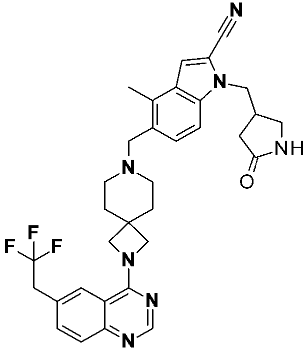 | 4-methyl-1-[(5-oxopyrrolidin-3-yl)methyl]-5-[[2-[6-(2,2,2-trifluoroethyl)quinazolin-4-yl]-2,7-diazaspiro[3.5]nonan-7-yl]methyl]indole-2-carbonitrile |
| **52** | 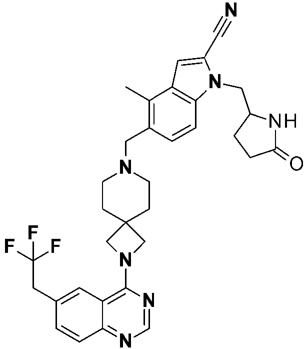 | 4-methyl-1-[(5-oxopyrrolidin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **53** | 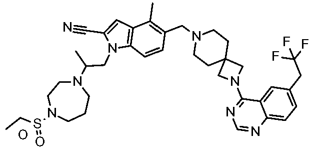 | 1-[2-(4-ethylsulfonyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **54** | 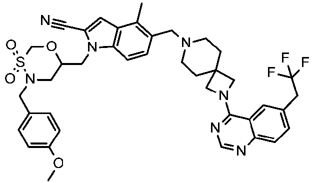 | 1-[[4-[(4-methoxyphenyl)methyl]-3,3-dioxo-1,3,4-oxathiazinan-6-yl]methyl] -4-methyl-5- [[2- [6-(2,2,2-trifluoroethyl)quinazolin-4-yl]-2,7-diazaspiro[3.5]nonan-7-yl]methyl]indole-2-carbonitrile |
| **55** | 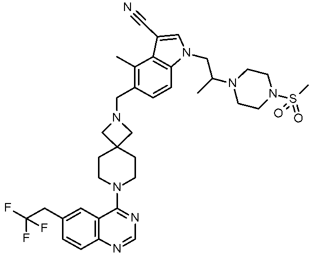 | 4-methyl-1-[2-(4-methylsulfonylpiperazin-1-yl)propyl]-5-[[7-[6-(2,2,2-trifluoroethyl)quinazolin-4-yl]-2,7-diazaspiro[3.5]nonan-2-yl]methyl]indole-3-carbonitrile |
| **56** | 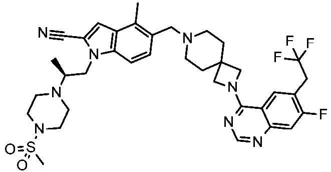 | 5-[[2-[7-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile |
| **57** | 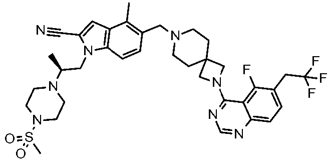 | 5-[[2-[5-fluoro-6-(2,2,2-trifluoroethyl)quinazolin-4-yl]-2,7-diazaspiro[3.5]nonan-7-yl]methyl]-4-methyl-1-[(2S)-2-(4-methylsulfonylpiperazin-1-yl)propyl]indole-2-carbonitrile |
| **58** | 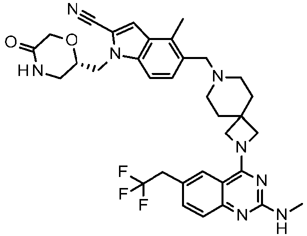 | 4-methyl-5-[[2-[2-(methylamino)-6-(2,2,2-trifluoroethyl)quinazolin-4-yl]-2,7-diazaspiro[3.5]nonan-7-yl]methyl]-1-[[(2S)-5-oxomorpholin-2-yl]methyl]indole-2-carbonitrile |
| **59** | 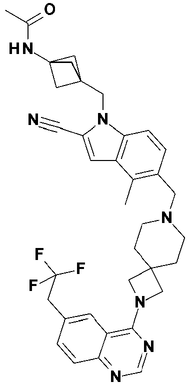 | *N*-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]acetamide |
| **60** | 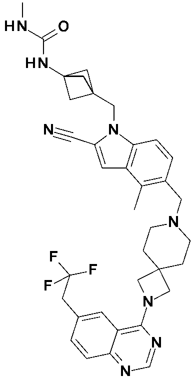 | 1-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]-3-methyl-urea |
| **61** | 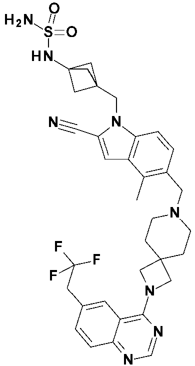 | 4-[7-[[2-cyano-4-methyl-1-[[3-(sulfamoylamino)-1-bicyclo[1.1.1]pentanyl]methyl]indol-5-yl]methyl]-2,7-diazaspiro[3.5]nonan-2-yl]-6-(2,2,2-trifluoroethyl)quinazoline |
| **62** | 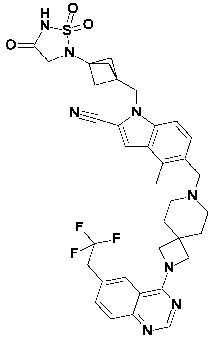 | 4-methyl-5-[[2-[6-(2,2,2-trifluoroethyl)quinazolin-4-yl]-2,7-diazaspiro[3.5]nonan-7-yl]methyl]-1-[[3-(1,1,4-trioxo-1,2,5-thiadiazolidin-2-yl)-1-bicyclo[1.1.1]pentanyl]methyl]indole-2-carbonitrile |
| **63** | 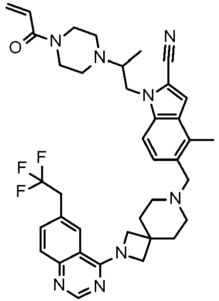 | 4-methyl-1-[2-(4-prop-2-enoylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **64** | 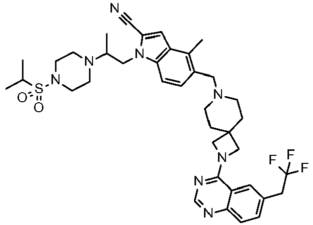 | 1-[2-(4-isopropylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **65** | 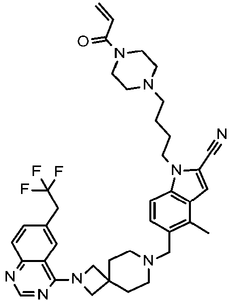 | 4-methyl-1-[4-(4-prop-2-enoylpiperazin-1-yl)butyl]-5-[[2-[6-(2,2,2-trifluoroethyl)quinazolin-4-yl]-2,7-diazaspiro[3.5]nonan-7-yl]methyl]indole-2-carbonitrile |
| **66** | 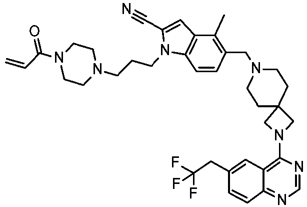 | 4-methyl-1-[3-(4-prop-2-enoylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **67** | 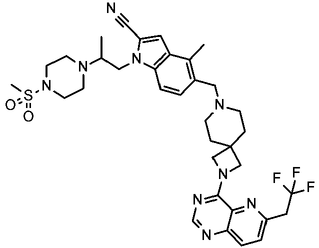 | 4-methyl-1-[2-(4-methylsulfonylpiperazin-1-yl)propyl]-5-[[2-[6-(2,2,2-trifluoroethyl)pyrido[3,2-d]pyrimidin-4-yl]-2,7-diazaspiro[3.5]nonan-7-yl]methyl]indole-2-carbonitrile |
| **68** | 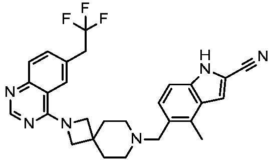 | 4-methyl-5-[[2-[6-(2,2,2-trifluoroethyl)quinazolin-4-yl]-2,7-diazaspiro[3.5]nonan-7-yl]methyl]-1H-indole-2-carbonitrile |
| **69** | 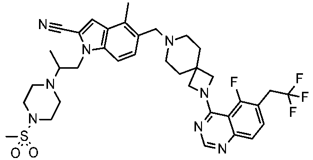 | 5-[[2-[5-fluoro-6-(2,2,2-trifluoroethyl)quinazolin-4-yl]-2,7-diazaspiro[3.5]nonan-7-yl]methyl]-4-methyl-1-[2-(4-methylsulfonylpiperazin-1-yl)propyl]indole-2-carbonitrile |
| **70** | 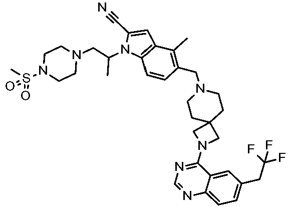 | 4-methyl-1-[1-methyl-2-(4-methylsulfonylpiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **71** | 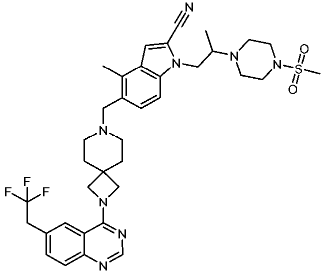 | 4-methyl-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **72** | 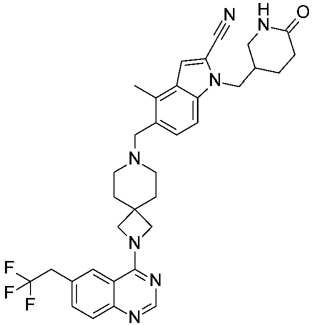 | 4-methyl-1-[(6-oxo-3-piperidyl)methyl]-5-[[2-[6-(2,2,2-trifluoroethyl)quinazolin-4-yl]-2,7-diazaspiro[3.5]nonan-7-yl]methyl]indole-2-carbonitrile |
| **73** | 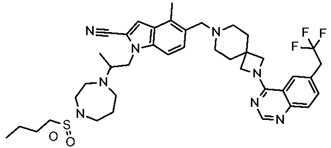 | 1-[2-(4-butylsulfonyl-1,4-diazepan-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **74** | 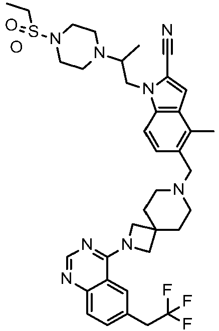 | 1-[2-(4-ethylsulfonylpiperazin-1-yl)propyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **75** | 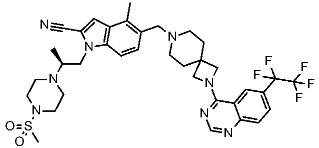 | 4-methyl-1-[(2S)-2-(4-methylsulfonylpiperazin-1-yl)propyl]-5-[[2-[6-(1,1,2,2,2-pentafluoroethyl)quinazolin-4-yl]-2,7-diazaspiro[3.5]nonan-7-yl]methyl]indole-2-carbonitrile |
| **76** | 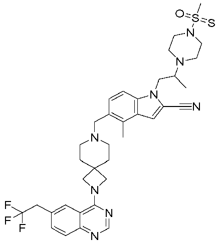 | 4-methyl-1-(2-(4-(methylsulfonothioyl)piperazin-1-yl)propyl)-5-((2-(6-(2,2,2-trifluoroethyl)quinazolin-4-yl)-2,7-diazaspiro[3.5]nonan-7-yl)methyl)-1H-indole-2-carbonitrile |
| **77** | 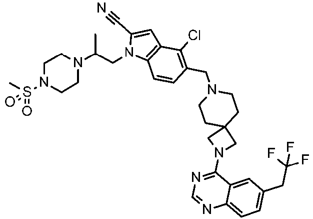 | 4-chloro-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **78** | 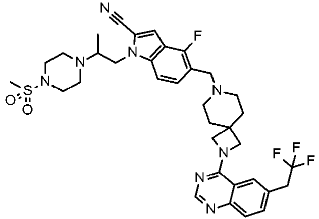 | 4-fluoro-1-[2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **79** | 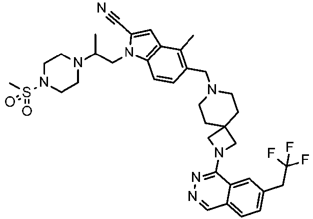 | 4-methyl-1-[2-(4-methylsulfonylpiperazin-1-yl)propyl]-5-[[2-[7-(2,2,2-trifluoroethyl)phthalazin-1-yl]-2,7-diazaspiro[3.5]nonan-7-yl]methyl]indole-2-carbonitrile |
| **80** | 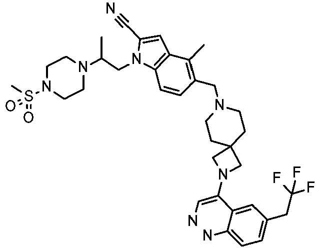 | 4-methyl-1-[2-(4-methylsulfonylpiperazin-1-yl)propyl]-5-[[2-[6-(2,2,2-trifluoroethyl)cinnolin-4-yl]-2,7-diazaspiro[3.5]nonan-7-yl]methyl]indole-2-carbonitrile |
| **81** | 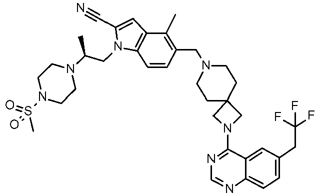 | 4-methyl-1-[(2S)-2-(4-methylsulfonylpiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **82** | 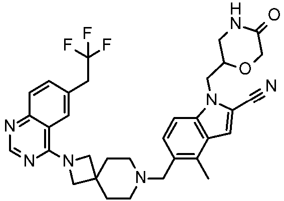 | 4-methyl-1-[(5-oxomorpholin-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **83** | 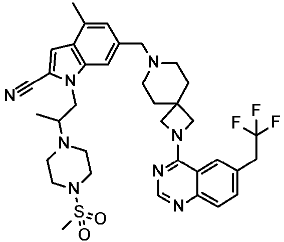 | 4-methyl-1-[2-(4-methylsulfonylpiperazin-1-yl)propyl]-6-[[2-[6-(2,2,2-trifluoroethyl)quinazolin-4-yl]-2,7-diazaspiro[3.5]nonan-7-yl]methyl]indole-2-carbonitrile |
| **84** | 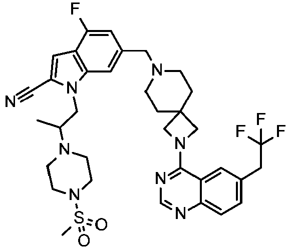 | 4-fluoro-1-[2-(4-methylsulfonylpiperazin-1-yl)propyl]-6-[[2-[6-(2,2,2-trifluoroethyl)quinazolin-4-yl]-2,7-diazaspiro[3.5]nonan-7-yl]methyl]indole-2-carbonitrile |
| **85** | 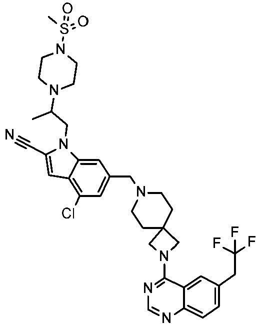 | 4-chloro-1-[2-(4-methylsulfonylpiperazin-1-yl)propyl]-6-[[2-[6-(2,2,2-trifluoroethyl)quinazolin-4-yl]-2,7-diazaspiro[3.5]nonan-7-yl]methyl]indole-2-carbonitrile |
| **86** | 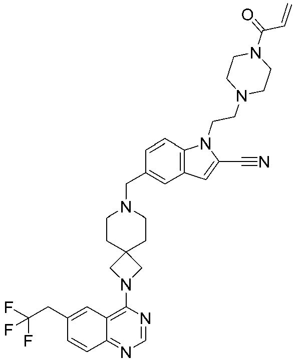 | 1-(2-(4-acryloylpiperazin-1-yl)ethyl)-5-((2-(6-(2,2,2-trifluoroethyl)quinazolin-4-yl)-2,7-diazaspiro[3.5]nonan-7-yl)methyl)-1H-indole-2-carbonitrile |
| **87** | 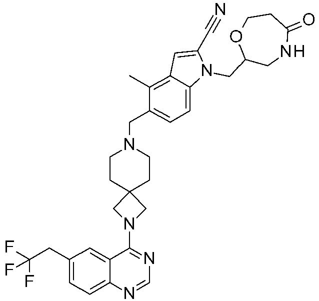 | 4-methyl-1-[(5-oxo-1,4-oxazepan-2-yl)methyl]-5-[[2-[6-(2,2,2-trifluoroethyl)quinazolin-4-yl]-2,7-diazaspiro[3.5]nonan-7-yl]methyl]indole-2-carbonitrile |
| **88** | 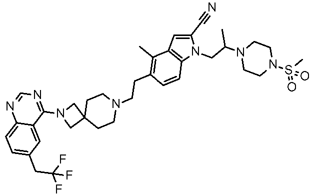 | 4-methyl-1-[2-(4-methylsulfonylpiperazin-1-yl)propyl]-5-[2-[2-[6-(2,2,2-trifluoroethyl)quinazolin-4-yl]-2,7-diazaspiro[3.5]nonan-7-yl]ethyl]indole-2-carbonitrile |
| **89** | 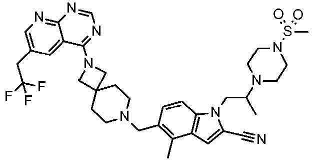 | 4-methyl-1-[2-(4-methylsulfonylpiperazin-1-yl)propyl]-5-[[2-[6-(2,2,2-trifluoroethyl)pyrido[2,3-d]pyrimidin-4-yl]-2,7-diazaspiro[3.5]nonan-7-yl]methyl]indole-2-carbonitrile |
| **90** | 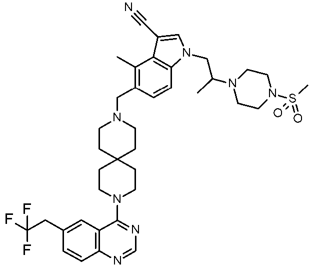 | 4-methyl-1-[2-(4-methylsulfonylpiperazin-1-yl)propyl]-5-[[3-[6-(2,2,2-trifluoroethyl)quinazolin-4-yl]-3,9-diazaspiro[5.5]undecan-9-yl]methyl]indole-3-carbonitrile |
| **91** | 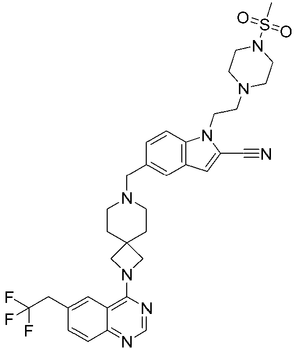 | 1-(2-(4-(methylsulfonyl)piperazin-1-yl)ethyl)-5-((2-(6-(2,2,2-trifluoroethyl)quinazolin-4-yl)-2,7-diazaspiro[3.5]nonan-7-yl)methyl)-1H-indole-2-carbonitrile |
| **92** | 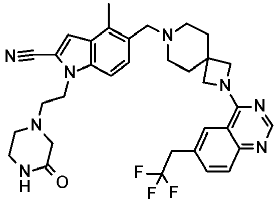 | 4-methyl-1-[2-(3-oxopiperazin-1-yl)ethyl]-5-[[2-[6-(2,2,2-trifluoroethyl)quinazolin-4-yl]-2,7-diazaspiro[3.5]nonan-7-yl]methyl]indole-2-carbonitrile |
| **93** | 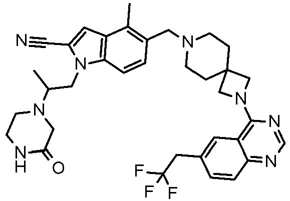 | 4-methyl-1-[2-(3-oxopiperazin-1-yl)propyl]-5-[[2-[6-(2,2,2-trifluoroethyl)quinazolin-4-yl]-2,7-diazaspiro[3.5]nonan-7-yl]methyl]indole-2-carbonitrile |
| **94** | 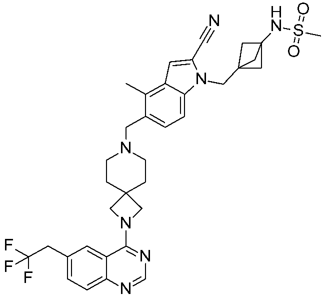 | N-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]methanesulfonamide |
| **95** | 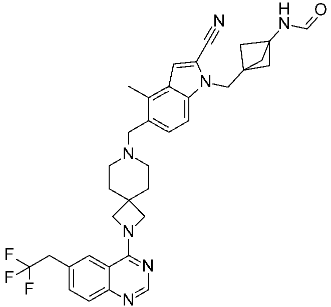 | N-[3-[[2-cyano-4-methyl-5-[[2-[6-(2,2,2-trifluoroethyl)quinazolin-4-yl]-2,7-diazaspiro[3.5]nonan-7-yl]methyl]indol-1-yl]methyl]-1-bicyclo[1.1.1]pentanyl]formamide |
| **96** | 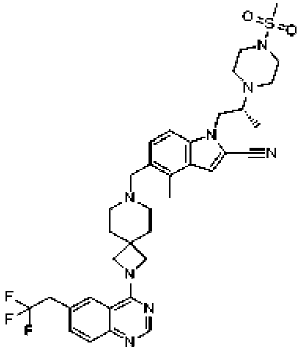 | rac-(R)-4-methyl-1-(2-(4-(methylsulfonyl)piperazin-1-yl)propyl)-5-((2-(6-(2,2,2-trifluoroethyl)quinazolin-4-yl)-2,7-diazaspiro[3.5]nonan-7-yl)methyl)-1H-indole-2-carbonitrile |
| **97** | 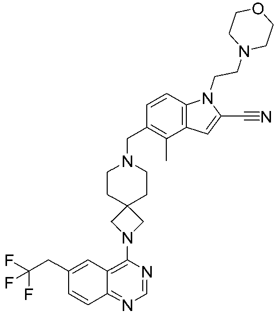 | 4-methyl-1-(2-morpholinoethyl)-5-((2-(6-(2,2,2-trifluoroethyl)quinazolin-4-yl)-2,7-diazaspiro[3.5]nonan-7-yl)methyl)-1H-indole-2-carbonitrile |
| **98** | 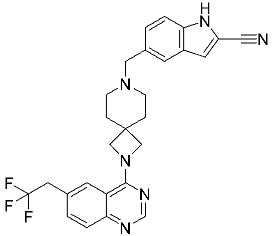 | 5-((2-(6-(2,2,2-trifluoroethyl)quinazolin-4-yl)-2,7-diazaspiro[3.5]nonan-7-yl)methyl)-1H-indole-2-carbonitrile |
| **99** | 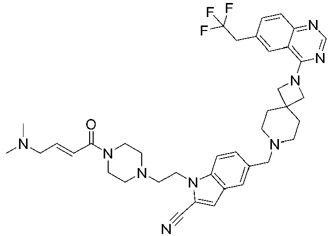 | (E)-1-(2-(4-(4-(dimethylamino)but-2-enoyl)piperazin-1-yl)ethyl)-5-((2-(6-(2,2,2-trifluoroethyl)quinazolin-4-yl)-2,7-diazaspiro[3.5]nonan-7-yl)methyl)-1H-indole-2-carbonitrile |
| **100** | 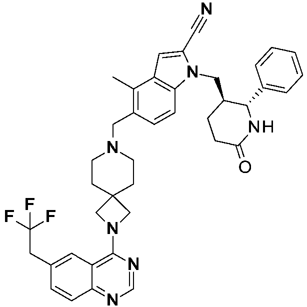 | 4-methyl-1-[[(2R,3S)-6-oxo-2-phenyl-3-piperidyl]methyl]-5-[[2-[6-(2,2,2-trifluoroethyl)quinazolin-4-yl]-2,7-diazaspiro[3.5]nonan-7-yl]methyl]indole-2-carbonitrile |
| **101** | 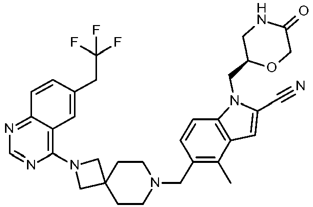 | 4-methyl-1-[[(2R)-5-oxomorpholin-2-yl]methyl]-5-[[2-[6-(2,2,2-trifluoroethyl)quinazolin-4-yl]-2,7-diazaspiro[3.5]nonan-7-yl]methyl]indole-2-carbonitrile |
| **102** | 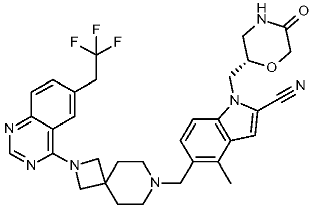 | 4-methyl-1-[[(2S)-5-oxomorpholin-2-yl]methyl]-5-[[2-[6-(2,2,2-trifluoroethyl)quinazolin-4-yl]-2,7-diazaspiro[3.5]nonan-7-yl]methyl]indole-2-carbonitrile |
| **103** | 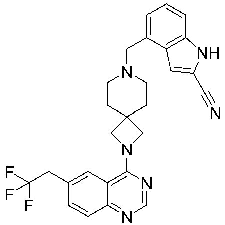 | 4-((2-(6-(2,2,2-trifluoroethyl)quinazolin-4-yl)-2,7-diazaspiro[3.5]nonan-7-yl)methyl)-1H-indole-2-carbonitrile |
| **104** | 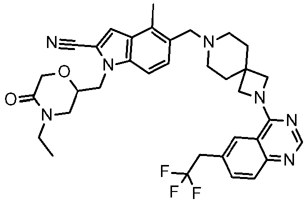 | 1-[(4-ethyl-5-oxo-morpholin-2-yl)methyl]-4-methyl-5-[[2-[6-(2,2,2-trifluoroethyl)quinazolin-4-yl]-2,7-diazaspiro[3.5]nonan-7-yl]methyl]indole-2-carbonitrile |
| **105** | 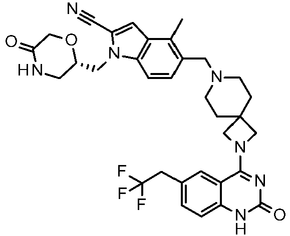 | 4-methyl-1-[[(2S)-5-oxomorpholin-2-yl]methyl]-5-[[2-[2-oxo-6-(2,2,2-trifluoroethyl)-1H-quinazolin-4-yl]-2,7-diazaspiro[3.5]nonan-7-yl]methyl]indole-2-carbonitrile |

or a pharmaceutically acceptable salt, stereoisomer, solvate, or tautomer thereof.
10. A further embodiment of the present invention is a pharmaceutical composition comprising the compound of any one of embodiments 1-9 or a pharmaceutically acceptable salt, stereoisomer, solvate, or tautomer thereof, and a pharmaceutically acceptable carrier.
11. A further embodiment of the present invention is the pharmaceutical composition of embodiments 10, further comprising one or more additional pharmaceutically active agents.
12. A further embodiment of the present invention is a method of inhibiting the interaction of menin and MLL in a cell, comprising contacting the cell with a compound of any one of embodiments 1-9 or a pharmaceutical composition of embodiment 10 or 11.
13. A further embodiment of the present invention is a method of inhibiting the interaction of menin and MLL1 in a cell, comprising contacting the cell with a compound of any one of embodiments 1-9 or a pharmaceutical composition of embodiment 10 or 11.
14. A further embodiment of the present invention is the method of embodiment 12 or 13, wherein the contacting is *in vitro* or *in vivo*.
15. A further embodiment of the present invention is a method for the treatment or prevention of a disease or disorder associated with the interaction of menin and MLL comprising administering to a subject in need thereof a compound of any one of embodiments 1-9 or a pharmaceutical composition of embodiment 10 or 11.
16. A further embodiment of the present invention is the method of embodiment 15, wherein the disease or disorder is selected from the group consisting of a leukemia, hematologic malignancy, solid tumor cancer, prostate cancer, breast cancer, liver cancer, brain tumor, and diabetes.
17. A further embodiment of the present invention is the method of embodiment 16, wherein the leukemia is selected from the group consisting of AML, ALL, Mixed Lineage Leukemia, and a leukemia with Partial Tandem Duplications of MLL.
18. A further embodiment of the present invention is the method of any one of embodiments 12-17, wherein the subject is a mammal.
19. A further embodiment of the present invention is the method of embodiment 18, wherein the subject is a human.
20. A further embodiment of the present invention is a compound selected from:

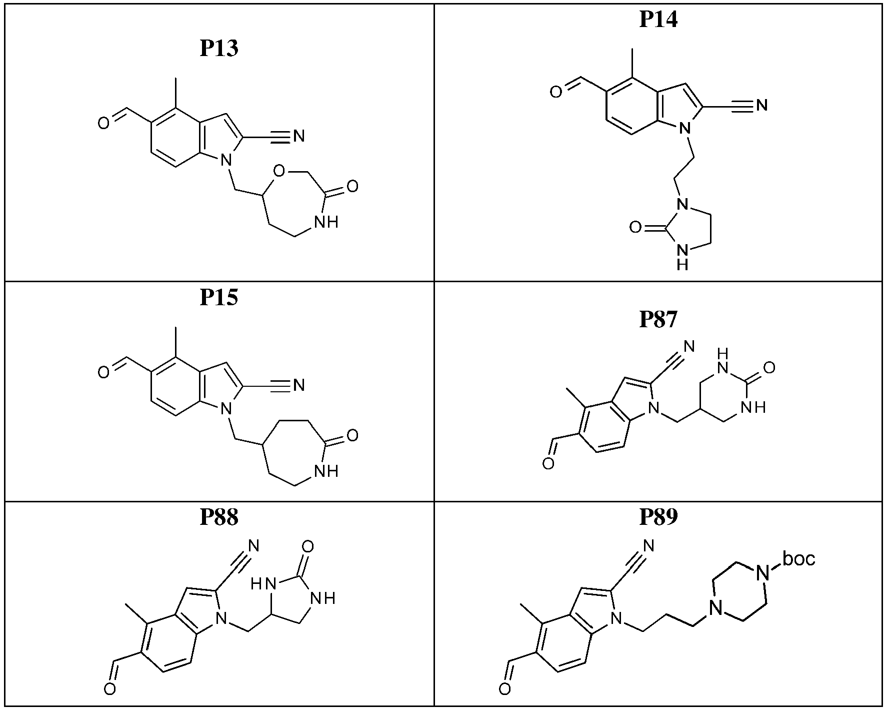

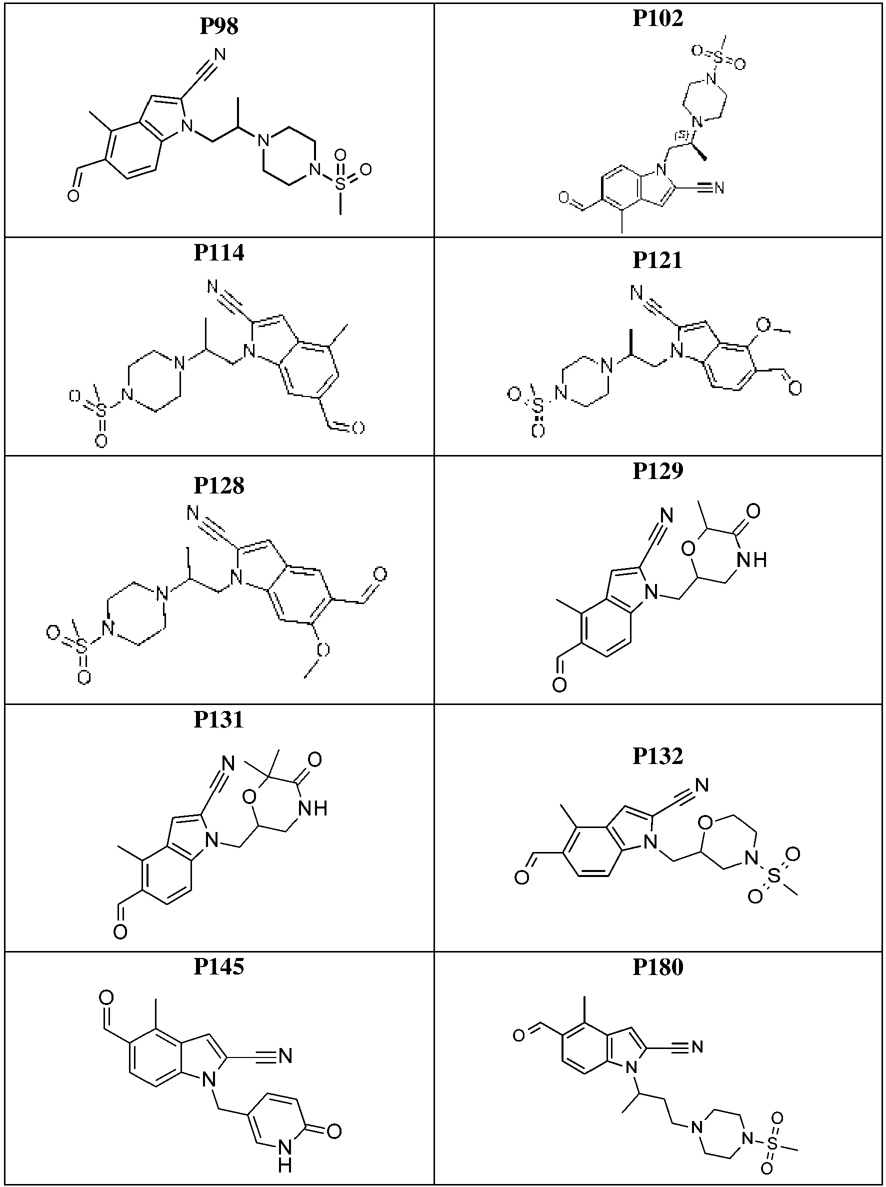

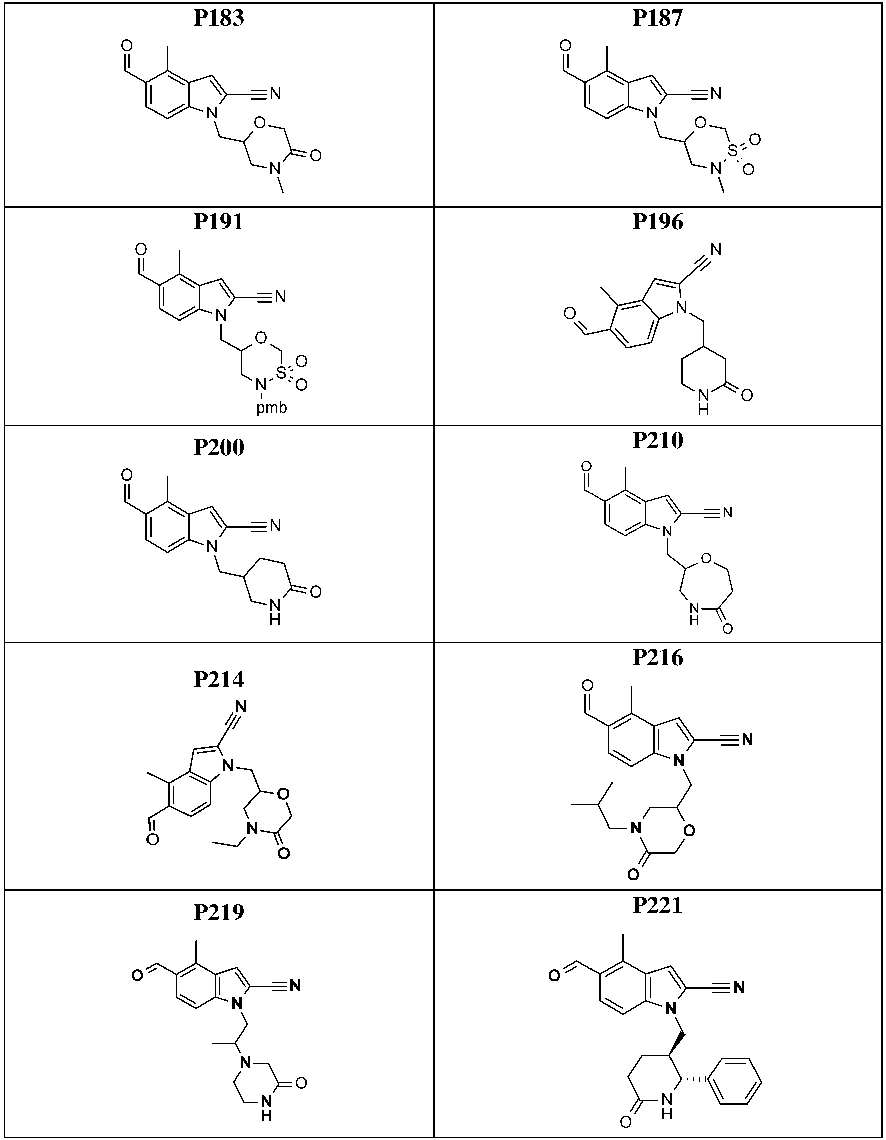

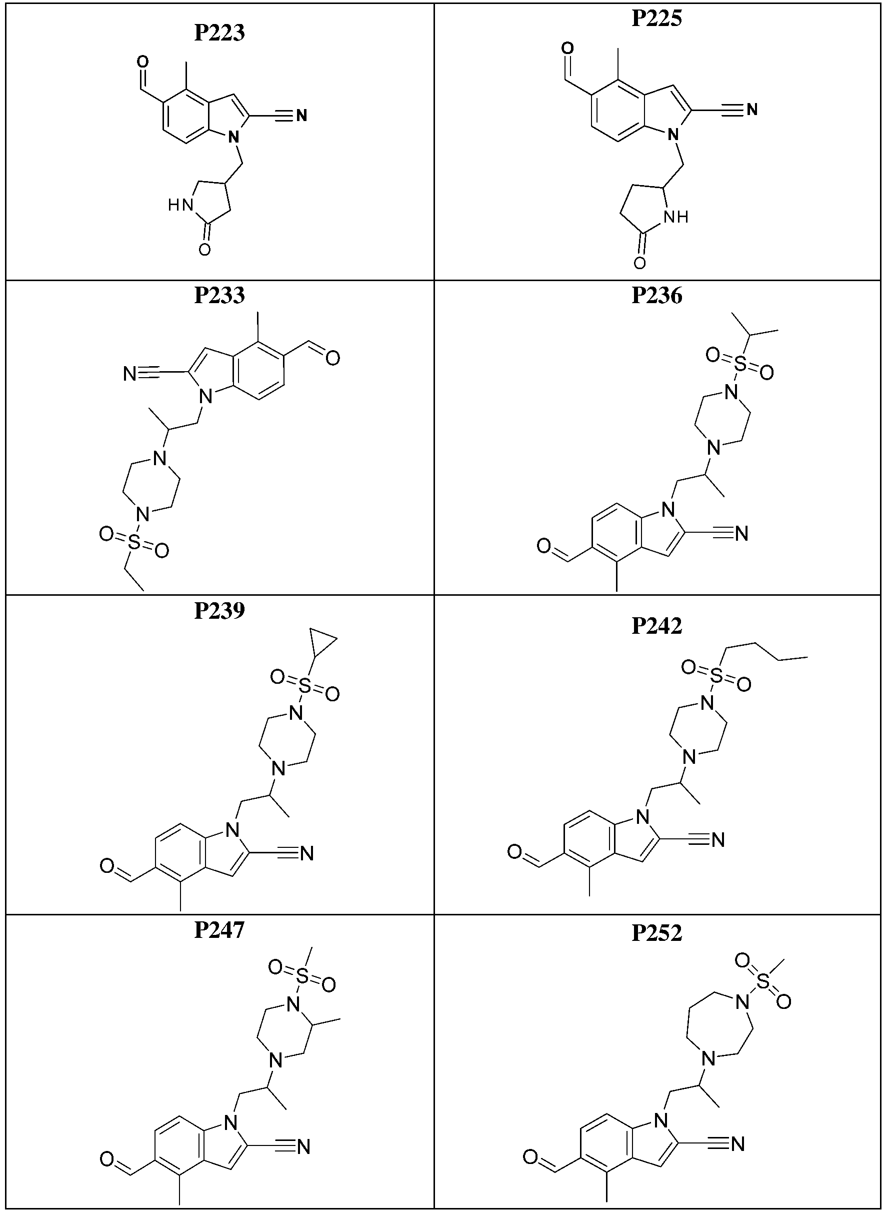

21. A further embodiment of the present invention is a compound selected from:

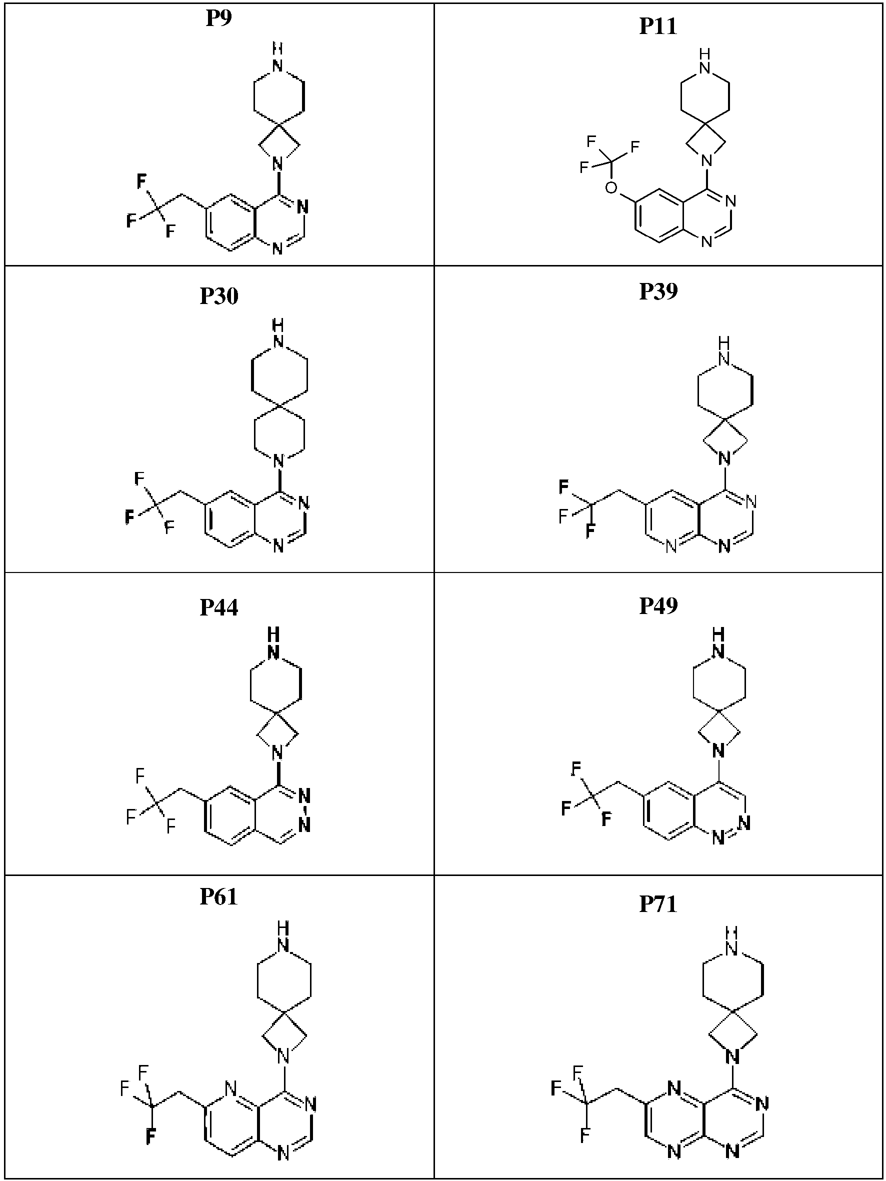

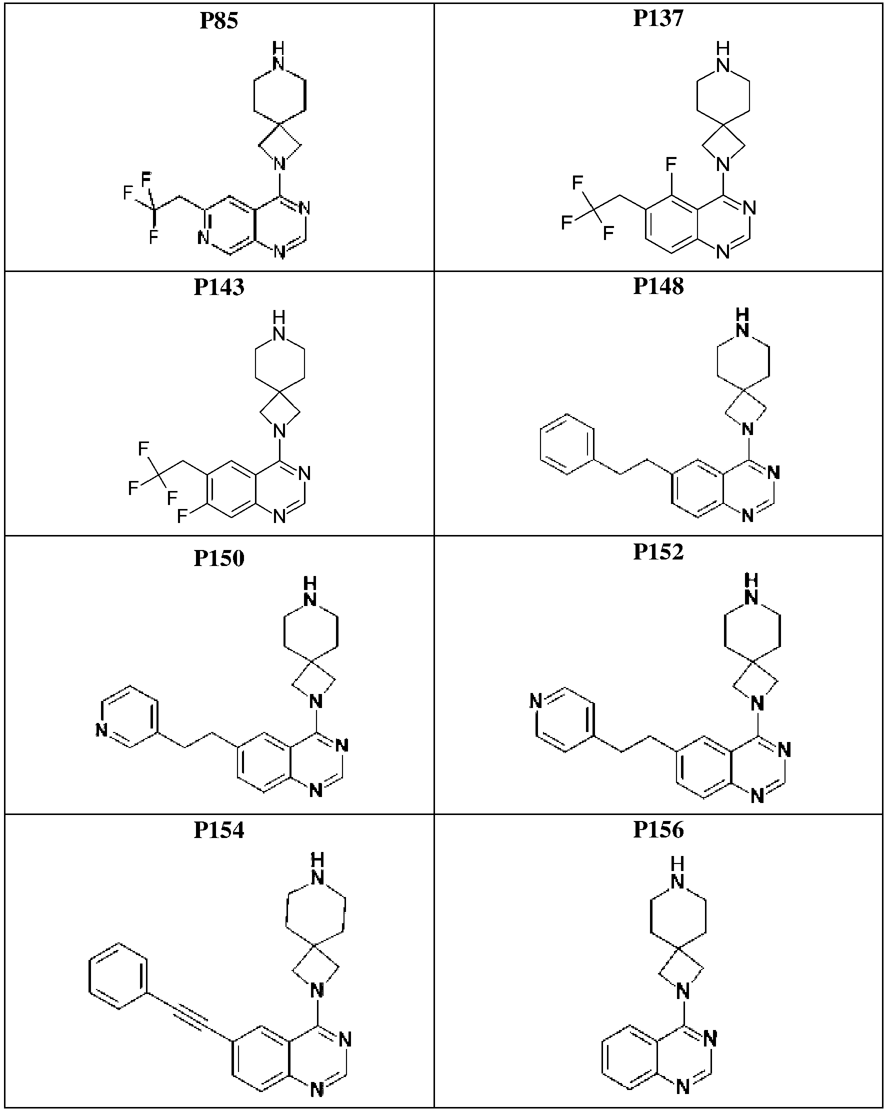

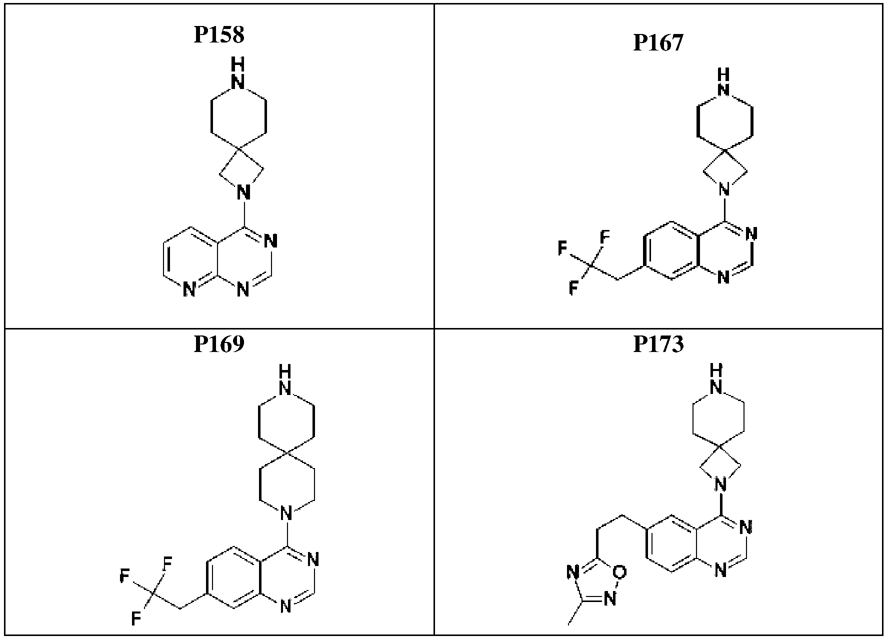

## Claims

1. A compound of formula: 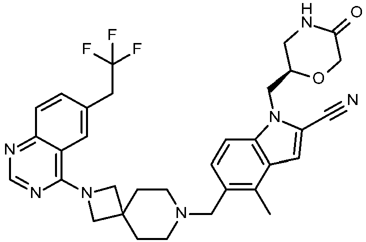 or 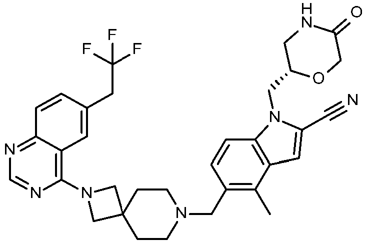 or a pharmaceutically acceptable salt, stereoisomer, solvate, or tautomer thereof.

2. A pharmaceutical composition comprising the compound of claim 1 or a pharmaceutically acceptable salt, stereoisomer, solvate, or tautomer thereof, and a pharmaceutically acceptable carrier,
preferably further comprising one or more additional pharmaceutically active agents.

3. A compound of claim 1 or a pharmaceutical composition of claim 2 for use in a method of inhibiting the interaction of menin and MLL in a cell, the method comprising contacting the cell with the compound or the pharmaceutical composition.

4. A compound of claim 1 or a pharmaceutical composition of claim 2 for use in a method of inhibiting the interaction of menin and MLL1 in a cell, the method comprising contacting the cell with the compound or the pharmaceutical composition.

5. The compound or pharmaceutical composition for use of claim 3 or 4, wherein the contacting is *in vitro* or *in vivo*.

6. A compound of claim 1 or a pharmaceutical composition of claim 2 for use in a method for the treatment or prevention of a disease or disorder associated with the interaction of menin and MLL comprising administering to a subject in need thereof the compound or the pharmaceutical composition,
wherein preferably the disease or disorder is selected from the group consisting of a leukemia, hematologic malignancy, solid tumor cancer, prostate cancer, breast cancer, liver cancer, brain tumor, and diabetes,
wherein more preferably the leukemia is selected from the group consisting of AML, ALL, Mixed Lineage Leukemia, and a leukemia with Partial Tandem Duplications of MLL.

7. The compound or pharmaceutical composition for use of any one of claims 3-6, wherein the subject is a mammal,
wherein preferably the subject is a human.

8. A compound of claim 1 or a pharmaceutical composition of claim 2 for use as a medicament.
